(19) Europäisches Patentamt
European Patent Office
Office européen des brevets

(11) EP 1 946 778 A1

(12) EUROPEAN PATENT APPLICATION

(43) Date of publication:
**23.07.2008 Bulletin 2008/30**

(51) Int Cl.:
***A61K 45/06*** *(2006.01)* ***A61P 3/04*** *(2006.01)*

(21) Application number: **07384008.4**

(22) Date of filing: **16.01.2007**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **Laboratorios del Dr. Esteve S.A.
08041 Barcelona (ES)**

(72) Inventors:
- **Fisas-Escasany, Maria, Angeles
08025 Barcelona (ES)**
- **Buschmann, Helmut, H. Dr.
08960 Sant Just Desvern (ES)**

(74) Representative: **Peters, Hajo et al
Bosch Graf von Stosch Jehle
Patentanwaltsgesellschaft mbH
Flüggenstrasse 13
80639 München (DE)**

# (54) Active substance combination for the treatment of diabetes

(57) The present invention relates to an active substance combination comprising at least one substituted pyrazoline compound and at least one drug, used to treat diabetes, a medicament comprising said active substance combination, a pharmaceutical formulation comprising said active substance combination and the use of said active substance combination for the manufacture of a medicament.

EP 1 946 778 A1

## Description

The present invention relates to an active substance combination comprising at least one substituted pyrazoline compound and at least one drug, used for the treatment of diabetes, a medicament comprising said active substance combination, a pharmaceutical formulation comprising said active substance combination and the use of said active substance combination for the manufacture of a medicament.

Diabetes mellitus can be classified clinically as being either insulin-dependent diabetes (IDDM or Type 1 diabetes) or non-insulin-dependent diabetes (NIDDM or Type 2 diabetes).

Type 1 diabetes is now thought to be an auto-immune disease of the pancreatic I1-cells. Patients develop antibodies to various components of their pancreatic islet cells which in turn leads to their destruction resulting in a loss of their ability to release insulin. Since the pancreas is unable to secrete insulin in its own right, therapy for Type 1 diabetes consists of insulin replacement.

In Type 2 diabetes, metabolic disturbances including obesity, and especially visceral adiposity and dyslipidaemia result in a decrease in the responsiveness of peripheral tissues to insulin ("insulin resistance"). In turn, this stimulates the pancreas to secrete greater concentrations of insulin in order to control the prandial intake of glucose, until a point is reached whereby glucose cannot be effectively handled by the major peripheral tissues involved in glucose storage and metabolism, ie the liver, skeletal muscle and adipocytes, even with greatly increased circulating levels of insulin ("impaired glucose tolerance"). According to the American Diabetes Association 2004 guidelines, a diagnosis of "impaired fasting glucose" occurs at a level of 100 - 125 mg/dL. A positive diagnosis of Type 2 diabetes is made when the patient's fasting plasma glucose concentration reaches or exceeds 126 mg/dL. In pre-diabetes and Type 2 diabetes, because patients often have some residual pancreatic function, they can be treated in the early stages at least with drugs that stimulate insulin release by the pancreas ("insulin secretogogues") and by drugs which potentiate the actions of insulin that is released ("insulin sensitisers"). In the later stages of Type 2 diabetes, patients are often treated with insulin to complement the actions of decreased insulin secretion by their own failing pancreatic β-cells.

As stated in 2005 by the American Diabetes Association (ADA) and the National Institute of Diabetes, Digestive and Kidney Diseases (NIDDKD), diabetes is one of the most costly and burdensome chronic diseases of our time and it is a condition that is increasing to epidemic proportions in the US and throughout the world (ADA & NIDDKD, 2004). The complications resulting from the disease are a significant cause of morbidity and mortality and are associated with the damage or failure of various organs such as the eyes, kidneys, and nerves. Individuals with Type 2 diabetes are also at a significantly higher risk for coronary heart disease, peripheral vascular disease, and stroke, and they have a greater likelihood of having hypertension, dyslipidaemia, and obesity.

There is also growing evidence that at glucose levels above normal but below the diabetes threshold diagnostic now referred to as pre-diabetes, there is a substantially increased risk of cardiovascular disease (CVD) and death. In these individuals, CVD risk factors are also more prevalent which further increases the risk, but is not sufficient to totally explain it.

It was therefore an object of the present invention to provide a way of ameliorating the known ways of treatment diabetes especially avoiding side effects related to that treatment.

It has now surprisingly been found that an active substance combination comprising at least one substituted pyrazoline compound as described herein and a drug used for the treatment of diabetes, does have a very beneficial effect on the treatment.

Thus, in one of its aspects the present invention relates to an active substance combination comprising

(A) at least one substituted pyrazoline compound of general formula I

I

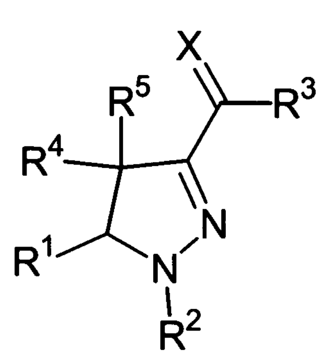

wherein

X is O or S;

$R^1$ and $R^2$, independently of one another, in each case represent an unsubstituted or at least mono-substituted aryl or heteroaryl radical, which may be condensed with an unsubstituted or at least mono-substituted mono-or polycyclic ring system;

or a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bridged by at least one unsubstituted or at least mono-substituted alkylene group;

$R^3$ an unsubstituted or at least mono-substituted aryl or heteroaryl radical, which may be condensed with an unsubstituted or at least mono-substituted mono-or polycyclic ring system;

a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bridged by at least one unsubstituted or at least mono-substituted alkylene group;

a saturated or unsaturated, unsubstituted or at least mono-substituted, optionally at least one heteroatom as a ring member containing cycloaliphatic radical which together with a saturated or unsaturated, unsubstituted or at least mono-substituted, optionally at least one heteroatom as a ring member containing cycloaliphatic radical forms a saturated or unsaturated, unsubstituted or at least mono-substituted, optionally at least one heteroatom as a ring member containing spirocyclic residue via a common ring atom;

a $-O-R^6$ moiety; a $-NR^7R^8$ moiety or a $-NR^9-O-R^{10}$ moiety;

$R^4$ represents H; F; Cl; Br; I; -CN; $-NO_2$; -NC; -OH; $-NH_2$; -SH; -C(=O)-H; -C(=O)-OH; $-C(=O)-OR^{17}$;

a saturated or unsaturated, unsubstituted or at least mono-substituted aliphatic radical;

a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;

an unsubstituted or at least mono-substituted aryl or heteroaryl radical, which may be condensed with an unsubstituted or at least mono-substituted mono-or polycyclic ring system and/or may be bonded via an optionally at least mono-substituted alkylene group, alkenylene group or alkinylene group;

a $-O-R^{11}$ moiety; a $-S-R^{12}$ moiety; a $-NH-R^{13}$ moiety or a $-NR^{14}R^{15}$ moiety;

$R^5$ represents H; F; Cl; Br; I; -CN; $-NO_2$; -NC; -OH; $-NH_2$; -SH; -C(=O)-H; -C(=O)-OH; $-C(=O)-OR^{17}$;

a saturated or unsaturated, unsubstituted or at least mono-substituted aliphatic radical;

a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;

an unsubstituted or at least mono-substituted aryl or heteroaryl radical, which may be condensed with an unsubstituted or at least mono-substituted mono-or polycyclic ring system and/or may be bonded via an unsubstituted or at least mono-substituted alkylene group, alkenylene group or alkinylene group;

a $-O-R^{11}$ moiety; a $-S-R^{12}$ moiety; a $-NH-R^{13}$ moiety or a $-NR^{14}R^{15}$ moiety;

or $R^4$ and $R^5$ together with the bridging carbon atom form a saturated or unsaturated, unsubstituted or at least mono-substituted, optionally at least one heteroatom as a ring member containing cycloaliphatic radical;

$R^6$ represents a hydrogen atom;

a saturated or unsaturated, unsubstituted or at least mono-substituted aliphatic radical;

a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;

an unsubstituted or at least mono-substituted aryl or heteroaryl radical, which may be condensed

with an unsubstituted or at least mono-substituted mono-or polycyclic ring system and/or may be bonded via an unsubstituted or at least mono-substituted alkylene group, alkenylene group or alkinylene group;
a -P(=O)(OR$^{16}$)$_2$ moiety; a -C(=O)OR$^{17}$ moiety; a -C(=O)-NH-R$^{18}$ moiety or a - C(=O)-R$^{19}$ moiety;

R$^7$ and R$^8$, independently of one another, in each case represent a hydrogen atom;
a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
a saturated or unsaturated, unsubstituted or at least mono-substituted, optionally at least one heteroatom as a ring member containing cycloaliphatic radical which together with a saturated or unsaturated, unsubstituted or at least mono-substituted, optionally at least one heteroatom as a ring member containing cycloaliphatic radical forms a saturated or unsaturated, unsubstituted or at least mono-substituted, optionally at least one heteroatom as a ring member containing spirocyclic residue via a common ring atom;
an unsubstituted or at least mono-substituted aryl or heteroaryl radical, which may be condensed with an unsubstituted or at least mono-substituted mono-or polycyclic ring system and/or may be bonded via an unsubstituted or at least mono-substituted alkylene group, alkenylene group or alkinylene group;

a-P(=O)(OR$^{16}$)$_2$ moiety; a -C(=O)-OR$^{17}$ moiety; a -C(=O)-NH-R$^{18}$ moiety; a -C(=O)-R$^{19}$ moiety; a -S(=O)$_2$-R$^{20}$ moiety; or a -NR$^{21}$R$^{22}$ moiety;

R$^9$ represents hydrogen or a saturated or unsaturated, unsubstituted or at least mono-substituted aliphatic radical;

R$^1$, R$^{11}$, R$^{12}$, R$^{13}$, R$^{14}$, R$^{15}$ and R$^{20}$, independently of one another, in each case represent a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;

R$^{16}$, R$^{17}$, R$^{18}$ and R$^{19}$, independently of one another, in each case represent a saturated or unsaturated, unsubstituted or at least mono-substituted aliphatic radical;
or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;
and

R$^{21}$ and R$^{22}$, independently of one another, in each case represent a hydrogen atom;
a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group;
a saturated or unsaturated, unsubstituted or at least mono-substituted, optionally at least one heteroatom as a ring member containing cycloaliphatic radical which together with a saturated or unsaturated, unsubstituted or at least mono-substituted, optionally at least one heteroatom as a ring member containing cycloaliphatic radical forms a saturated or unsaturated, unsubstituted or at least mono-substituted, optionally at least one heteroatom as a ring member containing spirocyclic residue via a common ring atom;
or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;

optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.
and

(B) at least one compound (B) used to treat diabetes.

For the purposes of this invention, the term "Type 2 diabetes" includes all metabolic disorders relating to impaired glycaemic control including, but not limited to, pre-diabetes, eg insulin resistance and/or impaired glucose tolerance, as well as Type 2 diabetes, and in addition, other diseases that have a strong association with pre-diabetes and/or Type 2 diabetes including, but not limited to, diabetic neuropathy and diabetic nephropathy, diabetic retinopathy, peripheral vascular disease, coronary heart disease (CHD), stroke, hypertension, dyslipidaemia (ADA & NIDDKD, 2004) and polycystic ovarian syndrome (Sheehan, 2004) and the Metabolic Syndrome as defined for example by the World Health Organisation (WHO, 1999), the National Cholesterol Education Programme - Third Adult Treatment Panel (NCEP ATP III, 2002), and finally, the recent classification by the International Diabetes Federation (IDF, 2005).

Type 2 diabetes has a complex aetiology; however, it is well known that there is a strong association between obesity and impaired glucose tolerance/insulin resistance or Type 2 diabetes (see reviews by Aronne & Segal, 2002, Pi-Sunyer et al, 2002 and Reaven et al, 2004) and in particular visceral adiposity (see reviews by Aronne & Segal, 2002, Pi-Sunyer et al, 2002 and Reaven et al, 2004). There are also strong associations between impaired glucose tolerance/insulin resistance and dyslipidaemia whereby increased circulating free fatty acids are believed to suppress insulin secretion from the pancreas, decrease glucose uptake by the muscle and other tissues and increase hepatic glucose production, which in turn lead to increased lipolysis, creating a vicious cycle resulting in further increases in FFA production; ultimately this cycle of events leads to the development of Type 2 diabetes (see reviews by Boden & Laakso, 2004 and Bays et al, 2004). Conversely, it is also well known that there is a high degree of association between Type 2 diabetes and dyslipidaemia whereby the latter is characterised by raised plasma levels of small, dense LDL-cholesterol particles, elevated triglycerides and low concentrations of HDL-cholesterol particles (Boden & Laakso, 2004).

For the purposes of this inention, a "compound to treat diabetes" is defined as one that improves impaired glycaemic control. For Type 1 diabetes, it will be insulin replacement therapy, whilst for insulin resistance, impaired glucose tolerance and Type 2 diabetes, it is drugs that stimulate insulin release and/or potentiate insulin's physiological actions and/or insulin replacement therapy.

"Compounds to treat diabetes" to be used in the active substance combination according tote invention are provided in the following non-limiting list of examples including sulphonylureas, including chlorpropamide, tolbutamide, acetoheximide, tolazamide, glibenclamide, glipizide, gliclazide, glimepiride and gliquidone, short-acting post-prandial insulin secreters, including nateglinide, repaglinide and mitiglitinide, PPARγ agonists, including thiazolidinediones including troglitazone, rosiglitazone, pioglitazone, ciglitazone, darglitazone, PPARα/γ agonists, including sipoglitazar, tesaglitazar, muraglitazar, reglitazar, peliglitazar, cevoglitazar, ragaglitazar, naveglitazar, imiglitazar, aloglitazar, sodelglitazar, peliglitazar, pemoglitazar and netoglitazone, biguanides, including metformin, dipeptidyl peptidase-4 (DPP-IV) inhibitors, saxagliptin, sitagliptin, vildagliptin, denagliptin and alogliptin, incretins and incretin analogues, including exendin-4 and liraglutide, amylin analogues, including pramlintide, α-glucosidase inhibitors, including acarbose, voglibose and miglitol, sodium/glucose co-transport inhibitors, including sergliflozin, and insulin preparations, including insulin lispro (human), insulin aspart (human), neutral insulin injection, human neutral insulin (pyr), human neutral insulin (prb), bovine neutral insulin, porcine neutral insulin, human isophane insulin, isophase insulin (prb), human isophane insulin

(pyr), porcine isophane insulin, bovine isophane insulin, human insulin zinc suspension (pyr), humulin (prb), porcine insulin zinc suspension, human insulin zinc suspension (prb) 30% amorphous 70% crystalline, insulin glargine (human), protamine zinc insulin injection (crystalline (prb), bovine insulin zinc suspension, bovine protamine zinc insulin, biphasic human insulin (pyr), biphasic human insulin (prb), biphasic neutral and isophane human, insulin (prb), human insulin (emp), biphasic porcine neutral and isophane insulin, biphasic insulin aspart and insulin determir. It should also be noted that also multiple agents from this list of "compounds to treat diabetes" can be used alone or as combination medicaments.

In this filing, the surprisingly beneficial actions of the pyrazolines as described herein on glycaemic control, together with improvements in metabolic risk factors implicated in impaired glucose tolerance/insulin resistance and the development of Type 2 diabetes, viz reduced obesity, reduced visceral adiposity, reduced plasma triglycerides and reduced plasma cholesterol, are exemplified here.

In the first aspect of this invention, it will be demonstrated in the following Examples that the pyrazolines as described herein have beneficial effects on obesity, visceral adiposity, appetency disorders, insulin resistance/impaired glucose tolerance [pre-diabetes], Type 2 diabetes, Metabolic Syndrome and drug-induced weight-gain/obesity.

In a second aspect of this invention, it will be demonstrated in the following Examples that these beneficial actions on cardio-metabolic risk factors are both dependent and independent of the weight-loss evoked by these agents.

In a third aspect of the invention, it will be demonstrated in the following Examples that within the pyrazolines as described herein some compounds have beneficial effects on obesity, visceral adiposity, appetency disorders, insulin resistance/impaired glucose tolerance [pre-diabetes], Type 2 diabetes, and the Metabolic Syndrome by a novel, and as yet unidentified, pharmacological mechanism of action.

In a fourth aspect of this invention, it will be demonstrated in the following Examples that prevent the drug-induced obesity and weight-gain that is associated with therapy with many of the most widely prescribed "compounds to treat diabetes".

Thus, it can be concluded from the above that an active substance combination according to the invention comprising at least 1 compound selected from the substituted pyrazolines of the general formula 1 together with at least one "compound to treat diabetes" from the non-limiting list given above will be synergistically beneficial to patients suffering from insulin resistance/impaired glucose tolerance [pre-diabetes] or Type 2 diabetes not only because of the direct actions the substituted pyrazolines of the general formula 1 to improve glycaemic control, but also because of their indirect beneficial effects on cardio-metabolic risk factors that mediate and/or exacerbate insulin resistance/impaired glucose tolerance [pre-diabetes], Type 2 diabetes and by their action to evoke weight-loss or prevent drug-induced obesity and weight-gain that is associated with therapy with many of the most widely prescribed "compounds to treat diabetes"

This broad spectrum of therapeutic improvements is predicted to provide additional clinical benefit to a patient with insulin resistance/impaired glucose tolerance [pre-diabetes], or Type 2 diabetes by preventing or ameliorating deleterious changes in food-related, cardio-metabolic risk factors, ie obesity, increased visceral adiposity and/or increases in plasma triglycerides, cholesterol and/or LDL-cholesterol, decreases in HDL-cholesterol, insulin resistance and/or impaired glucose tolerance and/or hypertension that predisposes him/her to a greatly increased risk of progressing from insulin resistance/impaired glucose tolerance [pre-diabetes] to Type 2 diabetes and/or Metabolic Syndrome, of developing drug-induced obesity or weight-gain that results from therapy with many of the most widely prescribed "compounds to treat diabetes" or of suffering a cardio- or cerebrovascular accident.

Preferably the active substance combination according to the present invention comprises as compound (A) one or more substituted pyrazoline compounds of general formula I given above, wherein

X is O or S;

$R^1$ and $R^2$, independently of one another, in each case represent an unsubstituted or at least mono-substituted 6- or 10-membered aryl radical, which may be condensed with an unsubstituted or at least mono-substituted mono- or polycyclic ring system;

an unsubstituted or at least mono-substituted 5- to 14-membered heteroaryl radical, which may be condensed with an unsubstituted or at least mono-substituted mono- or polycyclic ring system;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$alkylene group;

or an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system

and/or may be bridged by at least one unsubstituted or at least mono-substituted $C_{1-5}$alkylene group;

$R^3$ represents an unsubstituted or at least mono-substituted 6- or 10-membered aryl radical, which may be condensed with an unsubstituted or at least mono-substituted mono- or polycyclic ring system;

an unsubstituted or at least mono-substituted 5- to 14-membered heteroaryl radical, which may be condensed with an unsubstituted or at least mono-substituted mono- or polycyclic ring system;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$alkylene group;

an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group;

an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical which together with an unsubstituted or at least mono-substituted $C_{3-16}$ cycloalkyl radical, $C_{4-16}$ cycloalkenyl radical, $C_{4-16}$ heterocycloalkyl radical or **$C_5$-**16 heterocycloalkenyl radical forms an unsubstituted or at least mono-substituted spirocyclic residue via a common ring atom;

a -O-$R^6$ moiety; a -N$R^7R^8$ moiety or a -N$R^9$-O-$R^{10}$ moiety;

$R^4$ represents H; F; Cl; Br; I; -CN; -$NO_2$; -NC; -OH; -$NH_2$; -SH; -C(=O)-H; -C(=O)-OH; -C(=O)-O$R^{17}$;

an unsubstituted or at least mono-substituted $C_{1-16}$ alkyl radical, $C_{2-16}$alkenyl radical or $C_{2-16}$alkinyl radical;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group and/or may be bridged by at least one unsubstituted or at least mono-substituted $C_{1-5}$ alkylene group;

an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$alkenylene group or $C_{2-5}$alkinylene group and/or may be bridged by at least one unsubstituted or at least mono-substituted $C_{1-5}$ alkylene group;

an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$alkylene group, $C_{2-5}$alkenylene group or $C_{2-5}$ alkinylene group;

or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$alkylene group, $C_{2-5}$ alkenylene group or $C_{2-5}$alkinylene group;

a -O-$R^{11}$ moiety; a -S-$R^{12}$ moiety; a -NH-$R^{13}$ moiety or a -N$R^{14}R^{15}$ moiety;

$R^5$ represents H; F; Cl; Br; I; -CN; -$NO_2$; -NC; -OH; -$NH_2$; -SH; -C(=O)-H; -C(=O)-OH; -C(=O)-O$R^{17}$;

an unsubstituted or at least mono-substituted $C_{1-16}$ alkyl radical, $C_{2-16}$ alkenyl radical or $C_{2-16}$ alkinyl radical;

an unsubstituted or at least mono-substituted $C_{3-16}$ cycloalkyl radical or $C_{4-16}$

cycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$alkenylene group or $C_{2-5}$alkinylene group and/or may be bridged by at least one unsubstituted or at least mono-substituted $C_{1-5}$alkylene group;

an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group and/or may be bridged by at least one unsubstituted or at least mono-substituted $C_{1-5}$ alkylene group;

an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$alkylene group, $C_{2-5}$alkenylene group or $C_{2-5}$ alkinylene group;

or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$alkylene group, $C_{2-5}$alkenylene group or $C_{2-5}$ alkinylene group;

-O-$R^{11}$ moiety; a -S-$R^{12}$ moiety; a -NH-$R^{13}$ moiety or a -$NR^{14}R^{15}$ moiety;

or $R^4$ and $R^5$ together with the bridging carbon atom form an unsubstituted or at least mono-substituted $C_{3-16}$ cycloalkyl radical or $C_{4-16}$ cycloalkenyl radical;

$R^6$ represents a hydrogen atom;

an unsubstituted or at least mono-substituted $C_{1-16}$ alkyl radical, $C_{2-16}$ alkenyl radical or $C_{2-16}$ alkinyl radical;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$alkylene group and/or may be bonded via an unsubstituted or at least mono-substituted $C_{1-5}$alkylene group, $C_{2-5}$alkenylene group or $C_{2-5}$ alkinylene group;

an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bridged by at least one unsubstituted or at least mono-substituted $C_{1-5}$alkylene group and/or may be bonded via an unsubstituted or at least mono-substituted $C_{1-5}$alkylene group, $C_{2-5}$ alkenylene group or $C_{2-5}$ alkinylene group;

an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$alkylene group, $C_{2-5}$alkenylene group or $C_{2-5}$ alkinylene group;

an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$alkylene group, $C_{2-5}$ alkenylene group or $C_{2-5}$ alkinylene group;

a -P(=O)($OR^{16}$)$_2$ moiety; a -C(=O)-$OR^{17}$ moiety; a -C(=O)-NH-$R^{18}$ moiety or a - C(=O)-$R^{19}$ moiety;

$R^7$ and $R^8$, independently of one another, in each case represent a hydrogen atom;

an unsubstituted or at least mono-substituted $C_{1-16}$alkyl radical, $C_{2-16}$alkenyl radical or $C_{2-16}$alkinyl radical;

an unsubstituted or at least mono-substituted $C_{3-16}$ cycloalkyl radical or $C_{4-16}$

cycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group and/or may be bonded via an unsubstituted or at least mono-substituted $C_{1-5}$alkylene group, $C_{2-5}$alkenylene group or $C_{2-5}$ alkinylene group;

an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bridged by at least one unsubstituted or at least mono-substituted $C_{1-5}$alkylene group and/or may be bonded via an unsubstituted or at least mono-substituted $C_{1-5}$alkylene group, $C_{2-5}$alkenylene group or $C_{2-5}$ alkinylene group;

an unsubstituted or at least mono-substituted $C_{3-16}$ cycloalkyl radical, $C_{4-16}$ cycloalkenyl radical, $C_{4-16}$heterocycloalkyl radical or $C_{5-16}$heterocycloalkenyl radical which together with an unsubstituted or at least mono-substituted $C_{3-16}$ cycloalkyl radical, $C_{4-16}$cycloalkenyl radical, $C_{4-16}$heterocycloalkyl radical or $C_{5-16}$heterocycloalkenyl radical forms an unsubstituted or at least mono-substituted spirocyclic residue via a common ring atom;

an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$alkylene group, $C_{2-5}$alkenylene group or $C_{2-5}$ alkinylene group;

an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$alkylene group, $C_{2-5}$alkenylene group or $C_{2-5}$alkinylene group;

a $-P(=O)(OR^{16})_2$ moiety; a $-C(=O)-OR^{17}$ moiety; a $-C(=O)-NH-R^{18}$ moiety; a $-C(=O)-R^{19}$ moiety; a $-S(=O)_2-R^{20}$ moiety; or a $-NR^{21}R^{22}$ moiety;

$R^9$ represents a hydrogen atom or an unsubstituted or at least mono-substituted $C_{1-16}$alkyl radical, $C_{2-16}$alkenyl radical or $C_{2-16}$alkinyl radical;

$R^{10}$, $R^{11}$, $R^{12}$, $R^{13}$, $R^{14}$, $R^{15}$ and $R^{20}$, independently of one another, in each case represent an unsubstituted or at least mono-substituted $C_{1-16}$alkyl radical, $C_{2-16}$ alkenyl radical or $C_{2-16}$alkinyl radical;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$alkylene group and/or may be bonded via an unsubstituted or at least mono-substituted $C_{1-5}$alkylene group, $C_{2-5}$alkenylene group or $C_{2-5}$ alkinylene group;

an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bridged by at least one unsubstituted or at least mono-substituted $C_{1-5}$alkylene group and/or may be bonded via an unsubstituted or at least mono-substituted $C_{1-5}$alkylene group, $C_{2-5}$alkenylene group or $C_{2-5}$ alkinylene group;

an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$alkylene group, $C_{2-5}$alkenylene group or $C_{2-5}$ alkinylene group;

or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$alkylene group, $C_{2-5}$ alkenylene group

or $C_{2-5}$alkinylene group;

$R^{16}$, $R^{17}$, $R^{18}$ and $R^{19}$, independently of one another, in each case represent an unsubstituted or at least mono-substituted $C_{1-16}$ alkyl radical, $C_{2-16}$alkenyl radical or $C_{2-16}$ alkinyl radical;
or an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$alkinylene group;
or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$alkylene group, $C_{2-5}$ alkenylene group or $C_{2-5}$ alkinylene group;

and

$R^{21}$ and $R^{22}$, independently of one another, in each case represent a hydrogen atom;
an unsubstituted or at least mono-substituted $C_{1-16}$alkyl radical, $C_{2-16}$alkenyl radical or $C_{2-16}$alkinyl radical;
an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$alkenylene group or $C_{2-5}$alkinylene group;
an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$alkenylene group or $C_{2-5}$alkinylene group;
an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical which together with an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical forms an unsubstituted or at least mono-substituted spirocyclic residue via a common ring atom;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$alkenylene group or $C_{2-5}$ alkinylene group;
or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$alkylene group, $C_{2-5}$ alkenylene group or $C_{2-5}$ alkinylene group;

whereby
the rings of the aforementioned ring system are in each case independently of one another 5- 6- or 7-membered and may in each case independently of one another optionally contain 1, 2 or 3 heteroatom(s) independently selected from the group consisting of nitrogen, oxygen and sulfur;
the aforementioned heteroaryl radicals in each case optionally contain 1, 2, 3 or 4 heteroatom(s) independently selected from the group consisting of nitrogen, oxygen and sulfur as ring member(s);
the aforementioned heterocycloalkyl radicals and heterocycloalkenyl radicals in each case optionally contain 1, 2, 3 or 4 heteroatom(s) independently selected from the group consisting of nitrogen, oxygen and sulfur as ring member(s);
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

Preferably the active substance combination according to the present invention comprises as compound (A) one or more substituted pyrazoline compounds of general formula I given above, wherein

X is O or S;
$R^1$ represents a radical selected from the group consisting of phenyl and thienyl (thiophenyl), which may optionally

be substituted with 1, 2, 3, 4 or 5 substituent(s) independently selected from the group consisting of -OH, $-O-CH_3$, $-O-C_2H_5$, F, Cl, Br and I;

$R^2$ represents a phenyl radical, which may be substituted with 1, 2, 3, 4 or 5 substituent(s) independently selected from the group consisting of -OH, $-O-CH_3$, $-O-C_2H_5$, F, Cl, Br and I;

$R^3$ represents a radical selected from the group consisting of

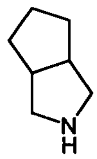 , 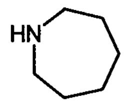 , 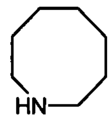 , 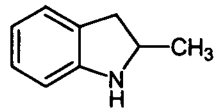 ,

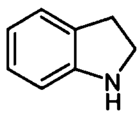 , 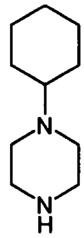 , 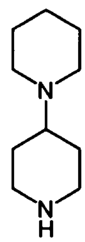 , 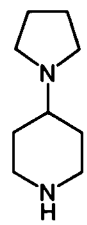 , 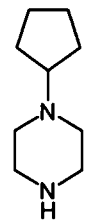 ,

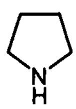 , 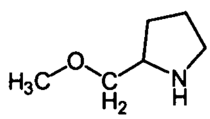 , 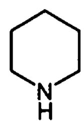 , 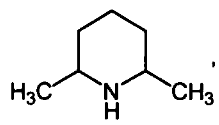 , 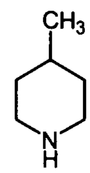 , 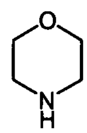 ,

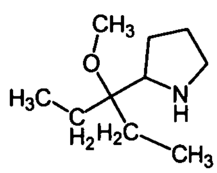 , 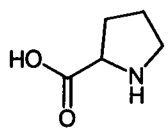 , 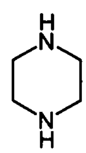 , 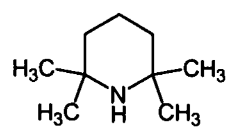 ,

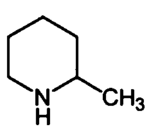 , 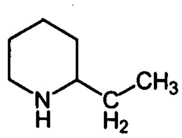 , 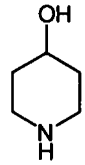 , 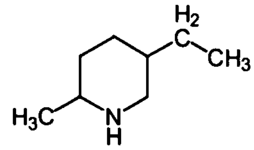 ,

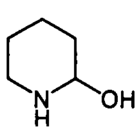 , 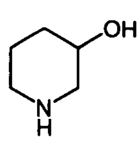 , 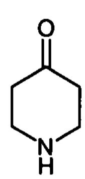 , 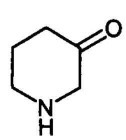 , 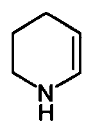 , 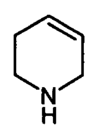 ,

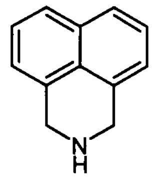 and 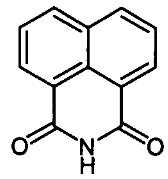 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
a $-OR^6$-moiety or a $-NR^7R^8$ moiety;

$R^4$ represents H; F; Cl; Br; I; -OH, -CN; -C(=O)-H; -C(=O)-OH; $-C(=O)-OR^{17}$;
a radical selected from the group consisting of methyl, $-CF_3$, $-CH_2F$, $-CF_2H$, - $C_2F_5$, ethyl, $-CH_2-CN$, $-CH_2-OH$, n-propyl, isopropyl, $-CH_2-CH_2-CN$, $-CH_2-CH_2-OH$, n-butyl, $-CH_2-CH_2-CH_2-CN$, $-CH_2-CH_2-CH_2-OH$, isobutyl, sec-butyl, tert-butyl, n-pentyl, 2-pentyl, 3-pentyl, neo-pentyl, n-hexyl, 2-hexyl and 3-hexyl;
a benzyl radical or a $-O-R^{11}$ moiety;

$R^5$ represents H; F; Cl; Br;-C(=O)-OH; $-C(=O)-OR^{17}$; or a radical selected from the group consisting of methyl, ethyl, n-propyl, iso-propyl and n-butyl;
or $R^4$ and $R^5$ together with the bridging carbon atom form a radical selected from the group consisting of cyclopropyl, cyclobutyl, cyclopentyl, cyclohexyl, cycloheptyl, cyclooctyl, cyclopentenyl, cyclohexenyl, cycloheptenyl and cyclooctenyl;

$R^6$ represents a hydrogen atom; a radical selected from the group consisting of methyl, ethyl, n-propyl, isopropyl, n-butyl and tert-butyl or a radical selected from the group consisting of cyclopropyl, cyclobutyl, cyclopentyl, cyclohexyl and cycloheptyl;

$R^7$ represents a hydrogen atom or a radical selected from the group consisting of methyl, ethyl, n-propyl, isopropyl, n-butyl, sec-butyl and tert-butyl;

$R^8$ represents a radical selected from the group consisting of [1,2,3,4]-tetrahydronaphthyl, bicyclo[2.2.1]heptyl, cyclopropyl, cyclobutyl, cyclopentyl, cyclohexyl, cycloheptyl, cyclooctyl, cyclononyl, cyclodecyl, cycloundecyl and cyclododecyl, which may be bonded via a $-(CH_2)-$, $-(CH_2)-(CH_2)-$ or $-(CH_2)-(CH_2)-(CH_2)-$group and/or subsituted with 1, 2, 3 or 4 substituents selected from the group consisting of methyl, ethyl, n-propyl, isopropyl, n-butyl, sec-butyl, tert-butyl, -OH, $-O-CH_3$ and $-O-C_2H_5$ ;

or a radical selected from the group consisting of

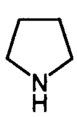 , 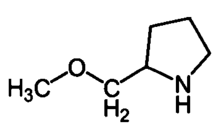 , 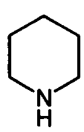 , 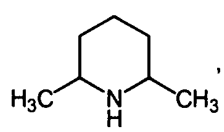 , 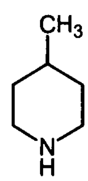 , 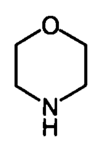 ,

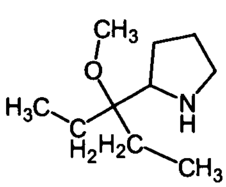 , 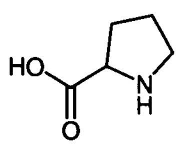 , 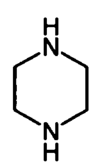 , 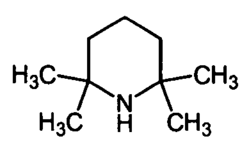 ,

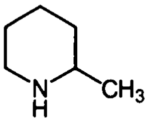, 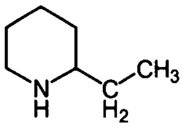, 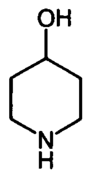, 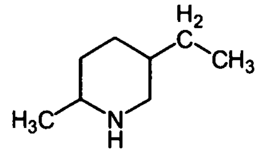,

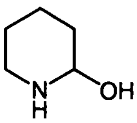, 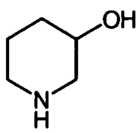, 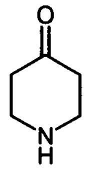, 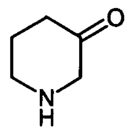, 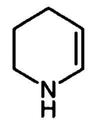, 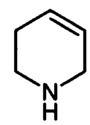,

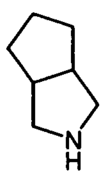, 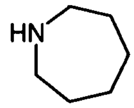, 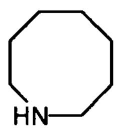, 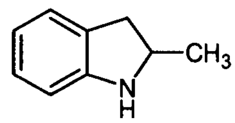,

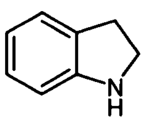, 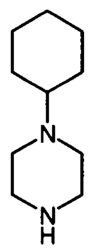, 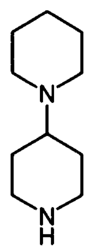, 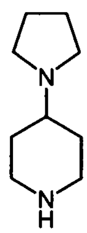, 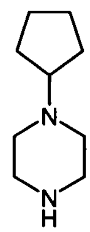,

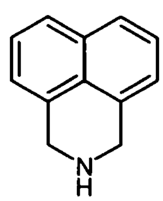 and 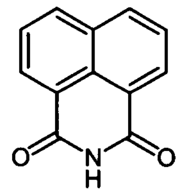 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
and
$R^{11}$ and $R^{17}$, independently of one another, each represent a radical selected from the group consisting of methyl, ethyl, n-propyl, isopropyl, n-butyl, isobutyl, sec-butyl, tert-butyl, n-pentyl, 2-pentyl, 3-pentyl and neo-pentyl;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

As a preferred embodiment the active substance combination according to the present invention comprises as compound (A) of subgroup A1 of general formula I given above, wherein

X represents O

$R^1$ represents a phenyl ring, which is mono-substituted with a halogen atom, preferably a chlorine atom, in its 4-position,

$R^2$ represents a phenyl ring, which is di-substituted with two halogen atoms, preferably chlorine atoms, in its 2- and 4-position,

$R^3$ represents a pyrrolidinyl group; a piperidinyl group; a piperazinyl group; a homo-piperazinyl group; a morpholinyl group; or an $-NR^7R^8$-moiety,

$R^4$ represents a hydrogen atom,

$R^5$ represents a hydrogen atom

$R^7$ represents a hydrogen atom or a linear or branched $C_{1-6}$-alkyl group,

$R^8$ represents a linear or branched $C_{1-6}$ alkyl group; an $SO_2$-$R^{20}$-moiety; a pyrrolidinyl group; a piperidinyl group; a piperazinyl group; a homo-piperazinyl group; a morpholinyl group; or a triazolyl group, whereby each of the heterocyclic rings may be substituted with one or more, identical or different, $C_{1-6}$-alkyl groups, and

$R^{20}$ represents a phenyl group, which is optionally substituted with one or more $C_{1-6}$ alkyl groups, which may be identical or different,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

As a preferred embodiment of subgroup A1 the compound (A) of general formula I given above, is selected from the group consisting of:

N-piperidinyl-5-(4-chloro-phenyl)-1-(2,4-dichlorophenyl)-4,5-dihydro-1H-pyrazol-3-carboxamide,

5-(4-Chloro-phenyl)-1-(2,4-dichloro-phenyl)-4,5-dihydro-1H-pyrazole-3-carboxylic acid-[1,2,4]-triazole-4-yl-amide,

5-(4-Chloro-phenyl)-1-(2,4-dichloro-phenyl)-4,5-dihydro-1H-pyrazole-3-carboxylic acid-(4-methyl-piperazin-1-yl)-amide,

5-(4-Chloro-phenyl)-1-(2,4-dichloro-phenyl)-4,5-dihydro-1H-pyrazole-3-carboxylic acid diethylamide,

[5-(4-Chloro-phenyl)-1-(2,4-dichloro-phenyl)-4,5-dihydro-1H-pyrazole-3-yl]-piperidine-1-yl-methanone,

N-[5-(4-Chloro-phenyl)-1-(2,4-dichlorophenyl)-4,5-dihydro-1H-pyrazole-3-carbonyl]-4-methylphenylsulfonamide,

optionally in the form of a corresponding N-oxide, a corresponding salt or a corresponding solvate.

As a preferred embodiment the active substance combination according to the present invention comprises as compound (A) of subgroup A2 compounds of general formula Ia or Ib

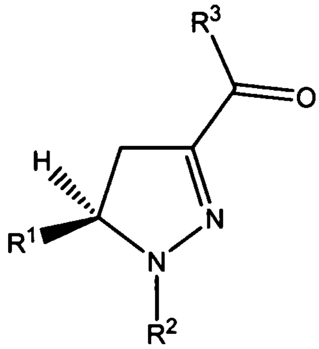
Ia

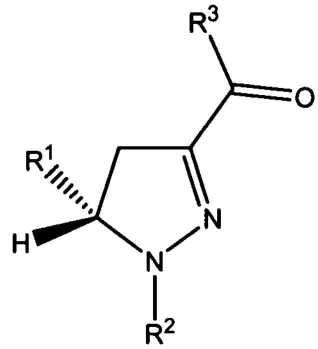
Ib

wherein
X represents O
$R^1$ represents a phenyl ring, which is mono-substituted with a halogen atom, preferably a chlorine atom, in its 4-position,
$R^2$ represents a phenyl ring, which is di-substituted with two halogen atoms, preferably chlorine atoms, in its 2- and 4-position,
$R^3$ represents a pyrrolidinyl group, a piperidinyl group, a piperazinyl group, a homo-piperazinyl group, a morpholinyl group, or an $-NR^7R^8$-moiety,
$R^4$ represents a hydrogen atom,
$R^5$ represents a hydrogen atom,
$R^7$ represents a hydrogen atom or a linear or branched $C_{1-6}$-alkyl group,
$R^8$ represents a linear or branched $C_{1-6}$ alkyl group; an $-SO_2-R^{20}$-moiety; a pyrrolidinyl group; a piperidinyl group; a piperazinyl group; a homo-piperazinyl group; a morpholinyl group; or a triazolyl group whereby each of the heterocyclic rings may be substituted with one or more, identical or different, $C_{1-6}$-alkyl groups, and
$R^{20}$ represents a phenyl group, which is optionally substituted with one or more $C_{1-6}$ alkyl groups, which may be identical or different,
optionally in form of a corresponding N-oxide thereof, or a corresponding salt thereof, or a corresponding solvate thereof.
As a preferred embodiment of compound (A) of subgroup A2 the compound is selected from:

- (R)-N-piperidinyl-5-(4-chlorophenyl)-1-(2,4-dichlorophenyl)-4,5-dihydro-1H-pyrazole-3-carboxamide and
- (S)-N-piperidinyl-5-(4-chlorophenyl)-1-(2,4-dichlorophenyl)-4,5-dihydro-1H-pyrazole-3-carboxamide;
  optionally in form of a corresponding N-oxide thereof, or a corresponding salt thereof, or a corresponding solvate thereof.

As a preferred embodiment the active substance combination according to the present invention comprises as compound (A) of subgroup A3 of general formula I given above, wherein

X is O;
$R^1$ represents a radical selected from the group consisting of

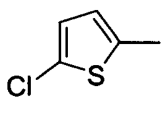 , 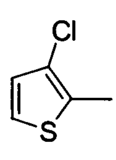 , 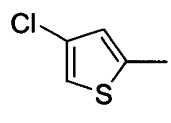 , 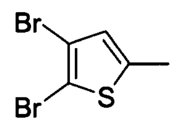 , 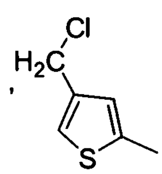 ,

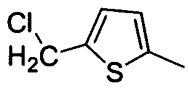, 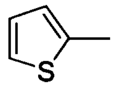, 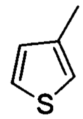, 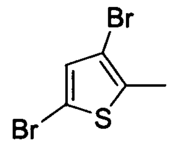, 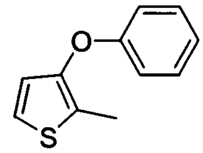,

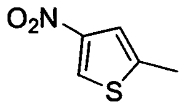, 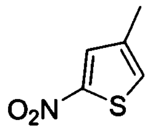, 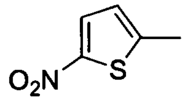, 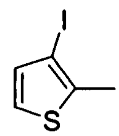, 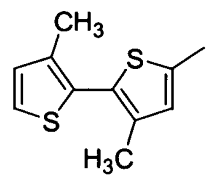,

28

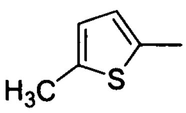, 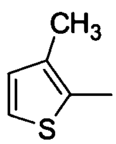, 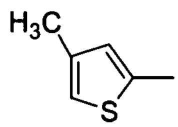, 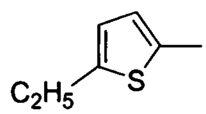, 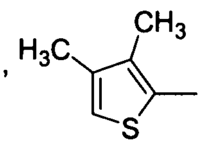,

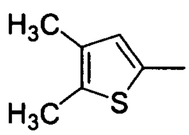, 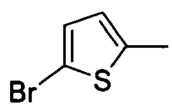, 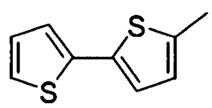, 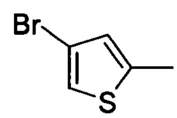,

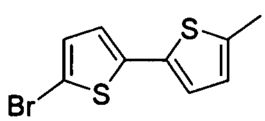, 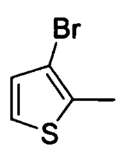, 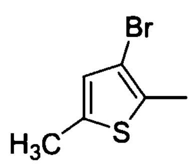, 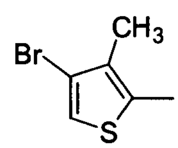,

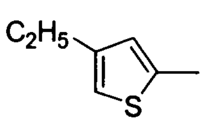, 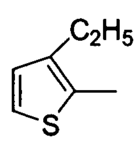, 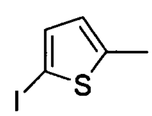 and 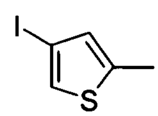;

wherein the single line in each case represents the bond to the pyrazoline compound of general formula I;

$R^2$ represents a phenyl radical, which may be substituted with 1, 2, 3, 4 or 5 substituent(s) independently selected from the group consisting of F, Cl, Br and I;

$R^3$ represents a radical selected from the group consisting

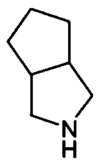 , 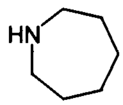 , 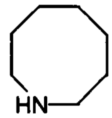 , 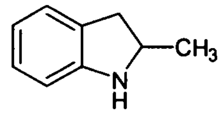 ,

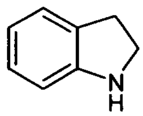 , 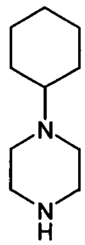 , 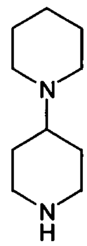 , 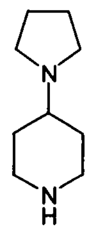 ,

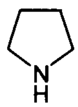 , 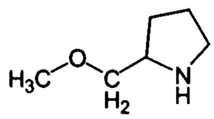 , 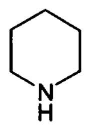 , 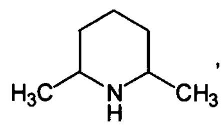 , 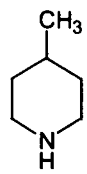 ,

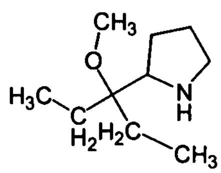 , 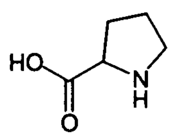 , 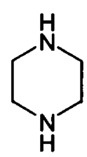 , 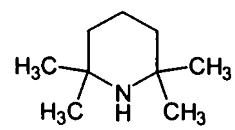 ,

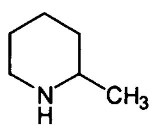 , 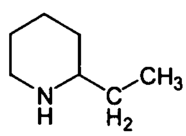 , 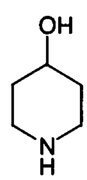 and 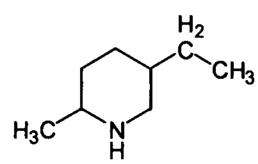 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
a -O-$R^6$ moiety or a -$NR^7R^8$-moiety;

$R^4$ represents a hydrogen atom;

$R^5$ represents a hydrogen atom;

$R^6$ represents a hydrogen atom, a radical selected from the group consisting of methyl, ethyl, n-propyl, isopropyl, n-butyl and tert-butyl or a radical selected from the group consisting of cyclopropyl, cyclobutyl, cyclopentyl, cyclohexyl and cycloheptyl;

$R^7$ represents a hydrogen atom;

$R^8$ a radical selected from the group consisting of cyclopropyl, cyclobutyl, cyclopentyl, cyclohexyl and cycloheptyl, which may be bonded via a -$(CH_2)$-, - $(CH_2)$-$(CH_2)$- or -$(CH_2)$-$(CH_2)$-$(CH_2)$-group;
or a radical selected from the group consisting of

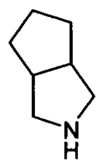, 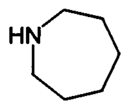, 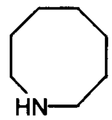, 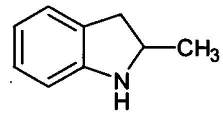,

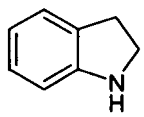, 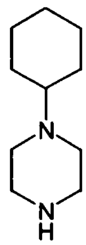, 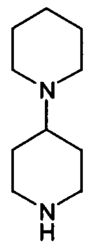, 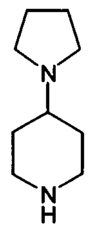,

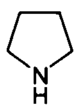, 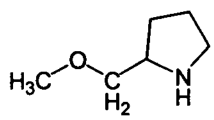, 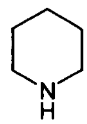, 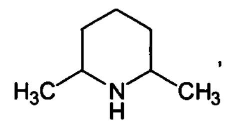, 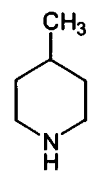,

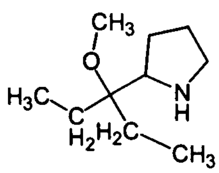, 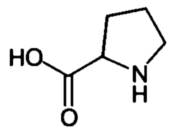, 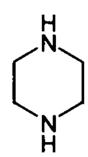, 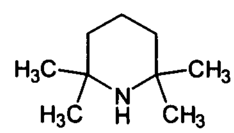,

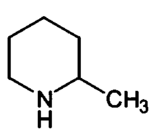, 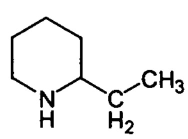, 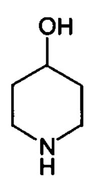 and 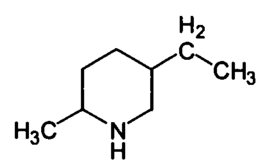;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

As a preferred embodiment of the compound (A) of subgroup A3 the compound is selected from

[1] 5-(5-Chloro-thiophen-2-yl)-1-(2,4-dichloro-phenyl)-4,5-dihydro-1H-pyrazole-3-carboxylic acid piperidin-1-ylamide
[2] 5-(5-Chloro-thiophen-2-yl)-1-(2,4-dichloro-phenyl)-4,5-dihydro-1H-pyrazole-3-carboxylic acid azepan-1-ylamide

[3] 5-(5-Chloro-thiophen-2-yl)-1-(2,4-dichloro-phenyl)-4,5-dihydro-1H-pyrazole-3-carboxylic acid pyrrolidin-1-ylamide
[4] 5-(5-Chloro-thiophen-2-yl)-1-(2,4-dichloro-phenyl)-4,5-dihydro-1H-pyrazole-3-carboxylic acid cyclohexyl-amide
[5] [5-(5-Chloro-thiophen-2-yl)-1-(2,4-dichloro-phenyl)-4,5-dihydro-1H-pyrazol-3-yl]-pyrrolidin-1-yl-methanone
[6] [5-(5-Chloro-thiophen-2-yl)-1-(2,4-dichloro-phenyl)-4,5-dihydro-1H-pyrazol-3-yl]-(4-cyclohexyl-piperazin-1-yl)-methanone
[7] 5-(5-Chloro-thiophen-2-yl)-1-(2,4-dichloro-phenyl)-4,5-dihydro-1H-pyrazole-3-carboxylic acid cyclohexylamide
[8] 5-(5-Chloro-thiophen-2-yl)-1-(2,4-dichloro-phenyl)-4,5-dihydro-1H-pyra zole-3-carboxylic acid
[9] 5-(5-Chloro-thiophen-2-yl)-1-(2,4-dichloro-phenyl)-4,5-dihydro-1H-pyrazole-3-carboxylic acid methyl ester
[10] 5-(5-Chloro-thiophen-2-yl)-1-(2,4-dichloro-phenyl)-4,5-dihydro-1H-pyrazole-3-carboxylic acid ethyl ester
[11] 5-(5-Chloro-thiophen-2-yl)-1-(2,4-dichloro-phenyl)-4,5-dihydro-1H-pyrazole-3-carboxylic acid cyclohexyl ester
[12] 5-(3-Chloro-thiophen-2-yl)-1-(2,4-dichloro-phenyl)-4,5-dihydro-1H-pyrazole-3-carboxylic acid piperidin-1-ylamide
[13] 5-(5-Bromo-thiophen-2-yl)-1-(2,4-dichloro-phenyl)-4,5-dihydro-1H-pyrazole-3-carboxylic acid piperidin-1-ylamide
[14] 5-(5-Bromo-thiophen-2-yl)-1-(2,4-dichloro-phenyl)-4,5-dihydro-1H-pyrazole-3-carboxylic acid (4-methyl-piperidin-1-yl)-amide
[15] 5-(5-Bromo-thiophen-2-yl)-1-(2,4-dichloro-phenyl)-4,5-dihydro-1H-pyrazole-3-carboxylic acid (hexahydro-cyclopenta[c]pyrrol-2-yl)-amide
[16] 5-(5-Bromo-thiophen-2-yl)-1-(2,4-dichloro-phenyl)-4,5-dihydro-1H-pyrazole-3-carboxylic acid (2,3-dihydroindol-1-yl)-amide
[17] 5-(5-Bromo-thiophen-2-yl)-1-(2,4-dichloro-phenyl)-4,5-dihydro-1H-pyrazole-3-carboxylic acid cyclopropylamide
[18] 5-(5-Bromo-thiophen-2-yl)-1-(2,4-dichloro-phenyl)-4,5-dihydro-1H-pyrazole-3-carboxylic acid cyclohexylmethyl-amide
[19] 5-(5-Bromo-thiophen-2-yl)-1-(2,4-dichloro-phenyl)-4,5-dihydro-1 H-pyrazole-3-carboxylic acid
[20] 5-(5-Bromo-thiophen-2-yl)-1-(2,4-dichloro-phenyl)-4,5-dihydro-1H-pyrazole-3-carboxylic acid cyclohexyl ester
[21] 5-(5-Bromo-thiophen-2-yl)-1-(2,4-dichloro-phenyl)-4,5-dihydro-1H-pyrazole-3-carboxylic acid methyl ester
[22] 5-(5-Bromo-thiophen-2-yl)-1-(2,4-dichloro-phenyl)-4,5-dihydro-1H-pyrazole-3-carboxylic acid ethyl ester
[23] 5-(4-Bromo-thiophen-2-yl)-1-(2,4-dichloro-phenyl)-4,5-dihydro-1H-pyrazole-3-carboxylic acid piperidin-1-ylamide hydrochloride
[24] 5-(4-Bromo-thiophen-2-yl)-1-(2,4-dichloro-phenyl)-4,5-dihydro-1H-pyrazole-3-carboxylic acid azepan-1-ylamide
[25] 5-(4-Bromo-thiophen-2-yl)-1-(2,4-dichloro-phenyl)-4,5-dihydro-1H-pyrazole-3-carboxylic acid (4-methyl-piperidin-1-yl)-amide
[26] [5-(4-Bromo-thiophen-2-yl)-1-(2,4-dichloro-phenyl)-4,5-dihydro-1H-pyra zol-3-yl]-piperidin-1-yl-methanone
[27] 5-(4-Bromo-thiophen-2-yl)-1-(2,4-dichloro-phenyl)-4,5-dihydro-1H-pyrazole-3-carboxylic acid
[28] 5-(4-Bromo-thiophen-2-yl)-1-(2,4-dichloro-phenyl)-4,5-dihydro-1H-pyrazole-3-carboxylic acid methyl ester
[29] 5-(4-Bromo-thiophen-2-yl)-1-(2,4-dichloro-phenyl)-4,5-dihydro-1H-pyrazole-3-carboxylic acid ethyl ester
[30] 5-(3-Bromo-thiophen-2-yl)-1-(2,4-dichloro-phenyl)-4,5-dihydro-1H-pyrazole-3-carboxylic acid piperidin-1-ylamide
[31] 5-(3-Bromo-5-methyl-thiophen-2-yl)-1-(2,4-dichloro-phenyl)-4,5-dihydro-1H-pyrazole-3-carboxylic acid piperidin-1-ylamide
[32] 5-(4-Bromo-3-methyl-thiophen-2-yl)-1-(2,4-dichloro-phenyl)-4,5-dihydro-1H-pyrazole-3-carboxylic acid piperidin-1-ylamide
[33] 5-(4,5-Dibromo-thiophen-2-yl)-1-(2,4-dichloro-phenyl)-4,5-dihydro-1H-pyrazole-3-carboxylic acid piperidin-1-ylamide
[34] 5-(3,5-Dibromo-thiophen-2-yl)-1-(2,4-dichloro-phenyl)-4,5-dihydro-1H-pyrazole-3-carboxylic acid piperidin-1-ylamide
[35] 1-(2,4-Dichloro-phenyl)-5-(3-iodo-thiophen-2-yl)-4,5-dihydro-1H-pyrazole-3-carboxylic acid piperidin-1-ylamide
[36] 5-(4-Chloromethyl-thiophen-2-yl)-1-(2,4-dichloro-phenyl)-4,5-dihydro-1H-pyrazole-3-carboxylic acid piperidin-1-ylamide
[37] 1-(2,4-Dichloro-phenyl)-5-(3,4-dimethyl-thiophen-2-yl)-4,5-dihydro-1H-pyrazole-3-carboxylic acid piperidin-1-ylamide
[38] 1-(2,4-Dichloro-phenyl)-5-(4,5-dimethyl-thiophen-2-yl)-4,5-dihydro-1H-pyrazole-3-carboxylic acid piperidin-1-ylamide
[39] 5-[2,2']Bithiophenyl-5-yl-1-(2,4-dichloro-phenyl)-4,5-dihydro-1H-pyrazole-3-carboxylic acid piperidin-1-ylamide

[40] 1-(2,4-Dichloro-phenyl)-5-(5-methyl-thiophen-2-yl)-4,5-dihydro-1H-pyrazole-3-carboxylic acid piperidin-1-ylamide

[41] 1-(2,4-Dichloro-phenyl)-5-(3-methyl-thiophen-2-yl)-4,5-dihydro-1H-pyrazole-3-carboxylic acid piperidin-1-ylamide

[42] 1-(2,4-Dichloro-phenyl)-5-(5-ethyl-thiophen-2-yl)-4,5-dihydro-1 H-pyrazole-3-carboxylic acid piperidin-1-ylamide

[43] 1-(2,4-Dichloro-phenyl)-5-(3,3'-dimethyl-[2,2']bithiophenyl-5-yl)-4,5-dihydro-1H-pyrazole-3-carboxylic acid piperidin-1-ylamide

[44] 1-(2,4-Dichloro-phenyl)-5-(5-nitro-thiophen-2-yl)-4,5-dihydro-1H-pyrazole-3-carboxylic acid piperidin-1-ylamide

[45] 1-(2,4-Dichloro-phenyl)-5-(4-nitro-thiophen-2-yl)-4,5-dihydro-1H-pyrazole-3-carboxylic acid piperidin-1-ylamide

[46] 1-(2,4-Dichloro-phenyl)-5-thiophen-2-yl-4,5-dihydro-1 H-pyrazole-3-carboxylic acid piperidin-1-ylamide

[47] 1-(2,4-Dichloro-phenyl)-5-thiophen-3-yl-4,5-dihydro-1H-pyrazole-3-carboxylic acid piperidin-1-ylamide

and

[48] 1-(2,4-Dichloro-phenyl)-5-(5-nitro-thiophen-3-yl)-4,5-dihydro-1H-pyrazole-3-carboxylic acid piperidin-1-ylamide;

optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

As a preferred embodiment the active substance combination according to the present invention comprises as compound (A) of subgroup A4 of general formula I given above, wherein

X represents S;

$R^1$ represents a phenyl radical which may be substituted with 1, 2, 3, 4 or 5 substituent(s) independently selected from the group consisting of F, Cl, Br, 1, $-O-CH_3$ and $-O-C_2H_5$;

$R^2$ represents a phenyl radical which may be substituted with 1, 2, 3, 4 or 5 substituent(s) independently selected from the group consisting of F, Cl, Br and 1;

$R^3$ represents a radical selected from the group consisting of

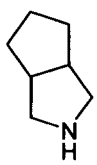 , 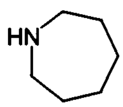 , 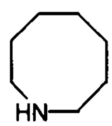 , 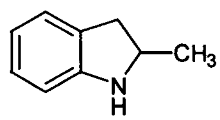 ,

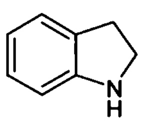 , 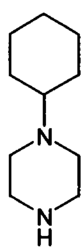 , 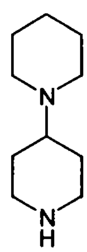 , 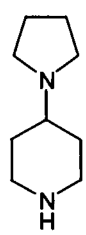 ,

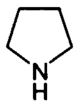 , 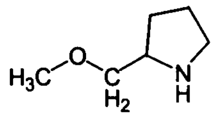 , 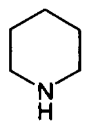 , 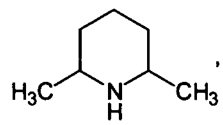 , 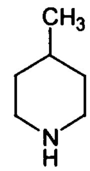 ,

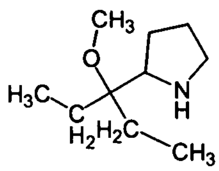 , 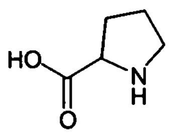 , 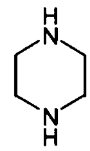 , 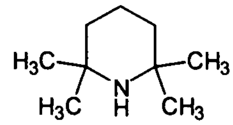 ,

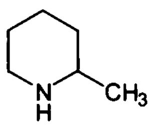 , 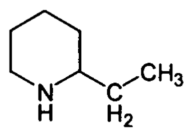 , 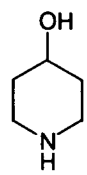 and 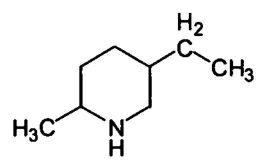 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;

a -O-$R^6$ moiety or a -$NR^7R^8$-moiety;

$R^4$ represents a hydrogen atom;

$R^5$ represents a hydrogen atom;

$R^6$ represents a hydrogen atom or a radical selected from the group consisting of cyclopropyl, cyclobutyl, cyclopentyl, cyclohexyl and cycloheptyl;

$R^7$ represents a hydrogen atom;

$R^8$ a radical selected from the group consisting of cyclopropyl, cyclobutyl, cyclopentyl, cyclohexyl and cycloheptyl, which may be bonded via a -$(CH_2)$-, - $(CH_2)$-$(CH_2)$- or -$(CH_2)$-$(CH_2)$-$(CH_2)$-group ;

or a radical selected from the group consisting of

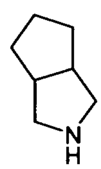 , 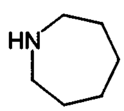 , 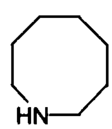 , 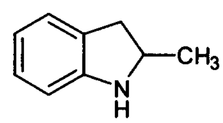 ,

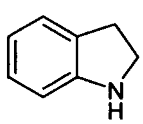 , 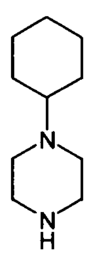 , 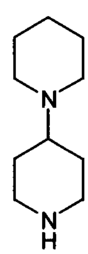 , 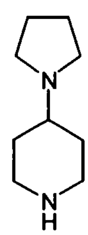 ,

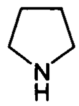 , 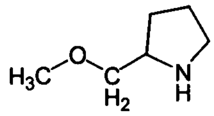 , 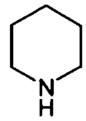 , 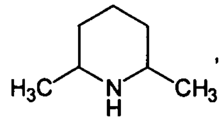 , 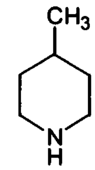 ,

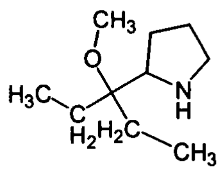 , 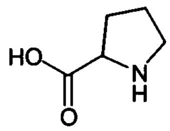 , 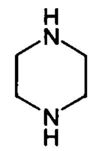 , 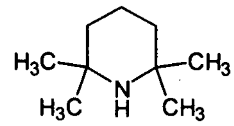 ,

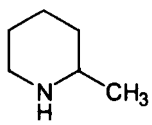 , 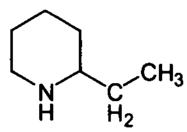 , 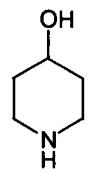 and 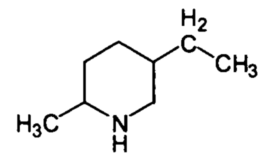 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

As a preferred embodiment the compound (A) of subgroup A4 is selected from:

[1] 5-(4-Chloro-phenyl)-1-(2,4-dichloro-phenyl)-4,5-dihydro-1H-pyrazole-3-carbothioic acid piperidin-1-ylamide
[2] 5-(4-Bromo-phenyl)-1-(2,4-dichloro-phenyl)-4,5-dihydro-1H-pyrazole-3-carbothioic acid piperidin-1-ylamide
[3] 1-(2,4-Dichloro-phenyl)-5-(4-iodo-phenyl)-4,5-dihydro-1H-pyrazole-3-carbothioic acid piperidin-1-ylamide
[4] 1-(2,4-Dichloro-phenyl)-5-(4-fluoro-phenyl)-4,5-dihydro-1H-pyrazole-3-carbothioic acid piperidin-1-ylamide
[5] 1-(2,4-Dichloro-phenyl)-5-(4-methoxy)-4,5-dihydro-1H-pyrazole-3-carbothioic acid piperidin-1-ylamide
[6] 5-(4-Chloro-phenyl)-1-(2,4-dichloro-phenyl)-4,5-dihydro-1H-pyrazole-3-carbothioic acid morpholin-4-ylamide
[7] 5-(4-Bromo-phenyl)-1-(2,4-dichloro-phenyl)-4,5-dihydro-1H-pyrazole-3-carbothioic acid morpholin-4-ylamide
[8] 1-(2,4-Dichloro-phenyl)-5-(4-iodo-phenyl)-4,5-dihydro-1H-pyrazole-3-carbothioic acid morpholin-4-ylamide
[9] 1-(2,4-Dichloro-phenyl)-5-(4-fluoro-phenyl)-4,5-dihydro-1H-pyrazole-3-carbothioic acid morpholin-4-ylamide
[10] 1-(2,4-Dichloro-phenyl)-5-(4-methoxy-phenyl)-4,5-dihydro-1H-pyrazole-3-carbothioic acid morpholin-4-ylamide
[11] 5-(4-Chloro-phenyl)-1-(2,4-dichloro-phenyl)-4,5-dihydro-1H-pyrazole-3-carbothioic acid (hexahydro-cyclopenta[c]pyrrol-2-yl)-amide
[12] 5-(4-Bromo-phenyl)-1-(2,4-dichloro-phenyl)-4,5-dihydro-1H-pyrazole-3-carbothioic acid (hexahydro-cyclopenta[c]pyrrol-2-yl)-amide
[13] 1-(2,4-Dichloro-phenyl)-5-(4-fluoro-phenyl)-4,5-dihydro-1H-pyrazole-3-carbothioic acid (hexahydro-cyclopenta[c]pyrrol-2-yl)-amide
[14] 1-(2,4-Dichloro-phenyl)-5-(4-iodo-phenyl)-4,5-dihydro-1H-pyrazole-3-carbothioic acid (hexahydro-cyclopenta[c]pyrrol-2-yl)-amide
[15] 1-(2,4-Dichloro-phenyl)-5-(4-methoxy-phenyl)-4,5-dihydro-1H-pyrazole-3-carbothioic acid (hexahydro-cyclopenta[c]pyrrol-2-yl)-amide
[16] 1-(2,4-Dichloro-phenyl)-5-(4-iodo-phenyl)-4,5-dihydro-1H-pyrazole-3-carbothioic acid azepan-1-ylamide

[17] 5-(4-Chloro-phenyl)-1-(2,4-dichloro-phenyl)-4,5-dihydro-1H-pyrazole-3-carbothioic acid azepan-1-ylamide
[18] 5-(4-Bromo-phenyl)-1-(2,4-dichloro-phenyl)-4,5-dihydro-1H-pyrazole-3-carbothioic acid azepan-1-ylamide
[19] 1-(2,4-Dichloro-phenyl)-5-(4-fluoro-phenyl)-4,5-dihydro-1H-pyrazole-3-carbothioic acid azepan-1-ylamide
[20] 1-(2,4-Dichloro-phenyl)-5-(4-methoxy-phenyl)-4,5-dihydro-1H-pyrazole-3-carbothioic acid azepan-1-ylamide
[21] 1-(2,4-Dichloro-phenyl)-5-(4-methoxy-phenyl)-4,5-dihydro-1H-pyrazole-3-carbothioic acid pyrrolidin-1-ylamide
[22] 5-(4-Bromo-phenyl)-1-(2,4-dichloro-phenyl)-4,5-dihydro-1H-pyrazole-3-carbothioic acid pyrrolidin-1-ylamide
[23] 5-(4-Chloro-phenyl)-1-(2,4-dichloro-phenyl)-4,5-dihydro-1H-pyrazole-3-carbothioic acid pyrrolidin-1-ylamide
[24] 1-(2,4-Dichloro-phenyl)-5-(4-fluoro-phenyl)-4,5-dihydro-1H-pyrazole-3-carbothioic acid pyrrolidin-1-ylamide
[25] 1-(2,4-Dichloro-phenyl)-5-(4-iodo-phenyl)-4,5-dihydro-1H-pyrazole-3-carbothioic acid pyrrolidin-1-ylamide
[26] 1-(2,4-Dichloro-phenyl)-5-(4-methoxy-phenyl)-4,5-dihydro-1H-pyrazole-3-carbothioic acid (4-cyclopentyl-piperazin-1-yl)-amide
[27] 5-(4-Chloro-phenyl)-1-(2,4-dichloro-phenyl)-4,5-dihydro-1H-pyrazole-3-carbothioic acid (4-cyclopentyl-piperazin-1-yl)-amide
[28] 5-(4-Bromo-phenyl)-1-(2,4-dichloro-phenyl)-4,5-dihydro-1H-pyrazole-3-carbothioic acid (1 H,3H-benzo[de]isoquinolin-2-yl)-amide
[29] 5-(4-Chloro-phenyl)-1-(2,4-dichloro-phenyl)-4,5-dihydro-1H-pyrazole-3-carbothioic acid (1H,3H-benzo[de]isoquinolin-2-yl)-amide
[30] 5-(4-Chloro-phenyl)-1-(2,4-dichloro-phenyl)-4,5-dihydro-1H-pyrazole-3-carbothioic acid cyclopentylamide
[31] Azocan-1-yl-[5-(4-chloro-phenyl)-1-(2,4-dichloro-phenyl)-4,5-dihydro-1H-pyrazole-3-yl]-methanethione and
[32] [5-(4-Chloro-phenyl)-1-(2,4-dichloro-phenyl)-4,5-dihydro-1H-pyrazole-3-yl]-piperidin-1-yl-methanethione;

optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

As a preferred embodiment the active substance combination according to the present invention comprises as compound (A) of subgroup A5.1 of general formula I given above, wherein

X represents O;
$R^1$ represents a phenyl radical that is substituted with a hydroxy, fluorine, chlorine, bromine or iodine atom or a -O-$CH_3$-group in the para-(4-)-position of the phenyl radical;
$R^2$ represents a (2,4)-dichloro-phenyl radical;
$R^3$ represents a radical selected from the group consisting of

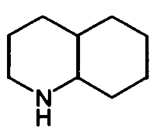 , 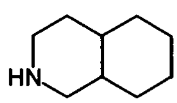 , 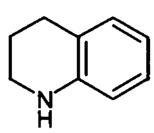 , 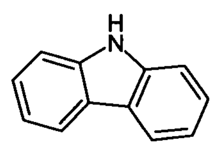 ,

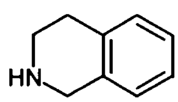 , 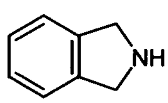 , 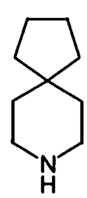 , 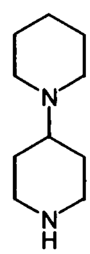 ,

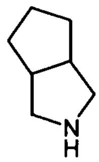 , 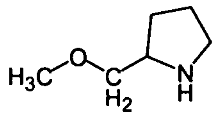 , 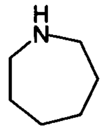 , 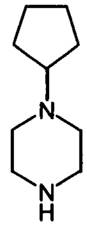 , 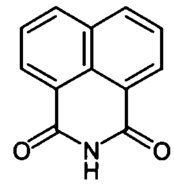 ,

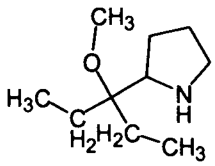 , 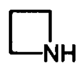 , 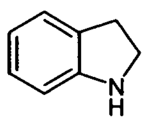 , 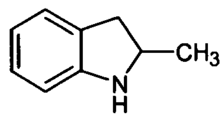 ,

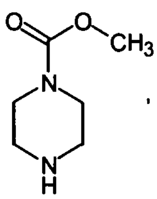 , 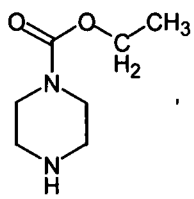 , 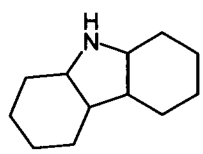 , 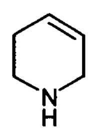 , 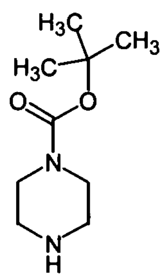 ,

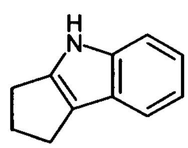 , 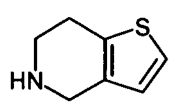 , 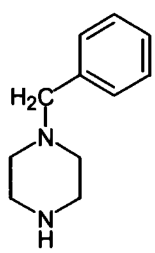 , 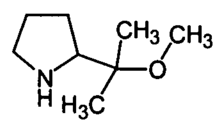 ,

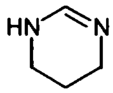 , 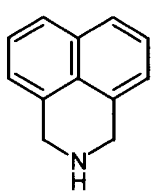 , 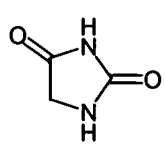 , 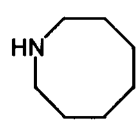 and 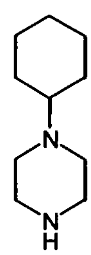 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
or a $-NR^7R^8$ moiety;

$R^4$ represents a hydrogen atom;

$R^5$ represents a hydrogen atom;

$R^7$ represents a hydrogen atom;

$R^8$ represents a radical selected from the group consisting of 2-heptyl, 3-heptyl, 4-heptyl, 2-octyl, 3-octyl, 4-octyl, $-CH_2-N(CH_3)_2$, $-CH_2-N(C_2H_5)_2$, $-CH_2-CH_2-N(CH_3)_2$, $-CH_2-CH_2-N(C_2H_5)_2$, $-CH_2-CH_2-CH_2-N(CH_3)_2$ and $-CH_2-CH_2-CH_2-N(C_2H_5)_2$;

a radical selected from the group consisting of adamantyl, bicyclo[2.2.1]heptyl, bicyclo[3.1.1]heptyl, (1,7,7)-trimethyl-bicyclo[2.2.1]heptyl, [1,2,3,4]-tetrahydronaphthyl, cyclononyl, cyclodecyl, cycloundecyl and cyclododecyl;
a radical selected from the group consisting of cyclopropyl, cyclobutyl, cyclopentyl, cyclohexyl, cycloheptyl, cyclooctyl, pyrrolidinyl, piperidinyl, piperazinyl, homopiperazinyl, morpholinyl, azepanyl, diazepanyl and azocanyl, which is bonded via a $-(CH_2)$, $-(CH_2)-(CH_2)-$ or $-(CH_2)-(CH_2)-(CH_2)$-group;
a substituted radical selected from the group consisting of cyclopentyl, cyclohexyl and cyloheptyl which is substituted with a -O-Benzyl radical;
or a radical selected from the group consisting of

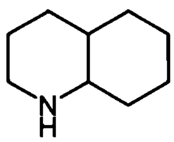 , 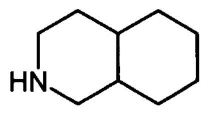 , 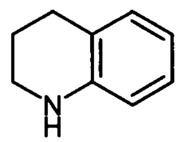 ,

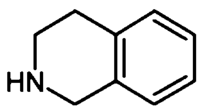 , 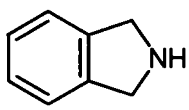 , 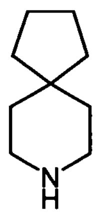 , 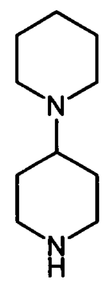 ,

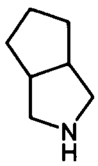 , 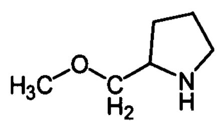 , 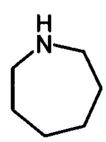 , 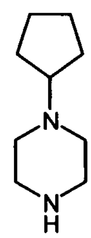 ,

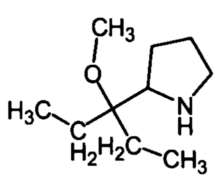 , 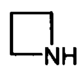 , 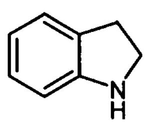 , 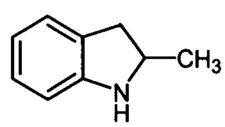 ,

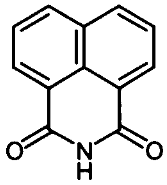 , 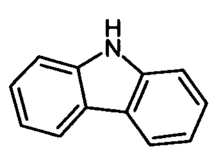 , 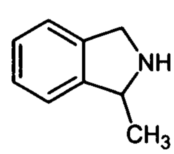 ,

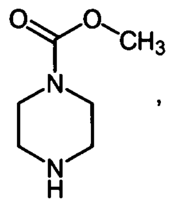 , 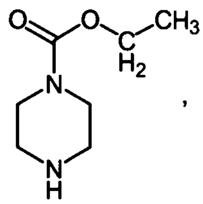 , 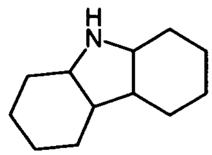 , 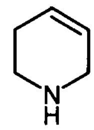 , 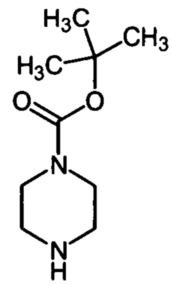 ,

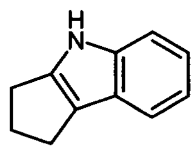 , 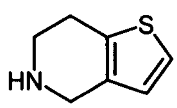 , 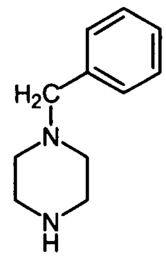 , 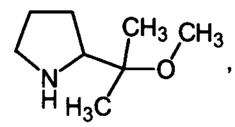 ,

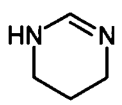 , 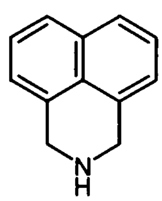 , 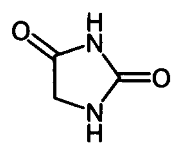 , 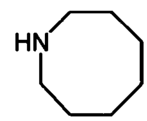 and 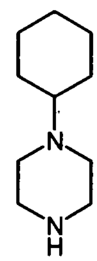 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.
acceptable salt thereof, or a corresponding solvate thereof.

As a preferred embodiment the compound (A) of subgroup A5.1 is selected from:

[10] 5-(4-Chloro-phenyl)-1-(2,4-dichloro-phenyl)-4,5-dihydro-1H-pyrazole-3-carboxylic acid (hexahydro-cyclopenta [c]pyrrol-2-yl)-amide
[13] 5-(4-Chloro-phenyl)-1-(2,4-dichloro-phenyl)-4,5-dihydro-1H-pyrazole-3-carboxylic acid [2-(S)-(1-methoxy-1-methyl-ethyl)-pyrrolidin-1-yl]-amide
[14] 5-(4-Chloro-phenyl)-1-(2,4-dichloro-phenyl)-4,5-dihydro-1H-pyrazole-3-carboxylic acid [2-(R)-(1-methoxy-1-methyl-ethyl)-pyrrolidin-1-yl]-amide
[15] 5-(4-Chloro-phenyl)-1-(2,4-dichloro-phenyl)-4,5-dihydro-1H-pyrazole-3-carboxylic acid [2-(1-methoxy-1-methyl-ethyl)-pyrrolidin-1-yl]-amide
[16] 5-(4-Chloro-phenyl)-1-(2,4-dichloro-phenyl)-4,5-dihydro-1H-pyrazole-3-carboxylic acid azepan-1-ylamide
[21] [5-(4-Chloro-phenyl)-1-(2,4-dichloro-phenyl)-4,5-dihydro-1H-pyrazole-3-yl]-(4-cyclohexyl-piperazin-1-yl)-methanone
[22] [5-(4-Chloro-phenyl)-1-(2,4-dichloro-phenyl)-4,5-dihydro-1H-pyrazole-3-yl]-(octahydro-isoquinolin-2-yl)-methanone
[24] 5-(4-Chloro-phenyl)-1-(2,4-dichloro-phenyl)-4,5-dihydro-1H-pyrazole-3-carboxylic acid (1,3-dioxo-1 H,3H-benzo[de]isoquinolin-2-yl)-amide
[25] 5-(4-Chloro-phenyl)-1-(2,4-dichloro-phenyl)-4,5-dihydro-1H-pyrazole-3-carboxylic acid (1 H,3H-benzo[de]isoquinolin-2-yl)-amide

[26] 5-(4-Chloro-phenyl)-1-(2,4-dichloro-phenyl)-4,5-dihydro-1H-pyrazole-3-carboxylic acid (4-cyclopentyl-piperazin-1-yl)-amide

[27] 5-(4-Chloro-phenyl)-1-(2,4-dichloro-phenyl)-4,5-dihydro-1H-pyrazole-3-carboxylic acid (2-methyl-2,3-dihydro-indol-1-yl)-amide

[28] 5-(4-Chloro-phenyl)-1-(2,4-dichloro-phenyl)-4,5-dihydro-1H-pyrazole-3-carboxylic acid (2,3-dihydro-indol-1-yl)-amide

[32] 5-(4-Chloro-phenyl)-1-(2,4-dichloro-phenyl)-4,5-dihydro-1H-pyrazole-3-carboxylic acid cyclohexylmethyl-amide

[35] 5-(4-Chloro-phenyl)-1-(2,4-dichloro-phenyl)-4,5-dihydro-1H-pyrazole-3-carboxylic acid (1-methyl-hexyl)-amide

[38] Azocan-1-yl-[5-(4-chloro-phenyl)-1-(2,4-dichloro-phenyl)-4,5-dihydro-1H-pyrazol-3-yl]-methanone

[39] [5-[4-Chloro-phenyl]-1-(2,4-dichloro-phenyl)-4,5-dihydro-1H-pyrazole-3-yl]-(1,3-dihydro-isoindo)-2-yl)-methanone

[40] Azetidin-1-yl-[5-(4-chloro-phenyl)-1-(2,4-dichloro-phenyl)-4,5-dihydro-1H-pyrazol-3-yl]-metha none

[42] [1,4']Bipiperidin-1'-yl-[5-(4-chloro-phenyl)-1-(2,4-dichloro-phenyl)-4,5-dihydro-1H-pyrazol-3-yl]-methanone

[44] 4-[5-(4-Chloro-phenyl)-1-(2,4-dichloro-phenyl)-4,5-dihydro-1H-pyrazole-3-carbonyl]-piperazine-1-carboxylic acid ethyl ester

[45] [5-(4-Chloro-phenyl)-1-(2,4-dichloro-phenyl)-4,5-dihydro-1H-pyrazole-3-yl]-(3,4-dihydro-1H-isoquinolin-2-yl)-methanone

[48] 5-(4-Chloro-phenyl)-1-(2,4-dichloro-phenyl)-4,5-dihydro-1H-pyrazole-3-carboxylic acid (2,4-dioxo-imidazolidin-1-yl)-amide

[49] 5-(4-Chloro-phenyl)-1-(2,4-dichloro-phenyl)-4,5-dihydro-1H-pyrazole-3-carboxylic acid cyclododecylamide

[52] (4-Benzy)-piperazin-1-yl)-[5-(4-chloro-phenyl)-1-(2,4-dichloro-phenyl)-4,5-dihydro-1H-pyrazol-3-yl]-methanone

[56] [5-(4-Chloro-phenyl)-1-(2,4-dichloro-phenyl)-4,5-dihydro-1H-pyrazole-3-yl]-(6,7-dihydro-4H-thieno[3,2-c]pyridin-5-yl)-methanone

[58] 5-(4-Chloro-phenyl)-1-(2,4-dichloro-phenyl)-4,5-dihydro-1H-pyrazole-3-carboxylic acid (1,7,7-trimethyl-bicyclo [2.2.1]hept-2-yl)-amide

[63] 5-(4-Bromo-phenyl)-1-(2,4-dichloro-phenyl)-4,5-dihydro-1H-pyrazole-3-carboxylic acid (hexahydro-cyclopenta [c]pyrrol-2-yl)-amide

[64] 5-(4-Bromo-phenyl)-1-(2,4-dichloro-phenyl)-4,5-dihydro-1H-pyrazole-3-carboxylic acid (4-cyclopentyl-piperazin-1-yl)-amide

[65] 5-(4-Bromo-phenyl)-1-(2,4-dichloro-phenyl)-4,5-dihydro-1H-pyrazole-3-carboxylic acid (1 H,3H-benzo[de]isoquinolin-2-yl)-amide

[66] 5-(4-Bromo-phenyl)-1-(2,4-dichloro-phenyl)-4,5-dihydro-1H-pyrazole-3-carboxylic acid (2,3-dihydro-indol-1-yl)-amide hydrochloride

[68] 5-(4-Bromo-phenyl)-1-(2,4-dichloro-phenyl)-4,5-dihydro-1H-pyrazole-3-carboxylic acid azepan-1-yl-amide

[71] 5-(4-Chloro-phenyl)-1-(2,4-dichloro-phenyl)-4,5-dihydro-1H-pyrazole-3-carboxylic acid (2-morpholin-4-yl-ethyl)-amide

[73] 5-(4-Chloro-phenyl)-1-(2,4-dichloro-phenyl)-4,5-dihydro-1H-pyrazole-3-carboxylic acid (3-dimethylamino-propyl)-amide

[74] [5-(4-Chloro-phenyl)-1-(2,4-dichloro-phenyl)-4,5-dihydro-1H-pyrazole-3-yl]-(3,6-dihydro-2H-pyridin-1-yl)-methanone

[75] [5-(4-Chloro-phenyl)-1-(2,4-dichloro-phenyl)-4,5-dihydro-1H-pyrazole-3-yl]-(5,6-dihydro-4H-pyrimidin-1-yl)-methanone

[76] 5-(4-Chloro-phenyl)-1-(2,4-dichloro-phenyl)-4,5-dihydro-1H-pyrazole-3-carboxylic acid (1,2,3,4-tetrahydro-naphthalen-1-yl)-amide

[78] [5-(4-Chloro-phenyl)-1-(2,4-dichloro-phenyl)-4,5-dihydro-1H-pyrazole-3-yl]-(2,3-dihydro-1H-cyclopenta[b]indol-4-ylmethanone

[81] [5-(4-Chloro-phenyl)-1-(2,4-dichloro-phenyl)-4,5-dihydro-1H-pyrazole-3-yl]-(3,4-dihydro-2H-quinolin-1-yl)-methanone

[84] 5-(4-Chloro-phenyl)-1-(2,4-dichloro-phenyl)-4,5-dihydro-1H-pyrazole-3-carboxylic acid (2-cyclohexyl-ethyl)-amide

[85] [5-(4-Chloro-phenyl)-1-(2,4-dichloro-phenyl)-4,5-dihydro-1H-pyrazole-3-yl]-(dodecahydro-carbazol-9-yl)-methanone

[87] 5-(4-Chloro-phenyl)-1-(2,4-dichloro-phenyl)-4,5-dihydro-1H-pyrazole-3-carboxylic acid (2-cyclopentyl-ethyl)-amide

[88] 5-(4-Chloro-phenyl)-1-(2,4-dichloro-phenyl)-4,5-dihydro-1H-pyrazole-3-carboxylic acid bicyclo[2.2.1]hept-2-ylamide

[91] 1-(2,4-Dichloro-phenyl)-5-(4-fluoro-phenyl)-4,5-dihydro-1H-pyrazole-3-carboxylic acid (hexahydro-cyclopenta[c]pyrrol-2-yl)-amide hydrochloride

[92] 1-(2,4-Dichloro-phenyl)-5-(4-fluoro-phenyl)-4,5-dihydro-1H-pyrazole-3-carboxylic acid (4-cyclopentyl-piperazin-1-yl)-amide

[93] 1-(2,4-Dichloro-phenyl)-5-(4-fluoro-phenyl)-4,5-dihydro-1H-pyrazole-3-carboxylic acid (1H,3H-benzo[de]isoquinolin-2-yl)-amide

[94] 1-(2,4-Dichloro-phenyl)-5-(4-fluoro-phenyl)-4,5-dihydro-1H-pyrazole-3-carboxylic acid (2,3-dihydro-indol-1-yl)-amide

[96] 1-(2,4-Dichloro-phenyl)-5-(4-fluoro-phenyl)-4,5-dihydro-1H-pyrazole-3-carboxylic acid azepan-1-yl-amide

[100] 1-(2,4-Dichloro-phenyl)-5-(4-iodo-phenyl)-4,5-dihydro-1H-pyrazole-3-carboxylic acid (hexahydro-cyclopenta[c]pyrrol-2-yl)-amide

[101] 1-(2,4-Dichloro-phenyl)-5-(4-iodo-phenyl)-4,5-dihydro-1H-pyrazole-3-carboxylic acid (4-cyclopentyl-piperazin-1-yl)-amide

[102] 1-(2,4-Dichloro-phenyl)-5-(4-iodo-phenyl)-4,5-dihydro-1 H-pyrazole-3-carboxylic acid (1 H,3H-benzo[de]isoquinolin-2-yl)-amide

[103] 1-(2,4-Dichloro-phenyl)-5-(4-iodo-phenyl)-4,5-dihydro-1H-pyrazole-3-carboxylic acid (2,3-dihydro-indol-1-yl)-amide

[105] 1-(2,4-Dichloro-phenyl)-5-(4-iodo-phenyl)-4,5-dihydro-1H-pyrazole-3-carboxylic acid azepan-1-ylamide;

[109] 1-(2,4-Dichloro-phenyl)-5-(4-methoxy-phenyl)-4,5-dihydro-1H-pyrazole-3-carboxylic acid (hexahydro-cyclopenta[c]pyrrol-2-yl)-amide hydrochloride

[110] 1-(2,4-Dichloro-phenyl)-5-(4-methoxy-phenyl)-4,5-dihydro-1H-pyrazole-3-carboxylic acid (4-cyclopentyl-piperazin-1-yl)-amide

[111] 1-(2,4-Dichloro-phenyl)-5-(4-methoxy-phenyl)-4,5-dihydro-1H-pyrazole-3-carboxylic acid (1 H,3H-benzo[de] isoquinolin-2-yl)-amide

[112] 1-(2,4-Dichloro-phenyl)-5-(4-methoxy-phenyl)-4,5-dihydro-1H-pyrazole-3-carboxylic acid (2,3-dihydro-indol-1-yl)-amide hydrochloride

[114] 1-(2,4-Dichloro-phenyl)-5-(4-methoxy-phenyl)-4,5-dihydro-1H-pyrazole-3-carboxylic acid azepan-1-ylamide hydrochloride

[145] 5-(4-Chloro-phenyl)-1-(2,4-dichloro-phenyl)-4,5-dihydro-1H-pyrazole-3-carboxylic acid azocan-1-ylamide hydrochloride

[146] 5-(4-bromo-phenyl)-1-(2,4-dichloro-phenyl)-4,5-dihydro-1H-pyrazole-3-carboxylic acid azocan-1-ylamide

[147] 5-(4-fluoro-phenyl)-1-(2,4-dichloro-phenyl)-4,5-dihydro-1H-pyrazole-3-carboxylic acid azocan-1-ylamide

[148] 5-(4-methoxy-phenyl)-1-(2,4-dichloro-phenyl)-4,5-dihydro-1H-pyrazole-3-carboxylic acid azocan-1-ylamide

[149] 5-(4-hydroxy-phenyl)-1-(2,4-dichloro-phenyl)-4,5-dihydro-1H-pyrazole-3-carboxylic acid azocan-1-ylamide hydrochloride

[150] 5-(4-iodo-phenyl)-1-(2,4-dichloro-phenyl)-4,5-dihydro-1H-pyrazole-3-carboxylic acid azocan-1-ylamide

[151] N-((1S,2S)-2-(benzyloxy)cyclohexyl)-5-(4-chlorophenyl)-1-(2,4-dichlorophenyl)-4,5-dihydro-1 H-pyrazole-3-carboxamide

[158] 5-(4-Chloro-phenyl)-1-(2,4-dichloro-phenyl)-4,5-dihydro-1H-pyrazole-3-carboxylic acid adamantan-1-ylamide

[159] (R)-5-(4-Chlro-phenyl)-1-(2,4-dichloro-phenyl)-4,5-dihydro-1H-pyrazole-3-carboxylic acid adamantan-1-ylamide

[160] (S)-5-(4-Chloro-phenyl)-1-(2,4-dichloro-phenyl)-4,5-dihydro-1H-pyrazole-3-carboxylic acid adamantan-1-ylamide

[161] 5-(4-bromo-phenyl)-1-(2,4-dichloro-phenyl)-4,5-dihydro-1H-pyrazole-3-carboxylic acid adamantan-1-ylamide

[162] 5-(4-fluoro-phenyl)-1-(2,4-dichloro-phenyl)-4,5-dihydro-1H-pyrazole-3-carboxylic acid adamantan-1-ylamide

[163] 5-(4-methoxy-phenyl)-1-(2,4-dichloro-phenyl)-4,5-dihydro-1H-pyrazole-3-carboxylic acid adamantan-1-ylamide

[164] 5-(4-hydroxy-phenyl)-1-(2,4-dichloro-phenyl)-4,5-dihydro-1H-pyrazole-3-carboxylic acid adamantan-1-ylamide

[165] 5-(4-iodo-phenyl)-1-(2,4-dichloro-phenyl)-4,5-dihydro-1H-pyrazole-3-carboxylic acid adamantan-1-ylamide

[166] 5-(4-Chloro-phenyl)-1-(2,4-dichloro-phenyl)-4,5-dihydro-1H-pyrazole-3-carboxylic acid adamantan-2-ylamide

[167] 5-(4-bromo-phenyl)-1-(2,4-dichloro-phenyl)-4,5-dihydro-1H-pyrazole-3-carboxylic acid adamantan-2-ylamide

[168] 5-(4-fluoro-phenyl)-1-(2,4-dichloro-phenyl)-4,5-dihydro-1H-pyrazole-3-carboxylic acid adamantan-2-ylamide

[169] 5-(4-methoxy-phenyl)-1-(2,4-dichloro-phenyl)-4,5-dihydro-1H-pyrazole-3-carboxylic acid adamantan-2-ylamide

[170] 5-(4-hydroxy-phenyl)-1-(2,4-dichloro-phenyl)-4,5-dihydro-1H-pyrazole-3-carboxylic acid adamantan-2-ylamide

[171] 5-(4-iodo-phenyl)-1-(2,4-dichloro-phenyl)-4,5-dihydro-1H-pyrazole-3-carboxylic acid adamantan-2-ylamide

[173] 1-(2,4-Dichloro-phenyl)-5-(4-hydroxy-phenyl)-4,5-dihydro-1H-pyrazole-3 -carboxylic acid azepan-1-ylamide hydrochloride

[174] 1-(2,4-Dichloro-phenyl)-5-(4-hydroxy-phenyl)-4,5-dihydro-1H-pyrazole-3 -carboxylic acid (hexahydro-cyclopenta[c]pyrrol-2-yl)-amide hydrochloride

[175] 1-(2,4-Dichloro-phenyl)-5-(4-hydroxy-phenyl)-4,5-dihydro-1H-pyrazole-3 -carboxylic acid (2,3-dihydro-indol-1-yl)-amide hydrochloride

[176] (R)-5-(4-Chloro-phenyl)-1-(2,4-dichloro-phenyl)-4,5-dihydro-1H-pyrazole-3-carboxylic acid azocan-1-ylamide hydrochloride

[177] (S)-5-(4-Chloro-phenyl)-1-(2,4-dichloro-phenyl)-4,5-dihydro-1H-pyrazole-3-carboxylic acid azocan-1-ylamide hydrochloride,

[178] N-((1R,2R)-2-(benzyloxy)cyclohexyl)-5-(4-chlorophenyl)-1-(2,4-dichlorophenyl)-4,5-dihydro-1H-pyrazole-3-carboxamide

[179] 1-(2,4-Dichloro-phenyl)-5-(4-fluoro-phenyl)-4,5-dihydro-1H-pyrazole-3-carboxylic acid (2,3-dihydro-indol-1-yl)-amide hydrochloride

[181] (R)-5-(4-Chloro-phenyl)-1-(2,4-dichloro-phenyl)-4,5-dihydro-1H-pyrazole-3-carboxylic acid (2,3-dihydro-indol-1-yl)-amide hydrochloride

[182] (S)-5-(4-Chloro-phenyl)-1-(2,4-dichloro-phenyl)-4,5-dihydro-1H-pyrazole-3-carboxylic acid (2,3-dihydro-indol-1-yl)-amide hydrochloride

[183] (S)-5-(4-Chloro-phenyl)-1-(2,4-dichloro-phenyl)-4,5-dihydro-1H-pyrazole-3-carboxylic acid azepan-1-ylamide hydrochloride

[184] (S)-5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide hydrochloride

[185] (S)-5-(4-Chloro-phenyl)-1-(2,4-dichloro-phenyl)-4,5-dihydro-1 H-pyrazole-3-carboxylic acid azepan-1-ylamide

[187] (R)-5-(4-Chloro-phenyl)-1-(2,4-dichloro-phenyl)-4,5-dihydro-1H-pyrazole-3-carboxylic acid azepan-1-ylamide hydrochloride

[188] (R)-5-(4-Chloro-phenyl)-1-(2,4-dichloro-phenyl)-4,5-dihydro-1 H-pyrazole-3-carboxylic acid (hexahydro-cyclopenta[c]pyrrol-2-yl)-amide

[190] (R)- 5-(4-Chloro-phenyl)-1-(2,4-dichloro-phenyl)-4,5-dihydro-1 H-pyrazole-3-carboxylic acid (2,3-dihydro-indol-1-yl)-amide

[191] 5-(4-Chloro-phenyl)-1-(2,4-dichloro-phenyl)-4,5-dihydro-1H-pyrazole-3-carboxylic acid cycloheptylmethyl-amide

[193] 5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide hydrochloride

optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

As a preferred embodiment the active substance combination according to the present invention comprises as compound (A) of subgroup A6.1 of general formula I given above, wherein

- X is O or S;
- $R^1$ represents a radical selected from the group consisting of phenyl and thienyl (thiophenyl), which may optionally be substituted with 1, 2, 3, 4 or 5 substituent(s) independently selected from the group consisting of -OH, -O-$CH_3$, -O-$C_2H_5$, F, Cl, Br and I;
- $R^2$ represents a phenyl radical, which may be substituted with 1, 2, 3, 4 or 5 substituent(s) independently selected from the group consisting of -OH, -O-$CH_3$, -O-$C_2H_5$, F, Cl, Br and I;
- $R^3$ represents a radical selected from the group consisting of

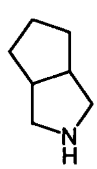 , 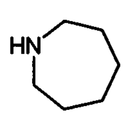 , 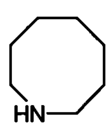 , 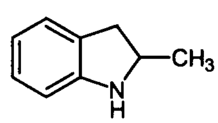 ,

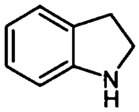 , 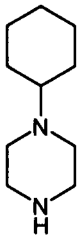 , 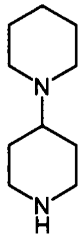 , 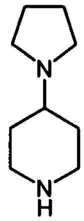 , 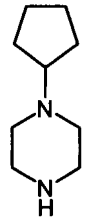 ,

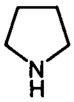 , 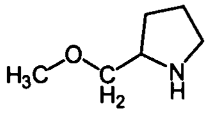 , 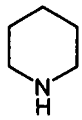 , 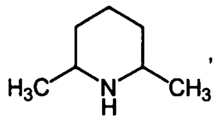 , 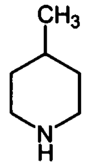 , 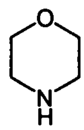 ,

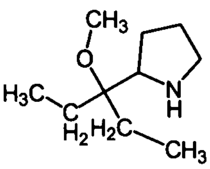 , 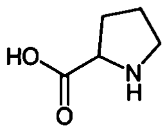 , 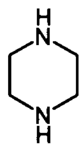 , 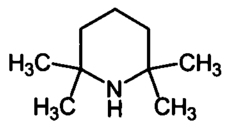 ,

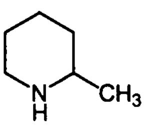 , 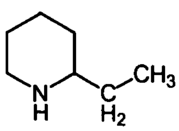 , 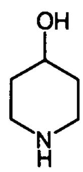 , 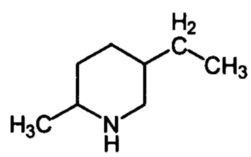 ,

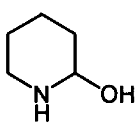 , 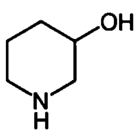 , 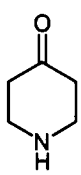 , 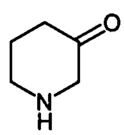 , 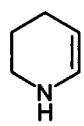 , 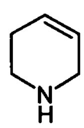 ,

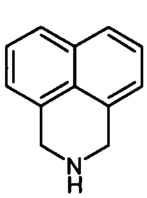 and 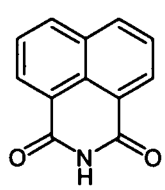 ;

which is in each case bonded to the pyrazoline compound of general formula I in any position of the cyclic part of the aforementioned radicals including the NH-groups,

preferably said radicals are bonded to the pyrazoline compound of general formula I at the nitrogen atom of the cyclic part of the aforementioned radicals;
a -$OR^6$-moiety or a -$NR^7R^8$ moiety;

$R^4$ represents F; Cl; Br; I; -OH, -CN; -C(=O)-H; -C(=O)-OH; -C(=O)-$OR^{17}$;
a radical selected from the group consisting of methyl, -$CF_3$, -$CH_2F$, -$CF_2H$, - $C_2F_5$, ethyl, -$CH_2$-CN, -$CH_2$-OH, n-propyl, isopropyl, -$CH_2$-$CH_2$-CN, -$CH_2$-$CH_2$-OH, n-butyl, -$CH_2$-$CH_2$-$CH_2$-CN, -$CH_2$-$CH_2$-$CH_2$-OH, isobutyl, sec-butyl, tert-butyl, n-pentyl, 2-pentyl, 3-pentyl, neo-pentyl, n-hexyl, 2-hexyl and 3-hexyl;
a benzyl radical or a -O-$R^{11}$ moiety;

$R^5$ represents H; F; Cl; Br;-C(=O)-OH; -C(=O)-$OR^{17}$; or a radical selected from the group consisting of methyl, ethyl, n-propyl, iso-propyl and n-butyl;
or $R^4$ and $R^5$ together with the bridging carbon atom form a radical selected from the group consisting of cyclopropyl, cyclobutyl, cyclopentyl, cyclohexyl, cycloheptyl, cyclooctyl, cyclopentenyl, cyclohexenyl, cycloheptenyl and cyclooctenyl;

$R^6$ represents a hydrogen atom; a radical selected from the group consisting of methyl, ethyl, n-propyl, isopropyl, n-butyl and tert-butyl or a radical selected from the group consisting of cyclopropyl, cyclobutyl, cyclopentyl, cyclohexyl and cycloheptyl;

$R^7$ represents a hydrogen atom or a radical selected from the group consisting of methyl, ethyl, n-propyl, isopropyl, n-butyl, sec-butyl and tert-butyl;

$R^8$ represents a radical selected from the group consisting of [1,2,3,4]-tetrahydronaphthyl, bicyclo[2.2.1]heptyl, cyclopropyl, cyclobutyl, cyclopentyl, cyclohexyl, cycloheptyl, cyclooctyl, cyclononyl, cyclodecyl, cycloundecyl and cyclododecyl, which may be bonded via a -($CH_2$)-, -($CH_2$)-($CH_2$)- or -($CH_2$)-($CH_2$)-($CH_2$)-group and/or subsituted with 1, 2, 3 or 4 substituents selected from the group consisting of methyl, ethyl, n-propyl, isopropyl, n-butyl, sec-butyl, tert-butyl, -OH, -O-$CH_3$ and -O-$C_2H_5$;
or a radical selected from the group consisting of

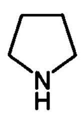 , 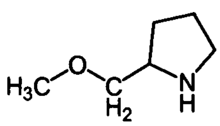 , 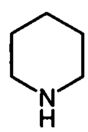 , 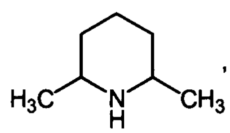 , 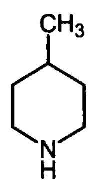 , 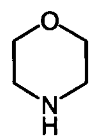 ,

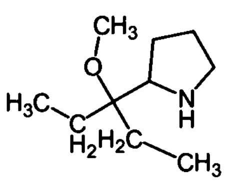 , 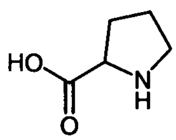 , 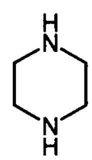 , 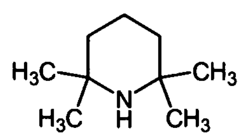 ,

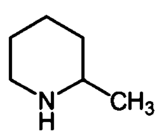 , 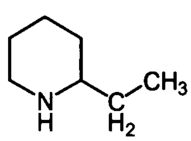 , 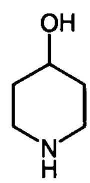 , 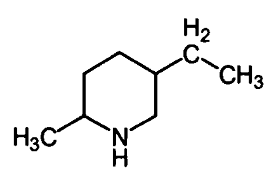 ,

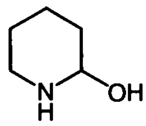, 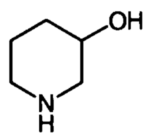, 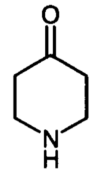, 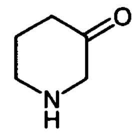, 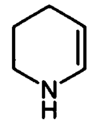, 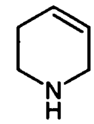,

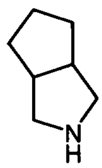, 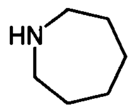, 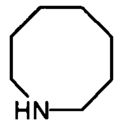, 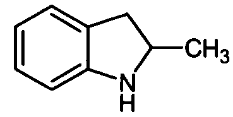,

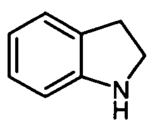, 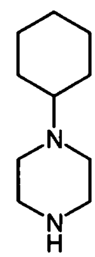, 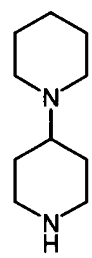, 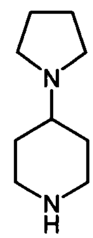, 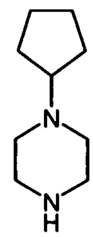,

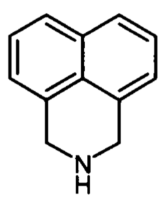 and 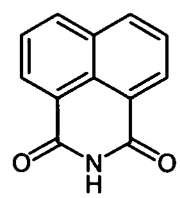 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
and

$R^{11}$ and $R^{17}$, independently of one another, each represent a radical selected from the group consisting of methyl, ethyl, n-propyl, isopropyl, n-butyl, isobutyl, sec-butyl, tert-butyl, n-pentyl, 2-pentyl, 3-pentyl and neo-pentyl;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

As a preferred embodiment the compound (A) of subgroup A6.1 is selected from:

[1] *cis*-5-(4-Chloro-phenyl)-1-(2,4-dichloro-phenyl)-4-methyl-4,5-dihydro-1H-pyrazole-3-carboxylic acid piperidin-1-ylamide hydro-chloride
[2] *trans*-5-(4-Chloro-phenyl)-1-(2,4-dichloro-phenyl)-4-methyl-4,5-dihydro-1H-pyrazole-3-carboxylic acid piperidin-1-ylamide hydro-chloride
[3] *cis*-5-(4-Chloro-phenyl)-1-(2,4-dichloro-phenyl)-4-methyl-4,5-dihydro-1H-pyrazole-3-carboxylic acid azepan-1-ylamide

[4] *trans*-5-(4-Chloro-phenyl)-1-(2,4-dichloro-phenyl)-4-methyl-4,5-dihydro-1H-pyrazole-3-carboxylic acid azepan-1-ylamide
[5] *cis*-[5-(4-Chloro-phenyl)-1-(2,4-dichloro-phenyl)-4-methyl-4,5-dihydro-1H-pyrazol-3-yl]-(4-cyclohexyl-piperazin-1-yl)-methanone
[6] *trans*-[5-(4-Chloro-phenyl)-1-(2,4-dichloro-phenyl)-4-methyl-4,5-dihydro-1H-pyrazol-3-yl]-(4-cyclohexyl-piperazin-1-yl)-methanone
[7] *cis*-5-(4-Chloro-phenyl)-1-(2,4-dichloro-phenyl)-4-methyl-4,5-dihydro-1H-pyrazole-3-carboxylic acid pyrrolidin-1-ylamide
[8] *trans*-5-(4-Chloro-phenyl)-1-(2,4-dichloro-phenyl)-4-methyl-4,5-dihydro-1H-pyrazole-3-carboxylic acid pyrrolidin-1-ylamide
[9] *cis*-5-(4-Chloro-phenyl)-1-(2,4-dichloro-phenyl)-4-methyl-4,5-dihydro-1H-pyrazole-3-carboxylic acid (4-methyl-piperidin-1-yl)-amide
[10] *trans*-5-(4-Chloro-phenyl)-1-(2,4-dichloro-phenyl)-4-methyl-4,5-dihydro-1H-pyrazole-3-carboxylic acid (4-methyl-piperidin-1-yl)-amide
[11] *cis*-[5-(4-Chloro-phenyl)-1-(2,4-dichloro-phenyl)-4-methyl-4,5-dihydro-1H-pyrazol-3-yl]-piperidin-1-yl-methanone
[12] *trans*-[5-(4-Chloro-phenyl)-1-(2,4-dichloro-phenyl)-4-methyl-4,5-dihydro-1H-pyrazol-3-yl]-piperidin-1-yl-methanone
[13] *cis*-5-(4-Chloro-phenyl)-1-(2,4-dichloro-phenyl)-4-methyl-4,5-dihydro-1 H-pyrazole-3-carboxylic acid (hexahydro-cyclopenta[c]pyrrol-2-yl)-amide
[14] *trans*-5-(4-Chloro-phenyl)-1-(2,4-dichloro-phenyl)-4-methyl-4,5-dihydro-1H-pyrazole-3-carboxylic acid (hexahydro-cyclopenta-[c]py-rrol-2-yl)-amide
[15] *cis*-5-(4-Chloro-phenyl)-1-(2,4-dichloro-phenyl)-4-methyl-4,5-dihydro-1H-pyrazole-3-carboxylic acid (2,3-dihydro-indol-1-yl)-amide
[16] *trans*-5-(4-Chloro-phenyl)-1-(2,4-dichloro-phenyl)-4-methyl-4,5-dihydro-1H-pyrazole-3-carboxylic acid (2,3-dihydro-indol-1-yl)-amide
[17] cis-5-(4-Chloro-phenyl)-1-(2,4-dichloro-phenyl)-4-methyl-4,5-dihydro-1 H-pyrazole-3-carboxylic acid cyclobutylamide
[18] 5-(4-Chloro-phenyl)-1-(2,4-dichloro-phenyl)-4-methyl-4,5-dihydro-1H-pyrazole-3-carboxylic acid cyclohexyl methyl-amide
[19] *cis*-5-(4-Chloro-phenyl)-1-(2,4-dichloro-phenyl)-4-methyl-4,5-dihydro-1H-pyrazole-3-carboxylic acid
[20] *trans*-5-(4-Chloro-phenyl)-1-(2,4-dichloro-phenyl)-4-methyl-4,5-dihydro-1H-pyrazole-3-carboxylic acid
[21] *cis*-5-(4-Chloro-phenyl)-1-(2,4-dichloro-phenyl)-4-ethyl-4,5-dihydro-1H-pyrazole-3-carboxylic acid
[22] *trans*-5-(4-Chloro-phenyl)-1-(2,4-dichloro-phenyl)-4-ethyl-4,5-dihydro-1H-pyrazole-3-carboxylic acid piperidin-1-ylamide
[23] *cis*-5-(4-Chloro-phenyl)-1-(2,4-dichloro-phenyl)-4-methyl-4,5-dihydro-1H-pyrazole-3-carboxylic acid methyl ester
[24] *trans*-5-(4-Chloro-phenyl)-1-(2,4-dichloro-phenyl)-4-methyl-4,5-dihydro-1H-pyrazole-3-carboxylic acid methyl ester
[25] *cis*-5-(4-Chloro-phenyl)-1-(2,4-dichloro-phenyl)-4-methyl-4,5-dihydro-1H-pyrazole-3-carboxylic acid ethyl ester
[26] *trans*-5-(4-Chloro-phenyl)-1-(2,4-dichloro-phenyl)-4-methyl-4,5-dihydro-1H-pyrazole-3-carboxylic acid ethyl ester
[27] 5-(4-Chloro-phenyl)-1-(2,4-dichloro-phenyl)-4-methyl-4,5-dihydro-1H-pyrazole-3-carboxylic acid cyclohe-xyl ester
[28] 5-(4-Chloro-phenyl)-1-(2,4-dichloro-phenyl)-4,4-dimethyl-4,5-dihydro-1-H-pyrazole-3-carboxylic acid
[29] 5-(4-Chloro-phenyl)-1-(2,4-dichloro-phenyl)-4,4-dimethyl-4,5-dihydro-1-H-pyrazole-3-carboxylic acid piperidin-1-ylamide
[30] 5-(4-Chloro-phenyl)-1-(2,4-dichloro-phenyl)-4-trifluoromethyl-4,5-dihydro-1H-pyrazole-3-carboxylic acid piperidin-1-ylamide
[31] 5-(4-Chloro-phenyl)-1-(2,4-dichloro-phenyl)-4-hydroxy-4,5-dihydro-1H-pyrazole-3-carboxylic acid
[32] 5-(4-Chloro-phenyl)-1-(2,4-dichloro-phenyl)-4-hydroxy-4,5-dihydro-1H-pyrazole-3-carboxylic acid piperidin-1-ylamide
[33] 5-(4-Chloro-phenyl)-1-(2,4-dichloro-phenyl)-4-(2-hydroxy-ethyl)-4,5-di-hydro-1 H-pyrazole-3-carboxylic acid piperidin-1-ylamide
[34] 5-(4-Chloro-phenyl)-1-(2,4-dichloro-phenyl)-4-(2-hydroxy-ethyl)-4,5-di-hydro-1 H-pyrazole-3-carboxylic acid
[35] 5-(4-Chloro-phenyl)-1-(2,4-dichloro-phenyl)-4-fluoromethyl-4,5-dihydro-1H-pyrazole-3-carboxylic acid piperidin-1-ylamide
[36] 5-(4-Chloro-phenyl)-1-(2,4-dichloro-phenyl)-4-fluoromethyl-4,5-dihydro-1H-pyrazole-3-carboxylic acid

[37] 5-(4-Chloro-phenyl)-1-(2,4-dichloro-phenyl)-4-formyl-4,5-dihydro-1H-pyrazole-3-carboxylic acid piperidin-1-ylamide
[38] 5-(4-Chloro-phenyl)-1-(2,4-dichloro-phenyl)-4-formyl-4,5-dihydro-1H-pyrazole-3-carboxylic acid
[39] 5-(4-chlorophenyl)-4-cyano-1-(2,4-dichlorophenyl)-N-(piperidin-1-yl)-4,5-dihydro-1H-pyrazole-3-carboxamide
[40] 5-(4-Chloro-phenyl)-4-cyano-1-(2,4-dichloro-phenyl)-4,5-dihydro-1H-pyrazole-3-carboxylic acid
[41] 1-(2,4-Dichloro-phenyl)-5-(4-fluoro-phenyl)-4-methyl-4,5-dihydro-1H-pyrazole-3-carboxylic acid piperidin-1-ylamide
[42] 1-(2,4-Dichloro-phenyl)-4-ethyl-5-(4-fluoro-phenyl)-4,5-dihydro-1H-pyrazole-3-carboxylic acid piperidin-1-ylamide
[43] 1-(2,4-Dichloro-phenyl)-5-(4-fluoro-phenyl)-4,4-dimethyl-4,5-dihydro-1-H-pyrazole-3-carboxylic acid piperidin-1-ylamide
[44] 1-(2,4-Dichloro-phenyl)-5-(4-fluoro-phenyl)-4-trifluoromethyl-4,5-dihydro-1 H-pyrazole-3-carboxylic acid piperidin-1-ylamide
[45] 1-(2,4-Dichloro-phenyl)-5-(4-fluoro-phenyl)-4-hydroxy-4,5-dihydro-1H-pyrazole-3-carboxylic acid piperidin-1-ylamide
[46] 1-(2,4-Dichloro-phenyl)-5-(4-fluoro-phenyl)-4-(2-hydroxy-ethyl)-4,5-dihydro-1H-pyrazole-3-carboxylic acid piperidin-1-ylamide
[47] 1-(2,4-Dichloro-phenyl)-4-fluoromethyl-5-(4-fluoro-phenyl)-4,5-dihydro-1H-pyrazole-3-carboxylic acid piperidin-1-ylamide
[48] 1-(2,4-Dichloro-phenyl)-5-(4-fluoro-phenyl)-4-formyl-4,5-dihydro-1 H-pyrazole-3-carboxylic acid piperidin-1-ylamide
[49] 4-Cyano-1-(2,4-dichloro-phenyl)-5-(4-fluoro-phenyl)-4,5-dihydro-1 H-pyrazole-3-carboxylic acid piperidin-1-ylamide
[50] 5-(4-Bromo-phenyl)-1-(2,4-dichloro-phenyl)-4-methyl-4,5-dihydro-1 H-pyrazole-3-carboxylic acid piperidin-1-ylamide
[51] 5-(4-Bromo-phenyl)-1-(2,4-dichloro-phenyl)-4-ethyl-4,5-dihydro-1H-pyrazole-3-carboxylic acid piperidin-1-ylamide
[52] 5-(4-Bromo-phenyl)-1-(2,4-dichloro-phenyl)-4,4-dimethyl-4,5-dihydro-1H-pyrazole-3-carboxylic acid piperidin-1-ylamide
[53] 5-(4-Bromo-phenyl)-1-(2,4-dichloro-phenyl)-4-trifluoromethyl-4,5-dihydro-1H-pyrazole-3-carboxylic acid piperidin-1-ylamide
[54] 5-(4-Bromo-phenyl)-1-(2,4-dichloro-phenyl)-4-hydroxy-4,5-dihydro-1H-pyrazole-3-carboxylic acid piperidin-1-ylamide
[55] 5-(4-Bromo-phenyl)-1-(2,4-dichloro-phenyl)-4-(2-hydroxy-ethyl)-4,5-dihydro-1 H-pyrazole-3-carboxylic acid piperidin-1-ylamide
[56] 5-(4-Bromo-phenyl)-1-(2,4-dichloro-phenyl)-4-fluoromethyl-4,5-dihydro-1H-pyrazole-3-carboxylic acid piperidin-1-ylamide
[57] 5-(4-Bromo-phenyl)-1-(2,4-dichloro-phenyl)-4-formyl-4,5-dihydro-1H-pyrazole-3-carboxylic acid piperidin-1-ylamide
[58] 5-(4-Bromo-phenyl)-4-cyano-1-(2,4-dichloro-phenyl)-4,5-dihydro-1H-pyrazole-3-carboxylic acid piperidin-1-ylamide
[59] *cis*-(5-Chloro-thiophen-2-yl)-1-(2,4-dichloro-phenyl)-4-methyl-4,5-dihydro-1H-pyrazole-3-carboxylic acid piperidin-1-ylamide
[60] *trans-5*-(5-Chloro-thiophen-2-yl)-1-(2,4-dichloro-phenyl)-4-methyl-4,5-dihydro-1 H-pyrazole-3-carboxylic acid piperidin-1-ylamide
[61] *cis*-5-(5-Chloro-thiophen-2-yl)-1-(2,4-dichloro-phenyl)-4-ethyl-4,5-dihydro-1H-pyrazole-3-carboxylic acid piperidin-1-ylamide
[62] *trans*-5-(5-Chloro-thiophen-2-yl)-1-(2,4-dichloro-phenyl)-4-ethyl-4,5-dihydro-1H-pyrazole-3-carboxylic acid piperidin-1-ylamide
[63] 5-(5-Chloro-thiophen-2-yl)-1-(2,4-dichloro-phenyl)-4,4-dimethyl-4,5-dihydro-1 H-pyrazole-3-carboxylic acid piperidin-1-ylamide
[64] 5-(5-Chloro-thiophen-2-yl)-1-(2,4-dichloro-phenyl)-4-trifluoromethyl-4,5-dihydro-1H-pyrazole-3-carboxylic acid piperidin-1-ylamide
[65] 5-(5-Chloro-thiophen-2-yl)-1-(2,4-dichloro-phenyl)-4-hydroxy-4,5-dihydro-1H-pyrazole-3-carboxylic acid piperidin-1-ylamide
[66] 5-(5-Chloro-thiophen-2-yl)-1-(2,4-dichloro-phenyl)-4-(2-hydroxy-ethyl)-4,5-dihydro-1H-pyrazole-3-carboxylic acid piperidin-1-ylamide
[67] 5-(5-Chloro-thiophen-2-yl)-1-(2,4-dichloro-phenyl)-4-fluoromethyl-4.5-dihydro-1 H-pyrazole-3-carboxylic acid

piperidin-1-ylamide
[68] 5-(5-Chloro-thiophen-2-yl)-1-(2,4-dichloro-phenyl)-4-formyl-4,5-dihydro-1H-pyrazole-3-carboxylic acid piperidin-1-ylamide
[69] 5-(5-Chloro-thiophen-2-yl)-4-cyano-1-(2,4-dichloro-phenyl)-4,5-dihydro-1H-pyrazole-3-carboxylic acid piperidin-1-ylamide
[70] *cis*-5-(5-Bromo-thiophen-2-yl)-1-(2,4-dichloro-phenyl)-4-methyl-4,5-dihydro-1 H-pyrazole-3-carboxylic acid piperidin-1-ylamide
[71] *trans*-5-(5-Bromo-thiophen-2-yl)-1-(2,4-dichloro-phenyl)-4-methyl-4,5-dihydro-1 H-pyrazole-3-carboxylic acid piperidin-1-ylamide
[72] *cis*-5-(5-Bromo-thiophen-2-yl)-1-(2,4-dichloro-phenyl)-4-ethyl-4,5-dihydro-1H-pyrazole-3-carboxylic acid piperidin-1-ylamide
[73] *trans*-5-(5-Bromo-thiophen-2-yl)-1-(2,4-dichloro-phenyl)-4-ethyl-4,5-dihydro-1 H-pyrazole-3-carboxylic acid piperidin-1-ylamide
[74] 5-(5-Bromo-thiophen-2-yl)-1-(2,4-di-chloro-phenyl)-4,4-dimethyl-4,5-dihydro-1 H-pyrazole-3-carboxylic acid piperidin-1-ylamide
[75] *cis*-5-(4-Bromo-thiophen-2-yl)-1-(2,4-dichloro-phenyl)-4-methyl-4,5-dihydro-1 H-pyrazole-3-carboxylic acid piperidin-1-ylamide
[76] *trans*-5-(4-Bromo-thiophen-2-yl)-1-(2,4-dichloro-phenyl)-4-methyl-4,5-dihydro-1 H-pyrazole-3-carboxylic acid piperidin-1-ylamide
[77] *cis*-5-(4-Bromo-thiophen-2-yl)-1-(2,4-dichloro-phenyl)-4-ethyl-4,5-dihydro-1H-pyrazole-3-carboxylic acid piperidin-1-ylamide
[78] *trans*-5-(4-Bromo-thiophen-2-yl)-1-(2,4-dichloro-phenyl)-4-ethyl-4,5-dihydro-1 H-pyrazole-3-carboxylic acid piperidin-1-ylamide
[79] 5-(4-Bromo-thiophen-2-yl)-1-(2,4-dichloro-phenyl)-4,4-dimethyl-4,5-dihydro-1 H-pyrazole-3-carboxylic acid piperidin-1-ylamide
[80] 5-(5-Bromo-thiophen-2-yl)-1-(2,4-dichloro-phenyl)-4-trifluoromethyl-4,5-dihydro-1 H-pyrazole-3-carboxylic acid piperidin-1-ylamide
[81] 5-(5-Bromo-thiophen-2-yl)-1-(2,4-di-chloro-phenyl)-4-hydroxy-4,5-dihydro-1H-pyrazole-3-carboxylic acid piperidin-1-ylamide
[82] 5-(5-Bromo-thiophen-2-yl)-1-(2,4-di-chloro-phenyl)-4-(2-hydroxy-ethyl)-4,5-dihydro-1H-pyrazole-3-carboxylic acid piperidin-1-ylamide
[83] 5-(5-Bromo-thiophen-2-yl)-1-(2,4-di-chloro-phenyl)-4-fluoromethyl-4,5-dihydro-1 H-pyrazole-3-carboxylic acid piperidin-1-ylamide
[84] 5-(5-Bromo-thiophen-2-yl)-1-(2,4-di-chloro-phenyl)-4-formyl-4,5-dihydro-1H-pyrazole-3-carboxylic acid piperidin-1-ylamide
[85] 5-(5-Bromo-thiophen-2-yl)-4-cyano-1-(2,4-dichloro-phenyl)-4,5-dihydro-1H-pyrazole-3-carboxylic acid piperidin-1-ylamide
[86] *cis*-5-(5-Chloro-thiophen-2-yl)-1-(2,4-dichloro-phenyl)-4-methyl-4,5-dihydro-1 H-pyrazole-3-carboxylic acid
[87] *trans*-[5-(5-Chloro-thiophen-2-yl)-1-(2,4-dichloro-phenyl)-4-methyl-4,5-dihydro-1H-pyrazol-3-yl]-piperidin-1-yl-methanone
[88] *cis*-5-(5-Bromo-thiophen-2-yl)-1-(2,4-dichloro-phenyl)-4-methyl-4,5-dihydro-1 H-pyrazole-3-carboxylic acid
[89] *trans*-5-(5-Bromo-thiophen-2-yl)-1-(2,4-dichloro-phenyl)-4-methyl-4,5-dihydro-1 H-pyrazole-3-carboxylic acid
[90] *cis*-5-(4-Bromo-thiophen-2-yl)-1-(2,4-dichloro-phenyl)-4-methyl-4,5-dihydro-1 H-pyrazole-3-carboxylic acid
[91] *trans*-5-(4-Bromo-thiophen-2-yl)-1-(2,4-dichloro-phenyl)-4-methyl-4,5-dihydro-1 H-pyrazole-3-carboxylic acid,
92 (+)-cis-5-(4-chlorophenyl)-1-(2,4-dichlorophenyl)-4-methyl-N-(piperidin-1-yl)-4,5-dihydro-1H-pyrazole-3-carboxamide hydrochloride
93 (-)-cis-5-(4-chlorophenyl)-1-(2,4-dichlorophenyl)-4-methyl-N-(piperidin-1-yl)-4,5-dihydro-1H-pyrazole-3-carboxamide hydrochloride
94 *cis*-1-(2-chlorophenyl)-5-(4-chlorophenyl)-4-methyl-N-(piperidin-1-yl)-4,5-dihydro-1H-pyrazole-3-carboxamide
95 *trans*-1-(2-chlorophenyl)-5-(4-chlorophenyl)-4-methyl-N-(piperidin-1-yl)-4,5-dihydro-1H-pyrazole-3-carboxamide
96 *cis*-5-(4-Chlorophenyl)-1-(2,4-dichlorophenyl)-4-methyl-4,5-dihydro-1H-pyrazole-3-carboxylic acid azepan-1-ylamide hydrochloride
97 *trans*-5-(4-Chlorophenyl)-1-(2,4-dichloro-phenyl)-4-methyl-4,5-dihydro-1H-pyrazole-3-carboxylic acid azepan-1-ylamide hydrochloride
98 *cis*-N-(azepan-1-yl)-1-(2-chlorophenyl)-5-(4-chlorophenyl)-4-methyl-4,5-dihydro-1H-pyrazole-3-carboxamide
99 *trans*-N-(azepan-1-yl)-1-(2-chlorophenyl)-5-(4-chlorophenyl)-4-methyl-4,5-dihydro-1H-pyrazole-3-carboxamide
100 *cis*-5-(4-chlorophenyl)-N-(4-cyclopentylpiperazin-1-yl)-1-(2,4-dichlorophenyl)-4-methyl-4,5-dihydro-1H-pyrazole-3-carboxamide hydrochloride

101 *cis-5*-(4-chlorophenyl)-1-(2,4-dichlorophenyl)-4-methyl-N-morpholino-4,5-dihydro-1H-pyrazole-3-carboxamide

102 *cis*-1-(2-chlorophenyl)-5-(4-chlorophenyl)-4-methyl-N-(4-methylpiperidin-1-yl)-4,5-dihydro-1H-pyrazole-3-carboxamide

103 *cis*-5-(4-chlorophenyl)-1-(2,4-dichlorophenyl)-N-(2,6-dimethylpiperidin-1-yl)-4-methyl-4,5-dihydro-1H-pyrazole-3-carboxamide

*104 cis*-5-(4-Chlorophenyl)-1-(2,4-dichlorophenyl)-4-methyl-4,5-dihydro-1H-pyrazole-3-carboxylic acid (1,3-dioxo-1 H,3H-benzo[de]isoquinolin-2-yl)-amide

105 *cis*-5-(4-Chlorophenyl)-1-(2,4-dichlorophenyl)-4-methyl-4,5-dihydro-1H-pyrazole-3-carboxylic acid (1 H,3H-benzo[de]isoquinolin-2-yl)-amide

106 *cis*-5-(4-chlorophenyl)-1-(2,4-dichlorophenyl)-N-((R)-2-(methoxymethyl)pyrrolidin-1-yl)-4-methyl-4,5-dihydro-1 H-pyrazole-3-carboxamide hydrochloride

107 *cis*-5-(4-chlorophenyl)-1-(2,4-dichlorophenyl)-N-((S)-2-(methoxymethyl)pyrrolidin-1-yl)-4-methyl-4,5-dihydro-1 H-pyrazole-3-carboxamide hydrochloride

108 *cis*-5-(4-Chlorophenyl)-1-(2,4-dichlorophenyl)-4-methyl-4,5-dihydro-1H-pyrazole-3-carboxylic acid (hexahydro-cyclopenta[c]pyrrol-2-yl)-amide hydrochloride

109 *cis*-1-(2-chlorophenyl)-5-(4-chlorophenyl)-N-(hexahydrocyclopenta[c]pyrrol-2(1 H)-yl)-4-methyl-4,5-dihydro-1 H-pyrazole-3-carboxamide

110 *trans*-1-(2-chlorophenyl)-5-(4-chlorophenyl)-N-(hexahydrocyclopenta[c]pyrrol-2(1H)-yl)-4-methyl-4,5-dihydro-1H-pyrazole-3-carboxamide

111 cis-1-(2-chlorophenyl)-5-(4-chlorophenyl)-N-(indolin-1-yl)-4-methyl-4,5-dihydro-1 H-pyrazole-3-carboxamide

112 trans-1-(2-chlorophenyl)-5-(4-chlorophenyl)-N-(indolin-1-yl)-4-methyl-4,5-dihydro-1 H-pyrazole-3-carboxamide

113 cis-5-(4-chlorophenyl)-1-(2,4-dichlorophenyl)-4-methyl-N-(2-methylindolin-1-yl)-4,5-dihydro-1 H-pyrazole-3-carboxamide

114 *cis*-5-(4-chlorophenyl)-N-(cyclohexylmethyl)-1-(2,4-dichlorophenyl)-4-methyl-4,5-dihydro-1H-pyrazole-3-carboxa mide

115 *cis*-5-(4-chlorophenyl)-N-(cycloheptylmethyl)-1-(2,4-dichlorophenyl)-4-methyl-4,5-dihydro-1H-pyrazole-3-carboxamide

116 *cis*-5-(4-chlorophenyl)-N-cyclododecyl-1-(2,4-dichlorophenyl)-4-methyl-4,5-dihydro-1H-pyrazole-3-carboxamide

117 *cis*-5-(4-chlorophenyl)-1-(2,4-dichlorophenyl)-4-methyl-N-(1,2,3,4-tetrahydronaphthalen-1-yl)-4,5-dihydro-1H-pyrazole-3-carboxamide

118 *cis*-N-(4-tert-butylcyclohexyl)-5-(4-chlorophenyl)-1-(2,4-dichlorophenyl)-4-methyl-4,5-dihydro-1H-pyrazole-3-carboxamide

119 *cis*-5-(4-chlorophenyl)-N-cyclooctyl-1-(2,4-dichlorophenyl)-4-methyl-4,5-dihydro-1H-pyrazole-3-carboxamide

120 cis-N-(azocan-1-yl)-5-(4-chlorophenyl)-1-(2,4-dichlorophenyl)-4-methyl-4,5-dihydro-1H-pyrazole-3-carboxamide

121 *cis*-N-(azocan-1-yl)-1-(2-chlorophenyl)-5-(4-chlorophenyl)-4-methyl-4,5-dihydro-1H-pyrazole-3-carboxamide

122 *cis*-azocan-1-yl(cis-5-(4-chlorophenyl)-1-(2,4-dichlorophenyl)-4-methyl-4,5-dihydro-1H-pyrazol-3-yl)methanone

123 *cis*-5-(4-chlorophenyl)-N-cycloheptyl-1-(2,4-dichlorophenyl)-4-methyl-4,5-dihydro-1H-pyrazole-3-carboxamide

124 *cis*-1-(2-chlorophenyl)-5-(4-chlorophenyl)-N-cycloheptyl-4-methyl-4,5-dihydro-1 H-pyrazole-3-carboxamide

125 *trans*-5-(4-chlorophenyl)-N-cycloheptyl-1-(2,4-dichlorophenyl)-4-methyl-4,5-dihydro-1H-pyrazole-3-carboxamide

126 *trans*-1-(2-chlorophenyl)-5-(4-chlorophenyl)-N-cycloheptyl-4-methyl-4,5-dihydro-1H-pyrazole-3-carboxamide

127 *cis*-5-(4-chlorophenyl)-N-(cyclohexylmethyl)-1-(2,4-dichlorophenyl)-4-methyl-4,5-dihydro-1H-pyrazole-3-carboxamide

128 (+)-cis-5-(4-chlorophenyl)-1-(2,4-dichlorophenyl)-N-((1S,2S)-2-hydroxycyclohexyl)-4-methyl-4,5-dihydro-1H-pyrazole-3-carboxamide

129 (-)-cis-5-(4-chlorophenyl)-1-(2,4-dichlorophenyl)-N-((1S,2S)-2-hydroxycyclohexyl)-4-methyl-4,5-dihydro-1H-pyrazole-3-carboxamide

130 (-)-cis-5-(4-chlorophenyl)-1-(2,4-dichlorophenyl)-N-((1R,2R)-2-hydroxycyclohexyl)-4-methyl-4,5-dihydro-1H-pyrazole-3-carboxamide

131 (+)-cis-5-(4-chlorophenyl)-1-(2,4-dichlorophenyl)-N-((1R,2R)-2-hydroxycyclohexyl)-4-methyl-4,5-dihydro-1H-pyrazole-3-carboxamide

132 (+)-cis-1-(2-chlorophenyl)-5-(4-chlorophenyl)-N-((1S,2S)-2-hydroxycyclohexyl)-4-methyl-4,5-dihydro-1H-pyrazole-3-carboxamide

133 (-)-cis-1-(2-chlorophenyl)-5-(4-chlorophenyl)-N-((1S,2S)-2-hydroxycyclohexyl)-4-methyl-4,5-dihydro-1H-pyrazole-3-carboxamide
134 *cis*-5-(4-chlorophenyl)-1-(2,4-dichlorophenyl)-N-(4-hydroxypiperidin-1-yl)-4-methyl-4,5-dihydro-1H-pyrazole-3-carboxamide
135 *cis*-1-(2-chlorophenyl)-5-(4-chlorophenyl)-N-(4-hydroxypiperidin-1-yl)-4-methyl-4,5-dihydro-1H-pyrazole-3-carboxamide
136 *cis*-5-(4-chlorophenyl)-1-(2,4-dichlorophenyl)-N-(3-hydroxypiperidin-1-yl)-4-methyl-4,5-dihydro-1H-pyrazole-3-carboxamide
137 *cis*-1-(2-chlorophenyl)-5-(4-chlorophenyl)-N-(3-hydroxypiperidin-1-yl)-4-methyl-4,5-dihydro-1H-pyrazole-3-carboxamide
138 *cis*-5-(4-chlorophenyl)-1-(2,4-dichlorophenyl)-N-(2-hydroxypiperidin-1-yl)-4-methyl-4,5-dihydro-1H-pyrazole-3-carboxamide
139 *cis*-1-(2-chlorophenyl)-5-(4-chlorophenyl)-N-(2-hydroxypiperidin-1-yl)-4-methyl-4,5-dihydro-1H-pyrazole-3-carboxamide
140 *cis*-5-(4-chlorophenyl)-1-(2,4-dichlorophenyl)-4-methyl-N-(4-oxopiperidin-1-yl)-4,5-dihydro-1 H-pyrazole-3-carboxamide
141 *cis*-1-(2-chlorophenyl)-5-(4-chlorophenyl)-4-methyl-N-(4-oxopiperidin-1-yl)-4,5-dihydro-1 H-pyrazole-3-carboxamide
142 *cis*-S-(4-chlorophenyl)-1-(2,4-dichlorophenyl)-4-methyl-N-(3-oxopiperidin-1-yl)-4,5-dihydro-1 H-pyrazole-3-carboxamide
143 *cis*-1-(2-chlorophenyl)-5-(4-chlorophenyl)-4-methyl-N-(3-oxopiperidin-1-yl)-4,5-dihydro-1H-pyrazole-3-carboxamide
144 *cis*-5-(4-chlorophenyl)-1-(2,4-dichlorophenyl)-N-(3,4-dihydropyridin-1(2H)-yl)-4-methyl-4,5-dihydro-1H-pyrazole-3-carboxamide
145 *cis*-1-(2-chlorophenyl)-5-(4-chlorophenyl)-N-(3,4-dihydropyridin-1(2H)-yl)-4-methyl-4,5-dihydro-1H-pyrazole-3-carboxamide
146 *cis*-5-(4-chlorophenyl)-1-(2,4-dichlorophenyl)-N-(5,6-dihydropyridin-1(2H)-yl)-4-methyl-4,5-dihydro-1H-pyrazole-3-carboxamide
147 *cis*-1-(2-chlorophenyl)-5-(4-chlorophenyl)-N-(5,6-dihydropyridin-1(2H)-yl)-4-methyl-4,5-dihydro-1H-pyrazole-3-carboxamide
148 *cis*-1-(2-chlorophenyl)-5-(4-chlorophenyl)-4-methyl-4,5-dihydro-1H-pyrazole-3-carboxylic acid
149 trans-1-(2-chlorophenyl)-5-(4-chlorophenyl)-4-methyl-4,5-dihydro-1H-pyrazole-3-carboxylic acid
150 *cis*-5-(4-Chlorophenyl)-1-(2,4-dichlorophenyl)-4-methyl-4,5-dihydro-1H-pyrazole-3-carboxylic acid cyclohexyl ester
151 N-(azepan-1-yl)-5-(4-chlorophenyl)-1-(2,4-dichlorophenyl)-4,4-dimethyl-4,5-dihydro-1 H-pyrazole-3-carboxamide
152 1-(2-chlorophenyl)-5-(4-chlorophenyl)-4,4-dimethyl-N-(piperidin-1-yl)-4,5-dihydro-1H-pyrazole-3-carboxamide
153 N-(azepan-1-yl)-1-(2-chlorophenyl)-5-(4-chlorophenyl)-4,4-dimethyl-4,5-dihydro-1H-pyrazole-3-carboxamide
154 *cis*-5-(4-Chlorophenyl)-1-(2,4-dichlorophenyl)-4-trifluoromethyl-4,5-dihydro-1H-pyrazole-3-carboxylic acid piperidin-1-ylamide
155 *trans*-5-(4-Chloro-phenyl)-1-(2,4-dichlorophenyl)-4-trifluoromethyl-4,5-dihydro-1H-pyrazole-3-carboxylic acid piperidin-1-ylamide
156 *cis*-N-(azepan-1-yl)-5-(4-chlorophenyl)-1-(2,4-dichlorophenyl)-4-(trifluoromethyl)-4,5-dihydro-1H-pyrazole-3-carboxamide
157 *trans*-N-(azepan-1-yl)-5-(4-chlorophenyl)-1-(2,4-dichlorophenyl)-4-(trifluoromethyl)-4,5-dihydro-1H-pyrazole-3-carboxamide
158 *cis*-1-(2-chlorophenyl)-5-(4-chlorophenyl)-N-(piperidin-1-yl)-4-(trifluoromethyl)-4,5-dihydro-1H-pyrazole-3-carboxamide
159 *trans* 1-(2-chlorophenyl)-5-(4-chlorophenyl)-N-(piperidin-1-yl)-4-(trifluoromethyl)-4,5-dihydro-1H-pyrazole-3-carboxamide
160 5-(4-chlorophenyl)-1-(2,4-dichlorophenyl)-3-(piperidin-1-ylcarbamoyl)-4,5-dihydro-1H-pyrazole-4-carboxylic acid
161 5-(4-chlorophenyl)-1-(2,4-dichlorophenyl)-4,4-difluoro-N-(piperidin-1-yl)-4,5-dihydro-1H-pyrazole-3-carboxamide
162 *cis*-5-(4-chlorophenyl)-4-cyano-1-(2,4-dichlorophenyl)-N-(piperidin-1-yl)-4,5-dihydro-1H-pyrazole-3-carboxamide
163 *cis*-N-(azepan-1-yl)-5-(4-chlorophenyl)-4-cyano-1-(2,4-dichlorophenyl)-4,5-dihydro-1H-pyrazole-3-carboxamide

164 *cis*-1-(2-chlorophenyl)-5-(4-chlorophenyl)-4-cyano-N-(piperidin-1-yl)-4,5-dihydro-1H-pyrazole-3-carboxamide
165 *cis*-N-(azepan-1-yl)-1-(2-chlorophenyl)-5-(4-chlorophenyl)-4-cyano-4,5-dihydro-1H-pyrazole-3-carboxamide
166 *trans*-5-(4-chlorophenyl)-4-cyano-1-(2,4-dichlorophenyl)-N-(piperidin-1-yl)-4,5-dihydro-1H-pyrazole-3-carboxamide
167 *trans*-N-(azepan-1-yl)-5-(4-chlorophenyl)-4-cyano-1-(2,4-dichlorophenyl)-4,5-dihydro-1H-pyrazole-3-carboxamide
168 *trans*-1-(2-chlorophenyl)-5-(4-chlorophenyl)-4-cyano-N-(piperidin-1-yl)-4,5-dihydro-1H-pyrazole-3-carboxamide
169 *cis*-1-(2-chlorophenyl)-5-(4-chlorophenyl)-4-ethyl-4,5-dihydro-1H-pyrazole-3-carboxylic acid
170 *trans*-1-(2-chlorophenyl)-5-(4-chlorophenyl)-4-ethyl-4,5-dihydro-1H-pyrazole-3-carboxylic acid
171 *cis*-5-(4-chlorophenyl)-1-(2,4-dichlorophenyl)-4-ethyl-N-(piperidin-1-yl)-4,5-dihydro-1H-pyrazole-3-carboxamide
172 *trans*-5-(4-chlorophenyl)-1-(2,4-dichlorophenyl)-4-ethyl-N-(piperidin-1-yl)-4,5-dihydro-1H-pyrazole-3-carboxamide
173 *cis*-1-(2-chlorophenyl)-5-(4-chlorophenyl)-4-ethyl-N-(piperidin-1-yl)-4,5-dihydro-1H-pyrazole-3-carboxamide
174 *trans*-1-(2-chlorophenyl)-5-(4-chlorophenyl)-4-ethyl-N-(piperidin-1-yl)-4,5-dihydro-1H-pyrazole-3-carboxamide
175 cis -N-(azepan-1-yl)-5-(4-chlorophenyl)-1-(2,4-dichlorophenyl)-4-ethyl-4,5-dihydro-1 H-pyrazole-3-carboxamide
176 *trans*-N-(azepan-1-yl)-5-(4-ch)orophenyl)-1-(2.4-dich)orophenyl)-4-ethy)-4,5-dihydro-1 H-pyrazole-3-carboxamide
177 *cis*-N-(azepan-1-yl)-1-(2-chlorophenyl)-5-(4-chlorophenyl)-4-ethyl-4,5-dihydro-1 H-pyrazole-3-carboxamide
178 *trans*-N-(azepan-1-yl)-1-(2-chlorophenyl)-5-(4-chlorophenyl)-4-ethyl-4,5-dihydro-1 H-pyrazole-3-carboxamide
179 *cis*-5-(4-chlorophenyl)-N-cycloheptyl-1-(2,4-dichlorophenyl)-4-ethyl-4,5-dihydro-1H-pyrazole-3-carboxamide
180 *cis*-1-(2-chlorophenyl)-5-(4-chlorophenyl)-N-cycloheptyl-4-ethyl-4,5-dihydro-1 H-pyrazole-3-carboxamide
181 *cis*-5-(4-chlorophenyl)-1-(2,4-dichlorophenyl)-4-ethyl-N-(hexahydrocyclopenta[c]pyrrol-2(1H)-yl)-4,5-dihydro-1H-pyrazole-3-carboxamide
182 *cis*-1-(2-chlorophenyl)-5-(4-chlorophenyl)-4-ethyl-N-(hexahydrocyclopenta[c]pyrrol-2(1H)-yl)-4,5-dihydro-1H-pyrazole-3-carboxamide
183 *cis*-5-(4-chlorophenyl)-1-(2,4-dichlorophenyl)-4-ethyl-N-(indolin-1-yl)-4,5-dihydro-1H-pyrazole-3-carboxamide
184 *cis*-1-(2-chlorophenyl)-5-(4-chlorophenyl)-4-ethyl-N-(indolin-1-yl)-4,5-dihydro-1 H-pyrazole-3-carboxamide
185 *cis*-5-(4-bromophenyl)-1-(2,4-dichlorophenyl)-4-ethyl-4,5-dihydro-1H-pyrazole-3-carboxylic acid
186 *trans*-5-(4-bromophenyl)-1-(2,4-dichlorophenyl)-4-ethyl-4,5-dihydro-1H-pyrazole-3-carboxylic acid
187 *cis*-5-(4-bromophenyl)-1-(2,4-dichlorophenyl)-4-ethyl-N-(piperidin-1-yl)-4,5-dihydro-1 H-pyrazole-3-carboxamide
188 *trans*-5-(4-bromophenyl)-1-(2,4-dichlorophenyl)-4-ethyl-N-(piperidin-1-yl)-4,5-dihydro-1 H-pyrazole-3-carboxa mide
189 *cis*-5-(4-bromophenyl)-1-(2-chlorophenyl)-4-ethyl-N-(piperidin-1-yl)-4,5-dihydro-1H-pyrazole-3-carboxamide
190 *trans*-5-(4-bromophenyl)-1-(2-chlorophenyl)-4-ethyl-N-(piperidin-1-yl)-4,5-dihydro-1 H-pyrazole-3-carboxamide
191 *cis*-N-(azepan-1-yl)-5-(4-bromophenyl)-1-(2,4-dichlorophenyl)-4-ethyl-4,5-dihydro-1 H-pyrazole-3-carboxamide
192 *cis*-N-(azepan-1-yl)-5-(4-bromophenyl)-1-(2-chlorophenyl)-4-ethyl-4,5-dihydro-1H-pyrazole-3-carboxamide
193 *cis*-5-(4-bromophenyl)-N-cycloheptyl-1-(2,4-dichlorophenyl)-4-ethyl-4,5-dihydro-1H-pyrazole-3-carboxamide
194 *cis*-5-(4-bromophenyl)-1-(2-chlorophenyl)-N-cycloheptyl-4-ethyl-4,5-dihydro-1H-pyrazole-3-carboxamide
195 *cis*-5-(4-bromophenyl)-1-(2,4-dichlorophenyl)-4-ethyl-N-(hexahydrocyclopenta[c]pyrrol-2(1H)-yl)-4,5-dihydro-1H-pyrazole-3-carboxamide
196 *cis*-5-(4-bromophenyl)-1-(2-chlorophenyl)-4-ethyl-N-(hexahydrocyclopenta[c]pyrrol-2(1H)-yl)-4,5-dihydro-1H-pyrazole-3-carboxamide
197 *cis*-5-(4-bromophenyl)-1-(2,4-dichlorophenyl)-4-ethyl-N-(indolin-1-yl)-4,5-dihydro-1H-pyrazole-3-carboxamide
198 *cis*-5-(4-bromophenyl)-1-(2-chlorophenyl)-4-ethyl-N-(indolin-1-yl)-4,5-dihydro-1H-pyrazole-3-carboxamide
199 *cis*-5-(4-bromophenyl)-1-(2,4-dichlorophenyl)-4-methyl-4,5-dihydro-1H-pyrazole-3-carboxylic acid
200 *trans*-5-(4-bromophenyl)-1-(2,4-dichlorophenyl)-4-methyl-4,5-dihydro-1H-pyrazole-3-carboxylic acid
201 *cis*-5-(4-bromophenyl)-1-(2-chlorophenyl)-4-methyl-4,5-dihydro-1H-pyrazole-3-carboxylic acid
202 *trans*-5-(4-bromophenyl)-(2-chlorophenyl)-4-methyl-4,5-dihydro-1H-pyrazole-3-carboxylic acid
203 *cis*-5-(4-bromophenyl)-1-(2,4-dichlorophenyl)-4-methyl-N-(piperidin-1-yl)-4,5-dihydro-1 H-pyrazole-3-carboxamide
204 *trans*-5-(4-bromophenyl)-1-(2,4-dichlorophenyl)-4-methyl-N-(piperidin-1-yl)-4,5-dihydro-1H-pyrazole-3-carboxamide

205 *cis*-5-(4-bromophenyl)-1-(2-chlorophenyl)-4-methyl-N-(piperidin-1-yl)-4,5-dihydro-1H-pyrazole-3-carboxamide
206 *trans*-5-(4-bromophenyl)-1-(2-chlorophenyl)-4-methyl-N-(piperidin-1-yl)-4,5-dihydro-1H-pyrazole-3-carboxamide
207 *cis*-N-(azepan-1-yl)-5-(4-bromophenyl)-1-(2,4-dichlorophenyl)-4-methyl-4,5-dihydro-1H-pyrazole-3-carboxamide hydrochloride
208 *trans*-N-(azepan-1-yl)-5-(4-bromophenyl)-1-(2,4-dichlorophenyl)-4-methyl-4,5-dihydro-1H-pyrazole-3-carboxamide hydrochloride
209 *cis*-N-(azepan-1-yl)-5-(4-bromophenyl)-1-(2-chlorophenyl)-4-methyl-4,5-dihydro-1 H-pyrazole-3-carboxamide
210 *cis*-N-(azepan-1-yl)-S-(4-bromophenyl)-1-(2-chlorophenyl)-4-methyl-4,5-dihydro-1 H-pyrazole-3-carboxamide
211 *cis*-5-(4-bromophenyl)-N-cyclophetyl-1-(2,4-dichlorophenyl)-4-methyl-4,5-dihydro-1H-pyrazole-3-carboxamide
212 *cis*-5-(4-bromophenyl)-1-(2-chlorophenyl)-N-cycloheptyl-4-methyl-4,5-dihydro-1 H-pyrazole-3-carboxamide
213 *cis*-5-(4-bromophenyl)-1-(2,4-dichlorophenyl)-N-(hexahydrocyclopenta[c]pyrrol-2(1H)-yl)-4-methyl-4,5-dihydro-1H-pyrazole-3-carboxamide hydrochloride
214 *cis*-5-(4-bromophenyl)-1-(2-chlorophenyl)-N-(hexahydrocyclopenta[c]pyrrol-2(1H)-yl)-4-methyl-4,5-dihydro-1H-pyrazole-3-carboxamide
215 *cis*-5-(4-bromophenyl)-1-(2,4-dichlorophenyl)-N-(indolin-1-yl)-4-methyl-4,5-dihydro-1H-pyrazole-3-carboxamide
216 *cis*-5-(4-bromophenyl)-1-(2-chlorophenyl)-N-(indolin-1-yl)-4-methyl-4,5-dihydro-1H-pyrazole-3-carboxamide
217 (+)-cis-5-(4-bromophenyl)-1-(2,4-dichlorophenyl)-N-((1S,2S)-2-hydroxycyclohexyl)-4-methyl-4,5-dihydro-1H-pyrazole-3-carboxamide
218 (-)-cis-5-(4-bromophenyl)-1-(2,4-dichlorophenyl)-N-((1S,2S)-2-hydroxycyclohexyl)-4-methyl-4,5-dihydro-1H-pyrazole-3-carboxamide
219 (+)-cis-5-(4-bromophenyl)-1-(2-chlorophenyl)-N-((1S,2S)-2-hydroxycyclohexyl)-4-methyl-4,5-dihydro-1H-pyrazole-3-carboxamide
220 (-)-cis-5-(4-bromophenyl)-1-(2-chlorophenyl)-N-((1S,2S)-2-hydroxycyclohexyl)-4-methyl-4,5-dihydro-1H-pyrazole-3-carboxamide
221 *cis*-1-(2,4-dichlorophenyl)-5-(4-fluorophenyl)-4-methyl-4,5-dihydro-1H-pyrazole-3-carboxylic acid
222 *trans*-1-(2,4-dichlorophenyl)-5-(4-fluorophenyl)-4-methyl-4,5-dihydro-1H-pyrazole-3-carboxylic acid
223 *cis*-1-(2,4-dichlorophenyl)-5-(4-fluorophenyl)-4-methyl-N-(piperidin-1-yl)-4,5-dihydro-1H-pyrazole-3-carboxamide
224 *trans*-1-(2,4-dichlorophenyl)-5-(4-fluorophenyl)-4-methyl-N-(piperidin-1-yl)-4,5-dihydro-1H-pyrazole-3-carboxamide
225 *cis*-1-(2-chlorophenyl)-5-(4-fluorophenyl)-4-methyl-N-(piperidin-1-yl)-4,5-dihydro-1H-pyrazole-3-carboxamide
226 *trans*-1-(2-chlorophenyl)-5-(4-fluorophenyl)-4-methyl-N-(piperidin-1-yl)-4,5-dihydro-1H-pyrazole-3-carboxamide
227 *cis*-N-(azepan-1-yl)-1-(2,4-dichlorophenyl)-5-(4-fluorophenyl)-4-methyl-4,5-dihydro-1H-pyrazole-3-carboxamide hydrochloride
228 *trans*-N-(azepan-1-yl)-1-(2,4-dichlorophenyl)-5-(4-fluorophenyl)-4-methyl-4,5-dihydro-1H-pyrazole-3-carboxamide hydrochloride
229 *cis*-N-(azepan-1-yl)-1-(2-chlorophenyl)-5-(4-fluorophenyl)-4-methyl-4,5-dihydro-1H-pyrazole-3-carboxamide
230 *trans*-N-(azepan-1-yl)-1-(2-chlorophenyl)-5-(4-fluorophenyl)-4-methyl-4,5-dihydro-1H-pyrazole-3-carboxamide
231 *cis*-N-cycloheptyl-1-(2,4-dichlorophenyl)-5-(4-fluorophenyl)-4-methyl-4,5-dihydro-1H-pyrazole-3-carboxamide
232 *cis*-1-(2-chlorophenyl)-N-cycloheptyl-5-(4-fluorophenyl)-4-methyl-4,5-dihydro-1H-pyrazole-3-carboxamide
233 *cis*-1-(2,4-dichlorophenyl)-5-(4-fluorophenyl)-N-(hexahydrocyclopenta[c]pyrrol-2(1H)-yl)-4-methyl-4,5-dihydro-1H-pyrazole-3-carboxamide hydrochloride
234 *cis*-1-(2-chlorophenyl)-5-(4-fluorophenyl)-N-(hexahydrocyclopenta[c]pyrrol-2(1H)-yl)-4-methyl-4,5-dihydro-1H-pyrazole-3-carboxamide
235 *cis*-1-(2,4-dichlorophenyl)-5-(4-fluorophenyl)-N-(indolin-1-yl)-4-methyl-4,5-dihydro-1H-pyrazole-3-carboxamide
236 *cis*-1-(2-chlorophenyl)-5-(4-fluorophenyl)-N-(indolin-1-yl)-4-methyl-4,5-dihydro-1 H-pyrazole-3-carboxamide
237 (+)-cis-1-(2,4-dichlorophenyl)-5-(4-fluorophenyl)-N-((1S,2S)-2-hydroxycyclohexyl)-4-methyl-4,5-dihydro-1H-pyrazole-3-carboxamide
238 (-)-cis-1-(2,4-dichlorophenyl)-5-(4-fluorophenyl)-N-((1S,2S)-2-hydroxycyclohexyl)-4-methyl-4,5-dihydro-1H-pyrazole-3-carboxamide
239 (+)-cis-1-(2-chlorophenyl)-5-(4-fluorophenyl)-N-((1S,2S)-2-hydroxycyclohexyl)-4-methyl-4,5-dihydro-1H-pyrazole-3-carboxamide

240 (-)-cis-1-(2-chlorophenyl)-5-(4-fluorophenyl)-N-((1S,2S)-2-hydroxycyclohexyl)-4-methyl-4,5-dihydro-1H-pyrazole-3-carboxamide

241 *cis*-1-(2,4-dichlorophenyl)-5-(4-iodophenyl)-4-methyl-4,5-dihydro-1H-pyrazole-3-carboxylic acid

242 *trans*-1-(2,4-dichlorophenyl)-5-(4-iodophenyl)-4-methyl-4,5-dihydro-H-pyrazole-3-carboxylic acid

243 *cis*-1-(2,4-dichlorophenyl)-5-(4-iodophenyl)-4-methyl-N-(piperidin-1-yl)-4,5-dihydro-1H-pyrazole-3-carboxamide

244 *trans*-1-(2,4-dichlorophenyl)-5-(4-iodophenyl)-4-methyl-N-(piperidin-1-yl)-4,5-dihydro-1H-pyrazole-3-carboxamide

245 *cis*-1-(2-chlorophenyl)-5-(4-iodophenyl)-4-methyl-N-(piperidin-1-yl)-4,5-dihydro-1H-pyrazole-3-carboxamide

246 *trans*-1-(2-chlorophenyl)-5-(4-iodophenyl)-4-methyl-N-(piperidin-1-yl)-4,5-dihydro-1H-pyrazole-3-carboxamide

247 *cis*-N-(azepan-1-yl)-1-(2,4-dichlorophenyl)-5-(4-iodophenyl)-4-methyl-4,5-dihydro-1H-pyrazole-3-carboxamide

248 *trans*-N-(azepan-1-yl)-1-(2,4-dichlorophenyl)-5-(4-iodophenyl)-4-methyl-4,5-dihydro-1H-pyrazole-3-carboxamide

249 *cis*-N-(azepan-1-yl)-1-(2-chlorophenyl)-5-(4-iodophenyl)-4-methyl-4,5-dihydro-1 H-pyrazole-3-carboxamide

250 *trans*-N-(azepan-1-yl)-1-(2-chlorophenyl)-5-(4-iodophenyl)-4-methyl-4,5-dihydro-1H-pyrazole-3-carboxamide

251 *cis*-N-cycloheptyl-1-(2 ,4-dichlorophenyl)-5-(4-iodophenyl)-4-methyl-4,5-dihydro-1H-pyrazole-3-carboxamide

252 *ci*s-1-(2-chlorophenyl)-N-cycloheptyl-5-(4-iodophenyl)-4-methyl-4,5-dihydro-1H-pyrazole-3-carboxamide

253 *cis*-1-(2,4-dichlorophenyl)-N-(hexahydrocyclopenta[c]pyrrol-2(1H)-yl)-5-(4-iodophenyl)-4-methyl-4,5-dihydro-1H-pyrazole-3-carboxamide

254 cis-1-(2-chlorophenyl)-N-(hexahydrocyclopenta[c]pyrrol-2(1H)-yl)-5-(4-iodophenyl)-4-methyl-4,5-dihydro-1 H-pyrazole-3-carboxamide

255 *cis*-1-(2,4-dichlorophenyl)-N-(indolin-1-yl)-5-(4-iodophenyl)-4-methyl-4,5-dihydro-1H-pyrazole-3-carboxamide

256 *cis*-1-(2-chlorophenyl)-N-(indolin-1-yl)-5-(4-iodophenyl)-4-methyl-4,5-dihydro-1 H-pyrazole-3-carboxamide

257 (+)-cis-1-(2,4-dichlorophenyl)-N-((1S,2S)-2-hydroxycyclohexyl)-5-(4-iodophenyl)-4-methyl-4,5-dihydro-1H-pyrazole-3-carboxamide

258 (-)-cis-1-(2,4-dich)orophenyl)-N-((1S.2S)-2-hydroxycyclohexyl)-5-(4-iodophenyl)-4-methyl-4,5-dihydro-1 H-pyrazole-3-carboxamide

259 (+)-cis-1-(2-chlorophenyl)-N-((1S,2S)-2-hydroxycyclohexyl)-5-(4-iodophenyl)-4-methyl-4,5-dihydro-1H-pyrazole-3-carboxamide

260 (-)-cis-1-(2-chlorophenyl)-N-((1S,2S)-2-hydroxycyclohexyl)-5-(4-iodophenyl)-4-methyl-4,5-dihydro-1H-pyrazole-3-carboxamide

261 *cis*-1-(2,4-dichlorophenyl)-5-(4-methoxyphenyl)-4-methyl-4,5-dihydro-1H-pyrazole-3-carboxylic acid

262 *trans*-1-(2,4-dichlorophenyl)-5-(4-methoxyphenyl)-4-methyl-4,5-dihydro-1H-pyrazole-3-carboxylic acid

263 *cis*-1-(2-chlorophenyl)-5-(4-methoxyphenyl)-4-methyl-4,5-dihydro-1H-pyrazole-3-carboxylic acid

264 *trans*-1-(2-chlorophenyl)-5-(4-methoxyphenyl)-4-methyl-4,5-dihydro-1H-pyrazole-3-carboxylic acid

265 *cis*-1-(2,4-dichlorophenyl)-5-(4-methoxyphenyl)-4-methyl-N-(piperidin-1-yl)-4,5-dihydro-1H-pyrazole-3-carboxamide hydrochloride

266 (+)-cis-1-(2,4-dichlorophenyl)-5-(4-methoxyphenyl)-4-methyl-N-(piperidin-1-yl)-4,5-dihydro-1H-pyrazole-3-carboxamide hydrochloride

267 (-)-cis-1-(2,4-dichlorophenyl)-5-(4-methoxyphenyl)-4-methyl-N-(piperidin-1-yl)-4,5-dihydro-1H-pyrazole-3-carboxamide hydrochloride

268 *trans*-1-(2,4-dichlorophenyl)-5-(4-methoxyphenyl)-4-methyl-N-(piperidin-1-yl)-4,5-dihydro-1H-pyrazole-3-carboxamide

269 *cis*-1-(2-chlorophenyl)-5-(4-methoxyphenyl)-4-methyl-N-(piperidin-1-yl)-4,5-dihydro-1H-pyrazole-3-carboxamide

270 *trans*-1-(2-chlorophenyl)-5-(4-methoxyphenyl)-4-methyl-N-(piperidin-1-yl)-4,5-dihydro-1H-pyrazole-3-carboxamide

271 *cis*-N-(azepan-1-yl)-1-(2,4-dichlorophenyl)-5-(4-methoxyphenyl)-4-methyl-4,5-dihydro-1H-pyrazole-3-carboxamide hydrochloride

272 *trans*-N-(azepan-1-yl)-1-(2,4-dichlorophenyl)-5-(4-methoxyphenyl)-4-methyl-4,5-dihydro-1H-pyrazole-3-carboxamide hydrochloride

273 *cis*-N-(azepan-1-yl)-1-(2-chlorophenyl)-5-(4-methoxyphenyl)-4-methyl-4,5-dihydro-1 H-pyrazole-3-carboxamide

274 *trans*-N-(azepan-1-yl)-1-(2-chlorophenyl)-5-(4-methoxyphenyl)-4-methyl-4,5-dihydro-1H-pyrazole-3-carboxamide

275 *cis*-N-cycloheptyl-1-(2,4-dichlorophenyl)-5-(4-methoxyphenyl)-4-methyl-4,5-dihydro-1H-pyrazole-3-carboxamide

276 *cis*-1-(2-chlorophenyl)-N-cycloheptyl-5-(4-methoxyphenyl)-4-methyl-4,5-dihydro-1H-pyrazole-3-carboxamide
277 *cis*-1-(2,4-dichlorophenyl)-N-(hexahydrocyclopenta[c]pyrrol-2(1H)-yl)-5-(4-methoxyphenyl)-4-methyl-4,5-dihydro-1H-pyrazole-3-carboxamide hydrochloride
278 *cis*-1-(2-chlorophenyl)-N-(hexahydrocyclopenta[c]pyrrol-2(1H)-yl)-5-(4-methoxyphenyl)-4-methyl-4,5-dihydro-1H-pyrazole-3-carboxamide
279 *cis*-1-(2,4-dichlorophenyl)-N-(indolin-1-yl)-5-(4-methoxyphenyl)-4-methyl-4,5-dihydro-1H-pyrazole-3-carboxamide
280 *cis*-1-(2-chlorophenyl)-N-(indolin-1-yl)-5-(4-methoxyphenyl)-4-methyl-4,5-dihydro-1H-pyrazole-3-carboxamide
281 (+)-cis-1-(2,4-dichlorophenyl)-N-((1S,2S)-2-hydroxycyclohexyl)-5-(4-methoxyphenyl)-4-methyl-4,5-dihydro-1H-pyrazole-3-carboxamide
282 (-)-cis-1-(2,4-dichlorophenyl)-N-((1S,2S)-2-hydroxycyclohexyl)-5-(4-methoxyphenyl)-4-methyl-4,5-dihydro-1H-pyrazole-3-carboxamide
283 (+)-cis-1-(2-chlorophenyl)-N-((1S,2S)-2-hydroxycyclohexyl)-5-(4-methoxyphenyl)-4-methyl-4,5-dihydro-1H-pyrazole-3-carboxamide
284 (-)-cis-1-(2-chlorophenyl)-N-((1S,2S)-2-hydroxycyclohexyl)-5-(4-methoxyphenyl)-4-methyl-4,5-dihydro-1H-pyrazole-3-carboxamide
285 *cis*-1-(2,4-dichlorophenyl)-N-(4-hydroxypiperidin-1-yl)-5-(4-methoxyphenyl)-4-methyl-4,5-dihydro-1H-pyrazole-3-carboxamide
286 *cis*-1-(2-chlorophenyl)-N-(4-hydroxypiperidin-1-yl)-5-(4-methoxyphenyl)-4-methyl-4,5-dihydro-1H-pyrazole-3-carboxamide
287 *cis*-1-(2,4-dichlorophenyl)-N-(3-hydroxypiperidin-1-yl)-5-(4-methoxyphenyl)-4-methyl-4,5-dihydro-1H-pyrazole-3-carboxamide
288 *cis*-1-(2-chlorophenyl)-N-(3-hydroxypiperidin-1-yl)-5-(4-methoxyphenyl)-4-methyl-4,5-dihydro-1H-pyrazole-3-carboxamide
289 *cis*-1-(2,4-dichlorophenyl)-N-(2-hydroxypiperidin-1-yl)-5-(4-methoxyphenyl)-4-methyl-4,5-dihydro-1H-pyrazole-3-carboxamide
290 *cis*-1-(2-chiorophenyl)-N-(2-hydroxypiperidin-1-yl)-5-(4-methoxyphenyl)-4-methyl-4,5-dihydro-1H-pyrazole-3-carboxamide
291 cis-1-(2,4-dichlorophenyl)-5-(4-methoxyphenyl)-4-methyl-N-(4-oxopiperidin-1-yl)-4,5-dihydro-1H-pyrazole-3-carboxamide
292 *cis*-1-(2-chlorophenyl)-5-(4-methoxyphenyl)-4-methyl-N-(4-oxopiperidin-1-yl)-4,5-dihydro-1H-pyrazole-3-carboxamide
293 *cis*-1-(2,4-dichlorophenyl)-5-(4-methoxyphenyl)-4-methyl-N-(3-oxopiperidin-1-yl)-4,5-dihydro-1H-pyrazole-3-carboxamide
294 *cis*-1-(2-chlorophenyl)-5-(4-methoxyphenyl)-4-methyl-N-(3-oxopiperidin-1-yl)-4,5-dihydro-1H-pyrazole-3-carboxamide
295 *cis*-1-(2,4-dichlorophenyl)-N-(3,4-dihydropyridin-1(2H)-yl)-5-(4-methoxyphenyl)-4-methyl-4,5-dihydro-1H-pyrazole-3-carboxamide
296 cis-1-(2-chlorophenyl)-N-(3,4-dihydropyridin-1(2H)-yl)-5-(4-methoxyphenyl)-4-methyl-4,5-dihydro-1H-pyrazole-3-carboxamide
297 *cis*-1-(2,4-dichlorophenyl)-N-(5,6-dihydropyridin-1(2H)-yl)-5-(4-methoxyphenyl)-4-methyl-4,5-dihydro-1H-pyrazole-3-carboxamide
298 *cis*-1-(2-chlorophenyl)-N-(5,6-dihydropyridin-1(2H)-yl)-5-(4-methoxyphenyl)-4-methyl-4,5-dihydro-1H-pyrazole-3-carboxamide
299 *cis*-1-(2,4-dichlorophenyl)-5-(4-methoxyphenyl)-N-(piperidin-1-yl)-4-(trifluoromethyl)-4,5-dihydro-1H-pyrazole-3-carboxamide
300 *trans*-1-(2,4-dichlorophenyl)-5-(4-methoxyphenyl)-N-(piperidin-1-yl)-4-(trifluoromethyl)-4,5-dihydro-1H-pyrazole-3-carboxamide
301 *cis*-N-(azepan-1-yl)-1-(2,4-dichlorophenyl)-5-(4-methoxyphenyl)-4-(trifluoromethyl)-4,5-dihydro-1H-pyrazole-3-carboxamide
302 *trans*-N-(azepan-1-yl)-1-(2,4-dichlorophenyl)-5-(4-methoxyphenyl)-4-(trifluoromethyl)-4,5-dihydro-1H-pyrazole-3-carboxamide
303 *cis*-1-(2-chlorophenyl)-5-(4-methoxyphenyl)-N-(piperidin-1-yl)-4-(trifluoromethyl)-4,5-dihydro-1H-pyrazole-3-carboxamide
304 *trans*-1-(2-chlorophenyl)-5-(4-methoxyphenyl)-N-(piperidin-1-yl)-4-(trifluoromethyl)-4,5-dihydro-1H-pyrazole-3-carboxamide
305 1-(2,4-dichlorophenyl)-5-(4-methoxyphenyl)-4,4-dimethyl-N-(piperidin-1-yl)-4,5-dihydro-1H-pyrazole-3-carboxamide

306 N-(azepan-1-yl)-1-(2,4-dichlorophenyl)-5-(4-methoxyphenyl)-4,4-dimethyl-4,5-dihydro-1 H-pyrazole-3-carboxamide
307 1-(2-chlorophenyl)-5-(4-methoxyphenyl)-4,4-dimethyl-N-(piperidin-1-yl)-4,5-dihydro-1 H-pyrazole-3-carboxamide
308 1-(2,4-dichlorophenyl)-4-formyl-5-(4-methoxyphenyl)-N-(piperidin-1-yl)-4,5-dihydro-1H-pyrazole-3-carboxamide
309 1-(2,4-dichlorophenyl)-5-(4-methoxyphenyl)-3-(piperidin-1-ylcarbamoyl)-4,5-dihydro-1 H-pyrazole-4-carboxylic acid
310 1-(2,4-dichlorophenyl)-4-(2-hydroxyethyl)-5-(4-methoxyphenyl)-N-(piperidin-1-yl)-4,5-dihydro-1 H-pyrazole-3-carboxamide
311 1-(2,4-dichlorophenyl)-4-(fluoromethyl)-5-(4-methoxyphenyl)-N-(piperidin-1-yl)-4,5-dihydro-1H-pyrazole-3-carboxamide
312 *cis*-4-cyano-1-(2,4-dichlorophenyl)-5-(4-methoxyphenyl)-N-(piperidin-1-yl)-4,5-dihydro-1 H-pyrazole-3-carboxamide
313 trans-4-cyano-1-(2,4-dichlorophenyl)-5-(4-methoxyphenyl)-N-(piperidin-1-yl)-4,5-dihydro-1H-pyrazole-3-carboxamide
314 *cis*-N-(azepan-1-yl)-4-cyano-1-(2,4-dichlorophenyl)-5-(4-methoxyphenyl)-4,5-dihydro-1H-pyrazole-3-carboxamide
315 *trans*-N-(azepan-1-yl)-4-cyano-1-(2,4-dichlorophenyl)-5-(4-methoxyphenyl)-4,5-dihydro-1 H-pyrazole-3-carboxamide
316 cis -1-(2-chlorophenyl)-4-cyano-5-(4-methoxyphenyl)-N-(piperidin-1-yl)-4,5-dihydro-1 H-pyrazole-3-carboxamide
317 *trans* -1-(2-chlorophenyl)-4-cyano-5-(4-methoxyphenyl)-N-(piperidin-1-yl)-4,5-dihydro-1H-pyrazole-3-carboxamide
318 1-(2,4-dichlorophenyl)-4-hydroxy-5-(4-methoxyphenyl)-N-(piperidin-1-yl)-4,5-dihydro-1H-pyrazole-3-carboxamide
319 1-(2-chlorophenyl)-4,4-difluoro-5-(4-methoxyphenyl)-N-(piperidin-1-yl)-4,5-dihydro-1H-pyrazole-3-carboxamide
320 1-(2-chlorophenyl)-4-formyl-5-(4-methoxyphenyl)-N-(piperidin-1-yl)-4,5-dihydro-1H-pyrazole-3-carboxamide
321 1-(2-chlorophenyl)-5-(4-methoxyphenyl)-3-(piperidin-1-ylcarbamoyl)-4,5-dihydro-1H-pyrazole-4-carboxylic acid
322 1-(2-chlorophenyl)-4-(2-hydroxyethyl)-5-(4-methoxyphenyl)-N-(piperidin-1-yl)-4,5-dihydro-1H-pyrazole-3-carboxamide
323 1-(2-chlorophenyl)-4-(fluoromethyl)-5-(4-methoxyphenyl)-N-(piperidin-1-yl)-4,5-dihydro-1H-pyrazole-3-carboxamide
324 1-(2-chlorophenyl)-4-hydroxy-5-(4-methoxyphenyl)-N-(piperidin-1-yl)-4,5-dihydro-1H-pyrazole-3-carboxamide
325 *cis*-1-(2,4-dichlorophenyl)-5-(4-ethoxyphenyl)-4-methyl-N-(piperidin-1-yl)-4,5-dihydro-1H-pyrazole-3-carboxamide
326 *trans*-1-(2,4-dichlorophenyl)-5-(4-ethoxyphenyl)-4-methyl-N-(piperidin-1-yl)-4,5-dihydro-1H-pyrazole-3-carboxamide
327 *cis*-1-(2-chlorophenyl)-5-(4-ethoxyphenyl)-4-methyl-N-(piperidin-1-yl)-4,5-dihydro-1H-pyrazole-3-carboxamide
328 *trans*-1-(2-chlorophenyl)-5-(4-ethoxyphenyl)-4-methyl-N-(piperidin-1-yl)-4,5-dihydro-1H-pyrazole-3-carboxamide
329 *cis*-N-(azepan-1-yl)-1-(2,4-dichlorophenyl)-5-(4-ethoxyphenyl)-4-methyl-4,5-dihydro-1H-pyrazole-3-carboxamide
330 *trans*-N-(azepan-1-yl)-1-(2,4-dichlorophenyl)-5-(4-ethoxyphenyl)-4-methyl-4,5-dihydro-1H-pyrazole-3-carboxamide
331 *cis*-N-(azepan-1-yl)-1-(2-chlorophenyl)-5-(4-ethoxyphenyl)-4-methyl-4,5-dihydro-1H-pyrazole-3-carboxamide
332 *trans*-N-(azepan-1-yl)-1-(2-chlorophenyl)-5-(4-ethoxyphenyl)-4-methyl-4,5-dihydro-1H-pyrazole-3-carboxamide
333 *cis*-N-cycloheptyl-1-(2,4-dichlorophenyl)-5-(4-ethoxyphenyl)-4-methyl-4,5-dihydro-1H-pyrazole-3-carboxamide
334 *cis*-1-(2-chlorophenyl)-N-cycloheptyl-5-(4-ethoxyphenyl)-4-methyl-4,5-dihydro-1 H-pyrazole-3-carboxamide
335 *cis*-1-(2,4-dichlorophenyl)-5-(4-ethoxyphenyl)-N-(hexahydrocyclopenta[c]pyrrol-2(1H)-yl)-4-methyl-4,5-dihydro-1H-pyrazole-3-carboxamide
336 *cis*-1-(2-chlorophenyl)-5-(4-ethoxyphenyl)-N-(hexahydrocyclopenta[c]pyrrol-2(1H)-yl)-4-methyl-4,5-dihydro-1H-pyrazole-3-carboxamide

337 *cis*-1-(2,4-dichlorophenyl)-5-(4-ethoxyphenyl)-N-(indolin-1-yl)-4-methyl-4,5-dihydro-1H-pyrazole-3-carboxamide
338 *cis*-1-(2-chlorophenyl)-5-(4-ethoxyphenyl)-N-(indolin-1-yl)-4-methyl-4,5-dihydro-1H-pyrazole-3-carboxamide
339 (+)-cis-1-(2,4-dichlorophenyl)-5-(4-ethoxyphenyl)-N-((1S,2S)-2-hydroxycyclohexyl)-4-methyl-4,5-dihydro-1H-pyrazole-3-carboxamide
340 (-)-cis-1-(2,4-dichlorophenyl)-5-(4-ethoxyphenyl)-N-((1S,2S)-2-hydroxycyclohexyl)-4-methyl-4,5-dihydro-1H-pyrazole-3-carboxamide
341 (+)-cis-1-(2-chlorophenyl)-5-(4-ethoxyphenyl)-N-((1S,2S)-2-hydroxycyclohexyl)-4-methyl-4,5-dihydro-1H-pyrazole-3-carboxamide
342 (-)-cis-1-(2-chlorophenyl)-5-(4-ethoxyphenyl)-N-((1S,2S)-2-hydroxycyclohexyl)-4-methyl-4,5-dihydro-1H-pyrazole-3-carboxamide
343 *cis*-1-(2,4-dichlorophenyl)-5-(4-hydroxyphenyl)-4-methyl-N-(piperidin-1-yl)-4,5-dihydro-1H-pyrazole-3-carboxamide
344 *trans*-1-(2,4-dichlorophenyl)-5-(4-hydroxyphenyl)-4-methyl-N-(piperidin-1-yl)-4,5-dihydro-1H-pyrazole-3-carboxamide
345 *cis*-1-(2-chlorophenyl)-5-(4-hydroxyphenyl)-4-methyl-N-(piperidin-1-yl)-4,5-dihydro-1H-pyrazole-3-carboxamide
346 *trans*-1-(2-chlorophenyl)-5-(4-hydroxyphenyl)-4-methyl-N-(piperidin-1-yl)-4,5-dihydro-1H-pyrazole-3-carboxamide
347 *cis*-N-(azepan-1-yl)-1-(2,4-dichlorophenyl)-5-(4-hydroxyphenyl)-4-methyl-4,5-dihydro-1H-pyrazole-3-carboxamide
348 *trans*-N-(azepan-1-yl)-1-(2,4-dichlorophenyl)-5-(4-hydroxyphenyl)-4-methyl-4,5-dihydro-1H-pyrazole-3-carboxamide
349 *cis*-N-(azepan-1-yl)-1-(2-chlorophenyl)-5-(4-hydroxyphenyl)-4-methyl-4,5-dihydro-1H-pyrazo le-3-ca rboxa m id e
350 *trans*-N-(azepan-1-yl)-1-(2-chlorophenyl)-5-(4-hydroxyphenyl)-4-methyl-4,5-dihydro-1H-pyrazole-3-carboxamide
351 *cis*-N-cycloheptyl-1-(2,4-dichlorophenyl)-5-(4-hydroxyphenyl)-4-methyl-4,5-dihydro-1H-pyrazole-3-carboxamide
352 *cis*-1-(2-chlorophenyl)-N-cycloheptyl-5-(4-hydroxyphenyl)-4-methyl-4,5-dihydro-1H-pyrazole-3-carboxamide
353 *cis*-1-(2,4-dichlorophenyl)-N-(hexahydrocyclopenta[c]pyrro)-2(1H)-yl)-5-(4-hydroxyphenyl)-4-methyl-4,5-dihydro-1H-pyrazole-3-carboxamide hydrochloride
354 *cis*-1-(2-chlorophenyl)-N-(hexahydrocyclopenta[c]pyrrol-2(1H)-yl)-5-(4-hydroxyphenyl)-4-methyl-4,5-dihydro-1H-pyrazole-3-carboxamide
355 *cis*-1-(2,4-dichlorophenyl)-5-(4-hydroxyphenyl)-N-(indolin-1-yl)-4-methy)-4,5-dihydro-1H-pyrazole-3-carboxamide
356 *cis*-1-(2-ch)orophenyl)-5-(4-hydroxyphenyl)-N-(indolin-1-yl)-4-methyl-4,5-dihydro-1H-pyrazole-3-carboxamide
357 (+)-cis-1-(2,4-dichlorophenyl)-N-((1S,2S)-2-hydroxycyclohexyl)-5-(4-hydroxyphenyl)-4-methyl-4,5-dihydro-1H-pyrazole-3-carboxamide
358 (-)-cis-1-(2.4-dichlorophenyl)-N-((1S,2S)-2-hydroxycyclohexyl)-5-(4-hydroxyphenyl)-4-methyl-4,5-dihydro-1H-pyrazole-3-carboxamide
359 (+)-cis-1-(2-chlorophenyl)-N-((1S,2S)-2-hydroxycyclohexyl)-5-(4-hydroxyphenyl)-4-methyl-4,5-dihydro-1H-pyrazole-3-carboxamide
360 (-)-cis-1-(2-chlorophenyl)-N-((1S,2S)-2-hydroxycyclohexyl)-5-(4-hydroxyphenyl)-4-methyl-4,5-dihydro-1H-pyrazole-3-carboxamide
361 *cis*-5-(5-chlorothiophen-2-yl)-1-(2,4-dichlorophenyl)-4-methyl-4,5-dihydro-1H-pyrazole-3-carboxylic acid
362 *trans*-5-(5-chlorothiophen-2-yl)-1-(2,4-dichlorophenyl)-4-methyl-4,5-dihydro-1H-pyrazole-3-carboxylic acid
363 *cis*-1-(2-chlorophenyl)-5-(5-chlorothiophen-2-yl)-4-methyl-4,5-dihydro-1H-pyrazole-3-carboxylic acid
364 *trans*-1-(2-chlorophenyl)-5-(5-chlorothiophen-2-yl)-4-methyl-4,5-dihydro-1H-pyrazole-3-carboxylic acid
365 *cis*-5-(5-bromothiophen-2-yl)-1-(2,4-dichlorophenyl)-4-ethyl-4,5-dihydro-1H-pyrazole-3-carboxylic acid
366 *trans*-5-(5-bromothiophen-2-yl)-1-(2,4-dichlorophenyl)-4-ethyl-4,5-dihydro-1H-pyrazole-3-carboxylic acid
367 *cis*-1-(2,4-dichlorophenyl)-4-methyl-5-(thiophen-2-yl)-4,5-dihydro-1H-pyrazole-3-carboxylic acid
368 *trans*-1-(2,4-dichlorophenyl)-4-methyl-5-(thiophen-2-yl)-4,5-dihydro-1H-pyrazole-3-carboxylic acid
369 *cis*-1-(2,4-dichlorophenyl)-4-methyl-N-(piperidin-1-yl)-5-(thiophen-2-yl)-4,5-dihydro-1H-pyrazole-3-carboxamide
370 *trans*-1-(2,4-dichlorophenyl)-4-methyl-N-(piperidin-1-yl)-5-(thiophen-2-yl)-4,5-dihydro-1H-pyrazole-3-carboxamide
371 *cis*-1-(2-chlorophenyl)-4-methyl-N-(piperidin-1-yl)-5-(thiophen-2-yl)-4,5-dihydro-1H-pyrazole-3-carboxamide

372 *trans*-1-(2-chlorophenyl)-4-methyl-N-(piperidin-1-yl)-5-(thiophen-2-yl)-4,5-dihydro-1H-pyrazole-3-carboxamide

373 *cis*-1-(2,4-dichlorophenyl)-4-methyl-5-phenyl-4,5-dihydro-1H-pyrazole-3-carboxylic acid

374 *trans*-1-(2,4-dichlorophenyl)-4-methyl-5-phenyl-4,5-dihydro-1H-pyrazole-3-carboxylic acid

375 *cis*-1-(2,4-dichlorophenyl)-4-methyl-5-phenyl-N-(piperidin-1-yl)-4,5-dihydro-1H-pyrazole-3-carboxamide

376 *trans*-1-(2,4-dichlorophenyl)-4-methyl-5-phenyl-N-(piperidin-1-yl)-4,5-dihydro-1H-pyrazole-3-carboxamide

377 *cis*-1-(2-chlorophenyl)-4-methyl-5-phenyl-N-(piperidin-1-yl)-4,5-dihydro-1H-pyrazole-3-carboxamide

378 *trans*-1-(2-chlorophenyl)-4-methyl-5-phenyl-N-(piperidin-1-yl)-4,5-dihydro-1H-pyrazole-3-carboxamide

379 *cis*-5-(4-chlorophenyl)-1-(2,4-dichlorophenyl)-4-methyl-N-(piperidin-1-yl)-4,5-dihydro-1H-pyrazole-3-carbothioamide hydrochloride

380 *trans*-5-(4-chlorophenyl)-1-(2,4-dichlorophenyl)-4-methyl-N-(piperidin-1-yl)-4,5-dihydro-1H-pyrazole-3-carbothioamide

381 *cis* -1-(2-chlorophenyl)-5-(4-chlorophenyl)-4-methyl-N-(piperidin-1-yl)-4,5-dihydro-1H-pyrazole-3-carbothioamide

382 *trans*-1-(2-chlorophenyl)-5-(4-chlorophenyl)-4-methyl-N-(piperidin-1-yl)-4,5-dihydro-1H-pyrazole-3-carbothioamide

383 *cis*-N-(azepan-1-yl)-5-(4-chlorophenyl)-1-(2,4-dichlorophenyl)-4-methyl-4,5-dihydro-1H-pyrazole-3-carbothioamide

384 *cis*-N-(azepan-1-yl)-1-(2-chlorophenyl)-5-(4-chlorophenyl)-4-methyl-4,5-dihydro-1H-pyrazole-3-carbothioamide

385 *cis*-5-(4-bromophenyl)-1-(2,4-dichlorophenyl)-4-methyl-N-(piperidin-1-yl)-4,5-dihydro-1H-pyrazole-3-carbothioamide

386 *trans*-5-(4-bromophenyl)-1-(2,4-dichlorophenyl)-4-methyl-N-(piperidin-1-yl)-4,5-dihydro-1H-pyrazole-3-carbothioamide

387 *cis*-5-(4-bromophenyl)-1-(2-chlorophenyl)-4-methyl-N-(piperidin-1-yl)-4,5-dihydro-1H-pyrazole-3-carbothioamide

388 *trans*-5-(4-bromophenyl)-1-(2-chlorophenyl)-4-methyl-N-(piperidin-1-yl)-4,5-dihydro-1H-pyrazole-3-carbothioamide

389 *cis*-1-(2,4-dichlorophenyl)-5-(4-fluorophenyl)-4-methyl-N-(piperidin-1-yl)-4,5-dihydro-1 H-pyrazole-3-carbothioamide

390 *trans*-1-(2,4-dichlorophenyl)-5-(4-fluorophenyl)-4-methyl-N-(piperidin-1-yl)-4,5-dihydro-1H-pyrazole-3-carbothioamide

391 *cis*-1-(2-chlorophenyl)-5-(4-fluorophenyl)-4-methyl-N-(piperidin-1-yl)-4,5-dihydro-1H-pyrazole-3-carbothioamide

392 *trans*-1-(2-chlorophenyl)-5-(4-fluorophenyl)-4-methyl-N-(piperidin-1-yl)-4,5-dihydro-1H-pyrazole-3-carbothioamide

393 *cis*-1-(2,4-dichlorophenyl)-5-(4-methoxyphenyl)-4-methyl-N-(piperidin-1-yl)-4,5-dihydro-1H-pyrazole-3-carbothioamide

394 *cis*-N-(azepan-1-yl)-1-(2,4-dichlorophenyl)-5-(4-methoxyphenyl)-4-methyl-4,5-dihydro-1H-pyrazole-3-carbothioamide

395 *trans*-1-(2,4-dichlorophenyl)-5-(4-methoxyphenyl)-4-methyl-N-(piperidin-1-yl)-4,5-dihydro-1H-pyrazole-3-carbothioamide

396 *cis*-1-(2-chlorophenyl)-5-(4-methoxyphenyl)-4-methyl-N-(piperidin-1-yl)-4,5-dihydro-1H-pyrazole-3-carbothioamide

397 cis-N-(azepan-1-yl)-1-(2-chlorophenyl)-5-(4-methoxyphenyl)-4-methyl-4,5-dihydro-1H-pyrazole-3-carbothioamide

398 *trans*-1-(2-chlorophenyl)-5-(4-methoxyphenyl)-4-methyl-N-(piperidin-1-yl)-4,5-dihydro-1H-pyrazole-3-carbothioamide

399 *cis*-1-(2,4-dichlorophenyl)-5-(4-ethoxyphenyl)-4-methyl-N-(piperidin-1-yl)-4,5-dihydro-1H-pyrazole-3-carbothioamide

400 *trans*-1-(2,4-dichlorophenyl)5-(4-ethoxyphenyl)-4-methyl-N-(piperidin-1-yl)-4,5-dihydro-1H-pyrazole-3-carbothioamide

401 *cis*-1-(2-chlorophenyl)-5-(4-ethoxyphenyl)-4-methyl-N-(piperidin-1-yl)-4,5-dihydro-1H-pyrazole-3-carbothioamide

402 *trans*-1-(2-chlorophenyl)-5-(4-ethoxyphenyl)-4-methyl-N-(piperidin-1-yl)-4,5-dihydro-1H-pyrazole-3-carbothioamide

403 *cis*-1-(2,4-dichlorophenyl)-5-(4-hydroxyphenyl)-4-methyl-N-(piperidin-1-yl)-4,5-dihydro-1H-pyrazole-3-carbothioamide

404 *trans*-1-(2,4-dichlorophenyl)-5-(4-hydroxyphenyl)-4-methyl-N-(piperidin-1-yl)-4,5-dihydro-1H-pyrazole-3-carbothioamide
405 *cis*-1-(2-chlorophenyl)-5-(4-hydroxyphenyl)-4-methyl-N-(piperidin-1-yl)-4,5-dihydro-1H-pyrazole-3-carbothioamide
406 *trans*-1-(2-chlorophenyl)-5-(4-hydroxyphenyl)-4-methyl-N-(piperidin-1-yl)-4,5-dihydro-1H-pyrazole-3-carbothioamide
407 7-(4-Chloro-phenyl)-6-(2,4-dichlorophenyl)-5,6-diaza-spiro[2.4]hept-4-ene-4-carboxylic acid piperidin-1-ylamide
408 6-(2,4-Dichloro-phenyl)-7-(4-methoxy-phenyl)-5,6-diaza-spiro[2.4]hept-4-ene-4-carboxylic acid piperidin-1-ylamide
409 (+)-*cis*-N-((1S,2S)-2-(benzyloxy)cyclohexyl)-5-(4-chlorophenyl)-1-(2,4-dichlorophenyl)-4-methyl-4,5-dihydro-1H-pyrazole-3-carboxamide
[410] *cis*-1-(2,4-dich)orophenyl)-5-(4-methoxyphenyl)-4-methyl-N-(piperidin-1-yl)-4,5-dihydro-1H-pyrazole-3-carboxamide
[411] *cis*-1-(2,4-dich)orophenyl)-5-(4-methoxyphenyl)-4-methyl-N-(piperidin-1-yl)-4,5-dihydro-1 H-pyrazole-3-carboxamide
[412] (4R,5S)-1-(2,4-dichlorophenyl)-5-(4-methoxyphenyl)-4-methyl-4,5-dihydro-1H-pyrazole-3-carboxylic acid
[413] cis-5-(4-bromophenyl)-1-(2,4-dichlorophenyl)-4-methyl-4,5-dihydro-1H-pyrazole-3-carboxylic acid
[414] cis-N-(azepan-1-yl)-1-(2,4-dichlorophenyl)-5-(4-fluorophenyl)-4-methyl-4,5-dihydro-1H-pyrazole-3-carboxamide
415 *cis*-5-(4-bromophenyl)-1-(2,4-dichlorophenyl)-N-(indolin-1-yl)-4-methyl-4,5-dihydro-1H-pyrazole-3-carboxamide
416 *trans*-ethyl 5-(4-chlorophenyl)-1-(2,4-dichlorophenyl)-4-(trifluoromethyl)-4,5-dihydro-1H-pyrazole-3-carboxylate
417 *cis*-1-(2,4-dichlorophenyl)-5-(4-fluorophenyl)-4-methyl-N-(piperidin-1-yl)-4,5-dihydro-1H-pyrazole-3-carboxamide hydrochloride
418 *cis*-5-(4-bromophenyl)-1-(2,4-dichlorophenyl)-4-methyl-N-(piperidin-1-yl)-4,5-dihydro-1H-pyrazole-3-carboxamide hydrochloride
419 (-)-*cis*-5-(4-chlorophenyl)-1-(2,4-dichlorophenyl)-4-methyl-N-(piperidin-1-yl)-4,5-dihydro-1H-pyrazole-3-carboxamide
420 (+)-*cis*-5-(4-chlorophenyl)-1-(2,4-dichlorophenyl)-4-methyl-N-(piperidin-1-yl)-4,5-dihydro-1 H-pyrazole-3-carboxamide
421 *trans*-ethyl 1-(2,4-dichlorophenyl)4-methyl-5-phenyl-4,5-dihydro-1H-pyrazole-3-carboxylate
422 cis-5-(4-chlorophenyl)-1-(2,4-dichlorophenyl)-4-methyl-N-((1R,2S,4R)-1,7,7-trimethylbicyclo[2.2.1]heptan-2-yl)-4,5-dihydro-1H-pyrazole-3-carboxamide
423 *cis*-5-(4-chlorophenyl)-1-(2,4-dichlorophenyl)-4-methyl-N-(3-methylcyclohexyl)-4,5-dihydro-1 H-pyrazole-3-carboxamide
424 *cis*-5-(4-chlorophenyl)-1-(2,4-dichlorophenyl)-4-methyl-N-(2-methylcyclohexyl)-4,5-dihydro-1H-pyrazole-3-carboxamide
426 *trans*-5-(5-chlorothiophen-2-yl)-1-(2,4-dichlorophenyl)-4-methyl-N-(piperidin-1-yl)-4,5-dihydro-1H-pyrazole-3-carboxamide hydrochloride
427 *cis*-5-(5-chlorothiophen-2-yl)-1-(2,4-dichlorophenyl)-4-methyl-N-(piperidin-1-yl)-4,5-dihydro-1H-pyrazole-3-carboxamide hydrochloride
428 *cis*-5-(4-bromophenyl)-1-(2,4-dichlorophenyl)-4-ethyl-N-(piperidin-1-yl)-4,5-dihydro-1H-pyrazole-3-carboxamide hydrochloride
[429] 1-(2,4-dichlorophenyl)-4-methyl-5-phenyl-4,5-dihydro-1H-pyrazole-3-carboxylic acid
[430] cis-1-(2,4-dichlorophenyl)-4-methyl-N-(piperidin-1-yl)-5-(thiophen-2-yl)-4,5-dihydro-1H-pyrazole-3-carboxamide hydrochloride
[431] trans-5-(4-chlorophenyl)-1-(2,4-dichlorophenyl)-4-methyl-N-(piperidin-1-yl)-4,5-dihydro-1H-pyrazole-3-carboxamide
432 *cis*-5-(4-chlorophenyl)-1-(2,4-dichlorophenyl)-4-ethyl-N-(piperidin-1-yl)-4,5-dihydro-1H-pyrazole-3-carboxamide
434 *cis*-5-(4-chlorophenyl)-1-(2,4-dichlorophenyl)-4-methyl-N-(piperidin-1-yl)-4,5-dihydro-1H-pyrazole-3-carboxamide
436 (+)-*cis*-N-((1S,2S)-2-(benzyloxy)cyclohexyl)-5-(4-chlorophenyl)-1-(2,4-dichlorophenyl)-4-methyl-4,5-dihydro-1H-pyrazole-3-carboxamide
437 (-)-cis-N-((11R,2R)-2-(benzyloxy)cyclohexyl)-5-(4-chlorophenyl)-1-(2,4-dichlorophenyl)-4-methyl-4,5-dihydro-1H-pyrazole-3-carboxamide

438 *cis*-N-(azocan-1-yl)-1-(2,4-dichlorophenyl)-5-(4-methoxyphenyl)-4-methyl-4,5-dihydro-1 H-pyrazole-3-carboxamide

439 *cis*-N-(azocan-1-yl)-1-(2-chlorophenyl)-5-(4-methoxyphenyl)-4-methyl-4,5-dihydro-1H-pyrazole-3-carboxamide

440 cis-N-(azocan-1-yl)-5-(4-bromophenyl)-1-(2,4-dichlorophenyl)-4-methyl-4,5-dihydro-1 H-pyrazole-3-carboxamide

441 *cis*-N-(azocan-1-yl)-5-(4-bromophenyl)-1-(2-chlorophenyl)-4-methyl-4,5-dihydro-1H-pyrazole-3-carboxamide

442 *cis*-N-(azocan-1-yl)-1-(2,4-dichlorophenyl)-5-(4-fluorophenyl)-4-methyl-4,5-dihydro-1H-pyrazole-3-carboxamide

443 *cis*-N-(azocan-1-yl)-(2-chlorophenyl)-5-(4-fluorophenyl)-4-methyl-4,5-dihydro-1H-pyrazole-3-carboxamide

444 (+)-*cis*-5-(4-Chlorophenyl)-1-(2,4-dichlorophenyl)-4-methyl-4,5-dihydro-1H-pyrazole-3-carboxylic acid azepan-1-ylamide hydrochloride

445 (-)-cis- (4-Chlorophenyl)-1-(2,4-dichlorophenyl)-4-methyl-4,5-dihydro-1H-pyrazole-3-carboxylic acid azepan-1-ylamide hydrochloride

[446] *cis-5*-(4-chlorophenyl)-1-(2,4-dichlorophenyl)-4-methyl-N-(piperidin-1-yl)-4,5-dihydro-1H-pyrazole-3-carboxamide N-oxide

[447] *cis*-5-(4-chlorophenyl)-1-(2-chlorophenyl)-4-methyl-N-(piperidin-1-yl)-4,5-dihydro-1H-pyrazole-3-carboxamide N-oxide

[448] *cis*-N-(azepan-1-yl)-5-(4-chlorophenyl)-1-(2,4-dichlorophenyl)-4-methyl-4,5-dihydro-1H-pyrazole-3-carboxamide N-oxide

[449] *cis*-N-(azepan-1-yl)-5-(4-chlorophenyl)-1-(2-chlorophenyl)-4-methyl-4,5-dihydro-1H-pyrazole-3-carboxamide N-oxide

[450] *cis*-1-(2,4-dichlorophenyl)-5-(4-methoxyphenyl)-4-methyl-N-(piperidin-1-yl)-4,5-dihydro-1H-pyrazole-3-carboxamide N-oxide and

[451] *cis*-1-(2-chlorophenyl)-5-(4-methoxyphenyl)-4-methyl-N-(piperidin-1-yl)-4,5-dihydro-1H-pyrazole-3-carboxamide N-oxide;

optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

Also preferred are Indoline-substituted pyrazoline compounds (A) of subgroup A5.1 of general formulas IV, IVa or IVb,

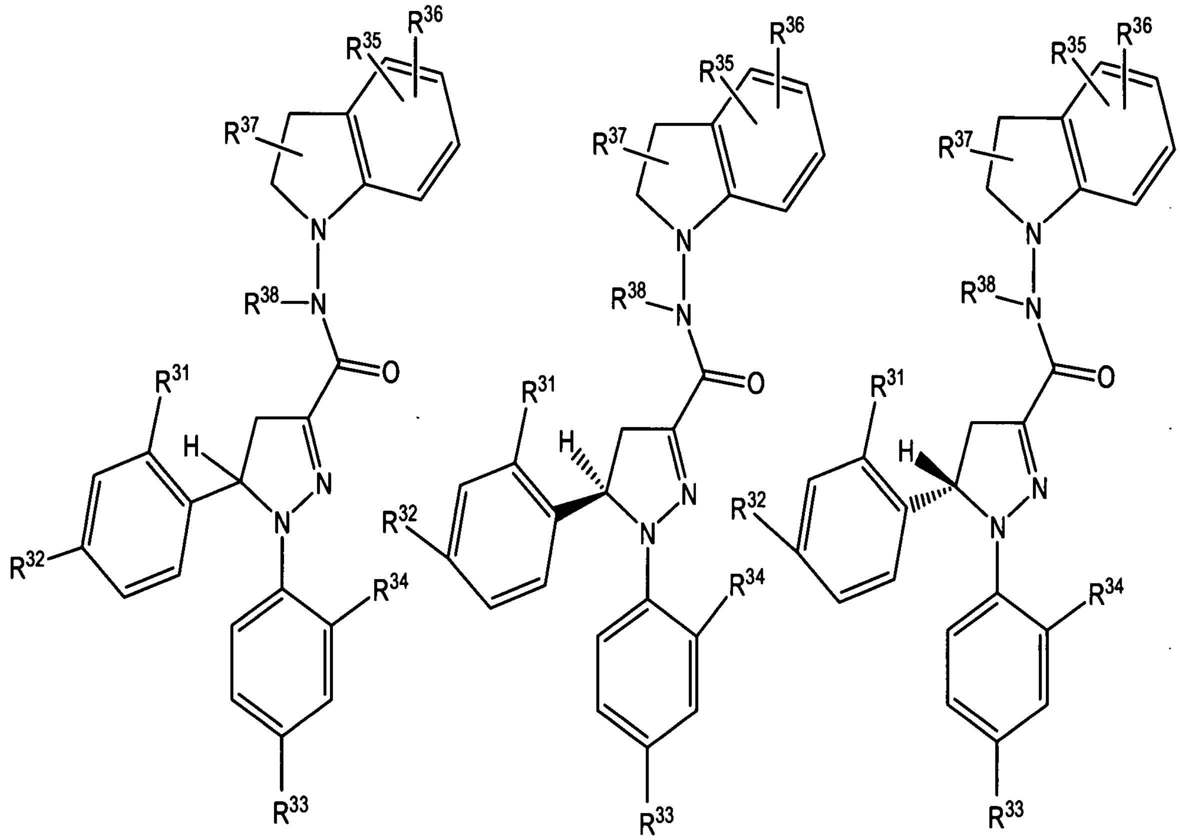

IV IVa IVb

wherein

| | |
|---|---|
| $R^{31}$, $R^{32}$, $R^{33}$ and $R^{34}$ | independently of one another represent: H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl; |
| $R^{35}$, $R^{36}$ and $R^{37}$ | are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$; |
| $R^{38}$ | representing a hydrogen atom or a branched or linear $C_{1-3}$-alkyl group; |

optionally in the form shown or in form of the acid or base or in form of a salt, especially a physiologically acceptable salt, or in form of a solvate, especially a hydrate or in form of a corresponding N-oxide thereof;
or Indoline-substituted pyrazoline compounds (A) of subgroup A5.1 of general formulas IVc, IVd or IVe

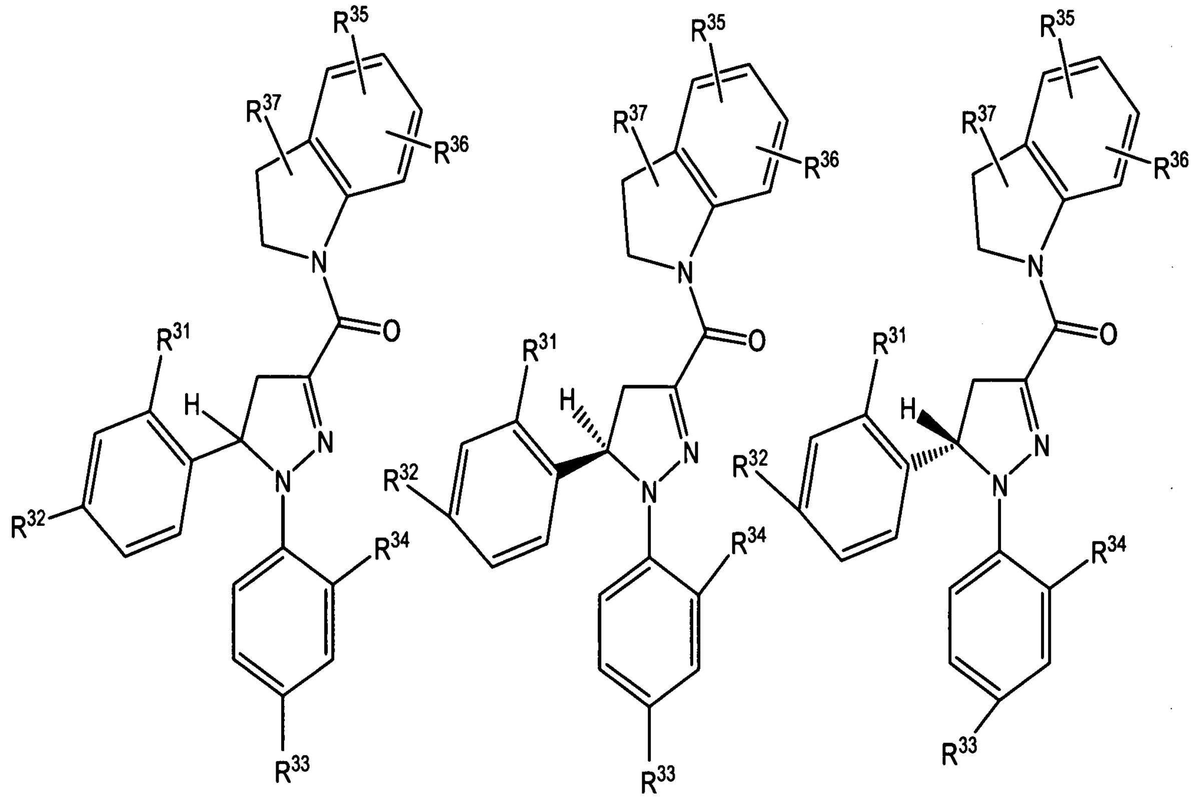

IVc IVd IVe

wherein

| | |
|---|---|
| $R^{31}$, $R^{32}$, $R^{33}$ and $R^{34}$ | independently of one another represent: H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)$C_{1-20}$-alkyl; |
| p | is 1 or 2; |
| $R^{35}$, $R^{36}$ and $R^{37}$ | are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$; |

optionally in the form shown or in form of the acid or base or in form of a salt, especially a physiologically acceptable salt, or in form of a solvate, especially a hydrate or in form of a corresponding N-oxide thereof.

Especially preferred compounds of subgroup A5.1 are selected from

- 5-(4-chlorophenyl)-1-(2,4-dichlorophenyl)-N-(indolin-1-yl)-4,5-dihydro-1H-pyrazole-3-carboxamide,
- (R)-5-(4-chlorophenyl)-1-(2,4-dichlorophenyl)-N-(indolin-1-yl)-4,5-dihydro-1H-pyrazole-3-carboxamide.
- (S)-5-(4-chlorophenyl)-1-(2,4-dichlorophenyl)-N-(indolin-1-yl)-4,5-dihydro-1H-pyrazole-3-carboxamide,
- 5-(4-bromophenyl)-1-(2,4-dichlorophenyl)-N-(indolin-1-yl)-4,5-dihydro-1H-pyrazole-3-carboxamide,

- 1-(2,4-dichlorophenyl)-N-(indolin-1-yl)-5-(4-methoxyphenyl)-4,5-dihydro-1 H-pyrazole-3-carboxamide.

Also preferred are cykloalkane-substituted pyrazoline compounds (A) of subgroup A5.2 of general formulas IV, IVa or IVb

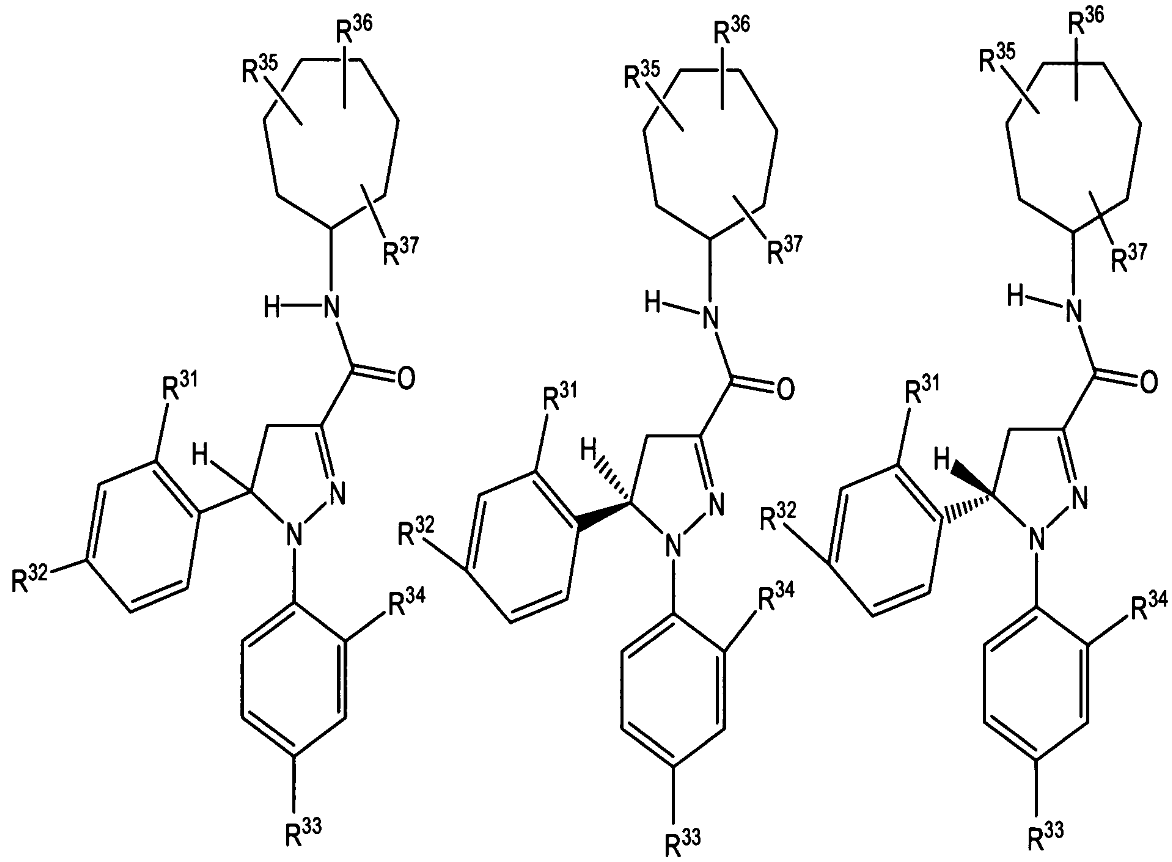

IV IVa IVb

wherein

$R^{31}$, $R^{32}$, $R^{33}$ and $R^{34}$ independently of one another represent:
H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of substituted or unsubstituted, branched or linear, saturated or unsaturated $C_{8-20}$-alkyl or C(O)-$C_{1-20}$-alkyl; substituted or unsubstituted aryl, alkyl- aryl, heterocyclyl or alkyl- heterocyclyl; substituted or unsubstituted C (O)-aryl, C (O)-alkyl- aryl, C (O)-heterocyclyl or C (O)-alkyl- heterocyclyl;

$R^{35}$, $R^{36}$ and $R^{17}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;

optionally in the form shown or in form of the acid or base or in form of a salt, especially a physiologically acceptable salt, or in form of a solvate, especially a hydrate or in form of a corresponding N-oxide thereof.

Especially preferred compounds of subgroup A5.2 are selected from

- S-(4-chlorophenyl)-N-cycloheptyl-1-(2,4-dichlorophenyl)-4,5-dihydro-1H-pyrazole-3-carboxamide,
- (R)- 5-(4-chlorophenyl)-N-cycloheptyl-1-(2,4-dichlorophenyl)-4,5-dihydro-1H-pyrazole-3-carboxamide,
- (S)- 5-(4-chlorophenyl)-N-cycloheptyl-1-(2,4-dichlorophenyl)-4,5-dihydro-1H-pyrazole-3-carboxamide,
- 5-(4-chlorophenyl)-N-(cycloheptylmethyl)-1-(2,4-dichlorophenyl)-4,5-dihydro-1H-pyrazole-3-carboxamide,
- 5-(4-bromophenyl)-N-cycloheptyl-1-(2,4-dichlorophenyl)-4,5-dihydro-1H-pyrazole-3-carboxamide,
- 5-(4-fluorophenyl)-N-cycloheptyl-1-(2,4-dichlorophenyl)-4,5-dihydro-1H-pyrazole-3-carboxamide,
- 5-(4-methoxyphenyl)-N-cycloheptyl-1-(2,4-dichlorophenyl)-4,5-dihydro-1H-pyrazole-3-carboxamide.

Also preferred are Octahydropentalene -substituted pyrazoline compounds (A) of subgroup A5.3 of general formulas IV, IVa or IVb

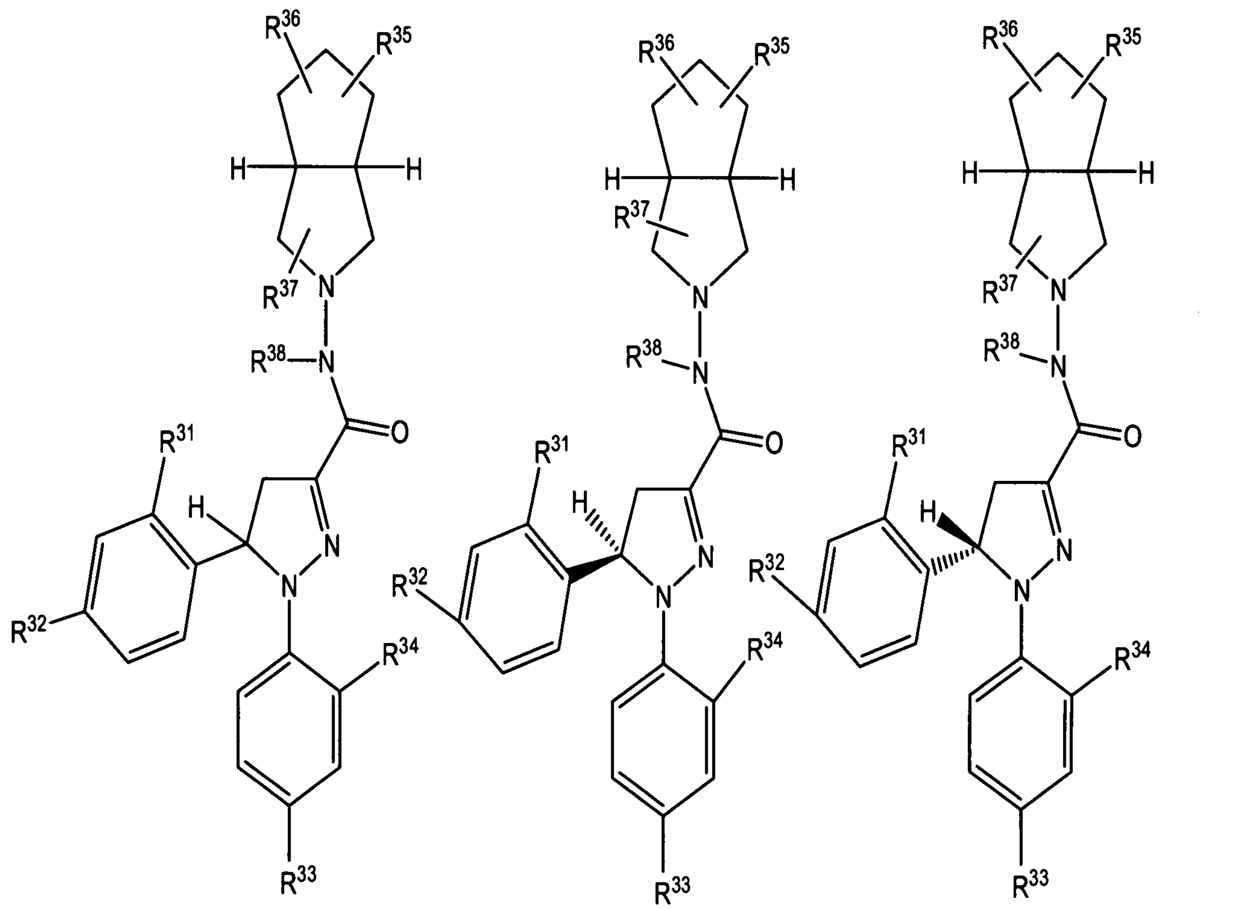

IV IVa IVb

wherein

| | |
|---|---|
| $R^{31}$, $R^{32}$, $R^{33}$ and $R^{34}$ | independently of one another represent: H; branched or linear $C_{1-3}$-alkyl or branched or linear $C_{1-3}$-alkoxy, phenyl, hydroxy, chloro, bromo, fluoro., iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl; |
| $R^{35}$, $R^{36}$ and $R^{37}$ | are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$; |
| $R^{38}$ | representing a hydrogen atom or a branched or linear $C_{1-3}$-alkyl group; |

optionally in the form shown or in form of the acid or base or in form of a salt, especially a physiologically acceptable salt, or in form of a solvate, especially a hydrate or in form of a corresponding N-oxide thereof;
or

octahydropentalene-substituted pyrazoline compounds of general formulas IVc, IVd or IVe

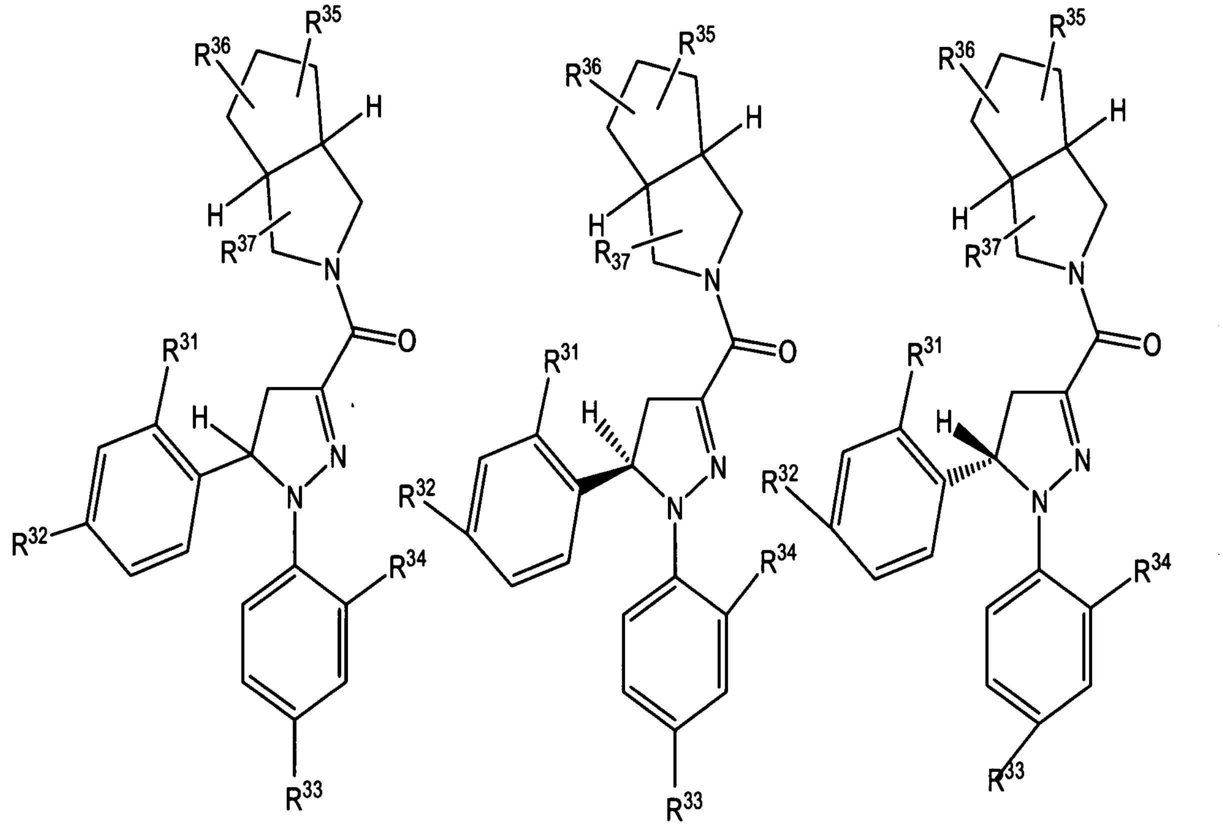

IVc IVd IVe

wherein

| | |
|---|---|
| $R^{31}$, $R^{32}$, $R^{33}$ and $R^{34}$ | independently of one another represent: H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, $C(O)_{1-20}$-alkyl; |
| $R^{35}$, $R^{36}$ and $R^{37}$ | are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$; |

optionally in the form shown or in form of the acid or base or in form of a salt, especially a physiologically acceptable salt, or in form of a solvate, especially a hydrate or in form of a corresponding N-oxide thereof.

Especially preferred compounds of subgroup A5.3 are selected from

- 5-(4-chlorophenyl)-1-(2,4-dichlorophenyl)-N-(hexahydrocyclopenta[c]pyrrol-2(1H)-yl)-4,5-dihydro-1H-pyrazole-3-carboxamide,
- (R)-5-(4-chlorophenyl)-1-(2,4-dichlorophenyl)-N-(hexahydrocyclopenta[c]pyrrol-2(1H)-yl)-4,5-dihydro-1H-pyrazole-3-carboxamide,
- (S)-5-(4-chlorophenyl)-1-(2,4-dichlorophenyl)-N-(hexahydrocyclopenta[c]pyrrol-2(1H)-yl)-4,5-dihydro-1H-pyrazole-3-carboxamide,
- 5-(4-bromophenyl)-1-(2,4-dichlorophenyl)-N-(hexahydrocyclopenta[c]pyrrol-2(1H)-yl)-4,5-dihydro-1H-pyrazole-3-carboxamide,
- 1-(2,4-dichlorophenyl)-5-(4-fluorophenyl)-N-(hexahydrocyclopenta[c]pyrrol-2(1 H)-yl)-4,5-dihydro-1H-pyrazole-3-carboxamide, 1-(2,4-dichlorophenyl)-N-(hexahydrocyclopenta[c]pyrrol-2(1H)-yl)-5-(4-methoxyphenyl)-4,5-dihydro-1H-pyrazole-3-carboxamide.

Also preferred are Azepane-substituted pyrazoline compounds (A) of subgroup A5.4 of general formulas IV, IVa or IVb

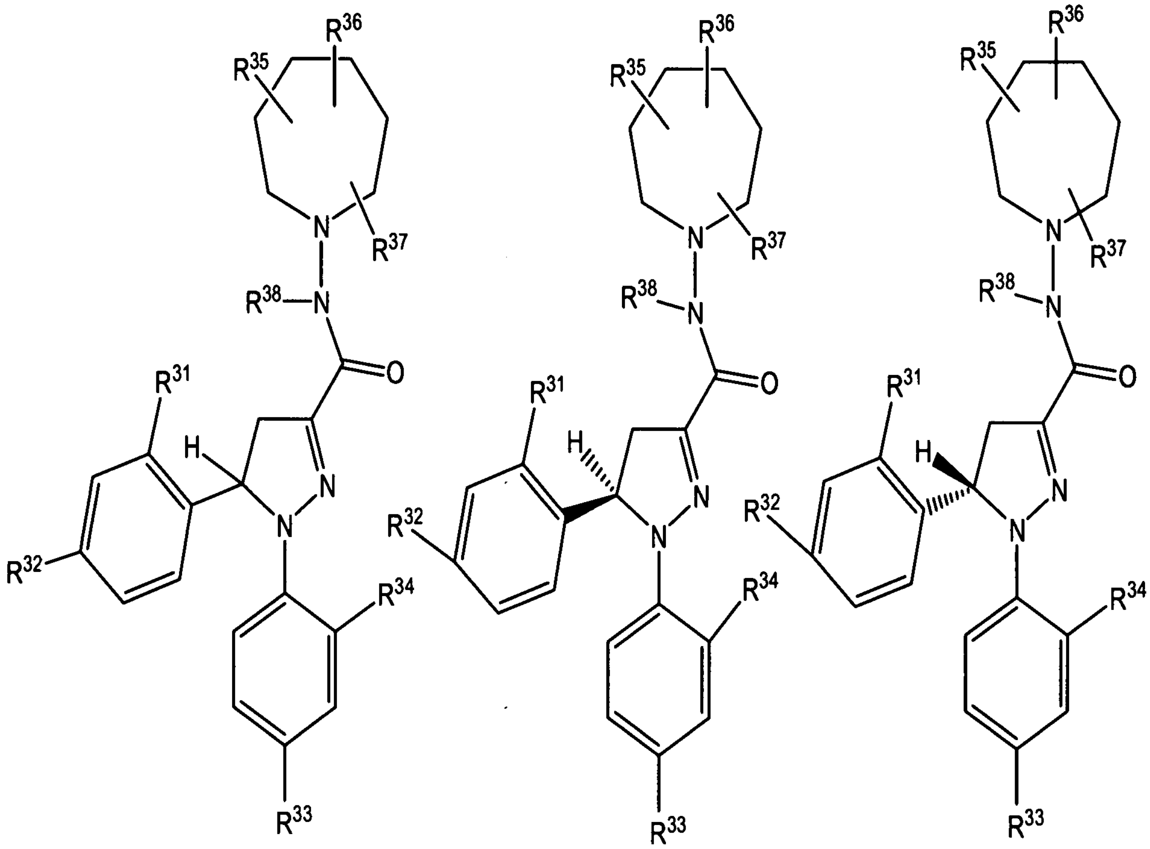

IV IVa IVb

wherein

| | |
|---|---|
| $R^{31}$, $R^{32}$, $R^{33}$ and $R^{34}$ | independently of one another represent: H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl; |
| $R^{35}$, $R^{36}$ and $R^{37}$ | are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$; |
| $R^{38}$ | representing a hydrogen atom or a branched or linear $C_{1-3}$-alkyl group; |

optionally in the form shown or in form of the acid or base or in form of a salt, especially a physiologically acceptable salt, or in form of a solvate, especially a hydrate or in form of a corresponding N-oxide thereof;

or

azepane substituted pyrazoline compounds of general formulas IVc, IVd or IVe

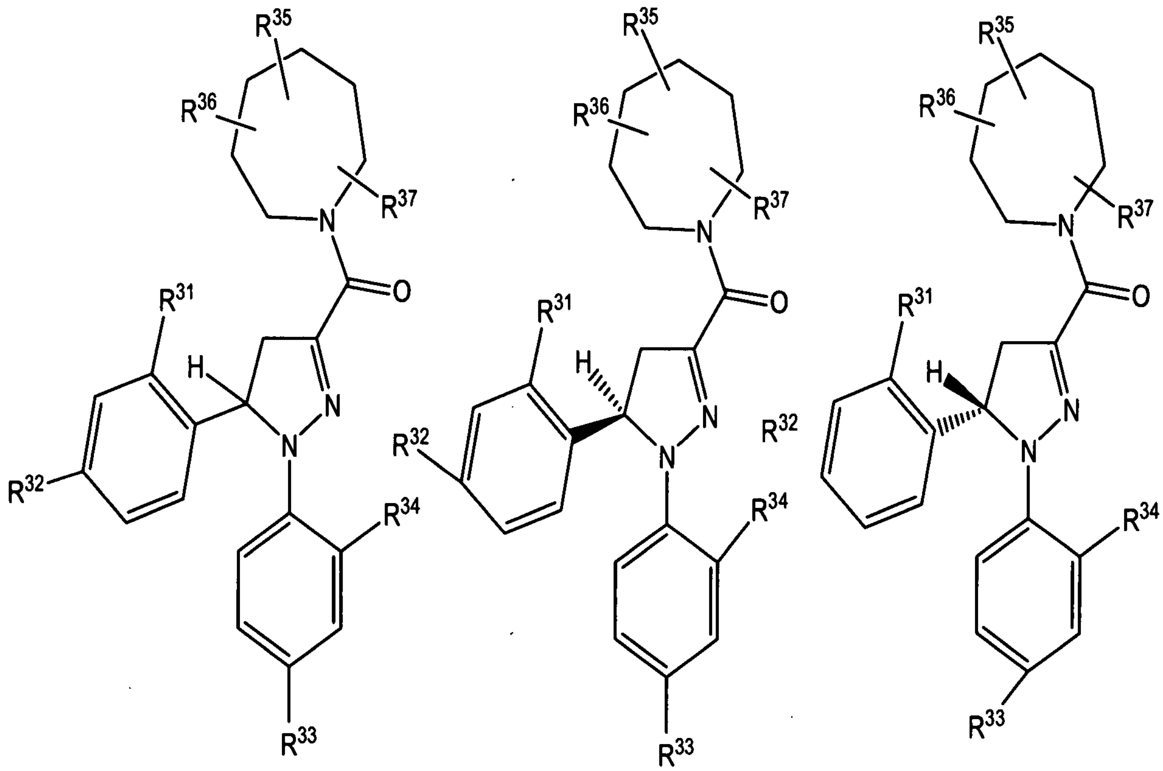

IVc IVd IVe

wherein

| | |
|---|---|
| $R^{31}$, $R^{32}$, $R^{33}$ and $R^{34}$ | independently of one another represent: H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, |

| | |
|---|---|
| | C(O)-$C_{1-20}$-alkyl; |
| $R^{35}$, $R^{36}$ and $R^{37}$ | are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$; |

optionally in the form shown or in form of the acid or base or in form of a salt, especially a physiologically acceptable salt, or in form of a solvate, especially a hydrate or in form of a corresponding N-oxide thereof.

Especially preferred compounds of subgroup A5.4 are selected from

- N-(azepan-1-yl)-5-(4-chlorophenyl)-1-(2,4-dichlorophenyl)-4,5-dihydro-1H-pyrazole-3-carboxamide;
- (R)-N-(azepan-1-yl)-5-(4-chlorophenyl)-1-(2,4-dichlorophenyl)-4,5-dihydro-1H-pyrazole-3-carboxamide;
- (S)-N-(azepan-1-yl)-5-(4-chlorophenyl)-1-(2,4-dichlorophenyl)-4,5-dihydro-1H-pyrazole-3-carboxamide;
- N-(azepan-1-yl)-5-(4-bromophenyl)-1-(2,4-dichlorophenyl)-4,5-dihydro-1H-pyrazole-3-carboxamide,
- N-(azepan-1-yl)-5-(4-fluorophenyl)-1-(2,4-dichlorophenyl)-4,5-dihydro-1H-pyrazole-3-carboxamide,
- N-(azepan-1-yl)-5-(4-methoxyphenyl)-1-(2,4-dichlorophenyl)-4,5-dihydro-1H-pyrazole-3-carboxamide,
- (azepan-1-yl)(1-(2,4-dichlorophenyl)-5-(4-chlorophenyl)-4,5-dihydro-1H-pyrazol-3-yl)methanone,

Also preferred are Indoline-substituted pyrazoline compounds (A) of subgroup A6.1 of general formulas IV, IVa or IVb

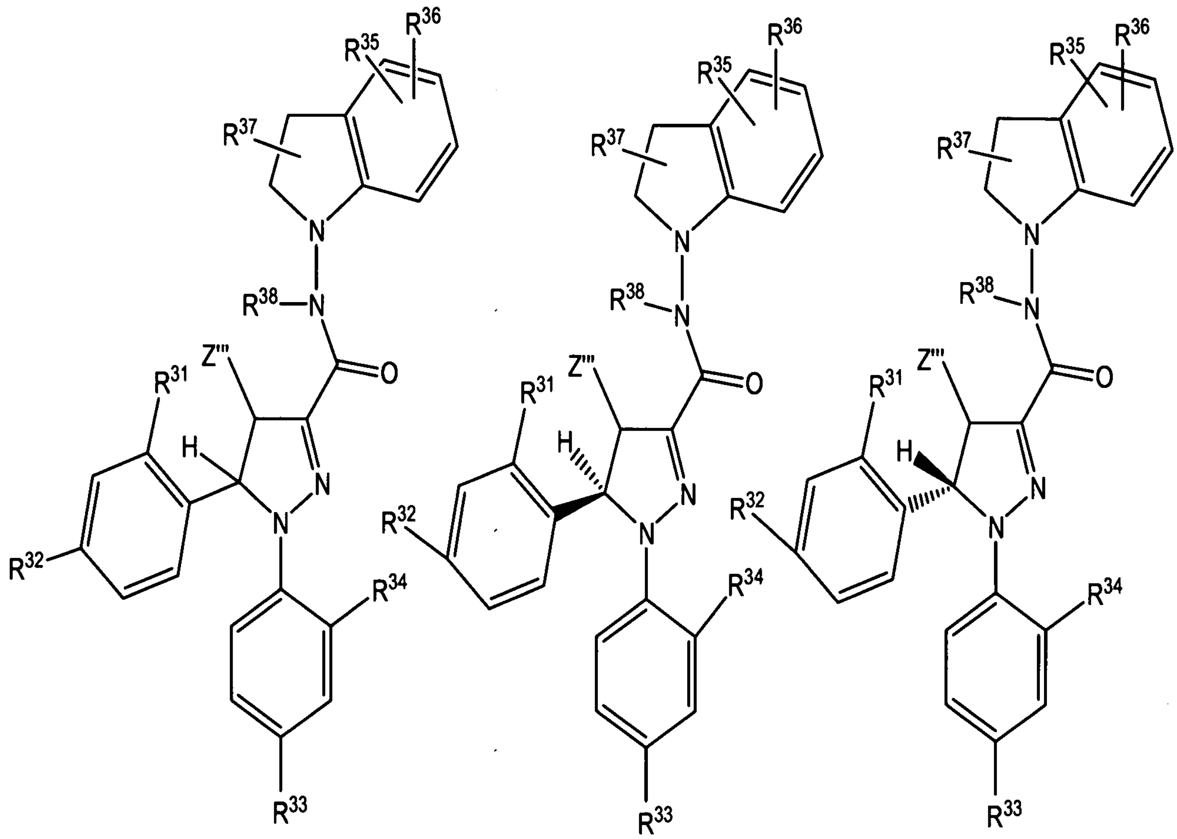

IV IVa IVb

wherein

| | |
|---|---|
| Z''' | is selected from $CH_3$ or $C_2H_5$; |
| $R^{31}$, $R^{32}$, $R^{33}$ and $R^{34}$ | independently of one another represent: H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl; |
| $R^{35}$, $R^{36}$ and $R^{37}$ | are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$; |
| $R^{38}$ | representing a hydrogen atom or a branched or linear $C_{1-3}$-alkyl group; |

optionally in the form shown or in form of the acid or base or in form of a salt, especially a physiologically acceptable salt, or in form of a solvate, especially a hydrate or in form of a corresponding N-oxide thereof;

or

Indoline-substituted pyrazoline compounds of general formulas IVc, IVd or IVe

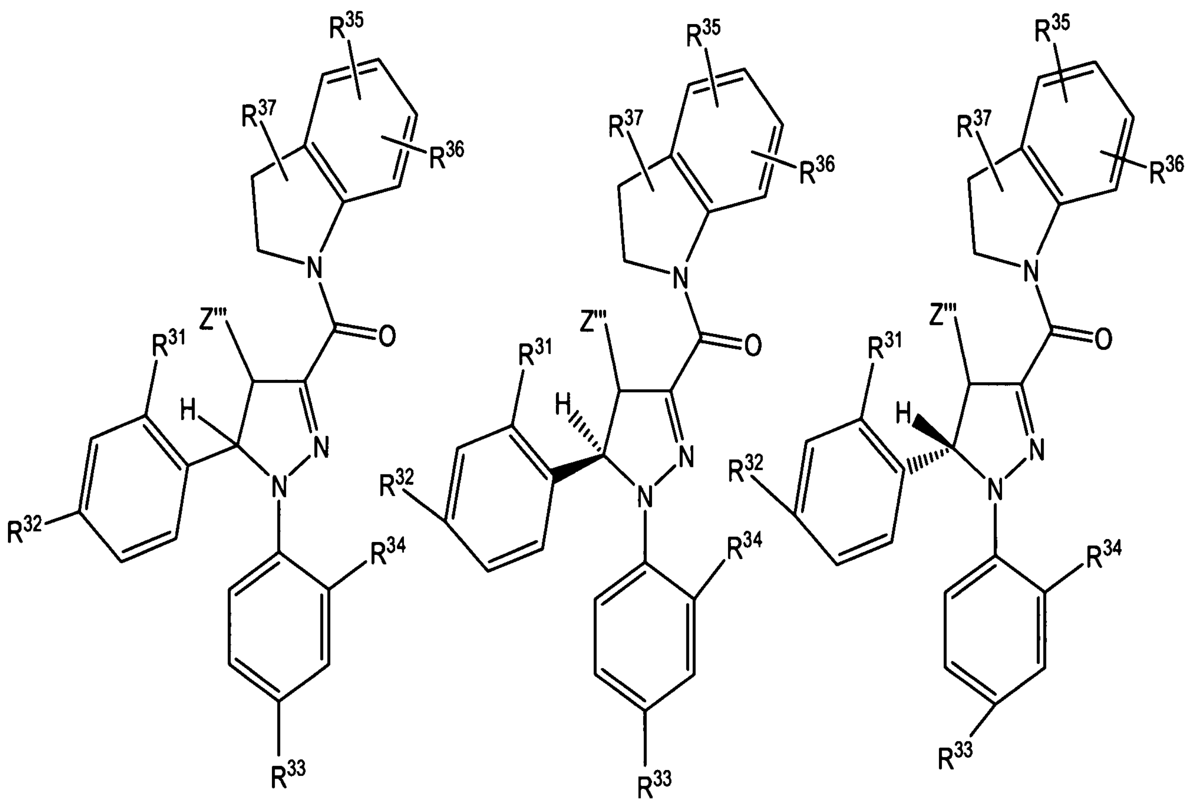

IVc IVd IVe

wherein

| | |
|---|---|
| Z''' | is selected from $CH_3$ or $C_2H_5$; |
| $R^{31}$, $R^{32}$, $R^{33}$ and $R^{34}$ | independently of one another represent: H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl; |
| p | is 1 or 2; |
| $R^{35}$, $R^{36}$ and $R^{37}$ | are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$; |

optionally in the form shown or in form of the acid or base or in form of a salt, especially a physiologically acceptable

salt, or in form of a solvate, especially a hydrate or in form of a corresponding N-oxide thereof.

Especially preferred compounds of subgroup A6.1 are selected from

- cis-5-(4-chlorophenyl)-1-(2,4-dichlorophenyl)-N-(indolin-1-yl)-4-methyl-4,5-dihydro-1H-pyrazole-3-carboxamide
- trans-5-(4-chlorophenyl)-1-(2,4-dichlorophenyl)-N-(indolin-1-yl)-4-methyl-4,5-dihydro-1H-pyrazole-3-carboxamide
- (4R,5R)-5-(4-chlorophenyl)-1-(2,4-dichlorophenyl)-N-(indolin-1-yl)-4-methyl-4,5-dihydro-1H-pyrazole-3-carboxamide
- (4S,5S)-5-(4-chlorophenyl)-(2,4-dichlorophenyl)-N-(indolin-1-yl)-4-methyl-4,5-dihydro-1H-pyrazole-3-carboxamide
- cis-5-(4-chlorophenyl)-1-(2,4-dichlorophenyl)-4-methyl-N-(2-methylindolin-1-yl)-4,5-dihydro-1H-pyrazole-3-carboxamide
- cis-5-(4-bromophenyl)-1-(2,4-dichlorophenyl)-N-(indolin-1-yl)-4-methyl-4,5-dihydro-1 H-pyrazole-3-carboxamide
- trans-5-(4-bromophenyl)-1-(2,4-dichlorophenyl)-N-(indolin-1-yl)-4-methyl-4,5-dihydro-1 H-pyrazole-3-carboxamide
- cis-1-(2,4-dichlorophenyl)-5-(4-fluorophenyl)-N-(indolin-1-yl)-4-methyl-4,5-dihydro-1H-pyrazole-3-carboxamide
- trans-1-(2,4-dichlorophenyl)-5-(4-fluorophenyl)-N-(indolin-1-yl)-4-methyl-4,5-dihydro-1H-pyrazole-3-carboxamide
- cis-1-(2,4-dichlorophenyl)-N-(indolin-1-yl)-5-(4-iodophenyl)-4-methyl-4,5-dihydro-1H-pyrazole-3-carboxamide
- trans-1-(2,4-dichlorophenyl)-N-(indolin-1-yl)-5-(4-iodophenyl)-4-methyl-4,5-dihydro-1H-pyrazole-3-carboxamide
- cis-1-(2,4-dichlorophenyl)-N-(indolin-1-yl)-5-(4-methoxyphenyl)-4-methyl-4,5-dihydro-1H-pyrazole-3-carboxamide
- trans-1-(2,4-dichlorophenyl)-N-(indolin-1-yl)-5-(4-methoxyphenyl)-4-methyl-4,5-dihydro-1H-pyrazole-3-carboxamide
- cis-1-(2,4-dichlorophenyl)-5-(4-hydroxyphenyl)-N-(indolin-1-yl)-4-methyl-4,5-dihydro-1H-pyrazole-3-carboxamide
- trans-1-(2,4-dichlorophenyl)-5-(4-hydroxyphenyl)-N-(indolin-1-yl)-4-methyl-4,5-dihydro-1H-pyrazole-3-carboxamide
- cis-5-(5-chlorothiophen-2-yl)-1-(2,4-dichlorophenyl)-N-(indolin-1-yl)-4-methyl-4,5-dihydro-1H-pyrazole-3-carboxamide
- trans-5-(5-chlorothiophen-2-yl)-1-(2,4-dichlorophenyl)-N-(indolin-1-yl)-4-methyl-4,5-dihydro-1H-pyrazole-3-carboxamide
- cis-5-(5-bromothiophen-2-yl)-1-(2,4-dichlorophenyl)-N-(indolin-1-yl)-4-methyl-4,5-dihydro-1H-pyrazole-3-carboxamide
- trans-5-(5-bromothiophen-2-yl)-1-(2,4-dichlorophenyl)-N-(indolin-1-yl)-4-methyl-4,5-dihydro-1H-pyrazole-3-carboxamide
- cis-5-(4-chlorophenyl)-1-(2,4-dichlorophenyl)-4-ethyl-N-(indolin-1-yl)-4,5-dihydro-1H-pyrazole-3-carboxamide
- trans-5-(4-chlorophenyl)-1-(2,4-dichlorophenyl)-4-ethyl-N-(indolin-1-yl)-4,5-dihydro-1 H-pyrazole-3-carboxamide
- cis-5-(4-bromophenyl)-1-(2,4-dichlorophenyl)-4-ethyl-N-(indolin-1-yl)-4,5-dihydro-1 H-pyrazole-3-carboxamide

trans-5-(4-bromophenyl)-1-(2,4-dichlorophenyl)-4-ethyl-N-(indolin-1-yl)-4,5-dihydro-1H-pyrazole-3-carboxamide.

Also preferred are Cycloalkane-substituted pyrazoline compounds (A) of subgroup A6.2 of general formulas IV, IVa or IVb

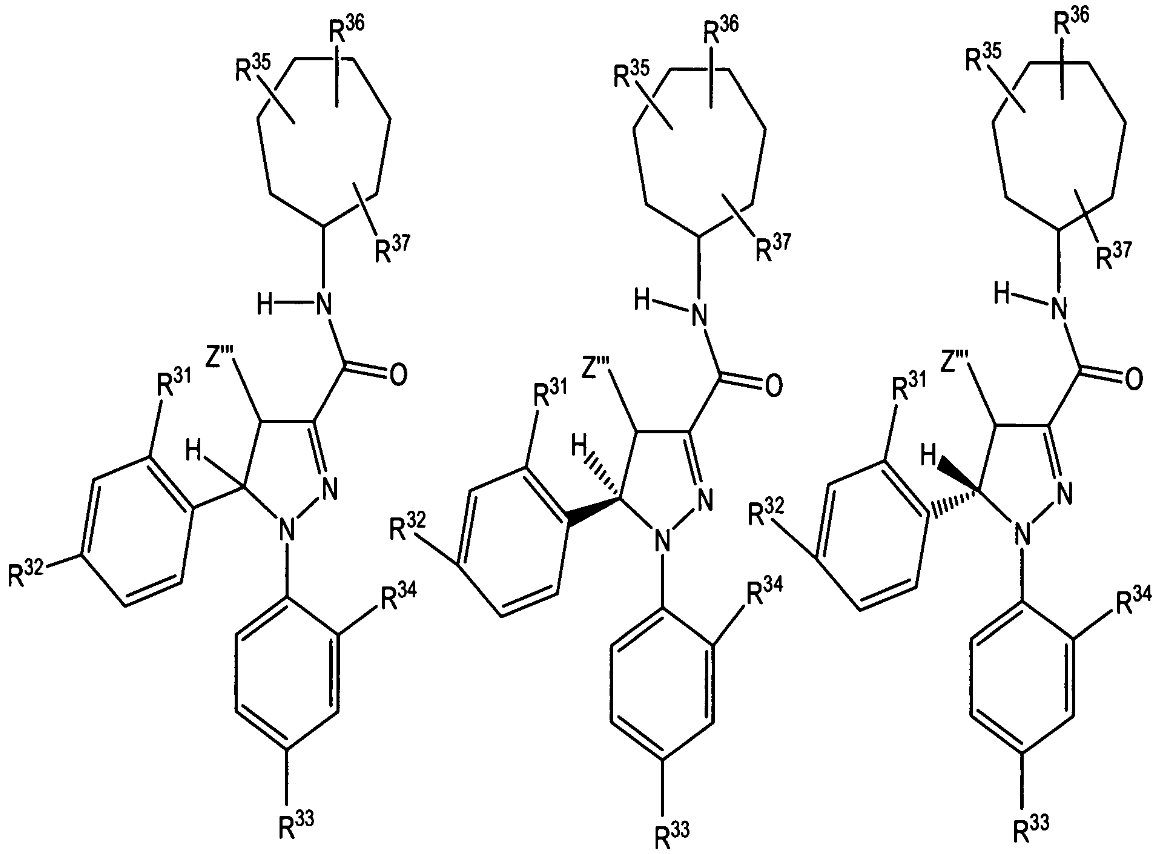

IV IVa IVb

wherein

| | |
|---|---|
| Z''' | is selected from $CH_3$ or $C_2H_5$; |
| $R^{31}$, $R^{32}$, $R^{33}$ and $R^{34}$ | independently of one another represent: H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of substituted or unsubstituted, branched or linear, saturated or unsaturated $C_{8-20}$-alkyl or C(O)-$C_{1-20}$-alkyl; substituted or unsubstituted aryl, alkyl- aryl, heterocyclyl or alkyl- heterocyclyl; substituted or unsubstituted C (O)-aryl, C (O)-alkyl- aryl, C (O)-heterocyclyl or C (O)-alkyl- heterocyclyl; |
| $R^{35}$, $R^{36}$ and $R^{37}$ | are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$; |

optionally in the form shown or in form of the acid or base or in form of a salt, especially a physiologically acceptable salt, or in form of a solvate, especially a hydrate or in form of a corresponding N-oxide thereof;

Especially preferred compounds of subgroup A6.2 are selected from

- cis-5-(4-chlorophenyl)-N-cycloheptyl-1-(2,4-dichlorophenyl)-4-methyl-4,5-dihydro-1H-pyrazole-3-carboxamide
- trans-5-(4-chlorophenyl)-N-cycloheptyl-1-(2,4-dichlorophenyl)-4-methyl-4,5-dihydro-1H-pyrazole-3-carboxamide
- (4R,5R)-5-(4-chlorophenyl)-N-cycloheptyl-1-(2,4-dichlorophenyl)-4-methyl-4,5-dihydro-1H-pyrazole-3-carboxamide
- (4S,5S)-5-(4-chlorophenyl)-N-cycloheptyl-1-(2,4-dichlorophenyl)-4-methyl-4,5-dihydro-1H-pyrazole-3-carboxamide
- cis-5-(4-bromophenyl)-N-cycloheptyl-1-(2,4-dichlorophenyl)-4-methyl-4,5-dihydro-1H-pyrazole-3-carboxamide
- trans-5-(4-bromophenyl)-N-cycloheptyl-1-(2,4-dichlorophenyl)-4-methyl-4,5-dihydro-1H-pyrazole-3-carboxamide
- cis-N-cycloheptyl-1-(2,4-dichlorophenyl)-5-(4-fluorophenyl)-4-methyl-4,5-dihydro-1H-pyrazole-3-carboxamide
- trans-N-cycloheptyl-1-(2,4-dichlorophenyl)-5-(4-fluorophenyl)-4-methyl-4,5-dihydro-1H-pyrazole-3-carboxamide
- cis-N-cycloheptyl-1-(2,4-dichlorophenyl)-5-(4-iodophenyl)-4-methyl-4,5-dihydro-1H-pyrazole-3-carboxamide
- trans-N-cycloheptyl-1-(2,4-dichlorophenyl)-5-(4-iodophenyl)-4-methyl-4,5-dihydro-1H-pyrazole-3-carboxamide
- *cis*-N-cycloheptyl-1-(2,4-dichlorophenyl)-5-(4-methoxyphenyl)-4-methyl-4,5-dihydro-1H-pyrazole-3-carboxamide
- *trans*-N-cycloheptyl-1-(2,4-dichlorophenyl)-5-(4-methoxyphenyl)-4-methyl-4,5-dihydro-1H-pyrazole-3-carboxamide
- cis-N-cycloheptyl-1-(2,4-dichlorophenyl)-5-(4-hydroxyphenyl)-4-methyl-4,5-dihydro-1H-pyrazole-3-carboxamide
- trans-N-cycloheptyl-1-(2,4-dichlorophenyl)-5-(4-hydroxyphenyl)-4-methyl-4,5-dihydro-1 H-pyrazole-3-carboxamide
- cis-5-(4-chlorophenyl)-N-cycloheptyl-1-(2,4-dichlorophenyl)-4-ethyl-4,5-dihydro-1H-pyrazole-3-carboxamide
- trans-5-(4-chlorophenyl)-N-cycloheptyl-1-(2,4-dichlorophenyl)-4-ethyl-4,5-dihydro-1H-pyrazole-3-carboxamide
- cis-5-(4-bromophenyl)-N-cycloheptyl-1-(2,4-dichlorophenyl)-4-ethyl-4,5-dihydro-1H-pyrazole-3-carboxamide
- trans-5-(4-bromophenyl)-N-cycloheptyl-1-(2,4-dichlorophenyl)-4-ethyl-4,5-dihydro-1H-pyrazole-3-carboxamide.

Also preferred are Octahydropentalene-substituted pyrazoline compounds (A) of subgroup A6.3 of general formulas IV, IVa or IVb

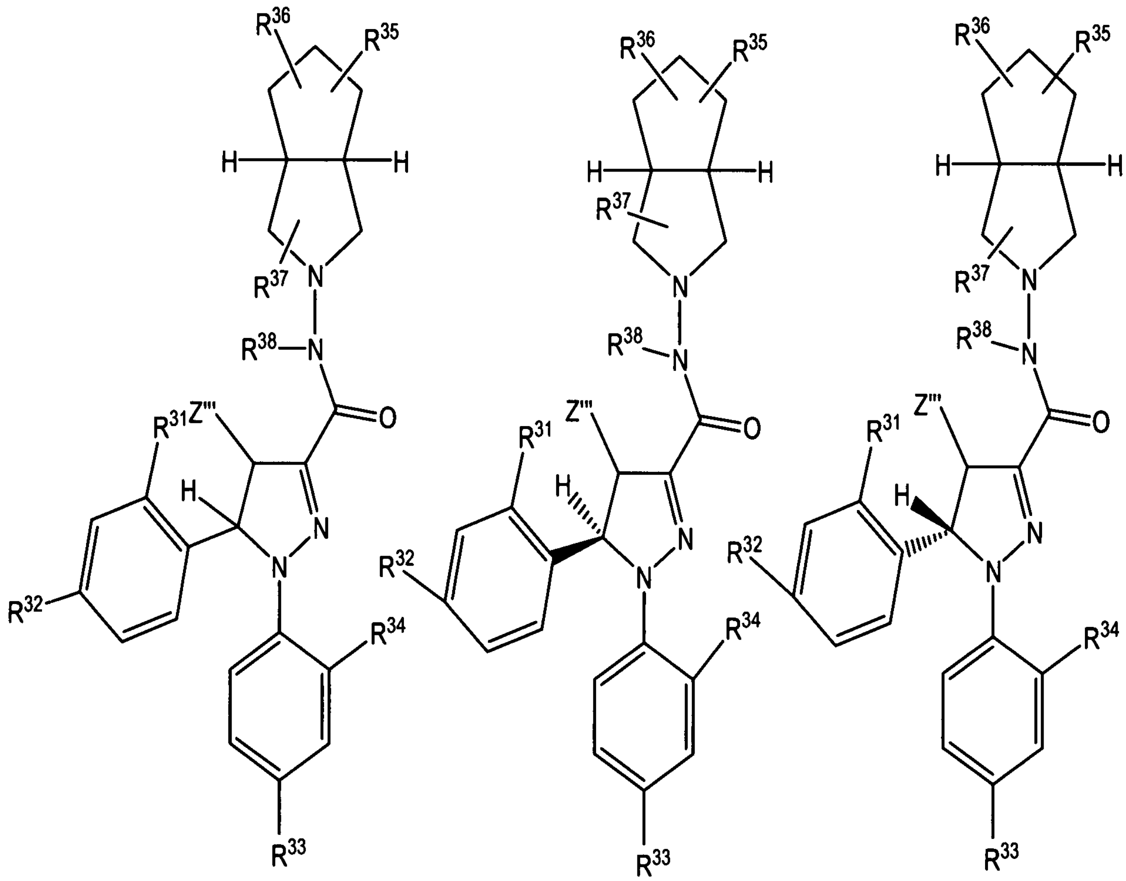

IV IVa IVb

wherein

| | |
|---|---|
| Z''' | is selected from $CH_3$ or $C_2H_5$; |
| $R^{31}$, $R^{32}$, $R^{33}$ and $R^{34}$ | independently of one another represent: H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl; |
| $R^{35}$, $R^{36}$ and $R^{37}$ | are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$; |
| $R^{38}$ | representing a hydrogen atom or a branched or linear $C_{1-3}$-alkyl group; |

optionally in the form shown or in form of the acid or base or in form of a salt, especially a physiologically acceptable salt, or in form of a solvate, especially a hydrate or in form of a corresponding N-oxide thereof;

or

Octahydropentalene-substituted pyrazoline compounds of general formulas IVc, IVd or IVe

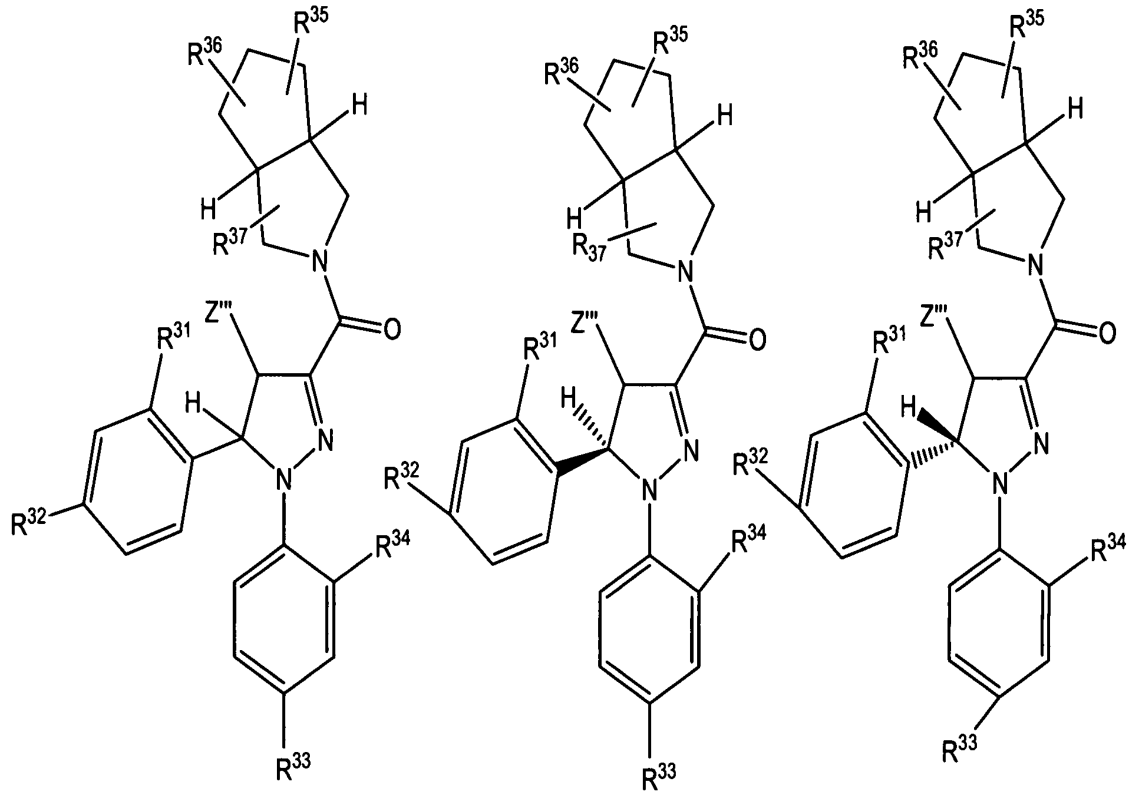

IVc IVd IVe

wherein

| | |
|---|---|
| Z''' | is selected from $CH_3$ or $C_2H_5$; |
| $R^{31}$, $R^{32}$, $R^{33}$ and $R^{34}$ | independently of one another represent: H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl; |
| $R^{35}$, $R^{36}$ and $R^{37}$ | are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$; |

optionally in the form shown or in form of the acid or base or in form of a salt, especially a physiologically acceptable salt, or in form of a solvate, especially a hydrate or in form of a corresponding N-oxide thereof.

Especially preferred compounds of subgroup A6.3 are selected from

- cis-5-(4-chlorophenyl)-1-(2,4-dichlorophenyl)-N-(hexahydrocyclopenta[c]pyrrol-2(1H)-yl)-4-methyl-4,5-dihydro-1H-pyrazole-3-carboxamide hydrochloride

- trans-5-(4-chlorophenyl)-1-(2,4-dichlorophenyl)-N-(hexahydrocyclopenta[c]pyrrol-2(1H)-yl)-4-methyl-4,5-dihydro-1 H-pyrazole-3-carboxamide hydrochloride
- cis-5-(4-bromophenyl)-1-(2,4-dichlorophenyl)-N-(hexahydrocyclopenta[c]pyrrol-2(1H)-yl)-4-methyl-4,5-dihydro-1H-pyrazole-3-carboxamide hydrochloride
- trans-5-(4-bromophenyl)-1-(2,4-dichlorophenyl)-N-(hexahydrocyclopenta[c]pyrrol-2(1H)-yl)-4-methyl-4,5-dihydro-1H-pyrazole-3-carboxamide hydrochloride
- cis-1-(2,4-dichlorophenyl)-5-(4-fluorophenyl)-N-(hexahydrocyclopenta[c]pyrrol-2(1H)-yl)-4-methyl-4,5-dihydro-1H-pyrazole-3-carboxamide hydrochloride
- trans-1-(2,4-dichlorophenyl)-5-(4-fluorophenyl)-N-(hexahydrocyclopenta[c]pyrrol-2(1H)-yl)-4-methyl-4,5-dihydro-1H-pyrazole-3-carboxamide hydrochloride
- cis-1-(2,4-dichlorophenyl)-N-(hexahydrocyclopenta[c]pyrrol-2(1H)-yl)-5-(4-methoxyphenyl)-4-methyl-4,5-dihydro-1H-pyrazole-3-carboxamide hydrochloride
- trans-1-(2,4-dichlorophenyl)-N-(hexahydrocyclopenta[c]pyrrol-2(1H)-yl)-5-(4-methoxyphenyl)-4-methyl-4,5-dihydro-1H-pyrazole-3-carboxamide hydrochloride
- cis-1-(2,4-dichlorophenyl)-N-(hexahydrocyclopenta[c]pyrrol-2(1H)-yl)-5-(4-hydroxyphenyl)-4-methyl-4,5-dihydro-1H-pyrazole-3-carboxamide hydrochloride
- trans-1-(2,4-dichlorophenyl)-N-(hexahydrocyclopenta[c]pyrrol-2(1H)-yl) 5-(4-hydroxyphenyl)-4-methyl-4,5-dihydro-1H-pyrazole-3-carboxamide hydrochloride
- cis-5-(4-chlorophenyl)-1-(2,4-dichlorophenyl)-N-(hexahydrocyclopenta[c]pyrrol-2(1H)-yl)-4-ethyl-4,5-dihydro-1H-pyrazole-3-carboxamide hydrochloride
- trans-5-(4-chlorophenyl)-1-(2,4-dichlorophenyl)-N-(hexahydrocyclopenta[c]pyrrol-2(1H)-yl)-4-ethyl-4,5-dihydro-1H-pyrazole-3-carboxamide hydrochloride
- cis-5-(4-bromophenyl)-1-(2,4-dichlorophenyl)-N-(hexahydrocyclopenta[c]pyrrol-2(1H)-yl)-4-ethyl-4,5-dihydro-1H-pyrazole-3-carboxamide hydrochloride
- trans-5-(4-bromophenyl)-1-(2,4-dichlorophenyl)-N-(hexahydrocyclopenta[c]pyrrol-2(1H)-yl)-4-ethyl-4,5-dihydro-1H-pyrazole-3-carboxamide hydrochloride.

Also preferred are Azepane-substituted pyrazoline compounds (A) of subgroup A6.4 of general formulas IV, IVa or IVb

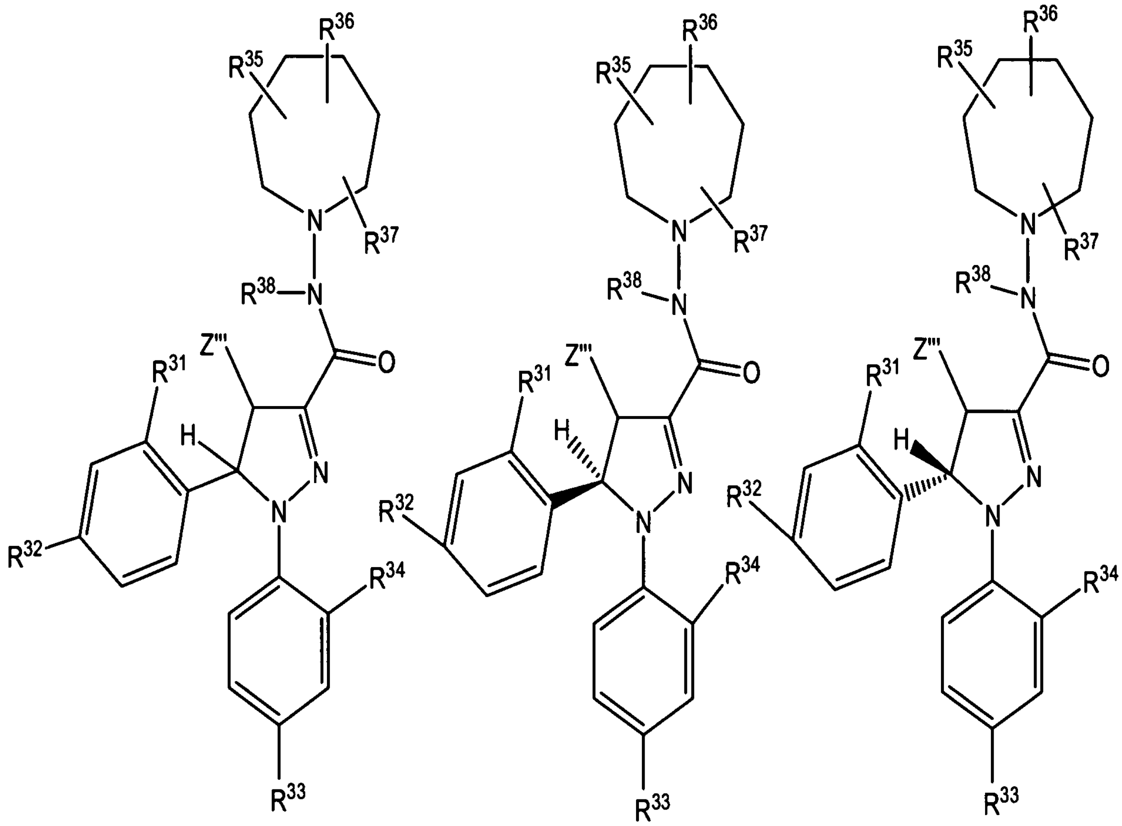

IV IVa IVb

wherein

| | |
|---|---|
| Z''' | is selected from $CH_3$ or $C_2H_5$; |
| $R^{31}$, $R^{32}$ , $R^{33}$ and $R^{34}$ | independently of one another represent: H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)$C_{1-20}$-alkyl; |
| $R^{35}$, $R^{36}$ and $R^{37}$ | are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$; |
| $R^{38}$ | representing a hydrogen atom or a branched or linear $C_{1-3}$-alkyl group; |

optionally in the form shown or in form of the acid or base or in form of a salt, especially a physiologically acceptable salt, or in form of a solvate, especially a hydrate or in form of a corresponding N-oxide thereof. or

Azepane substituted pyrazoline compounds of general formulas IVc, IVd or IVe

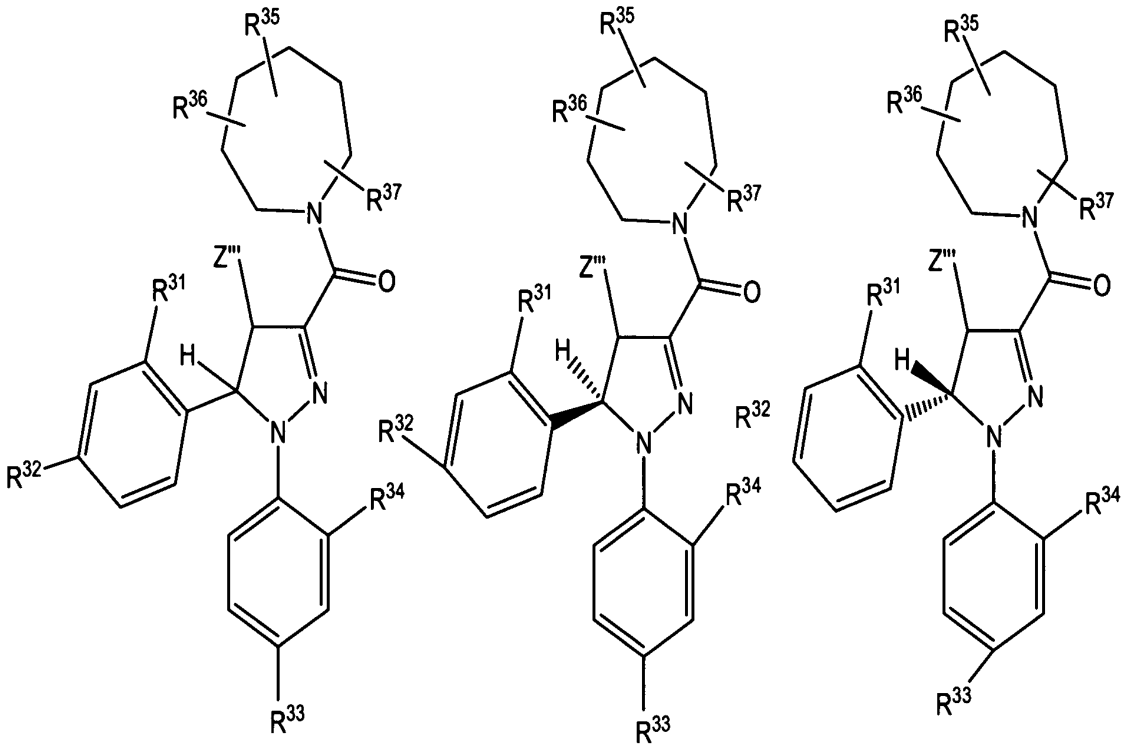

IVc IVd IVe

wherein

| | |
|---|---|
| Z''' | is selected from $CH_3$ or $C_2H_5$; |
| $R^{31}$, $R^{32}$, $R^{33}$ and $R^{34}$ | independently of one another represent: H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl; |
| $R^{35}$, $R^{36}$ and $R^{37}$ | are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$; |

optionally in the form shown or in form of the acid or base or in form of a salt, especially a physiologically acceptable salt, or in form of a solvate, especially a hydrate or in form of a corresponding N-oxide thereof.

Especially preferred compounds of subgroup A6.4 are selected from

- cis-N-(azepan-1-yl)-5-(4-chlorophenyl)-1-(2,4-dichlorophenyl)-4-methyl-4,5-dihydro-1H-pyrazole-3-carboxamide hydrochloride

- trans-N-(azepan-1-yl)-5-(4-chlorophenyl)-1-(2,4-dichlorophenyl)-4-methyl-4,5-dihydro-1H-pyrazole-3-carboxamide hydrochloride
- (4R,5R)-N-(azepan-1-yl)-5-(4-chlorophenyl)-1-(2,4-dichlorophenyl)-4-methyl-4,5-dihydro-1H-pyrazole-3-carboxamide hydrochloride
- (4S,5S)-N-(azepan-1-yl)-5-(4-chlorophenyl)-1-(2,4-dichlorophenyl)-4-methyl-4,5-dihydro-1H-pyrazole-3-carboxamide hydrochloride
- cis-N-(azepan-1-yl)-5-(4-bromophenyl)-1-(2,4-dichlorophenyl)-4-methyl-4,5-dihydro-1H-pyrazole-3-carboxamide hydrochloride
- trans-N-(azepan-1-yl)-5-(4-bromophenyl)-1-(2,4-dichlorophenyl)-4-methyl-4,5-dihydro-1H-pyrazole-3-carboxamide hydrochloride
- cis-N-(azepan-1-yl)-5-(4-fluorophenyl)-1-(2,4-dichlorophenyl)-4-methyl-4,5-dihydro-1H-pyrazole-3-carboxamide hydrochloride
- trans-N-(azepan-1-yl)-5-(4-fluorophenyl)-1-(2,4-dichlorophenyl)-4-methyl-4,5-dihydro-1H-pyrazole-3-carboxamide hydrochloride
- cis-N-(azepan-1-yl)-1-(2,4-dichlorophenyl)-5-(4-methoxyphenyl)-4-methyl-4,5-dihydro-1H-pyrazole-3-carboxamide hydrochloride
- trans-N-(azepan-1-yl)-1-(2,4-dichlorophenyl)-5-(4-methoxyphenyl)-4-methyl-4,5-dihydro-1H-pyrazole-3-carboxamide hydrochloride
- cis-N-(azepan-1-yl)-1-(2,4-dichlorophenyl)-5-(4-hydroxyphenyl)-4-methyl-4,5-dihydro-1H-pyrazole-3-carboxamide hydrochloride
- trans-N-(azepan-1-yl)-1-(2,4-dichlorophenyl)-5-(4-hydroxyphenyl)-4-methyl-4,5-dihydro-1H-pyrazole-3-carboxamide hydrochloride
- cis-N-(azepan-1-yl)-5-(4-chlorophenyl)-1-(2,4-dichlorophenyl)-4-ethyl-4,5-dihydro-1H-pyrazole-3-carboxamide hydrochloride
- trans-N-(azepan-1-yl)-5-(4-chlorophenyl)-1-(2,4-dichlorophenyl)-4-ethyl-4,5-dihydro-1H-pyrazole-3-carboxamide hydrochloride
- cis-N-(azepan-1-yl)-5-(4-bromophenyl)-1-(2,4-dichlorophenyl)-4-ethyl-4,5-dihydro-1H-pyrazole-3-carboxamide hydrochloride
- *trans*-N-(azepan-1-yl)-5-(4-bromophenyl)-1-(2,4-dichlorophenyl)-4-ethyl-4,5-dihydro-1 H-pyrazole-3-carboxamide hydrochloride
- azepan-1-yl-cis-5-(4-chlorophenyl)-1-(2,4-dichlorophenyl)-4-methyl-4,5-dihydro-1H-pyrazol-3-yl)methanone
- azepan-1-yl-trans-5-(4-chlorophenyl)-1-(2,4-dichlorophenyl)-4-methyl-4,5-dihydro-1H-pyrazol-3-yl)methanone.

If one or more of the residues $R^1$ to $R^{22}$ represents or comprises an aryl or heteroaryl radical, which may be substituted, unless defined otherwise, preferably said aryl or heteroaryl radical may optionally be substituted with 1, 2, 3, 4 or 5 substituent(s) independently selected from the group consisting of -$C_{1-6}$-perfluoralkyl, -$C_{1-6}$-alkyl substituted with one or more methoxy and/or ethoxy groups, -$C_{1-6}$-alkyl, -$C_{1-6}$-alkyl substituted with one or more hydroxy groups, -$C_{1-6}$-alkyl substituted with one or more chlorine atoms, -O-$C_{1-6}$-alkyl, -O-$C_{1-6}$-alkyl substituted with one or more methoxy and/or ethoxy groups, -S-$C_{1-6}$-alkyl, -C(=O)-OH, -C(=O)-O-$C_{1-6}$-alkyl, -O-C(=O)-$C_{1-6}$-alkyl, F, Cl, Br, I, -CN, -$OCF_3$, -O-$C_2F_5$, -O-$C_3F_7$, -O-$C_4F_9$, -$SCF_3$, -$SCF_3$, -$SCF_2H$, -$SCFH_2$, -OH, -SH, -$SO_3H$, -NH-C(=O)-$C_{1-6}$-alkyl, -N($C_{1-6}$-alkyl)-C(=O)-$C_{1-6}$-alkyl, - $NO_2$, -CHO, -C(=O)-$C_{1-6}$-alkyl, -C(=O)-$C_{1-6}$-perfluoroalkyl, -C(=S)-NH-$C_{1-6}$-alkyl, -$CF_2H$, - $CFH_2$, -C(=O)-$NR^AR^B$, -C(=O)-NH-$NR^CR^D$, -S(=O)-$C_{1-6}$-alkyl, -S(=O)$_2$-$C_{1-6}$-alkyl, -S(=O)$_2$-phenyl, -($C_{1-5}$-alkylene)-S-$C_{1-6}$-alkyl, -($C_{1-5}$-alkylene)-S(=O)-$C_{1-6}$-alkyl, -($C_{1-5}$-alkylene)S(=O)$_2$-$C_{1-6}$-alkyl, -$NR^ER^F$, -($C_{1-5}$-alkylene)-$NR^ER^F$, -S(=O)-$NH_2$, -S(=O)$_2$-NH-$C_{1-6}$-alkyl, -S(=O)$_2$-NH-phenyl, -NH-S(=O)$_2$-$C_{1-6}$-alkyl, cyclopropyl, cyclobutyl, cyclopentyl, cyclohexyl, pyrrolidinyl, piperidinyl, phenyl, thiophenyl, phenoxy and benzyl;
whereby in each case the cyclic moieties cyclopropyl, cyclobutyl, cyclopentyl, cyclohexyl, pyrrolidinyl, piperidinyl, phenyl, thiophenyl, phenoxy and benzyl can optionally be substituted with 1, 2, 3, 4 or 5 substituent(s) independently selected from the group consisting of F, Cl, Br, I, -OH, -$CF_3$, -CN, -$NO_2$, -$C_{1-6}$-alkyl, -O-$C_{1-6}$-alkyl, -O-$CF_3$ and -S-$CF_3$ and whereby $R^A$, $R^B$, $R^E$and $R^F$, independently of one another, represent hydrogen or -$C_{1-6}$-alkyl or $R^A$ and $R^B$ in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more identical or different $C_{1-6}$ alkyl radicals
and whereby $R^C$ and $R^D$, independently of one another, represent hydrogen, -$C_{1-6}$-alkyl, - C(=O)-O-$C_{1-6}$-alkyl, $C_{3-8}$-cycloalkyl, -($C_{1-5}$-alkylene)-$C_{3-8}$-cycloalkyl,-($C_{1-6}$-alkylene)-O-$C_{1-6}$-alkyl or -$C_{1-6}$-alkyl substituted with one or more hydroxy groups or $R^C$ and $R^D$ in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more substituents independently selected from the group consisting -$C_{1-6}$-alkyl, -C(=O)-$C_{1-6}$-alkyl, -C(=O)-O-$C_{1-6}$-alkyl, -C(=O)-NH-$C_{1-6}$-alkyl, -C(=S)-NH-$C_{1-6}$-alkyl, oxo (=O), -$C_{1-6}$-alkyl

substituted with one or more hydroxy groups, -($C_{1-6}$-alkylene)-O-$C_{1-6}$-alkyl and -C(=O)-$NH_2$.

More preferably said aryl and heteroaryl radicals may optionally be substituted with 1, 2, 3, 4 or 5 substituent (s) independently selected from the group consisting of -$CF_3$, -$C_2F_5$, -$C_3F_7$, - $C_4F_9$, -$CH_2Cl$, -$CHCl_2$, -$C_2H_4Cl$, methyl, ethyl, n-propyl, isopropyl, n-butyl, isobutyl, 2-butyl, tert-butyl, n-pentyl, 2-pentyl, n-hexyl, -$CH_2$-OH, -$CH_2$-$CH_2$-OH, -$CH_2$-$CH_2$-$CH_2$-OH, -O-$CH_2$-O-$CH_3$, -O-$CH_2$-$CH_2$-O-$CH_3$, -O-$CH_2$-O-$C_2H_5$, -C($OCH_3$)($C_2H_5$)$_2$, -C($OCH_3$)($CH_3$)$_2$, -O-$CH_3$, -O-$C_2H_5$, -O-$CH_2$-$CH_2$-$CH_3$, -O-CH($CH_3$)$_2$, -O-$CH_2$-$CH_2$-$CH_2$-$CH_3$, -O-C($CH_3$)$_3$, -S-$CH_3$, -S-$C_2H_5$,-S-$CH_2$-$CH_2$-$CH_3$, -S-CH($CH_3$)$_2$, -S-$CH_2$-$CH_2$-$CH_2$-$CH_3$, -S-C($CH_3$)$_3$, -C(=O)-OH, -C(=O)-O-$CH_3$, -C(=O)-O-$C_2H_5$, -C (=O)-O-$C_3H_7$, -C(=O)-O-C($CH_3$)$_3$, -O-C(=O)-$CH_3$, -O-C(=O)-$C_2H_5$, -O-C(=O)-CH($CH_3$)$_2$, -O-C(=O)-$CH_2$-$CH_2$-$CH_3$, -O-C(=O)-C($CH_3$)$_3$, F, Cl, Br, I, -CN, -$OCF_3$, -O-$C_2F_5$, -O-$C_3F_7$, -O-$C_4F_9$, -$SCF_3$, -$SCF_2H$, -$SCFH_2$, -OH, -SH, -$SO_3H$, -NH-C(=O)-$CH_3$, -NH-C(=O)-$C_2H_5$, -NH-C(=O)C($CH_3$)$_3$, -$NO_2$, -CHO, -C(=O)-$CH_3$, -C(=O)-$C_2H_5$, -C(=O)-C($CH_3$)$_3$,-C (=O)-$CF_3$, -C(=O)-$C_2F_5$, -C(=O)$C_3F_7$, -C(=S)-NH-$CH_3$, -C(=S)-NH-$C_2H_5$, -$CF_2H$, -$CFH_2$, - C(=O)-$NH_2$, -C(=O)-NH-$CH_3$, -C(=O)-NH-$C_2H_5$, -C(=O)-NH-$C_3H_7$, -C(=O)-N($CH_3$)$_2$, -C(=O)-N($C_2H_5$)$_2$, -C(=O)-NH-NH-$CH_3$, -C(=O)-NH-NH-$C_2H_5$, -C (=O)-NH-$NH_2$, -C(=O)-NH-N($CH_3$)$_2$, - S(=O)-$CH_3$, -S(=O)-$C_2H_5$, -S(=O)-$C_3H_7$, -S(=O)$_2$-$CH_3$, -S(=O)$_2$-$C_2H_5$, -S (=O)$_2$-$C_3H_7$, -S(=O)$_2$-phenyl, -$NH_2$, -NH-$CH_3$, -NH-$C_2H_5$, -N($CH_3$)$_2$, -N($C_2H_5$)$_2$, -$CH_2$-N($CH_3$)$_2$, -($CH_2$)-morpholinyl, -($CH_2$)-piperidinyl, -($CH_2$)-piperazinyl, -($CH_2$)-N($C_2H_5$)$_2$, -$CH_2$-N($C_3H_7$)$_2$, -$CH_2$-N($C_4H_9$)$_2$, -$CH_2$-N($CH_3$)($C_2H_5$), -S (=O)-$NH_2$, -S(=O)$_2$-NH-$CH_3$, -S(=O)$_2$-NH-phenyl, -NH-S(=O)$_2$-$CH_3$, cyclopropyl, cyclobutyl, cyclopentyl, cyclohexyl, pyrrolidinyl, piperidinyl, phenyl, thiophenyl, phenoxy and benzyl, whereby said thiophenyl radical can be substituted with 1, 2 or 3 substituent(s) independently selected from the group consisting of F, Cl, Br, methyl, ethyl and n-propyl.

Preferred aryl radicals which are optionally at least mono-substituted are phenyl and naphthyl (1- and 2-naphthyl).

Preferably the heteroatoms which are present as ring members in the heteroaryl radical may, unless defined otherwise, independently be selected from the group consisting of nitrogen, oxygen and sulfur. More preferably a heteroaryl radical is 5- to 14-membered and may comprise 1, 2, 3 or 4 heteroatoms independently selected from the group consisting of nitrogen, oxygen and sulfur.

Preferred heteroaryl radicals which are unsubstituted or at least mono-substituted are pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzthiazolyl, benzisoxazolyl, benzisothiazolyl, imidazo[2,1-b]thiazolyl, 2H-chromenyl, pyranyl, indazolyl and quinazolinyl.

Preferred aryl and heteroaryl radicals which are condensed with a mono- or polycyclic ring system are [1,3]-benzodioxolyl, [1,4]-benzodioxanyl, [1,2,3,4]-tetrahydronaphthyl, (2,3)-dihydro-1H-cyclopenta[b]indolyl, [1,2,3,4]-tetrahydroquinolinyl, [1,2,3,4]-tetrahydroisoquinolinyl, [1,2,3,4]-tetrahydroquinazolinyl and [3,4]-dihydro-2H-benzo[1,4]oxazinyl.

If one or more of the residues $R^1$ to $R^{22}$ represents or comprises a saturated or unsaturated, optionally at least one heteroatom as a ring member containing cycloaliphatic radical, preferably a $C_{3-18}$ cycloaliphatic radical, a heterocyclic ring, preferably a 4- to 10-membered heterocyclic ring, a $C_{3-16}$cycloalkyl radical, a $C_{4-16}$cycloalkenyl radical, a $C_{4-16}$heterocycloalkyl radical, or a $C_{5-16}$ heterocycloalkenyl radical" which may be substituted, unless defined otherwise, preferably said cycloaliphatic radical, heterocyclic ring, $C_{3-16}$ cycloalkyl radical, $C_{4-16}$ cycloalkenyl radical, $C_{4-16}$ heterocycloalkyl radical, or $C_{5-16}$ heterocycloalkenyl radical, may in each case optionally be substituted with 1, 2, 3, 4 or 5 substituent(s) independently selected from the group consisting of oxo (=O), thioxo (=S), -$C_{1-6}$-perfluoralkyl, -$C_{1-6}$-alkyl, - $C_{1-6}$-alkyl substituted with one or more hydroxy groups, -$C_{1-6}$-alkyl substituted with one or more chlorine atoms, -$C_{1-6}$-alkyl substituted with one or more methoxy and/or ethoxy groups, -O-$C_{1-6}$-alkyl, -O-$C_{1-6}$-alkyl substituted with one or more methoxy and/or ethoxy groups, -S-$C_{1-6}$-alkyl, -C(=O)-OH, -C(=O)-O-$C_{1-6}$-alkyl, -O-C(=O)-$C_{1-6}$-alkyl, F, Cl, Br, I. -CN, -$OCF_3$, -O-$C_2F_5$, -O-$C_3F_7$, -O-$C_4F_9$, -$SCF_3$, -$SCF_2H$, -$SCFH_2$, -OH, -SH, -$SO_3H$, -NH-C(=O)-$C_{1-6}$-alkyl, - N ($C_{1-6}$-alkyl)-C(=O)-$C_{1-6}$-alkyl, -$NO_2$, -CHO, -C(=O)-$C_{1-6}$-alkyl, -C(=O)-$C_{1-6}$-perfluoroalkyl, - C(=S)-NH-$C_{1-6}$-alkyl, -$CF_2H$, -$CFH_2$, -C(=O)-$NR^AR^B$, -C(=O)-NH-$NR^CR^D$, -S(=O)-$C_{1-6}$-alkyl, - S(=O)$_2$-$C_{1-6}$-alkyl, -S(=O)$_2$-phenyl, -($C_{1-5}$-alkylene)-S-$C_{1-6}$-alkyl, -($C_{1-5}$-alkylene)-S(=O)$C_{1-6}$-alkyl, -($C_{1-5}$-alkylene)-S(=O)$_2$-$C_{1-6}$-alkyl, -$NR^ER^F$, -($C_{1-5}$-alkylene)-$NR^ER^F$, -S (=O)$NH_2$, - S(=O)$_2$-NH-$C_{1-6}$-alkyl, -S(=O)$_2$-NH-phenyl, -NH-S(=O)$_2$-$C_{1-6}$-alkyl, -O-Benzyl, -O-Phenyl, cyclopropyl, cyclobutyl, cyclopentyl, cyclohexyl, pyrrolidinyl, piperidinyl, phenyl, thiophenyl, phenoxy and benzyl; whereby in each case the cyclic moieties cyclopropyl, cyclobutyl, cyclopentyl, cyclohexyl, pyrrolidinyl, piperidinyl, phenyl, thiophenyl, phenoxy and benzyl can optionally be substituted with 1, 2, 3, 4 or 5 substituent(s) independently selected from the group consisting of F, Cl, Br, I, -OH, -$CF_3$, -CN, -$NO_2$, -$C_{1-6}$-alkyl, -O-$C_{1-6}$-alkyl, -O-$CF_3$ and -S-$CF_3$ and

whereby $R^A$, $R^B$, $R^E$and $R^F$, independently of one another, represent hydrogen or -$C_{1-6}$-alkyl or $R^A$ and $R^B$ in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more identical or different $C_{1-6}$ alkyl radicals

and whereby $R^C$ and $R^D$, independently of one another, represent hydrogen, -$C_{1-6}$-alkyl, - C(=O)-O-$C_{1-6}$-alkyl, $C_{3-8}$-cycloalkyl, -($C_{1-5}$-alkylene)-$C_{3-8}$-cycloalkyl, -($C_{1-6}$-alkylene)-O-$C_{1-6}$-alkyl or -$C_{1-6}$-alkyl substituted with one or more hydroxy

groups or $R^C$ and $R^D$ in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more substituents independently selected from the group consisting $-C_{1-6}$-alkyl, $-C(=O)-C_{1-6}$-alkyl, $-C(=O)-O-C_{1-6}$-alkyl, $-C(=O)-NH-C_{1-6}$-alkyl, $-C(=S)-NH-C_{1-6}$-alkyl, oxo (=O), $-C_{1-6}$-alkyl substituted with one or more hydroxy groups, $-(C_{1-6}$-alkylene$)-O-C_{1-6}$-alkyl and $-C(=O)-NH_2$.

More preferably said cycloaliphatic radicals, heterocyclic rings, $C_{3-16}$ cycloalkyl radicals, $C_{4-16}$ cycloalkenyl radicals, $C_{4-16}$ heterocycloalkyl radicals, or $C_{5-16}$ heterocycloalkenyl radicals, may optionally be substituted with 1, 2, 3, 4 or 5 substituent(s) independently selected from the group consisting of oxo (=O), thioxo (=S), $-CF_3$, $-C_2F_5$, $-C_3F_7$, $-C_4F_9$, $-CH_2Cl$, $-CHCl_2$, $-C_2H_4Cl$, methyl, ethyl, n-propyl, isopropyl, n-butyl, isobutyl, 2-butyl, tert-butyl, n-pentyl, 2-pentyl, n-hexyl, $-CH_2-OH$, $-CH_2-CH_2-OH$, $-CH_2-CH_2-CH_2-OH$, $-O-CH_2-O-CH_3$, $-O-CH_2-CH_2-O-CH_3$, $-O-CH_2-O-C_2H_5$, $-C(OCH_3)(C_2H_5)_2$, $-C(OCH_3)(CH_3)_2$, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, $-C(=O)-OH$, $-C(=O)-O-CH_3$, $-C(=O)-O-C_2H_5$, $-C(=O)-O-C_3H_7$, $-C(=O)O-C(CH_3)_3$, $-O-C(=O)-CH_3$, $-O-C(=O)-C_2H_5$, $-O-C(=O)-CH(CH_3)_2$, $-O-C(=O)CH_2-CH_2-CH_3$, $-O-C(=O)-C(CH_3)_3$, F, Cl, Br, I, -CN, $-OCF_3$, $-O-C_2F_5$, $-O-C_3F_7$, $-O-C_4F_9$, $-SCF_3$, $-SCF_2H$, $-SCFH_2$, -OH, -SH, $-SO_3H$, $-NH-C(=O)-CH_3$, $-NH-C(=O)-C_2H_5$, $-NH-C(=O)-C(CH_3)_3$, $-NO_2$, -CHO, $-C(=O)CH_3$, $-C(=OYC_2H_5$, $-C(=O)-C(CH_3)_3$, $-C(=O)-CF_3$, $-C(=O)C_2F_5$, $-C(=O)-C_3F_7$, $-C(=S)-NH-CH_3$, $-C(=S)-NH-C_2H_5$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-C(=O)-NH-NH-CH_3$, $-C(=O)-NH-NH-C_2H_5$, $-C(=O)-NH-NH_2$, $-C(=O)-NH-N(CH_3)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-S(=O)_2$-phenyl, $-NH_2$, $-NH-CH_3$, $-NH-C_2H_5$, $-N(CH_3)_2$, $-N(C_2H_5)_2$, $-CH_2-N(CH_3)_2$, $-(CH_2)$-morpholinyl, $-(CH_2)$-piperidinyl, $-(CH_2)$-piperazinyl, $-(CH_2)-N(C_2H_5)_2$, $-CH_2-N(C_3H_7)_2$, $-CH_2-N(C_4H_9)_2$, $-CH_2-N(CH_3)(C_2H_5)$, $-S(=O)-NH_2$, $-S(=O)_2-NH-CH_3$, $-S(=O)_2-NH$-phenyl, $-NH-S(=O)_2-CH_3$, -O-Benzyl, -O-Phenyl, cyclopropyl, cyclobutyl, cyclopentyl, cyclohexyl, pyrrolidinyl, piperidinyl, phenyl, thiophenyl, phenoxy and benzyl, whereby said thiophenyl radical can be substituted with 1, 2 or 3 substituents independently selected from the group consisting of F, Cl, Br, methyl, ethyl and n-propyl.

If one or more of the residues $R^1$ to $R^{22}$ represents or comprises a cycloaliphatic radical, preferably a $C_{3-16}$ cycloaliphatic radical, which contains one or more heteroatoms as ring members, unless defined otherwise, each of these heteroatoms may preferably be selected from the group consisting of nitrogen, oxygen and sulfur. More preferably a cycloaliphatic group may optionally contain 1, 2, 3 or 4 heteroatom(s) independently selected from the group consisting of N, O and S as ring members.

Suitable saturated or unsaturated, optionally at least one heteroatom as ring member containing cycloaliphatic radicals may preferably be selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1 H-pyrrolyl, (2,3)-dihydro-1 H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl,(1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, oxazolidinyl, (1,3)-dioxanyl, (1,4)-dioxanyl and (1,3)-dioxolanyl.

Suitable saturated or unsaturated, optionally at least one heteroatom as ring member containing cycloaliphatic radicals which are condensed with an unsubstituted or at least mono-substituted mono- or polycyclic ring system may preferably be selected from the group consisting of indolinyl, isoindolinyl, decahydronaphthyl, (1,2,3,4)-tetrahydroquinolinyl, (1,2,3,4)-tetrahydroisoquinolinyl, (1,2,3,4)-tetrahydronaphthyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1H-benzo[de]isoquinolinyl and (1,2,3,4)-tetrahydroquinoxazlinyl.

Preferably a cycloaliphatic radical, a $C_{3-16}$ cycloalkyl radical, a $C_{4-16}$ cycloalkenyl radical, a $C_{4-16}$ heterocycloalkyl radical or a $C_{5-16}$ heterocycloalkenyl radical may be bridged by 1, 2 or 3 unsubstituted or at least mono-substituted alkylene group(s).

Suitable saturated or unsaturated, optionally at least one heteroatom as ring member containing cycloaliphatic radicals which are bridged by at least one unsubstituted or at least mono-substituted alkylene group may preferably be selected from the group consisting of adamantyl, bicyclo[2.2.1]heptyl, bicyclo[3.1.1]heptyl, norbornenyl and 8-azabicyclo [3.2.1]octyl.

A suitable saturated or unsaturated, optionally at least one heteroatom as ring member containing cycloaliphatic radical which together with a saturated or unsaturated, unsubstituted or at least mono-substituted cycloaliphatic radical forms a spirocyclic residue via a common ring atom is 8-aza-spiro[4.5]decanyl.

A mono- or polycyclic ring system according to the present invention - if not defined otherwise - means a mono- or polycyclic hydrocarbon ring system, preferably a mono- or bicyclic ring system, that may be saturated, unsaturated or aromatic. Each of its different rings may show a different degree of saturation, i.e. they may be saturated, unsaturated

or aromatic. Optionally each of the rings of the mono- or bicyclic ring system may contain one or more, preferably 1, 2 or 3, heteroatom(s) as ring member(s), which may be identical or different and which can preferably be selected from the group consisting of nitrogen, oxygen and sulfur. The rings of the mono- or bicyclic ring system are preferably 5-, 6- or 7-membered.

The term "condensed" according to the present invention means that a ring or ring system is attached to another ring or ring system, whereby the terms "annulated" or "annelated" are also used by those skilled in the art to designate this kind of attachment.

If one or more of the residues $R^1$ to $R^{22}$ comprises a mono- or polycyclic ring system, which may be substituted, unless defined otherwise, preferably said mono- or polycyclic ring system may optionally be substituted with 1, 2, 3, 4 or 5 substituent(s) independently selected from the group consisting of oxo (=O), thioxo (=S), $-C_{1-6}$-perfluoralkyl, $-C_{1-6}$-alkyl, - $C_{1-6}$-alkyl substituted with one or more hydroxy groups, $-C_{1-6}$-alkyl substituted with one or more chlorine atoms, $-C_{1-6}$-alkyl substituted with one or more methoxy and/or ethoxy groups, $-O-C_{1-6}$-alkyl, $-O-C_{1-6}$-alkyl substituted with one or more methoxy and/or ethoxy groups, $-S-C_1$-6-alkyl,- C(=O)-OH, $-C(=O)-O-C_{1-6}$-alkyl, $-O-C(=O)-C_{1-6}$-alkyl, F, Cl, Br, I, -CN, $-OCF_3$, $-O-C_2F_5$, $-O-C_3F_7$, $-O-C_4F_9$, $-SCF_3$, $-SCF_2H$, $-SCFH_2$, -OH, -SH, $-SO_3H$, $-NH-C(=O)-C_{1-6}$-alkyl, - $N(C_{1-6}$-alkyl)-$C(=O)-C_{1-6}$-alkyl, $-NO_2$, -CHO, $-C(=O)-C_{1-6}$-alkyl, $-C(=O)-C_{1-6}$-perfluoroalkyl, - $C(=S)-NH-C_{1-6}$-alkyl, $-CF_2H$, $-CFH_2$, $-C(=O)-NR^AR^B$, $-C(=O)-NH-NR^CR^D$, $-S(=O)-C_{1-6}$-alkyl, - $S(=O)_2-C_{1-6}$-alkyl, $-S(=O)_2$-phenyl, $-(C_{1-5}$-alkylene)-$S-C_{1-6}$-alkyl, $-(C_{1-5}$-alkylene)-$S(=O)-C_{1-6}$-alkyl, $-(C_{1-5}$-alkyleneyl)$(=O)_2-C_{1-6}$-alkyl, $-NR^ER^F$, $-(C_{1-5}$-alkylene)-$NR^ER^F$, $-S(=O)-NH_2$, - $S(=O)_2-NH-C_{1-6}$-alkyl, $-S(=O)_2$-NH-phenyl, $-NH-S(=O)_2-C_{1-6}$-alkyl, cyclopropyl, cyclobutyl, cyclopentyl, cyclohexyl, pyrrolidinyl, piperidinyl, phenyl, thiophenyl, phenoxy and benzyl;
whereby in each case the cyclic moieties cyclopropyl, cyclobutyl, cyclopentyl, cyclohexyl, pyrrolidinyl, piperidinyl, phenyl, thiophenyl, phenoxy and benzyl can optionally be substituted with 1, 2, 3, 4 or 5 substituent(s) independently selected from the group consisting of F, Cl, Br, I, -OH, $-CF_3$, -CN, $-NO_2$, $-C_{1-6}$-alkyl, $-O-C_{1-6}$-alkyl, $-O-CF_3$ and $-S-CF_3$ and
whereby $R^A$, $R^B$, $R^E$ and $R^F$, independently of one another, represent hydrogen or $-C_{1-6}$-alkyl or $R^A$ and $R^B$ in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more identical or different $C_{1-6}$ alkyl radicals
and whereby $R^C$ and $R^D$, independently of one another, represent hydrogen, $-C_{1-6}$-alkyl, - $C(=O)-O-C_{1-6}$-alkyl, $C_{3-8}$-cycloalkyl, $-(C_{1-5}$-alkylene)-$C_{3-8}$-cycloalkyl, $-(C_{1-6}$-alkylene)-$O-C_{1-6}$-alkyl or $-C_{1-6}$-alkyl substituted with one or more hydroxy groups or $R^C$ and $R^D$ in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more substituents independently selected from the group consisting $-C_{1-6}$-alkyl, $-C(=O)-C_{1-6}$-alkyl, $-C(=O)-O-C_{1-6}$-alkyl, $-C(=O)-NH-C_{1-6}$-alkyl, $-C(=S)-NH-C_{1-6}$-alkyl, oxo (=O), $-C_{1-6}$-alkyl substituted with one or more hydroxy groups, $-(C_{1-6}$-alkylene)-$O-C_{1-6}$-alkyl and $-C(=O)-NH_2$.

More preferably said mono- or polycyclic ring system may optionally be substituted with 1, 2, 3, 4 or 5 substituent (s) independently selected from the group consisting of oxo (=0), thioxo (=S), $-CF_3$, $-C_2F_5$, $-C_3F_7$, $-C_4F_9$, $-CH_2Cl$, $-CHCl_2$, $-C_2H_4Cl$, methyl, ethyl, n-propyl, isopropyl, n-butyl, isobutyl, 2-butyl, tert-butyl, n-pentyl, 2-pentyl, n-hexyl, $-CH_2-OH$, $-CH_2-CH_2-OH$, $-CH_2-CH_2-CH_2-OH$, $-O-CH_2-O-CH_3$, $-O-CH_2-CH_2-O-CH_3$, $-O-CH_2-O-C_2H_5$, $-C(OCH_3)(C_2H_5)_2$, - $C(OCH_3)(CH_3)_2$, $-O-CH_3$, $-O-C_2H_5$, $-O-CH_2-CH_2-CH_3$, $-O-CH(CH_3)_2$, $-O-CH_2-CH_2-CH_2-CH_3$,$-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$,- C(=O)-OH, $-C(=O)-O-CH_3$, $-C(=O)-O-C_2H_5$, $-C(=O)O-C_3H_7$, $-C(=O)O-C(CH_3)_3$, $-O-C(=O)-CH_3$, $-O-C(=O)C_2H_5$, $-O-C(=O)-CH(CH_3)_2$, $-O-C(=O)-CH_2-CH_2-CH_3$, $-O-C(=O)-C(CH_3)_3$, F, Cl, Br, I, -CN, $-OCF_3$, $-O-C_2F_5$, $-O-C_3F_7$, $-O-C_4F_9$, $-SCF_3$, $-SCF_2H$, $-SCFH_2$, -OH, -SH, $-SO_3H$, $-NH-C(=O)-CH_3$, $-NH-C(=O)-C_2H_5$, $-NH-C(=O)-C(CH_3)_3$, $-NO_2$, -CHO, $-C(=O)-CH_3$, $-C(=O)-C_2H_5$, $-C(=O)C(CH_3)_3$, $-C(=O)-CF_3$, $-C(=O)-C_2F_5$, $-C(=O)-C_3F_7$, $-C(=S)-NH-CH_3$, $-C(=S)-NH-C_2H_5$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-C(=O)-NH-NH-CH_3$, $-C(=O)-NH-NH-C_2H_5$, $-C(=O)-NH-NH_2$, $-C(=O)NH-N(CH_3)_2$, $-S(=O)CH_3$, $-S(=O)C_2H_5$, $-S(=O)C_3H_7$, $-S(=O)_2-CH_3$, $-S(=O)_2-C_2H_5$, $-S(=O)-C_3H_7$, $-S(=O)_2$-phenyl, $-NH_2$, $-NH-CH_3$, $-NH-C_2H_5$, $-N(CH_3)_2$, $-N(C_2H_5)_2$, $-CH_2-N(CH_3)_2$, $-(CH_2)$-morpholinyl, $-(CH_2)$-piperidinyl, $-(CH_2)$-piperazinyl, $-(CH_2)-N(C_2H_5)_2$, $-CH_2-N(C_3H_7)_2$, $-CH_2-N(C_4H_9)_2$, $-CH_2-N(CH_3)(C_2H_5)$, $-S(=O)NH_2$, $-S(=O)_2-NH-CH_3$, $-S(=O)_2$-NH-phenyl, $-NH-S(=O)_2-CH_3$, cyclopropyl, cyclobutyl, cyclopentyl, cyclohexyl, pyrrolidinyl, piperidinyl, phenyl, thiophenyl, phenoxy and benzyl, whereby said thiophenyl radical can be substituted with 1, 2 or 3 substituents independently selected from the group consisting of F, Cl, Br, methyl, ethyl and n-propyl.

If one or more of the residues $R^4$ to $R^{22}$ represent or comprise a saturated or unsaturated, unsubstituted or at least mono-substituted aliphatic radical, preferably a $C_{1-16}$ aliphatic radical, said aliphatic radical may be linear or branched.

Preferably aliphatic radicals, $C_{1-16}$ alkyl radicals, $C_{2-16}$ alkenyl radical and $C_{2-16}$ alkinyl radicals, unless defined otherwise, may optionally be substituted with 1, 2, 3, 4, 5, 6, 7, 8 or 9 substituents independently selected from the group consisting of -OH, F, Cl, Br, I, $-O-C_{1-6}$ alkyl, $-OCF_3$, $-O-C_2F_5$, $-O-C_3F_7$, $-O-C_4F_9$, $-CF_3$, $-C_2F_5$, $-C_3F_7$, $-C_4F_9$, $-NH_2$, $-NH-C_{1-6}$-alkyl, - $N(C_{1-6}$-alkyl$)_2$, -C(=O)-OH. $-C(=O)-O-C_{1-6}$-alkyl, $-C(=O)-NH_2$, $-C(=O)-NH-C_{1-6}$-alkyl, $-C(=O)-N(C_{1-6}$-alkyl$)_2$, -CN, $-NO_2$, $-S(=O)-NH_2$, -CHO, $-C(=O)-C_{1-6}$-alkyl, $-S(=O)-C_{1-6}$-alkyl, $-S(=O)_2-C_{1-6}$-alkyl, $-NH-S(=O)-C_{1-6}$-alkyl, -NH-C

(=O)-O-$C_{1-6}$-alkyl and -NH-C(=O)-$C_{1-6}$-alkyl.

More preferably aliphatic radicals, $C_{1-16}$ alkyl radicals, $C_{2-16}$ alkenyl radical and $C_{2-16}$ alkinyl radicals may optionally be substituted with 1, 2, 3, 4, 5, 6, 7, 8 or 9 substituents independently selected from the group consisting of -OH, F, Cl, Br, I, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -O-$C(CH_3)_3$, -$NH_2$, -NH-$CH_3$, -NH-$C_2H_5$, - $N(CH_3)_2$, -$N(C_2H_5)_2$, -CN, -$NO_2$, -NH-C(=O)-$CH_3$, -NH-C(=O)-$C_2H_5$, -NH-C(=O)-$C(CH_3)_3$, -NH-C(=O)-O-$CH_3$, -NH-C(=O)-O-$C_2H_5$, -NH-C(=O)-O-$C(CH_3)_3$, -C(=O)-NH-$CH_3$, -C(=O)-NH-$C_2H_5$, -C(=O)-NH-$C(CH_3)_3$, -C(=O)-N$(CH_3)_2$, -C(=O)-$N(C_2H_5)_2$, -C(=O)-OH, -C(=O)-O-$CH_3$, -C(=O)-O-$C_2H_5$, -C(=O)-O-$C(CH_3)_3$, -C(=O)-$CH_3$, -C(=O)-$C_2H_5$ and -C(=O)-$C(CH_3)_3$.

Suitable alkyl radicals, preferably $C_{1-16}$alkyl radicals, are selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl,2-(6-methyl)-octyl, n-nonyl, n-decyl, n-undecyl, n-dodecyl, n-tridecyl, n-tetradecyl, n-pentadecycl and n-hexadecyl.

Suitable at least mono-substituted alkyl radicals are selected from the group consisting of - $CF_3$, -$CH_2F$, -$CF_2H$, -$CH_2$-O-$CH_3$, -$C_2F_5$, -$CH_2$-$CH_2$-F, -$CH_2$-CN, -$CH_2$-OH, -$CH_2$-$CH_2$-CN, - $CH_2$-$CH_2$-OH, -$CH_2$-$CH_2$-$OCH_3$, -$CH_2$-$CH_2$-$CH_2$-CN, -$CH_2$-$CH_2$-$CH_2$-OH, -$CH_2$-$CH_2$-$CH_2$-O-$CH_3$, -$CH_2$-$CH_2$-$CH_2$-$CH_2$-O-$CH_3$, -$CH_2$-$NH_2$, -$CH_2$-$N(CH_3)_2$, -$CH_2$-N$(C_2H_5)_2$, -$CH_2$-CH-$NH_2$, - $CH_2$-$CH_2$-$N(CH_3)_2$, -$CH_2$-$CH_2$-$N(C_2H_5)_2$, -$CH_2$-$CH_2$-$CH_2$-$NH_2$, -$CH_2$-$CH_2$-$CH_2$-$N(CH_3)_2$ and -$CH_2$-$CH_2$-$CH_2$-$N(C_2H_5)_2$.

An alkenyl radical according to the present invention comprises at least one carbon-carbon double bond. Suitable alkenyl radicals, preferably $C_{2-16}$ alkenyl radicals, are selected from the group consisting of vinyl, n-propenyl, n-butenyl, n-pentenyl, n-hexenyl, n-heptenyl, n-octenyl, n-nonenyl, n-decenyl, n-undecenyl, n-dodecenyl, n-tridecenyl, n-tetradecenyl, n-pentadecenyl and n-hexadecenyl.

An alkinyl radical comprises at least one carbon-carbon triple bond. Suitable alkinyl radicals, preferably $C_{2-16}$alkinyl radicals, are selected from the group consisting of ethinyl, propinyl, n-butinyl, n-pentinyl, n-hexinyl, n-octinyl, n-noninyl, n-decinyl, n-undecinyl, n-dodecinyl, n-tridecinyl, n-tetradecinyl, n-pentadecinyl and n-hexadecinyl.

If any of the substituents represents an alkylene group, an alkenylene group or an alkinylene group, which may be substituted, said alkylene group, alkenylene group or alkinylene group may - if not defined otherwise - be unsubstituted or substituted by one or more substituents, preferably unsubstituted or substituted with 1, 2 or 3 substituent(s). Said substituent(s) may preferably be selected independently from the group consisting of -O-$C_{1-6}$-alkyl, -S-$C_{1-6}$-alkyl, -F, Cl, Br, I, -CN, -$CF_3$, -$OCF_3$, -$SCF_3$, -OH, -SH, -$SO_3H$, -$NH_2$, -NH($C_{1-6}$-alkyl), -N($C_{1-6}$-alkyl$)_2$ and phenyl. More preferably said substituent(s) may be selected from the group consisting of -F, Cl, Br, I, -CN, -$CF_3$, -$OCF_3$, -$SCF_3$, -OH, -SH, -$SO_3H$, -$NH_2$, -NH-$CH_3$, -$N(CH_3)_2$, -O-$CH_3$ and -O-$C_2H_5$. An alkenylene group comprises at least one carbon-carbon double bond, an alkinylene group comprises at least one carbon-carbon triple bond.

Suitable alkylene groups, preferably $C_{1-5}$-alkylene groups, include -$(CH_2)$- -$CH(CH_3)$-, - CH(phenyl), -$(CH_2)_2$-, -$(CH_2)_3$-,-$(CH_2)_4$-,-$(CH_2)_5$ and -$(CH_2)_6$-, suitable alkenylene groups, preferably $C_{2-5}$-alkenylene groups, include -CH=CH-, -$CH_2$-CH=CH- and -CH=CH-$CH_2$- and suitable alkinylene groups, preferably $C_{2-5}$-alkinylene groups, include -C≡C-, -$CH_2$-C≡C- and -C≡C-$CH_2$-.

The substituted pyrazoline compounds of general formula I given above, wherein $R^5$ represents hydrogen, may be prepared by a process wherein at least one compound of general formula II,

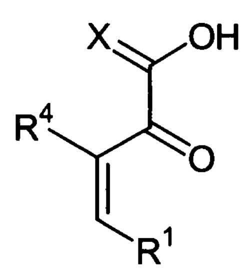

II,

wherein $R^1$, X and $R^4$ have the meaning given above, is reacted with at least one compound of general formula III,

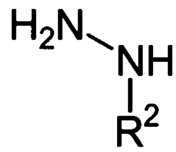

III,

or a corresponding salt thereof, wherein $R^2$ has the meaning given above, in a reaction medium, optionally in an inert atmosphere, optionally in the presence of at least one acid, to yield at least one compound of general formula IV,

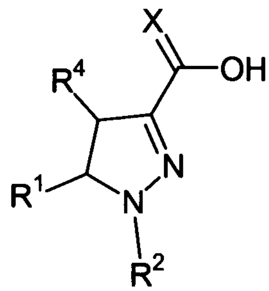

IV,

wherein $R^1$, X, $R^2$ and $R^4$ have the meaning given above, which is optionally isolated and/or purified, and at least one compound of general formula IV is reacted with an activating agent in a reaction medium, optionally in an inert atmosphere, to yield at least one compound of general formula V,

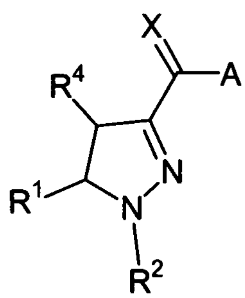

V,

wherein $R^1$, X, $R^2$ and $R^4$ have the meaning according given above and A represents a leaving group, which is optionally purified and/or isolated,
and at least one compound of general formula V is reacted with at least one compound of general formula $R^3$-H, wherein $R^3$ has the meaning given above, in a reaction medium, optionally in an inert atmosphere, optionally in the presence of at least one base selected from the group consisting of diisopropylethylamine, triethylamine, pyridine, dimethylaminopyridine and N-methylmorpholine, to yield at least one compound of general formula I, wherein $R^1$, $R^2$, X, $R^3$ and $R^4$ have the meaning given above and $R^5$ represents hydrogen, which is optionally purified and/or isolated;
or at least one compound of general formula IV is reacted with at least one compound of general formula $R^3$-H, wherein $R^3$ represents a -$NR^7R^8$ moiety, whereby $R^7$ and $R^8$ have the meaning given above, in a reaction medium, in the presence of at least one coupling agent, optionally in the presence of at least one base, to yield at least one compound of general formula I, wherein $R^1$, $R^2$, X and $R^4$ have the meaning given above, $R^3$ represents a -$NR^7R^8$ moiety and $R^5$ represents hydrogen, which is optionally purified and/or isolated.

Also described is the process for the preparation of a compound of general formula I given above, wherein $R^5$ represents a hydrogen atom, according to which
at least one compound of general formula $R^1$-C(=O)-H (general formula VII), wherein $R^1$ has the meaning given above,

is reacted with at least one compound of general formula VI,

VI,

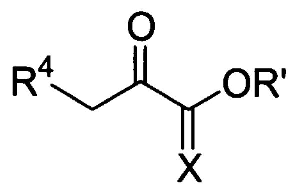

wherein $R^4$ and X have the meaning given above and R' represents a linear or branched $C_{1-6}$-alkyl radical, preferably an ethyl radical, a potassium cation or a sodium cation, in a reaction medium, optionally in an inert atmosphere, optionally in the presence of at least one base, to yield at least one compound of general formula II,

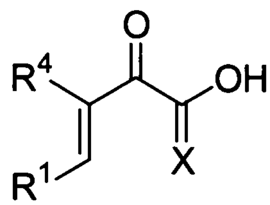

II,

wherein $R^1$, X and $R^4$ have the meaning given above, which is optionally purified and/or isolated,
and at least one compound of general formula II is reacted with an activating agent in a reaction medium, optionally in an inert atmosphere, to yield at least one compound of general formula VIII,

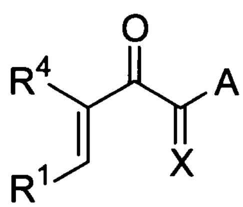

VIII,

wherein $R^1$, X and $R^4$ have the meaning given above and A represents a leaving group, which is optionally purified and/or isolated,
and at least one compound of general formula VIII is reacted with at least one compound of general formula $R^3$-H, wherein $R^3$ has the meaning given above, in a reaction medium, optionally in an inert atmosphere, optionally in the presence of at least one base selected from the group consisting of diisopropylethylamine, triethylamine, pyridine, dimethylaminopyridine and N-methylmorpholine, to yield at least one compound of general formula IX,

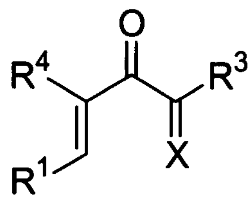

IX,

wherein $R^1$, X, $R^3$ and $R^4$ have the meaning given above, which is optionally purified and/or isolated;
or at least one compound of general formula II is reacted with at least one compound of general formula $R^3$-H, wherein $R^3$ represents a -$NR^7R^8$ moiety, whereby $R^7$ and $R^8$ have the meaning given above, in a reaction medium, in the presence of at least one coupling agent, optionally in the presence of at least one base, to yield at least one compound of general formula IX, wherein $R^3$ represents a -$NR^7R^8$ moiety, which is optionally purified and/or isolated,
and at least one compound of general formula IX is reacted with at least one compound of general formula III,

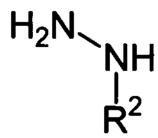

III,

wherein $R^2$ has the meaning given above, in a reaction medium, optionally in an inert atmosphere, optionally in the presence of at least one acid, to yield a compound of general formula I, wherein $R^1$, X, $R^2$, $R^3$ and $R^4$ have the meaning given above and $R^5$ represents hydrogen, which is optionally purified and/or isolated.

The process is also illustrated in scheme I given below:

Scheme I.

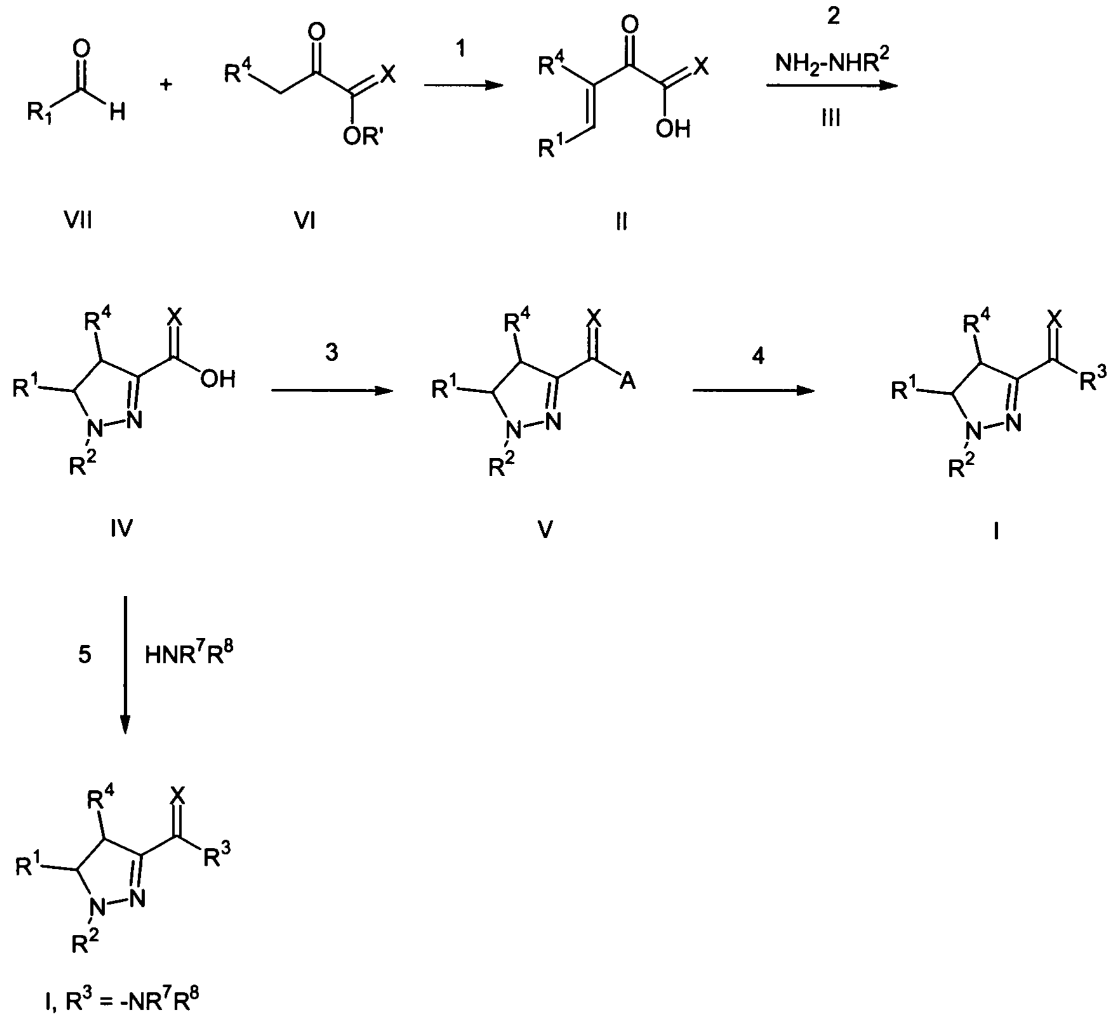


VII VI II

IV V I

I, $R^3$ = $-NR^7R^8$

In step 1 a compound of general formula VI is reacted with a compound of general formula VII in a protic reaction medium, preferably in a reaction medium selected from the group consisting of methanol, ethanol, isopropanol, n-butanol, water and mixtures thereof, in the presence of at least one base, preferably in the presence of an alkali metal hydroxide such as sodium hydroxide or potassium hydroxide or an alkali metal methoxide such as sodium methoxide, as described, for example, in Synthetic Communications, 26(11), 2229-33, (1996). The respective description is hereby incorporated by reference and forms part of the disclosure. Reaction temperature as well as the duration of the reaction may vary over a broad range. Preferred reaction temperatures range from -10 °C to the boiling point of the reaction medium. Suitable reaction times may vary for example from several minutes to several hours.

Preferably the reaction between a compound of general formula VI and general formula VII can also be carried out under acid catalysed conditions, more preferably by refluxing the above mentioned compounds in dichloromethane in the presence of copper(II)trifluoromethanesulfonate as described, for example, in Synlett, (1), 147-149, 2001. The respective description is hereby incorporated by reference and forms part of the disclosure.

In step 2 a compound of general formula II is reacted with a compound of general formula III in a reaction medium, preferably in a reaction medium selected from the group consisting of methanol, ethanol, isopropanol, n-butanol, diethylether, tert-butyl-methylether, dioxane, Tetrahydrofuran or mixtures of at least two of these afore mentioned reaction media. Also preferably, said reaction may be carried out in the presence of an acid, whereby the acid may be organic such as acetic acid and/or inorganic such as hydrochloric acid. Alternatively the reaction may also be carried out in the presence of a base such as piperidine, piperazine, sodium hydroxide, potassium hydroxide, sodium methoxide or sodium ethoxide or mixtures of at least two of these bases.

Reaction temperature as well as the duration of the reaction may vary over a broad range. Suitable reaction temperatures range from room temperature, i.e. approximately 25 °C to the boiling point of the reaction medium. Suitable

reaction times may vary for example from several minutes to several hours. This reaction preferably leads to racemates of compounds of general formula IV, wherein the substituents $R^1$ and $R^4$ are located cis to one another on the pyrazoline ring. The respective enantiomers can be separated by conventional methods including chiral HPLC or resolution via the formation of diastereomeric salts and thus leading to compounds of general formula IV with an ee > 99 %. The configuration of compounds of general formula IV is maintained during the subsequent reaction steps, thus leading to enantiomerically pure compounds of general formula I (ee > 99 %). In step 2 racemates of compounds of general formula IV, wherein the substituents $R^1$ and $R^4$ are located trans to one another on the pyrazoline ring, are also formed, albeit in a low yield.

In step 3 the carboxylic group of the compound of general formula IV may be activated for further reactions by the introduction of a suitable leaving group according to conventional methods well known to those skilled in the art. Preferably the compounds of general formula IV are transferred into an acid chloride, an acid anhydride, a mixed anhydride, a $C_{1-4}$ alkyl ester or an activated ester such as p-nitrophenylester. Suitable activating agent therefore are selected from the group consisting of thionyl chloride, oxalyl chloride and ethylchloroformate.

If said activated compound of general formula V is an acid chloride, wherein A represents a chlorine atom, that compound is preferably prepared by the reaction of the corresponding acid of general formula IV with thionyl chloride or oxalyl chloride, whereby said chlorinating agent is also used as the reaction medium, in the presence of at least one base, preferably in the presence of a base selected from the group consisting of triethylamine, N-methylmorpholine, pyridine, dimethylaminopyridine and diisopropylethylamine. Also preferably an additional reaction medium may be used. Suitable reaction media include hydrocarbons such as benzene, toluene or xylene, halogenated hydrocarbons such as dichloromethane, chloroform or carbon tetrachloride, ethers such as diethyl ether, dioxane, Tetrahydrofuran or dimethoxyethane or dimethylformamide and mixtures thereof. More preferably toluene in the presence of a catalytic amount of dimethylformamide is used as reaction medium. Preferred reaction temperature range from 0° C to the boiling point of the solvent and reaction times vary from several minutes to several hours.

If said activated compound of general formula V is a mixed anhydride, wherein A represents $-O-C(=O)-O-C_2H_5$, said anhydride may preferably be prepared, for example, by reaction of the corresponding acid of general formula IV with ethylchloroformate in the presence of a base such as triethylamine, pyridine or diisopropylethylamine, in a suitable solvent such as dichloromethane, optionally in an inert atmosphere, at a temperature between -50 °C and 50 °C.

In step 4 the reaction between a compound of general formula V with a compound of general formula $H-R^3$ to yield a compound of general formula I, wherein $R^3$ represents a $-NR^7R^8$ moiety, is preferably carried out in the presence of a base such as triethylamine in a reaction medium such as methylenchloride. The temperature is preferably in the range from 0 °C to the boiling point of the reaction medium. The reaction time may vary over a broad range, e.g. from several hours to several days.

Alternatively the reaction of a compound of general formula V with a compound of general formula $H-R^3$ to yield compounds of general formula I may be carried out according to conventional methods well known to those skilled in the art, e.g. from Pascual, A., J. Prakt Chem., 1999, 341(7), 695-700; Lin, S. et al., Heterocycles, 2001, 55(2), 265-277; Rao, P. et al., J. Org. Chem., 2000, 65(22), 7323-7344, Pearson D.E and Buehler, C.A., Synthesis, 1972, 533-542 and references cited therein. The respective descriptions are hereby incorporated by reference and form part of the present disclosure.

Preferably said reaction is carried out in the presence of a Lewis acid, which is preferably selected from the group consisting of $FeCl_3$, $ZnCl_2$ and $AlCl_3$, in a suitable reaction medium such as toluene, benzene, Tetrahydrofuran or similar reaction media. The temperature is preferably in the range from 0° C to the boiling point of the reaction medium, more preferably from 15 to 25 °C. The reaction time may vary over a broad range, e.g. from several minutes to several hours.

In step 5 a compound of general formula IV is reacted with a compound of general formula $H-R^3$, wherein $R^3$ represents a $-NR^7R^8$ moiety, in a reaction medium, preferably in a reaction medium selected from the group consisting of diethylether, Tetrahydrofuran, acetonitrile, methanol, ethanol, (1,2)-dichlorethane, dimethylformamide, dichlormethane and mixtures thereof, in the presence of at least one coupling agent, preferably in the presence of a coupling agent selected from the group consisting of 1-benzotriazolyloxy-tris-(dimethylamino)-phosphonium hexafluorophosphate (BOP), dicyclohexylcarbodiimide (DCC), N'-(3-dimethylaminopropyl)-N-ethylcarbodiimide (EDCI), diisoproylcarbodiimide, 1,1'-carbonyl-diimidazole (CDI), N-[(dimethyamino)-1H-1, 2, 3-triazolo[4, 5-b]pyridino-1-ylmethylen]-N-methylmethanaminium hexafluorophosphate N-oxid (HATU), O-(benzotriazol-1-yl)-N, N, N', N'-tetramethyluroniom hexafluorophosphate (HBTU), O-(benzotriazol-1-yl)-N,N,N',N'-tetramethyluronium-tetrafluoroborate (TBTU), 1-hydroxy-benzotriazole (HOBt) and 1-hydroxy-7-azabenzotriazol (HOAt), optionally in the presence of a base, preferably in the presence of a base selected from the group consisting of pyridine, dimethylaminopyridine, N-methylmorpholine, triethylamine and diisopropylethylamine to yield a compound of general formula I, wherein $R^3$ represents a $-NR^7R^8$ moiety.

Preferably said reaction is carried out in the presence of EDCI and HOBt, optionally in the presence of N-methylmorpholine or triethylamine, in an aprotic reaction medium such as dimethylformamide or Tetrahydrofuran, at a temperature between 20 °C and 30 °C for 15 to 24 hours as described in Tetrahedron Lett. 2004, 45, 4977. The respective description is hereby incorporated by reference and forms part of the disclosure. Polymer-supported EDCI (P-EDCI)

can also suitably be used for this process instead of EDCI as described in Tetrahedron Lett. 1998, 39, 1487 and Tetrahedron Lett. 2002, 43, 7685. The respective descriptions are hereby incorporated by reference and form part of the disclosure.

Alternatively said reaction can be carried out by using HBTU in the presence of a base such as diisopropylethylamine in an aprotic solvent, such as acetonitrile, preferably at a temperature between 20 and 30 °C for 15 to 24 hours.

A further process to obtain compounds of general formula IV is illustrated in scheme II given below.

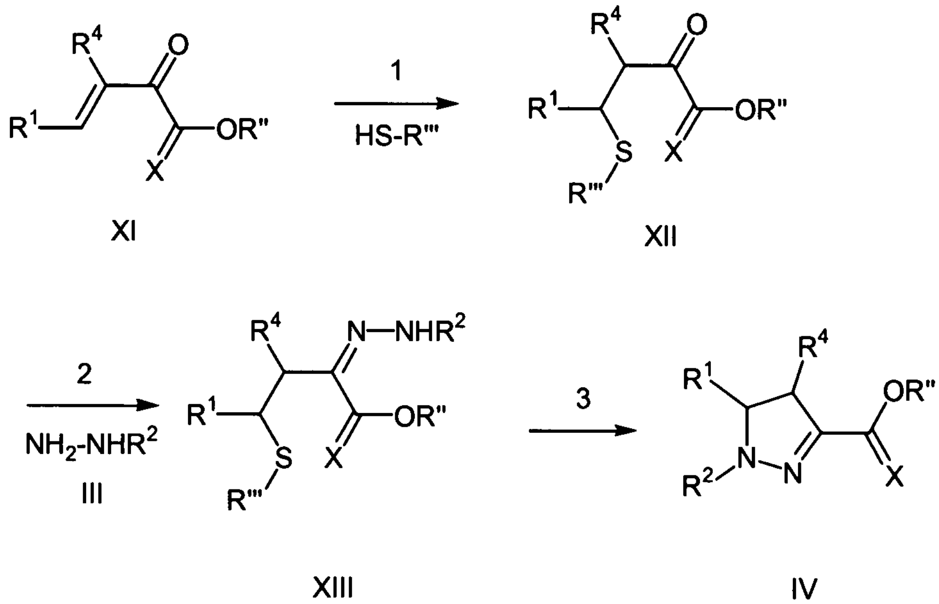


XI XII XIII IV

Scheme II

In step 1 a compound of general formula XI, wherein $R^1$, $R^4$ and X have the meaning given above and R" represents a hydrogen atom or a $C_{1-6}$-alkyl radical, is reacted with a compound of general formula HS-R''', wherein R''' represents an unsubstituted or at least mono-substituted phenyl radical, in a reaction medium, preferably in an dry aprotic reaction medium, more preferably in toluene, optionally in the presence of an organic base, preferably in the presence of an organic base selected from the group consisting of triethylamine, pyridine, diisopropylethylamine, dimethylaminopyridine and N-methylmorpholine, preferably at a temperature between - 50 °C and 50 °C, preferably for 4 to 24 hours, to yield a compound of general formula XII, wherein $R^1$, $R^4$, R", R''' and X have the meaning given above.

In step 2 a compound of general formula XII is reacted with a compound of general formula III, wherein $R^2$ has the meaning given above, in a reaction medium, preferably in a protic reaction medium, more preferably in methanol, optionally in the presence of an inorganic base, preferably in the presence of $KHSO_4$, preferably at a temperature between 0 °C and 100 °C, preferably for 4 to 15 hours, to yield a compound of general formula XIII, wherein $R^1$, $R^2$, $R^4$, R", R''' and X have the meaning given above.

In step 3 the compound of general formula XIII is cyclized intramolecularly in a reaction medium, preferably in a dry aprotic reaction medium, more preferably in dimethylformamide, preferably under an inert atmosphere, in the presence of a base, preferably in the presence of a metal hydride salt, more preferably in the presence of sodium hydride and/or potassium hydride to yield a compound of general formula IV. If R" represents a $C_{1-6}$-alkyl radical, the compound of general formula IV, wherein R" represents a hydrogen atom, is obtained after saponification of the cyclized compound according to methods known to those skilled in the art.

The sequence illustrated in scheme 1 is also described in, for example, Tetrahedron 2005, 81, 5235 - 5240 and Tetrahedron Asymmetry 2001, 12, 1923 - 1928. The respective descriptions are hereby incorporated by reference and form part of the disclosure.

A compound of general formula IV can also be obtained as described in scheme III given below.

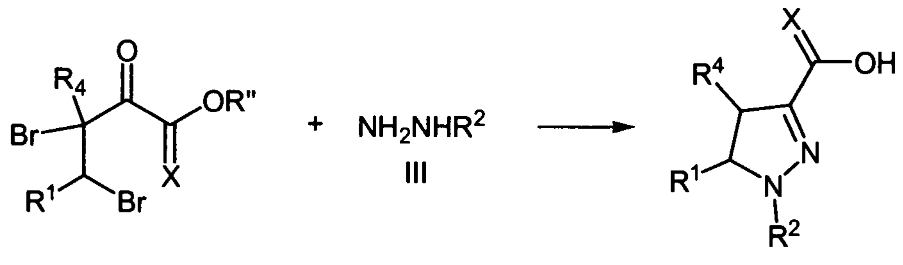

XIV IV

Scheme III

The compound of general formula XIV, wherein $R^1$, $R^4$ and X have the meaning given above and R" represents a hydrogen atom or a $C_{1-6}$-alkyl radical, is obtained by the bromination of a compound of general formula XI in a reaction medium, preferably in an aprotic reaction medium, more preferably in dichloromethane, with bromine at a temperature between 0 ˚C and 30 ˚C for several hours as described in Tetrahedron Lett. 1998, 39 (44), 8163 - 8166; J. Chem. Soc. Perkin Trans 1, 1999, 21, 3069 - 3070; Tetrahedron 1999, 55 (36), 11127 - 11142 and J. Heterocyclic Chem. 1986, 23, 1199. Preferably a compound of general formula XIV is reacted with bromine in the presence of an aprotic solvent, preferably in the presence of dichloromethane, at ambient temperature for 1 to 2 hours. The respective descriptions are hereby incorporated by reference and form part of the disclosure.

The compound of general formula XIV is reacted with a compound of general formula III, wherein $R^2$ has the meaning given above, and cyclized intramolecularly in a reaction medium, preferably in a dry aprotic reaction medium, more preferably in dimethylformamide or in a mixture of dioxane, water and acetic acid, at a temperature between 0 ˚C and 250 ˚C to yield a compound of general formula IV as described in Chemistry of Heterocyclic Compounds 1997, 33 (6); Indian J. Chem. 20B, 1981, 1090; Indian J. Chem. 29B, 1990, 887 and J. Indian Chem. Soc. 1997, 74(3), 202 - 205. The respective descriptions are hereby incorporated by reference and form part of the disclosure. If R" represents a $C_{1-6}$-alkyl radical, the compound of general formula IV, wherein R" represents a hydrogen atom, is obtained after saponification of the cyclized compound according to methods known to those skilled in the art.

A compound of general formula IV can also be obtained by the process described in scheme IV.

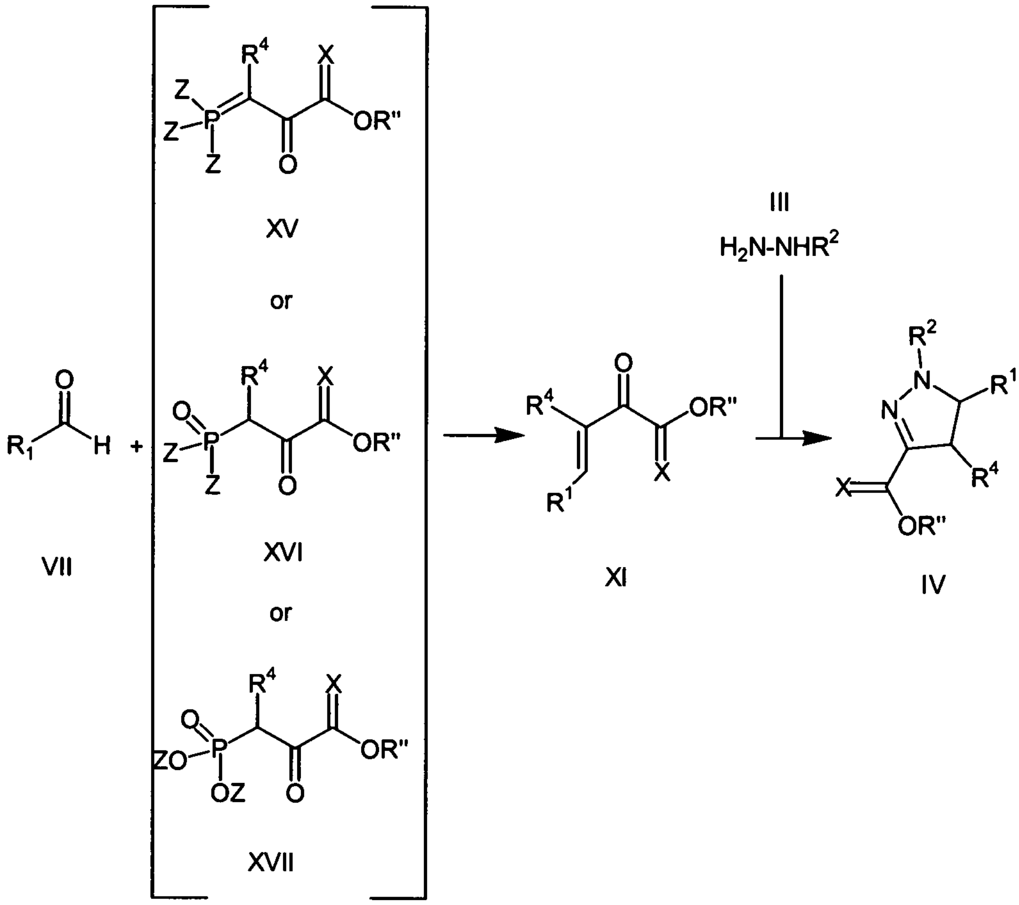


XV

or

VII

XVI

or

XVII

XI

IV

Scheme IV

An aldehyde of general formula VII, wherein $R^1$ has the meaning given above, is reacted with either a phosphonium ylide of general formula XV, a phosphine oxide of general formula XVI or a phosphonate of general formula XVII, wherein in each case $R^4$ and X have the meaning given above, R" represents a hydrogen atom or a $C_{1-6}$-alkyl radical and Z represents an unsubstituted or at least mono-substituted phenyl radical, in a reaction medium, preferably in an aprotic reaction medium, more preferably in Tetrahydrofuran, optionally in the presence of at least one base, preferably in the presence of a base selected from the group consisting of potassium tert-butylat, n-butyllithium, sodium hydride and lithium diisopropylamide, to yield a compound of general formula XI which is reacted with a compound of general formula III, wherein $R^2$ has the meaning given above, and cyclized intramolecularly to yield a compound of general formula IV as described above. The process is also described in Tetrahedron 1994, 50 (44), 12727 - 12742 and Zhumal Obshchei Khimii 1986, 56 (2), 347 - 353. The respective descriptions are hereby incorporated by reference and form part of the disclosure. If R" represents a $C_{1-6}$-alkyl radical, the compound of general formula IV, wherein R" represents a hydrogen atom, is obtained after saponification of the cyclized compound according to methods known to those skilled in the art.

Another method for the preparation of compounds of general formula XI is illustrated in scheme V below.

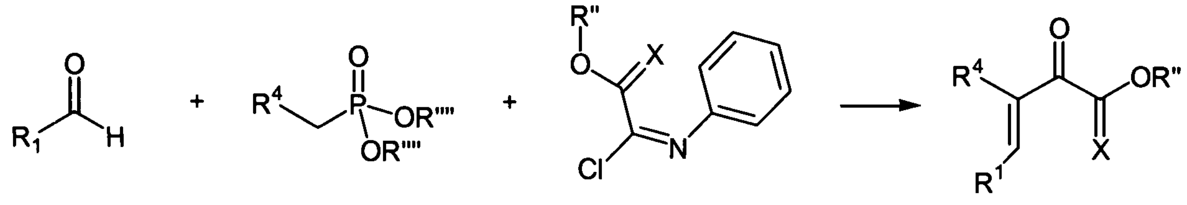

VII XVIII XIX XI

Scheme V

A compound of general formula VII, wherein $R^1$ has the meaning given above, is reacted with a phosphonate of general formula XVIII, wherein $R^4$ has the meaning given above and R'''' represents a $C_{1-6}$-alkyl radical, preferably an ethyl radical, and a compound of general formula XIX, wherein X has the meaning given above and R'' represents a hydrogen atom or a $C_{1-6}$-alkyl radical to yield a compound of general formula XI, wherein $R^1$, $R^4$, X and R'' have the meaning given above. The process is described in J. Chem. Soc. Perkin Trans 1, 1995, 741-742. Preferably the reaction is carried out by the addition of phosphonate of general formula XVIII to a solution of n-butyllithium in a dry reaction medium, preferably in Tetrahydrofuran, at a temperature between -100 °C and - 50 °C, followed by the addition of N-phenylalcoxycarbonylacetimidoyl chloride of general formula XIX and aldehyde of general formula VII and stirring at a temperature between 0 °C and 30 °C for several hours. The respective description is hereby incorporated by reference and forms part of the disclosure.

A compound of general formula IV can also be obtained according to the process described in scheme VI.

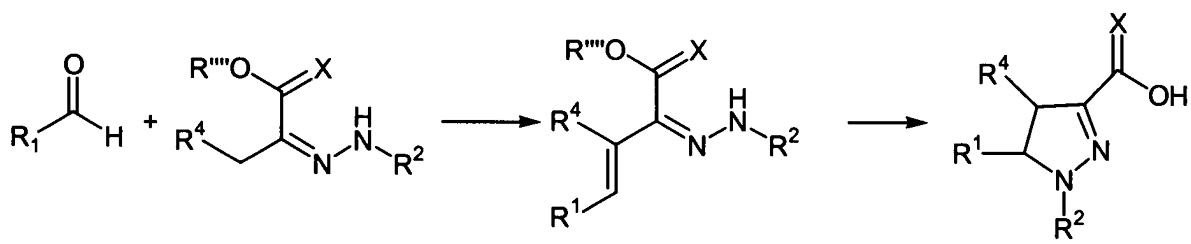

VII XX XXI IV

Scheme VI

A compound of general formula VII, wherein $R^1$ has the meaning given above, is reacted with a compound of general formula XX, wherein $R^2$, $R^4$ and X have the meaning given above and R'''' represents a $C_{1-6}$-alkyl radical, is reacted in a reaction medium, preferably in ethanol, in the presence of a base, preferably sodium acetate, at a temperature between 30 °C and 90 °C, or in a reaction medium, preferably in ethanol, in the presence of glacial acetic acid at a temperature between 0 °C and 50 °C to yield a compound of general formula XXI, wherein $R^1$, $R^2$, $R^4$ and X have the meaning given above and R'''' represents a $C_{1-6}$-alkyl radical. The compound of general formula XXI is converted into the compound of general formula IV in a reaction medium, preferably in ethanol and/or water, in the presence of an acid, preferably in the presence of hydrochloric acid, at a temperature between 50 °C and 120 °C to yield a compound of general formula IV. The process is described in J. Chem. Engineering Data 1984, 29(2), 225 - 229 and Indian J. Chem. 27B, 1988, 3, 245-249. The respective descriptions are hereby incorporated by reference and form part of the disclosure.

A compound of general formula IV can also be obtained according to the process described in scheme VII. Said process is also described in WO88/005046. The respective description is hereby incorporated by reference and forms part of the disclosure.

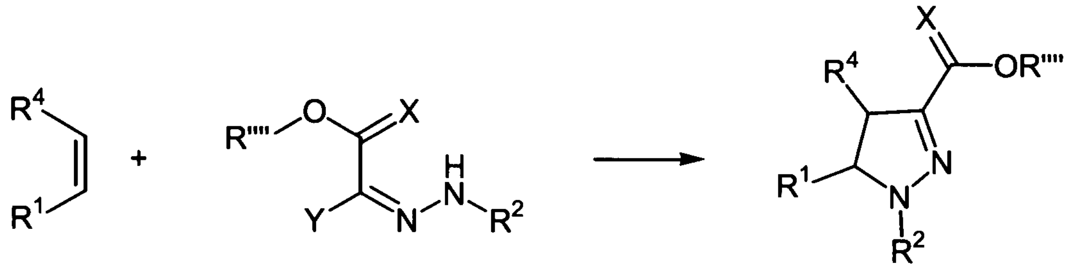

XXII XXIII IV

Scheme VII

A compound of general formula XXII, wherein $R^1$ and $R^4$ have the meaning given above, is reacted with a compound of general formula XXIII, wherein $R^2$ and X have the meaning given above, R'''' represents a $C_{1-6}$-alkyl radical and Y represents a chlorine or bromine atom, in a reaction medium, preferably in an aprotic or protic reaction medium, more preferably in toluene and/or chloroform and/or ethanol, in the presence of a base, preferably an organic base, more preferably an organic base selected from the group consisting of triethylamine, pyridine, diisopropylethylamine, dimethylaminopyridine, 1,4-diazabicyclo[2.2.2]octane and N-methylmorpholine, at a temperature between 0 °C and 150 °C to yield a compound of general formula IV. If regioisomers are obtained during the reaction, these regioisomers can be separated by conventional chromatographic techniques. The compound of general formula IV, wherein R'''' represents a $C_{1-6}$-alkyl radical is converted into the corresponding acid by using standard methods which are known to those skilled in the art. The process is disclosed in Bull. Chem. Soc. Japan 1984, 57 (3), 787 - 790 and Chem. Lett. 1982, 543 - 546. The respective descriptions are hereby incorporated by reference and form part of the disclosure. The method as depicted in scheme VII is stereospecific. Thus, using (E)-olefins of general formula XII compounds of general formula IV are obtained, wherein the radicals $R^1$ and $R^4$ on the pyrazoline ring are orientated trans to one another. Starting from (Z)-olefins the respective pyrazolines with cis-configuration are obtained. The racemates of pyrazoline compounds with either trans- or cis-configuration can be separated by conventional methods including chiral HPLC separation and separation via the formation of diastereomeric salts. Thus, enantiomerically pure compounds are obtained (ee > 99%). The configuration of compounds of general formula IV is maintained during the subsequent reaction steps, thus leading to enantiomerically pure compounds of general formula I (ee > 99 %).

The compound of general formula XXIII can be prepared according to the processes described in scheme VIII.

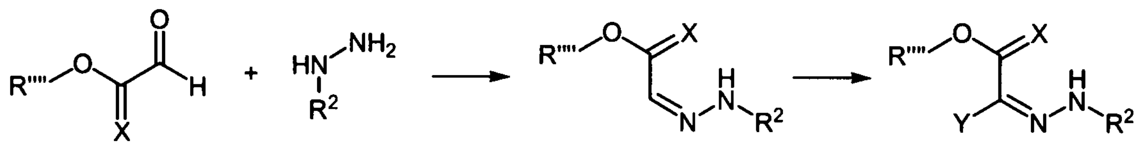

XXIV III XXV XXIII

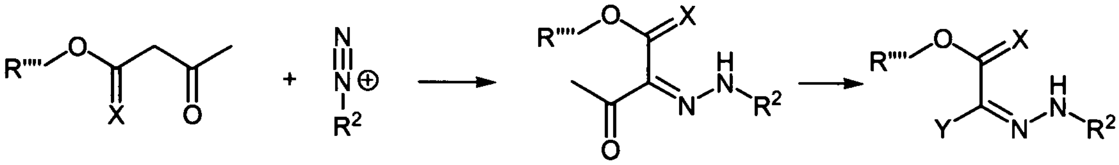

XXVI XXVII XXVIII XXIII

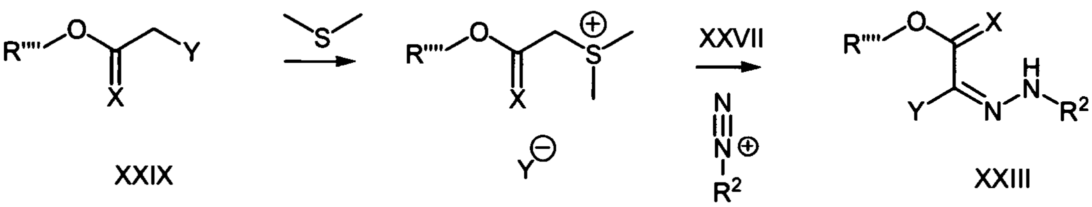


XXIX XXIII

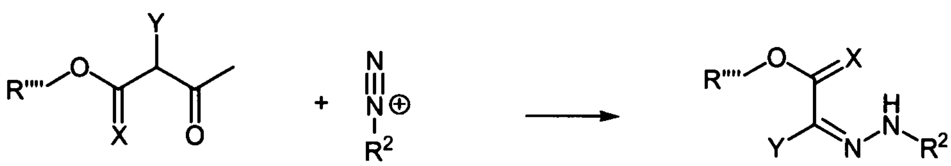

XXXXVIII XXVII XXIII

Scheme VIII

The compound of general formula XXIV, wherein X has the meaning given above and R'''' represents a $C_{1-6}$-alkyl radical, is reacted in a reaction medium, preferably in a mixture of water and ethanol, in the presence of at least one acid, preferably in the presence of acetic acid, at a temperature between 70 °C and 120 °C with a compound of general formula III, wherein $R^2$ has the meaning given above, to yield a compound of general formula XXV, wherein $R^2$ and X have the meaning given above and R'''' represents a $C_{1-6}$-alkyl radical, which is reacted with N-chloro-succinimide or N-bromo-succinimide in a reaction medium, preferably in an aprotic reaction medium, more preferably in dimethylformamide, at a temperature between 0 °C and 30 °C, to yield a compound of general formula XXIII. The process is described in Synth. Commun. 2001, 31(1), 111 - 115 and Tetrahedron 1994, 50 (25), 7543 - 7556. The respective descriptions are hereby incorporated by reference and form part of the disclosure.

The compound of general formula XXIII can also be prepared by the reaction of a compound of general formula XXVI, wherein X has the meaning given above and R'''' represents a $C_{1-6}$-alkyl radical, with a compound of general formula XXVII, wherein $R^2$ has the meaning given above and subsequent bromination of the resulting compound of general formula XXVIII, wherein $R^2$ and X have the meaning given above and R'''' represents a $C_{1-6}$-alkyl radical, by using bromine in the presence of acetic acid as described in Synthesis 1975, 333 and J. Chem. Soc. Perkin Trans. 1, 1977, 2092. The diazonium salt of general formula XXVII can preferably be obtained by the addition of an aqueous solution of sodium nitrite to a compound of general formula $R^2$-$NH_2$ in aqueous hydrochloride acid, wherein $R^2$ has the meaning given above. Alternatively this transformation can also be achieved in the presence of a compound of general formula XXVI by adjusting the pH of the reaction medium to 4 by the addition of sodium acetate at a temperature between 0 °C and 30 °C.

The respective descriptions are hereby incorporated by reference and form part of the disclosure.

The compound of general formula XXIII can also be prepared by the reaction of a compound of general formula XXIX, wherein X has the meaning given above, R'''' represents a $C_{1-6}$-alkyl radical and Y represents a chlorine or bromine

atom, with dimethylsulfide, in a reaction medium, preferably in ethanol, at a temperature between 70 °C and 120 °C. Optionally the dimethylsulfonium salt is isolated and further reacted with a compound of general formula XXVII, wherein $R^2$ has the meaning given above, in the presence of sodium acetate and acetic acid at a temperature between 0 °C and 30 °C as described in Heterocycles 1991, 32(6), 1101 - 1107. The respective description is hereby incorporated by reference and forms part of the disclosure.
The compound of general formula XXIII can also be prepared by the reaction of a compound of general formula XXXXVIII, wherein X has the meaning given above, R'''' represents a $C_{1-6}$-alkyl radical and Y represents a leaving group, preferably a leaving group selected from the group consisting of chlorine and bromine, with a compound of general formula XXVII, wherein $R^2$ has the meaning given above, in the presence of a protic solvent, preferably in the presence of a protic solvent selected from the group consisting of methanol and ethanol, or in the presence of an aprotic solvent, preferably in the presence of tetrahydrofuran, in the presence of a base, preferably in the presence of sodium acetate, or in the presence of an acid, preferably in the presence of acetic acid. The method is described in J. Chem. Soc. Perkin Transaction 1 1998, 24, 4103-4106; Tetrahedron Asymmetry 2000, 11(9), 1975-1983; Tetrahedron 1998, 54(49), 14859-14868; Synthesis 1996, 9, 1076-1078 and Synthesis 1995, 12, 1483-1484. The respective descriptions are hereby incorporated by reference and form part of the disclosure.

Another method for the preparation of a compound of general formula IV is described in scheme IX.

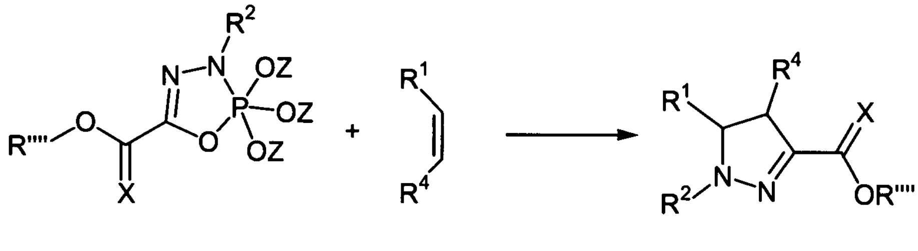

XXX XXII IV

Scheme IX

A compound of general formula XXX, wherein $R^2$ and X have the meaning given above, Z represents an unsubstituted or at least mono-substituted phenyl radical, preferably an unsubstituted phenyl radical, and R'''' represents a $C_{1-6}$-alkyl radical, preferably an ethyl radical, is reacted with a compound of general formula XXII, wherein $R^1$ and $R^4$ have the meaning given above, in a reaction medium, preferably in xylene, at a temperature between 50 °C and 200 °C for 2 to 30 hours to yield a compound of general formula IV. The process is described in Chem. Lett. 1983, 507 - 510 and Bull. Chem. Soc. Japan 1984, 57(9), 2689 - 2690. The respective descriptions are hereby incorporated by reference and form part of the disclosure. The compound of general formula IV, wherein R'''' represents a $C_{1-6}$-alkyl radical, is converted into the corresponding acid by using standard methods which are known to those skilled in the art.

A process for the preparation of a compound of general formula XXX is described in scheme X.

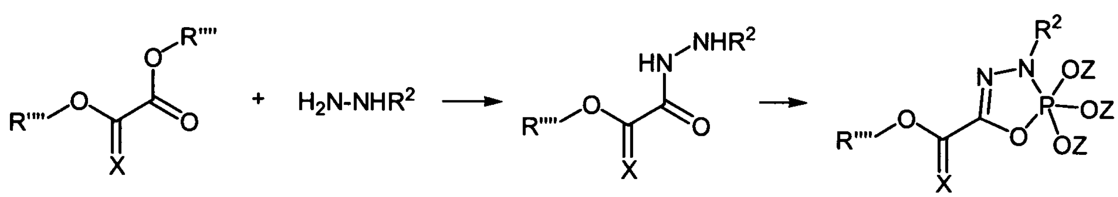

XXXI III XXXII XXX

Scheme X

A compound of general formula XXXI, wherein X has the meaning given above and R'''' is a $C_{1-6}$-alkyl radical, is reacted with a compound of general formula III, wherein $R^2$ has the meaning given above, to yield a compound of general formula XXXII, wherein $R^2$ and X have the meaning given above and R'''' represents a $C_{1-6}$-alkyl radical. Subsequently, the compound of general formula XXXIII is reacted with phosphorous pentachloride or $POCl_3$ in a reaction medium, preferably in toluene, at a temperature between 0 °C and 50 °C, followed by the addition of a phenolic compound, preferably O-trimethylsilyl-p-cresol, in refluxing toluene to yield a compound of general formula XXX.

Another method for the preparation of a compound of general formula IV is described in scheme XI.

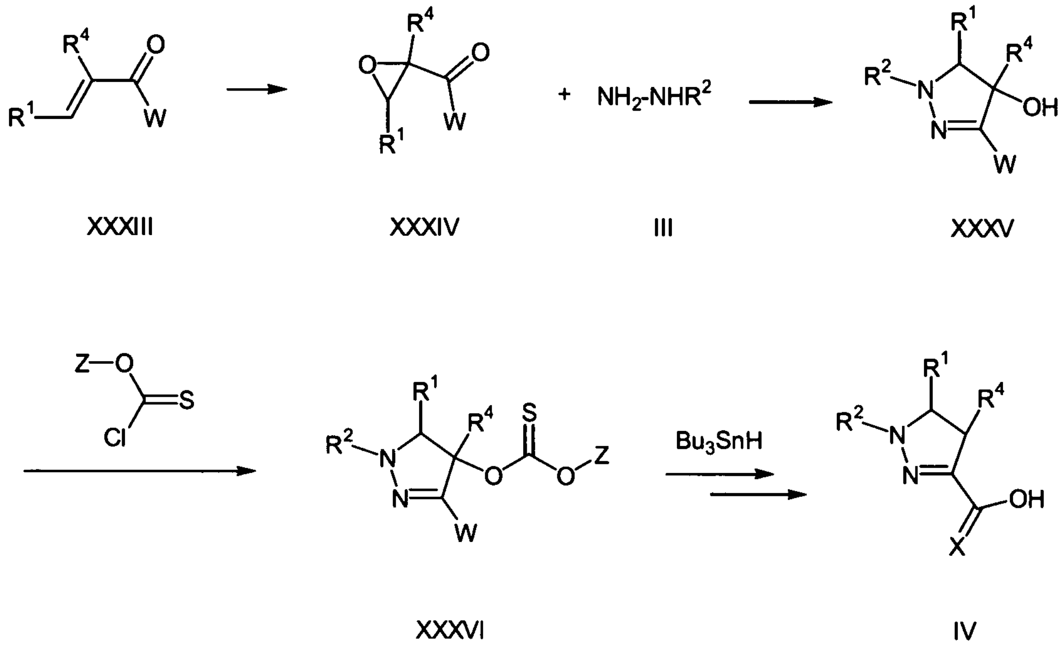


XXXIII XXXIV III XXXV

XXXVI IV

Scheme XI

A compound of general formula XXXIII, wherein $R^1$ and $R^4$ have the meaning given above and W represents -C(=X)-OH, -C(=X)-OR'''' or -CN, whereby X has the meaning given above and R'''' represents a $C_{1-6}$-alkyl radical, is converted to a compound of general formula XXXIV, wherein $R^1$, $R^4$ and W have the meaning given above, by means of epoxidation with a reagent selected from the group consisting of perbenzoic acid, preferably m-chloroperbenzoic acid, sodium peroxocarbonate, hydrogen peroxide, dioxirane and hydroperoxide. The compound of general formula XXIV is reacted with a compound of general formula III, wherein $R^2$ has the meaning given above, in a reaction medium, preferably in ethanol, to yield a compound of general formula XXXV, wherein $R^1$, $R^2$, W and $R^4$ have the meaning given above. Subsequently, the compound of general formula XXXV is converted into the corresponding xanthate of general formula XXXVI, wherein $R^1$, $R^2$, W and $R^4$ have the meaning given above, by reaction with an unsubstituted or at least mono-substituted phenylthionochloroformate of general formula ZO-C(=S)-Cl, wherein Z represents an unsubstituted or at least mono-substituted phenyl radical. The compound of general formula XXXVI is reacted with tributyltinhydride optionally followed by saponification and/or hydrolysis to yield a compound of general formula IV, wherein $R^1$, $R^2$, X and $R^4$ have the meaning given above. The process is described in Synlett 1990, 11, 705-706. The respective description is hereby incorporated by reference and forms part of the disclosure.

Yet another method for the preparation of a compound of general formula IV is described in scheme XII.

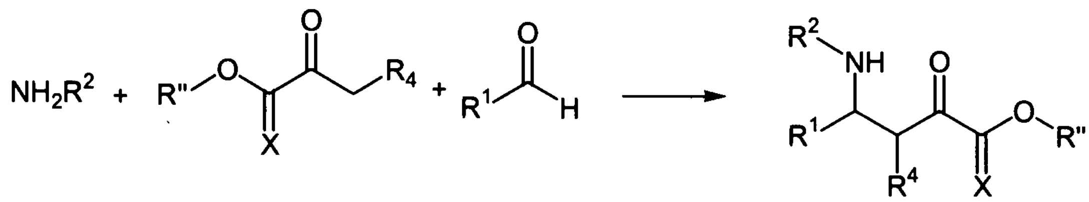

XXXVII XXXVIII VII XXXIX

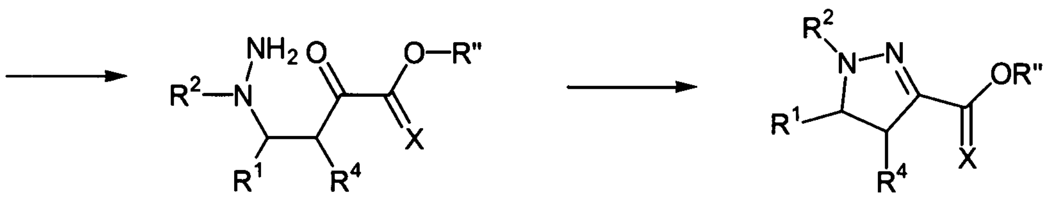

XXXX IV

Scheme XII

A compound of general formula XXXVII, wherein $R^2$ has the meaning given above, is reacted with a compound of general formula XXXVIII, wherein $R^4$ and X have the meaning given above and R" represents a hydrogen atom or a $C_{1-6}$-alkyl radical, and a compound of general formula VII, wherein $R^1$ has the meaning given above, to yield a compound of general formula XXXIX, wherein $R^1$, $R^2$, $R^4$ and X have the meaning given above and R" represents a hydrogen atom or a $C_{1-6}$-alkyl radical. The compound of general formula XXXIX is converted into the compound of general formula XXXX by using O-substituted hydroxylamines according to the method described in J. Org. Chem. 2002, 67, 6237 - 6239. The respective description is hereby incorporated by reference and forms part of the disclosure. Alternatively, this transformation can be achieved by using nitrites and subsequent reduction according to methods known to those skilled in the art. Upon cyclization of a compound of general formula XXXX according to the methods described above a compound of general formula IV is obtained. If R" represents a $C_{1-6}$-alkyl radical, the compound of general formula IV, wherein R" represents hydrogen, is obtained after saponification of the cyclized compound according to methods known to those skilled in the art.

In case $R^5$ is unlike hydrogen the reaction sequence given in Scheme I is adapted as follows.

Scheme XIII

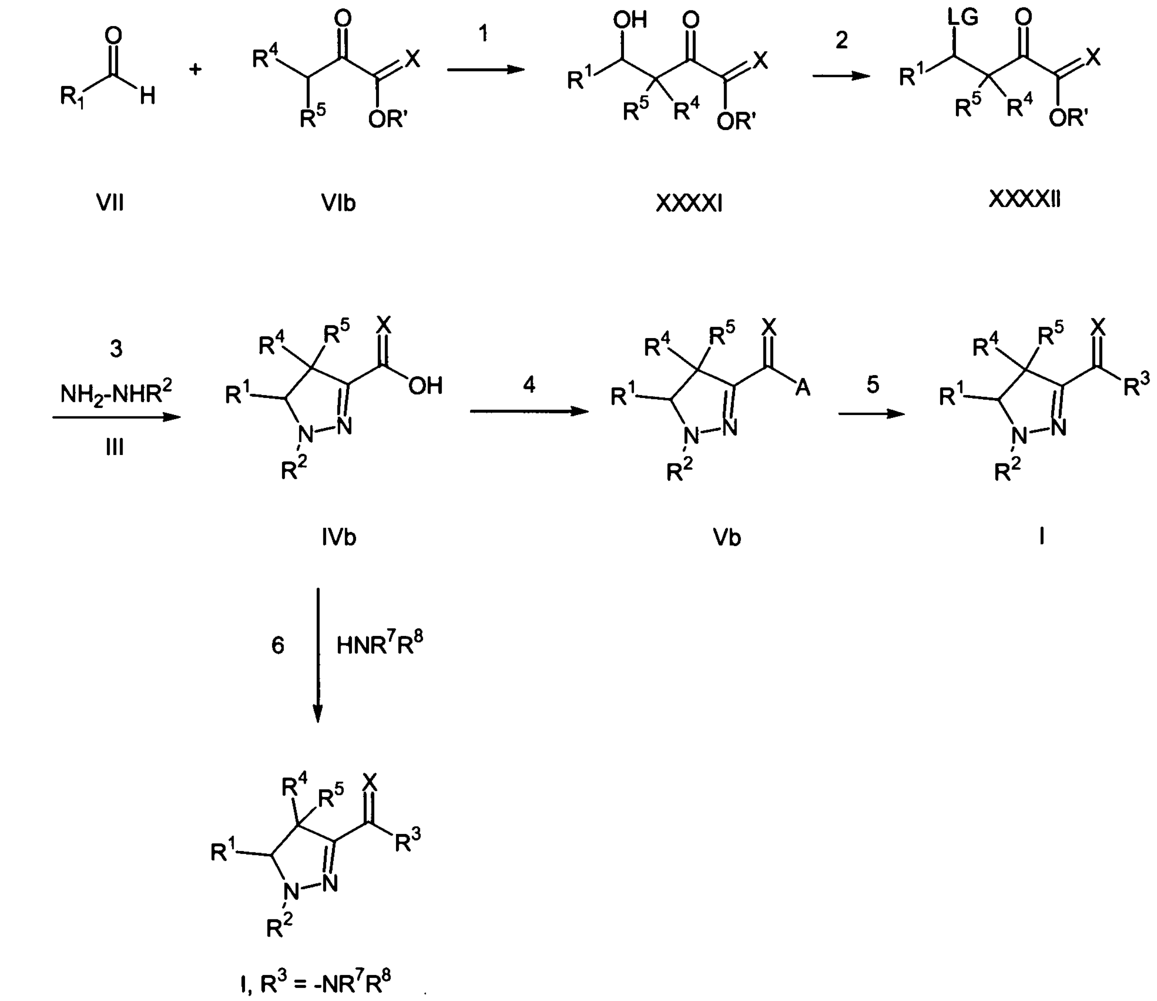


VII, VIb, XXXXI, XXXXII, IVb, Vb, I, I, $R^3$ = $-NR^7R^8$

In step 1 a compound of general formula VIb or a corresponding enolate of said compound is reacted with a compound of general formula VII in a reaction medium , preferably in a protic reaction medium, more preferably in a reaction medium selected from the group consisting of methanol, ethanol, isopropanol, n-butanol, water and mixtures thereof, in the presence of at least one base, preferably in the presence of an alkali metal hydroxide such as sodium hydroxide or potassium hydroxide or an alkali metal methoxide such as sodium methoxide or in the presence of lithium diisopropylamide in an aprotic solvent, preferably in tetrahydrofuran.

In step 2 a compound of general formula XXXXI is transformed into a compound of general formula XXXXII which contains a good living group LG, preferably a leaving group selected from the group consisting of mesyl and tosyl, using conventional methods known to those skilled in the art.

In step 3 a compound of general formula XXXXII is reacted with a compound of general formula III in a reaction medium, preferably in a reaction medium selected from the group consisting of methanol, ethanol, isopropanol, n-butanol, dieethylether, tert-butyl-methylether, dioxane, tetrahydrofuran or mixtures of at least two of these afore mentioned reaction media. Also preferably, said reaction may be carried out in the presence of an acid, whereby the acid may be organic such as acetic acid and/or inorganic such as hydrochloric acid. Alternatively the reaction may also be carried out in the presence of a base such as piperidine, piperazine, sodium hydroxide, potassium hydroxide, sodium methoxide or sodium ethoxide or mixtures of at least two of these bases. Reaction temperature as well as the duration of the reaction may vary over a broad range. Suitable reaction temperatures range from room temperature, i.e. approximately 25 °C to the boiling point of the reaction medium. Suitable reaction times may vary for example from several minutes to several hours.

Step 4 can be carried out as described for step 3, scheme I; step 5 can be carried out as described for step 4, scheme I and step 6 can be carried out as described for step 5, scheme 1.

The compounds of general formula I, wherein $R^5$ is unlike hydrogen, can also be obtained by the reaction sequence described in scheme XIV.

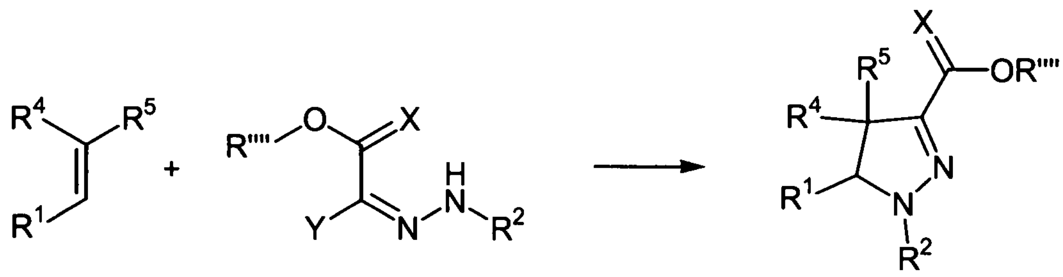

XXIIb XXIII IVb

Scheme XIV

The reaction is carried out as described for the analogues reaction depicted in scheme VII. If a mixture of regioisomers is obtained, said regioisomers can be separated by standard methods known to those skilled in the art, e. g. chromatographic methods or crystallization. The process is disclosed in Bull. Chem. Soc. Japan 1984, 57 (3), 787 - 790. The respective description is hereby incorporated by reference and forms part of the disclosure.
The compounds of general formula I, wherein $R^3$ represents $-NR^7R^8$, can also be obtained by the reaction sequence described in scheme XV.

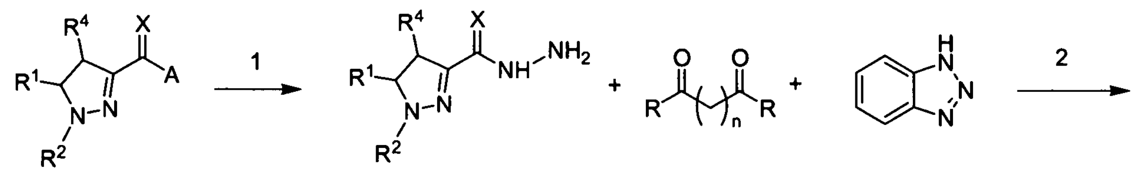


V XXXXIII XXXXIV

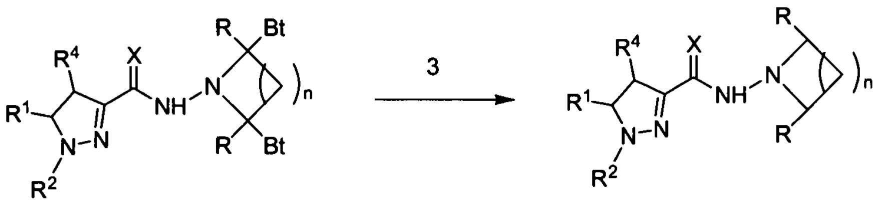


XXXXV XXXXVI

Scheme XV.

In step 1 a compound of general formula V is reacted with hydrazine hydrate in the presence of an aprotic or protic solvent, preferably in the presence of ethanol, at reflux temperature to yield a compound of general formula XXXXIII, wherein $R^1$, $R^2$, $R^4$ and X have the meaning as defined above.

In step 2 a compound of general formula XXXXIII is reacted with a compound of general formula XXXXIV, wherein R represents a hydrogen atom or a linear or branched $C_{1-12}$ alkyl radical and n is 0, 1, 2, 3, 4, 5, 6, 7, 8, 9 or 10, in the presence of benzotriazole to yield a compound of general formula XXXXV, wherein R, n, $R^1$, $R^2$, $R^4$ and X have the meaning as defined above and Bt represents a benzotriazolyl radical. A compound of general formula XXXXV can be transformed into a compound of general formula XXXXVI, wherein R, n, $R^1$, $R^2$, $R^4$ and X have the meaning as

defined above, in the presence of a reducing agent, preferably in the presence of sodium borohydride, in the presence of an aprotic solvent, preferably in the presence of tetrahydrofuran. Alternatively, the benzotriazole moiety in compounds of general formula XXXXV can be replaced by a linear or branched $C_{1-10}$ alkyl group via reaction with the respective alkyl Grignard reagents The process is disclosed J. Org. Chem. 1990, 55, 3205-3209. The respective description is hereby incorporated by reference and forms part of the disclosure.

The compounds of general formula I, wherein $R^3$ represents $-NR^7R^8$, can also be obtained by the reaction sequence described in scheme XVI.

Scheme XVI.

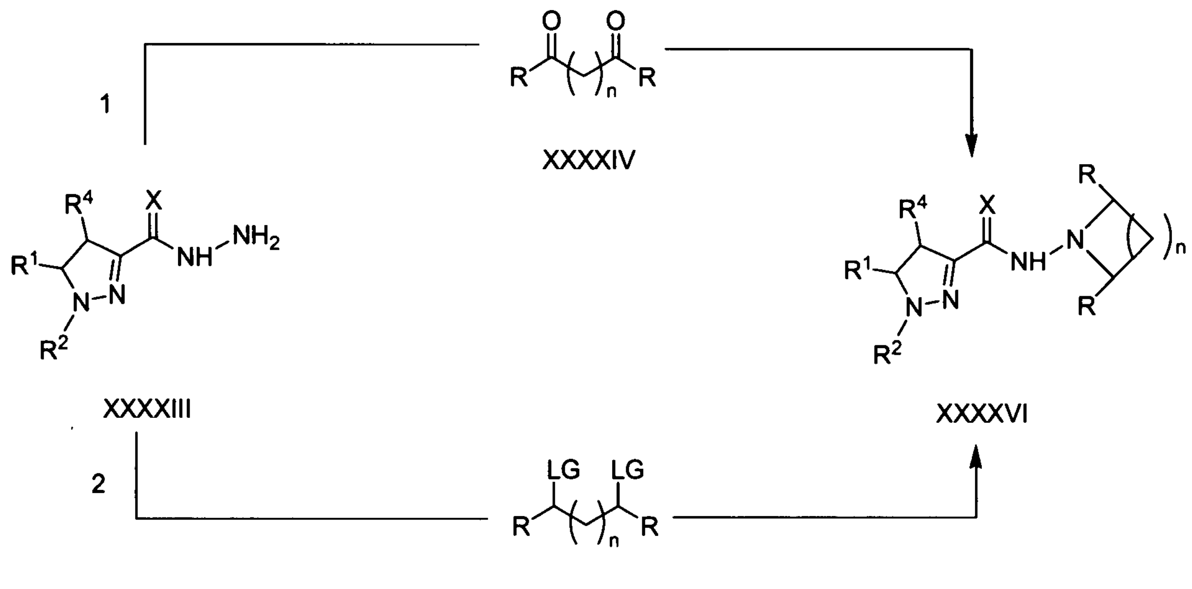

1

XXXXIV

XXXXIII

XXXXVI

2

XXXXVII

In step 1 a compound of general formula XXXXIII is reacted with a compound of general formula XXXXIV, wherein R represents a hydrogen atom or a linear or branched $C_{1-12}$ alkyl radical and n is 0, 1, 2, 3, 4, 5, 6, 7, 8, 9 or 10, in the presence of a reducing agent, preferably in the presence of a reducing agent selected from the group consisting of sodium borohydride, sodium cyanoborohydride or triacetoxyborohydride, to yield a compound of general formula XXXXVI, wherein R, n, $R^1$, $R^2$, $R^4$ and X have the meaning as defined above.

In step 2 a compound of general formula XXXXIII is reacted with a compound of general formula XXXXVII, wherein R represents a hydrogen atom or a linear or branched $C_{1-12}$ alkyl radical, n is 0, 1, 2, 3, 4, 5, 6, 7, 8, 9 or 10 and LG represents a leaving group, preferably a leaving group selected from the group consisting of chlorine and bromine, more preferably LG represents bromine, in the presence of a base, preferably in the presence of potassium carbonate, to yield a compound of general formula XXXXVI, wherein R, n, $R^1$, $R^2$, $R^4$ and X have the meaning as defined above.

The compounds of general formula I can also be obtained by the reaction sequence described in scheme XVII.

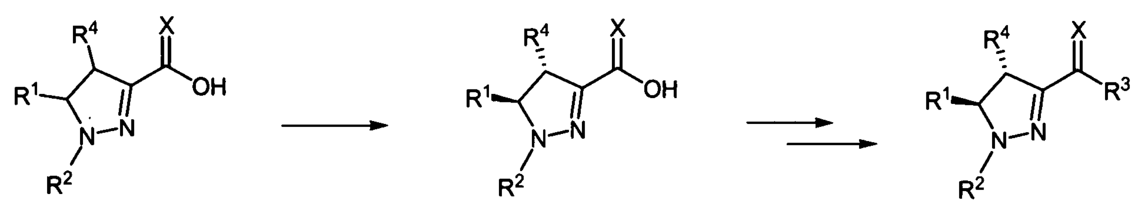

IV, $R^4$ = H

IV, $R^4$ is unlike H

I, $R^4$ is unlike H

Scheme XVII.

A compound of general formula IV, wherein $R^4$ represents hydrogen and $R^1$, $R^2$ and X have the above defined meaning, is transformed into a compound of general formula IV, wherein $R^4$ is unlike hydrogen and $R^1$, $R^2$, $R^4$ and X have the above defined meaning, in the presence of a base, preferably in the presence of a base selected from the group consisting of sodium hydride, lithium diisopropylamide and lithium hexamethyldisilazide, in the presence of an aprotic solvent, preferably in the presence of an aprotic solvent selected from the group consisting of tetrahydrofuran, dichloromethane and chloroform, and in the presence of a compound of general formula $R^4$-X, wherein $R^4$ has the above defined meaning, preferably $R^4$ represents a linear or branched $C_{1-10}$ alkyl radical or CN, and X represents a leaving group, preferably a leaving group selected from the group consisting of bromine and iodine. The compounds of general formula IV, wherein $R^4$ is unlike hydrogen, are obtained as racemates, and can be separated by conventional methods including HPLC and separation via formation of diastereomeric salts. The compounds of general formula IV can be transformed into compounds of general formula I while retaining the absolute configuration of the compounds of general formula I.

A compound of general formula IV, wherein both $R^4$ and $R^5$ are different from hydrogen and $R^1$, $R^4$, $R^2$ and X have the above defined meaning, can be prepared from compounds of general formula IV, wherein $R^5$ is hydrogen, $R^4$ is unlike hydrogen and $R^1$, $R^4$, $R^2$ and X have the above defined meaning, and a compound of general formula $R^5$-X, wherein $R^5$ has the above defined meaning, preferably $R^5$ represents a linear or branched $C_{1-10}$alkyl radical or CN, and X represents a leaving group, preferably a leaving group selected from the group consisting of bromine and iodine, by applying the same method as described in scheme XVII.

Compounds of general formula I, wherein X represents O, can be transformed in the corresponding compound, wherein X represents S, by reacting the first compound with a dithiaphosphetane, preferably with 2,4-bis(4-methoxyphenyl)-1,3,2,4-dithiadiphosphetan-2,4-disulfide (Lawesson reagent) or phosphorous pentasulfide, in a reaction medium, preferably in a reaction medium selected from the group consisting of toluene, xylene, acetonitrile, dichloromethane and dimethylformamide, at a temperature between 50 ˚C to 150 ˚C.

The afore mentioned reactions involving the synthesis of the 4,5-dihydro-pyrazole ring or the reaction of a compound comprising said ring are preferably carried out under an inert atmosphere, preferably under a nitrogen or argon atmosphere, to avoid oxidation of the ringsystem.

During some synthetic reactions described above the protection of sensitive or reactive groups may be necessary and/or desirable. This can be performed by using conventional protective groups like those described in Protective groups in Organic Chemistry, ed. J. F. W. McOmie, Plenum Press, 1973; T.W. Greene & P.G.M. Wuts and Protective Groups in Organic Chemistry, John Wiley & sons, 1991. The respective parts of the description is hereby incorporated by reference and forms part of the disclosure. The protective groups may be eliminated when convenient by means well-known to those skilled in the art.

If the substituted pyrazoline compounds of general formula I are obtained in form of a mixture of stereoisomers, particularly enantiomers or diastereomers, said mixtures may be separated by standard procedures known to those skilled in the art, e.g. chromatographic methods or crystallization with chiral reagents. It is also possible to obtain pure stereoisomers via stereoselective synthesis.

It is also described a process for the preparation of salts of substituted pyrazoline compounds of general formula I and stereoisomers thereof, wherein at least one compound of general formula I having at least one basic group is reacted with at least one inorganic and/or organic acid, preferably in the presence of a suitable reaction medium. Suitable reaction media include, for example, any of the ones given above. Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid, methanesulfonic acid or camphersulfonic acid.

It is also described a process for the preparation of salts of substituted pyrazoline compounds of general formula I or stereoisomers thereof, wherein at least one compound of general formula I having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. $[NH_nR_{4-n}]^+$, wherein n is 0, 1, 2, 3 or 4 and R represents a branched or unbranched $C_{1-4}$-alkyl-radical. Suitable reaction media are, for example, any of the ones given above.

In the presence of several acidic or basic groups, mono- or poly-salts may be formed. Compounds of the formula I having an acidic group, for example a free carboxyl group, and a basic group, for example an amino group, may also be present in the form of inner salts, i.e., in zwitterionic form, or a part of the molecule may be present in the form of an inner salt and another part in the form of a normal salt.

Solvates, preferably hydrates, of the substituted pyrazoline compounds of general formula I, of corresponding stereoisomers, of corresponding N-oxides or of corresponding salts thereof may also be obtained by standard procedures known to those skilled in the art.

substituted pyrazoline compounds of general formula I, which comprise nitrogen-atom containing saturated, unsaturated or aromatic rings may also be obtained in the form of their N-oxides by methods well known to those skilled in the art.

The purification and isolation of the substituted pyrazoline compounds of general formula I, of a corresponding stereoisomer, or salt, or N-oxide, or solvate or any intermediate thereof may, if required, be carried out by conventional methods known to those skilled in the art, e.g. chromatographic methods or recrystallization.

The compounds of general formula I given above may also act as prodrugs, i.e. they represent a drug precusor, which following administration to a patient releases a drug in vivo via some kind of chemical and/or physiological process (e.g., a prodrug on being brought to a physiological pH and/or through an enzyme action is converted to a desired drug form; see, e.g., R. B. Silverman, 1992, "The Organic Chemistry of Drug Design and Drug Action", Academic Press, Chp. 8). In particular, the compounds of general formula I give rise to a compound of general formula I, wherein $R^3$ represents a -OH moiety, upon administration to a patient.

Prodrugs can be used to alter the biodistribution (e.g., to allow compounds which would not typically enter the reactive site of the protease) or the pharmacokinetics for a particular compound. For example, a hydroxyl group, can be esterified, e.g., with a carboxylic acid group to yield an ester. When the ester is administered to a subject, the ester is cleaved, enzymatically or non-enzymatically, reductively or hydrolytically, to reveal the hydroxyl group.

The substituted pyrazoline compounds of general formula I given above, their stereoisomers, corresponding N-oxides, corresponding salts thereof and corresponding solvates are toxicologically acceptable and are therefore suitable as pharmaceutical active substances for the preparation of medicaments.

It has been found that the substituted pyrazoline compounds of general formula I given above, stereoisomers thereof, N-oxides thereof, corresponding salts and corresponding solvates have a high affinity to cannabinoid receptors, particularly cannabinoid 1 ($CB_1$)-receptors, i.e. they are selective ligands for the $CB_1$-receptor and act as modulators, e.g. antagonists, inverse agonists or agonists, on these receptors. In particular, these pyrazoline compounds show little or no development of tolerance during treatment, particularly with respect to food intake, i.e. if the treatment is interrupted for a given period of time and then continued afterwards, the pyrazoline compounds will again show the desired effect. After ending the treatment with the pyrazoline compounds, the positive influence on the body weight is found to continue.

Furthermore, these substituted pyrazoline compounds show relatively weak Herg channel affinity, thus a low risk of prolongation of the QT-interval is to be expected for these compounds.

In summary, the 4-subsituted pyrazoline compounds are distinguished by a broad spectrum of beneficial effects, while at the same time showing relatively little undesired effects, i.e. effects which do not positively contribute to or even interfere with the well being of the patient.

The purification and isolation of the substituted pyrazoline compounds of general formula (I), of a corresponding stereoisomer, or salt, or N-oxide, or solvate or any intermediate thereof may, if required, be carried out by conventional methods known to those skilled in the art, e.g. chromatographic methods or recrystallization.

It is highly preferred if the active substance combination according to the invention is comprising

(A) one substituted pyrazoline compounds of general formula I selected from the group consisting of
(rac)-N-piperidinyl-5-(4-chloro-phenyl)-1-(2,4-dichlorophenyl)-4,5-dihydro-1H-pyrazol-3-carboxamide,
(R)-N-piperidinyl-5-(4-chloro-phenyl)-1-(2,4-dichlorophenyl)-4,5-dihydro-1H-pyrazol-3-carboxamide, or
(S)-N-piperidinyl-5-(4-chloro-phenyl)-1-(2,4-dichlorophenyl)-4,5-dihydro-1H-pyrazol-3-carboxamide,
in any mixing ratio, or a corresponding N-oxide thereof, or a corresponding salt thereof, or a corresponding solvate thereof,
and
at least one drug of component (B) selected from
sulphonylureas, including chlorpropamide, tolbutamide, acetoheximide, tolazamide, glibenclamide, glipizide, gliclazide, glimepiride and gliquidone, short-acting post-prandial insulin secreters, including nateglinide, repaglinide and mitiglitinide, PPARγ agonists, including thiazolidinediones including troglitazone, rosiglitazone, pioglitazone, ciglitazone, darglitazone, PPARα/γ agonists, including sipoglitazar, tesaglitazar, muraglitazar, reglitazar, peliglitazar, cevoglitazar, ragaglitazar, naveglitazar, imiglitazar, aloglitazar, sodelglitazar, peliglitazar, pemoglitazar and netoglitazone, biguanides, including metformin, dipeptidyl peptidase-4 (DPP-IV) inhibitors, saxagliptin, sitagliptin, vildagliptin, denagliptin and alogliptin, incretins and incretin analogues, including exendin-4 and liraglutide, amylin analogues, including pramlintide, α-glucosidase inhibitors, including acarbose, voglibose and miglitol, sodium/glucose co-transport inhibitors, including sergliflozin, and insulin preparations, including insulin lispro (human), insulin aspart (human), neutral insulin injection, human neutral insulin (pyr), human neutral insulin (prb), bovine neutral insulin, porcine neutral insulin, human isophane insulin, isophase insulin (prb), human isophane insulin (pyr), porcine isophane insulin, bovine isophane insulin, human insulin zinc suspension (pyr), humulin (prb), porcine insulin zinc suspension, human insulin zinc suspension (prb) 30% amorphous 70% crystalline, insulin glargine (human), protamine zinc insulin injection (crystalline (prb), bovine insulin zinc suspension, bovine protamine zinc insulin, biphasic human insulin (pyr), biphasic human insulin (prb), biphasic neutral and isophane human, insulin (prb), human insulin (emp), biphasic porcine neutral and isophane insulin, biphasic insulin aspart and insulin determir.

The inventive active substance combination may comprise the components (A) and (B) in a molar ratio of component (A) to component (B) in the range of 1:10 to 10:1, preferably 1:5 to 5:1.

The inventive active substance combination is suitable for the administration to humans, including infants, children and grown-ups, as well as animals.

Preferably the total amount of the active substance(s) according to component (A), calculated as the free compound(s), to be administered to the patient in a 24 hours period does not exceed 800 mg, preferably does not exceed 500 mg.

The total amount of the active substance(s) according to component (B), calculated as the free compound(s), to be administered to the patient in a 24 hours period does preferably not exceed 160 mg, more preferably does not exceed 80 mg.

Preferably the inventive active substance combination comprises components (A) and (B) in the above defined molar ratios and within the afore given limits for the maximum dosis to be administered per day. Medicaments on the basis of the inventive active substance combination may preferably be administered once daily, twice daily or three times daily, more preferably once daily or twice daily, most preferably once daily.

In another aspect the present invention relates to a medicament comprising an inventive active substance combination and optionally at least one further active substance and/or optionally at least one auxiliary substance.

A further aspect of the invention provides the use of said active substance combination for the manufacture of a medicament for the treatment of metabolic disorders including, but not limited to, pre-diabetes (insulin resistance, impaired glucose tolerance) or Type 2 diabetes and/or obesity and/or appetency disorders and/or dyslipidaemia and/or Metabolic Syndrome, including drug-induced weight-gain or drug-induced obesity.

Preferably the inventive pharmaceutical formulation is suitable for oral or parenteral administration, more preferably for oral, intravenous, intraperitoneal, intramuscular, subcutaneous, intrathekal, rectal, transdermal, transmucosal or nasal administration.

Inventive pharmaceutical formulation for oral administration are preferably selected from the group consisting of tablets, drageés, capsules, powders, drops, gels, juices, sirups, solutions and suspensions.

The pharmaceutical formulation of the present invention for oral administration may also be in the form of multiparticulates, preferably microparticles, microtablets, pellets or granules, optionally compressed into a tablet, filled into a capsule or suspended in a suitable liquid. Suitable liquids are known to those skilled in the art.

The respective pharmaceutical formulations may - depending on their route of administration - also contain one or more auxiliary substances known to those skilled in the art. The pharmaceutical formulations according to the present invention may be produced according to standard procedures known to those skilled in the art, e.g. from the tables of contents from "Pharmaceutics: the Science of Dosage Forms", Second Edition, Aulton, M.E. (Ed.) Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modem Pharmaceutics", Fourth Edition, Banker G.S. and Rhodes C.T. (Eds.) Marcel Dekker, Inc. New York 2002 and "The Theory and Practice of Industrial Pharmacy", Lachman L., Lieberman H. and Kanig J. (Eds.), Lea & Febiger, Philadelphia (1986). The respective descriptions are incorporated by reference and are part of the present disclosure.

In one embodiment of the present invention the pharmaceutical formulation comprises one or both of the components (A) and (B) at least partially in a sustained-release form. Preferably the inventive pharmaceutical formulation comprises component (B) at least partially in a sustained-release form.

By incorporating one or both of these components at least partially or completely in a sustained-release form it is possible to extend the duration of their effect, allowing for the beneficial effects of such a sustained release form, e.g. the maintenance of even concentrations in the blood.

Suitable sustained-release forms as well as materials and methods for their preparation are known to those skilled in the art, e.g. from the tables of contents from "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000);"Controlled Drug Delivery", Vol. I, Basic Concepts, Bruck, S.D. (Ed.), CRC Press Inc., Boca Raton (1983) and from Takada, K. and Yoshikawa, H., "Oral Drug delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encylopedia of Controlled Drug Delivery, Mathiowitz, E. (Ed.), John Wiley & Sons, Inc., New York (1999), Vol. 2, 698-728. The respective descriptions are incorporated by reference and are part of the disclosure.

If the pharmaceutical formulation according to the present invention comprises at least one of the components (A) and (B) at least partially in a sustained-release form, said sustained release may preferably be achieved by the application of at least one coating or provision of a matrix comprising at least one sustained-release material.

The sustained-release material is preferably based on an optionally modified, water-insoluble, natural, semisynthetic or synthetic polymer, or a natural, semisynthetic or synthetic wax or fat or fatty alcohol or fatty acid, or on a mixture of at least two of these afore mentioned components.

The water-insoluble polymers used to produce a sustained-release material are preferably based on an acrylic resin, which is preferably selected from the group of poly(meth)acrylates, particularly preferably poly($C_{1-4}$)alkyl (meth) acrylates, poly($C_{1-4}$)dialkylamino($C_{1-4}$)alkyl (meth)acrylates and/or copolymers or mixtures thereof, and very particularly preferably copolymers of ethyl acrylate and methyl methacrylate with a monomer molar ratio of 2:1 (Eudragit NE30D®), copolymers of ethyl acrylate, methyl methacrylate and trimethylammonium ethyl methacrylate-chloride with a monomer molar ratio of 1:2:0.1 (Eudragit RS®), copolymers of ethyl acrylate, methyl methacrylate and trimethylammonium ethyl methacrylate-chloride with a monomer molar ratio of 1:2:0.2 (Eudragit RL®), or a mixture of at least two of the above-mentioned copolymers. These coating materials are commercially available as 30 wt.% aqueous latex dispersions, i.e. as Eudragit RS30D®, Eudragit NE30D® or Eudragit RL30D®, and may also be used as such for coating purposes.

In another embodiment, the sustained-release material is based on water-insoluble cellulose derivatives, preferably alkyl celluloses, particularly preferably ethyl cellulose, or cellulose esters, e.g. cellulose acetate. Aqueous ethyl cellulose dispersions are commercially available, for example, under the trademarks Aquacoat® or Surelease®.

As natural, semisynthetic or synthetic waxes, fats or fatty alcohols, the sustained-release material may be based on carnauba wax, beeswax, glycerol monostearate, glycerol monobehenate, glycerol ditripalmitostearate, microcrystalline wax, cetyl alcohol, cetylstearyl alcohol or a mixture of at least two of these components.

The afore mentioned polymers of the sustained-release material may also comprise a conventional, physiologically acceptable plasticizer in amounts known to those skilled in the art.

Examples of suitable plasticizers are lipophilic diesters of a $C_6$-$C_{40}$ aliphatic or aromatic dicarboxylic acid and a $C_1$-$C_8$ aliphatic alcohol, e.g. dibutyl phthalate, diethyl phthalate, dibutyl sebacate or diethyl sebacate, hydrophilic or lipophilic citric acid esters, e.g. triethyl citrate, tributyl citrate, acetyltributyl citrate or acetyltriethyl citrate, polyethylene glycols, propylene glycol, glycerol esters, e.g. triacetin, Myvacet® (acetylated mono- and diglycerides, $C_{23}H_{44}O_5$ to $C_{25}H_{47}O_7$), medium-chain triglycerides (Miglyol®), oleic acid or mixtures of at least two of said plasticizers.
Aqueous dispersions of Eudragit RS® and optionally Eudragit RL® preferably contain triethyl citrate. The sustained-release material may comprise one or more plasticisers in amounts of, for example, 5 to 50 wt.% based on the amount of polymer(s) used.

The sustained-release material may also contain other conventional auxiliary substances known to those skilled in the art, e.g. lubricants, coloured pigments or surfactants.

The pharmaceutical formulation of the present invention may also comprise at least one of the components (A) and (B) covered by an enteric coating form which dissolves as a function of pH. Because of this coating, part or all of the pharmaceutical formulation can pass through the stomach undissolved and the components (A) and/or (B) are only released in the intestinal tract. The enteric coating preferably dissolves at a pH of between 5 and 7.5.

The enteric coating may be based on any enteric material known to those skilled in the art, e.g. on methacrylic acid/methyl methacrylate copolymers with a monomer molar ratio of 1:1 (Eudragit L®), methacrylic acid/methyl methacrylate copolymers with a monomer molar ratio of 1:2 (Eudragit S®), methacrylic acid/ethyl acrylate copolymers with a monomer molar ratio of 1:1 (Eudragit L30D-55®), methacrylic acid/methyl acrylate/methyl methacrylate copolymers with a monomer molar ratio of 7:3:1 (Eudragit FS®), shellac, hydroxypropyl methyl cellulose acetate-succinates, cellulose acetate-phthalates or a mixture of at least two of these components, which can optionally also be used in combination with the above-mentioned water-insoluble poly(meth)acrylates, preferably in combination with Eudragit NE30D® and/or Eudragit RL® and/or Eudragit RS®.

The coatings of the pharmaceutical formulations of the present invention may be applied by the conventional processes known to those skilled in the art, e.g. from Johnson, J.L., "Pharmaceutical tablet coating", Coatings Technology Handbook (Second Edition), Satas, D. and Tracton, A.A. (Eds), Marcel Dekker, Inc. New York, (2001), 863-866; Carstensen, T., "Coating Tablets in Advanced Pharmaceutical Solids", Swarbrick, J. (Ed.), Marcel Dekker, Inc. New York (2001), 455-468; Leopold, C.S., "Coated dosage forms for colon-specific drug delivery", Pharmaceutical Science & Technology Today, 2(5), 197-204 (1999), Rhodes, C.T. and Porter, S.C., Coatings, in Encyclopedia of Controlled Drug Delivery. Mathiowitz, E. (Ed.), John Wiley & Sons, Inc., New York (1999), Vol. 1, 299-311. The respective descriptions are incorporated by reference and are part of the present disclosure.

In another embodiment, the pharmaceutical formulation of the present invention contains one or both of components (A) and (B) not only in sustained-release form, but also in non-retarded form. By combination with the immediately released form, a high initial dose can be achieved for the rapid onset of the beneficial effect. The slow release from the sustained release form then prevents the beneficial effect from diminishing. Such a pharmaceutical formulation is particularly useful for the treatment of acute health problems.

This may be achieved, for example, by a pharmaceutical formulation having at least one immediate-release coating comprising at least one of the components (A) and (B) to provide for rapid onset of the beneficial effect after administration to the patient.

## EXAMPLES

## CHEMICAL EXAMPLES

## SUBGROUP A1

**Soubgroup A1: Chemical Example 1:**

**N-piperidinyl-5-(4-chlorophenyl)-1-(2,4-dichlorophenyl)-4,5-dihydro-1H-pyrazole-3-carboxamide**

**a) 4-(4-chlorophenyl)-2-oxo-3-butenoic acid**

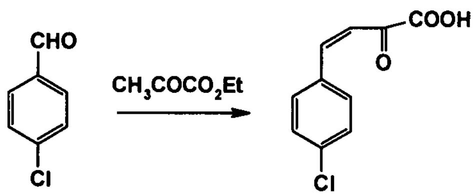


In a three neck flask p-chlorobenzaldehyde (13,3 g, 95 mmoles) and ethyl pyruvate (10 g, 86 mmoles) were dissolved in 150 ml of absolute ethanol.The solution was ice-cooled to 0˚C and an aqueous solution of NaOH (3.8 g in 45 mL water) was added dropwise keeping the temperature below or equal to 10˚C, whereby a yellow-orange colored precipitate was formed. The reaction mixture was stirred for 1 hour at 0˚C and an additional 1.5 hours at room temperature (approximately 25 ˚C). Afterwards the reaction mixture was cooled down to approximately 5˚C and the insoluble sodium salt of 4-(4-chlorophenyl)-2-oxo-3-butenoic acid was isolated by filtration.
The filtrate was left in the refrigerator overnight, whereby more precipitate is formed, which was filtered off, combined with the first fraction of the salt and washed with diethyl ether. The sodium salt of 4-(4-chlorophenyl)-2-oxo-3-butenoic acid was then treated with a solution of 2N HCl, stirred for some minutes and solid 4-(4-chlorophenyl)-2-oxo-3-butenoic acid was separated via filtration and dried to give 12.7 g of the desired product (70% of theoretical yield).
IR (KBr, $cm^{-1}$) : 3500-2500, 1719,3, 1686,5, 1603,4, 1587,8, 1081,9.
$^1H$ NMR($CDCl_3$, δ) : 7,4 (d, J=8,4Hz, 2H), 7,5 (d, J=16,1Hz, 1H), 7,6 (d, J=8,4Hz, 2H), 8,1(d, J=16,1Hz, 1H).

**b) 5-(4-chlorophenyl)-1-(2,4-dichlorophenyl)-4,5-dihydro-pyrazole-3-carboxylic acid**

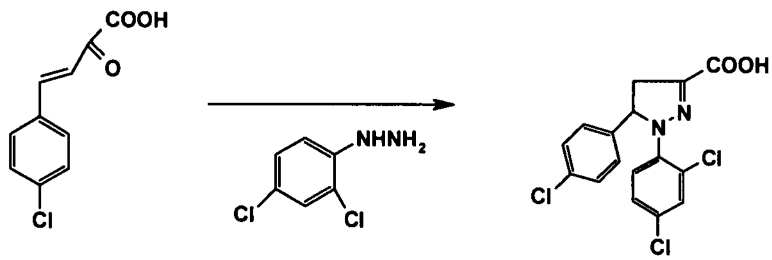

4-(4-chlorophenyl)-2-oxo-3-butenoic acid obtained according to step a) (12.6 g, 60 mmoles), 2,4-dichlorophenylhydrazine hydrochloride (12.8 g, 60 mmoles) and glacial acetic acid (200 mL) were mixed under a nitrogen atmosphere and heated to reflux for 4 hours, cooled down to room temperature (approximately 25 ˚C) and given into ice-water, whereby a sticky mass was obtained, which was extracted with methylene chloride. The combined methylene chloride fractions were washed with water, dried with sodium sulfate, filtered and evaporated to dryness to give a pale yellow solid (12.7 g, 57% of theoretical yield).
IR (KBr, $cm^{-1}$) : 3200-2200, 1668,4, 1458, 1251,4, 1104,8.

$^1H$ NMR ($CDCl_3$, δ) : 3,3 (dd, 1H), 3,7 (dd, 1 H), 5,9 (dd, 1H), 7,09-7,25 (m, 7H).

**(c) 5-(4-chlorophenyl)-1-(2,4-dichlorophenyl)-4,5-dihydro-pyrazole-3-carboxylic acid chloride**

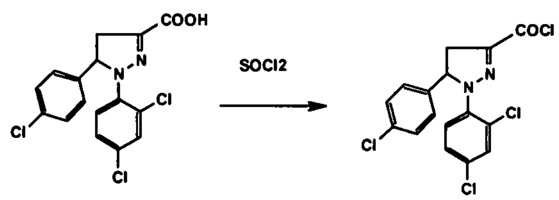


Under nitrogen atmosphere 5-(4-chlorophenyl)-1-(2,4-dichlorophenyl)-4,5-dihydro-pyrazole-3-carboxylic acid (2.5 g, 6.8 mmols) obtained according to step (b) was dissolved in 4 mL of in thionyl chloride and heated to reflux for 2.5 hours. The excess thionyl chloride is removed from the reaction mixture under reduced pressure and the resulting crude residue (2.6 g) is used without any further purification.
IR (KBr, $cm^{-1}$) : 1732,3, 1700, 1533,3, 1478,1, 1212,9, 826,6.

**d) N-piperidinyl-5-(4-chlorophenyl)-1-(2,4-dichlorophenyl)-4,5-dihydropyrazole-3-carboxamide**

[this compound may also be referred to as 5-(4-Chloro-phenyl)-1-(2,4-dichloro-phenyl)-4,5-dihydro-1H-pyrazole-3-carboxylic acid piperidin-1-ylamide or as 1-(2,4-dichlorophenyl)-5-(4-chlorophenyl)-4,5-dihydro-N-(piperidin-1-yl)-1 H-pyrazole-3-carboxamide]

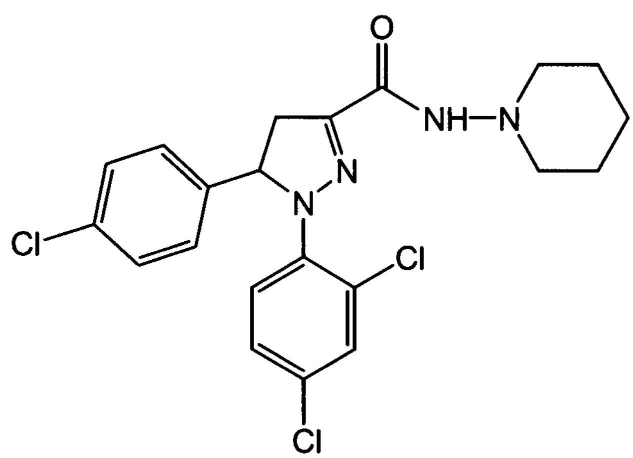

Under nitrogen atmosphere N-aminopiperidine (0.6 mL, 5.6 mmoles) and triethylamine (4 mL) were dissolved in methylene chloride (25 mL). The resulting mixture was ice-cooled down to 0˚C and a solution of 5-(4-chlorophenyl)-1-(2,4-dichlorophenyl)-4,5-dihydro-pyrazole-3-carboxylic acid chloride obtained in step (c) in methylene chloride (15 mL) was added dropwise. The resulting reaction mixture was stirred at room temperature (approximately 25 ˚C) overnight. Afterwards the reaction mixture was washed with water, followed by a saturated aqueous solution of sodium bicarbonate, then again with water, dried over sodium sulfate, filtered and evaporated to dryness in a rotavapor. The resulting crude solid was crystallized from ethanol. The crystallized solid was removed via filtration and the mother liquors were concentrated to yield a second fraction of crystallized product. The two fractions were combined to give a total amount of 1.7 g (57% of theoretical yield) of N-piperidinyl-5-(4-chlorophenyl)-1-(2,4-dichlorophenyl)-4,5-dihydropyrazole-3-carboxamide having a melting point of 183-186˚C.
IR (KBr, $cm^{-1}$): 3222,9, 2934,9, 1647,4, 1474,7, 1268,3, 815,6.
$^1H$ NMR ($CDCl_3$, δ) : 1,4 (m, 2H), 1,7 (m, 4H), 2,8 (m, 4H), 3,3 (dd, J=6,1 y 18,3Hz, 1H), 3,7 (dd, J=12,5 and 18,3 Hz, 1H), 5,7 (dd, J=6,1 and 12,5 Hz, 1 H), 7,0-7,2 (m, 6H), 7,4 (s, 1H).
The compounds according to the following examples 2-6 have been prepared analogously to the process

described in Example 1.

**Subgroup A1: Chemical Example 2:**

**5-(4-Chloro-phenyl)-1-(2,4-dichloro-phenyl)-4,5-dihydro-1H-pyrazole-3-carboxylic acid-[1,2,4]triazol-4-yl amide**

Melting point: 134-138 ˚C.
IR (KBr, $cm^{-1}$): 3448, 1686, 1477, 1243, 1091, 821.
$^1H$ NMR($CDCl_3$, δ): 3,1 (dd, J=6,2 and 17,9Hz, 1 H), 3,7 (dd, J=12,3 and 17,9Hz, 1 H), 5,9 (dd, J=6,2 and 12,3 Hz, 1 H), 7,2-7,5 (m, 7H), 8,7 (s, 2H), 12,0 (bs, 1H).

**Subgroup A1: Chemical Example 3:**

**5-(4-Chloro-phenyl)-1-(2,4-dichloro-phenyl)-4,5-dihydro-1H-pyrazole-3-carboxylic acid-(4-methyl-piperazin-1-yl)-amide hydrochloride**

Melting point: 150-155˚C.
IR (KBr, $cm^{-1}$): 3433, 1685, 1477, 1296, 1246, 1088, 1014, 825.
$^1H$ NMR ($CDCl_3$, δ): 2,7 (d, J=4,2Hz, 3H), 3,0-3,4 (m, 9H), 3,6 (dd, J=11,9 and 17,9 Hz, 1H), 5,8 (dd, J=5,5 and 11,9 Hz, 1H), 7,1 (d, J=8,4Hz, 2H), 7,25 (2d, J= 8,4 and 8,7 Hz, 3H), 7,4 (d, J=2,2Hz, 1 H), 7,5 (d, J=8,7Hz, 1 H), 9,8 (s, 1H), 11,2 (bs).

**Subgroup A1: Chemical Example 4:**

**5-(4-Chloro-phenyl)-1-(2,4-dichloro-phenyl)-4,5-dihydro-1H-pyrazole-3-carboxylic acid diethylamide**

This compound was obtained in form of an oil.
IR (film, $cm^{-1}$): 2974, 1621, 1471, 1274, 1092, 820.
$^1H$ NMR ($CDCl_3$, δ): 1,2 (m, 6H), 3,3-3,9 (m, 6H), 5,6 (dd, J=5,8 and 11,7 Hz, 1 H), 7-7,25 (m, 7H).

**Subgroup A1: Chemical Example 5:**

**[5-(4-Chloro-phenyl)-1-(2,4-dichloro-phenyl)-4,5-dihydro-1H-pyrazol-3-yl]-piperidin-1-yl-methanone**

Melting point: 105-110˚C.
IR (KBr, $cm^{-1}$) : 2934, 1622, 1470, 1446, 1266, 1010, 817.
$^1H$ NMR ($CDCl_3$, δ): 1,7 (m, 6H), 3,4 (dd, J=5,7 and 17,9Hz, 1H), 3,7 (m, 3H), 3,9 (m, 2H), 5,6 (dd, J=6,1 y 11,9 Hz, 1H), 7-7,25 (m, 7H).

**Subgroup A1: Chemical Example 6:**

**N-[5-(4-Chloro-phenyl)-1-(2,4-dichloro-phenyl)-4,5-dihydro-1 H-pyrazole-3-carbonyl]-4-methyl-phenylsulfonamide**

This compound was obtained in form of an amorph solid.
IR (KBr, $cm^{-1}$) : 1697, 1481, 1436, 1340, 1169, 1074, 853.
$^1H$ NMR ($CDCl_3$, δ): 2,4 (s, 3H), 3,2 (dd, J=6,6 and 18,3Hz, 1H), 3,6 (dd, J=12,8 and 18,3Hz, 1 H), 5,8 (dd, J=6,6 and 12,8Hz, 1 H), 7 (d, J=8,2Hz, 2H), 7,2 (s, 1H), 7,3-7,4 (m, 6H), 8 (d, J=8,1 Hz, 2H), 9 (s, 1 H).

**Subgroup A1: Chemical Example 7:**

N-oxide of N-piperidinyl-5-(4-chlorophenyl)-1-(2,4-dichlorophenyl)-4,5-dihydropyrazole-3-carboxamide

Under nitrogen gas as an inert atmosphere N-piperidinyl-5-(4-chlorophenyl)-1-(2,4-dichlorophenyl)-4,5-dihydropyrazole-3-carboxamide (0,15 g, 332 mmoles) was dissolved in 7 ml of dichloromethane. The resulting solution was ice-cooled to 0 ˚C and m-chloroperbenzoic acid (0,204 g, 0,83 mmoles) added in several portions. After stirring for 15 minutes a control via thin layer chromatography showed that no starting material was remaining. A saturated solution

of sodium bicarbonate was then slowly added, the organic phase separated, washed with water, dried over sodium sulfate and filtered. The filtered solution was evaporated to dryness and the crude product was purified via column chromatography yielding 78 mg (50 % of theoretical yield) of the N-oxide of N-piperidinyl-5-(4-chlorophenyl)-1-(2,4-dichlorophenyl)-4,5-dihydropyrazole-3-carboxamide in form of a white solid having a melting point of 115-120 ˚C.

IR (KBr, $cm^{-1}$): 3202, 1678, 1654, 1474, 1309, 1107.
$^1$H-NMR ($CDCl_3$, δ): 1.6 (m, 2H), 1.8-2.0 (m, 4H), 2.55 (m, 2H), 3.3 (dd, J = 6.3 Hz and 18.2 Hz, 1 H), 3.7 (m, 3H), 5.8 (dd, J = 6.3 Hz and 12.5 Hz, 1 H), 7.0-7.3 (m, 7H), 8.5 (s, 1H.)

**SUBGROUP A2**

**Soubgroup A2: Chemical Example 1:**

**(R)-N-piperidinyl-5-(4-chlorophenyl)-1-(2,4-dichlorophenyl)-4,5-dihydro-1H-pyrazole-3-carboxamide**

**Resolution of 5-(4-chlorophenyl)-1-(2,4-dichlorophenyl)-4,5-dihydro-pyrazole-3-carboxylic acid with (+)-Cinchonine**

35 g (294.68 mmol) of racemic 5-(4-chlorophenyl)-1-(2,4-dichiorophenyl)-4,5-dihydro-pyrazole-3-carboxylic acid in 630 m acetonitril were added to a suspension of 16.39 g (47.34 mmol) of (+)-Cinchonine in 920 ml acetonitrile under vigorous stirring. The resulting suspension was heated to reflux and acetonitrile (1300 ml) was added until dissolution was complete. The resulting solution was left to crystallise at room temperature over the weekend, whereupon a crystalline solid was obtained. The crystalline solid was filtered off, washed with 50 ml of cold acetonitrile and dried to give 25.26 g of a white solid (ee≈91-92%). No second fraction of crystals could be obtained from the mother liquors, neither upon cooling in the refrigerator nor upon concentration. Thus, recrystallization of the diastereomeric salt was carried out in different solvents in order to improve the enantiomeric excess:

| Solvent | Yield | Ratio of enantiomers % (R) / % (S) |
|---|---|---|
| Acetonitrile | 90 % | 99,6/0,4 |
| EtOH-$H_2O$ | 68,5 % | 99,3 / 0,7 |
| Isopropanol | 55 % | 98,9 /1,1 |

Consequently, the product was recrystallized from acetonitrile to give 22.6 g of crystals (yield 72 mol-% related to chiral base). The ratio of enantiomers determined by capillary electrophoresis was:
99.7 % of (R)-5-(4-chlorophenyl)-1-(2,4-dichlorophenyl)-4,5-dihydro-pyrazole-3-carboxylic acid (+)-Cinchonine
0.3 % of (S)-5-(4-chlorophenyl)-1-(2,4-dichiorophenyl)-4,5-dihydro-pyrazole-3-carboxylic acid (+)-Cinchonine ee= 99.4 %.

Preparation of (R)-5-(4-chlorophenyl)-1-(2,4-dichlorophenyl)-4,5-dihydro-pyrazole-3-carboxylic acid from addition compound (R)-5-(4-chlorophenyl)-1-(2,4-dichlorophenyl)-4,5-dihydro-pyrazole-3-carboxylic acid-(+)-Cinchonine
22.59 g (34 mmol) of the diastereomeric salt, which was recrystallized from acetonitrile, were suspended in 200 ml of 6N HCl and stirred at room temperature for 15 minutes. Afterwards, 200 ml of toluene were added until dissolution was complete. The mixture was stirred for 30 minutes and the phases were separated. The aqueous phase was extracted with toluene, the combined organic phases were washed with water, dried over sodium sulfate, filtered and evaporated to dryness, whereupon 11.9 g (95 %) of a white microcrystalline solid (melting point 129-133) were obtained.

Enantiomeric excess determined bY capillary electrophoresis. ee = 99.2 %
Chemical purity determined by HPLC: 99,3 %
$[\alpha]_D$ (c=1,23˚C, MeOH) = -429.

**Isolation of the enantiomer (R)-5-(4-chlorophenyl)-1-(2,4-dichlorophenyl)-4,5-dihydro-pyrazole-3-carboxylic acid from the mother liquors obtained from the crystallisation of the enantiomer (S)-5-(4-chlorophenyl)-1-(2,4-dichlorophenyl)-4,5-dihydro-pyrazole-3-carboxylic acid with Brucine**

The residue obtained from the evaporation of the mother liquors obtained from the crystallisation of (S)-5-(4-chlorophenyl)-1-(2,4-dichlorophenyl)-4,5-dihydro-pyrazole-3-carboxylic acid with Brucine (see below) was treated with 6N HCl as described above for obtaining the free acid. After evaporating the organic phase 21.86 g of a mixture with a

theoretical composition of 20 mmoles (S)-S-(4-chlorophenyl)-1-(2,4-dichlorophenyl)-4,S-dihydro-pyrazole-3-carboxylic acid and 39.9 mmoles of (R)-5-(4-chlorophenyl)-1-(2,4-dichlorophenyl)-4,5-dihydro-pyrazole-3-carboxylic acid were obtained. A suspension of 13.82 g (39.9 mmoles) of (+)-cinchonine in 1000 ml of acetonitrile were added and the mixture was heated to reflux. More acetonitrile was added until dissolution was complete (total volume of acetonitrile 4000 ml). The mixture was then slowly cooled to room temperature to obtain crystals. After a few hours a white solid was obtained, which was filtered off and dried to give 22.42 (yield 84,5% related to the salt of (R)-5-(4-chlorophenyl)-1-(2,4-dichlorophenyl)-4,5-dihydro-pyrazole-3-carboxylic acid).

Enantiomeric excess as determined by capillary electrophoresis was ee = 92 %.

The purity of the isolated salt was comparable to the one of the salt obtained directly from the resolution of the racemate. Consequently, the same purification process as described above could be applied.

Resolution of racemic 5-(4-chlorophenyl)-1-(2,4-dichlorophenyl)-4,5-dihydro-pyrazole-3-carboxylic acid with Brucine

**Preparation of the diastereomeric salt with Brucine**

At room temperature 15.74 g (39.9 mmol) of Brucine were dissolved in 475 ml of acetone under vigorous stirring. A solution of 29.5 g (79.8 mmol) of the racemic 5-(4-chlorophenyl)-1-(2,4-dichlorophenyl)-4,5-dihydro-pyrazole-3-carboxylic acid in 85 ml acetone was added. The resulting mixture was allowed to crystallise for a couple of days, whereupon a crystalline solid was obtained. The crystalline solid was filtered off, washed with cold acetone and dried to yield 13.66 g (45 mol % related to the base) of a beige-coloured solid were obtained.

The ratio of the enantiomers as determined by capilar electrophoresis was:
99 % of (S)-5-(4-chlorophenyl)-1-(2,4-dichlorophenyl)-4,5-dihydro-pyrazole-3-carboxylic acid Brucine
1 % of (R)-5-(4-chiorophenyl)-1-(2,4-dichlorophenyl)-4,5-dihydro-pyrazole-3-carboxylic acid Brucine
**Enantiomeric excess ee = 98 %**

The filtered mother liquors were left in the refrigerator and yielded a second fraction of crystals, which were filtered off and dried to give another 1.56 g (5.1 molar % related to the base).

The analysis of the 2$^{nd}$ fraction via capillar electrophoresis gave
99.3 % of (S)-5-(4-chlorophenyl)-1-(2,4-dichlorophenyl)-4,5-dihydro-pyrazole-3-carboxylic acid Brucine
0.7 % of (R)-5-(4-chlorophenyl)-1-(2,4-dichlorophenyl)-4,5-dihydro-pyrazole-3-carboxylic acid Brucine
Enantiomeric excess ee = 98.6 %
Total yield:
15.22 g (50.1 mol-% related to base) of the salt with ee = 98 %.

Determination of the chirality of the Brucine salt via X-ray crystallography

The chirality of the enantiomer that crystallized with Brucine (ee 98 %) was determined via x-ray crystallography and corresponds to the S-enantiomer. Accordingly, the other enantiomer that crystallized with (+)-cinchonine is the corresponding R-enantiomer.

**Preparation of (S)-5-(4-chlorophenyl)-1-(2,4-dichlorophenyl)-4,5-dihydro-pyrazole-3-carboxylic acid from addition compound (S)-5-(4-chlorophenyl)-1-(2,4-dichlorophenyl)-4,5-dihydro-pyrazole-3-carboxylic acid brucine**

15.17 g (19.85 mmol) of (S)-5-(4-chlorophenyl)-1-(2,4-dichlorophenyl)-4,5-dihydro-pyrazole-3-carboxylic acid Brucine were suspended in 150 ml of 6N HCl and the suspension was stirred for 10 minutes, followed by the addition of 150 ml of toluene upon which complete dissolution was observed. The resulting mixture was stirred for an additional 30 minutes under control with column chromatography (silical gel, $Cl_2CH_2$:MeOH 95:5). Afterwards, the phases were separated, the aqueous phase was extracted with toluene and the combined toluene phases were washed with water three times, dried over sodium sulfate, filtered and evaporated to dryness. 7 g (95,5 %) of an oil were obtained, which solidified upon addition of a small amount of diethylether to yield a white amorphous solid that showed a crystalline transformation under melting at 130-133 °C.

CCF($Cl_2CH_2$:MeOH 95:5) : $R_f$ = 0.3
The analysis of the enantiomers via capillar electrophoresis gave

99.1 % (S)-5-(4-chlorophenyl)-1-(2,4-dichlorophenyl)-4,5-dihydro-pyrazole-3-carboxylic acid 0.9 % (R)-5-(4-chlorophenyl)-1-(2,4-dichlorophenyl)-4,5-dihydro-pyrazole-3-carboxylic acid Enantiomeric excess ee = 98.2 %
$[\alpha]_D$ (c=1,23°C, MeOH) = -480.5.
Chemical purity determined by HPLC: 99.1 %
$^1$H-NMR ($CDCl_3$) $\delta$ ppm: 3,2 (dd, J=6,4 y 18,2 Hz, 1H), 3,7 (dd, J=12,7 y 18,2 Hz, 1H), 5,85 (dd, J=6,4 y 12,6 Hz, 1H),

7,0-7,2 (m, 7H).

**Isolation of the enantiomer (S)-5-(4-chlorophenyl)-1-(2,4-dichlorophenyl)-4,5-dihydro-pyrazole-3-carboxylic acid from the mother liquors obtained from the crystallisation of the other enantiomer (R)-5-(4-chlorophenyl)-1-(2,4-dichlorophenyl)-4,5-dihydro-pyrazole-3-carboxylic acid with (+)-cinchonine**

The residue obtained from the evaporation of the mother liquors obtained from the crystallisation of (R)-5-(4-chlorophenyl)-1-(2,4-dichlorophenyl)-4,5-dihydro-pyrazole-3-carboxylic acid with (+)-Cinchonine was treated with 6N HCl as described above for obtaining the free acid. After evaporating the organic phase 23.63 g of a mixture with a theoretical composition of 13.34 mmoles of (R)-5-(4-chiorophenyl)-1-(2,4-dich)orophenyl)-4,5-dihydro-pyrazole-3-carboxylic acid and 47.34 mmoles of (S)-5-(4-chlorophenyl)-1-(2,4-dichlorophenyl)-4,5-dihydro-pyrazole-3-carboxylic acid were obtained. Said mixture, dissolved in 118 ml of acetone, was added on top of a solution of 18.7 g (47.4 mmoles) of brucine in 650 ml of acetone, stirred for a couple of minutes and left to crystallise at room temperature overnight. A creme-coloured solid was obtained that was filtered off and dried to yield 28 g (77.5 % relative to the (S)-salt).

Enantiomeric excess as determined by capilar electrophoresis was ee = 98.2 %

The purity of the isolated salt was comparable to the one of the salt obtained directly from the resolution of the racemate. Consequently, the same purification process as described above could be applied.

**Preparation of (R)-N-piperidinyl-5-(4-chlorophenyl)-1-(2,4-dichlorophenyl)-4,5-dihydro-1 H-pyrazole-3-carboxamide**

9.94 g (26.89 mmol) of (R)-5-(4-chlorophenyl)-1-(2,4-dichlorophenyl)-4,5-dihydro-pyrazole-3-carboxylic acid were dissolved in 95 ml of toluene, followed by the addition of 2.35 ml (32.3 mmoles) of thionyl chloride and 4 drops of DMF. Under anhydrous nitrogen atmosphere the mixture was heated to a temperature of 75-80˚ C under vigorous stirring for two hours and then allowed to cool to room temperature. Said acid chloride solution was then added dropwise to a solution of 3.47 ml (31.2 mmol) of N-Aminopiperidine, 15 ml (107.6 mmol) of triethylamine and 38 ml of anhydrous toluene, which was cooled to 0-5 ˚C, thereby keeping the temperature below 10 ˚C during the addition. The reaction mixture was stirred at room temperature overnight, whereupon a suspension was obtained. Water and Toluene were added to said suspension until dissolution was complete and the phases were separated.

The aqueous phase was extracted with toluene, the combined organic phases washed with an NaOH solution (10 %) and water, dried over sodium sulfate, filtered off and evaporated to dryness to yield 13.41 g of light yellow oil, which failed to crystallise. The oil was purified via column chromatography over silica gel (eluent: petrol ether ethylacetate 90:10 to 60: 40) to give 10.5 g (83.5 %) of (R)-N-piperidinyl-5-(4-chlorophenyl)-1-(2,4-dichlorophenyl)-4,5-dihydro-1 H-pyrazole-3-carboxamide in form of an amorphous solid of light yellow colour (melting point 75-78 ˚C).

$^1$H-NMR (DMSO-$d_6$) $\delta$ ppm : 9,25 (s, 1H), 7,5 (d, J=8,8Hz, 1H), 7,4 (d, J=2,3Hz, 1H), 7,3-7,25 (2d, J=8,8 and 8,5Hz, 3H), 7,1 (d, J=8,5Hz, 2H), 5,8 (dd, J=5,7 and 11,8 Hz, 1H), 3,65 (dd, J=11,8 and 18,1 Hz, 1H), 3,0 (dd, J=5,7 and 18,1 Hz, 1 H), 2,75 (m, 4H), 1,5 (m, 4H), 1,2 (m, 2H).

Analysis of the enantiomer via chiral HPLC: ee = 100 %
Chemical purity determined by HPLC: 98,5%
$[\alpha]_D$ (c=1, 23˚C, MeOH) = - 293,5

**Preparation of Hydrochloride salt of (R)-N-piperidinyl-5-(4-chlorophenyl)-1-(2,4-dichlorophenyl-4,5-dihydro-1H-pyrazole-3-carboxamide**

0.5 g (1.1 mmol) of (R)-N-piperidinyl-5-(4-chlorophenyl)-1-(2,4-dichlorophenyl)-4,5-dihydro-1 H-pyrazole-3-carboxamide were dissolved in 10 ml isopropanol, cooled with ice andistsaturated HCl-ethanol solution was added. The solution changed its colour and a white solid formed, which was filtered off, washed with isopropanol:diethylether (1:1) and dried to yield 0.38 g (71 %) of a white solid with a melting point of 160-165 ˚C. The purity determined by HPLC was 99.3 %. Determination of chlorine: 7.15 % (98.5 % of the theoretical value).

$^1$H-NMR (DMSO-$d_6$) $\delta$ ppm : 10,3 (bs, 1 H), 7,5 (m, 2H), 7,3 (2d, J= 2,5 y 8,5Hz, 3H), 7,15 (d, J=8,5Hz, 2H), 5,8 (dd, J=6,3 y 12,0Hz, 1 H), 3,7 (dd+$H_2O$), 3,05 (m, 5H), 1,7 (m, 4H), 1,4 (m, 2H).

**<u>Subgroup A2: Chemical Example 2:</u>**

**Preparation of (S)-N-piperidinyl-5-(4-chlorophenyl)-1-(2,4-dichlorophenyl)-4,5-dihydro-1H-pyrazole-3-carbox-**

**amide**

5.27 g (14.26 mmol) of (S)-5-(4-chlorophenyl)-1-(2,4-dichlorophenyl)-4,5-dihydro-pyrazole-3-carboxylic acid were dissolved in 30 ml of toluene, followed by the rapid dropwise addition of 1.25 ml (17.11 mmoles) of thionyl chloride and 7 drops of DMF.Under anhydrous nitrogen atmosphere the mixture was heated to a temperature of 75-80° C under vigorous stirring for two hours and then allowed to cool to room temperature. Said acid chloride solution was then added dropwise to a solution of 1.84 ml (16.54 mmol) of N-Aminopiperidine, 8 ml (57 mmol) of triethylamine and 25 ml of anhydrous toluene, which was cooled to 0-5 °C, thereby keeping the temperature below 10 °C during the addition. The reaction mixture was stirred at room temperature overnight, whereupon a suspension was obtained. Water and Toluene were added to said suspension until dissolution was complete and the phases were separated. The aqueous phase was extracted with toluene, the combined organic phases washed with an NaOH solution (10 %) and water, dried over sodium sulfate, filtered off and evaporated to dryness to yield 6.85 g of an oil, which failed to crystallize using the solvents Ethanol, Ethylacetate and Acetone. The oil was purified via column chromatography over silica gel (eluent: petrol ether ethylacetate 90: 10 to 50: 50) to give 4.7 g (73 %) of (S)-N-piperidinyl-5-(4-chlorophenyl)-1-(2,4-dichlorophenyl)-4,5-dihydro-1H-pyrazole-3-carboxamide in form of an amorphous solid of light yellow color (melting point 73-76 °C).

$^1$H-NMR (DMSO-$d_6$) δ ppm : 9,25 (s, 1H), 7,5 (d, J=8,8Hz, 1H), 7,4 (d, J=2,3Hz, 1H), 7,3-7,25 (2d, J=8,8 and 8,5Hz, 3H), 7,1 (d, J=8,5Hz, 2H), 5,8 (dd, J=5,7 and 11,8 Hz, 1H), 3,65 (dd, J=11,8 and 18,1Hz, 1H), 3,0 (dd, J=5,7 and 18,1Hz, 1H), 2,75 (m, 4H), 1,5 (m, 4H), 1,2 (m, 2H).

Analysis of the enantiomer via chiral HPLC: ee = 98 %
Chemical purity determined by HPLC: 98,7%

**Preparation of Hydrochloride salt of (S)-N-piperidinyl-5-(4-chlorophenyl)-1-(2,4-dichlorophenyl)-4,5-dihydro-1H-pyrazole-3-carboxamide**

0.2 g (0.4 mmol) of (S)-N-piperidinyl-5-(4-chlorophenyl)-1-(2,4-dichlorophenyl)-4,5-dihydro-1 H-pyrazole-3-carboxamide were dissolved in 3 ml isopropanol, cooled with ice and a saturated HCl-ethanol solution was added. The solution changed its color and a white solid formed, which was filtered off, washed with isopropanol:diethylether (1:1) and dried to yield 0.14 g (72 %) of a white solid with a melting point of 165-175 °C. The purity determined by HPLC was 99.3 %. Determination of chlorine: 7.15 % (98.5 % of the theoretical value).

$^1$H-NMR (DMSO-$d_6$) δ ppm : 10,4 (bs, 1H), 7,45 (m, 2H), 7,3 (2d, J= 2,6 y 8,5Hz, 3H), 7,15 (d, J=8,5Hz, 2H), 5,8 (dd, J=6,3 y 12,0Hz, 1H), 3,7 (dd, J=12,0 y 18,0Hz, 1H), 3,05 (m, 5H), 1,7 (m, 4H), 1,4 (m, 2H).

**<u>SUBGROUP A3:</u>**

**Subgroup A3: Chemical Example**

**Preparation of (*E*)-4-(5-Chloro-thiophen-2-yl)-2-oxo-but-3-enoic acid**

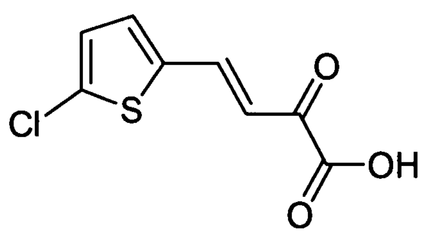

A two neck 50 mL round bottom flask equipped with a thermometer and an addition funnel was charged with 5-chlorothiophene (1.14 g, 7.6 mmol, 1.05 equiv.) and ethyl pyruvate (0.8 mL, 7.22 mmol, 1 equiv.) under nitrogen atmosphere in absolute ethanol (17 mL). The mixture was cooled to 0°C and an aqueous 2 M solution of NaOH (0.95g, 3.3 equiv. of NaOH in 10.5 mL water) was added drop wise at a rate to maintain the temperature below 10°C. The mixture was left stirring at 0°C for 1 h, the ice bath was removed and the mixture stirred at room temperature for 1 hand 30 min. The solution was cooled down in an ice bath for 30 min and a brown precipitate appeared. The solid was filtered and the mother liquors were kept at 4 °C overnight. A second crop was collected and the combined crops were washed with diethylether to eliminate traces of unreacted starting material. The remaining solid was suspended in HCl (40 mL

of a 2 M aqueous solution) and stirred for 30 min, after filtration the desired product (*E*)-4-(5-chlorothiophen-2-yl) -2-oxo-3-butenoic acid was obtained as a yellow solid (0.74 g, 47%).

$^{1}H$ NMR (400 MHz, $CDCl_3$) δ 3.5 (1 H, bs, OH), 6.98 (1 H, d, J 4.0 Hz, thiophene H), 7.19 (1H, d, J 16 Hz, ArH,), 7.29 (1 H, d, J 4.0 Hz, thiophene H), 8.09 (2H, d, J 16.0 Hz); $^{13}C$ NMR (100 MHz, $CDCl_3$): δ 117.6 (CH), 128.5 (CH), 134.4 (CH), 137.5 (C), 138.6 (C), 142.1 (CH), 161.9 (CO), 182.3 (CO).

**Subgroup A3: Chemical Example 8:**

**Preparation of 5-(5-Chloro-thiophen-2-yl)-1-(2,4-dichloro-phenyl)-4,5-dihydro-1H-pyrazole-3-carboxylic acid**

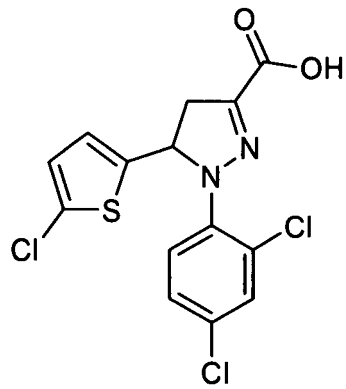

A mixture of (*E*)-4-(5-chlorothiophen-2-yl) -2-oxo-3-butenoic acid (0.72 g, 3.3 mmol) and 2,4-dichlorophenylhydrazine hydrochloride (0.71 g, 3.3 mmol) in 10.5 mL glacial acetic acid under nitrogen atmosphere was heated at reflux for 4 h. The reaction mixture was allowed to cool to room temperature and poured into ice. The dark mixture was extracted with dichloromethane, washed with $H_2O$, dried over $Na_2SO_4$ and evaporated to dryness to yield 1.35 g (≥100% of the crude acid), which was purified by semi-preparative HPLC.

$^{1}H$ NMR (400 MHz, CHLOROFORM-*d*) δ ppm 3.39 (dd, J=17.88, 4.59 Hz, 1 H) 3.64 (dd, *J*=17.88, 11.92 Hz, 1 H) 6.11 (dd, *J*=11.92, 4.59 Hz, 1 H) 6.60 (d, *J*=3.78 Hz, 1 H) 6.61 (d, *J*=3.78 Hz, 1 H) 7.12 (dd, *J*=8.60, 2.15 Hz, 1 H) 7.25 (d, *J*=8.60 Hz, 1H) 7.32 (d, J= 2.15 Hz, 1 H)
MS $(M+H)^{+}$: 375

**Subgroup A3: Chemical Example 1:**

**Preparation of 5-(5-Chloro-thiophen-2-yl)-1-(2,4-dichloro-phenyl)-4,5-dihydro-1 H-pyrazole-3-carboxylic acid piperidin-1-ylamide**

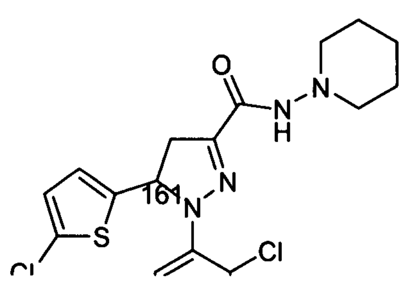

Crude 5-(5-chlorothiophen-2-yl)-1-(2,4-dichlorophenyl)-4,5-dihydropyrazol-3-carboxylic acid (0.25 g, 0.66 mmol) was dissolved several times in 5 mL of anhydrous toluene and the solvate was removed in vacuo. The acid was dissolved in 3 mL of anhydrous toluene under nitrogen atmosphere, and thionylchloride (0.8 mL, 4.86 mmol, 16 equiv)

and anhydrous dimethylformamide (55 μL) were added. The reaction mixture was heated at reflux and monitored by IR (NaCl, film). After 2 h reaction mixture was left to cool to room temperature and the solvents were evaporated to dryness under high vacuum. The desired product 5-(5-chlorothiophen-2-yl)-1-(2,4-dichlorophenyl)-4,5-dihydropyrazol-3-carbonyl chloride (0.19 g, 74%) was obtained as a dark oil and used for the next step without further purification.

IR (NaCl, film) u: 802, 1106, 1213, 1477, 1735 $cm^{-1}$

Triethylamine (0.4 mL, 6 equiv.) was added drop wise to a solution of N-aminopiperidine (71 μL, 0.66 mmol, 1.3 equiv.) in anhydrous dichloromethane (3.5mL). The mixture was cooled in an ice bath and a solution of 5-(5-chlorothiophen-2-yl)-1-(2,4-dichlorophenyl)-4,5-dihydropyrazol-3-carbonyl chloride (0.19 g, 0.48 mmol, 1 equiv.) in anhydrous DCM (3.5 mL) was added. After 15 min the ice bath was removed and the reaction was left stirring at room temperature overnight. The solution was poured in a separating funnel and washed once with water, with a saturated aqueous solution of $NaHCO_3$ and again with water, dried over $Na_2SO_4$ and evaporated to dryness to give 0.22 g of the crude product.

Purification by column chromatography ($SiO_2$ with 2.5% v/v of $Et_3N$, 30:1 ratio $SiO_2$: product, packed with 5% ethylacetate/hexane and eluted with a gradient of 5% ethylacetate/hexane to 20% ethylacetate/hexane) provided racemic *N*-(piperidin-1-yl)-5-(5-chlorothiophen-2-yl)-1-(2,4-dichlorophenyl)-4,5-dihydropyrazole-3-carboxamide (35 mg, 16% yield, 81% pure by HPLC) as a dark oil.

$^1H$ NMR (400 MHz, CHLOROFORM-*d*) δ ppm 1.44 (m, 2 H) 1.76 (dt, *J*=11.23, 5.71 Hz, 4 H) 2.84 (m, 4 H) 3.45 (dd, *J*=18.17, 4.69 Hz, 1 H) 3.61 (dd, *J*=18.17, 11.14 Hz, 1 H) 5.93 (dd, J=11.14, 4.69 Hz, 1 H) 6.57 (m, 2 H) 7.06 - 7.19 (m, 2 H) 7.32 (d, J=2.15 Hz, 1 H) 7.40 (m, 1H)

$^{13}C$ NMR (100 MHz, $CDCl_3$): δ 23.4 ($CH_2$), 25.3 ($CH_2$), 40.5 ($CH_2$), 57.3 ($CH_2$), 63.7 (CH), 125.7 (CH), 125.8 (CH), 126.1 (CH), 127.1 (C), 127.8 (CH), 130.1 (CH), 131.1 (C), 139.6 (C), 139.9 (C), 146.1 (C), 158.9 (CO).

IR (NaCl, film) u 1476, 1663, 2833, 2938 $cm^{-1}$

MS $(M+H)^+$: 457

**Subgroup A3: Chemical Example**

**Preparation of (*E*)-4-(4-Bromothiophen-2-yl)-2-oxo-3-butenoic acid**

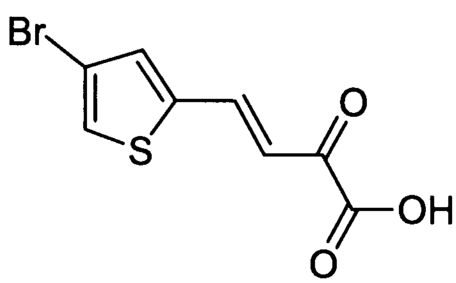

A two neck 100 mL round bottom flask equipped with a thermometer and an addition funnel was charged with 4-bromothiophene (5 g, 26.17 mmol, 1.05 equiv.) and ethyl pyruvate (2.76 mL, 24.9 mmol, 1 equiv.) under nitrogen atmosphere in absolute ethanol (61 mL). The reaction mixture was cooled to 0˚C and a 2 M solution of NaOH (3.3 g, 3.2 equiv. of NaOH in 37 mL of water) was added drop wise at a rate to maintain the temperature below 10˚C. The mixture was left stirring at 0˚C for 1 h, the ice bath was removed and the mixture stirred at room temperature for 1h and 30 min. The solution was cooled in an ice bath for 1 h and a yellow precipitate appeared. The solid was filtered and the mother liquors were kept at 4 ˚C overnight. A second crop was collected and the combined crops were washed with diethylether to eliminate traces of unreacted starting material. The solid was suspended in HCl (138 mL of a 2 M aqueous solution) and stirred for 20 min. After filtration the desired product (*E*)-4-(4-bromothiophen-2-yl) -2-oxo-3-butenoic acid was obtained as a yellow solid (3.07 g, 45%).

$^1H$ NMR (400 MHz, $CDCl_3$): 7.33 (2H, d, J 14.8 Hz), 7.41 (1 H, s, thiophene H)), 7.47 (1 H, s, thiophene H), 8.13 (2H, d, J 14.8 Hz).

**Subgroup A3: Chemical Example 27:**

**Preparation of 5-(5-Bromo-thiophen-2-yl)-1-(2,4-dichloro-phenyl)-4,5-dihydro-1H-pyrazole-3-carboxylic acid**

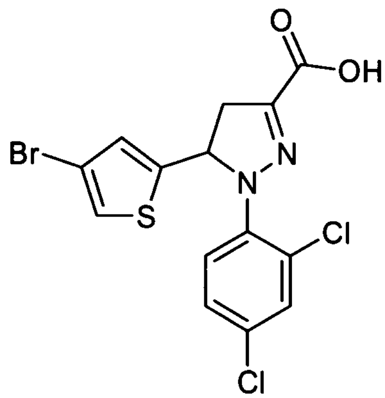

A mixture of (*E*)-4-(4-bromothiophen-2-yl)-2-oxo-3-butenoic acid (3.07 g, 11.5 mmol, 1 equiv.) and 2,4-dichlorophenylhydrazine hydrochloride (2.46 g, 11.5 mmol, 1 equiv.) in 37 mL of glacial acetic acid under nitrogen atmosphere was heated at reflux for 3 h. The reaction mixture was allowed to cool to room temperature and poured into ice. The dark mixture was extracted with dichloromethane, washed with $H_2O$, dried over $Na_2SO_4$ and evaporated to dryness to yield 5.5 g (≥100% of the crude acid).
The product was purified by preparative HPLC using samples of 1 g each of the crude product dissolved in 9 mL of MeOH which were eluted (90% heptane, 10% EtOH, 0.2% TFA, 55mL/min, 254 nm, in a Lichrospher Si60 12 μl) to provide 0.41 g of a 99% pure compound by analytical HPLC (41% yield).

$^1$H NMR (400 MHz, CHLOROFORM-*d*) δ ppm 3.42 (dd, J=18.07, 4.98 Hz, 1 H) 3.69 (dd, J=18.07, 12.02 Hz, 1H) 6.14 (dd, *J*=12.02, 4.98 Hz, 1 H) 6.77 (d, *J*=1.37 Hz, 1H) 7.04 (d, *J*=1.37 Hz, 1H) 7.13 (dd, *J*=8.60, 2.15 Hz, 1 H) 7.20 (d, *J*=8.60 Hz, 1H) 7.35 (d, *J*=2.15 Hz, 1H)
$^{13}$C NMR (100 MHz, $CDCl_3$): δ 40.4 ($CH_2$), 63.5 (CH), 110.2 (CH), 109.95 (C), 123.3 (CH), 126.8 (CH), 127.3 (C), 128.0 (CH), 129.1 (CH), 130.2 (CH), 132.0 (C), 138.3(C), 140.9 (C), 142.5 (C), 165.8 (CO).
MS $(M+H)^+$: 419

**Subgroup A3: Chemical Example 23:**

**Preparation of 5-(4-Bromo-thiophen-2-yl)-1-(2,4-dichloro-phenyl)-4,5-dihydro-1 H-pyrazole-3-carboxylic acid piperidin-1-ylamide hydrochloride**

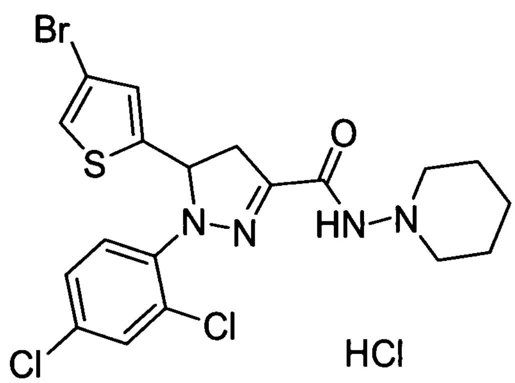

5-(5-Bromo-thiophen-2-yl)-1-(2,4-dichloro-phenyl)-4,5-dihydro-1H-pyrazole-3-carboxylic acid (0.290 g, 0.69

mmol) was dissolved in toluene (8 mL) and partially distilled at atmospheric pressure to eliminate water traces. The resulting solution was cooled to 75 °C - 85 °C. Dimethylformamide was added (2 drops) and thionylchloride (0.060 mL, 0.83 mmol) was added drop by drop. The solution was stirred until the complete formation of the acid chloride (monitoring by IR). The mixture was cooled to 20 °C - 25 °C which was added drop wise under nitrogen atmosphere to a solution of 1-aminopiperidine (0.090 mL, 0.82 mmol), dichloromethane (5 mL) and N,N-diisopropylethylamine (0.281 mL, 1.64 mmol) while keeping the temperature of the mixture between 5-10 °C. The mixture was warmed to 20 °C - 25 °C and stirred overnight. The organic layer was washed with water (3 x 5 mL), with a saturated aqueous solution of $NaHCO_3$ (3 x 25 mL) and again with water (3 x 5 mL). The final organic extract was dried with $Na_2SO_4$, filtered and concentrated in vacuo to afford a beige solid (263 mg, 77 % yield).

The solid was dissolved in 3 mL of ethyl acetate and 2.8 N HCl in ethanol was added (3 mL, 1.2 eq). The mixture was stirred at room temperature for 30 min and a beige solid appeared. The solid was filtered and washed several times with ethyl acetate to obtain a white solid (170 mg, 47 % yield).

$^1$H NMR (300 MHz, DMSO-$d_6$) δ ppm 1.42 (m, 2 H) 1.72 (m, 4 H) 3.15 (m, 4 H) 3.25 (dd, *J*=18.02, 5.13 Hz, 1 H) 3.68 (dd, *J*=18.02, 11.50 Hz, 1 H) 6.08 (dd, *J*=11.50, 5.13 Hz, 1 H) 6.95 (d, *J*=1.17 Hz, 1 H) 7.34 (m, 1 H) 7.46 (m, 2 H) 7.58 (d, *J*=2.20 Hz, 1 H) 10.68 (s, 1 H)
MS $(M+H)^+$: 501

The following compounds are prepared according to the processes described above. Those skilled in the art are familiar with the starting materials that are needed to obtain said compounds.

**Subgroup A3-Chemical Examples**

| N° | STRUCTURE | Name | $^1$H-NMR | MS $(M+H)^+$ |
|---|---|---|---|---|
| 1 | 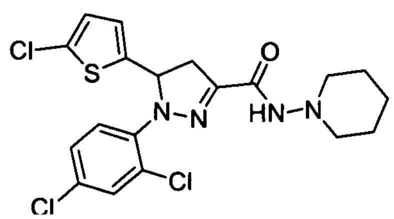 | 5-(5-Chloro-thiophen-2-yl)-1- (2,4- dichloro-phenyl)-4,5-dihydro-1H-pyrazole-3-carboxylic acid piperidin-1-ylamide | 1 H NMR (400 MHz, CHLOROFORM-*d*) δ ppm 1.44 (m, 2 H) 1.76 (dt, *J*=11.23, 5.71 Hz, 4 H) 2.84 (m, 4 H) 3.45 (dd *J*=18.17, 4.69 Hz, 1 H) 3.61 (dd, *J*=18.17, 11.14 Hz, H) 5.93 (dd, *J*=11.14, 4.69 Hz, 1 H) 6.57 (m, 2 H) 7.06-7.19 (m, 2 H) 7.32 (d, *J*=2.15 Hz,1 H) 7.40 (m,1 H) | 457 |
| 2 | 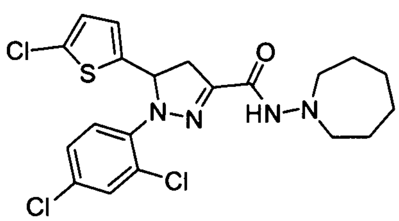 | 5-(5-Chloro-thiophen-2-yl)-1-(2,4-dichloro-phenyl)-4,5-dihydro-1H-pyrazole-3-carboxylic acid azepan-1-ylamide | | |
| 3 | 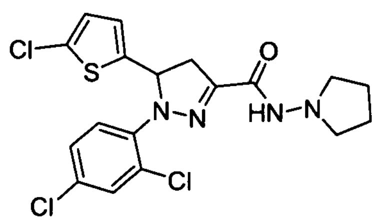 | 5-(5-Chloro-thiophen-2-yl)-1-(2,4-dichloro-phenyl)-4,5-dihydro-1 H-pyrazole-3-carboxylic acid pyrrolidin-1-ylamide | | |

(continued)

| N° | STRUCTURE | Name | $^1$H-NMR | MS (M+H)$^+$ |
|---|---|---|---|---|
| 4 | 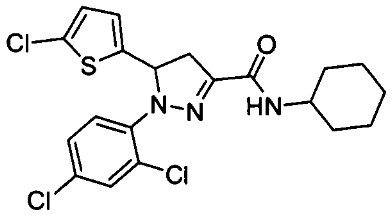 | 5-(5-Chloro-thiophen-2-yl)-1-(2,4-d ichloro-phenyl)-4,5-dihydro-1H-pyra zole-3-carboxylic acid cyclohexyl-amide | | |
| 5 | 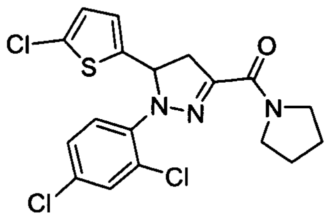 | [5-(5-Chloro-thiophen-2-yl)-1-(2,4-dichloro-phenyl)-4,5-dihydro-1H-pyr azol-3-yl]-pyrrolidin-1-yl-methanone | | |
| 6 | 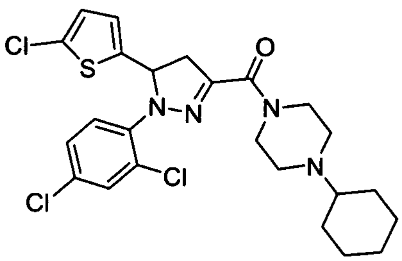 | [5-(5-Chloro-thiophen-2-yl)-1-(2,4-dichloro-phenyl)-4,5-dihydro-1H-pyr azol-3-yl]-(4-cyclohexyl-piperazin-1-yl)-methanone | | |
| 7 | 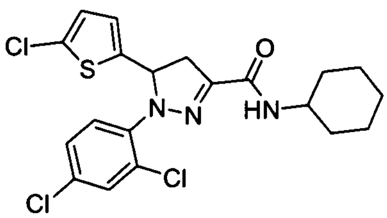 | 5-(5-Chloro-thiophen-2-yl)-1-(2,4-d ichloro-phenyl)-4,5-dihydro-1H-pyra zole-3-carboxylic acid cyclohexylamide | | |
| 8 | 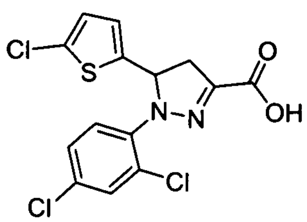 | 5-(5-Chloro-thiophen-2-yl)-1-(2,4-d ichloro-phenyl)-4,5-dihydro-1H-pyra zole-3-carboxylic acid | 1 H NMR (400 MHz, CHLOROFORM-d) δ ppm 3.39 (dd, *J*=17.88, 4.59 Hz, 1H) 3.64 (dd, *J*=17.88, 11.92 Hz, 1 H) 6.11 (dd, J=11.92, 4.59 Hz, 1H) 6.60 (d, *J*=3.78 Hz, 1H) 6.61 (d, *J*=3.78 Hz, 1H) 7.12 (dd, *J*=8.60, 2.15 Hz, 1 H) 7.25 (d, J=8.60 Hz, 1 H) 7.32 (d, *J*= 2.15 Hz, 1H) | 375 |

(continued)

| N° | STRUCTURE | Name | $^1$H-NMR | MS (M+H)$^+$ |
|---|---|---|---|---|
| 9 | 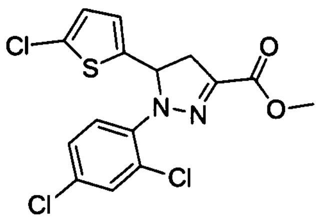 | 5-(5-Chloro-thiophen-2-yl)-1-(2,4-d ichloro-phenyl)-4,5-dihydro-1H-pyra zole-3-carboxylic acid methyl ester | | |
| 10 | 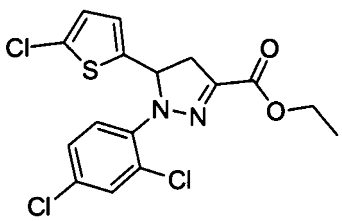 | 5-(5-Chloro-thiophen-2-yl)-1-(2,4-d ichloro-phenyl)-4,5-dihydro-1H-pyra zole-3-carboxylic acid ethyl ester | | |
| 11 | 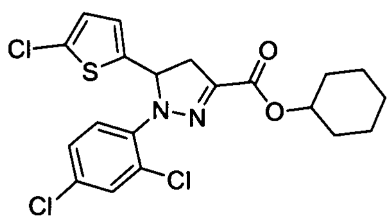 | 5-(5-Chloro-thiophen-2-yl)-1-(2,4-d chloro-phenyl)-4,5-dihydro-1H-pyra zole-3-carboxylic acid cyclohexyl ester | | |
| 12 | 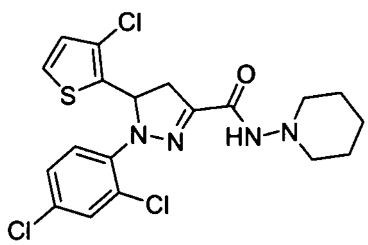 | 5-(3-Chloro-thiophen-2-yl)-1-(2,4-d ichloro-phenyl)-4,5-dihydro-1H-pyra zole-3-carboxylic acid piperidin-1-ylamide | | |
| 13 | 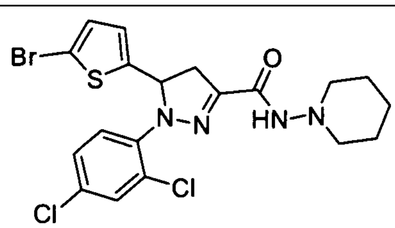 | 5-(5-Bromo-thiophen-2-yl)-1-(2,4-di chloro-phenyl)-4,5-dihydro-1H-pyrazole-3-carboxylic acid piperidin-1-ylamide | | |
| 14 | 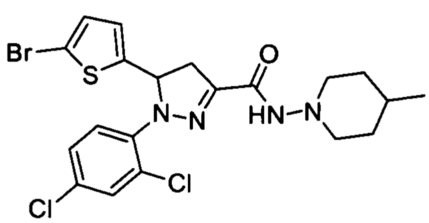 | 5-(5-Bromo-thiophen-2-yl)-1-(2,4-di chloro-phenyl)-4,5-dihydro-1H-pyrazole-3-carboxylic acid (4-methyl-piperidin-1-yl)-amide | | |

(continued)

| N° | STRUCTURE | Name | 1H-NMR | MS (M+H)+ |
|---|---|---|---|---|
| 15 | 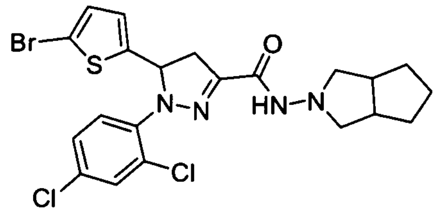 | 5-(5-Bromo-thiophen-2-yl)-1-(2,4-di chloro-phenyl)-4,5-dihydro-1H-pyrazole-3-carboxylic acid (hexahydro-cyclopenta[c]pyrrol-2-yl)-amide | | |
| 16 | 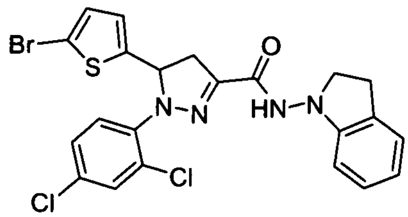 | 5-(5-Bromo-thiophen-2-yl)-1-(2,4-di chloro-phenyl)-4,5-dihydro-1H-pyrazole-3-carboxylic acid (2,3-dihydroindol-1-yl)-amide | | |
| 17 | 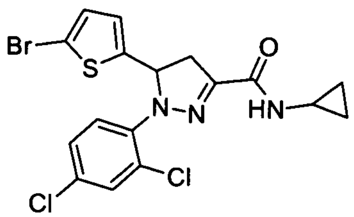 | 5-(5-Bromo-thiophen-2-yl)-1-(2,4-di chloro-phenyl)-4,5-dihydro-1H-pyrazole-3-carboxylic acid cyclopropylamide | | |
| 18 | 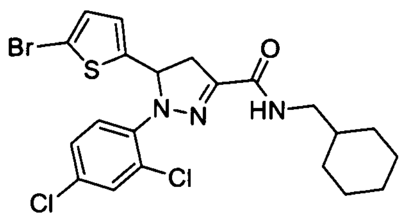 | 5-(5-Bromo-thiophen-2-yl)-1-(2,4-di chloro-phenyl)-4,5-dihydro-1H-pyrazole-3-carboxylic acid cyclohexylmethyl-amide | | |
| 19 | 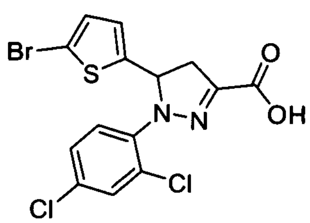 | 5-(5-Bromo-thiophen-2-yl)-1-(2,4-di chloro-phenyl)-4,5-dihydro-1H-pyrazole-3-carboxylic acid | | |
| 20 | 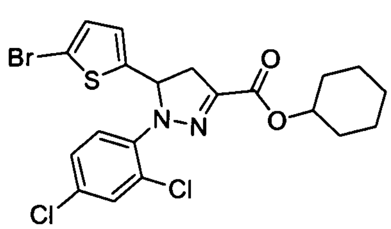 | 5-(5-Bromo-thiophen-2-yl)-1-(2,4-di chloro-phenyl)-4,5-dihydro-1H-pyrazole-3-carboxylic acid cyclohexyl ester | | |

(continued)

| N° | STRUCTURE | Name | $^1$H-NMR | MS (M+H)$^+$ |
|---|---|---|---|---|
| 21 | 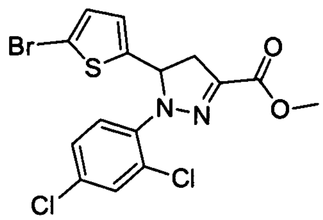 | 5-(5-Bromo-thiophen-2-yl)-1-(2,4-di chloro-phenyl)-4,5-dihydro-1H-pyrazole-3-carboxylic acid methyl ester | | |
| 22 | 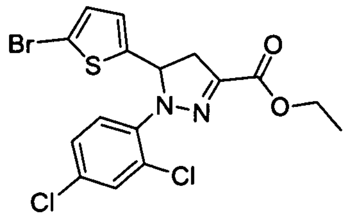 | 5-(5-Bromo-thiophen-2-yl)-1-(2,4-di chloro-phenyl)-4,5-dihydro-1H-pyrazole-3-carboxylic acid ethyl ester | | |
| 23 | 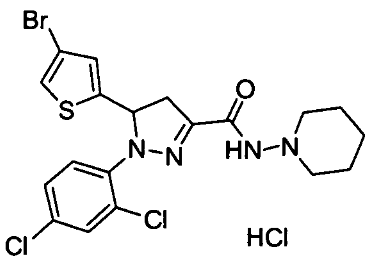 | 5-(4-Bromo-thiophen-2-yl)-1-(2,4-di chloro-phenyl)-4,5-dihydro-1H-pyrazole-3-carboxylic acid piperidin-1-ylamide hydrochloride | $^1$H NMR (300 MHz, DMSO-$d_6$) δ ppm 1.42 (m, 2 H) 1.72 (m, 4 H) 3.15 (m, 4 H) 3.25 (dd, *J*=18.02, 5.13 Hz, 1 H) 3.68 (dd, *J*=18.02, 11.50 Hz, 1 H) 6.08 (dd, *J*=11.50, 5.13 Hz, 1 H) 6.95 (d, *J*=1.17 Hz, 1 H) 7.34 (m, 1 H) 7.46 (m.2H) 7.58 (d, *J*=2.20 Hz. | 501 |
| 24 | 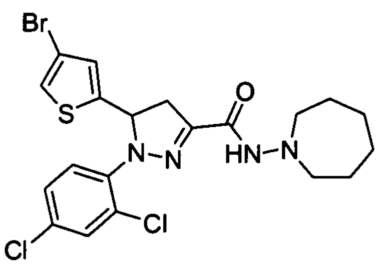 | 5-(4-Bromo-thiophen-2-yl)-1-(2,4-di chloro-phenyl)-4,5-dihydro-1H-pyrazole-3-carboxylic acid azepan-1-ylamide | | |
| 25 | 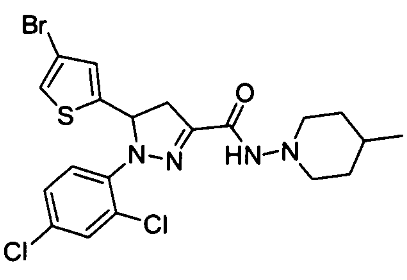 | 5-(4-Bromo-thiophen-2-yl)-1-(2,4-di chloro-phenyl)-4,5-dihydro-1H-pyrazole-3-carboxylic acid (4-methyl-piperidin-1-yl)-amide | | |

(continued)

| N° | STRUCTURE | Name | $^1$H-NMR | MS (M+H)$^+$ |
|---|---|---|---|---|
| 26 | 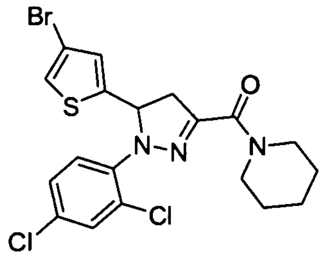 | [5-(4-Bromo-thiophen-2-yl)-1-(2,4-d ichloro-phenyl)-4,5-dihydro-1H-pyra zol-3-yl]-piperidin-1-yl-methanone | | |
| 27 | 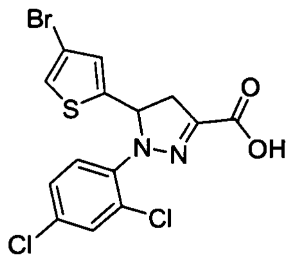 | 5-(4-Bromo-thiophen-2-yl)-1- (2,4-di chloro-phenyl)-4,5-dihydro-1H-pyrazole-3-carboxylic acid | 1H NMR (400 MHz, CHLOROFORM-*d*) δ ppm 3.42 (dd, *J*=18.07, 4.98 Hz, 1 H) 3.69 (dd, *J*=18.07, 12.02 Hz, 1 H) 6.14 (dd, 3.69 *J*=12.02, 4.98 Hz, 1 H) 6.77 (d, *J*=1.37 Hz, 1H) 7.04 (d, *J*=1.37 Hz, 1H) 7.13 (dd, *J*=8.60, 2.15 Hz, 1H) 7.20 (d, J=8.60 Hz, 1 H)7.35 (d, *J*=2.15 Hz, 1 H) | 419 |
| 28 | 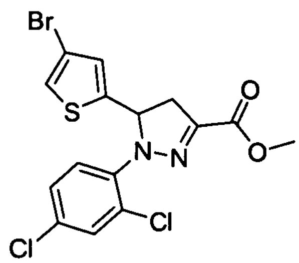 | 5-(4-Bromo-thiophen-2-yl)-1-(2,4-di chloro-phenyl)-4,5-dihydro-1H-pyrazole-3-carboxylic acid methyl ester | | |
| 29 | 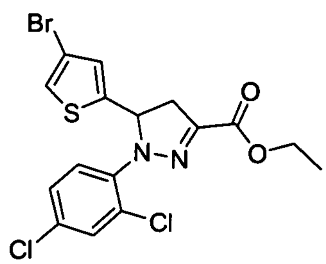 | 5-(4-Bromo-thiophen-2-yl)-1-(2,4-di chloro-phenyl)-4,5-dihydro-1H-pyrazole-3-carboxylic acid ethyl ester | | |
| 30 | 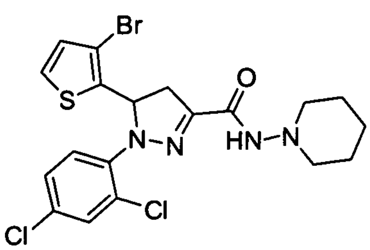 | 5-(3-Bromo-thiophen-2-yl)-1-(2,4-di chloro-phenyl)-4,5-dihydro-1H-pyrazole-3-carboxylic acid piperidin-1-ylamide | | |

(continued)

| N° | STRUCTURE | Name | $^1$H-NMR | MS (M+H)$^+$ |
|---|---|---|---|---|
| 31 | 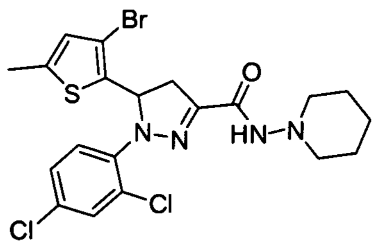 | 5-(3-Bromo-5-methyl-thiophen-2-yl)-1-(2,4-dichloro-phenyl)-4,5-dihydro-1H-pyrazole-3-carboxylic acid piperidin-1-ylamide | | |
| 32 | 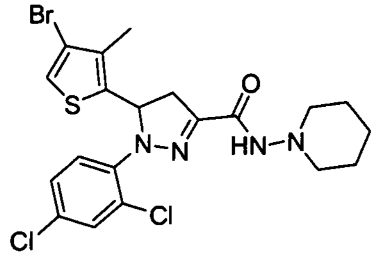 | 5-(4-Bromo-3-methyl-thiophen-2-yl)-1-(2,4-dichloro-phenyl)-4,5-dihydro-1H-pyrazole-3-carboxylic acid piperidin-1-ylamide | | |
| 33 | 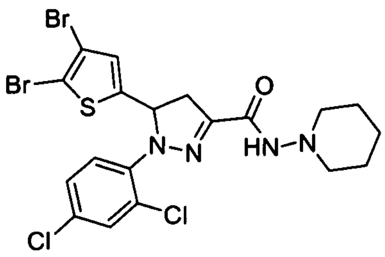 | 5-(4,5-Dibromo-thiophen-2-yl)-1-(2, 4-dichloro-phenyl)-4,5-dihydro-1H-p yrazole-3-carboxylic acid piperidin-1-ylamide | | |
| 34 | 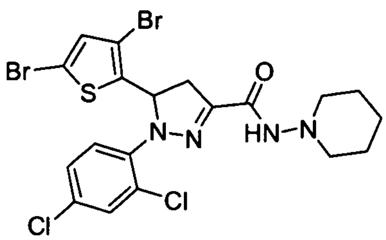 | 5-(3,5-Dibromo-thiophen-2-yl)-1-(2, 4-dichloro-phenyl)-4,5-dihydro-1H-p yrazole-3-carboxylic acid piperidin-1-ylamide | | |
| 35 | 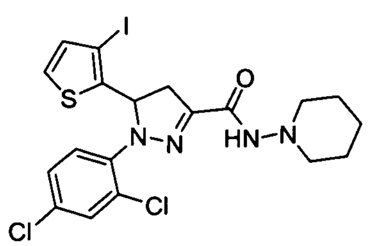 | 1-(2,4-Dichloro-phenyl)-5-(3-iodo-t hiophen-2-yl)-4,5-dihydro-1H-pyrazole-3-carboxylic acid piperidin-1-ylamide | | |
| 36 | 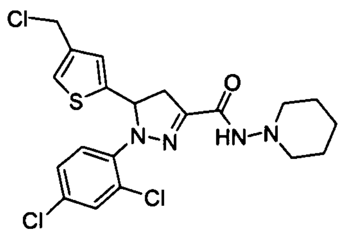 | 5-(4-Chloromethyl-thiophen-2-yl)-1-(2,4-dichloro-phenyl)-4,5-dihydro-1H-pyrazole-3-carboxylic acid piperi din-1-ylamide | | |

(continued)

| N° | STRUCTURE | Name | 1H-NMR | MS (M+H)+ |
|---|---|---|---|---|
| 37 | 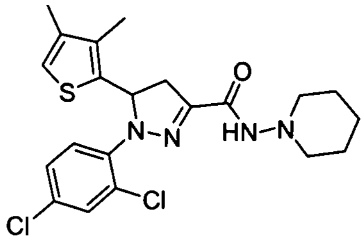 | 1-(2,4-Dichloro-phenyl)-5-(3,4-dimethyl-thiophen-2-yl)-4,5-dihydro-1H-pyrazole-3-carboxylic acid piperidin-1-ylamide | | |
| 38 | 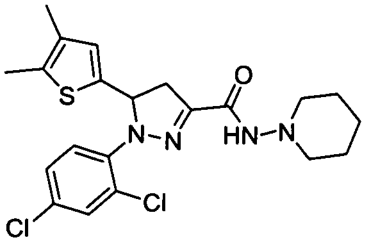 | 1-(2,4-Dichloro-phenyl)-5-(4,5-dimethyl-thiophen-2-yl)-4,5-dihydro-1H-pyrazole-3-carboxylic acid piperidin-1-ylamide | | |
| 39 | 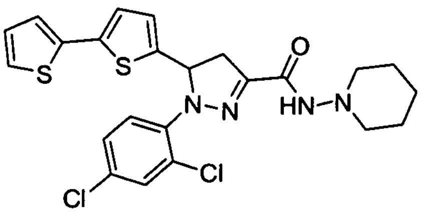 | 5-[2,2']Bithiophenyl-5-yl-1-(2,4-di chloro-phenyl)-4,5-dihydro-1H-pyrazole-3-carboxylic acid piperidin-1-ylamide | | |
| 40 | 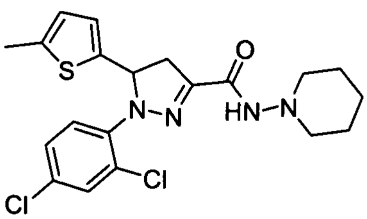 | 1-(2,4-Dichloro-phenyl)-5-(5-methyl-thiophen-2-yl)-4,5-dihydro-1 H-pyra zole-3-carboxylic acid piperidin-1-ylamide | | |
| 41 | 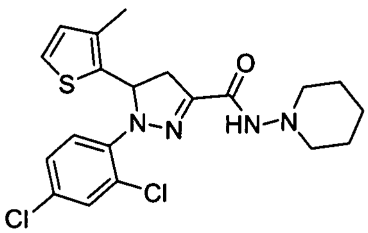 | 1-(2,4-Dichloro-phenyl)-5-(3-methyl-thiophen-2-yl)-4,5-dihydro-1H-pyra zole-3-carboxylic acid piperidin-1-ylamide | | |
| 42 | 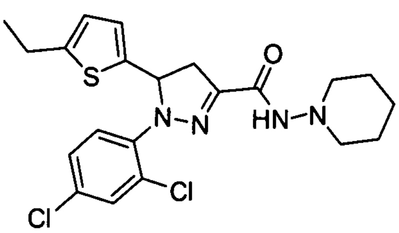 | 1-(2,4-Dichloro-phenyl)-5-(5-ethyl-thiophen-2-yl)-4,5-dihydro-1H-pyraz ole-3-carboxylic acid piperidin-1-ylamide | | |

(continued)

| N° | STRUCTURE | Name | $^1$H-NMR | MS (M+H)$^+$ |
|---|---|---|---|---|
| 43 | 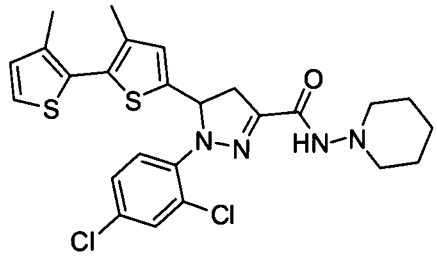 | 1-(2,4-Dichloro-phenyl)-5-(3,3'-dimethyl-[2,2']bithiophenyl-5-yl)-4,5-dihydro-1H-pyrazole-3-carboxylic acid piperidin-1-ylamide | | |
| 44 | 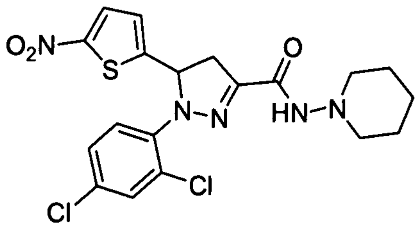 | 1-(2,4-Dichloro-phenyl)-5-(5-nitro-thiophen-2-yl)-4,5-dihydro-1H-pyrazole-3-carboxylic acid piperidin-1-ylamide | | |
| 45 | 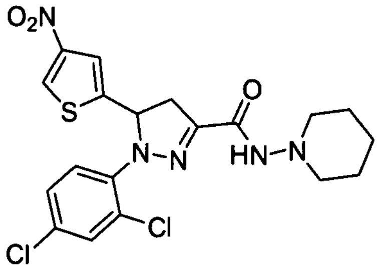 | 1-(2,4-Dichloro-phenyl)-5-(4-nitro-thiophen-2-yl)-4,5-dihydro-1H-pyrazole-3-carboxylic acid piperidin-1-ylamide | | |
| 46 | 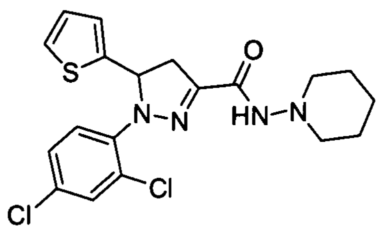 | 1-(2,4-Dichloro-phenyl)-5-thiophen-2-yl-4,5-dihydro-1H-pyrazole-3-carboxylic acid piperidin-1-ylamide | | |
| 47 | 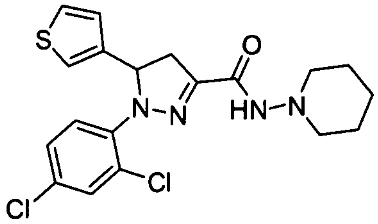 | 1-(2,4-Dichloro-phenyl)-5-thiophen-3-yl-4,5-dihydro-1H-pyrazole-3-carboxylic acid piperidin-1-ylamide | | |
| 48 | 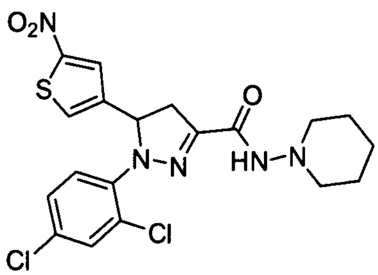 | 1-(2,4-Dichloro-phenyl)-5-(5-nitro-thiophen-3-yl)-4,5-dihydro-1H-pyraz ole-3-carboxylic acid piperidin-1-ylamide | | |

## SUBGROUP A4

### Subgroup A4-Chemical Examples

The following compounds are prepared according to the processes described above. Those skilled in the art are familiar with the starting materials that are needed to obtain said compounds

| N° | STRUCTURE | Name | $^1$H-NMR | MS (M+H)$^+$ |
|---|---|---|---|---|
| 1 | 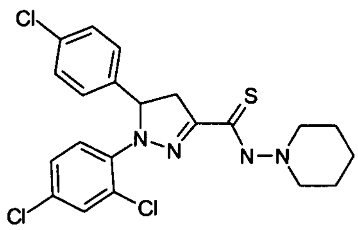 | 5-(4-Chloro-phenyl)-1-(2,4-dichloro -phenyl)-4,5-dihydro-1H-pyrazole-3-carbothioic acid piperidin-1-ylamid e | | |
| 2 | 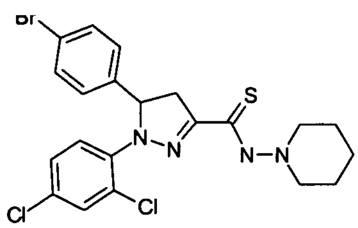 | 5-(4-Bromo-phenyl)-1-(2,4-dichloro-phenyl)-4,5-dihydro-1H-pyrazole-3-c arbothioic acid piperidin-1-ylamide | | |
| 3 | 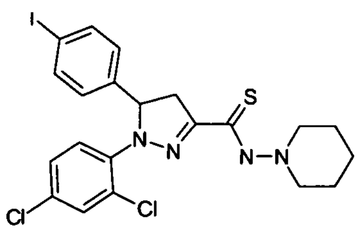 | 1-(2,4-Dichloro-phenyl)-5-(4-iodo-p henyl)-4,5-dihydro-1H-pyrazole-3-ca rbothioic acid piperidin-1-ylamide | | |
| 4 | 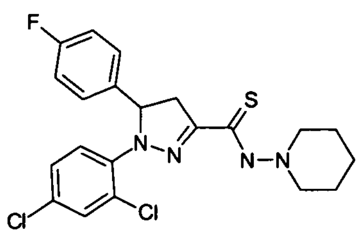 | 1-(2,4-Dichloro-phenyl)-5-(4-fluoro -phenyl)-4,5-dihydro-1 H-pyrazole-3-carbothioic acid piperidin-1-ylamide | | |
| 5 | 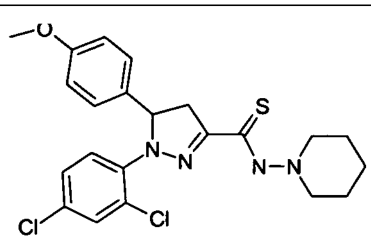 | 1-(2,4-Dichloro-phenyl)-5-(4-methox y-phenyl)-4,5-dihydro-1H-pyrazole-3 -carbothioic acid piperidin-1-ylami de | | |

(continued)

| N° | STRUCTURE | Name | 1H-NMR | MS (M+H)+ |
|---|---|---|---|---|
| 6 | 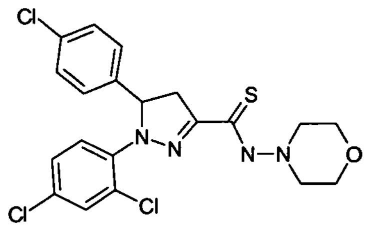 | 5-(4-Chloro-phenyl)-1-(2,4-dichloro -phenyl)-4,5-dihydro-1H-pyrazole-3-carbothioic acid morpholin-4-ylamid e | | |
| 7 | 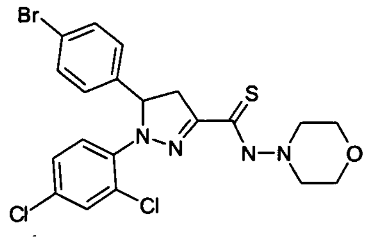 | 5-(4-Bromo-phenyl)-1-(2,4-dichloro-phenyl)-4,5-dihydro-1H-pyrazole-3-c arbothioic acid morpholin-4-ylamide | | |
| 8 | 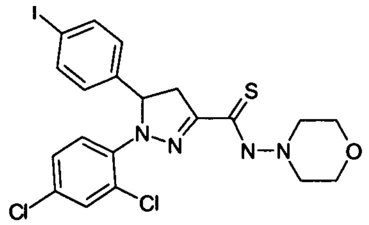 | 1-(2,4-Dichloro-phenyl)-5-(4-iodo-p henyl)-4,5-dihydro-1H-pyrazole-3-ca rbothioic acid morpholin-4-ylamide | | |
| 9 | 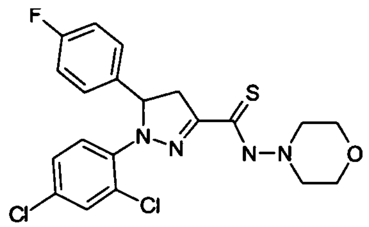 | 1-(2,4-Dichloro-phenyl)-5-(4-fluoro -phenyl)-4,5-dihydro-1H-pyrazole-3-carbothioic acid morpholin-4-ylamid e | | |
| 10 | 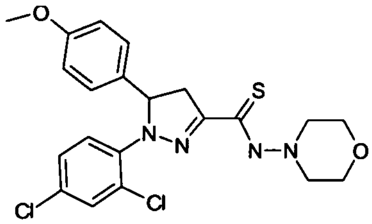 | 1-(2,4-Dichloro-phenyl)-5-(4-methox y-phenyl)-4,5-dihydro-1H-pyrazole-3 -carbothioic acid morpholin-4-ylami de | | |
| 11 | 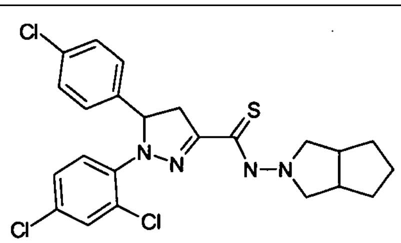 | 5-(4-Chloro-phenyl)-1-(2,4-dichloro -phenyl)-4,5-dihydro-1H-pyrazole-3-carbothioic acid (hexahydro-cyclope nta[c]pyrrol-2-yl)-amide | | |

(continued)

| N° | STRUCTURE | Name | $^1$H-NMR | MS $(M+H)^+$ |
|---|---|---|---|---|
| 12 | 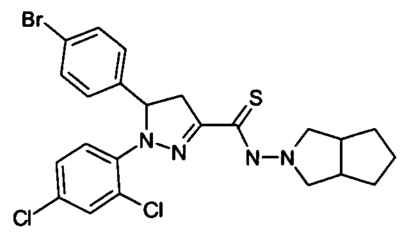 | 5-(4-Bromo-phenyl)-1-(2,4-dichloro-phenyl)-4,5-dihydro-1H-pyrazole-3-c arbothioic acid (hexahydro-cyclopen ta[c]pyrrol-2-yl)-amide | | |
| 13 | 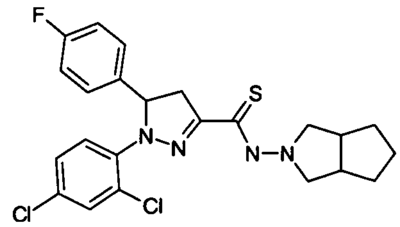 | 1-(2,4-Dichloro-phenyl)-5-(4-fluoro -phenyl)-4,5-dihydro-1H-pyrazole-3-carbothioic acid (hexahydro-cyclope nta[c]pyrrol-2-yl)-amide | | |
| 14 | 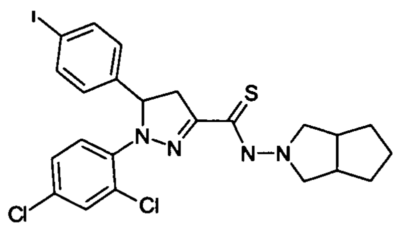 | 1-(2,4-Dichloro-phenyl)-5-(4-iodo-p henyl)-4,5-dihydro-1H-pyrazole-3-ca rbothioic acid (hexahydro-cyclopent a[c]pyrrol-2-yl)-amide | | |
| 15 | 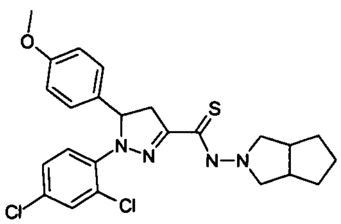 | 1-(2,4-Dichloro-phenyl)-5-(4-methox y-phenyl)-4,5-dihydro-1H-pyrazole-3 -carbothioic acid (hexahydro-cyclop enta[c]pyrrol-2-yl)-amide | | |
| 16 | 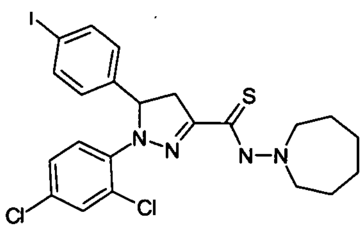 | 1-(2,4-Dichloro-phenyl)-5-(4-iodo-p henyl)-4,5-dihydro-1H-pyrazole-3-ca rbothioic acid azepan-1-ylamide | | |
| 17 | 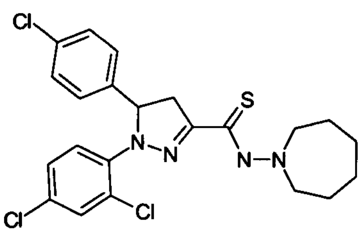 | 5-(4-Chloro-phenyl)-1-(2,4-dichloro -phenyl)-4,5-dihydro-1H-pyrazole-3-carbothioic acid azepan-1-ylamide | | |

(continued)

| N° | STRUCTURE | Name | $^1$H-NMR | MS (M+H)$^+$ |
|---|---|---|---|---|
| 18 | 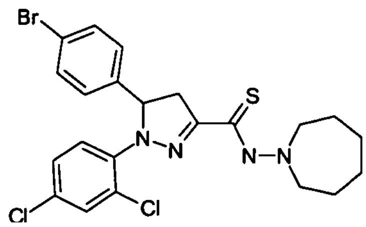 | 5-(4-Bromo-phenyl)-1-(2,4-dichloro-phenyl)-4,5-dihydro-1H-pyrazole-3-c arbothioic acid azepan-1-ylamide | | |
| 19 | 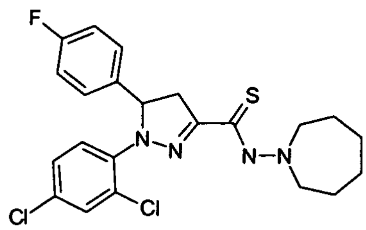 | 1-(2,4-Dichloro-phenyl)-5-(4-fluoro -phenyl)-4,5-dihydro-1H-pyrazole-3-carbothioic acid azepan-1-ylamide | | |
| 20 | 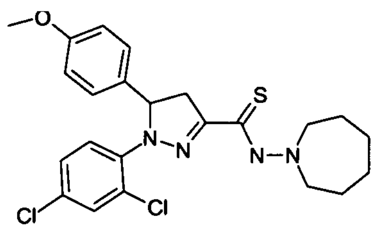 | 1-(2,4-Dichloro-phenyl)-5-(4-methox y-phenyl)-4,5-dihydro-1 H-pyrazole-3 -carbothioic acid azepan-1-ylamide | | |
| 21 | 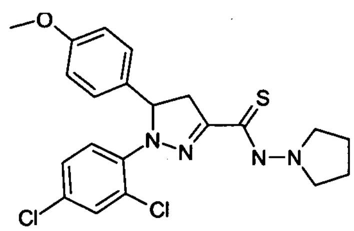 | 1-(2,4-Dichloro-phenyl)-5-(4-methox y-phenyl)-4,5-dihydro-1H-pyrazole-3 -carbothioic acid pyrrolidin-1-ylam ide | | |
| 22 | 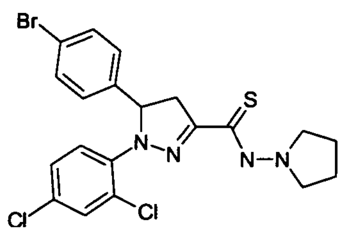 | 5-(4-Bromo-phenyl)-1-(2,4-dichloro-phenyl)-4,5-dihydro-1H-pyrazole-3-c arbothioic acid pyrrolidin-1-ylamid e | | |
| 23 | 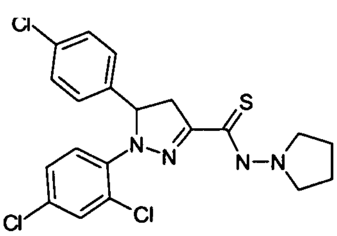 | 5-(4-Chloro-phenyl)-1-(2,4-dichloro -phenyl)-4,5-dihydro-1H-pyrazole-3-carbothioic acid pyrrolidin-1-ylami de | | |

(continued)

| N° | STRUCTURE | Name | 1H-NMR | MS (M+H)+ |
|---|---|---|---|---|
| 24 | 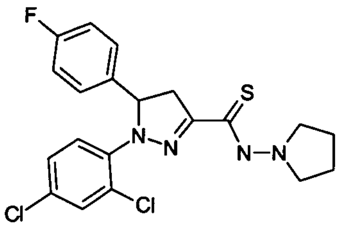 | 1-(2,4-Dichloro-phenyl)-5-(4-fluoro -phenyl)-4,5-dihydro-1H-pyrazole-3-carbothioic acid pyrrolidin-1-ylami de | | |
| 25 | 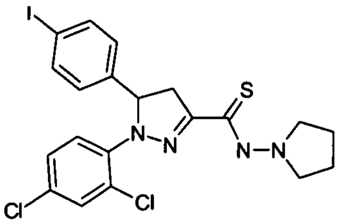 | 1-(2,4-Dichloro-phenyl)-5-(4-iodo-p henyl)-4,5-dihydro-1H-pyrazole-3-ca rbothioic acid pyrrolidin-1-ylamide | | |
| 26 | 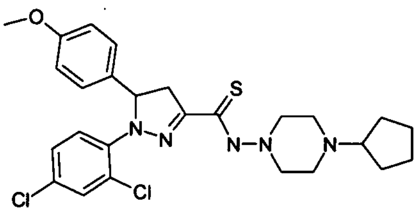 | 1-(2,4-Dichloro-phenyl)-5-(4-methox y-phenyl)-4,5-dihydro-1 H-pyrazole-3 -carbothioic acid (4-cyclopentyl-pi perazin-1-yl)-amide | | |
| 27 | 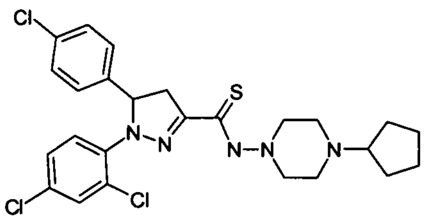 | 5-(4-Chloro-phenyl)-1-(2,4-dichloro -phenyl)-4,5-dihydro-1 H-pyrazole-3-carbothioic acid (4-cyclopentyl-pip erazin-1-yl)-amide | | |
| 28 | 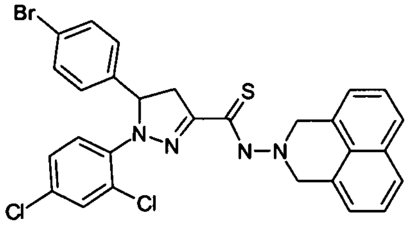 | 5-(4-Bromo-phenyl)-1-(2,4-dichloro-phenyl)-4,5-dihydro-1H-pyrazole-3-c arbothioic acid (1 H, 3H-benzo[de]iso quinolin-2-yl)-amide | | |
| 29 | 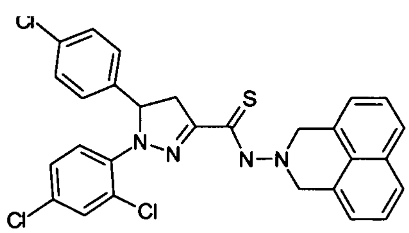 | 5-( 4-Chloro-phenyl)-1-(2,4-dichloro -phenyl)-4,5-dihydro-1 H-pyrazole-3-carbothioic acid (1H,3H-benzo[de]is oquinolin-2-yl)-amide | | |

(continued)

| N° | STRUCTURE | Name | $^1$H-NMR | MS (M+H)$^+$ |
|---|---|---|---|---|
| 30 | 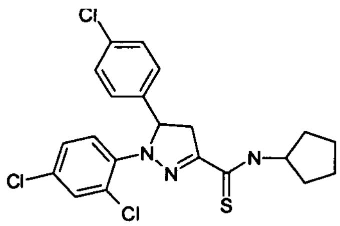 | 5-(4-Chloro-phenyl)-1-(2,4-dichloro -phenyl)-4,5-dihydro-1H-pyrazole-3-carbothioic acid cyclopentylamide | | |
| 31 | 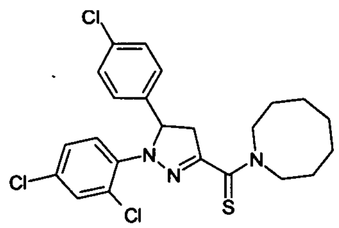 | Azocan-1-yl-[5-(4-chloro-phenyl)-1-(2,4-dichloro-phenyl)-4,5-dihydro-1H-pyrazol-3-yl]-methanethione | | |
| 32 | 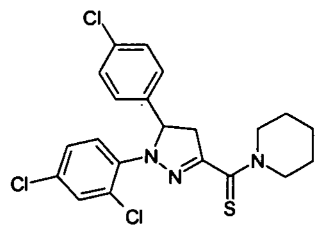 | [5-(4-Chloro-phenyl)-1-(2,4-dichlor o-phenyl)-4,5-dihydro-1 H-pyrazol-3-yl]-piperidin-1-yl-methanethione | | |

## SUBGROUP A5.1

**Subgroup A5.1: Chemical**

**Preparation of 4-(4-chlorophenyl)-2-oxo-3-butenoic acid**

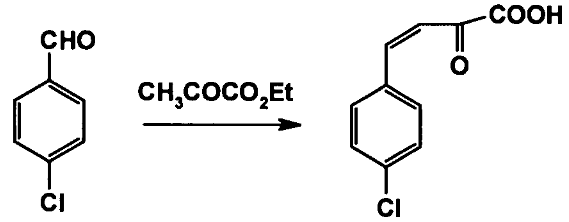


p-Chlorobenzaldehyde (13,3 g, 95 mmol) and ethyl pyruvate (10 g, 86 mmol) were dissolved in 150 mL of absolute ethanol. The solution was cooled to 0°C and an aqueous solution of NaOH (3.8 g in 45 mL water) was added drop wise keeping the temperature below or equal to 10°C, whereby a yellow-orange coloured precipitate was formed. The reaction mixture was stirred for 1 hour at 0°C and an additional 1.5 hours at room temperature (approximately 25 °C). Afterwards the reaction mixture was cooled down to approximately 5°C and the insoluble sodium salt of 4-(4-chlorophenyl)-2-oxo-3-butenoic acid was isolated by filtration.

The filtrate was left in the refrigerator overnight, whereby more precipitate was formed, which was filtered off, combined with the first fraction of the salt and washed with diethyl ether. The sodium salt of 4-(4-chlorophenyl)-2-oxo-3-butenoic acid was then treated with a solution of 2N HCl, stirred for some minutes and solid 4-(4-chlorophenyl)-2-oxo-3-butenoic acid was separated via filtration and dried to give 12.7 g of the desired product (70% of theoretical yield).

IR (KBr, $cm^{-1}$): 3500-2500, 1719,3, 1686,5, 1603,4, 1587,8, 1081,9.
$^{1}H$ NMR($CDCl_3$, 300 MHz) δ 7,4 (d, J=8,4Hz, 2H), 7,5 (d, J=16,1Hz, 1H), 7,6 (d, J=8,4Hz, 2H), 8,1(d, J=16,1 Hz, 1H).

**Preparation of 5-(4-chlorophenyl)-1-(2,4-dichlorophenyl)-4,5-dihydro-pyrazole-3-carboxylic acid**

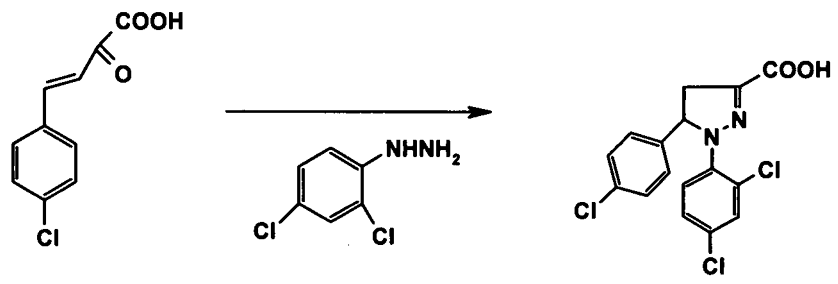

4-(4-Chlorophenyl)-2-oxo-3-butenoic acid (12.6 g, 60 mmol), 2,4-dichlorophenylhydrazine hydrochloride (12.8 g, 60 mmol) and glacial acetic acid (200 mL) were mixed under a nitrogen atmosphere and heated to reflux for 4 hours, cooled down to room temperature (approximately 25 °C) and poured into ice, whereby a sticky mass was obtained, which was extracted with methylene chloride. The combined methylene chloride fractions were washed with water, dried over sodium sulfate, filtered and evaporated to dryness to give a pale yellow solid (12.7 g, 57% of theoretical yield).

IR (KBr, $cm^{-1}$) : 3200-2200, 1668,4, 1458, 1251,4, 1104,8.
$^{1}H$ NMR ($CDCl_3$, 300 MHz) δ 3,3 (dd, 1 H), 3,7 (dd, 1H), 5,9 (dd, 1H), 7,09-7,25 (m, 7H).

Preparation of 5-(4-chlorophenyl)-1-(2,4-dichlorophenyl)-4,5-dihydro-pyrazole-3-carboxylic acid chloride

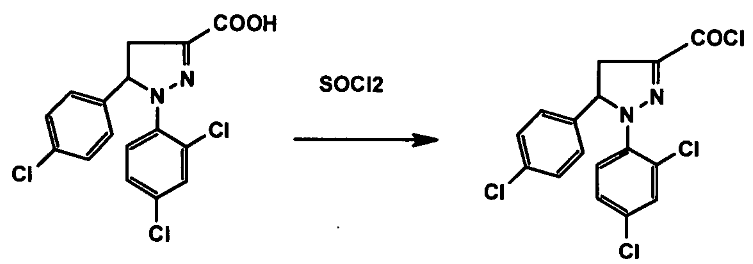


5-(4-Chlorophenyl)-1-(2,4-dichlorophenyl)-4,5-dihydro-pyrazole-3-carboxylic acid (5,54 g, 15 mmols) was dissolved in 120 mL of dry toluene and thionyl chloride (2.14g, 18 mmol) was added. The mixture was heated to 80 °C for 2.5 hours. The solvent was removed under reduced pressure and the resulting crude residue (6.06 g, 100 %) was used without any further purification.

IR (KBr, $cm^{-1}$) : 1732, 1700, 1533, 1478, 1212, 826

**Subgroup A5.1: Chemical Example 1: Preparation of 5-(4-Chloro-phenyl)-1-(2,4-dichloro-phenyl)-4,5-dihydro-1H-pyrazole-3-carboxylic acid piperidin-1-ylamide**

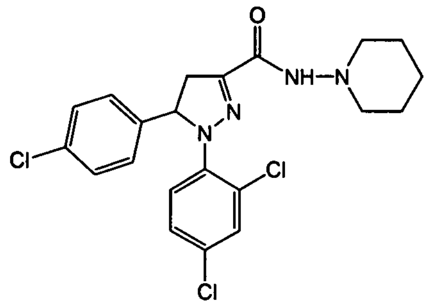

N-Aminopiperidine (0.6 mL, 5.6 mmol) and triethylamine (4 mL) were dissolved in methylene chloride (25 mL) under nitrogen atmosphere. The resulting mixture was - cooled to 0°C and a solution of 5-(4-chlorophenylyl-(2,4-dichlorophenyl)-4,5-dihydro-pyrazole-3-carboxylic acid chloride in methylene chloride (15 mL) was added drop wise. The resulting reaction mixture was stirred at room temperature (approximately 25 °C) overnight. The reaction mixture was washed with water, followed by a saturated aqueous solution of sodium bicarbonate, again with water, dried over sodium sulfate, filtered and evaporated to dryness in a rotavapor. The resulting crude solid was crystallised from ethanol. The crystallised solid was removed via filtration and the mother liquors were concentrated to yield a second fraction of crystallised product. The two fractions were combined to give a total amount of 1.7 g (57% of theoretical yield) of the title compound having a melting point of 183-186°C.

IR (KBr, $cm^{-1}$): 3222,9, 2934,9, 1647,4, 1474,7, 1268,3, 815,6.
$^1H$ NMR ($CDCl_3$, δ) : 1,4 (m, 2H), 1,7 (m, 4H), 2,8 (m, 4H), 3,3 (dd, J=6,1 y 18,3Hz, 1 H), 3,7 (dd, J=12,5 and 18,3 Hz, 1H), 5,7 (dd, J=6,1 and 12,5 Hz, 1H), 7,0-7,2 (m, 6H), 7,4 (s, 1H).

The following compounds were prepared according to the processes described for the preparation of compound 1 above. Those skilled in the art are familiar with the starting materials that are needed to obtain said compounds.

**Subgroup A5.1: Chemical Example 7: N-oxide of N-piperidinyl-5-(4-chlorophenyl)-1-(2,4-dichlorophenyl)-4,5-dihydropyrazole-3-carboxamide**

Under nitrogen gas as an inert atmosphere N-piperidinyl-5-(4-chlorophenyl)-1-(2,4-dichlorophenyl)-4,5-dihydropyrazole-3-carboxamide (0.15 g, 0.33 mmol) was dissolved in 7 mL of dichloromethane. The resulting solution was ice-cooled to 0 °C and m-chloroperbenzoic acid (0.204 g, 0.83 mmol) was added in several portions. After stirring for 15 minutes a control via thin layer chromatography showed that no starting material was present. A saturated solution of sodium bicarbonate was then slowly added, the organic phase separated, washed with water, dried over sodium sulfate and filtered. The filtered solution was evaporated to dryness and the crude product was purified via column chromatography yielding 78 mg (50 % of theoretical yield) of the N-oxide of N-piperidinyl-5-(4-chlorophenyl)-1-(2,4-dichlorophenyl)-4,5-dihydropyrazole-3-carboxamide in form of a white solid having a melting point of 115-120 °C.
IR (KBr, $cm^{-1}$): 3202, 1678, 1654, 1474, 1309, 1107.
$^1H$-NMR ($CDCl_3$, δ): 1.6 (m, 2H), 1.8-2.0 (m, 4H), 2.55 (m, 2H), 3.3 (dd, J = 6.3 Hz and 18.2 Hz, 1 H), 3.7 (m, 3H), 5.8 (dd, J = 6.3 Hz and 12.5 Hz, 1 H), 7.0-7.3 (m, 7H), 8.5 (s, 1H.)

**Subgroup A5.1: Chemical Example 120:[5-(4-Chloro-phenyl)-1-(2,4-dichloro-phenyl)-4,5-dihydro-1H-pyrazol-3-carboxylic acid ethyl ester**

5-(4-chlorophenyl)-1-(2,4-dichlorophenyl)-4,5-dihydro-pyrazole-3-carboxylic acid (0.55 g, 1.50 mmol) was dissolved in 20 mL of toluene and (2 mL) ethanol and (0.05 g) of p-toluenesulfonic acid monohydrate were added. The mixture was heated at 80 °C for 72 hours. After cooling to room temperature, the reaction mixture washed with sodium hydrogen carbonate solution and water, dried over sodium sulphate and evaporated to dryness to give the title compound in form of oil.

IR (film, $cm^{-1}$): 2981, 1728, 1704, 1478, 1246.

The compounds according to the following examples 68 to 172 have been prepared by the methods described for the preparation of Example 1. The other examples were prepared or can be prepared by the methods described above.

**Subgroup A5.1: Chemical Example 68: 5-(4-Bromo-phenyl)-1-(2,4-dichloro-phenyl)-4,5-dihydro-1H-pyrazole-3-carboxylic acid azepan-1-ylamide**

**Preparation of 4-(4-bromophenyl)-2-oxo-3-butenoic acid**

This compound was obtained following the same method described in Example 1 starting from p-bromobenzaldehyde.
$^1$H NMR (400 MHz, $CDCl_3$,) : δ7.55 (2H, m, ArH,), 7.61 (3H, m, Ar H), 8.09 (1 H, d, J 16.4 Hz)
$^{13}$C NMR (100 MHz, $CDCl_3$): δ 118.1 (CH), 127.2 (C), 130.7 (CH), 132.7 (CH), 149.7 (C + CH), 160.0 (CO), 182.2 (CO).

**Preparation of 5-(4-bromophenyl)-1-(2,4-dichlorophenyl)-4,5-dihydro-pyrazole-3-carboxylic acid**

IR (KBr, $cm^{-1}$) : 3200-2200, 1685, 1571,1549, 1480, 1112
$^1$H NMR (400 MHz, $CDCl_3$)) : δ 3.27 (1 H, dd, J 18.0, 6.4Hz, ArH,), 3.71 (1 H, dd, J 18, 12.8 Hz, Ar H), 5.88 (1 H, dd, J 12.8, 6.4Hz, CH), 7.02 (2H, d, J 7.6Hz, ArH), 7.08 (1 H, m, ArH), 7.19 (1 H, d J 8.4Hz, ArH), 7.26 (1 H, m, ArH), 7.37 (2H, d, 7.6 Hz, ArH)
$^{13}$C NMR (100 MHz, $CDCl_3$): δ 40.4 ($CH_2$), 67.4 (CH), 122.7 (C), 126.0 (CH), 126.5 (C), 127.6 (CH), 128.3 (CH), 130.2 (CH), 131.2 (C), 132.2 (CH), 138.4 (C), 138.5 (C), 140.2(C), 165.7(C).

**Preparation of 5-(4-bromophenyl)-1-(2,4-dichlorophenyl)-4,5-dihydro-pyrazole-3-carboxylic acid chloride**

IR (KBr, $cm^{-1}$) : 1737, 1534, 1477, 1212, 1127.

**Preparation of 5-(4-Bromo-phenyl)-1-(2,4-dichloro-phenyl)-4,5-dihydro-1H-pyrazole-3-carboxylic acid azepan-1-ylamide.**

This compound was obtained following the same method described in Example 1 starting from 5-(4-bromophenyl)-1-(2,4-dichlorophenyl)-4,5-dihydro-pyrazole-3-carboxylic acid chloride and azepan-1-amine.
$^1$H NMR (300 MHz, CHLOROFORM-*d*) δ ppm 1.75 (br. s., 4 H) 2.00 (br. s., 4 H) 3.26 (dd, *J*=18.16, 6.15 Hz, 1 H) 3.71 (dd, *J*=18.16, 12.45 Hz, 1 H) 3.65 (br. s., 4 H) 5.82 (dd, *J*=12.45, 6.15 Hz, 1 H) 6.97 (d, *J*=8.35 Hz, 2 H) 7.10 (d, *J*=2.20 Hz, 1 H) 7.16 - 7.22 (m, 1 H) 7.24 (d, *J*=2.20 Hz, 1 H) 7.35 (d, *J*=8.50 Hz, 2 H) MS $(M+H)^+$: 510

**Subgroup A5.1: Chemical Example 96: 1-(2,4-Dichloro-phenyl)-5-(4-fluoro-phenyl)-4,5-dihydro-1H-pyrazole-3-carboxylic acid azepan-1-ylamide**

**Preparation of 4-(4-fluorophenyl)-2-oxo-3-butenoic acid**

$^1$H NMR (400 MHz, $CDCl_3$,): δ 7.15 (2H, apt, J 8.4 Hz, ArH,), 7.53 (1 H, d, J 16.0Hz, CH), 7.70 (2H, m, ArH), 8.11(1H, d, J 16.0 Hz)
$^{13}$C NMR (100 MHz, $CDCl_3$): δ 116.5 (CH, d, $J_F$ 24 Hz), 117.4 (CH, d, $J_F$ 8.7 Hz), 130.1 (C, d, $J_F$ 2.4 Hz), 131.7 (CH, d, $J_F$ 9.1Hz), 149.9 (CH), 162.0 (C, d, $J_F$ 320 Hz), 166.7 (CO), 182.4 (CO).

**Preparation of 5-(4-fluorophenyl)-1-(2,4-dichlorophenyl)-4,5-dihydro-pyrazole-3-carboxylic acid**

IR (KBr, $cm^{-1}$): 3200-2200, 1687.5, 1478, 1230, 1107
$^1$H NMR (400 MHz, $CDCl_3$): δ 3.30 (1 H, dd, J 18.0, 6.4Hz, ArH,), 3.72 (1 H, dd, J 18, 12.8 Hz, Ar H), 5.93 (1 H, dd, J 12.8, 6.4 Hz, CH), 6.92 (2H, t, J 8.4Hz, ArH), 7.09-7.12 (3H, m, ArH), 7.22 (1 H, bs, ArH), 7.26 (1 H, m, ArH)
$^{13}$C NMR (100 MHz, $CDCl_3$): δ 40.7 ($CH_2$), 67.8 (CH), 115.9 (CH, d, $J_F$ 21 Hz), 126.1 (CH), 126.5 (C), 127.6 (CH), 128.3 (CH, d, $J_F$ 8.2 Hz), 130.1 (CH), 131.2 (C), 135.1 (C, d, $J_F$ 3Hz ), 138.6 (C), 140.1 (C), 160.6 (C, d, $J_F$ 240 Hz), 166.3 (C).

**Preparation of 5-(4-fluorophenyl)-1-(2,4-dichlorophenyl)-4,5-dihydro-pyrazole-3-carboxylic acid chloride**

IR (KBr, $cm^{-1}$) : 1733, 1548, 1511, 1478, 1212, 832.

**Preparation of 1-(2,4-Dichloro-phenyl)-5-(4-fluoro-phenyl)-4,5-dihydro-1H-pyrazole-3-carboxylic acid azepan-1-ylamide**

1H NMR (300 MHz, CHLOROFORM-*d*) δ ppm 1.75 (br. s., 4 H) 2.00 (br. s., 4 H) 3.28 (dd, *J*=18.16, 6.08 Hz,

1 H) 3.68 (dd, *J*=18.16, 12.38 Hz, 1 H) 3.68 (br. s., 4 H) 5.84 (dd, *J*=12.38, 6.08 Hz, 1 H) 6.90 (t, *J*=8.57 Hz, 2 H) 7.08 (m, 3 H) 7.14 - 7.19 (m, 1 H) 7.24 (d, J=2.20 Hz, 1 H)
MS $(M+H)^+$: 449

**Subgroup A5.1: Chemical Example 106: 1-(2,4-Dichloro-phenyl)-5-(4-methoxyphenyl)-4,5-dihydro-1H-pyrazole-3-carboxylic acid piperidin-1-ylamide** hydrochloride

**Preparation of 4-(4-methoxyphenyl)-2-oxo-3-butenoic acid**

$^1$H NMR (400 MHz, $CDCl_3$): δ 3.88 (3H, s, $OCH_3$), 6.95 (2H, d, J 6.8 Hz), 7.45 (2H, d, J 15.6 H), 7.65 (2H, d, J 6.8 Hz), 8.09 (2H, d, J 15.6 Hz).

**Preparation of 5-(4-methoxyphenyl)-1-(2,4-dichlorophenyl)-4,5-dihydro-pyrazole-3-carboxylic acid**

IR (KBr, $cm^{-1}$) 3200-2200, 1685, 1513, 1478, 1248, 1105
$^1$H NMR (400 MHz, $CDCl_3$): δ 1.45 (2H, m, $CH_2$), 1.75 (4H, m), 2.84 (4H, m), 3.45 (1 H, dd J 4.8, 18Hz), 3.61 (1 H, dd J 11.2, 19.6 Hz), 5.93 (1 H, dd J 4.8, 11.2 Hz), 6.57 (2H, ap s), 7.13 (2H, m), 7.32 (1 H, m), 7.40 (1 H, m)
$^{13}$C NMR (100 MHz, $CDCl_3$): δ 23.4 ($CH_2$), 25.3 ($CH_2$), 40.5 ($CH_2$), 57.3 ($CH_2$), 63.7 (CH), 125.7 (CH), 125.8 (CH), 126.1 (CH), 127.1 (C), 127.8 (CH), 130.1 (CH), 131.1 (C), 139.6 (C), 139.9 (C), 146.1 (C), 158.9 (CO).

**Preparation of 5-(4-methoxyphenyl)-1-(2,4-dichlorophenyl)-4,5-dihydro-pyrazole-3-carboxylic acid chloride**

IR (KBr, $cm^{-1}$): 1735, 1513, 1477, 1249, 1129

**Preparation of 1-(2,4-Dichloro-phenyl)-5-(4-methoxy-phenyl)-4,5-dihydro-1H-pyrazole-3-carboxylic acid piperidin-1-ylamide hydrochloride**

$^1$H NMR (300 MHz, CHLOROFORM-*d*) δ ppm 1.67 (br. s., 2 H) 2.10 (br. s., 4 H) 3.25 (dd, *J*=18.02, 6.01 Hz, 1 H) 3.64 (dd, *J*=18.02, 12.60 Hz, 1 H) 3.72 (s, 3 H) 3.89 (br. s., 4 H) 5.85 (dd, *J*=12.60, 6.01 Hz, 1 H) 6.72 (d, *J*=8.79 Hz, 2 H) 7.00 (d, *J*=8.64 Hz, 2 H) 7.07 (dd, *J*=8.64, 2.20 Hz, 1 H) 7.22 (d, *J*=8.64 Hz, 1 H) 7.22 (d, *J*=2.20 Hz, 1 H) 9.63 (br. s., 1 H)
MS $(M+H)^+$: 447

**Subgroup A5.1: Chemical Example 109: 1-(2,4-Dichloro-phenyl)-5-(4-methoxyphenyl)-4,5-dihydro-1H-pyrazole-3-carboxylic acid (hexahydro-cyclopenta[c]pyrrol-2-yl)-amide hydrochloride**

$^1$H NMR (300 MHz, CHLOROFORM-*d*) δ ppm 1.61 - 1.91 (m, 6 H) 2.96 (br. s., 2 H) 3.27 (dd, *J*=18.09, 5.86 Hz, 1 H) 3.49 (m, 2 H) 3.64 (dd, *J*=18.09, 12.60 Hz, 1 H) 3.72 (s, 3 H) 3.90 (br. s., 2 H) 5.82 (dd, *J*=12.60, 5.86 Hz, 1 H) 6.71 (d, *J*=8.64 Hz, 2 H) 7.00 (d, *J*=8.64 Hz, 2 H) 7.06 (dd, *J*=8.72, 2.27 Hz, 1 H) 7.21 (m, 2 H) 9.25 (br. s., 1 H)
MS $(M+H)^+$: 473

**Subgroup A5.1: Chemical Example 172: 1-(2,4-Dichloro-phenyl)-5-(4-hydroxyphenyl)-4,5-dihydro-1H-pyrazole-3-carboxylic acid piperidin-1-ylamide** hydrochloride

The free base of compound 106 (0.82 mmol) was dissolved in dichloromethane (10 ml) and a solution of 1 M $BBr_3$ (5 eq) in dichloromethane was slowly added at 0 ˚C and the reation mixture was stirred at room temperature for 8 h. The solid formed was filtered off and the solvent was evaporated. A solution of 2 N HCl in diethyl ether was added to form the hydrochloride.
$^1$H NMR (300 MHz, METHANOL-$d_4$) δ ppm 1.69 (br. s., 2 H) 2.00 (br. s., 4 H) 3.24 (dd, *J*=17.94, 6.23 Hz, 1 H) 3.55 (br. s., 4 H) 3.69 (dd, *J*=17.94, 12.38 Hz, 1 H) 5.86 (dd, *J*=12.38, 6.23 Hz, 1 H) 6.61 (d, *J*=8.50 Hz, 2 H) 7.00 (d, *J*=8.50 Hz, 2 H) 7.17 (dd, *J*=8.64, 2.20 Hz, 1 H) 7.26 - 7.38 (m, 2 H)
MS $(M+H)^+$: 433

**Subgroup A5.1: Chemical Example 174: 1-(2,4-Dichloro-phenyl)-5-(4-hydroxyphenyl)-4,5-dihydro-1H-pyrazole-3-carboxylic acid (hexahydro-cyclopenta[c]pyrrol-2-yl)-amide hydrochloride**

The free base of compound 109 (0.82 mmol) was dissolved in dichloromethane (10 ml) and a solution of 1 M $BBr_3$ (5 eq) in dichloromethane was slowly added at 0 ˚C and the reation mixture was stirred at room temperature for 8

h. The solid formed was filtered off and the solvent was evaporated. A solution of 2 N HCl in diethyl ether was added to form the hydrochloride.

1 H NMR (300 MHz, METHANOL-$d_4$) δ ppm 1.68 (br. s., 2 H) 1.81 (br. s., 4 H) 2.98 (br. s., 2 H) 3.06 - 3.21 (m, 2 H) 3.26 (dd, *J*=18.02, 6.30 Hz, 1 H) 3.71 (dd, *J*=18.02, 12.45 Hz, 1 H) 4.06 (m, 2 H) 5.88 (dd, *J*=12.45, 6.30 Hz, 1 H) 6.63 (d, *J*=8.64 Hz, 2 H) 7.02 (d, J=8.64 Hz, 2 H) 7.18 (dd, *J*=8.64, 2.34 Hz, 1 H) 7.37 (d, *J*=2.20 Hz, 1 H) 7.34 (d, *J*=8.79 Hz, 1 H)

MS $(M+H)^+$: 459

| N° | STRUCTURE | Name | $^1$H-NMR | MS $(M+H)^+$ |
|---|---|---|---|---|
| 7 | 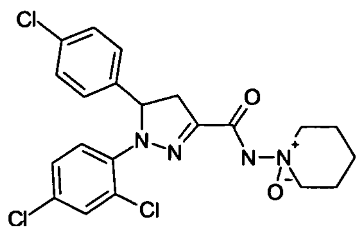 | N-oxide of 5-(4-Chloro-phenyl)-1-(2,4-dichloro -phenyl)-4,5-dihydro-1H-pyrazole-3-carboxylic acid piperidin-1-ylamide | 1 H NMR (300 MHz, CHLOROFORM-*d*) δ ppm 1.6 (m, 2 H) 1.8-2.0 (m, 4 H) 2.55 (m, 2 H) 3.3 (dd, *J*= 18.2, 6.3 Hz, 1 H) 3.7 (m, 3 H) 5.8 (dd, *J* = 12.5, 6.3 Hz, 1 H) 7.0-7.3 (m, 7 H) 8.5 (s, 1 H). | |
| 8 | 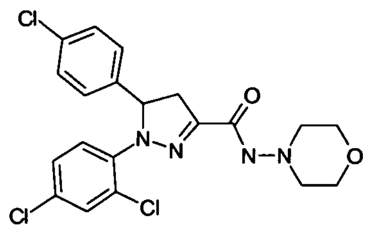 | 5-(4-Chloro-phenyl)-1-(2,4-dichloro -phenyl)-4,5-dihydro-1H-pyrazole-3-carboxylic acid morpholin-4-ylamide | 1 H NMR (300 MHz, CHLOROFORM-*d)* δ ppm 3.00 (m, 4 H) 3.32 (dd, *J*=18.31, 6.30 Hz, 1 H) 3.69 (dd, *J*=18.31, 12.30 Hz, 1 H) 3.90 (m, 4 H) 5.75 (dd, *J*=12.30, 6.30 Hz, 1 H) 7.07 (m, 4 H) 7.17 (m, 3 H) 7.68 (s, 1 H) | 453 |
| 9 | 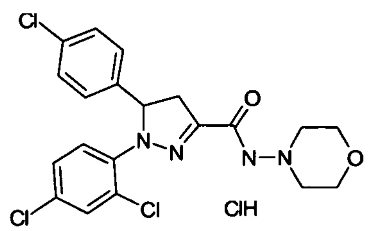 | 5-(4-Chloro-phenyl)-1-(2,4-dichloro -phenyl)-4,5-dihydro-1H-pyrazole-3-carboxylic acid morpholin-4-ylamide hydrochloride | | 453 |

(continued)

| N° | STRUCTURE | Name | $^1$H-NMR | MS $(M+H)^+$ |
|---|---|---|---|---|
| 10 | 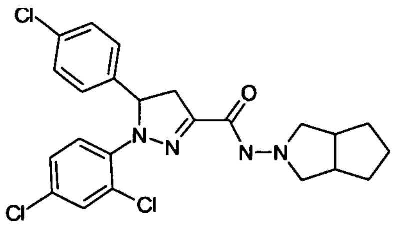 | 5-(4-Chloro-phenyl)-1-(2,4-dichloro -phenyl)-4,5-dihydro-1H-pyrazole-3-carboxylic acid (hexahydro-cyclopen ta[c]pyrrol-2-yl)-amide | 1 H NMR (400 MHz, CHLOROFORM-*d*) δ ppm 1.61 (m, 6 H) 2.77 (m, 4 H) 3.31 (dd, *J*=18.17, 6.06 Hz, 1 H) 3.47 (s, 2 H) 3.68 (dd, *J*=18.17, 12.11 Hz, 1 H) 5.75 (dd, *J*=12.11, 6.06 Hz, 1 H) 7.05 (m, 3 H) 7.16 (t, *J*=8.60 Hz, 2 H) 7.25 (m, 2 H) 7.75 (br, 1 H) | 477 |
| 11 | 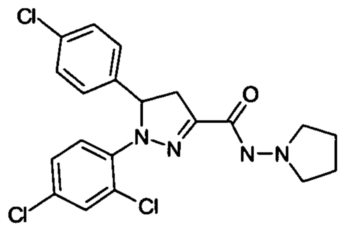 | 5-(4-Chloro-phenyl)-1-(2,4-dichloro -phenyl)-4,5-dihydro-1H-pyrazole-3-carboxylic acid pyrrolidin-1-ylamid e | 1H NMR (400 MHz, CHLOROFORM-*d*) δ ppm 2.06 (s, 4 H) 3.28 (m, 5H) 3.69 (dd, *J=17.98,* 12.50 Hz, 1 H) 5.79 (dd, *J*=12.50, 5.86 Hz, 1 H) 7.06 (m, 3 H) 7.18 (m, 3 H) 7.25 (m, 1 H) 8.22 (br, 1 H) | 437 |
| 12 | 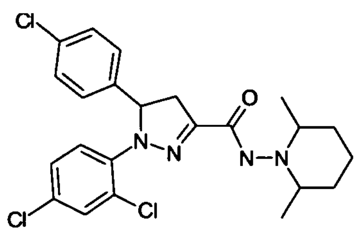 | 5-(4-Chloro-phenyl)-1-(2,4-dichloro -phenyl)-4,5-dihydro-1H-pyrazole-3-carboxylic acid (2,6-dimethyl-piper idin-1-yl)-amide | 1H NMR (400 MHz, CHLOROFORM-*d*) δ ppm 1.16 (m, 6H) 1.59 (m, 2H) 1.71(m, 4 H) 2.39 (m, 2 H) 3.35 (dd, *J*=18.56, 4.88 Hz, 1 H) 3.70 (dd, *J*=18.56, 12.50 Hz, 1 H) 5.71 (dd, *J*=12.50, 6.06 Hz, 1 H) 6.84 (br, 1 H) 7.06 (m, 4 H) 7.18 (d, *J*=8.21 Hz, 2 H) 7.26 (m, 1H) | 479 |

(continued)

| N° | STRUCTURE | Name | $^1$H-NMR | MS (M+H)$^+$ |
|---|---|---|---|---|
| 13 | 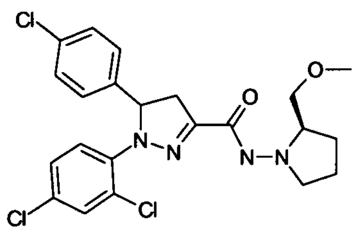 | 5-(4-Chloro-phenyl)-1-(2,4-dichloro -phenyl)-4,5-dihydro-1H-pyrazole-3-carboxylic acid (2-methoxymethyl-py rrolidin-1-yl)-amide | 1 H NMR (400 MHz, CHLOROFORM-*d*) δ ppm 1.88 (m, 1 H) 2.06 (m, 2 H) 2.20 (m, 1 H) 3.30 (dd, *J*=18.37, 5.86 Hz, 1 H) 3.37 (m, 1H) 3.40 (s, 3H) 3.59 (m, 2 H) 3.71 (m, 3 H) 5.82 (dd, *J*=12.50, 5.86 Hz, 1 H) 7.05 (m, 3 H) 7.19 (d, *J*=8.60 Hz, 2 H) 7.24 (m, 2 H) | 481 |
| 14 | 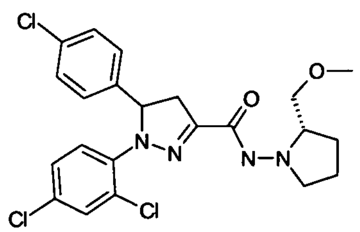 | 5-(4-Chloro-phenyl)-1-(2,4-dichloro -phenyl)-4,5-dihydro-1 H-pyrazole-3-carboxylic acid (2-methoxymethyl-py rrolidin-1-yl)-amide | 1 H NMR (400 MHz, CHLOROFORM-*d*) δ ppm 1.79 (m, 1 H) 1.98 (m, 2 H) 2.13 (m, 1H) 3.32 (dd, *J*=18. 76, 6.25 Hz, 1 H) 3.36 (m, 1H) 3.38 (s, 3 H) 3.51 (m, 2 H) 3.64 (m, 2 H) 3.70 (dd, *J*=18. 76, 12.31 Hz, 1 H) 5.77 (dd, *J=12.31,* 6.25 Hz, 1 H) 7.07 (td, J=8.99, 2.34 Hz, 3 H) 7.18 (d, *J*=8.21 Hz, 2 H) 7.24 (m, 2 H) | 481 |
| 15 | 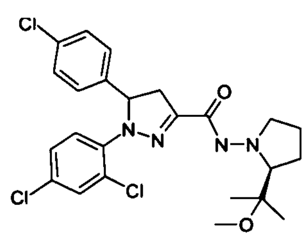 | 5-(4-Chloro-phenyl)-1-(2,4-dichloro -phenyl)-4,5-dihydro-1H-pyrazole-3-carboxylic acid [2-(1-methoxy-1-met hyl-ethyl]-pyrrolidin-1-yl]-amide | 1 H NMR (300 MHz, DMSO-$d_6$) δ ppm 1.07 (m, 6 H) 1.71 (m, 3 H) 1.82 (d, *J*=7.03 Hz, 2 H) 2.89 (m, 1 H) 3.01 (m, 1 H) 3.07 (m, 3 H) 3.18 (m, 1 H) 3.64 (dd, *J*=18.02, 11.86 Hz, 1 H) 5.79(m, 1 H) 7.12 (m, 2 H) 7.28 (m, 3 H) 7.46 (m, 2 H) 9.33 (s, 1 H) | 509 |

(continued)

| N° | STRUCTURE | Name | $^1$H-NMR | MS $(M+H)^+$ |
|---|---|---|---|---|
| 16 | 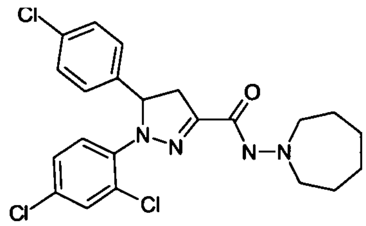 | 5-(4-Chloro-phenyl)-1-(2,4-dichloro -phenyl)-4,5-dihydro-1H-pyrazole-3-carboxylic acid azepan-1-ylamide | 1 H NMR (300 MHz, CHLOROFORM-*d*) δ ppm 1.63 (m, 4 H) 1.80 (m, 4 H) 3.22 (dd, *J*=18.31, 6.15 Hz, 1 H) 3.30 (m, 4 H) 3.61 (dd, *J*=18.31, 12.30 Hz, 1 H) 5.70 (dd, *J*=12.30, 6.15 Hz, 1 H) 6.99(m, 3H) 7.10 (m, 3 H) 7.19 (m, 1 H) | 465 |
| 17 | 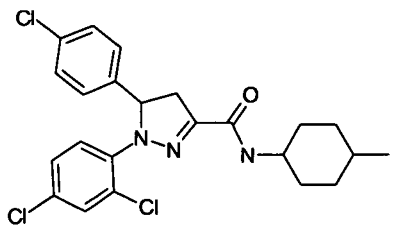 | 5-( 4-Chloro-phenyl)-1-(2,4-dichloro -phenyl)-4,5-dihydro-1H-pyrazole-3-carboxylic acid (4-methyl-cyclohexy l)-amide | | 464 |
| 18 | 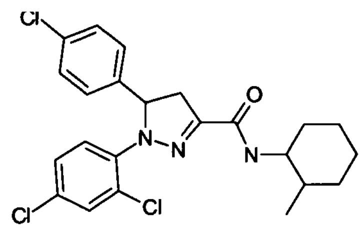 | 5-(4-Chloro-phenyl)-1-(2,4-dichloro -phenyl)-4,5-dihydro-1H-pyrazole-3-carboxylic acid (2-methyl-cyclohexy l)-amide | | 464 |
| 19 | 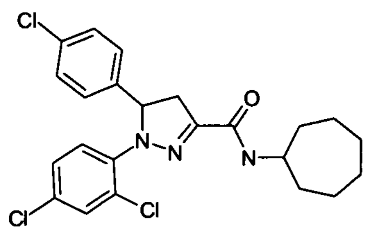 | 5-(4-Chloro-phenyl)-1-(2,4-dichloro -phenyl)-4,5-dihydro-1H-pyrazole-3-carboxylic acid cycloheptylamide | 1 H NMR (300 MHz, DMSO-$d_6$) δ ppm 1.53 (m, 10 H) 1.79 (m, 2 H) 3.05 (dd, *J*=18.16, 5.71 Hz, 1H) 3.64 (dd, *J*=18.16, 11.94 Hz, 1H)3.85 (m, 1 H)5.80 (dd, *J*= 11.94, 5.71 Hz, 1H) 7.13 (m, 2H) 7.27 (m, 2H) 7.43 (d, *J*=2.34 Hz, 1H) 7.51 (d, *J*=8.79 Hz, 1 H) 8.10 (d, *J*=8.35 Hz, 1 H) | 464 |

(continued)

| N° | STRUCTURE | Name | $^1$H-NMR | MS $(M+H)^+$ |
|---|---|---|---|---|
| 20 | 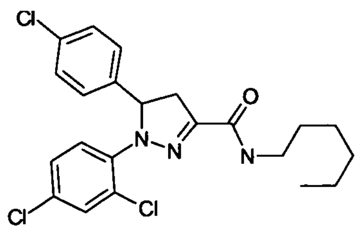 | 5-(4-Chloro-phenyl)-1-(2,4-dichloro -phenyl)-4,5-dihydro-1H-pyrazole-3-carboxylic acid hexylamide | 1 H NMR (300 MHz, DMSO-$d_6$) δ ppm 0.85 (m, 3 H) 1.25 (m, 6 H) 1.47 (m, 2 H) 3.05 (dd, *J*=18.16, 5.79 Hz, 1 H) 3.17 (m, 2 H) 3.65 (dd, *J*=18.16, 11.86 Hz, 1H) 5.81 (dd, *J*=11.86, 5.79 Hz, 1H) 7.14 (d, *J*=8.50 Hz, 2H) 7.27 (m, 3 H) 7.45 (m, 2 H) 8.32 (t, *J*=5.86 Hz, 1 H) | 452 |
| 21 | 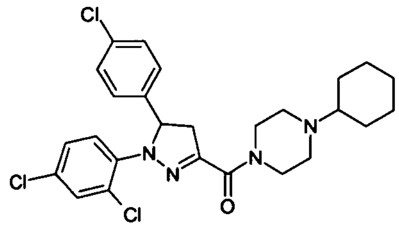 | [5-(4-Chloro-phenyl)-1-(2,4-dichlor o-phenyl)-4,5-dihydro-1H-pyrazol-3-yl]-(4-cyclohexyl-piperazin-1-yl)-m ethanone | | 519 |
| 22 | 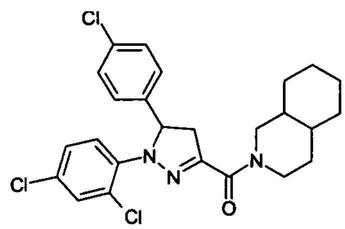 | [5-(4-Chloro-phenyl)-1-(2,4-dichloro-phenyl)-4,5-dihydro-1H-pyrazol-3-yl]-(octahydro-isoquinolin-2-yl)-me thanone | | 490 |
| 23 | 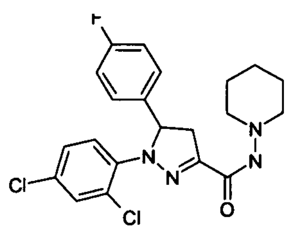 | 1-(2,4-Dichloro-phenyl)-5-(4-fluoro -phenyl)-4,5-dihydro-1H-pyrazole-3-carboxylic acid piperidin-1-ylamide | 1 H NMR (300 MHz, CHLOROFORM-*d*) δ ppm 1.48 (s, 2H) 1.84 (s, 4 H) 3.25 (s, 4 H ) 3.28 (dd, *J*=8.16, 6.15 Hz, 1H) 3.61 (dd, *J*=18.16, 12.38 Hz, 1H) 5.73(dd, Hz, *J*=12.38, 6.15 Hz, 1 H) 6.83 (t, *J*=8.57 Hz, 2 H) 7.02 (m, 4 H) 7.18 (m, 1H) 8.21 (br, 1 H) | 435 |

(continued)

| N° | STRUCTURE | Name | $^1$H-NMR | MS $(M+H)^+$ |
|---|---|---|---|---|
| 24 | 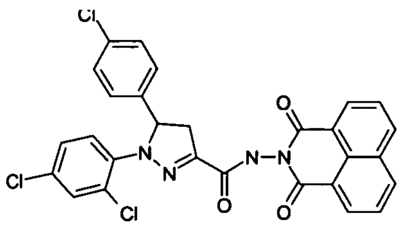 | 5-(4-Chloro-phenyl)-1-(2,4-dichloro -phenyl)-4,5-dihydro-1H-pyrazole-3- carboxylic acid (1,3-dioxo-1H,3H-be nzo[de]isoquinolin-2-yl)-amide | 1 H NMR (300 MHz, DMSO-$d_6$) δ ppm 3.16 (dd, J=18.02, 6.45 Hz, 1 H) 3.79 (dd, J=18.02, 12.01 Hz, 1H) 5.98 (dd, Hz, J=12.01. 6.45 Hz, 1 H) 7.24 (d, J=8.06 Hz, 2 H) 7.36 (m, 3 H) 7.55 (m, 2 H) 7.92 (m, 2 H) 8.55 (m, 4 H) 11.08 (s, 1 H) | 563 |
| 25 | 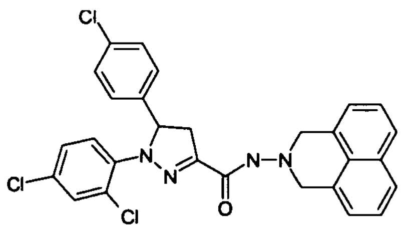 | 5-(4-Chloro-phenyl)-1-(2,4-dichloro -phenyl)-4,5-dihydro-1H-pyrazole-3-carboxylic acid (1H,3H-benzo[de] iso quinolin-2-yl)-amide | 1 H NMR (300 MHz, DMSO-$d_6$) δ ppm 3.13 (dd, J=18.09, 5.86 Hz, 1 H) 3.71 (dd, J=18.09, 11.86 Hz, 1 H) 4.35 (s, 4 H) 5.84 (dd, J=11.86, 5.86 Hz, 1H) 7.19 (d, J=8.50 Hz, 2 H) 7.31 (m, 5 H) 7.47 (m, 4 H) 7.79 (d, J=8.20 Hz, 2H) 9.77 (s, 1H) | 535 |
| 26 | 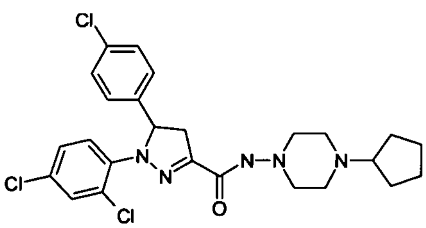 | 5-(4-Chloro-phenyl)-1-(2,4-dichloro -phenyl)-4,5-dihydro-1H-pyrazole-3- carboxylic acid (4-cyclopentyl-pipe razin-1-yl)-amide | | 520 |
| 27 | 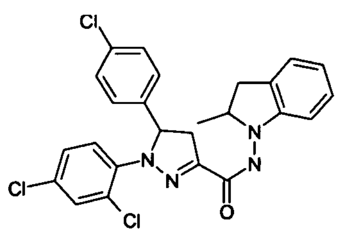 | 5-(4-Chloro-phenyl)-1-(2,4-dichloro -phenyl)-4,5-dihydro-1H-pyrazole-3-carboxylic acid (2-methyl-2,3-dihyd ro-indol-1-yl)-amide | | 499 |

(continued)

| N° | STRUCTURE | Name | $^1$H-NMR | MS $(M+H)^+$ |
|---|---|---|---|---|
| 28 | 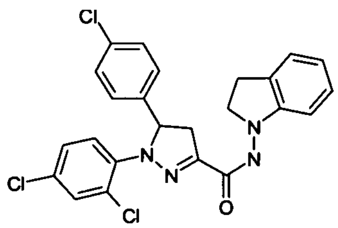 | 5-(4-Chloro-phenyl)-1-(2,4-dichloro -phenyl)-4,5-dihydro-1H-pyrazole-3-carboxylic acid (2,3-dihydro-indol-1-yl)-amide | 1 H NMR (300 MHz, DMSO-$d_6$) δ ppm 2.96 (t, *J*=8.20 Hz, 2 H) 3.11 (dd, *J*=18.02, 5.71 Hz, 1 H) 3.59 (t, *J*=8.20 Hz, 2 H) 3.73 (dd, *J*=18.02, 12.01 Hz, 1 H) 5.87 (dd, *J*=12.01, 5.71 Hz, 1 H) 6.58 (d, *J*=7.76 Hz, 1 H) 6.75 (t, *J*=7.32 Hz, 1 H) 7.10 (m, 4 H) 7.29 (m, 3 H) 7.45 (d, *J*=2.34 Hz, 1 H) 7.54 (d, *J*=8.79 Hz, 1 H) 10.20 (s, 1H) | 485 |
| 29 | 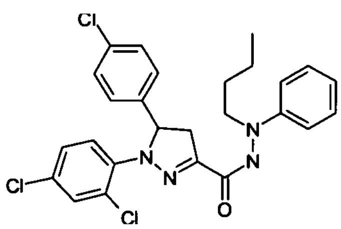 | 5-(4-Chloro-phenyl)-1-(2,4-dichloro -phenyl)-4,5-dihydro-1H-pyrazole-3-carboxylic acid N'-butyl-N'-phenyl-hydrazide | 1H NMR (300 MHz, CHLOROFORM-*d*) δ ppm 0.98 (t, *J*=7.32 Hz, 3 H) 1.45 (m, 2 H) 1.69 (m, 2 H) 3.36 (dd, *J*=18.31, 6.84 Hz, H) 1 H) 3.57 (m, 2 H) 3.75 (dd, *J*=18.31, 12.21 Hz, 1 H) 5.77 (dd, *J*=12.21, 6.84 Hz, 1 H) 6.90 (m, 3 H) 7.10 (m, 4 H) 7.26 (m, 5 H) 8.22 (s, 1 H) | 515 |
| 30 | 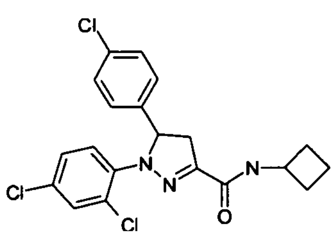 | 5-(4-Chloro-phenyl)-1-(2,4-dichloro -phenyl)-4,5-dihydro-1H-pyrazole-3-carboxylic acid cyclobutylamide | 1H NMR (300 MHz, DMSO-$d_6$) δ ppm 1.61 (m, 2 H) 2.14 (m, 4 H) 3.05 (dd, *J*=18.16, 5.57 Hz, 1 H) 3.63 (dd, *J*=18.16, 11.94 Hz, 1 H) 4.33 (m, 1 H) 5.81 (dd, *J*=11.94, 5.57 Hz, 1H)7.13(m,2H)7.27 (m, 3 H) 7.43 (d, *J*=2.34 Hz, 1 H) 7.52 (d, *J*=8.79 Hz, 1 H) 8.53 (d, *J*=8.06 Hz, 1 H) | 422 |

(continued)

| N° | STRUCTURE | Name | $^1$H-NMR | MS $(M+H)^+$ |
|---|---|---|---|---|
| 31 | 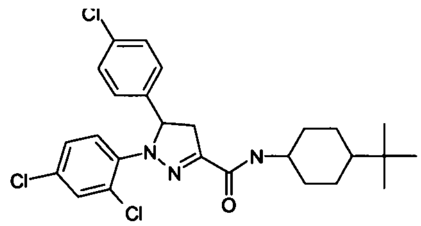 | 5-(4-Chloro-phenyl)-1-(2,4-dichloro -phenyl)-4,5-dihydro-1 H-pyrazole-3-carboxylic acid (4-tert-butyl-cyclo hexyl)-amide | | 506 |
| 32 | 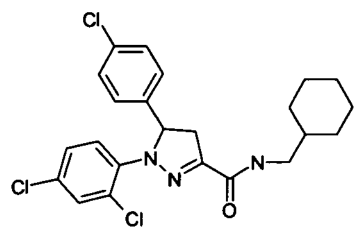 | 5-(4-Chloro-phenyl)-1-(2,4-dichloro -phenyl)-4,5-dihydro-1H-pyrazole-3-carboxylic acid cyclohexylmethyl-am ide | 1 H NMR (400 MHz, CHLOROFORM-*d*) δ ppm 0.99 (m, 2 H) 1.21 (m, 4 H) 1.69 (m, 1H) 1.76 (m, 4 H) 3.15 (dd, *J*=17.98, 6.06 Hz, 1 H) 3.32 (dt, *J*=12.60, 6.40 Hz, 2 H) 3.67 (dd, *J*=17.98, 12.11 Hz, 1 H) 5.73 (dd, *J*=12.11, 6.06 Hz, 1 H) 6.75 (t, *J*=6.06 Hz, 1 H) 7.06 (m, 3 H) 7.12 (m, 1 H) 7.17 (d, *J*=8.60 Hz, 2 H) 7.26 (m, 1 H) | 464 |
| 33 | 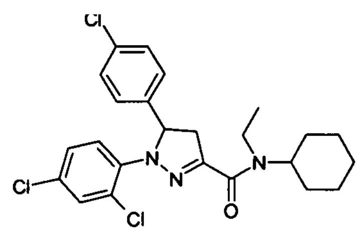 | 5-(4-Chloro-phenyl)-1-(2,4-dichloro -phenyl)-4,5-dihydro-1H-pyrazole-3-carboxylic acid cyclohexyl-ethyl-am ide | | 478 |

(continued)

| N° | STRUCTURE | Name | $^1$H-NMR | MS $(M+H)^+$ |
|---|---|---|---|---|
| 34 | 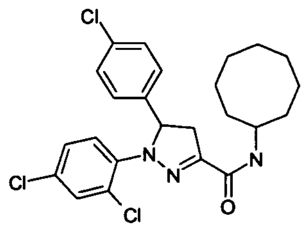 | 5-(4-Chloro-phenyl)-1-(2,4-dichforo -phenyl)-4,5-dihydro-1H-pyrazole-3-carboxylic acid cyclooctylamide | 1H NMR (400 MHz, CHLOROFORM-*d*) δ ppm 1.59 (m, 12 H) 1.91 (m, 2 H) 3.32 (dd, *J*=18.37, 5.86 Hz, 1 H) 3.66 (dd, *J*=18.37, 12.11 Hz, 1 H) 4.10 (ddd, *J*=8.60, 4.01, 3.71 Hz, 1 H) 5.72 (dd, *J*=12.11, 5.86 Hz, 1H) 6.65 (d, *J*=8.60 Hz, 1 H) 7.07 (m, 3 H) 7.15 (m, 3 H) 7.25 (m, 1 H) | 478 |
| 35 | 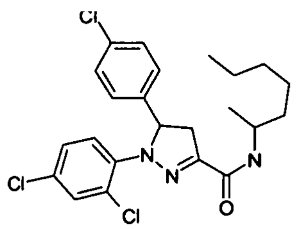 | 5-(4-Chloro-phenyl)-1-(2,4-dichloro -phenyl)-4,5-dihydro-1H-pyrazole-3-carboxylic acid (1-methyl-hexyl)-am ide | 1 H NMR (400 MHz, CHLOROFORM-*d*) δ ppm 0.89 (m, J=6.94 Hz, 3 H) 1.21 (m, *J*=6.64 Hz, 3 H) 1.34 (m, 6 H) 1.50 (m, 2 H) 3.32 (ddd, J=18.37, 6.06, 1.76 Hz, 1 H) 3.67 (ddd, *J=18.37,* 12.02, 3.32 Hz, 1H) 4.08 (m, 1 H) 5.73 (dt, J=12.02, 6.06 Hz, 1 H) 6.48 (dd, J=8.01, 5.67 Hz, 1 H) 7.08 (m,3H) 7.11 (m, 1 H) 7.16 (m, 2 H) 7.25 | 466 |

(continued)

| N° | STRUCTURE | Name | $^1$H-NMR | MS (M+H)+ |
|---|---|---|---|---|
| 36 | 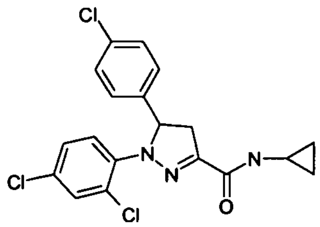 | 5-(4-Chloro-phenyl)-1-(2,4-dichloro -phenyl)-4,5-dihydro-1H-pyrazole-3-carboxylic acid cyclopropylamide | 1 H NMR (300 MHz, DMSO-$d_6$) δ ppm 0.63 (m, 4 H) 2.77 (m, 1 H) 3.06 (dd, *J*=18.02, 5.57 Hz, 1 H) 3.64 (dd, *J*=18.02, 11.86 Hz, 1 H) 5.81 (dd, *J*=11.86, 5.57 Hz, (d, *J*=8.50 Hz, 2 H) 7.26 (m, 3 H) 7.42 (d, *J*=2.34 Hz, 1 H) 7.49 (d, *J*=8.64 Hz, 1 H) 8.36 (d, *J*=4.39 Hz, 1 H) | 408 |
| 37 | 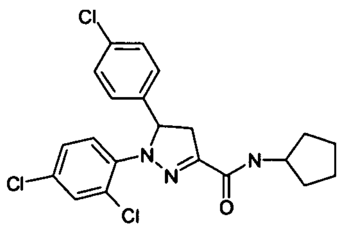 | 5-(4-Chloro-phenyl)-1-(2,4-dichloro -phenyl)-4,5-dihydro-1H-pyrazole-3-carboxylic acid cyclopentylamide | 1H NMR (400 MHz, CHLOROFORM-*d*) δ ppm 1.44 - 1.75 (m, 6H) 2.06 (td, *J*=13.09, 5.86 Hz, 2 H) 3.32 (dd, *J*=18.37, 6.25 Hz, 1H) 3.66 (dd, *J*=18.37, 12.11 Hz, 1H) 4.31 (m, 1H) 5.73 (dd, *J*=12.11, 6.25 Hz, 1 H) 6.62 (d, *J*=7.82 Hz, 1 H) 7.06 (m, 3 H) 7.12 (s, 1H) 7.17 (m, 2 H) 7.25 (m, 1H) | 436 |
| 38 | 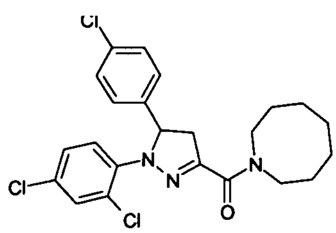 | Azocan-1-yl-[5-(4-chloro-phenyl)-1-(2,4-dichloro-phenyl)-4,5-dihydro-1 H-pyrazol-3-yl]-methanone | 1H NMR (400 MHz, CHLOROFORM-*d*) δ ppm 1.61 (m, 6 H) 1.82 (m, 4 H) 3.37 (dd, *J*=17.98, 5.86 Hz, 1 H) 3.62 (m, 2 H) 3.72 (dd, *J*=17.98, 11.72 Hz, 1 H) 3.87 (m, 2 H) 5.65 (dd, *J*=11.72, 5.86 Hz, 1 H) 7.06 (m, 4H) 7.17 (m, 2H) 7.25 (m, 1H) | 464 |

(continued)

| N° | STRUCTURE | Name | 1H-NMR | MS (M+H)+ |
|---|---|---|---|---|
| 39 | 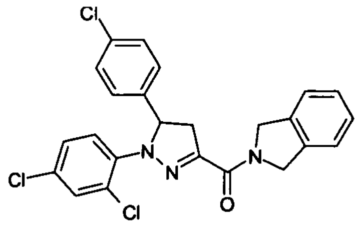 | [5-(4-Chloro-phenyl)-1-(2,4-dichlor o-phenyl)-4,5-dihydro-1 H-pyrazol-3-yl]-(1,3-dihydro-isoindol-2-yl)-met hanone | 1H NMR (400 MHz, CHLOROFORM-*d*) δ ppm 3.44 (dd, *J*=17.98, 6.25 Hz, 1 H) 3.78 (dd, *J*=17.98, 12.11 Hz, 1H) 4.98 (d, 3.78 *J*=3.13 Hz, 2 H) 5.24 (d, *J*=16.02 Hz, 1 H) 5.32 (d, *J*=16.02 Hz, 1 H) 5.70 (dd, *J*=12.11, 6.25 Hz, 1 H) 7.10 (m, 3 H) 7.17 (m, 3 H) 7.32 (m, 5 H) | 470 |
| 40 | 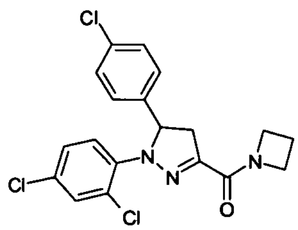 | Azetidin-1-yl-[5-(4-chloro-phenyl)-1-(2,4-dichloro-phenyl)-4,5-dihydro -1H-pyrazol-3-yl]-methanone | 1H NMR (300 MHz, DMSO-$d_6$) δ ppm 2.26 (m, *J*=7.58, 2 H) 3.01 (dd, *J*=18.02, 6.30 Hz, 1 H) 3.64 (dd, *J*=18.02, 12.01 Hz, 1H) 3.99 (ddd, *J*=7.43, 4.10, 3.85 Hz, 2H) 4.38 (q, *J*=7.67 Hz, 1H) 4.53 (m, 1 H) 5.77 (dd, *J*=12.01, 6.30 Hz, 1 H) 7.17 (m, 2H) 7.27 (m, 4 H) 7.46 (d, *J*=2.20 Hz, 1 H) | 408 |
| 41 | 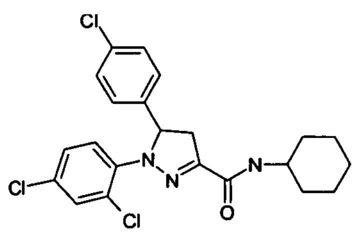 | 5-(4-Chloro-phenyl)-1-(2,4-dichloro -phenyl)-4,5-dihydro-1H-pyrazole-3-carboxylic acid cyclohexylamide | | 450 |
| 42 | 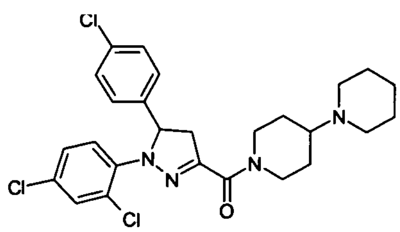 | [1,4']Bipiperidinyl-1'-yl-[5-(4-chl oro-phenyl)-1-(2,4-dichloro-phenyl) -4,5-dihydro-1H-pyrazol-3-yl]-metha none | | 519 |

(continued)

| N° | STRUCTURE | Name | $^1$H-NMR | MS (M+H)+ |
|---|---|---|---|---|
| 43 | 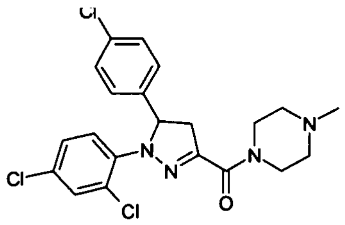 | [5-(4-Chloro-phenyl)-1-(2,4-dichlor o-phenyl]-4,5-dihydro-1H-pyrazol-3-yl]-(4-methyl-piperazin-1-yl)-metha none | 1H NMR (300 MHz, DMSO-$d_6$) δ ppm 2.18 (s, 3H) 2.35 (m, 4H) 3.11 (dd, *J*= 17.80, 6.96 Hz, 1H) 3.52 (m, 1H) 3.61 (m, 1 H) 3.70 (dd, *J*= 17.80, 11.64 Hz, 1 H) 3.87 (m, 2 H) 5.71 (dd, *J*= 11.64, 6.96 Hz, 1 H) 7.22 (m, 4 H) 7.31 (m, 2 H) 7.48 (d, *J*=1.61 Hz, 1H) | 451 |
| 44 | 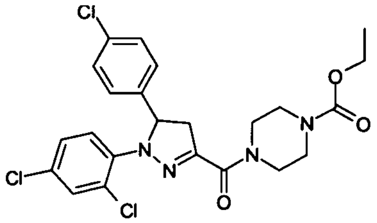 | 4-[5-(4-Chloro-phenyl)-1-(2,4-dichl oro-phenyl)-4,5-dihydro-1H-pyrazole -3-carbonyl]-piperazine-1-carboxyli c acid ethyl ester | | 509 |
| 45 | 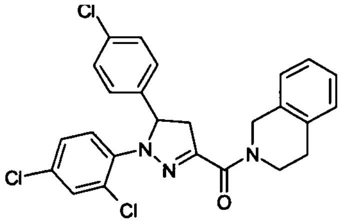 | [5-(4-Chloro-phenyl)-1-(2,4-dichlor o-phenyl)-4,5-dihydro-1 H-pyrazol-3-yl]-(3,4-dihydro-1H-isoquinolin-2-y l)-methanone | | 484 |
| 46 | 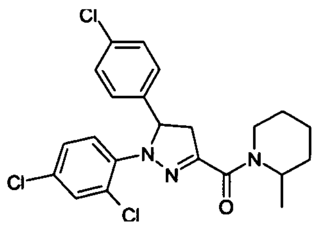 | [5-(4-Chloro-phenyl)-1-(2,4-dichlor o-phenyl)-4,5-dihydro-1 H-pyrazol-3-yl]-(2-methyl-piperidin-1-yl)-methanone | | 450 |
| 47 | 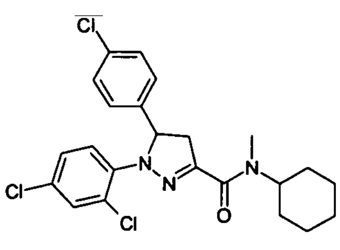 | 5-(4-Chloro-phenyl)-l-(2,4-dichloro -phenyl)-4,5-dihydro-1H-pyrazole-3-carboxylic acid cyclohexyl-methyl-a mide | 1H NMR (300 MHz, DMSO-$d_6$) δ ppm 1.22 (m, 4 H) 1.57 (m, 3 H) 1.73 (m, 3 H) 2.97 (s, 3H) 3.10 (dd, *J*=17.80, 7.03 Hz, 1 H) 3.71 (dd, *J*=17.80, 11.64 Hz, 1H) 4.28 (m,1H) 5.71 (m, 1H) 1H) 7.22 (m, 4H) 7.30 (m, 2H) 7.48 (m, 1 H) | 464 |

(continued)

| N° | STRUCTURE | Name | 1H-NMR | MS $(M+H)^+$ |
|---|---|---|---|---|
| 48 | 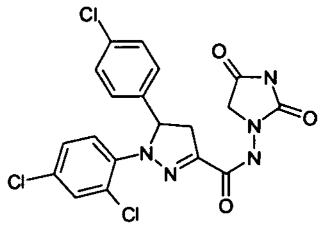 | 5-(4-Chloro-phenyl)-1-(2,4-dichloro -phenyl)-4,5-dihydro-1H-pyrazole-3-carboxylic acid (2,4-dioxo-imidazol idin-1-yl)-amide | | 466 |
| 49 | 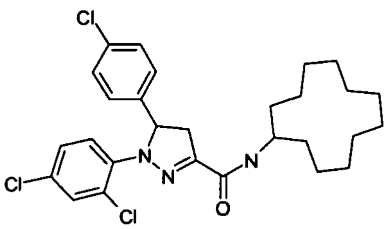 | 5-(4-Chloro-phenyl)-1-(2,4-dichloro -phenyl)-4,5-dihydro-1H-pyrazole-3-carboxylic acid cyclododecylamide | 1H NMR (300 MHz, DMSO-$d_6$) δ ppm 1.29 (m, 20 H) 1.63 (m, 2 H) 3.06 (dd, *J*=18.09, 5.93 Hz, 1 H) 3.65 (dd, *J*=18.09, 11.86 Hz, 1 H) 4.04 (m, 1 H) 5.81 (dd, *J*=11.86, 5.93 Hz, 1H) 7.14 (m, 2 H) 7.28 (m, 3 H) 7.43 (d, *J*=2.34 Hz, 1 H) 7.50 (d, *J*=8.79 Hz, 1 H) 8.00 (d, *J*=8.79 Hz, 1 H) | 534 |
| 50 | 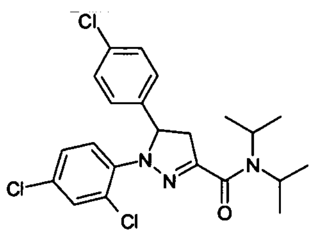 | 5-(4-Chloro-phenyl)-1-(2,4-dichloro -phenyl)-4,5-dihydro-1H-pyrazole-3-carboxylic acid diisopropylamide | 1H NMR (300 MHz, DMSO-$d_6$) δ ppm 1.20 (m, 6 H) 1.36 (m, 6 H) 3.09 (dd, *J*=17.65. 6.66 Hz, 1 H) 3.60 (br, 1 H) 3.67 (dd, *J*=17.65, 11.43 Hz, 1 H) 4.52 (br, 1H) 5.69 (dd, *J*=11.43, 6.66 Hz, 1 H) 7.20 (m, 4 H) 7.32 (m, 2 H) 7.46 (d, J=2.05 Hz, 1 H) | 452 |
| 51 | 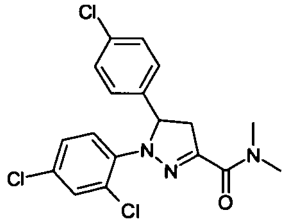 | 5-(4-Chloro-phenyl)-1-(2,4dichloro -phenyl)-4,5-dihydro-1 H-pyrazole-3-carboxylic acid dimethylamide | | 396 |

(continued)

| N° | STRUCTURE | Name | $^1$H-NMR | MS $(M+H)^+$ |
|---|---|---|---|---|
| 52 | 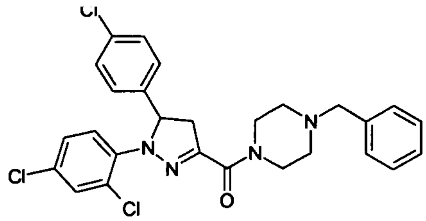 | (4-Benzyl-piperazin-1-yl)-[5-(4-chl oro-phenyl)-1-(2,4-dichloro-phenyl) -4,5-dihydro-1 H-pyrazol-3-yl]-metha none | | 527 |
| 53 | 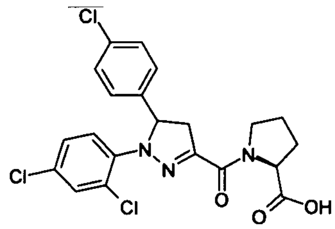 | 1-[5-(4-Chloro-phenyl)-1-(2,4-dichl oro-phenyl)-4,5-dihydro-1H-pyrazole -3-carbonyl]-pyrrolidine-2-carboxyl ic acid | | 466 |
| 54 | 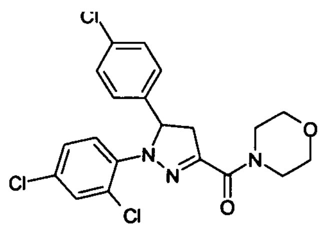 | [5-(4-Chloro-phenyl)-1-(2,4-dichloro-phenyl)-4,5-dihydro-1H-pyrazol-3-yl]-morpholin-4-yl-methanone | 1H NMR (300 MHz, DMSO-$d_6$) δ ppm 3.12 (dd, *J*=17.87, 6.37 Hz, 1 H) 3.62 (m, 6 H) 3.72 (dd, *J*=17.87, 11.06 Hz, 1 H) 3.93 (m, 2 H) 5.71 (dd, *J*=11.06, 6.37 Hz, 1 H) 7.22 (m, 4 H) 7.31 (m, 2 H) 7.48 (s, 1 H) | 438 |
| 55 | 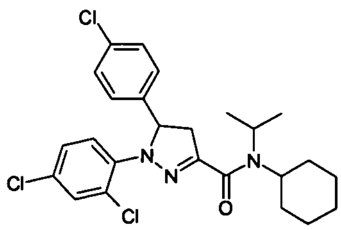 | 5-(4-Chloro-phenyl)-1-(2,4-dichloro -phenyl)-4,5-dihydro-1H-pyrazole-3-carboxylic acid cyclohexyl-isopropy l-amide | | 492 |
| 56 | 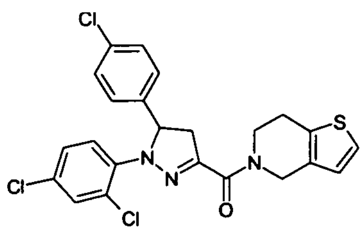 | [5-(4-Chloro-phenyl)-1-(2,4-dichlor o-phenyl)-4,5-dihydro-1H-pyrazol-3-yl]-(6,7-dihydro-4H-thieno[3,2-c]py ridin-5-yl)-methanone | | 490 |
| 57 | 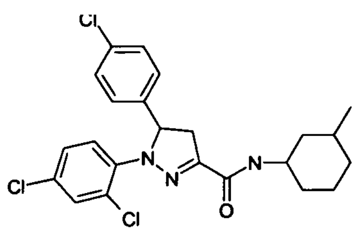 | 5-(4-Chloro-phenyl)-1-(2,4-dichloro -phenyl)-4,5-dihydro-1H-pyrazole-3-carboxylic acid (3-methyl-cyclohexy l)-amide | | 464 |

(continued)

| N° | STRUCTURE | Name | $^1$H-NMR | MS (M+H)$^+$ |
|---|---|---|---|---|
| 58 | 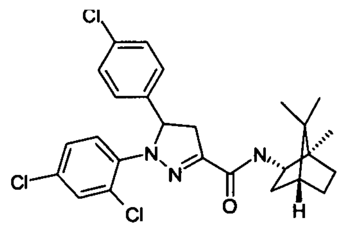 | 5-(4-Chloro-phenyl)-1-(2,4-dichloro -phenyl)-4,5-dihydro-1H-pyrazole-3-carboxylic acid (1,7,7-trimethyl-bi cyclo[2.2.1]hept-2-yl)-amide | | 504 |
| 59 | 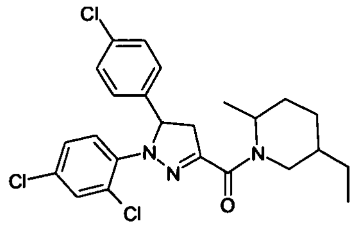 | [5-(4-Chloro-phenyl)-1-(2,4-dichlor o-phenyl)-4,5-dihydro-1H-pyrazol-3- yl]-(5-ethyl-2-methyl-piperidin-1-yl)-methanone | | 478 |
| 60 | 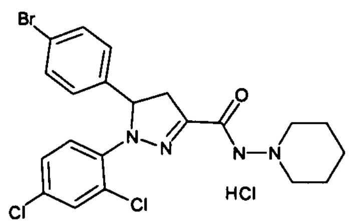 | 5-(4-Bromo-phenyl)-1-(2,4- dichloro-phenyl)-4,5-dihydro-1H-pyrazole-3-carboxylic acid piperidin-1-ylamide hydrochloride | 1H NMR (300 MHz, METHANOL-$d_4$) δ ppm 1.68 (m, 2H) 1.92 - 2.03 (m, 4 H) 3.24 (dd, $J$=18.02, 6.52 Hz, 1 H) 3.49 (br. s., 4 H) 3.76 (dd, $J$=18.02 12.52 Hz, 1 H) 5.95 (dd, $J$=12.52, 6.52 Hz, 1H) 7.14 (d, $J$=8.50 Hz, 2 H) 7.21 (dd, $J$=8.72, 2.42 Hz, 1H) 7.40 (m, 3 H) 7.37 (d, $J$=2.42 Hz, 1 H) | 495 |
| 61 | 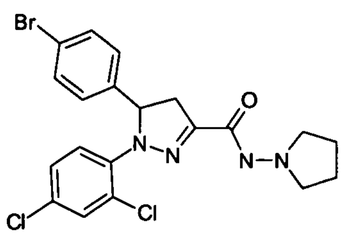 | 5-(4-Bromo-phenyl)-1-(2,4-dichloro-phenyl)-4,5-dihydro-1H-pyrazole-3-c arboxylic acid pyrrolidin-1-ylamide | | 482 |
| 62 | 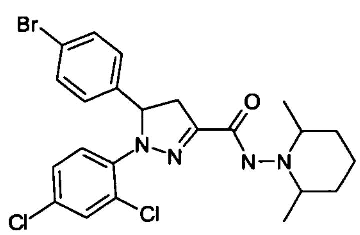 | 5-(4-Bromo-phenyl)-1-(2,4-dichloro-phenyl)-4,5-dihydro-1H-pyrazole-3-carboxylic acid (2,6-dimethyl-piperi din-1-yl)-amide | | 524 |

(continued)

| N° | STRUCTURE | Name | $^1$H-NMR | MS $(M+H)^+$ |
|---|---|---|---|---|
| 63 | 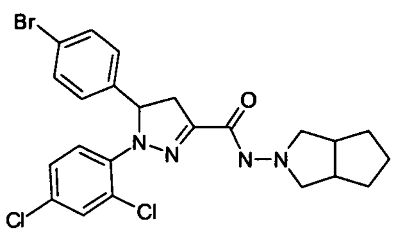 | 5-(4-Bromo-phenyl)-1-(2,4-dichloro- phenyl)-4,5-dihydro-1H-pyrazole-3-c arboxylic acid (hexahydro-cyclopent a[c]pyrro)-2-yl)-amide | 1H NMR (300 MHz, CHLOROFORM-*d*) δ ppm 1.54 - 1.85 (m, 6 H) 2.91 (br. s., 2 H) 3.25 (dd, *J*=18.16, 6.08 Hz, 1 H) 3.29 (br. s., 2 H) 3.68 (dd, *J*=18.16, 12.52 Hz, 1 H) 3.79 (br. s., 2 H) 5.81 (dd, *J*=12.52, 6.08 Hz, 1 H) 6.98 (d, *J*=8.50 Hz, 2 H) 7.09 (dd, *J*=8.64, 2.34 Hz, 1 H) 7.20 - 7.25 (m, 2 H) 7.34 (d, *J*=*8.35* Hz, 2 H) 9.04 (br. s., | 522 |
| 64 | 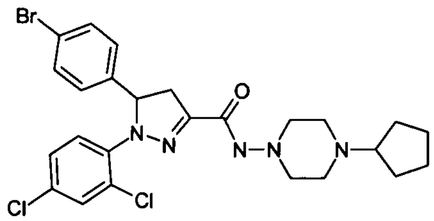 | 5-(4-Bromo-phenyl)-1-(2,4-dichloro-phenyl)-4,5-dihydro-1H-pyrazole-3-c arboxylic acid (4-cyclopentyl-piper azin-1-yl)-amide | | 565 |
| 65 | 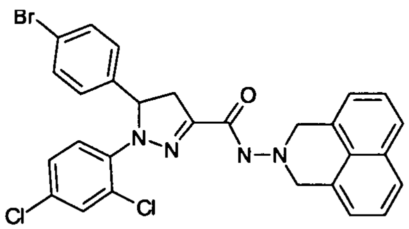 | 5-(4-Bromo-phenyl)-1-(2,4-dichloro-phenyl)-4,5-dihydro-1H-pyrazole-3-c arboxylic acid (1H,3H-benzo[de] isoq uinolin-2-yl)-amide | | 580 |

(continued)

| N° | STRUCTURE | Name | $^1$H-NMR | MS $(M+H)^+$ |
|---|---|---|---|---|
| 66 | 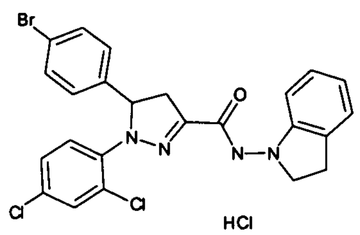 | 5-(4-Bromo-phenyl)-1-(2,4- dichloro-phenyl)-4,5-dihydro-1H-pyrazole-3-c arboxylic acid (2,3-dihydro-indol-1 | 1H NMR (300 MHz, METHANOL-$d_4$) δ ppm 3.05 (t, *J*=7.98 Hz, 2 H) 3.28 (dd, *J*=18.09, 5.86 Hz, 1 H) 3.64 (t, *J*=8.50 Hz, 2 H) 3.77 (dd, *J*=18.09. 12.16 Hz, 1 H) 5.93 (dd, *J*=12.16 5.86 Hz, 1 H) 6.71 (d, *J*=7.76 Hz, 1H) 6.84 (t, *J*=7.40 Hz, 1 H) 7.07 - 7.21 (m, 5 H) 7.31 (d, *J*=2.34 Hz, 1 H) 7.44 (d, *J*=8.64 Hz, 1 H) 7.40 (d, | 529 |
| 67 | 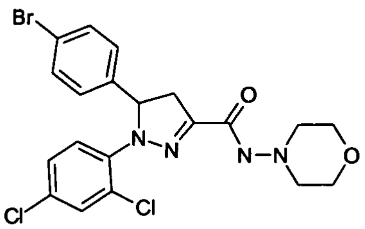 | 5-(4-Bromo-phenyl)-1-(2,4-dichloro-phenyl)-4,5-dihydro-1H-pyrazole-3-c arboxylic acid morpholin-4-ylamide | | 498 |
| 68 | 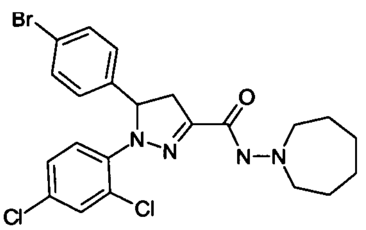 | 5-(4-Bromo-phenyl)-1-(2,4-dichloro- phenyl)-4,5-dihydro-1H-pyrazole-3-c arboxylic acid azepan-1-ylamide | 1 H NMR (300 MHz, CHLOROFORM-*d*) δ ppm 1.75 (br. s., 4 H) 2.00 (br. s., 4 H) 3.26 (dd, *J*=18.16, 6.15 Hz, 1 H) 3.71 (dd, *J*=18.16, 12.45 Hz, 1 H) 3.65 (br. s., 4 H) 5.82 (dd, *J*=12.45, 6.15 Hz, 1 H) 6.97 (d, *J*=8.35 Hz, 2 H) 7.10 (d, *J*=2.20 Hz, 1 H) 7.16 - 7.22 (m, 1 H) 7.24 (d, *J*=2.20 Hz, 1 H) 7.35 (d, *J*=8.50 Hz, 2 H) | 510 |

(continued)

| N° | STRUCTURE | Name | $^1$H-NMR | MS $(M+H)^+$ |
|---|---|---|---|---|
| 69 | 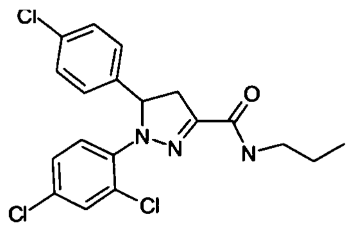 | 5-(4-Chloro-phenyl)-1-(2,4-dichloro -phenyl)-4,5-dihydro-1H-pyrazole-3-carboxylic acid propylamide | | 410 |
| 70 | 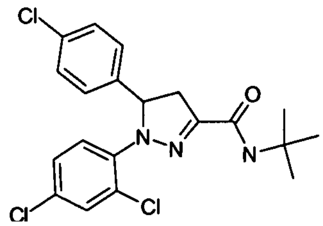 | 5-(4-Chloro-phenyl)-1-(2,4-dichloro -phenyl)-4,5-dihydro-1H-pyrazole-3-carboxylic acid tert-butylamide | | 424 |
| 71 | 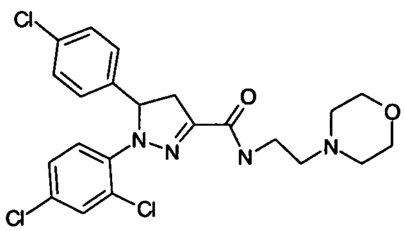 | 5-(4-Chloro-phenyl)-1-(2,4-dichloro -phenyl)-4,5-dihydro-1H-pyrazole-3-carboxylic acid (2-morpholin-4-yl-e thyl)-amide | | 481 |
| 72 | 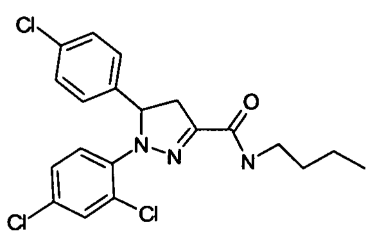 | 5-(4-Chloro-phenyl)-1-(2,4-dichloro -phenyl)-4,5-dihydro-1H-pyrazole-3-carboxylic acid butylamide | | 424 |
| 73 | 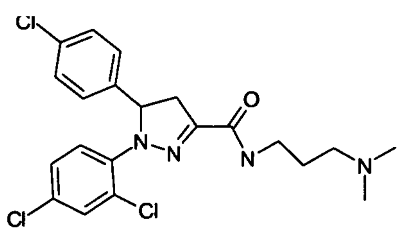 | 5-(4-Chloro-phenyl)-1-(2,4-dichloro -phenyl)-4,5-dihydro-1H-pyrazole-3-carboxylic acid (3-dimethylamino-pr opyl)-amide | | 453 |
| 74 | 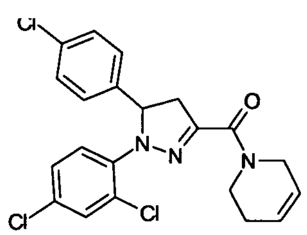 | [5-(4-Chloro-phenyl)-1-(2,4-dichlor o-phenyl)-4,5-dihydro-1H-pyrazol-3-yl]-(3,6-dihydro-2H-pyridin-1-yl)-m ethanone | | 434 |

(continued)

| N° | STRUCTURE | Name | $^1$H-NMR | MS $(M+H)^+$ |
|---|---|---|---|---|
| 75 | 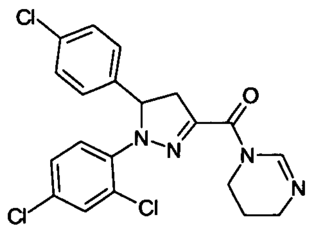 | [5-(4-Chloro-phenyl)-1-(2,4-dichlor o-phenyl)-4,5-dihydro-1H-pyrazol-3- yl]-(5,6-dihydro-4H-pyrimidin-1-yl) -methanone | | 435 |
| 76 | 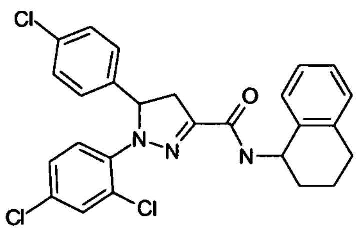 | 5-(4-Chloro-phenyl)-1-(2,4-dichloro -phenyl)-4,5-dihydro-1H-pyrazole-3-carboxylic acid (1,2,3,4-tetrahydro -naphthalen-1-yl)-amide | | 498 |
| 77 | 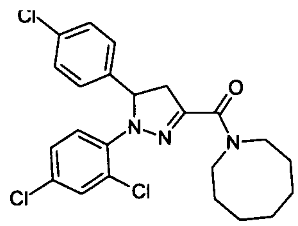 | Azocan-1-yl-[5-(4-chloro-phenyl)-1-(2,4-dichloro-phenyl)-4,5-dihydro-1 H-pyrazol-3-yl]-methanone | | 464 |
| 78 | 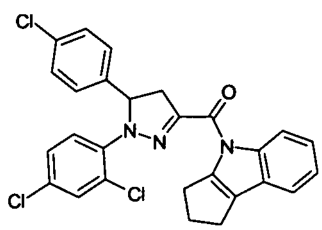 | [5-(4-Chloro-phenyl)-1-(2,4-dichlor o-phenyl)-4,5-dihydro-1H-pyrazol-3-yl]-(2,3-dihydro-1 H-cyclopenta[b]in dol-4-yl)-methanone | | 508 |
| 79 | 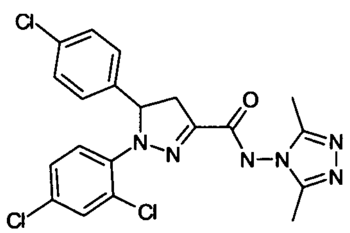 | 5-(4-Chloro-phenyl)-1-(2,4-dichloro -phenyl)-4,5-dihydro-1H-pyrazole-3-carboxylic acid (3,5-dimethyl-[1,2, 4]triazol-4-yl)-amide | | 463 |
| 80 | 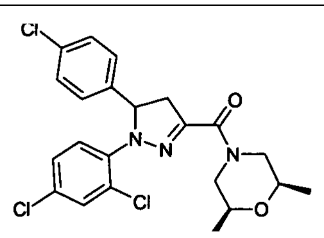 | [5-(4-Chloro-phenyl)-1-(2,4-dichlor o-phenyl)-4,5-dihydro-1H-pyrazol-3-yl]-(2,6-dimethyl-morpholin-4-yl)-methanone | | 466 |

(continued)

| N° | STRUCTURE | Name | 1H-NMR | MS (M+H)+ |
|---|---|---|---|---|
| 81 | 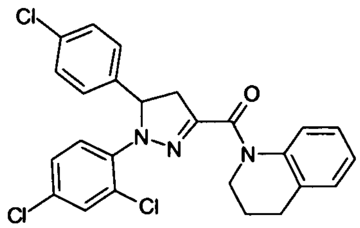 | [5-(4-Chloro-phenyl)-1-(2,4-dichlor o-phenyl)-4,5-dihydro-1H-pyrazol-3- yl]-(3,4-dihydro-2H-quinolin-1-yl)-methanone | | 484 |
| 82 | 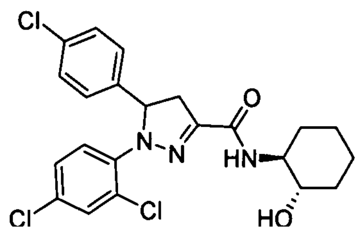 | 5-(4-chlorophenyl)-1-(2,4-dichlorophenyl)-N-((1R, 2R)-2-hydroxycyclohexyl)-4,5-dihydro-1H-pyrazole-3-carboxamide | 1H NMR (300 MHz, METHANOL-$d_4$) δ ppm 1.36 (m, 4 H) 1.75 (m, 2 H) 2.02 (m, 2 H) 3.24 (dd, *J*=5.71, 18.08 Hz, 1 H) 3.50 (m, 1 H) 3.70 (dd, *J*=18.10, 12.01 Hz, 1 H) 3.74 (m, 1 H) 5.91 (dd, *J*=12.01, 5.71 Hz, 1 H) 7.12 - 7.32 (m, 6 H) 7.45 (t, *J*=8.28 Hz, 1 H) | 466 |
| 83 | 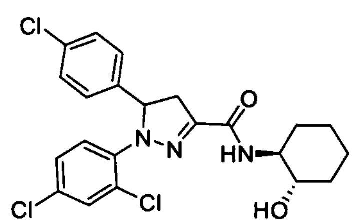 | 5-(4-chlorophenyl)-1-(2,4-dichlorophenyl)-N-((1S, 2S)-2-hydroxycyclohexyl)-4,5-dihydro-1H-pyrazole-3-carboxamide | 1 H NMR (300 MHz, METHANOL-$d_4$)δ ppm 1.36 (m, 4 H) 1.75 (m, 2 H) 2.02 (m, 2 H) 3.24 (dd, *J*=5.71, 18.08 Hz, 1 H) 3.50 (m, 1 H) 3.70 (dd, *J*=18.10, 12.01 Hz, 1 H) 3.74 (m, 1 H) 5.91 (dd, *J*=12.01, 5.71 Hz, 1 H) 7.12 - 7.32 (m, 6 H) 7.45 (t, *J*=8.28 Hz, 1 H) | 466 |
| 84 | 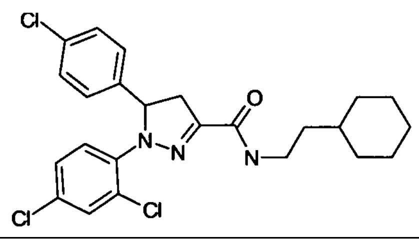 | 5-(4-Chloro-phenyl)-1-(2,4-dichloro -phenyl)-4,5-dihydro-1H-pyrazole-3-carboxylic acid (2-cyclohexyl-ethyl )-amide | | 478 |

(continued)

| N° | STRUCTURE | Name | $^1$H-NMR | MS (M+H)$^+$ |
|---|---|---|---|---|
| 85 | 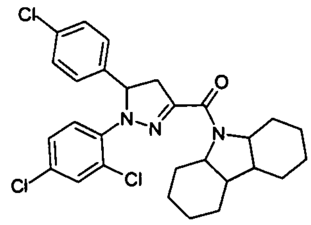 | [5-(4-Chloro-phenyl)-1-(2,4-dichlor o-phenyl)-4,5-dihydro-1H-pyrazol-3-yl]-(dodecahydro-carbazol-9-yl)-met hanone | | 530 |
| 86 | 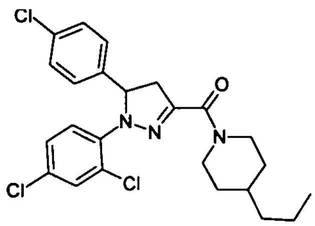 | [5-(4-Chloro-phenyl)-1-(2,4-dichlor o-phenyl)-4,5-dihydro-1H-pyrazol-3-yl]-(4-propyl-piperidin-1-yl)-metha none | | 478 |
| 87 | 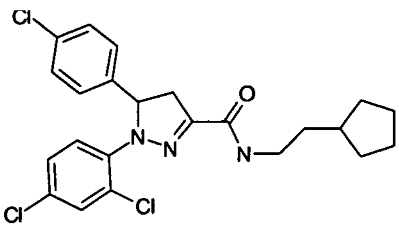 | 5-(4-Chloro-phenyl)-1-(2,4-dichloro -phenyl)-4,5-dihydro-1H-pyrazole-3-carboxylic acid (2-cyclopentyl-ethy l)-amide | | 464 |
| 88 | 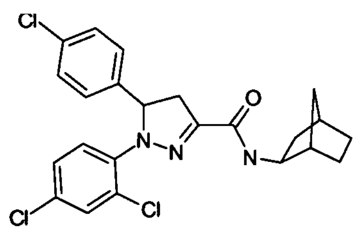 | 5-(4-Chloro-phenyl)-1-(2,4-dichloro -phenyl)-4,5-dihydro-1H-pyrazole-3-carboxylic acid bicyclo[2.2.1]hept-2-ylamide | | 462 |
| 89 | 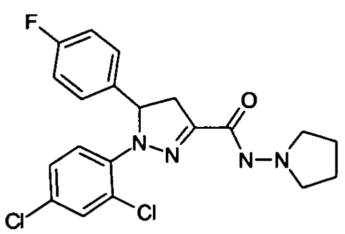 | 1-(2,4-Dichloro-phenyl)-5-(4-fluoro -phenyl)-4,5-dihydro-1H-pyrazole-3-carboxylic acid pyrrolidin-1-ylamid e | | 421 |
| 90 | 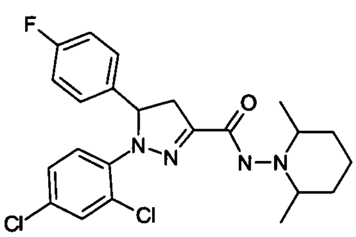 | 1-(2,4-Dichloro-phenyl)-5-(4-fluoro -phenyl)-4,5-dihydro-1H-pyrazole-3-carboxylic acid (2,6-dimethyl-piper idin-1-yl)-amide | | 463 |

(continued)

| N° | STRUCTURE | Name | $^1$H-NMR | MS $(M+H)^+$ |
|---|---|---|---|---|
| 91 | 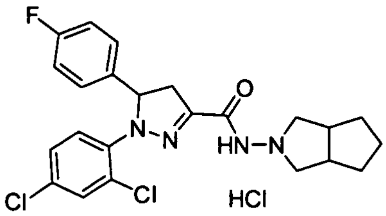 | 1-(2,4-Dichloro-phenyl)-5-(4-fluoro -phenyl) 4,5-dihydro-1H- pyrazole-3-carboxylic acid (hexahydro- cyclopen | 1H NMR (300 MHz, DMSO-$d_6$) δ ppm 1.48 (d, *J*=8.20 Hz, 2 H) 1.52 - 1.68 (m, 4 H) 2.66 (br. s., 2 H) 2.87 (m, 2 H) 3.11 (dd, *J*=18.02, 6.30 Hz, 1 H) 3.54 (m, 2 H) 3.66 (dd, *J*=18.02, 12.01 Hz, 1 H) 5.85 (dd, *J*=12.01, 6.30 Hz, 1 H) 7.06 (t, *J*=8,79 Hz, 2 H) 7.16 - 7.24 (m, 2 H) 7.30 (dd, *J*=8.72, 2.27 Hz, 1 H) 7.47 (m, 2 H) 10.58 | 461 |
| 91A | 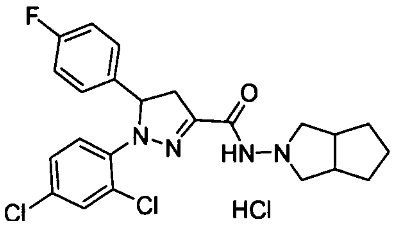 | 1-(2,4-Dichloro-phenyl)-5-(4-fluoro -phenyl)-4,5-dihydro-1 H-pyrazole-3-carboxylic acid (hexahydro-cyclopen | | |
| 92 | 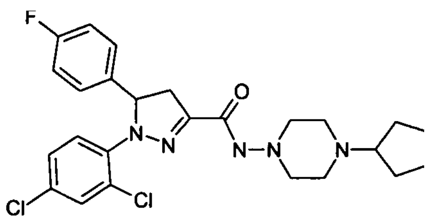 | 1-(2,4-Dichloro-phenyl)-5-(4-fluoro -phenyl)-4,5-dihydro-1H-pyrazole-3-carboxylic acid (4-cyclopentyl-pipe razin-1-yl)-amide | | 504 |
| 93 | 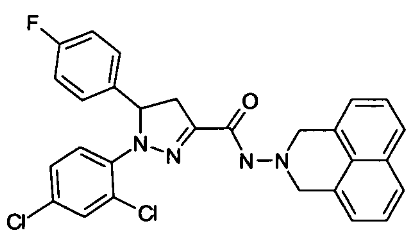 | 1-(2,4-Dichloro-phenyl)-5-(4-fluoro -phenyl)-4,5-dihydro-1H-pyrazole-3-carboxylic acid (1H,3H-benzo[de]iso quinolin-2-yl)-amide | | 519 |
| 94 | 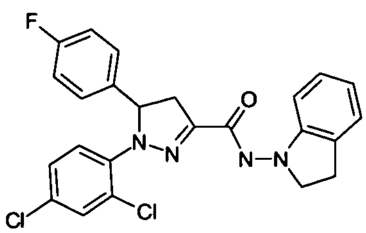 | 1-(2,4-Dichloro-phenyl)-5-(4-fluoro -phenyl)-4,5-dihydro-1H-pyrazole-3-carboxylic acid (2,3-dihydro-indol-1-yl)-amide | | 469 |

(continued)

| N° | STRUCTURE | Name | ¹H-NMR | MS (M+H)⁺ |
|---|---|---|---|---|
| 95 | 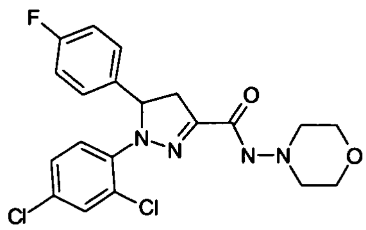 | 1-(2,4-Dichloro-phenyl)-5-(4-fluoro -phenyl)-4,5-dihydro-1H-pyrazole-3-carboxylic acid morpholin-4-ylamide | | 437 |
| 96 | 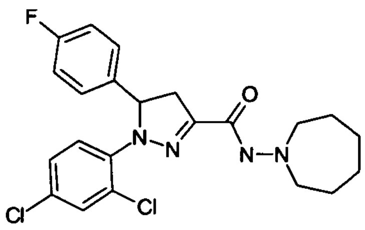 | 1-(2,4-Dichloro-phenyl)-5-(4-fluoro -phenyl)-4,5-dihydro-1H-pyrazole-3-carboxylic acid azepan-1-ylamide | 1H NMR (300 MHz, CHLOROFORM-*d*) δ ppm 1.75 (br. s., 4 H) 2.00 (br. s., 4 H) 3.28 (dd, *J*=18.16, 6.08 Hz, 1 H) 3.68 (dd, *J*=18.16, 12.38 Hz, 1 H) 3.68 (br. s., 4 H) 5.84 (dd, | 449 |
| 97 | 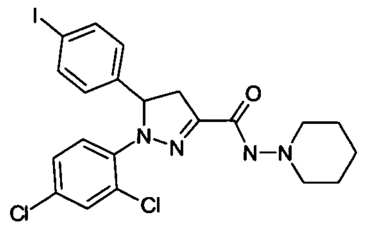 | 1-(2,4-Dichloro-phenyl)-5-(4-iodo-p henyl)-4,5-dihydro-1 H-pyrazole-3-carboxylic acid piperidin-1-ylamide | | 543 |
| 98 | 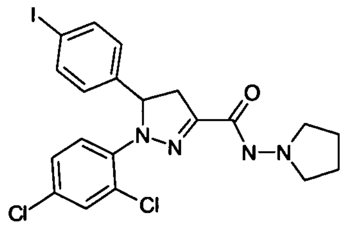 | 1-(2,4-Dichloro-phenyl)-5-(4-iodo-p henyl)-4,5-dihydro-1H-pyrazole-3-carboxylic acid pyrrolidin-1-ylamide | | 529 |
| 99 | 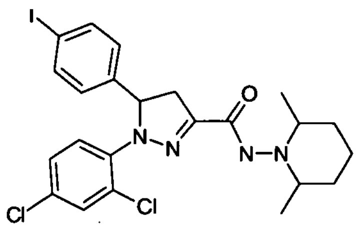 | 1-(2,4-Dichloro-phenyl)-5-(4-iodo-p henyl)-4,5-dihydro-1H-pyrazole-3-carboxylic acid (2,6-dimethyl-piperid in-1-yl)-amide | | 571 |
| 100 | 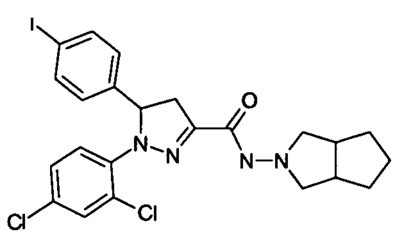 | 1-(2,4-Dichloro-phenyl)-5-(4-iodo-p henyl)-4,5-dihydro-1H-pyrazole-3-carboxylic acid (hexahydro-cyclopenta [c]pyrrol-2-yl)-amide | | 569 |

(continued)

| N° | STRUCTURE | Name | $^1$H-NMR | MS (M+H)$^+$ |
|---|---|---|---|---|
| 101 | 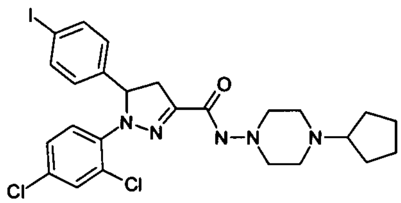 | 1-(2,4-Dichloro-phenyl)-5-(4-iodo-p henyl)-4,5-dihydro-1H-pyrazole-3-carboxylic acid (4-cyclopentyl-pipera zin-1-yl)-amide | | 612 |
| 102 | 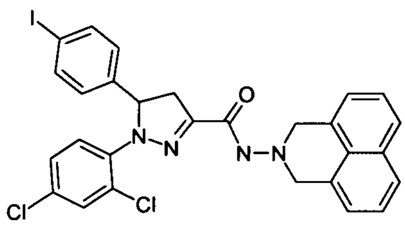 | 1-(2,4-Dichloro-phenyl)-5-(4-iodo-p henyl)-4,5-dihydro-1H-pyrazole-3-carboxylic acid (1 H,3H-benzo[de]isoqu inolin-2-yl)-amide | | 627 |
| 103 | 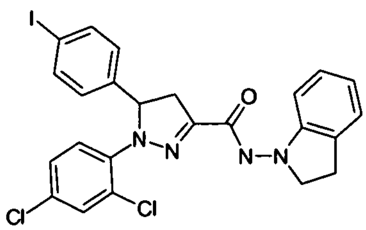 | 1-(2,4-Dichloro-phenyl)-5-(4-iodo-p henyl)-4,5-dihydro-1H-pyrazole-3-carboxylic acid (2,3-dihydro-indol-1-yl)-amide | | 577 |
| 104 | 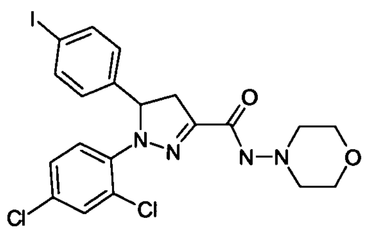 | 1-(2,4-Dichloro-phenyl)-5-(4-iodo-p henyl)-4,5-dihydro-1H-pyrazole-3-carboxylic acid morpholin-4-ylamide | | 545 |
| 105 | 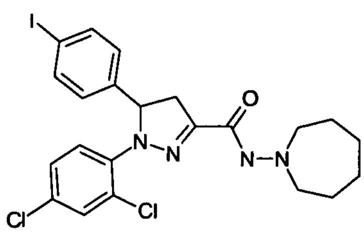 | 1-(2,4-Dichloro-phenyl)-5-(4-iodo-p henyl)-4,5-dihydro-1H-pyrazole-3-carboxylic acid azepan-1-ylamide | | 557 |

(continued)

| N° | STRUCTURE | Name | $^1$H-NMR | MS $(M+H)^+$ |
|---|---|---|---|---|
| 106 | 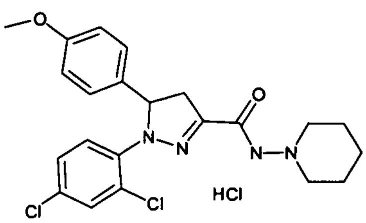 | 1-(2,4-Dichloro-phenyl)-5-(4-methox y-phenyl)-4,5-dihydro-1H-pyrazole-3-carboxylic acid piperidin-1-ylamid | 1H NMR (300 MHz, CHLOROFORM-*d*) δ ppm 1.67 (br. s., 2 H) 2.10 (br. s., 4 H) 3.25 (dd, *J*=18.02, 6.01 Hz, 1 H) 3.64 (dd, *J*=18.02, 12.60 Hz, 1H) 3.72 (s, 3 H) 3.89 (br. s., 4 H) 5.85 (dd, *J*=12.60, 6.01 Hz, 1 H) 6.72 (d, *J*=8.79 Hz, 2H) 7.00 (d, *J*=8.64 Hz, 2H) 7.07 (dd, *J*=8.64, 2.20 Hz, 1H) 7.22 (d, *J*=8.64 Hz, 1 H) 7.22 (d, | 447 |
| 107 | 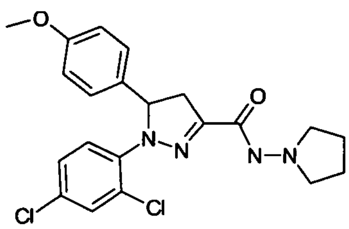 | 1-(2,4-Dichloro-phenyl)-5-(4-methox y-phenyl)-4,5-dihydro-1H-pyrazole-3-carboxylic acid pyrrolidin-1-ylami de | | 433 |
| 108 | 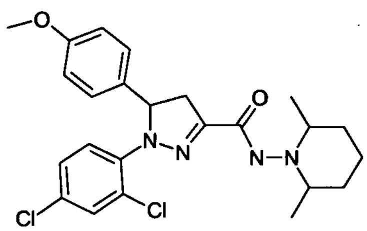 | 1-(2,4-Dichloro-phenyl)-5-(4-methox y-phenyl)-4,5-dihydro-1H-pyrazole-3-carboxylic acid (2,6-dimethyl-pipe ridin-1-yl)-amide | | 475 |

(continued)

| N° | STRUCTURE | Name | $^1$H-NMR | MS $(M+H)^+$ |
|---|---|---|---|---|
| 109 | 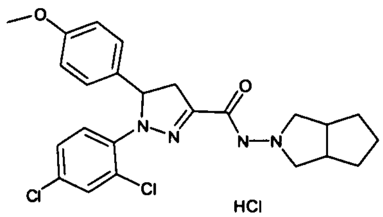 | 1-(2,4-Dichloro-phenyl)-5-(4-methoxy-phenyl)-4,5-dihydro-1H-pyrazole-3 -carboxylic acid hexahydro-cyclope | 1H NMR (300 MHz, CHLOROFORM-*d*) δ ppm 1.61 - 1.91 (m, 6 H) 2.96 (br. s., 2 H) 3.27 (dd, *J*=18.09, 5.86 Hz, 1 H) 3.49 (m, 2 H) 3.64 (dd, *J*=18.09, 12.60 Hz, 1 H) 3.72 (s, 3 H) 3.90 (br. s., 2 H) 5.82 (dd, *J*=12.60, 5.86 Hz, 1 H) 6.71 (d, *J*=8.64 Hz, 2 H) 7.00 (d, *J*=8.64 Hz, 2 H) 7.06 (dd, *J*=8.72, 2.27 Hz, 1 H) 7.21 (m, 2 H) 9.25 | 473 |
| 109 A | 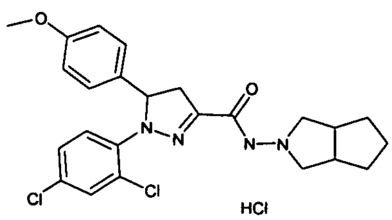 | 1-(2,4-Dichloro-phenyl)-5-(4-methox y-phenyl)-4,5-dihydro-1H-pyrazole-3-carboxylic acid (hexahydro-cyclope | | |
| 110 | 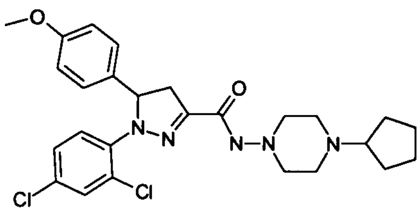 | 1-(2,4-Dichloro-phenyl)-5-(4-methox y-phenyl)-4,5-dihydro-1H-pyrazole-3-carboxylic acid (4-cyclopentyl-pip erazin-1-yl)-amide | | 516 |
| 111 | 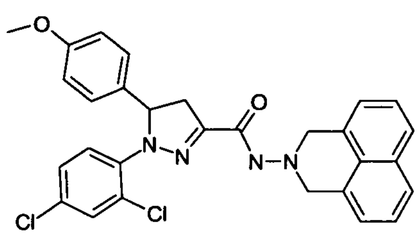 | 1-(2,4-Dichloro-phenyl)-5-(4-methox y-phenyl)-4,5-dihydro-1H-pyrazole-3-carboxylic acid (1 H,3H-benzo[de] is oquinolin-2-yl)-amide | | 531 |
| 112 | 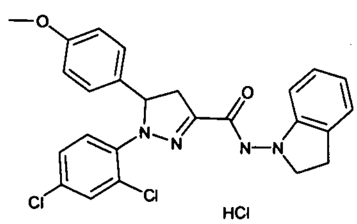 | 1-(2,4-Dichloro-phenyl)-5-(4-methox y-phenyl)-4,5-dihydro-1H-pyrazole-3-carboxylic acid (2,3-dihydro-indol -1-yl)-amide hydrochloride | | 481 |

(continued)

| N° | STRUCTURE | Name | 1H-NMR | MS (M+H)+ |
|---|---|---|---|---|
| 113 | 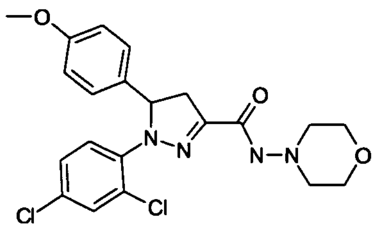 | 1-(2,4-Dichloro-phenyl)-5-(4-methox y-phenyl)-4,5-dihydro-1H-pyrazole-3-carboxylic acid morpholin-4-ylamide | | 449 |
| 114 | 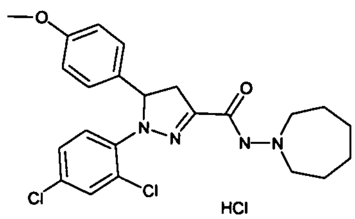 HCl | 1-(2,4-Dichloro-phenyl)-5-(4-methoxy-phenyl)-4,5-dihydro-1H-pyrazole-3 -carboxylic acid azepan-1-ylamide hydrochloride | 1H NMR (300 MHz, METHANOL-$d_4$) δ ppm 1.77 (br. s., 4 H) 1.98 (br. s., 4 H) 3.27 (dd, *J*=6.15 Hz, 1 H) 3.63 (m, 5 H) 3.70 (s, 3 H) 5.90 (dd, *J*=12.45, 6.15 Hz, 1 H) 6.76 (d, *J*=8.64 Hz, 2 H) 7.16 (dd, *J*=8.64, 2.20 Hz, 1 H) 7.10 (d, *J*=8.64 Hz, 2 H) 7.33 (m, 2 H) | 461 |
| 115 | 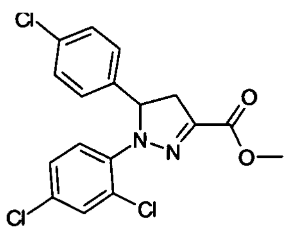 | 5-(4-Chloro-phenyl)-1-(2,4-dichloro -phenyl)-4,5-dihydro-1H-pyrazole-3- carboxylic acid methyl ester | | 383 |
| 116 | 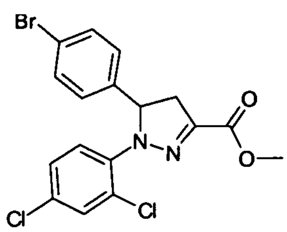 | 5-(4-Bromo-phenyl)-1-(2,4-dichloro-phenyl)-4,5-dihydro-1H-pyrazole-3-carboxylic acid methyl ester | | 427 |
| 117 | 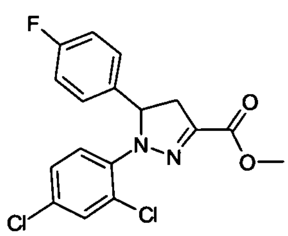 | 1-(2,4-Dichloro-phenyl)-5-(4-fluoro -phenyl)-4,5-dihydro-1H-pyrazole-3-carboxylic acid methyl ester | | 367 |

(continued)

| N° | STRUCTURE | Name | $^1$H-NMR | MS (M+H)$^+$ |
|---|---|---|---|---|
| 118 | 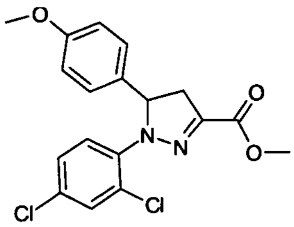 | 1-(2,4-Dichloro-phenyl)-5-(4-methox y-phenyl)-4,5-dihydro-1H-pyrazole-3-carboxylic acid methyl ester | | 379 |
| 119 | 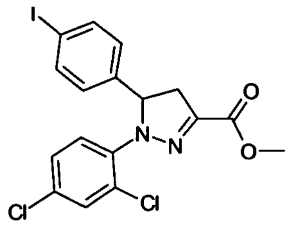 | 1-(2,4-Dichloro-phenyl)-5-(4-iodo-p henyl)-4,5-dihydro-1 H-pyrazole-3-carboxylic acid methyl ester | | 475 |
| 120 | 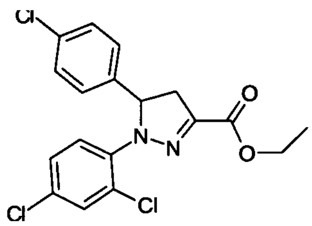 | 5-(4-Chloro-phenyl)-1-(2,4-dichloro -phenyl)-4,5-dihydro-1H-pyrazole-3-carboxylic acid ethyl ester | | 397 |
| 121 | 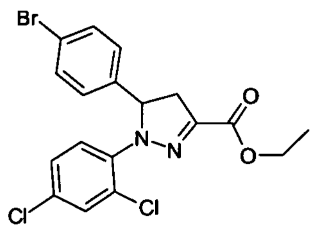 | 5-(4-Bromo-phenyl)-1-(2,4-dichloro-phenyl)-4,5-dihydro-1H-pyrazole-3-carboxylic acid ethyl ester | | 441 |
| 122 | 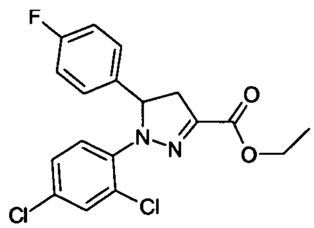 | 1-(2,4-Dichloro-phenyl)-5-(4-fluoro -phenyl)-4,5-dihydro-1H-pyrazole-3-carboxylic acid ethyl ester | | 381 |
| 123 | 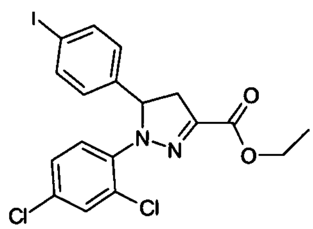 | 1-(2,4-Dichloro-phenyl)-5-(4-iodo-p henyl)-4,5-dihydro-1H-pyrazole-3-carboxylic acid ethyl ester | | 489 |

(continued)

| N° | STRUCTURE | Name | $^1$H-NMR | MS (M+H)$^+$ |
|---|---|---|---|---|
| 124 | 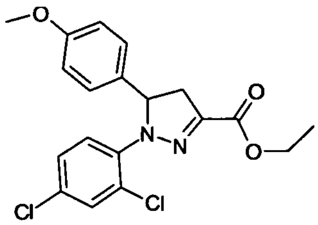 | 1-(2,4-Dichloro-phenyl)-5-(4-methox y-phenyl)-4,5-dihydro-1H-pyrazole-3-carboxylic acid ethyl ester | | 393 |
| 125 | 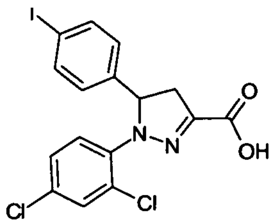 | 1-(2,4-Dichloro-phenyl)-5-(4-iodo-p henyl)-4,5-dihydro-1H-pyrazole-3-ca rboxylic acid | | 461 |
| 126 | 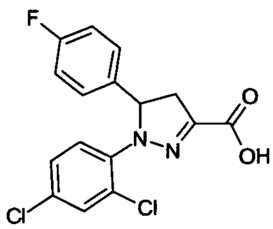 | 1-(2,4-Dichloro-phenyl)-5-(4-fluoro-phenyl)-4,5-dihydro-1H- pyrazole-3-carboxylic acid | | 353 |
| 127 | 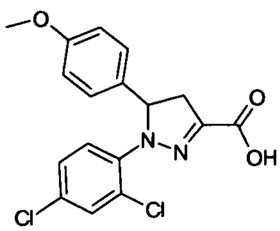 | 1-(2,4-Dichloro-phenyl)-5-(4-methox y-phenyl)-4,5-dihydro-1 H-pyrazole-3-carboxylic acid | | 365 |
| 128 | 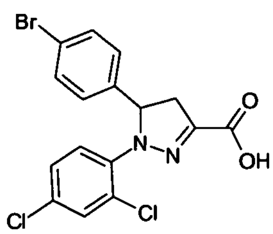 | 5-(4-Bromo-phenyl)-1-(2,4-dichloro-phenyl)-4,5-dihydro-1H-pyrazole-3-carboxylic acid | | 413 |
| 129 | 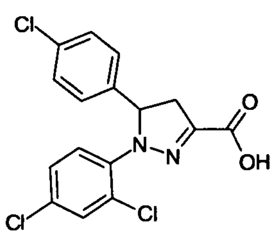 | 5-(4-Chloro-phenyl)-1-(2,4-dichloro -phenyl)-4,5-dihydro-1H-pyrazole-3-carboxylic acid | | 369 |

(continued)

| N° | STRUCTURE | Name | $^1$H-NMR | MS $(M+H)^+$ |
|---|---|---|---|---|
| 130 | 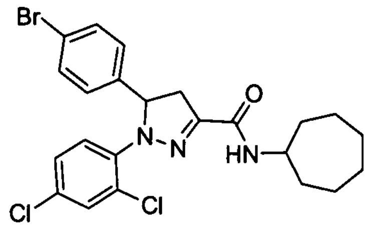 | 5-(4-Bromo-phenyl)-1-(2,4-dichloro- phenyl)-4,5-dihydro-1H-pyrazole-3-carboxylic acid cycloheptylamide | 1 H NMR (300 MHz, METHANOL-$d_4$) δ ppm 1.49 - 1.78 (m, 10 H) 1.95 (br. s., 2 H) 3.21 (dd, *J*=18.09, 5.79 Hz, 1 H) 3.67 (dd, *J*=18.09, 12.08 Hz, 1 H) 3.99 (m, 1 H) 5.86 (dd, *J*=12.08, 5.79 Hz, 1 H) 7.09 (d, | 508 |
| 131 | 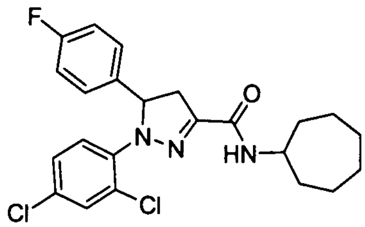 | 5-(4-Fluorophenyl)-1-(2,4- dichloro-phenyl)-4,5-dihydro-1H-pyrazole-3-carboxylic acid cycloheptylamide | 1H NMR (300 MHz, DMSO-$d_6$) δ ppm 1.36 - 1.67 (m, 10 H) 1.72 - 1.87 (m, 2 H) 3.05 (dd, *J*=18.02, 5.64 Hz, 1H) 3.63 (dd, *J*=18.02, 11.79 Hz, 1H) 3.79 - 3.92 (m, *J*=8.95, 1 H) 5.80 (dd, *J*=11.79, 5.64 Hz, 1 H) 7 04 (t *J*=8 79 Hz 2H) 7 12 | 448 |
| 132 | 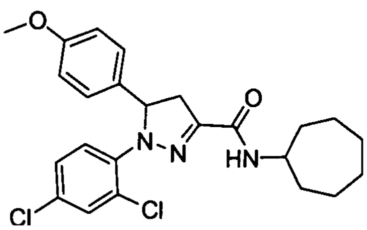 | 5-(4-methoxyphenyl)-1-(2,4-dichloro-phenyl)-4,5-dihydro-1H-pyrazole-3-carboxylic acid cycloheptylamide | | 460 |
| 133 | 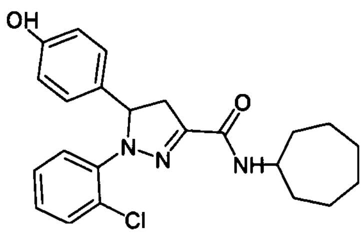 | 5-(4-hydroxyphenyl)-1-(2,4-dichloro-phenyl)-4,5-dihydro-1H-pyrazole-3-carboxylic acid cycloheptylamide | | 412 |
| 134 | 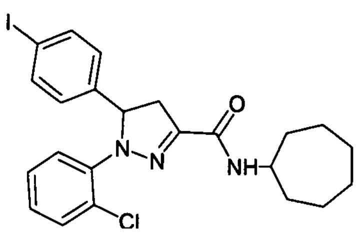 | 5-(4-iodophenyl)-1-(2,4-dichloro-phenyl)-4,5-dihydro-1H-pyrazole-3-carboxylic acid cycloheptylamide | | 522 |

(continued)

| N° | STRUCTURE | Name | $^1$H-NMR | MS $(M+H)^+$ |
|---|---|---|---|---|
| 135 | 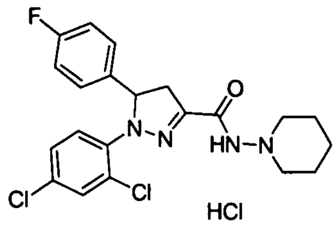 | 1-(2,4-Dichloro-phenyl)-5-(4- fluoro -phenyl)-4,5-dihydro-1H- pyrazole-3-carboxylic acid piperidin-1-ylamide hydrochloride | 1H NMR (300 MHz, DMSO-$d_6$) δ ppm 1.42 (br. s., 2 H) 1.72 (br. s., 4 H) 3.08 (dd, *J*=18.02, 6.23 Hz, 1 H) 3.14 (br. s., 4 H) 3.69 (dd, *J*=18.02, 12.08 Hz, 1 H) 5.84 (dd, *J*=12.08, 6.23 Hz, 1 H) 7.06 (t, *J*=8.79 Hz, 2 H) 7 20 (dd. *J*=8 72.5 49 Hz.2 H) | 435 |
| 136 | 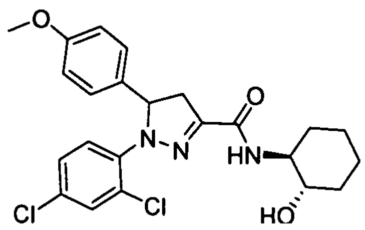 | 1-(2,4-dichlorophenyl)-N-((1S, 2S)-2-hydroxycyclohexyl)-5-(4-methoxyphenyl)-4,5-dihydro-1H-pyrazole-3-carboxamide | | 462 |
| 137 | 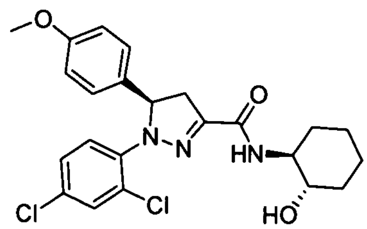 | (R)-1-(2,4-dichlorophenyl)-N-((1 S, 2S)-2-hydroxycyclohexyl)-5-(4-methoxyphenyl)-4,5-dihydro-1H-pyrazole-3-carboxamide | | 462 |
| 138 | 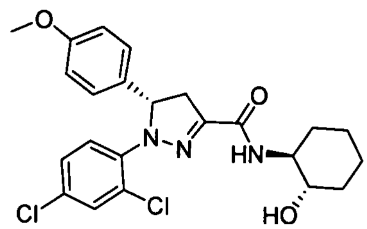 | (S)-1-(2,4-dichlorophenyl)-N-((1 S, 2S)-2-hydroxycyclohexyl)-5-(4-methoxyphenyl)-4,5- dihydro-1H-pyrazole-3-carboxamide | | 462 |
| 139 | 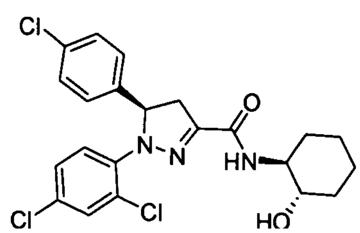 | (R)-5-(4-chlorophenyl)-1-(2,4-dichlorophenyl)-N-((1 S, 2S)-2-hydroxycyclohexyl)-4,5-dihydro-1H-pyrazole-3-carboxamide | | 466 |

(continued)

| N° | STRUCTURE | Name | 1H-NMR | MS (M+H)+ |
|---|---|---|---|---|
| 140 | 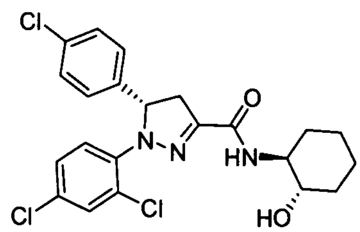 | (S)-5-(4-chlorophenyl)-1-(2,4-dichlorophenyl)-N-((1S, 2S)-2-hydroxycyclohexyl)-4,5-dihydro-1H-pyrazole-3-carboxamide | | 466 |
| 141 | 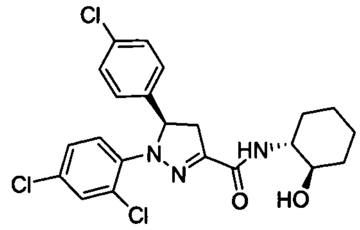 | (R)-5-(4-chlorophenyl)-1-(2,4-dichlorophenyl)-N-((1 R, 2R)-2-hydroxycyclohexyl)-4,5-dihydro-1H-pyrazole-3-carboxamide | | 466 |
| 142 | 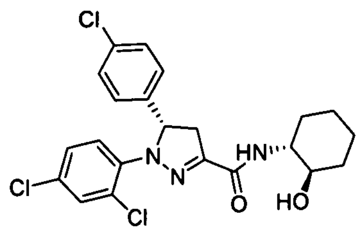 | (S)-5-(4-chlorophenyl)-1-(2,4-dichlorophenyl)-N-((1R, 2R)-2-hydroxycyclohexyl)-4,5-dihydro-1H-pyrazole-3-carboxamide | | 466 |
| 143 | 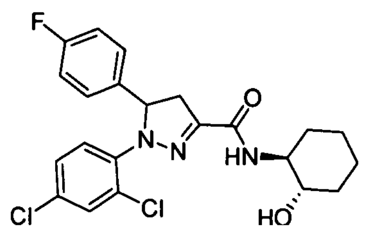 | 1-(2,4-dichlorophenyl)-5-(4-fluorophenyl)-N-((1S, 2S)-2-hydroxycyclohexyl)-4,5-dihydro-1H-pyrazole-3-carboxamide | | 450 |
| 144 | 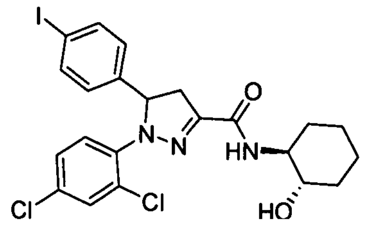 | 1-(2,4-dichlorophenyl)-5-(4-iodophenyl)-N-((1S, 2S)-2-hydroxycyclohexyl)-4,5-dihydro-1H-pyrazole-3-carboxamide | | 558 |
| 145 | 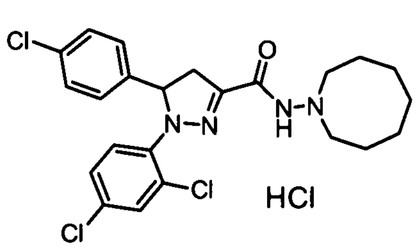 | 5-(4-Chloro-phenyl)-1-(2,4- dichloro -phenyl)-4,5-dihydro-1H- pyrazole-3-carboxylic acid azocan-1-ylamide hydrochloride | 1 H NMR (300 MHz, METHANOL-$d_4$) δ ppm 1.76 (br. s., 6 H) 2.01 (br. s., 4 H) 3.26 (dd, *J*=18.02, 6.52 Hz, 1 H) 3.65 (m, 4 H) 3.76 (dd, *J*=18.02, 12.52 Hz, 1 H) 5.96 (dd, *J*=12.52, 6.52 Hz, 1 H) | 479 |

(continued)

| N° | STRUCTURE | Name | $^1$H-NMR | MS (M+H)$^+$ |
|---|---|---|---|---|
| 146 | 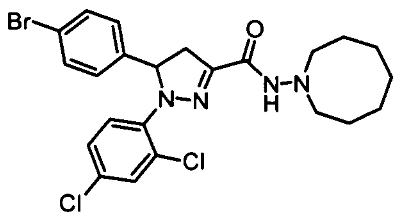 | 5-(4-bromo-phenyl)-1-(2,4-dichloro -phenyl)-4,5-dihydro-1H-pyrazole-3-carboxylic acid azocan-1-ylamide | | 523 |
| 147 | 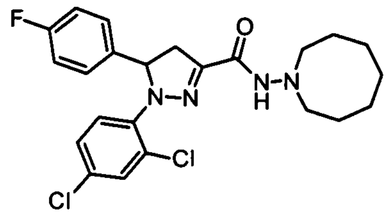 | 5-(4-fluoro-phenyl)-1-(2,4-dichloro -phenyl)-4,5-dihydro-1H-pyrazole-3-carboxylic acid azocan-1-ylamide | | 463 |
| 148 | 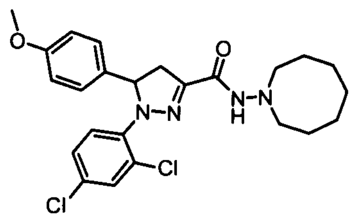 | 5-(4-methoxy-phenyl)-1-(2,4- dichloro -phenyl)-4,5-dihydro-1H- pyrazole-3-carboxylic acid azocan-1- ylamide | 1H NMR (300 MHz, CHLOROFORM-*d*) δ ppm 1.53 -1.62 (m, 2 H) 1.68 (br. s., 8 H) 3.10 (m, 4 H) 3.34 (dd, *J*=18.31, 6.01 Hz, 1 H) 3.64 (dd, *J*=18.31, 12.01 Hz, 1 H) 3.72 (s, 3 H) 5.68 (dd, *J*=12.01, 6 01 $H_7$ 1 H) 6 70 (d *J*=8 64 | 475 |
| 149 | 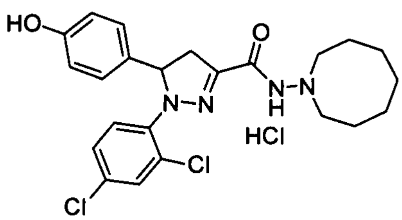 | 5-(4-hydroxy-phenyl)-1-(2,4-dichloro -phenyl)-4,5-dihydro-1H-pyrazole-3-carboxylic acid azocan-1-ylamide hydrochloride | | 461 |
| 150 | 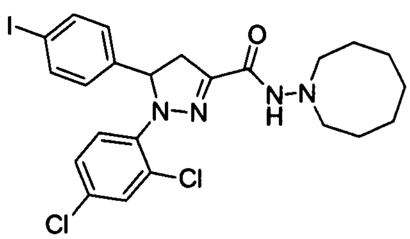 | 5-(4-iodo-phenyl)-1-(2,4-dichloro -phenyl)-4,5-dihydro-1H-pyrazole-3-carboxylic acid azocan-1-ylamide | | 571 |

(continued)

| N° | STRUCTURE | Name | $^1$H-NMR | MS $(M+H)^+$ |
|---|---|---|---|---|
| 151 | 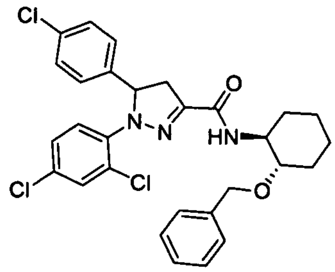 | N-((1S,2S)-2-(benzyloxy) cyclohexyl)-5-(4-chlorophenyl)-1-(2,4-dichlorophenyl)-4,5-dihydro-1H-pyrazole-3-carboxamide | | 556 |
| 152 | 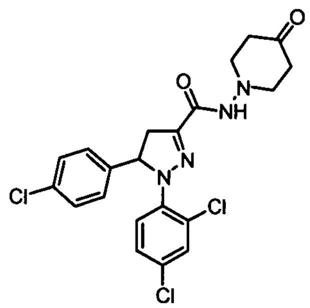 | 5-(4-Chloro-phenyl)-1-(2,4-dichloro -phenyl)-4,5-dihydro-1H-pyrazole-3-carboxylic acid (4-oxo-piperidin-1-yl)-amide | | 465 |
| 153 | 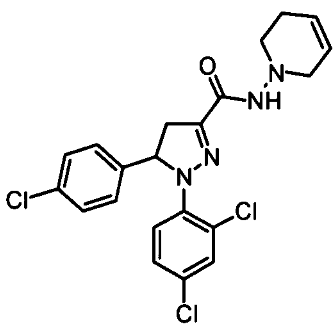 | 5-(4-Chloro-phenyl)-1-(2,4-dichloro -phenyl)-4,5-dihydro-1H-pyrazole-3-carboxylic acid (3,6-dihydro- 2H-pyridin-1-yl)-amide | 1 H NMR (250 MHz, DMSO-$d_6$) δ ppm 2.20 (br. s., 2 H) 2.96 (t, *J*=5.63 Hz, 2 H) 3.08 (dd, *J*=17.98, 5.76 Hz, 1 H) 3.37 (br. s., 2 H) 3.67 (dd, *J*=17.98, 11.80 Hz, 1 H) 5.58 - 5.74 (m, 2 H) 5.81 (dd, *J*=11.80, 5.76 Hz, 1 H) 7.13 - 7.24 (m, 2 H) 7.30 (d *J*=8.23 Hz 3 H) 7.51 (d | 449 |
| 154 | 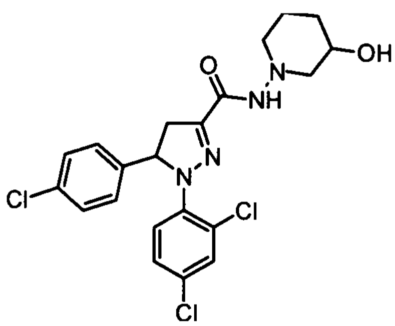 | 5-(4-Chloro-phenyl)-1-(2,4-dichloro -phenyl)-4,5-dihydro-1H-pyrazole-3-carboxylic acid (3-hydroxy-piperidi n-1-yl)-amide | 1 H NMR (300 MHz, DMSO-$d_6$) δ ppm 1.05 (d, *J*=10.26 Hz, 1 H) 1.43 (d, *J*=10.55 Hz, 1 H) 1.52- 1.82 (m, 2 H) 2.57 (m, 1 H) 2.83 (m, 1 H) 3.04 (m, 2 H) 3.41 - 3.70 (m, 2 H) 4.75 (t, J=3.66 Hz, 1 H) 5.78 (dd, *J*=11.57, 5.42 Hz, 1 H) 7.14 (d, *J*=8 20 Hz 2 H) 7 28 (d *J*=7 91 | 467 |

(continued)

| N° | STRUCTURE | Name | 1H-NMR | MS (M+H)+ |
|---|---|---|---|---|
| 155 | 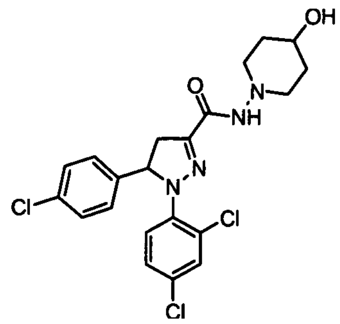 | 5-(4-Chloro-phenyl)-1-(2,4-dichloro -phenyl)-4,5-dihydro-1H-pyrazole-3-carboxylic acid (4-hydroxy- piperidin-1-yl)-amide | 1 H NMR (250 MHz, CHLOROFORM-*d*) δ ppm 1.91 (m, 2 H) 2.08 (m, 2 H) 2.89 (m, 2 H) 3.15 (m, 2 H) 3.40 (dd, *J*=18.39, 6.31 Hz, 1 H) 3.76 (dd, *J*=18.39, 12.08 Hz, 1 H) 3.91 (d, *J*=3.57 Hz, 1 H) 5.80 (dd, *J*=12.08, 6.31 Hz, 1 H) 7.09 - 7.20 (m 4 H) 7.24 (m 2 | 467 |
| 156 | 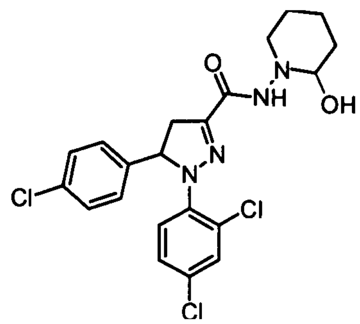 | 5-(4-Chloro-phenyl)-1-(2,4-dichloro -phenyl)-4,5-dihydro-1H-pyrazole-3-carboxylic acid (2-hydroxy-piperidin-1-yl)-amide | | 467 |
| 157 | 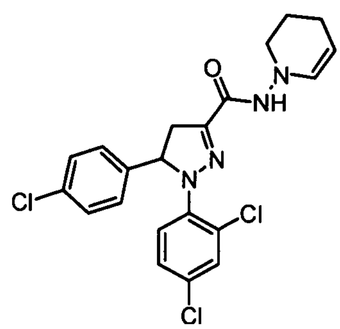 | 5-(4-Chloro-phenyl)-1-(2,4-dichloro -phenyl)-4,5-dihydro-1H-pyrazole-3-carboxylic acid (3,4-dihydro-2H-pyr idin-1-yl)-amide | | 449 |
| 158 | 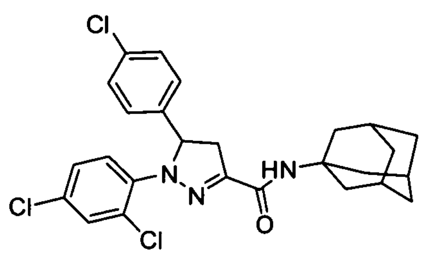 | 5-(4-Chloro-phenyl)-1-(2,4-dichloro -phenyl)-4,5-dihydro-1H-pyrazole-3-carboxylic acid adamantan-1-ylamide | | 502 |

(continued)

| N° | STRUCTURE | Name | $^1$H-NMR | MS (M+H)$^+$ |
|---|---|---|---|---|
| 159 | 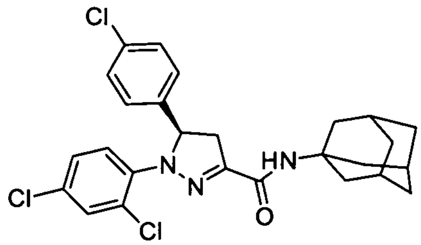 | (R)-5-(4-Chloro-phenyl)-1-(2,4-dichloro-phenyl)-4,5-dihydro-1H-pyrazole-3-carboxylic acid adamantan-1-ylamide | | 502 |
| 160 | 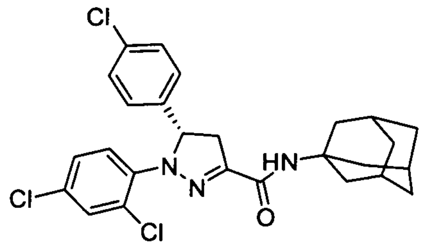 | (S)-5-(4-Chloro-phenyl)-1-(2,4-dichloro-phenyl)-4,5-dihydro-1H-pyrazole-3-carboxylic acid adamantan-1-ylamide | | 502 |
| 161 | 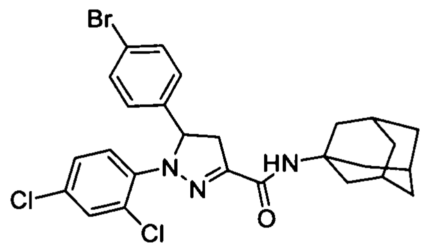 | 5-(4-bromo-phenyl)-1-(2,4-dichloro -phenyl)-4,5-dihydro-1H-pyrazole-3-carboxylic acid adamantan-1-ylamide | | 546 |
| 162 | 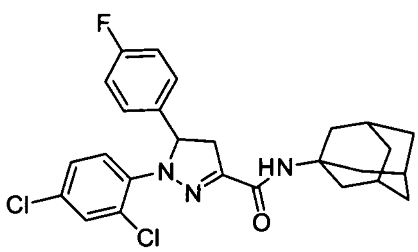 | 5-(4-fluoro-phenyl)-1-(2,4-dichloro -phenyl)-4,5-dihydro-1H-pyrazole-3-carboxylic acid adamantan-1-ylamide | | 486 |
| 163 | 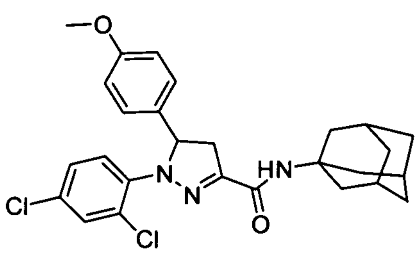 | 5-(4-methoxy-phenyl)-1-(2,4-dichloro -phenyl)-4,5-dihydro-1H-pyrazole-3-carboxylic acid adamantan-1-ylamide | | 498 |

(continued)

| N° | STRUCTURE | Name | $^1$H-NMR | MS $(M+H)^+$ |
|---|---|---|---|---|
| 164 | 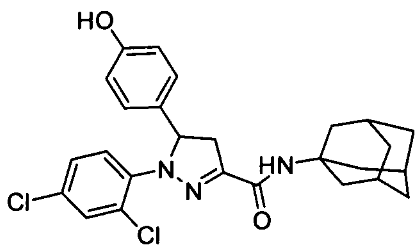 | 5-(4-hydroxy-phenyl)-1-(2,4-dichloro -phenyl)-4,5-dihydro-1H-pyrazole-3-carboxylic acid adamantan-1-ylamide | | 484 |
| 165 | 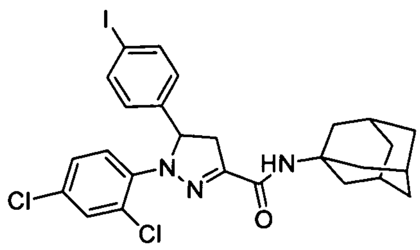 | 5-(4-iodo-phenyl)-1-(2,4-dichloro -phenyl)-4,5-dihydro-1H-pyrazole-3-carboxylic acid adamantan-1-ylamide | | 594 |
| 166 | 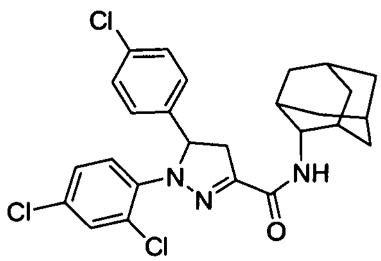 | 5-(4-Chloro-phenyl)-1-(2,4-dichloro -phenyl)-4,5-dihydro-1H-pyrazole-3-carboxylic acid adamantan-2-ylamide | | 502 |
| 167 | 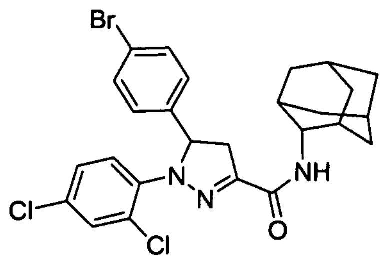 | 5-(4-bromo-phenyl)-1-(2,4-dichloro -phenyl)-4,5-dihydro-1H-pyrazole-3-carboxylic acid adamantan-2-ylamide | | 546 |
| 168 | 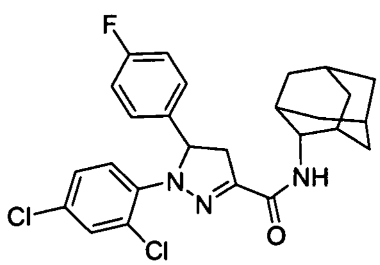 | 5-(4-fluoro-phenyl)-1-(2,4-dichloro -phenyl)-4,5-dihydro-1H-pyrazole-3-carboxylic acid adamantan-2-ylamide | | 486 |

(continued)

| N° | STRUCTURE | Name | $^1$H-NMR | MS $(M+H)^+$ |
|---|---|---|---|---|
| 169 | 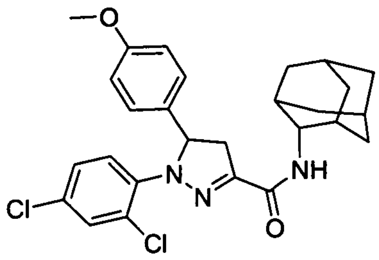 | 5-(4-methoxy-phenyl)-1-(2,4-dichloro -phenyl)-4,5-dihydro-1H-pyrazole-3-carboxylic acid adamantan-2-ylamide | | 498 |
| 170 | | 5-(4-hydroxy-phenyl)-1-(2,4-dichloro -phenyl)-4,5-dihydro-1H-pyrazole-3-carboxylic acid adamantan-2-ylamide | | 484 |
| 171 | 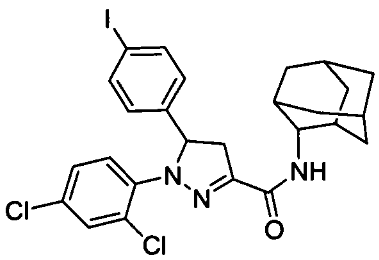 | 5-(4-iodo-phenyl)-1-(2,4-dichloro -phenyl)-4,5-dihydro-1H-pyrazole-3-carboxylic acid adamantan-2-ylamide | | 594 |
| 172 | 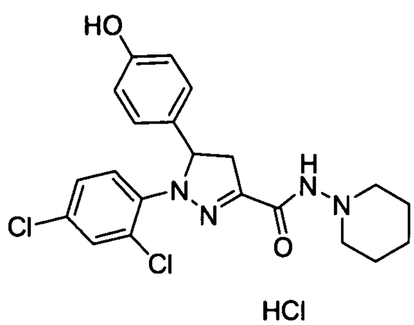 | 1-(2,4-Dichloro-phenyl)-5-(4- hydrox y-phenyl)-4,5-dihydro-1H-pyrazole-3-carboxylic acid piperidin-1-ylamide hydrochloride | 1 H NMR (300 MHz, METHANOL-$d_4$) δ ppm 1.69 (br. s., 2 H) 2.00 (br. s., 4 H) 3.24 (dd, *J*=17.94, 6.23 Hz, 1 H) 3.55 (br. s., 4 H) 3.69 (dd, *J*=17.94, 12.38 Hz, 1H) 5.86 (dd, *J*=12.38, 6.23 Hz, 1H) 6.61 (d, *J*=8.50 Hz, 2 H) 7.00 (d*J*=8.50 Hz 2 H) 7.17 (dd | 433 |

(continued)

| N° | STRUCTURE | Name | $^1$H-NMR | MS $(M+H)^+$ |
|---|---|---|---|---|
| 173 | 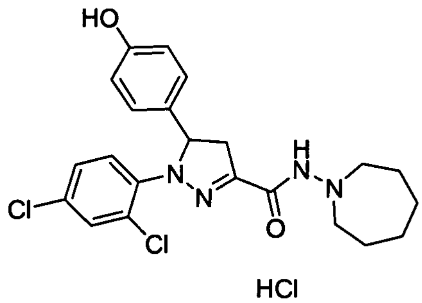 | 1-(2,4-Dichloro-phenyl)-5-(4-hydrox y-phenyl)-4,5-dihydro-1 H-pyrazole-3-carboxylic acid azepan-1-ylamide hydrochloride | 1 H NMR (300 MHz, DMSO-$d_6$) δ ppm 0.81 (d, J=7.18 Hz, 3 H) 1.59 (br. s., 4 H) 1.78 (br. s., 4 H) 3.36 (br. s., 4 H) 3.76 (dq, *J*=11.28, 7.18 Hz, 1H) 5.81 (d, *J*=11.28 Hz, 1 H) 6.59 (d, *J*=8.50 Hz, 2 H) 6.89 (d, *J*=8.50 Hz, 2 H) 7.31 (dd, *J*=8.79, 2.34 Hz 1 H) 7.49 (d *J*=2.34 Hz 1 | 447 |
| 174 | 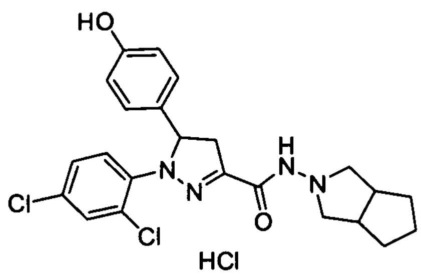 | 1-(2,4-Dichloro-phenyl)-5-(4-hydrox y-phenyl)-4,5-dihydro-1H-pyrazole-3-carboxylic acid (hexahydro-cyclope nta[c]pyrrol-2-yl)-amide hydrochloride | 1 H NMR (300 MHz, METHANOL-$d_4$) □ ppm 1.68 (br. s., 2 H) 1.81 (br. s., 4 H) 2.98 (br. s., 2 H) 3.06 - 3.21 (m, 2 H) 3.26 (dd, *J*=18.02, 6.30 Hz, 1 H) 3.71 (dd, *J*=18.02, 12.45 Hz, 1 H) 4.06 (m, 2 H) 5.88 (dd, *J*=12.45, 6.30 Hz, 1 H) 6.63 (d *J*=8.64 Hz 2 H) | 459 |
| 175 | 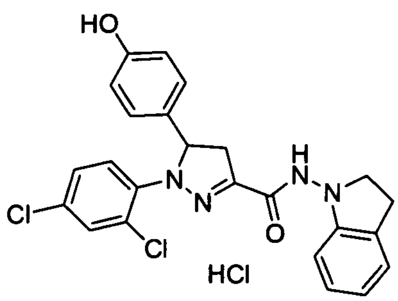 | 1-(2,4-Dichloro-phenyl)-5-(4-hydrox y-phenyl)-4,5-dihydro-1H-pyrazole-3 -carboxylic acid (2,3-dihydro-indol -1-yl)-amide hydrochloride | | 467 |

(continued)

| N° | STRUCTURE | Name | $^1$H-NMR | MS $(M+H)^+$ |
|---|---|---|---|---|
| 176 | 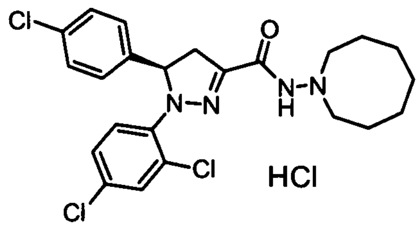 | (R)-5-(4-Chloro-phenyl)-1-(2,4- dichloro-phenyl)-4,5-dihydro-1H-pyrazole-3-carboxylic acid azocan-1-ylamide hydrochloride | 1 H NMR (300 MHz, METHANOL-$d_4$) δ ppm 1.76 (br. s., 6 H) 2.01 (br. s., 4 H) 3.26 (dd, *J*=18.02, 6.52 Hz, 1 H) 3.65 (m, 4 H) 3.76 (dd, *J*=18.02, 12.52 Hz, 1 H) 5.96 (dd, *J*=12.52, 6.52 Hz, 1 H) 7.16 - 7.28 (m, 5 H) 7.40 (d, *J*=8.64 Hz 1 H) 7.37 (d *J*=2.34 | 479 |
| 177 | 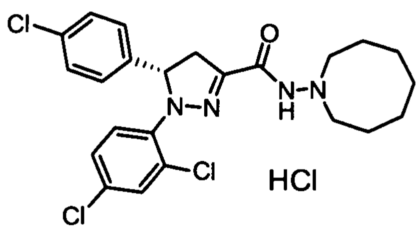 | (S)-5-(4-Chloro-phenyl)-1-(2,4- dichloro-phenyl)-4,5-dihydro-1H-pyrazole-3-carboxylic acid azocan-1-ylamide hydrochloride | 1 H NMR (300 MHz, METHANOL-$d_4$) δ ppm 1.76 (br. s., 6 H) 2.01 (br. s., 4 H) 3.26 (dd, *J*=18.02, 6.52 Hz, 1 H) 3.65 (m, 4 H) 3.76 (dd, *J*=18.02, 12.52 Hz, 1 H) 5.96 (dd, *J*=12.52, 6.52 Hz, 1 H) 7.16 - 7.28 (m, 5 H) 7.40 (d, *J*=8.64 Hz 1 H) 7.37 (d *J*= 34 | 479 |
| 184 | 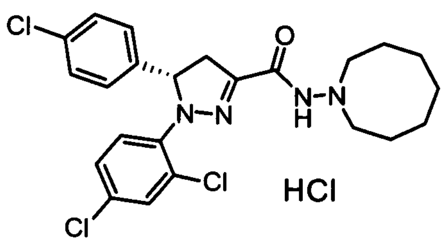 | (S)-5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide hydrochloride | | 477 |
| 188 | 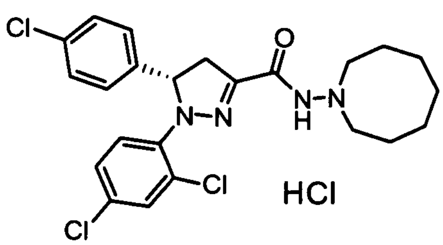 | (R)-5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide | | 477 |

(continued)

| N° | STRUCTURE | Name | $^1$H-NMR | MS (M+H)$^+$ |
|---|---|---|---|---|
| 193 | 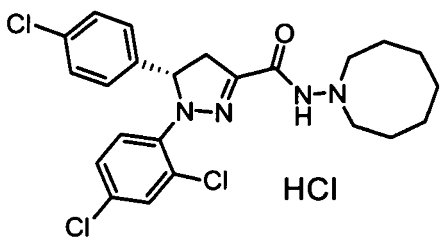 | 5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide hydrochloride | | 477 |
| 196 | 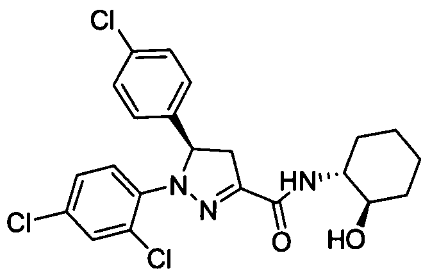 | (R)-5-(4-chlorophenyl)-1-(2,4-dichlorophenyl)-N-((1 R, 2R)-2-hydroxycyclohexyl)-4,5-dihydro-1H-pyrazole-3-carboxamide | | 466 |
| 197 | 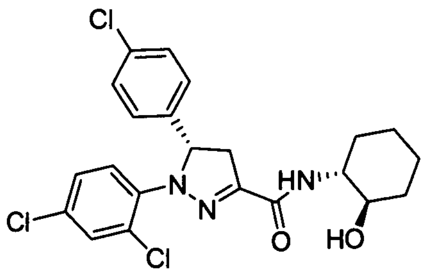 | (S)-5-(4-chlorophenyl)-1-(2,4-dichlorophenyl)-N-((1R, 2R)-2-hydroxycyclohexyl)-4,5-dihydro-1H-pyrazole-3-carboxamide | | 466 |
| 198 | 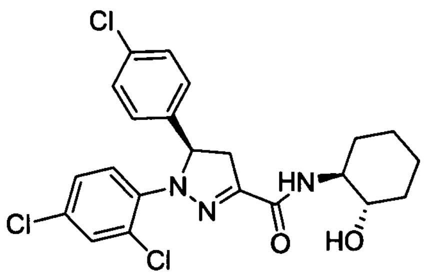 | (R)-5-(4-chlorophenyl)-1-(2,4-dichlorophenyl)-N-((1S, 2S)-2-hydroxycyclohexyl)-4,5-dihydro-1H-pyrazole-3-carboxamide | | 466 |
| 199 | 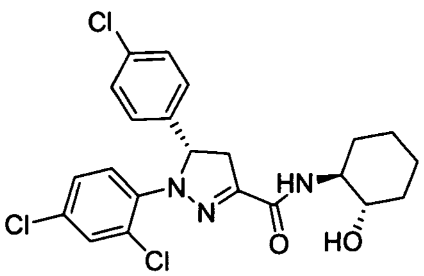 | (S)-5-(4-chlorophenyl)-1-(2,4-dichlorophenyl)-N-((1S, 2S)-2-hydroxycyclohexyl)-4,5-dihydro-1H-pyrazole-3-carboxamide | | 466 |

(continued)

| N° | STRUCTURE | Name | $^1$H-NMR | MS (M+H)$^+$ |
|---|---|---|---|---|
| 200 | 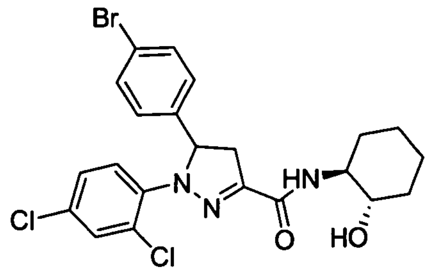 | 5-(4-bromophenyl)-1-(2,4-dichlorophenyl)-N-((1S,2S)-2-hydroxycyclohexyl)-4,5-dihydro-1H-pyrazole-3-carboxamide | | 510 |
| 201 | 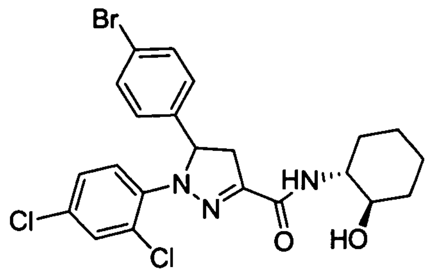 | 5-(4-bromophenyl)-1-(2,4-dichlorophenyl)-N-((1R,2R)-2-hydroxycyclohexyl)-4,5-dihydro-1H-pyrazole-3-carboxamide | | 510 |
| 202 | 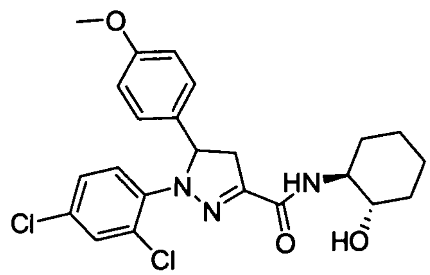 | 1-(2,4-dichlorophenyl)-N-((1S,2S)-2-hydroxycyclohexyl)-5-(4-methoxyphenyl)-4,5- dihydro-1H-pyrazole-3-carboxamide | | 462 |
| 203 | 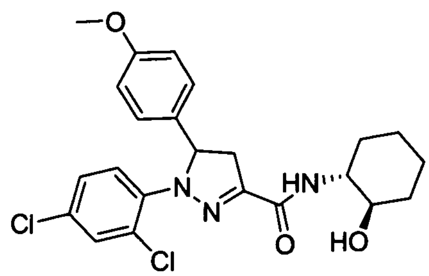 | 1-(2,4-dichlorophenyl)-N-((1 R,2R)-2-hydroxycyclohexyl)-5-(4-methoxyphenyl)-4,5- dihydro-1H-pyrazole-3-carboxamide | | 462 |

[178] N-((1R,2R)-2-(benzyloxy)cyclohexyl)-5-(4-chlorophenyl)-1-(2,4-dichlorophenyl)-4,5-dihydro-1 H-pyrazole-3-carboxamide $[M+H]^+$ = 556
[179] 1-(2,4-Dichloro-phenyl)-5-(4-fluoro-phenyl)-4,5-dihydro-1 H-pyrazole-3-carboxylic acid (2,3-dihydro-indol-1-yl)-amide hydrochloride $[M+H]^+$ = 469
[180] 1-(2,4-Dichloro-phenyl)-5-(4-hydroxy-phenyl)-4,5-dihydro-1H-pyrazole-3-carboxylic acid $[M+H]^+$ = 351
[181] (R)-5-(4-Chloro-phenyl)-1-(2,4-dichloro-phenyl)-4,5-dihydro-1H-pyrazole-3-carboxylic acid (2,3-dihydro-indol-1-yl)-amide hydrochloride $[M+H]^+$ = 485
[182] (S)-5-(4-Chloro-phenyl)-1-(2,4-dichloro-phenyl)-4,5-dihydro-1H-pyrazole-3-carboxylic acid (2,3-dihydro-indol-1-yl)-amide hydrochloride $[M+H]^+$ = 485
[183] (S)-5-(4-Chloro-phenyl)-1-(2,4-dichloro-phenyl)-4,5-dihydro-1H-pyrazole-3-carboxylic acid azepan-1-ylamide hydrochloride $[M+H]^+$ = 465
[184] (S)-5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide hydrochloride $[M+H]^+$ = 477

[185] (S)-5-(4-Chloro-phenyl)-1-(2,4-dichloro-phenyl)-4,5-dihydro-1H-pyrazole-3-carboxylic acid azepan-1-ylamide $[M+H]^+$ = 465

[186] (S)- 5-(4-Chloro-phenyl)-1-(2,4-dichloro-phenyl)-4,5-dihydro-1H-pyrazole-3-carboxylic acid cycloheptylamide $[M+H]^+$ = 464

[187] (R)-5-(4-Chloro-phenyl)-1-(2,4-dichloro-phenyl)-4,5-dihydro-1H-pyrazole-3-carboxylic acid azepan-1-ylamide hydrochloride $[M+H]^+$ = 465

[188] (R)-5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide $[M+H]^+$ = 477

[189] (R)- 5-(4-Chloro-phenyl)-1-(2,4-dichloro-phenyl)-4,5-dihydro-1H-pyrazole-3-carboxylic acid cycloheptylamide $[M+H]^+$ = 464

[190] (R)- 5-(4-Chloro-phenyl)-1-(2,4-dichloro-phenyl)-4,5-dihydro-1H-pyrazole-3-carboxylic acid (2,3-dihydro-indol-1-yl)-amide $[M+H]^+$ = 485

[191] 5-(4-Chloro-phenyl)-1-(2,4-dichloro-phenyl)-4,5-dihydro-1H-pyrazole-3-carboxylic acid cycloheptylmethyl-amide $[M+H]^+$ = 478

[192] 5-(4-Chloro-phenyl)-1-(2,4-dichloro-phenyl)-4,5-dihydro-1H-pyrazole-3-carboxylic acid (2,6-dimethyl-piperidin-1-yl)-amide hydrochloride $[M+H]^+$ = 479

[193] 5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide hydrochloride $[M+H]^+$ = 477

[194] 5-(4-Chloro-phenyl)-1-(2,4-dichloro-phenyl)-4,5-dihydro-1H-pyrazole-3-carboxylic acid pyrrolidin-1-ylamide hydrochloride $[M+H]^+$ = 437

[195] [5-(4-Chloro-phenyl)-1-(2,4-dichloro-phenyl)-4,5-dihydro-1H-pyrazole-3-yl]-(4-methyl-piperazin-1-yl)-methanone hydrochloride $[M+H]^+$ = 451

[60A] 5-(4-Bromo-phenyl)-1-(2,4-dichloro-phenyl)-4,5-dihydro-1H-pyrazole-3-carboxylic acid piperidin-1-ylamide

[66A] 5-(4-Bromo-phenyl)-1-(2,4-dichloro-phenyl)-4,5-dihydro-1H-pyrazole-3-carboxylic acid (2,3-dihydro-indol-1-yl)-amide

[82A] 5-(4-Chloro-phenyl)-1-(2,4-dichloro-phenyl)-4,5-dihydro-1H-pyrazole-3-carboxylic acid (2-(R)-hydroxy-cyclohexyl)-amide

[83A] 5-(4-Chloro-phenyl)-1-(2,4-dichloro-phenyl)-4,5-dihydro-1H-pyrazole-3-carboxylic acid (2-(S)-hydroxy-cyclohexyl)-amide

[91A] 1-(2,4-Dichloro-phenyl)-5-(4-fluoro-phenyl)-4,5-dihydro-1H-pyrazole-3-carboxylic acid (hexahydro-cyclopenta[c]pyrrol-2-yl)-amide

[106A]1-(2,4-Dichloro-phenyl)-5-(4-methoxy-phenyl)-4,5-dihydro-1H-pyrazole-3-carboxylic acid piperidin-1-ylamide

[109A]1-(2,4-Dichloro-phenyl)-5-(4-methoxy-phenyl)-4,5-dihydro-1H-pyrazole-3-carboxylic acid (hexahydro-cyclopenta[c]pyrrol-2-yl)-amide

[112A] 1-(2,4-Dichloro-phenyl)-5-(4-methoxy-phenyl)-4,5-dihydro-1H-pyrazole-3-carboxylic acid (2,3-dihydro-indol-1-yl)-amide

[114A] 1-(2,4-Dichloro-phenyl)-5-(4-methoxy-phenyl)-4,5-dihydro-1H-pyrazole-3-carboxylic acid azepan-1-ylamide

## SUBGROUP A6.1

### Subgroup A6.1: Chemical Example

### Preparation of 4-(4-Chlorophenyl)-3-methyl-2-oxo-but-3-enoic acid

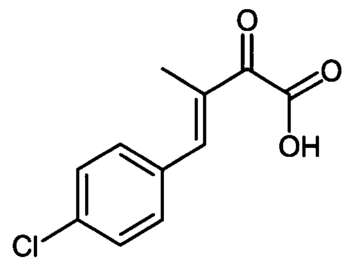

4-Chlorobenzaldehyde (1.4 g, 10 mmol) was dissolved in MeOH (1 mL) and 2-oxobutyric acid (1 g, 9.8 mmol) was subsequently added. A third of a 2 M KOH solution (0.84 g) in MeOH (2.5 mL) was added drop wise at a rate that the temperature of the reaction medium did not rise above 27 °C. The remaining alkaline solution was added quickly causing an increase of the temperature to 42 °C and the yellow colour of the solution turns into orange-red. After approximately five minutes a voluminous and yellow precipitate appeared. After 30 min, water (5 mL) was added and the organic layer was separated. After extraction with *tert*-butyl methyl ether (8 mL), the aqueous phase was acidified with conc. HCl to pH 1 and thoroughly extracted with *tert*-butyl methyl ether (3 x 8 mL). The combined organic layers were washed with aq. NaCl solution, dried with $Na_2SO_4$, and evaporated to dryness to afford a cream-white solid (0.88 g, 40% yield). recrystallization of 0.6 g of the crude solid in hexane (40 mL) and ethyl acetate (5 mL) afforded 0.44 g of pure product (80 % recovery).

$^1$H NMR (400 MHz, $CDCl_3$): δ 2.17 (3H, s, $CH_3$), 7.44 (4H, d, J 3.28 Hz, ArH), 8.41 (1 H, s, CH); $^{13}$C NMR (100 MHz, $CDCl_3$): δ 13.2 ($CH_3$), 129.2 (CH), 129.6 (C), 131.9 (CH), 133.4 (C), 136.4 (C), 147.6 (CH), 164.4 (CO), 187.1 (CO)

**Preparation of 5-(4-Chlorophenyl)-1-(2,4-dichlorophenyl)-4-methyl-4,5-dihydro-1H-pyrazole-3-carboxylic acid**

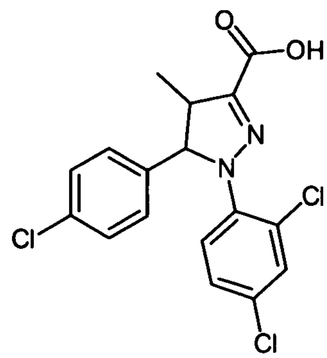

A mixture of 4-(4-chlorophenyl)-3-methyl-2-oxo-but-3-enoic acid (0.72 g, 3.2 mmol) and 2,4-dichlorophenyl hydrazine hydrochloride (0.68 g, 3.2 mmol) in glacial acetic acid (10.6 mL) was heated under nitrogen atmosphere to reflux for 4 h. The crude mixture was allowed to cool down to room temperature and poured into ice. The dark mixture was extracted with dichloromethane, washed with aq. NaCl, dried over $Na_2SO_4$, and evaporated to dryness to yield 1.18 g of crude product (100 % yield) as a mixture of two diastereomers. The ratio of the two diastereomers was determined by NMR spectroscopy to be 80:20.

**Preparation of 5-(4-Chlorophenyl)-1-(2,4-dichlorophenyl)-4-methyl-4,5-dihydro-1H-pyrazole-3-carboxylic acid methyl ester**

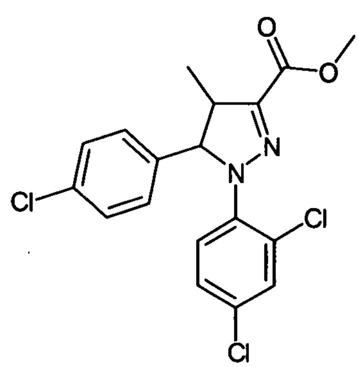

Methyl iodide (0.16 mL, 2.25 mmol) was added drop wise to a mixture of 5-(4-chlorophenyl)-1-(2,4-dichlorophenyl)-4-methyl-4,5-dihydro-1H-pyrazole-3-carboxylic acid (0.47 g, 1.27 mmol) and $KHCO_3$ (0.19 g, 1.9 mmol) in anhydrous dimethylformamide (10 mL) under nitrogen atmosphere. The mixture was stirred at room temperature under nitrogen overnight (ca 16 h). Water was added, the mixture was extracted with ethyl acetate and the combined organic layers were thoroughly washed with aq. NaCl solution. After drying over $Na_2SO_4$ and evaporation under reduced pressure, 0.5 g of the crude product were obtained. Purification by column chromatography ($SiO_2$, 40:1 $SiO_2$, packed with 100% hexane and eluted with a gradient of 1% to 5% ethyl acetate) affords 0.045 g (9% yield) of the minor isomer (RS and SR as a racemic mixture, trans isomer) and 0.23 g (48% yield) of the major isomer (RR and SS as a racemic mixture, cis isomer).

**Subgroup A6.1: Chemical Example 24: Minor isomer: *trans*-5-(4-Chlorophenyl)-1-(2,4-dichlorophenyl)-4-methyl-4,5-dihydro-1H-pyrazole-3-carboxylic acid methyl ester**

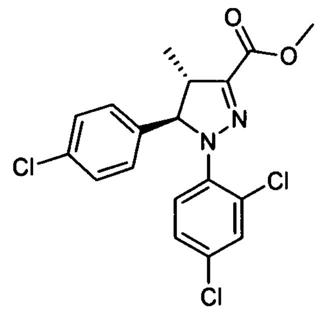

$^1$H NMR (400 MHz, $CDCl_3$): δ 1.49 (3H, d, J 7.6 Hz, $CH_3$), 3.51 (1H, m, CH), 3.90 (3H, s, $OCH_3$), 5.32 (1H, d, J 5.6 Hz, CH), 7.03-7.10 (3H, m, ArH), 7.20 (2H, ap d J 8.4 Hz, ArH), 7.24-7.28 (2H, m, ArH) $^{13}$C NMR (100 MHz, $CDCl_3$):δ 18.4 ($CH_3$), 49.6 (CH), 52.5 ($OCH_3$), 75.5 (CH), 126.2 (CH), 126.7 (C), 127.8 (CH), 128.1 (CH), 129.3 (CH), 130.4 (CH), 134.5 (C), 137.6 (C), 139.1 (C), 145.2 (C), 162.8 (CO)
MS $(M+H)^+$: 398

**Subgroup A6.1: Chemical Example 23: Major isomer: *cis*-5-(4-Chlorophenyl)-1-(2,4-dichlorophenyl)-4-methyl-4,5-dihydro-1H-pyrazole-3-carboxylic acid methyl ester**

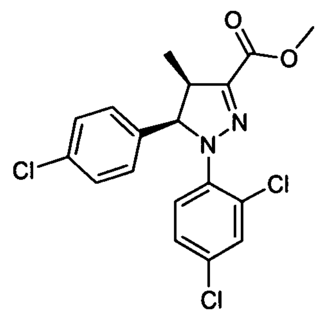

$^1$H NMR (400 MHz, $CDCl_3$): δ 0.94 (3H, d, *J* 7.2 Hz, $CH_3$), 3.80 (1H, m, CH), 3.88 (3H, s, $OCH_3$), 5.82 (1H, d, *J* 11.2 Hz, CH), 7.04-7.10 (3H, m, ArH), 7.22 (2H, m, ArH), 7.24-7.29 (2H, m, ArH) $^{13}$C NMR (100 MHz, $CDCl_3$): δ 17.8 ($CH_3$), 49.5 (CH), 52.7 ($OCH_3$), 75.7 (CH), 126.2 (CH), 127.7 (C), 127.9 (CH), 129.4 (CH), 130.4 (CH), 130.8 (C), 134.4 (C), 137.9 (C), 139.0 (C), 144.8 (C), 162.8 (CO)
MS $(M+H)^+$: 398

**Hydrolysis of the racemic mixture of (RR)- and (*SS*)-5-(4-Chlorophenyl)-1-(2,4-dichlorophenyl)-4-methyl-4,5-dihydro-1H-pyrazole-3-carboxylic acid methyl ester**

Aq. 2 M NaOH (6.4 mL) was added to a solution of the racemic mixture of (*RR*)- and (*SS*)-5-(4-chlorophenyl)-(2,4-dichlorophenyl)-methyl-4,5-dihydro-1H-pyrazole-3-carboxylic acid methyl ester (0.36 g, 0.9 mmol) in THF (6.4 mL). The mixture was left to stir at room temperature for 3 h (until TLC showed disappearance of the starting material). Water was added and the reaction mixture was concentrated under reduced pressure. The residue was washed once with $Et_2O$ and the aqueous phase acidified to pH 1 with 1 M HCl. Extraction with ethyl acetate, drying with $Na_2SO_4$ and concentration to dryness yielded pure acid (0.23 g, 68% yield).

**Subgroup A6.1: Chemical Example 19: Major isomer: *cis*-5-(4-Chloro-phenyl)-1-(2,4-dichloro-phenyl)-4-methyl-4,5-dihydro-1H-pyrazole-3-carboxylic acid**

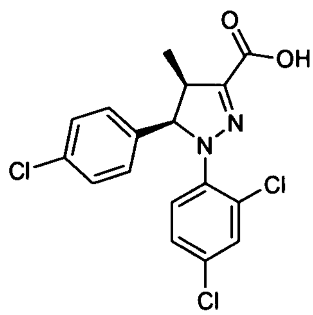

$^1$H NMR (400 MHz, CHLOROFORM-*d*) δ ppm 0.96 (d, *J*=7.42 Hz, 3 H) 3.82 (td, *J*=11.72, 7.42 Hz, 1 H) 5.91 (d, *J*=11.72 Hz, 1 H) 7.04 (d, *J*=8.60 Hz, 2 H) 7.11 (dd, *J*=8.60, 2.34 Hz, 1 H) 7.21 - 7.30 (m, 4 H)
$^{13}$C NMR (100 MHz, $CDCl_3$): δ 13.6 ($CH_3$), 43.5 (CH), 72.0 (CH), 124.9 (CH), 127.6 (CH), 129.1 (CH), 129.6 (CH), 130.9 (CH), 131.3 (C), 132.7 (C), 134.5 (C), 138.7 (C), 144.6 (C), 165.9 (CO).
MS $(M+H)^+$: 384

**Hydrolysis of the racemic mixture of (*RS*)- and (*SR*)-5-(4-Chlorophenyl)-1-(2,4-dichlorophenyl)-4-methyl-4,5-dihydro-1H-pyrazole-3-carboxylic acid methyl ester**

The hydrolysis was carried out as described above for the racemic mixture of (*RR*)- and (*SS*)-5-(4-Chlorophenyl)-1-(2,4-dichlorophenyl)-4-methyl-4,5-dihydro-1H-pyrazole-3-carboxylic acid methyl ester

**Subgroup A6.1: Chemical Example 20: Minor isomer: *trans*-5-(4-Chlorophenyl)-1-(2,4-dichlorophenyl)-4-methyl-4,5-dihydro-H-pyrazole-3-carboxylic acid**

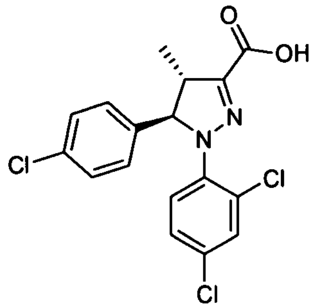

$^1H$ NMR (400 MHz, CHLOROFORM-*d*) δ ppm 1.52 (d, *J*=7.03 Hz, 3 H) 3.52 (m, 1 H) 5.39 (d, *J*=5.47 Hz, 1 H) 7.08 (ddd, *J*=13.92, 5.62, 5.28 Hz, 3 H) 7.14-7.34 (m, 4 H)
$^{13}C$ NMR (100 MHz, $CDCl_3$): δ 18.4 ($CH_3$), 48.9 (CH), 76.2 (CH), 126.4 (CH), 126.7 (C), 127.8 (CH), 128.0 (CH), 129.5 (CH), 130.5 (CH), 131.5 (C), 134.7 (C), 137.6 (C), 138.6 (C), 144.2 (C), 166.5 (CO).
MS $(M+H)^+$: 384

**Preparation of 4-(4-Chlorophenyl)-3-methyl-2-oxo-but-3-enoic acid methyl ester**

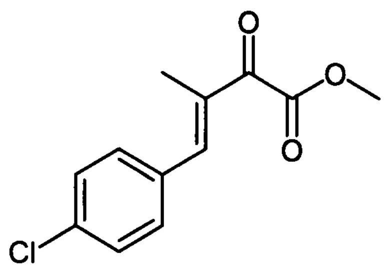

4-(4-Chlorophenyl)-3-methyl-2-oxo-but-3-enoic acid (0.13 g, 0.58 mmol) was dissolved in $Et_2O$ (0.8 mL) and EtOH (1.2 mL) and the solution was cooled down to 0 ˚C. $TMSCH_2N_2$ (0.43 mL of a 2 M solution in $Et_2O$, 0.87 mmol) was added drop wise and the mixture was stirred at 0 ˚C for 1 h. A second portion of $TMSCH_2N_2$ (0.2 mL of a 2 M solution in $Et_2O$, 0.40 mmol) was added and the reaction was allowed to reach room temperature. After 30 min, the solvents were evaporated to dryness and the residue was taken up in a mixture of $Et_2O$/hexane 1:1 (10 mL). The organic layer was washed with 2% aq. HCl (10 mL), aq. $NaHCO_3$ (10 mL) and aq. NaCl (10 mL), dried over $Na_2SO_4$ and concentrated to dryness to give pure product (68 mg, 49% yield).

**Preparation of 5-(4-Chlorophenyl)-1-(2,4-dichlorophenyl)-4-methyl-4,5-dihydro-1H-pyrazole-3-carboxylic acid methyl ester**

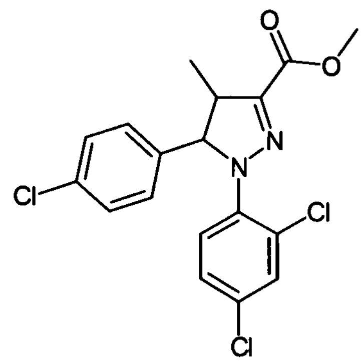

A mixture of 4-(4-chlorophenyl)-3-methyl-2-oxo-but-3-enoic acid methyl ester (0.4 g, 1.68 mmol) and 2,4-dichlorohydrazine hydrochloride (0.356 g, 1.68 mmol) in glacial acetic acid (4 mL) was heated at reflux under nitrogen atmosphere for 4 h. The reaction mixture was then allowed to cool down to room temperature and poured into ice. The dark mixture was extracted with dichloromethane, washed with aq. NaCl, dried over $Na_2SO_4$ and evaporated to dryness to yield the crude product (0.64 g, 95 % yield, as a mixture of two diastereomers). Purification by column chromatography ($SiO_2$, 40:1 $SiO_2$, packed with 100% hexane and eluted with a gradient of 1% to 5% ethyl acetate) of 0.5 g of crude product afforded 0.051 g of the minor isomer (RS and SR as a racemic mixture) and 0.20 g of the major isomer (RR and SS as racemic mixture).

**Preparation of Example 1: cis-S-(4-Chloro-phenyl)-1-(2,4-dichlorophenyl)-4-methyl-4,5-dihydro-1H-pyrazole-3-carboxylic acid piperidin-1-ylamide Hydrochloride**

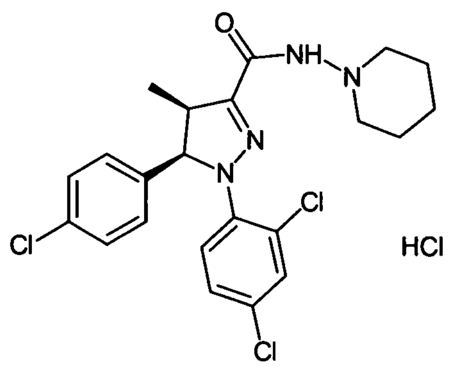

5-(4-Chlorophenyl)-1-(2,4-dichlorophenyl)-4-methyl-4,5-dihydro-1H-pyrazole-3-carboxylic acid (0.160 g, 0.417 mmol) was dissolved in toluene (4 mL) and was partially distilled at atmospheric pressure to eliminate water traces. The resulting solution was cooled to 75-85 ˚C. DMF was added (2 drops) as a catalyst and thionyl chloride (0.060 mL, 0.5 mmol) was added drop by drop. The solution was stirred until formation of the acid chloride was complete (monitoring by IR). The mixture was cooled to 20 - 25 ˚C and was added drop wise to a solution of 1-aminopiperidine (0.053 mL, 0.500 mmol), dichloromethane (5 mL) and N,N-diisopropylethylamine (0.170 mL, 1 mmol) under nitrogen at a temperature between 5-10˚C. After complete addition the mixture was warmed to 20 - 25 ˚C and stirred overnight. The organic layer was washed with water (3 x 5 mL), a sat. $NaHCO_3$ solution (3 x 25 mL) and again with water (3 x 5 mL). The combined organic layers were dried with $Na_2SO_4$, filtered and concentrated under vacuum to give a solid (130 mg, 68 % yield).

The crude solid was dissolved in ethyl acetate and a 2.8 M HCl solution in ethanol was added drop wise to form the hydrochloride which was obtained as a beige solid (82 mg, 49 % yield).

$^1$H NMR (400 MHz, METHANOL-$d_4$) $\delta$ ppm 0.94 (d, *J*=7.42 Hz, 3 H) 1.68 (br, 2 H) 1.98 (dt, *J*=11.28, 5.59 Hz, 4 H) 3.53 (br, 4 H) 3.90 (td, *J*=11.53, 7.42 Hz, 1 H) 5.99 (d, *J*=11.53 Hz, 1 H) 7.16 (d, *J*=8.40 Hz, 2 H) 7.20 - 7.29 (m, 3 H) 7.36 - 7.48 (m, 2 H)
MS $(M+H)^+$: 466

**Subgroup A6.1: Chemical Example 2: trans-5-(4-Chloro-phenyl)-1-(2,4-dichlorophenyl)-4-methyl-4,5-dihydro-1H-pyrazole-3-carboxylic acid piperidin-1-ylamide Hydrochloride**

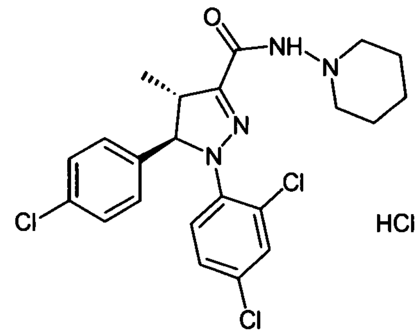

5-(4-Chlorophenyl)-1-(2,4-dichlorophenyl)-4-methyl-4,5-dihydro-1H-pyrazole-3-carboxylic acid (0.190 g, 0.50 mmol) was dissolved in toluene (4 mL) and was partially distilled at atmospheric pressure to eliminate water traces. The resulting solution was cooled to 75-85 ˚C. DMF was added (2 drops) as a catalyst and thionyl chloride (0.060 mL, 0.5 mmol) was added drop by drop. The solution was stirred until formation of the acid chloride was complete (monitoring by IR). The mixture was cooled to 20 - 25 ˚C and was added drop wise to a solution of 1-aminopiperidine (0.0623 mL, 0.57 mmol), dichloromethane (5 mL) and N,N-diisopropylethylamine (0.197 mL, 1.15 mmol) under nitrogen at a temperature between 5-10 ˚C. After complete addition the mixture was warmed to 20 - 25 ˚C and stirred overnight. The organic layer was washed with water (3 x 5 mL), a sat. $NaHCO_3$ solution (3 x 25 mL) and again with water (3 x 5 mL). The combined organic layers were dried with $Na_2SO_4$, filtered and concentrated under vacuum to give a solid.
The crude solid was dissolved in ethyl acetate and a 2.8 M HCl solution in ethanol was added drop wise to form the hydrochloride which was obtained as a beige solid (120 mg, 50 % yield).

$^1$H NMR (400 MHz, DMSO-$d_6$) δ ppm 1.38 (d, *J*=7.03 Hz, 3 H) 1.42 (m, 2 H) 1.71 (m, 4 H) 3.12 (m, 4 H) 3.42 (dt, *J=13.38,* 5.86 Hz, 1 H) 5.36 (d, *J*=5.86 Hz, 1 H) 7.19 (d, *J*=8.21 Hz, 2 H) 7.31 (d, *J*=8.60 Hz, 3 H) 7.49 (d, *J*=2.34 Hz, 1 H) 7.53 (d, *J*=8.60 Hz, 1 H) 10.49 (s, 1 H)
MS $(M+H)^+$: 466

The following compounds were prepared according to the processes described above. Those skilled in the art are familiar with the starting materials that are needed to obtain said compounds.

**Subgroup A6.1: Chemical Example: Preparation of compounds of general formula II**

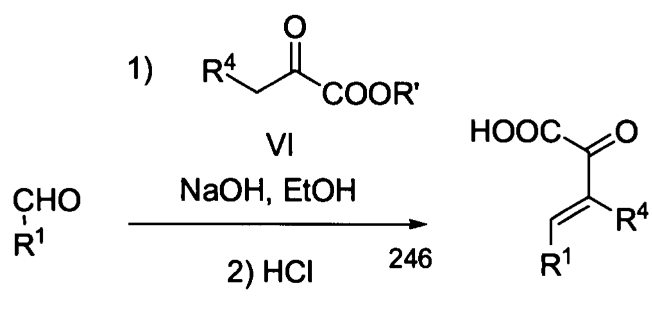


Acid of general formula VI (1.1 equivalents) was added in portions to a solution of 0.5 M aqueous NaOH (1.5 equivalents) under $N_2$ at room temperature. The reaction was then left stirring for 5 min and a solution of a compound of general formula VII (1 equivalent) in abs. EtOH (0.9 M) was then slowly added (10 mL/h). The reaction was left stirring at 25 ˚C overnight. Water was added and the solvent was evaporated under reduced pressure to eliminate the excess of EtOH. The solution was then washed with toluene and the solvent was again evaporated. The aqueous solution was then cooled in an ice bath and conc. aqueous HCl (0.2 mL of conc. HCl per mL of base) were slowly added while stirring.

A white solid precipitated from the solution which was kept at 0 ˚C for another hour. The solid was filtered under vacuum through a sintered funnel (porosity 3) and dried at 40 ˚C under vacuum.
As a example, if the starting material was 2-oxobutyric acid, the yield range for this reaction condensation was around 60-86%. However, if the starting material was 2-oxopentanoic acid (oxovaleric acid) instead of 2-oxobutyric acid, the yield of the reaction was slightly lower (around 40-60%)

**Compound A1. (*E*)-4-(4-chlorophenyl)-3-methyl-2-oxobut-3-enoic acid**

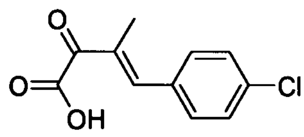

$^1$H NMR (400 MHz, $CDCl_3$): δ 2.17 (3H, s, $CH_3$), 7.44 (4H, ap d, *J*= 3.28 Hz, ArH), 8.41 (1H, s, CH).

**Compound A2: (*E*)-4-(4-bromophenyl)-3-methyl-2-oxobut-3-enoic acid**

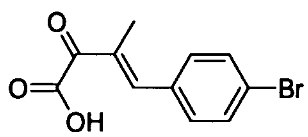

$^1$H NMR (400 MHz, $CD_3OD$): δ 1.99 (3H, s, $CH_3$), 7.36 (2H, d, *J*= 8.5 Hz, ArH), 7.40 (1 H, s, CH), 7.53 (2H, d, *J*= 8.5 Hz, ArH).

**Compound A3: (*E*)-4-(4-fluorophenyl)-3-methyl-2-oxobut-3-enoic acid**

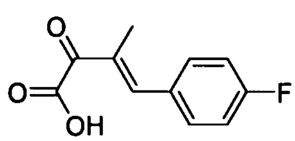

$^1$H NMR (300 MHz, DMSO-d6): δ 2.18 (3H, s, $CH_3$), 7.20 (2H, m), 7.53 (2H, m), 8.26 (1 H, s, CH).

**Compound A4: (*E*)-4-(4-methoxyphenyl)-3-methyl-2-oxobut-3-enoic acid.** This compound was prepared following the method described above but using instead 3 eq. of NaOH.

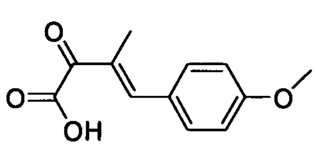

$^1$H NMR (200 MHz, $CDCl_3$): δ 2.09 (3H, s, $CH_3$), 3.84 (3H, s, $OCH_3$), 7.01 (2H, d, J= 8.8 Hz, ArH), 7.50 (1 H, s, CH), 7.52 (2H, d, J= 8.8 Hz, ArH).

**Compound A5: (E)-3-methyl-2-oxo-4-phenylbut-3-enoic acid.**

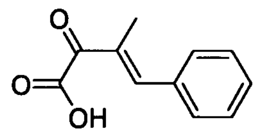

$^1$H NMR (200 MHz, $CDCl_3$): δ 2.19 (3H, s, $CH_3$), 7.33-7.51 (5H, m, ArH), 8.36 (1 H, s, CH)

**Compound A6: (E)-4-(4-bromophenyl)-3-ethyl-2-oxobut-3-enoic acid**

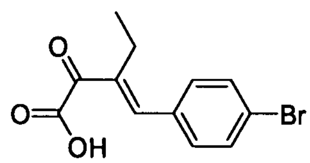

$^1$H NMR (400 MHz, $CDCl_3$): δ 1.16 (3H, t, J 7.5 Hz, $CH_3$), 2.62 (2H, q, J 7.5 Hz, $CH_2$), 7.35 (2H, d, J 8.4 Hz, ArH), 7.59 (2H, d, J 8.4 Hz, ArH), 8.23 (1 H, s, CH).

**Compound A7: (*E*)-4-(4-chlorophenyl)-3-ethyl-2-oxobut-3-enoic acid**

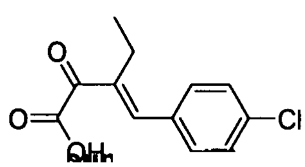

$^1$H NMR (400 MHz, $CDCl_3$): δ 1.16 (3H, t, *J* 7.2 Hz, $CH_3$), 2.61 (2H, q, *J* 15.6 Hz, *J* 7.2 Hz ArH), 7.44 (4H, m, ArH), 7.85 (1 H, bs, CH).

**Compound A8:(E)-4-(5-chlorothiophen-2-yl)-3-methyl-2-oxobut-3-enoic acid**

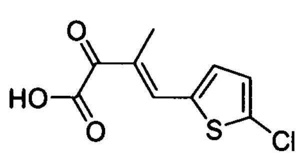

$^1H$ NMR (400 MHz, $CDCl_3$): δ 2.09 (3H, s, $CH_3$), 7.10 (1H, d, J 4.8, ArH), 7.32 (1 H, d, J 4.8 Hz, ArH), 7.64 (1H, s, ArH).

**Compound A9: (E)-4-(5-bromothiophen-2-yl)-3-methyl-2-oxobut-3-enoic acid**

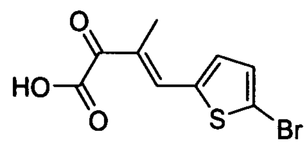

$^1H$ NMR (400 MHz, $CDCl_3$): δ 2.19 (3H, d, *J* 0.9 Hz, $CH_3$), 7.19 (1 H, d, J 3.7Hz thiophene H), 7.27 (1 H, d, J 3.7 Hz, thiophene H), 8.74 (1 H, bs, CH).

**Compound A10: (E)-3-methyl-2-oxo-4-(thiophen-2-yl)but-3-enoic acid**

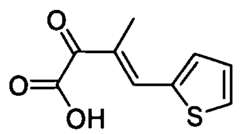

$^1H$ NMR (400 MHz, $CDCl_3$): δ 2.25 (3H, s, $CH_3$), 7.22 (1H, dd, J 5.1, 3.6 Hz, ArH), 7.52 (1H, d, J 3.6 Hz, ArH), 7.72 (1H, d, J 5.1, Hz, ArH), 8.68 (1 H, s, CH).

**Subgroup A6.1: Chemical Example: Preparation of compounds of general formula V**

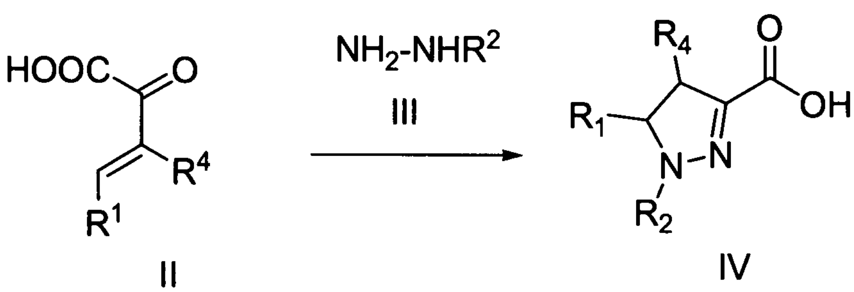


A suspension of a compound of general formula III (1 equivalent) in glacial acetic acid (40 equivalents) was heated at 80 ˚C under nitrogen atmosphere. When the suspension became a solution, a solution of acid of general formula II (1 equivalent) in acetic acid (20 equivalents) was added and the solution was left stirring at 80 ˚C for 2 h. The reaction mixture was allowed to crystallise at 0 - 4 ˚C overnight after partial evaporation of acetic acid. The beige solid formed was filtered off under vacuum through a sintered funnel (porosity 4) and washed several times with water. This crystallization process led to the major diastereomeric acid with *cis* configuration. The yield range for the cyclization with hydrazine to obtain the main diastereomer was around 46 - 80%.

The reaction mixture can also be cooled down to room temperature and poured through an addition funnel into water which is cooled in an ice bath with magnetically stirring. The addition must be slow and at least double volume of water per volume of acetic acid is required. A precipitate should form, but in the case a gum starts to form, it should be filtered and the rest of the material should be poured into another large volume of water. The solid obtained is suspended in water several times and filtered off until the pH of the water was above 3. This solid also corresponds to the *cis* form.

Alternatively, the dark mixture can be extracted with dichloromethane washed thoroughly with $H_2O$, dried over $Na_2SO_4$ and evaporated to dryness. Recrystallisation of the crude material from toluene (3 to 4 mL of toluene per gram of material) allows the recovery of the major diastereomer with cis configuration.

Formation of the methyl esters as described in the method above, followed by purification by column chromatography, allows for the separation of the two diastereoisomers.

**Subgroup A6.1: Chemical Example**

**Preparation of compounds of general formula IV with trans-configuration**

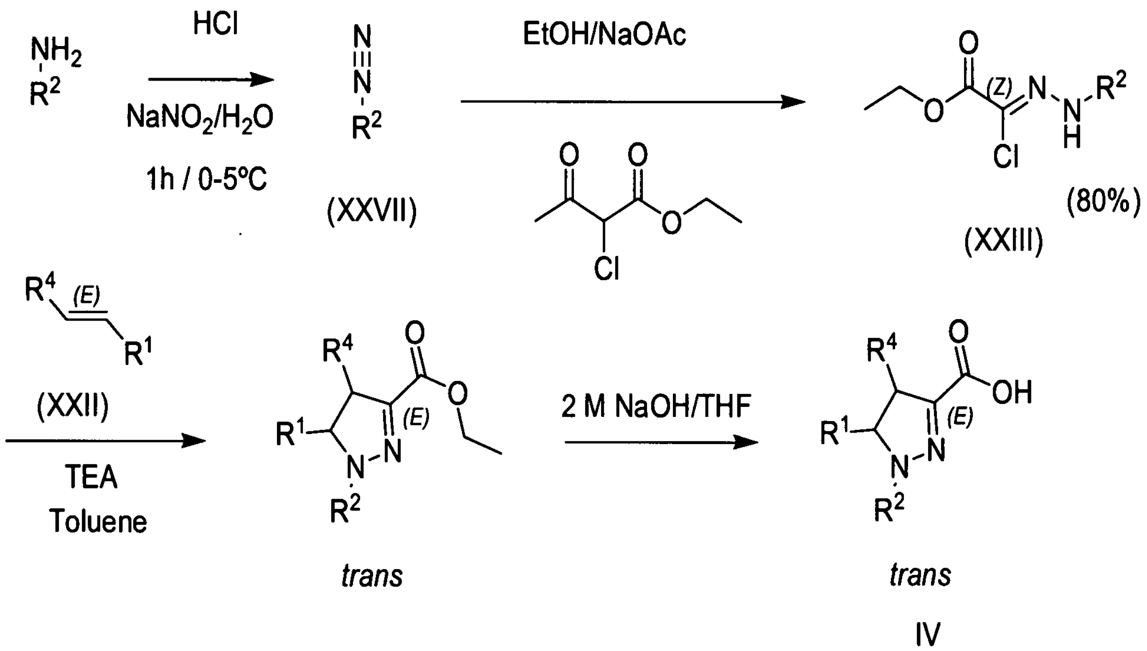


A solution of $NaNO_2$ (0.460 mg, 6.68 mmol) in water (0.8 ml) was added to a stirred solution of a an amine, wherein $R^2$ denotes an unsubstituted or at least mono-substituted phenyl radical, (6.17 mmol) and concentrated hydrochloric acid (1.5 ml) in ice (1.5 ml). The reaction mixture was stirred for 1 h at 0 - 5 ˚C and added to a cold mixture of NaOAc (1.64 g, 19.6 mmol), ethanol (26 ml) and ethyl-2-chloro-3-oxobutanoate (1.0 g, 6.06 mmol). The reaction mixture was left stirring for 1 hour until a precipitate formed which was collected by filtration, washed with ethanol and dichloromethane and dried in vacuo to give the yellow solid ethyl of general formula XXIII (70-80% yield), which was used in the next step without any further purification.

Triethylamine (2.8 eq) was added to a solution of a compound of general formula XXIII (1 eq) and a disubstituted (E)-alkene of general formula XXII (3 eq) in toluene, and the reaction mixture was stirred at reflux temperature for 1 hour. A precipitate formed which was removed by filtration after cooling to room temperature. The filtrate was concentrated and purified using a Combiflash system from Isco, eluted with cyclohexane and ethyl acetate (in a gradient program until 30% AcOEt), to obtain ethyl ester derivatives of compounds of general formula IV, (- 40 % yield).

Ethyl esters derivatives of compounds of general formula IV were hydrolysed in the presence of aqueous 2 M NaOH (2 eq) and tetrahydrofuran for 4 hours. Tetrahydofuran was partially removed by evaporation, 1 M aqueous HCl was added until pH was below 3 and the aqueous mixture was extracted with ethyl acetate, dried over $Na_2SO_4$, filtered and concentrated in vacuo to yield a white solid identified as carboxylic acid of general formula IV (85 % yield).

**The enantiomers of each acid (*cis* or *trans)* can be separated by chiral HPLC or by crystallization of the diastereomeric salts formed with chiral amines.**

**Subgroup A6.1: Chemical Example 199: *cis*-5-(4-bromophenyl)-1-(2,4-dichlorophenyl)-4-methyl-4,5-dihydro-1H-pyrazole-3-carboxylic acid**

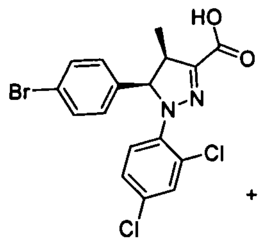

+ enantiomer

IR (NaCl film, $cm^{-1}$): 2976, 1683, 1541, 1486, 1385, 1270, 1242,1116.
$^1$H NMR (400 MHz, $CDCl_3$): δ 0.96 (3H, d, *J*= 7.3 Hz, $CH_3$), 3.82 (1H, qd, *J*= 11.9, 7.3, 7.3, 7.3 Hz, 1H), 5.88 (1H, d, *J*= 11.9 Hz, CH), 6.99 (2H, ap d, *J*= 8.4 Hz, ArH), 7.12 (1H, dd, *J*= 8.7, 2.3 Hz, 1H), 7.17 (1H, m, ArH), 7.27 (1H, m, ArH), 7.39 (2H, d, *J*= 8.4 Hz, ArH).

**Subgroup A6.1: Chemical Example 221: *cis*-5-(4-fluorophenyl)-1-(2,4-dichlorophenyl)- 4-methyl-4,5-dihydro-1H-pyrazole-3-carboxylic acid**

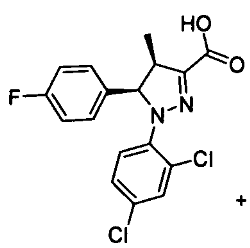

+ enantiomer

IR (NaCl film, $cm^{-1}$) : 2978, 1682, 1486, 1471, 1264, 1117.
$^1$H NMR (400 MHz, CHLOROFORM-*d*) δ ppm 0.97 (d, *J*= 7.42 Hz, 3 H) 3.82 (td, J= 11.72, 7.42 Hz, 1 H) 5.91 (d, J=12.0 Hz, 1 H), 6.95 (m, 2 H), 7.10 (m, 3 H), 7.20 (d, *J*=9.0 Hz, 1 H), 7.30 (d, J= 3.0 Hz, 1 H).

**Subgroup A6.1: Chemical Example 261: *cis*-5-(4-methoxyphenyl)-1-(2,4-dichlorophenyl)-4-methyl-4,5-dihydro-1 H-pyrazole-3-carboxylic acid**

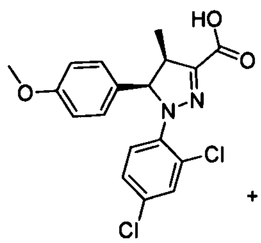

+ enantiomer

IR (NaCl film, $cm^{-1}$): 2936, 2836, 1681, 1612, 1512, 1480, 1460, 1248, 1113.
$^1H$ NMR (400 MHz, $CDCl_3$): δ 0.97 (3H, d, *J*= 7.3 Hz, $CH_3$), 3.74 (3H, s, $OCH_3$), 3.80 (1 H, m, 1H), 5.88 (1 H, d, *J*= 11.8 Hz, CH), 6.76 (2H, ap d, J= 8.6 Hz, ArH), 7.00 (2H, d, J= 8.6, ArH), 7. 09 (1 H, dd, J= 8.6, 2.3 Hz, ArH), 7.16-7.28 (2H, m, ArH).

**Subgroup A6.1: Chemical Example 185: *cis*-5-(4-bromophenyl)-1-(2,4-dichlorophenyl)-4-ethyl-4,5-dihydro-1H-pyrazole-3-carboxylic acid**

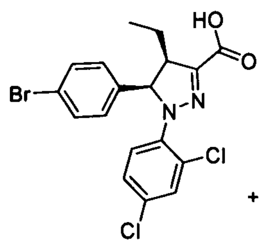

+ enantiomer

IR (NaCl film, $cm^{-1}$) : 2969, 1682, 1480, 1452, 1270, 1236, 1151, 1106.
$^1H$ NMR (400 MHz, $CDCl_3$): δ 0.69 (3H, t, J 7.4 Hz, $CH_3$), 1.28-1.34 (1 H, m, C*H*H), 1.95-2.00 (1 H, m, CH*H*), 3.68 (1 H, ddd, *J* = 11.5, 9.7, 4.0 Hz, CH), 5.92 (1 H, d, J 11.5 Hz, CH), 7.02 (2H, ap d, J 8.4 Hz, ArH), 7.09 (1H, dd, *J* = 8.7, 2.4 Hz, ArH), 7.20 (1H, d, J 8.7 Hz, ArH), 7.28 (1H, d, J 2.4 Hz, ArH), 7.36 (2H, ap d, J 8.4 Hz, ArH);
$^{13}C$ NMR (100 MHz, $CDCl_3$): δ 12.4 ($CH_3$), 20.0 ($CH_2$), 50.5 ($CH_3$), 52.3 ($OCH_3$), 72.0 (CH), 122.5 ©, 124.8 (CH), 127.4 ©, 127.5 (CH), 129.9 (CH), 130.4 (CH), 131.1 ©, 131.7 (CH), 132.7 ©, 138.5 ©, 143.8 ©, 165.4 (CO).

**Subgroup A6.1: Chemical Example 21: *cis*-5-(4-chlorophenyl)-1-(2,4-dichlorophenyl)-4-ethyl-4,5-dihydro-1H-pyrazole-3-carboxylic acid**

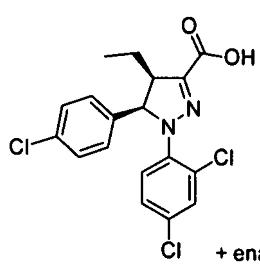

+ enantiomer

$^1H$ NMR (400 MHz, $CDCl_3$): δ 0.70 (3H, t, *J* 7.2 Hz, $CH_3$), 1.31 (1 H, m, CHH), 2.01 (1 H, m, CHH), 3.68 (1 H, m, CH), 5.92 (1 H, d, J 11.6 Hz, CH), 7.07-7.31 (7H, m, ArH).

**Subgroup A6.1: Chemical Example 22: *trans*-5-(4-chlorophenyl)-1-(2,4-dichlorophenyl)-4-ethyl-4,5-dihydro-1H-pyrazole-3-carboxylic acid**

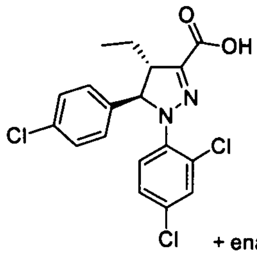

+ enantiomer

$^1H$ NMR (400 MHz, $CDCl_3$): δ 1.08 (3H, t, J 7.2 Hz, $CH_3$), 1.90 (1H, m, CHH), 1.96 (1 H, m, CHH), 3.50 (1 H, m, CH), 5.59 (1 H, d, J 4.80 Hz, CH), 7.01-7.31 (7H, m, ArH).

**Subgroup A6.1: Chemical Example 200: *trans*-5-(4-bromophenyl)-1-(2,4-dichlorophenyl)-4-methyl-4,5-dihydro-1H-pyrazole-3-carboxylic acid**

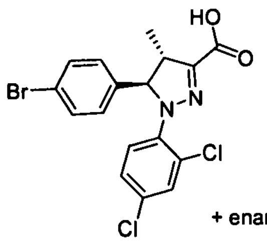

+ enantiomer

$^1H$ NMR (400 MHz, $CDCl_3$): δ 1.52 (3H, d, *J*= 7.1 Hz, $CH_3$), 3.52 (1 H, m, 1 H), 5.35(1H, d, *J*= 5.8 Hz, CH), 7.00 (2H, ap d, *J*= 8.4 Hz, ArH), 7.10 (1 H, dd, *J*= 8.7, 2.3 Hz, 1H), 7.22 (1H, d, *J*= 8.7, ArH), 7.27 (1 H, d, J= 2.3 Hz, ArH), 7.37 (2H, d, J= 8.4 Hz, ArH).

**Subgroup A6.1: Chemical Example 373: cis-1-(2,4-dichlorophenyl)-4-methyl-5-phenyl-4,5-dihydro-1H-pyrazole-3-carboxylic acid**

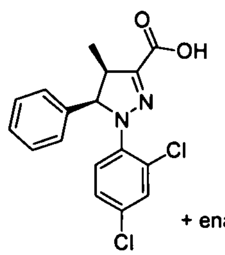

+ enantiomer

$^1H$ NMR (400 MHz, $CDCl_3$): δ 0.96 (3 H, d, 7.6 Hz, $CH_3$), 3.82 (1 H, m, CH), 3.88 (3 H, s, $OCH_3$), 5.91 (1 H, d, J 11.6 Hz, CH), 7.09-7.31 (8 H, m , ArH)

**Subgroup A6.1: Chemical Example 374: *trans*-1-(2,4-dichlorophenyl)-4-methyl-5-phenyl-4,5-dihydro-1H-pyrazole-3-carboxylic acid**

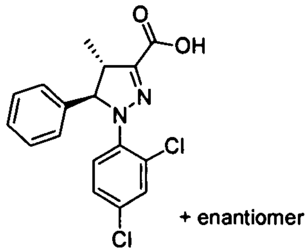


IR (NaCl film, $cm^{-1}$ ) : 3400-2800, 1680, 1479, 1456, 1108
$^1H$ NMR (400 MHz, $CDCl_3$): δ 1.53 (3 H, d, 7.2 Hz, $CH_3$), 3.57 (1 H, m, CH), 3.90 (3 H, s, $OCH_3$), 5.37 (1 H, d, J 5.6 Hz, CH), 7.06-7.28 (8 H, m , ArH)

**Subgroup A6.1: Chemical Example 361: *cis*-5-(5-chlorothiophen-2-yl)-1-(2,4-dichlorophenyl)-4-methyl-4,5-dihydro-1H-pyrazole-3-carboxylic acid**

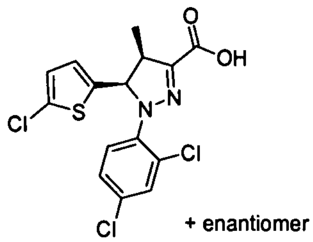


IR (NaCl film, $cm^{-1}$) : 3400-2800, 1684, 1479, 1447, 1107.
$^1H$ NMR (400 MHz, $CDCl_3$): δ 1.24 (3H, d, J 7.2, $CH_3$), 3.81 (1 H, m, CH), 6.09 (1H, d, J 12.0, CH), 6.64 (2H, ap dd, $J_1$ 12.0, $J_2$ 3.8 Hz, ArH), 7.16 (1 H, dd,, $J_1$ 8.8, $J_2$ 2.2 Hz, ArH), 7.32 (1 H, d J 2.2 Hz, ArH), 7.34 (1 H, aps, ArH), 7.36 (1 H, aps, ArH).

**Subgroup A6.1: Chemical Example 362: trans-5-(5-chlorothiophen-2-yl)-1-(2,4-dichlorophenyl)-4-methyl-4,5-dihydro-1 H-pyrazole-3-carboxylic acid**

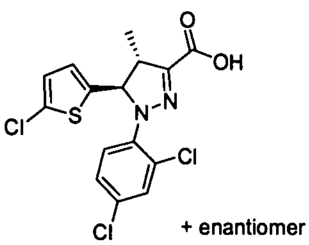


IR (NaCl film, $cm^{-1}$) : 3400-2800, 1682, 1569, 1544, 1479, 1448, 1246, 1105.

$^1H$ NMR (400 MHz, $CDCl_3$): δ 1.50 (3H, d, J 7.1 Hz, $CH_3$), 3.64 (1H, m, CH), 5.6 (1H, d, J 3.9 Hz, CH), 6.60 (2H, aps, ArH), 7.12 (1H, dd, $J_1$ 8.8, $J_2$ 2.3 Hz, ArH), 7.27 (1 H, m, ArH), 7.33 (1 H, d, J 2.3 Hz, ArH).

**Subgroup A6.1: Chemical Example 88: *cis*-5-(5-bromothiophen-2-yl)-1-(2,4-dichlorophenyl)-4-methyl-4,5-dihydro-1H-pyrazole-3-carboxylic acid**

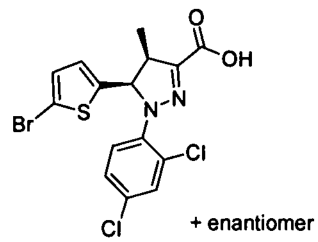

IR (NaCl film, $cm^{-1}$) : 3400-2800, 1682, 1569, 1545, 1479, 1459, 1245,1105.
$^1H$ NMR (400 MHz, $CDCl_3$): δ 1.23 (3H, d, J 7.2 Hz, $CH_3$), 3.81 (1 H, m, CH), 6.11 (1H, d, J 11.6 Hz, CH), 6.61 (1H, d, J 3.9 Hz, ArH), 6.80 (1 H, d, *J* 4.4 Hz, ArH), 7.16 (1 H, dd, $J_1$ 8.4 Hz, $J_2$ 3.0 Hz, ArH), 7.30-7.34 (2H, m, ArH).
$^{13}C$ NMR (100 MHz, $CDCl_3$): δ 12.3 ($CH_3$), 43.7 (CH), 69.5 (CH), 112.9 ©, 125.9 (CH), 126.8 ©, 127.8 (CH), 128.7 (CH), 129.7 (CH), 130.5 (CH), 131.8 ©, 137.8 ©, 138.6 ©, 144.9 ©, 164.5 (CO).

**Subgroup A6.1: Chemical Example 367: cis-1-(2,4-dichlorophenyl)-4-methyl-5-(thiophen-2-yl)-4,5-dihydro-1H-pyrazole-3-carboxylic** acid

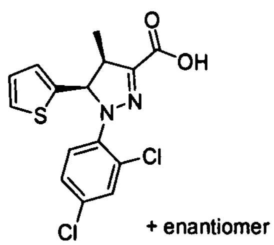

IR (NaCl film, $cm^{-1}$): 3400-2800, 1682, 1567, 1543, 1479, 1449, 1245, 1108.
$^1H$ NMR (400 MHz, $CDCl_3$): δ 1.19 (3H, d, J 7.2 Hz, $CH_3$), 3.83 (1H, m, CH), 6.22 (1H, d, J 11.2 Hz, CH), 6.84 (2H, m, ArH), 7.12 (2H, m, ArH), 7.29 (1 H, d, J 2.3, ArH), 7.34 (1 H, d, J 8.8 Hz, ArH).

**Subgroup A6.1: Chemical Example: Preparation of compounds of general formula V**

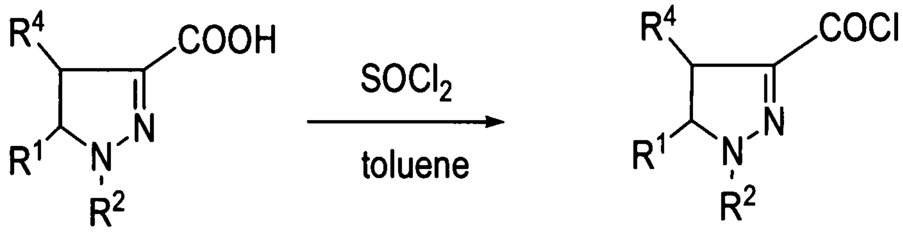


IV V

A compound of general formula IV (15 mmol) was dissolved in 120 mL of dry toluene and thionyl chloride (18 mmol) was added. The mixture was heated to 80 °C for 2.5 hours. The solvent was removed under reduced pressure and the resulting crude residue was used without any further purification.

***Cis*-5-(4-chlorophenyl)-1-(2,4-dichlorophenyl)-4-methyl-4,5-dihydro-1H-pyrazole-3-carbonyl chloride**

IR (NaCl film, $cm^{-1}$): 1732, 1700, 1533, 1478, 1212, 826.

***Cis*-5-(4-bromophenyl)-1-(2,4-dichlorophenyl)-4-methyl-4,5-dihydro-1H-pyrazole-3-carbonyl chloride**

IR (NaCl film, $cm^{-1}$): 1731, 1527, 1477, 1204, 1153, 1132, 825, 802.

***Cis*-5-(4-fluorophenyl)-1-(2,4-dichlorophenyl)-4-methyl-4,5-dihydro-1H-pyrazole-3-carbonyl chloride**

IR (NaCl film, $cm^{-1}$) : 1731, 1509, 1478, 1227, 1153, 1.132, 853, 803.

***Cis*-5-(4-methoxyphenyl)-1-(2,4-dichlorophenyl)-4-methyl-4,5-dihydro-1H-pyrazole-3-carbonyl chloride**

IR (NaCl film, $cm^{-1}$) : 1730, 1611, 1512, 1477, 1271, 1250, 1034, 831, 800.

***Cis*-5-(4-bromophenyl)-1-(2,4-dichlorophenyl)-4-ethyl-4,5-dihydro-1H-pyrazole-3-carbonyl** chloride

IR (NaCl film, $cm^{-1}$) : 1728, 1526, 1478, 1227, 1200, 1153, 1129, 834, 801.

***Cis*-5-(5-chlorothiophen-2-yl)-1-(2,4-dichlorophenyl)-4-methyl-4,5-dihydro-1H-pyrazole-3-carbonyl chloride**

IR (NaCl film, $cm^{-1}$): 1732, 1528, 1477, 1446, 1226, 1112, 808.

***Trans*-5-(5-chlorothiophen-2-yl)-1-(2,4-dichlorophenyl)-4-methyl-4,5-dihydro-1H-pyrazole-3-carbonyl chloride**

IR (NaCl film, $cm^{-1}$): 1730, 1531, 1479,1204,1122, 806.

***Cis*-5-(5-bromothiophen-2-yl)-1-(2,4-dichlorophenyl)-4-methyl-4,5-dihydro-1H-pyrazole-3-carbonyl chloride**

IR (NaCl film, $cm^{-1}$): 1732, 1528, 1477, 1109, 806

**cis-1-(2,4-dichlorophenyl)-4-methyl-5-(thiophen-2-yl)-4,5-dihydro-1 H-pyrazole-3- carbonyl** chloride

IR (NaCl film, $cm^{-1}$): 1731, 1527, 1477, 1224, 1111, 803.

**Subgroup A6.1: Chemical Example: Preparation of compounds of general formula I**

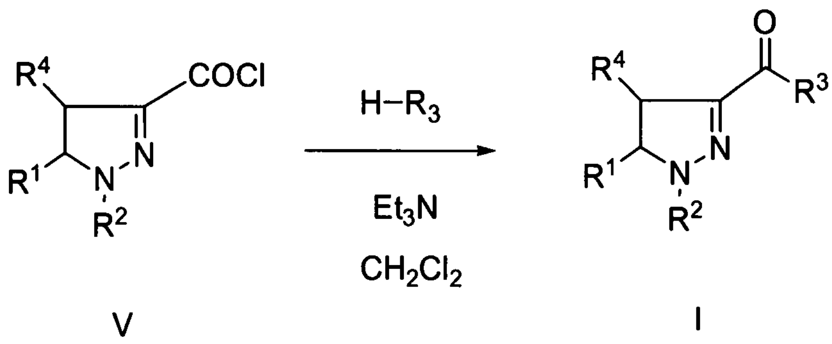


A compound of general formula $R^3$-H (5.6 mmol) and triethylamine (4 mL) were dissolved in dichloromethane (25 mL) under nitrogen atmosphere. The resulting mixture was cooled to 0˚C and a solution of a compound of general formula V (4.6 mmol) in dichloromethane (15 mL) was added dropwise. The resulting reaction mixture was stirred at room temperature (approximately 25 ˚C) overnight, washed with water, saturated aqueous solution of sodium bicarbonate and again with water, dried over sodium sulfate, filtered and evaporated to dryness in a rotavapor. The residue was crystallized from ethanol, ethyl acetate or acetone. The crystallized solid was removed via filtration and the mother liquors were concentrated to yield a second fraction of crystallized product. The two fractions were combined to give the desired product (yield range: 60-80 %). Alternatively, a solution of 2 N HCl in diethyl ether or 2.8 N in ethanol was added to form the respective hydrochloride, which was collected by filtration.

**Subgroup A6.1: Chemical Example 1: *cis*-5-(4-Chlorophenyl)-1-(2,4-dichlorophenyl)-4-methyl-4,5-dihydro-1H-pyrazole-3-carboxylic acid piperidin-1-ylamide hydrochloride**

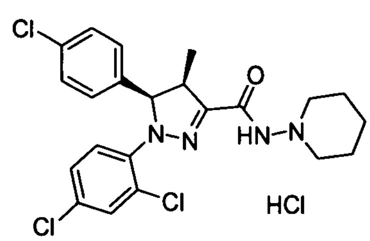

$^1$H NMR (400 MHz, METHANOL-$d_4$) δ ppm 0.94 (d, *J*=7.42 Hz, 3 H) 1.68 (br, 2 H) 1.98 (dt, *J*=11.28, 5.59 Hz, 4 H) 3.53 (br, 4 H) 3.90 (td, *J*=11.53, 7.42 Hz, 1 H) 5.99 (d, *J*=11.53 Hz, 1 H) 7.16 (d, *J*=8.40 Hz, 2 H) 7.20 - 7.29 (m, 3 H) 7.36 - 7.48 (m, 2 H)
MS $(M+H)^+$: 466

**Subgroup A6.1: Chemical Example 2: *trans*-5-(4-Chloro-phenyl)-1-(2,4-dichlorophenyl)-4-methyl-4,5-dihydro-1 H-pyrazole-3-carboxylic acid piperidin-1-ylamide hydrochloride**

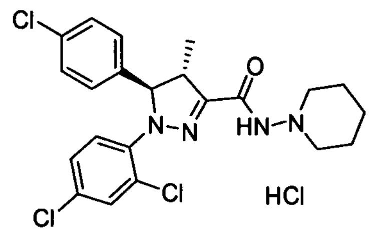

$^1$H NMR (400 MHz, DMSO-$d_6$) δ ppm 1.38 (d, *J*=7.03 Hz, 3 H) 1.42 (m, 2 H) 1.71 (m, 4 H) 3.12 (m, 4 H) 3.42 (dt, *J*=13.38, 5.86 Hz, 1 H) 5.36 (d, *J*=5.86 Hz, 1 H) 7.19 (d, *J*=8.21 Hz, 2 H) 7.31 (d, *J*=8.60 Hz, 3 H) 7.49 (d, *J*=2.34 Hz, 1 H) 7.53 (d, *J*=8.60 Hz, 1 H) 10.49 (s, 1 H)
MS $(M+H)^+$: 466

**Subgroup A6.1: Chemical Example 15: *cis*-5-(4-chlorophenyl)-1-(2,4-dichlorophenyl)-N-(indolin-1-yl)-4-methyl-4,5-dihydro-1 H-pyrazole-3-carboxamide**

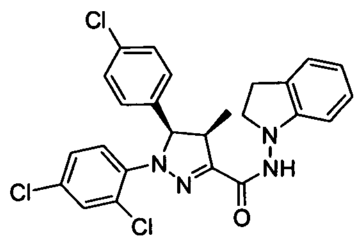

$^1$H NMR (300 MHz, CHLOROFORM-*d*): δ ppm 0.97 (d, *J*=7.18 Hz, 3 H) 3.08 (t, *J*=7.98 Hz, 2 H) 3.67 (t, *J*=7.98 Hz, 2 H) 3.86 (dd, *J*=11.06, 7.40 Hz, 1 H) 5.69 (d, *J*=11.28 Hz, 1 H) 6.73 (d, J=7.76 Hz, 1 H) 6.88 (t, *J*=7.40 Hz, 1 H) 7.02-7.12 (m, 5 H) 7.15 (d, *J*=7.76 Hz, 2 H) 7.24 (s, 1 H) 7.33 (d, *J*=1.61 Hz, 1 H) 7.99 (s, 1 H)
MS $(M+H)^+$: 499

**Subgroup A6.1: Chemical Example 113: *cis*-5-(4-chlorophenyl)-1-(2,4-dichlorophenyl)-4-methyl-N-(2-methylindolin-1-yl)-4,5-dihydro-1H-pyrazole-3-carboxamide**

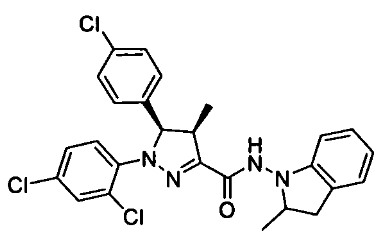

$^1$H NMR (300 MHz, DMSO-$d_6$): δ ppm 0.81 (t, *J*=7.40 Hz, 3 H) 1.29 (d, *J*=5.86 Hz, 3 H) 2.54 - 2.65 (m, 1 H) 3.14 (m, 1 H) 3.92 (m, 2 H) 5.99 (d, *J*=11.28 Hz, 1 H) 6.45 (dd, *J*=7.54, 2.86 Hz, 1 H) 6.68 - 6.82 (m, 1 H) 6.96 - 7.23 (m, 4 H) 7.30 - 7.36 (m, 3H) 7.48 (d, *J*=2.05 Hz, 1 H) 7.71 (d, *J*=8.79 Hz, 1 H) 10.09 (s, 1 H)
MS $(M+H)^+$: 513

**Subgroup A6.1: Chemical Example 215: *cis*-5-(4-bromophenyl)-1-(2,4-dichlorophenyl)-N-(indolin-1-yl)-4-methyl-4,5-dihydro-1H-pyrazole-3-carboxamide**

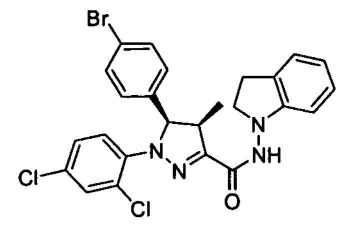

$^1$H NMR (300 MHz, DMSO-$d_6$):δ ppm 0.80 (d, *J*=7.18 Hz, 3 H) 2.96 (t, *J*=8.50 Hz, 2 H) 3.59 (t, J=8.50 Hz, 2 H) 3.89 (dd, *J*=11.13, 7.18 Hz, 1 H) 5.95 (d, *J*=11.13 Hz, 1 H) 6.49 (d, *J*=7.62 Hz, 1 H) 6.75 (t, *J*=7.03 Hz, 1 H) 6.99 - 7.14 (m, 4 H) 7.31 (dd, *J*=8.79, 2.49 Hz, 1 H) 7.42 - 7.50 (m, 3 H) 7.66 (d, *J*=8.79 Hz, 1 H) 10.19 (s, 1 H)
MS $(M+H)^+$: 543

**Subgroup A6.1: Chemical Example 235: *cis*-1-(2,4-dichlorophenyl)-5-(4-fluorophenyl)-N-(indolin-1-yl)-4-methyl-4,5-dihydro-1H-pyrazole-3-carboxamide**

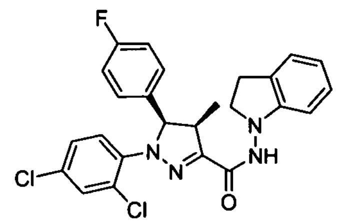

$^1$H NMR (300 MHz, DMSO-$d_6$) δ ppm 0.80 (d, *J*=7.32 Hz, 3 H) 2.96 (t, *J*=8.50 Hz, 2 H) 3.59 (t, *J*=8.50 Hz, 2 H) 3.87 (dd, *J*=11.13, 7.32 Hz, 1 H) 5.96 (d, *J*=11.13 Hz, 1 H) 6.49 (d, *J*=7.62 Hz, 1 H) 6.75 (t, *J*=7.40 Hz, 1 H) 7.00 - 7.19 (m, 6 H) 7.30 (dd, *J*=8.72, 2.42 Hz, 1 H) 7.47 (d, J=2.34 Hz, 1 H) 7.67 (d, *J*=8.79 Hz, 1 H) 10.18 (s, 1 H)
MS $(M+H)^+$: 483

**Subgroup A6.1: Chemical Example 279: cis-1-(2,4-dichlorophenyl)-N-(indolin-1-yl)-5-(4-methoxyphenyl)-4-methyl-4,5-dihydro-1H-pyrazole-3-carboxamide**

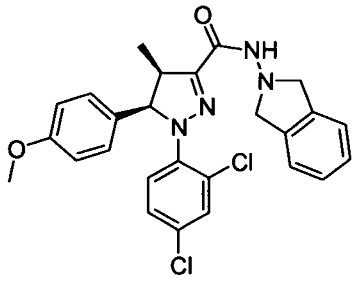

$^1$H NMR (300 MHz, CHLOROFORM-*d*) δ ppm 0.99 (d, *J*=7.18 Hz, 3 H) 3.08 (t, *J*=7.98 Hz, 2 H) 3.68 (t, *J*=7.91 Hz, 2 H) 3.76 (s, 3 H) 3.83 (dd, *J*=11.13, 7.18 Hz, 1 H) 5.68 (d, *J*=11.13 Hz, 1 H) 6.79 (d, *J*=8.50 Hz, 2 H) 6.74 (d, *J*=7.76 Hz, 1 H) 6.88 (t, *J*=7.40 Hz, 1 H) 7.02 - 7.09 (m, 4 H) 7.16 (d, *J*=7.47 Hz, 2 H) 7.32 (s, 1 H) 8.01 (s, 1 H)
MS $(M+H)^+$: 495

**Subgroup A6.1: Chemical Example 123: *cis*-5-(4-chlorophenyl)-N-cycloheptyl-1-(2,4-dichlorophenyl)-4-methyl-4,5-dihydro-1H-pyrazole-3-carboxamide**

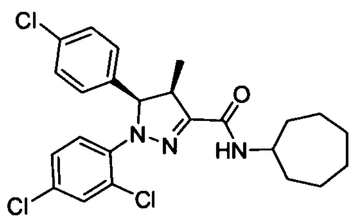

$^1H$ NMR (300 MHz, DMSO-$d_6$) δ ppm 0.76 (d, *J*=7.18 Hz, 3 H) 1.39 -1.70 (m, 10 H) 1.78 (m, 2 H) 3.83 (m, 2 H) 5.89 (d, *J*=10.99 Hz, 1 H) 7.14 (d, *J*=8.35 Hz, 2 H) 7.32 (m, 3 H) 7.49 (d, *J*=2.34 Hz, 1 H) 7.62 (d, *J*=8.79 Hz, 1 H) 8.04 (d, *J*=8.06 Hz, 1 H)
MS $(M+H)^+$: 478

**Subgroup A6.1: Chemical Example 193: *cis*-5-(4-bromophenyl)-N-cycloheptyl-1-(2,4-dichlorophenyl)-4-methyl-4,5-dihydro-1H-pyrazole-3-carboxamide**

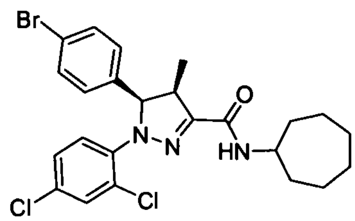

$^1H$ NMR (300 MHz, DMSO-$d_6$) δ ppm 0.74 (d, *J*=7.18 Hz, 3 H) 1.35 -1.66 (m, 10 H) 1.78 (m, 2 H) 3.72 - 3.90 (m, 2 H) 5.85 (d, *J*=10.99 Hz, 1 H) 7.06 (d, *J*=8.35 Hz, 2 H) 7.30 (dd, J=8.72, 2.27 Hz, 1 H) 7.44 (d, *J*=8.35 Hz, 2 H) 7.47 (d, *J*=2.20 Hz, 1 H) 7.60 (d, *J*=8.79 Hz, 1 H) 8.02 (d, *J*=8.20 Hz, 1 H)
MS $(M+H)^+$: 522

**Subgroup A6.1: Chemical Example 231: cis-N-cycloheptyl-1-(2,4-dichlorophenyl)-5-(4-fluorophenyl)-4-methyl-4,5-dihydro-1 H-pyrazole-3-carboxamide**

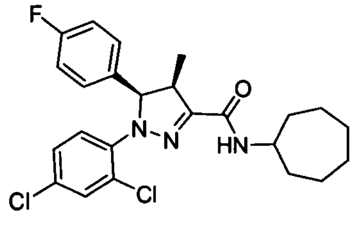

$^1H$ NMR (300 MHz, DMSO-$d_6$) δ ppm 0.75 (d, *J*=7.32 Hz, 3 H) 1.36 -1.67 (m, 10 H) 1.71 - 1.85 (m, 2 H) 3.70 - 3.90 (m, 2 H) 5.86 (d, *J*=10.99 Hz, 1 H) 7.02 - 7.18 (m, 4 H) 7.29 (dd, *J*=8.79, 2.34 Hz, 1 H) 7.46 (d, *J*=2.34 Hz, 1 H) 7.61 (d, *J*=8.79 Hz, 1 H) 8.02 (d, J=8.20 Hz, 1 H)
MS $(M+H)^+$: 462

**Subgroup A6.1: Chemical Example 275: cis-N-cycloheptyl-1-(2,4-dichlorophenyl)-5-(4-methoxyphenyl)-4-methyl-4,5-dihydro-1H-pyrazole-3-carboxamide**

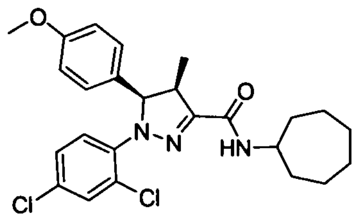

$^1$H NMR (300 MHz, DMSO-$d_6$) δ ppm 0.75 (d, *J*=7.32 Hz, 3 H) 1.36-1.67 (m, 10 H) 1.75-1.86 (m, 2 H) 3.65 (s, 3 H) 3.72 (dd, *J*=10.99, 7.32 Hz, 1 H) 3.82 (dd, *J*=8.72, 4.47 Hz, 1 H) 5.79 (d, J=10.99 Hz, 1 H) 6.77 (d, *J*=8.64 Hz, 2 H) 7.01 (d, *J*=8.64 Hz, 2 H) 7.27 (dd, *J*=8.79, 2.34 Hz, 1 H) 7.44 (d, *J*=2.34 Hz, 1 H) 7.61 (d, *J*=8.79 Hz, 1 H) 7.99 (d, *J*=8.20 Hz, 1 H)
MS $(M+H)^+$: 474

**Subgroup A6.1: Chemical Example 128: (+)-cis-5-(4-chlorophenyl)-1-(2,4-dichlorophenyl)-N-((1 S,2S)-2-hydroxycyclohexyl)-4-methyl-4,5-dihydro-1 H-pyrazole-3-carboxamide**

$^1$H NMR (300 MHz, CHLOROFORM-*d*) δ ppm 0.94 (d, *J*=7.32 Hz, 3 H) 1.19-1.45 (m, 4 H) 1.76 (m, 2 H) 2.00 (m, 1 H) 2.09 (m, 1 H) 3.43 (m, 1 H) 3.78 (m, 2 H) 5.66 (d, *J*=11.28 Hz, 1 H) 6.65 (d, *J*=7.32 Hz, 1 H) 7.00 - 7.15 (m, 4 H) 7.24 (m, 2 H) 7.33 (s, 1 H)
MS $(M+H)^+$: 480

**Subgroup A6.1: Chemical Example 129: (-)-cis-5-(4-chlorophenyl)-1-(2,4-dichlorophenyl)-N-((1S,2S)-2-hydroxycyclohexyl)-4-methyl-4,5-dihydro-1H-pyrazole-3-carboxamide**

$^1$H NMR (300 MHz, CHLOROFORM-*d*) δ ppm 0.93 (d, *J*=7.32 Hz, 3 H) 1.24-1.40 (m, 4 H) 1.75 (m, 2 H) 2.06 (m, 2 H) 3.39 (m, 1 H) 3.75 (m, 2 H) 5.64 (d, *J*=11.13 Hz, 1 H) 6.68 (d, *J*=7.32 Hz, 1 H) 7.00 - 7.13 (m, 4 H) 7.23 (d, *J*=8.50 Hz, 2 H) 7.35 (d, *J*=2.20 Hz, 1 H)
MS $(M+H)^+$: 480

**Subgroup A6.1: Chemical Example 108: cis-5-(4-chlorophenyl)-1-(2,4-dichlorophenyl)-N-(hexahydrocyclopenta-[c]pyrrol-2(1H)-yl)-4-methyl-4,5-dihydro-1 H-pyrazole-3-carboxamide hydrochloride**

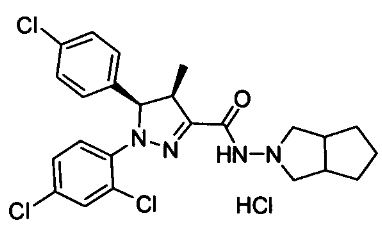

$^1$H NMR (300 MHz, DMSO-$d_6$) δ ppm 0.79 (d, *J*=7.18 Hz, 3 H) 1.49 (d, *J*=7.76 Hz, 2 H) 1.61 (m, 4 H) 2.66 (br. S., 2 H) 2.86 (m, 2 H) 3.54 (br. S., 2 H) 3.85 (m, 1 H) 5.96 (d, J=11.28 Hz, 1 H) 7.17 (d, *J*=8.35 Hz, 2 H) 7.35 (m, 3 H) 7.55 (d, *J*=2.20 Hz, 1 H) 7.62 (d, J=8.64 Hz, 1 H) 10.52 (br. S., 1 H)
MS $(M+H)^+$: 491

**Subgroup A6.1: Chemical Example 213: *cis*-5-(4-bromophenyl)-1-(2,4-dichlorophenyl)-N-(hexahydrocyclopenta-[c]pyrrol-2(1 H)-yl)-4-methyl-4,5-dihydro-1 H-pyrazole-3-carboxamide hydrochloride**

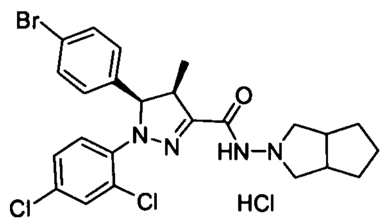

$^1$H NMR (300 MHz, DMSO-$d_6$) δ ppm 0.77 (d, *J*=7.32 Hz, 3 H) 1.47 (d, *J*=7.47 Hz, 2 H) 1.58-1.64 (m, 4 H) 2.64 (br. S., 2 H) 2.83 (dd, *J*=16.70, 7.47 Hz, 2 H) 3.52 (br. S., 2 H) 3.83 (dd, *J*=11.21, 7.40 Hz, 1 H) 5.92 (d, *J*=11.13 Hz, 1 H) 7.08 (d, *J*=8.20 Hz, 2 H) 7.33 (dd, *J*=8.72, 2.27 Hz, 1 H) 7.46 (d, *J*=8.35 Hz, 2 H) 7.52 (d, *J*=2.20 Hz, 1 H) 7.59 (d, J=8.79 Hz, 1 H) 10.47 (br. S., 1 H)
MS $(M+H)^+$: 535

**Subgroup A6.1: Chemical Example 233: *cis*-1-(2,4-dichlorophenyl)-5-(4-fluorophenyl)-N-(hexahydrocyclopenta-[c]pyrrol-2(1H)-yl)-4-methyl-4,5-dihydro-1H-pyrazole-3-carboxamide hydrochloride**

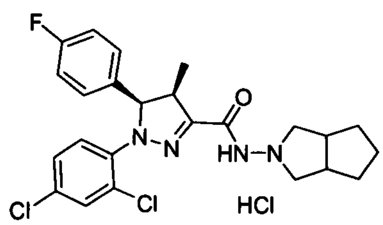

$^1$H NMR (300 MHz, DMSO-$d_6$) δ ppm 0.77 (d, *J*=7.32 Hz, 3 H) 1.47 (d, *J*=8.64 Hz, 2 H) 1.55-1.67 (m, 4 H) 2.63 (br. S., 2 H) 2.81 (dd, *J*=15.16, 8.86 Hz, 2 H) 3.49 (br. S., 3 H) 5.93 (d, *J*=11.28 Hz, 1 H) 7.01 - 7.25 (m, 4 H) 7.32 (dd, *J*=8.72, 2.42 Hz, 1 H) 7.51 (d, *J*=2.34 Hz, 1 H) 7.60 (d, *J*=8.64 Hz, 1 H) 10.35 (br. S., 1 H)
MS $(M+H)^+$: 475

**Subgroup A6.1: Chemical Example 96: *cis*-N-(azepan-1-yl)-5-(4-chlorophenyl)-1-(2,4-dichlorophenyl)-4-methyl-4,5-dihydro-1H-pyrazole-3-carboxamide hydrochloride**

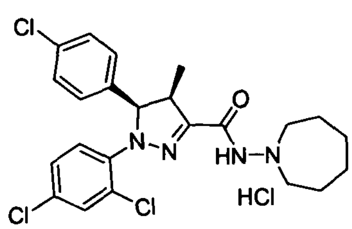

$^1$H NMR (300 MHz, DMSO-$d_6$) δ ppm 0.78 (d, *J*=7.18 Hz, 3 H) 1.58 (br. S., 4 H) 1.76 (br. S., 4 H) 3.32 (br. S, 4 H) 3.85 (m, 1 H) 5.95 (d, *J*=11.28 Hz, 1 H) 7.15 (d, *J*=8.50 Hz, 2 H) 7.23 - 7.36 (m, 3 H) 7.51 (d, *J*=2.34 Hz, 1 H) 7.61 (d, *J*=8.79 Hz, 1 H) 10.89 (br. S., 1 H)
MS $(M+H)^+$: 479

**Subgroup A6.1: Chemical Example 207: cis-N-(azepan-1-yl)-5-(4-bromophenyl)-1-(2,4-dichlorophenyl)-4-methyl-4,5-dihydro-1 H-pyrazole-3-carboxamide hydrochloride**

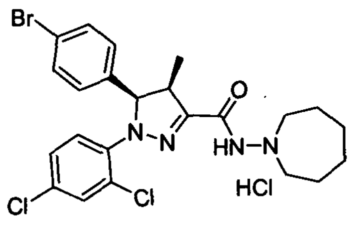

$^1$H NMR (300 MHz, DMSO-$d_6$) δ ppm 0.78 (d, *J*=7.18 Hz, 3 H) 1.58 (br. S., 4 H) 1.77 (br. S., 4 H) 3.33 (br. S., 4 H) 3.85 (dd, *J*=11.28, 7.18 Hz, 1 H) 5.94 (d, *J*=11.28 Hz, 1 H) 7.08 (d, *J*=8.35 Hz, 2 H) 7.34 (dd, *J*=8.72, 2.27 Hz, 1 H) 7.46 (d, *J*=8.35 Hz, 2 H) 7.52 (d, J=2.34 Hz, 1 H) 7.61 (d, *J*=8.64 Hz, 1 H) 10.98 (br. S., 1 H)
MS $(M+H)^+$: 523

**Subgroup A6.1: Chemical Example 227: *cis*-N-(azepan-1-yl)-5-(4-fluorophenyl)-1-(2,4-dichlorophenyl)-4-methyl-4,5-dihydro-1H-pyrazole-3-carboxamide hydrochloride**

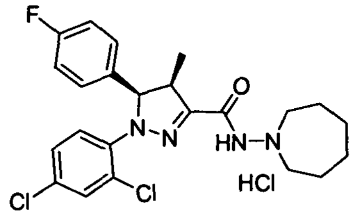

$^1$H NMR (300 MHz, DMSO-$d_6$) δ ppm 0.78 (d, *J*=7.32 Hz, 3 H) 1.59 (br. S., 4 H) 1.78 (br. S., 4 H) 3.36 (br. S., 4 H) 3.84 (dd, *J* = 11.43, 7.32 Hz, 1 H) 5.96 (d, *J* = 11.43 Hz, 1 H) 6.99- 7.21 (m, 4 H) 7.33 (dd, *J*=8.79, 2.20 Hz, 1 H) 7.51 (d, *J*= 2.34 Hz, 1 H) 7.63 (d, *J*=8.79 Hz, 1 H), 11.06 (br. S., 1 H)
MS $(M+H)^+$: 463

**Subgroup A6.1: Chemical Example 271:**

***cis*-N-(azepan-1-yl)-1-(2,4-dichlorophenyl)-5-(4-methoxyphenyl)-4-methyl-4,5-dihydro-1H-pyrazole-3-carboxamide hydrochloride**

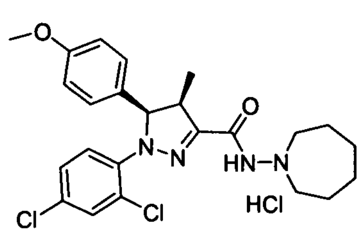

$^1$H NMR (300 MHz, DMSO-$d_6$) δ ppm 0.80 (d, *J*=7.18 Hz, 3 H) 1.59 (br. S., 4 H) 1.79 (br. S., 4 H) 3.38 (d, *J*=4.54 Hz, 4 H) 3.66 (s, 3 H) 3.80 (dd, *J*=11.28, 7.32 Hz, 1 H) 5.89 (d, *J*=11.28 Hz, 1 H) 6.78 (d, *J*=8.50 Hz, 2 H) 7.03 (d, *J*=8.50

Hz, 2 H) 7.32 (dd, *J*=8.79, 2.34 Hz, 1 H) 7.49 (d, J= 2.34 Hz, 1 H) 7.63 (d, *J*=8.79 Hz, 1 H) 11.12 (br. S., 1 H)
MS $(M+H)^+$: 475

**Subgroup A6.1: Chemical Example 277: cis-1-(2,4-dichlorophenyl)-N-(hexahydrocyclopenta[c]pyrrol-2(1H)-yl)-5-(4-methoxyphenyl)-4-methyl-4,5-dihydro-1H-pyrazole-3-carboxamide hydrochloride**

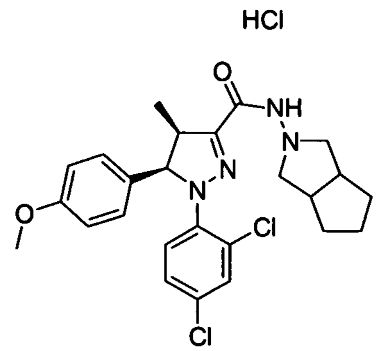

$^1$H NMR (300 MHz, CHLOROFORM-*d*) δ ppm 0.94 (d, *J*=7.32 Hz, 3 H) 1.78 (m, 6 H) 3.03 (br. S., 2 H) 3.74 (s, 3 H) 3.66 - 3.88 (m, 3 H) 3.99 (br. S., 2 H) 5.86 (d, *J*=11.86 Hz, 1 H) 6.75 (d, J=8.50 Hz, 2 H) 6.96 (d, *J*=8.50 Hz, 2 H) 7.12 (dd, *J*=8.64, 2.20 Hz, 1 H) 7.25 (d, *J*=2.20 Hz, 1 H) 7.33 (d, *J*=8.64 Hz, 1 H) 9.58 (br. S., 1 H)
MS $(M+H)^+$: 487

**Subgroup A6.1: Chemical Example 343: *cis*-1-(2,4-dichlorophenyl)-N-(hexahydrocyclopenta[c]pyrrol-2(1H)-yl)-5-(4-hydroxyphenyl)-4-methyl-4,5-dihydro-1H-pyrazole-3-carboxamide hydrochloride**

Example compound 277 (0.82 mmol) was dissolved in 10 mL dichloromethane. A solution of 1 M $BBr_3$ (5 eq.) in dichloromethane was added at 0 ˚C and the reaction was stirred overnight at ambient temperature. The solid formed was filtered off and the solvent was evaporated. A solution of 2 N HCl/diethyl ether was added to form the hydrochloride.

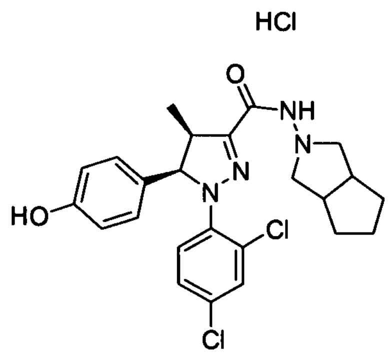

$^1$H NMR (300 MHz, METHANOL-$d_4$) δ ppm 0.95 (d, *J*=7.18 Hz, 3 H) 1.65 (br. S., 2 H) 1.79 (br. S., 4 H) 2.95 (br. S., 2 H) 3.11 (dd, *J*=16.48, 10.03 Hz, 2 H) 3.81 (dd, *J*=11.43, 7.18 Hz, 1 H) 4.03 (m, 2 H) 5.90 (d, *J*=11.43 Hz, 1 H) 6.64 (d, *J*=8.06 Hz, 2 H) 6.95 (d, *J*=8.06 Hz, 2 H) 7.20 (m, 1 H) 7.39 (d, *J*=2.20 Hz, 1 H) 7.42 (d, *J*=8.79 Hz, 1 H)
MS $(M+H)^+$: 473

**Subgroup A6.1: Chemical Example 92: (+)-cis-5-(4-Chlorophenyl)-1-(2,4-dichlorophenyl)-4-methyl-4,5-dihydro-1H-pyrazole-3-carboxylic acid piperidin-1-ylamide hydrochloride**

$^1$H NMR (400 MHz, METHANOL-$d_4$) δ ppm 0.94 (d, *J*=7.42 Hz, 3 H) 1.68 (br, 2 H) 1.98 (dt, *J*=11.28, 5.59 Hz, 4 H) 3.53 (br, 4 H) 3.90 (td, *J*=11.53, 7.42 Hz, 1 H) 5.99 (d, *J*=11.53 Hz, 1 H) 7.16 (d, *J*=8.40 Hz, 2 H) 7.20 - 7.29 (m, 3 H) 7.36 - 7.48 (m, 2 H)

$[\alpha]_{589}^{RT}$= + 131.73

**Subgroup A6.1: Chemical Example 93: (-)-*cis*-5-(4-Chlorophenyl)-1-(2,4-dichlorophenyl)-4-methyl-4,5-dihydro-1H-pyrazole-3-carboxylic acid piperidin-1-ylamide hydrochloride**

$^1$H NMR (400 MHz, METHANOL-$d_4$) δ ppm 0.94 (d, *J*=7.42 Hz, 3 H) 1.68 (br, 2 H) 1.98 (dt, *J*=11.28, 5.59 Hz, 4 H) 3.53 (br, 4 H) 3.90 (td, *J*=11.53, 7.42 Hz, 1 H) 5.99 (d, *J*=11.53 Hz, 1 H) 7.16 (d, *J*=8.40 Hz, 2 H) 7.20 - 7.29 (m, 3 H) 7.36 - 7.48 (m, 2 H)
$[\alpha]_{589}^{RT}$ = - 126.71

**Subgroup A6.1: Chemical Example 379: *cis*-5-(4-chlorophenyl)-1-(2,4-dichlorophenyl)-4-methyl-N-(piperidin-1-yl)-4,5-dihydro-1H-pyrazole-3-carbothioamide hydrochloride**

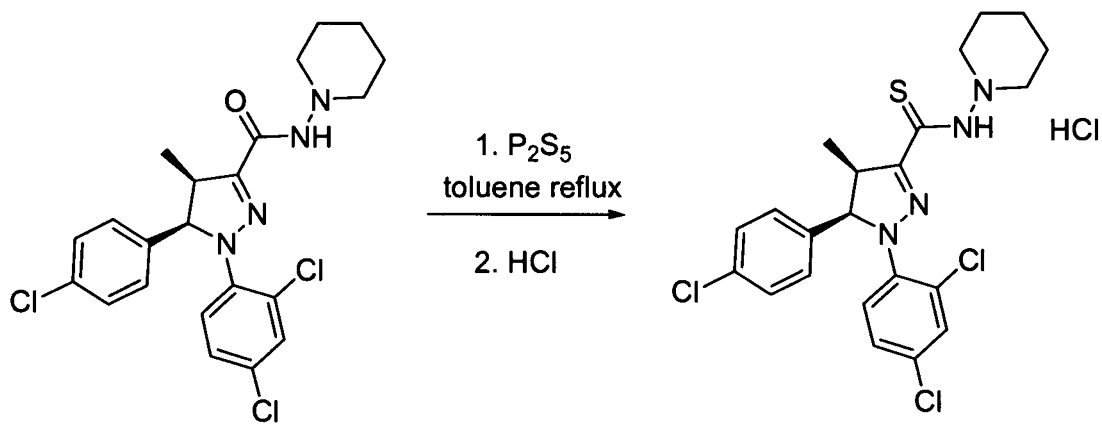


Phosphorous pentasulfide was added in portions (5 x 0.570 g, 12.9 mmol) to a solution of example compound 1 (6.46 mmol) in dry toluene (30 ml). The mixture was stirred and heated to reflux for 15 hours. The solid was filtered off and washed with dichloromethane. The solvent was evaporated and a bright yellow solid was isolated, which was then treated with aqueous ammonium solution and extracted with ethyl acetate, dried over $Na_2SO_4$ and filtered off. The solid obtained after evaporation of the solvent was dissolved in acetone and a solution of 2 N HCl in diethyl ether to form the corresponding hydrochloride. The pale yellow solid obtained by filtration was identified as *cis*-5-(4-chlorophenyl)-1-(2,4-dichlorophenyl)-4-methyl-N-(piperidin-1-yl)-4,5-dihydro-1H-pyrazole-3-carbothioamide hydrochloride (85% yield).
$^1$H NMR (300 MHz, METHANOL-$d_4$) δ ppm 0.95 (d, *J*=7.32 Hz, 3 H) 1.62 -1.78 (m, 2 H) 2.00 (m, 4 H) 3.60 (m, 4 H) 3.99 (dd, *J*=10.84, 7.32 Hz, 1 H) 5.91 (d, *J*=10.84 Hz, 1 H) 7.22 (d, *J*=8.79 Hz, 1 H) 7.18 - 7.25 (m, 2 H) 7.25 - 7.31 (m, 2 H) 7.40 (d, J=8.79 Hz, 1 H) 7.45 (d, J=2.20 Hz, 1 H)
MS $(M+H)^+$: 481

**Subgroup A6.1: Chemical Example 1: *cis*-5-(4-Chlorophenyl)-1-(2,4-dichlorophenyl)-4-methyl-4,5-dihydro-1H-pyrazole-3-carboxylic acid piperidin-1-ylamide hydrochloride**

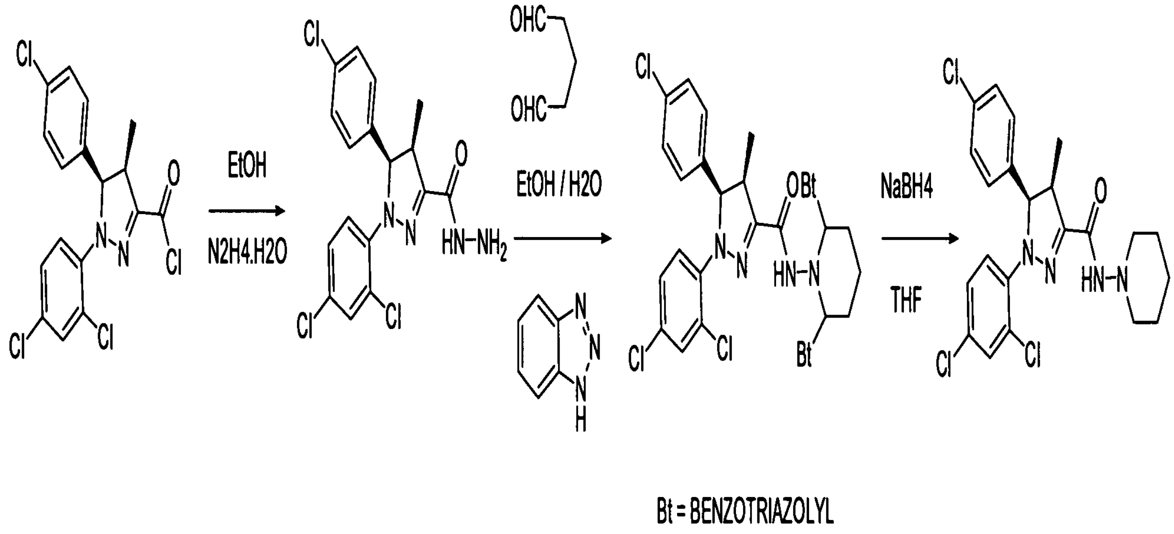


**Step 1: Preparation of *cis*-5-(4-Chloro-phenyl)-1-(2,4-dichlorophenyl)-4-methyl-4,5-dihydro-1H-pyrazole-3-carboxylic acid hydrazide**

*cis*-5-(4-Chloro-phenyl)-(2,4-dichloro-phenyl)-4-methyl-4,5-dihydro-1H-pyrazole-3-carbonyl chloride (5.40 mmol) was refluxed for 2 hours in ethanol (25 mL). The reaction mixture was cooled to room temperature and 5 g of the hydrazine hydrate were added. The reaction mixture was heated to reflux for 4 hours, cooled to room temperature and the solvent was removed in vacuum. The resulting residue was taken up in dichloromethane (30 mL) and washed with water, dried and concentrated in vacuum. Chromatography with silica gel, eluting with Chloroform/ Methanol system of appropriate polarity, yielded the title compound (65% yield) as a white solid.

**Step 2: *cis*-5-(4-Chloro-phenyl)-1-(2,4-dichloro-phenyl)-4-methyl-4,5-dihydro-1H-pyrazole-3-carboxylic acid (2,6-benzotriazlyl-piperidin-1-yl)amide**

Benzotriazole (119 mg, 1 mmol), *cis*-5-(4-Chloro-phenyl)-1-(2,4-dichloro-phenyl)-4-methyl-4,5-dihydro-1H-pyrazole-3-carboxylic acid hydrazide (0.5 mmol ), water (5 mL) and ethanol (5 mL) were stirred for 10 min at room temperature. Glutaraldehyde (200 mg, 0.5 mMol, 25% aqueous solution) was slowly added to the reaction mixture, and stirring was continued overnight at room temperature. The solvent was removed and the insoluble products were filtered off, washed with water, and dried under vacuum, yielding the title compound (66% yield) as a white solid.

**Step 3: *cis*-5-(4-Chloro-phenyl)-1-(2,4-dichloro-phenyl)-4-methyl-4,5-dihydro-1H-pyrazole-3-carboxylic acid piperidin-1-ylamide**

*cis*-5-(4-Chloro-phenyl)-1-(2,4-dichloro-phenyl)-4-methyl-4,5-dihydro-1H-pyrazole-3-carboxylic acid(2,6-benzotriazlyl-piperidin-1-yl)amide (0.33mmol) in tetrahydrofuran (10 mL) and sodium borohydride ( 38 mg, 1 mmol) were stirred at room temperature overnight. The reaction was quenched with water, and the product was extracted with dichloromethane, washed with water and dried. Solvent evaporation under reduced pressure led to the title compound. The product was purified by recrystallization from ethanol, yielding the title compound (41% yield) as a white solid.

**Subgroup A6.1: Chemical Examples**

The following examples were prepared using the methods described above.

| N° | STRUCTURE | Name | $^1$H-NMR | MS (M+H)$^+$ |
|---|---|---|---|---|
| 1 | 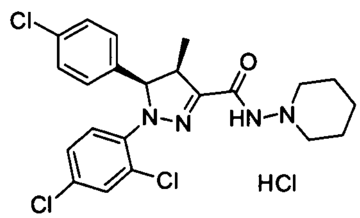 | *cis*-5-(4-Chloro-phenyl)-1- (2,4-dichloro-phenyl)-4-methyl-4,5-dihydro-1H- pyrazole-3-carboxylic acid piperidin-1-ylamide hydro-chloride | 1H NMR (400 MHz, METHANOL-$d_4$) δ ppm 0.94 (d, *J*=7.42 Hz, 3 H) 1.68 (br, 2 H) 1.98 (dt, *J*=11.28, 5.59 Hz, 4 H) 3.53 (br, 4 H) 3.90 (td, *J*=11.53, 7.42 Hz, 1 H) 5.99 (d, *J*=11.53 Hz, 1 H) 7.16 (d, | 466 |
| 2 | 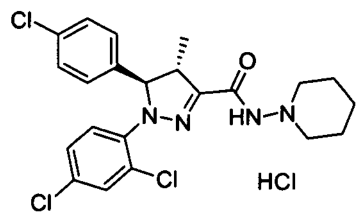 | *trans*-5-(4-Chloro-phenyl)-1- (2,4-dichloro-phenyl)-4-methyl-4,5-dihydro-1H- pyrazole-3-carboxylic acid piperidin-1-ylamide hydro-chloride | $^1$H NMR (400 MHz, DMSO-$d_6$) δ ppm 1.38 (d, *J*=7.03 Hz, 3 H) 1.42 (m, 2 H) 1.71 (m, 4 H) 3.12 (m, 4 H) 3.42 (dt, *J*=13.38, 5.86 Hz, 1 H) 5.36 (d, *J*=5.86 Hz, 1 H) 7.19 (d, *J*=8.21 Hz, 2 H) 7.31 (d, | 466 |
| 3 | 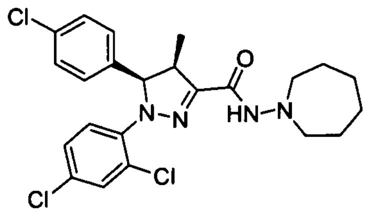 | cis-5-(4-Chloro-phenyl)-1-(2,4-dichloro-phenyl)-4-methyl-4,5-dihydro-1H-pyrazole-3-carboxylic acid azepan-1-ylamide | | |
| 4 | 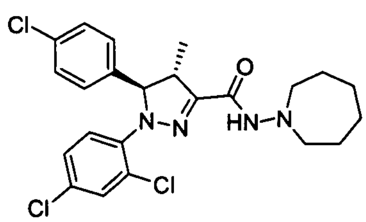 | trans-5-(4-Chloro-phenyl)-1-(2,4-dichloro-phenyl)-4-methyl-4,5-dihydro-1H-pyrazole-3-carboxylic acid azepan-1-ylamide | | |
| 5 | 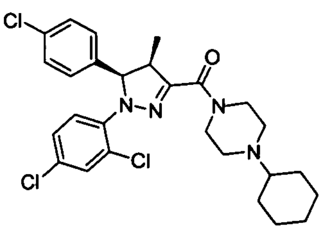 | cis-[5-(4-Chloro-phenyl)-1-(2,4-dichloro-phenyl)-4-methyl-4,5-dihydro-1H- pyrazol-3-yl]-(4-cyclohexyl-piperazin-1-yl)-methanone | | 533 |
| 6 | 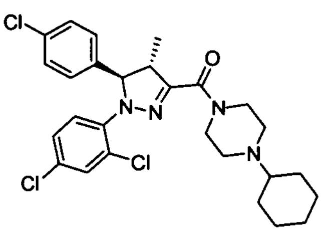 | *trans*-[5-(4-Chloro-phenyl)-1-(2,4-dichloro-phenyl)-4-methyl-4,5-dihydro-1H- pyrazol-3-yl]-(4-cyclohexyl-piperazin-1-yl)-methanone | | 533 |

(continued)

| N° | STRUCTURE | Name | $^1$H-NMR | MS (M+H)$^+$ |
|---|---|---|---|---|
| 7 | 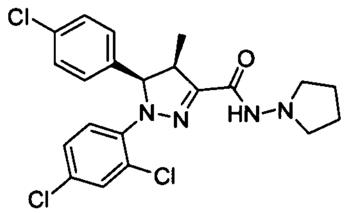 | *cis*-5-(4-Chloro-phenyl)-1-(2,4-dichloro-phenyl)-4-methyl-4,5-dihydro-1H- pyrazole-3-carboxylic acid pyrrolidin-1-ylamide | | 451 |
| 8 | 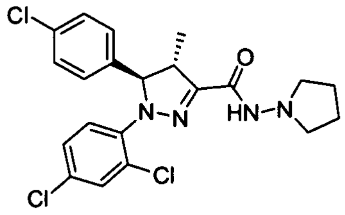 | trans-5-(4-Chloro-phenyl)-1-(2,4-dichloro-phenyl)-4-methyl-4,5-dihydro-1H- pyrazole-3-carboxylic acid pyrrolidin-1-ylamide | | 451 |
| 9 | 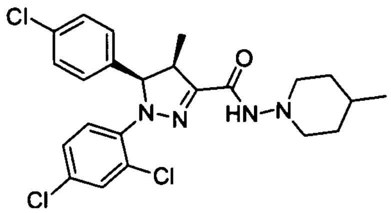 | *cis*-5-(4-Chloro-phenyl)-1-(2,4-dichloro-phenyl)-4-methyl-4,5-dihydro-1H- pyrazole-3-carboxylic acid (4-methyl-piperidin-1-yl)-amide | | 479 |
| 10 | 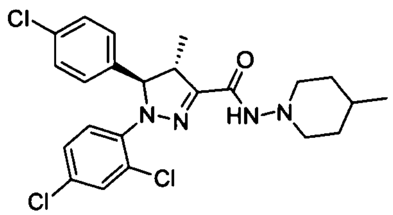 | *trans*-5-(4-Chloro-phenyl)-1-(2,4-dichloro-phenyl)-4-methyl-4,5-dihydro-1H- pyrazole-3-carboxylic acid (4-methyl-piperidin-1-yl)-amide | | 479 |
| 11 | 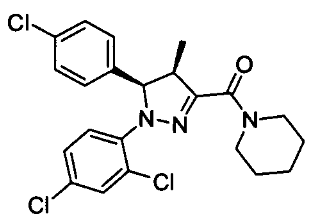 | *cis*-[5-(4-Chloro-phenyl)-1-(2,4-dichloro-phenyl)-4-methyl-4,5-dihydro-1H- pyrazol-3-yl]-piperidin-1-yl-methanone | | 450 |
| 12 | 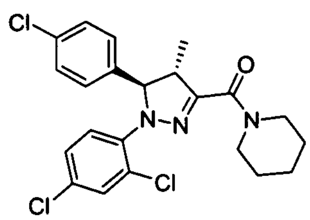 | *trans*-[5-(4-Chloro-phenyl)-1-(2,4-dichloro-phenyl)-4-methyl-4,5-dihydro-1H- pyrazol-3-yl]-piperidin-1-yl-methanone | | 450 |

(continued)

| N° | STRUCTURE | Name | 1H-NMR | MS (M+H)+ |
|---|---|---|---|---|
| 13 | 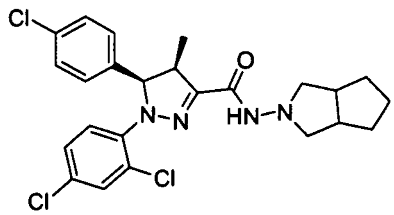 | *cis*-5-(4-Chloro-phenyl)-1-(2,4-dichloro-phenyl)-4-methyl-4,5-dihydro-1H- pyrazole-3-carboxylic acid (hexahydro-cyclopenta[c]pyrrol-2-yl)- | | 491 |
| 14 | 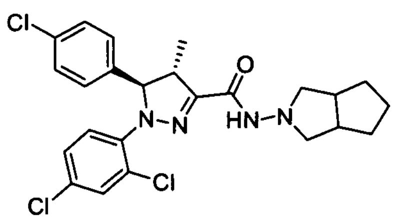 | trans-5-(4-Chloro-phenyl)-1-(2,4-dichloro-phenyl)-4-methyl-4,5-dihydro-1 H- pyrazole-3-carboxylic acid (hexahydro-cyclopenta-[c]py-rrol-2-yl)-amide | | 491 |
| 15 | 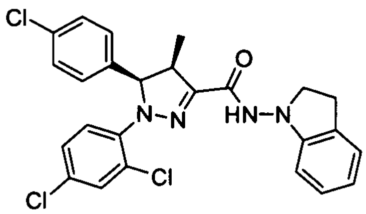 | cis-5-(4-Chloro-phenyl)-1-(2,4-dichloro-phenyl)-4-methyl-4,5-dihydro-1H- pyrazole-3-carboxylic acid (2,3-dihydro-indol-1-yl)-amide | | 499 |
| 16 | 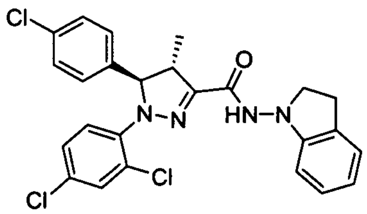 | *trans*-5-(4-Chloro-phenyl)-1-(2,4-dichloro-phenyl)-4-methyl-4,5-dihydro-1H- pyrazole-3-carboxylic acid (2,3-dihydro-indol-1-yl)-amide | | 499 |
| 17 | 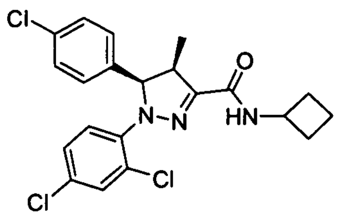 | *cis*-5-(4-Chloro-phenyl)-1-(2,4-dichloro-phenyl)-4-methyl-4,5-dihydro-1H- pyrazole-3-carboxylic acid cyclobutylamide | | 436 |
| 18 | 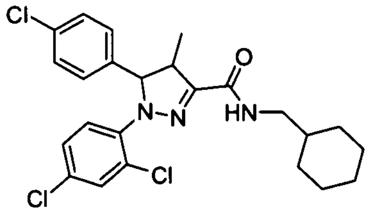 | 5-(4-Chloro-phenyl)-1-(2,4-dichloro-phenyl)-4-methyl-4,5-dihydro-1H-pyrazole-3-carboxylic acid cyclo-hexyl methyl-amide | | 478 |

(continued)

| N° | STRUCTURE | Name | $^1$H-NMR | MS (M+H)$^+$ |
|---|---|---|---|---|
| 19 | 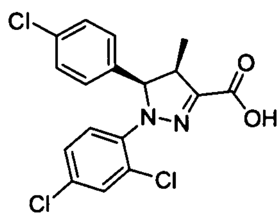 | *cis*-5-(4-Chloro-phenyl)-1- (2,4-dichloro-phenyl)-4-methyl-4,5-dihydro-1H-pyrazole-3-carboxylic acid | 1H NMR (400 MHz, CHLOROFORM-*d*) δ ppm 0.96 (d, *J*=7.42 Hz, 3 H) 3.82 (td, *J*=11.72, 7.42 Hz, 1 H) 5.91 (d, *J*=11.72 Hz, 1H) 7.04 (d, *J*=8.60 Hz, 2 H) 7.11 (dd, *J*=8.60, 2.34 Hz, 1 H) 7.21 - | 384 |
| 20 | 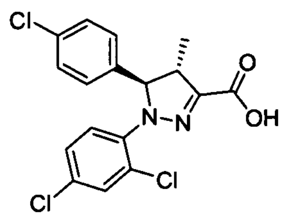 | *trans*-5-(4-Chloro-phenyl)-1- (2,4-dichloro-phenyl)-4-methyl-4,5-dihydro-1H-pyrazole-3-carboxylic acid | 1 H NMR (400 MHz, CHLOROFORM-*d*) δ ppm 1.52 (d, *J*=7.03 Hz, 3 H) 3.52 (m, 1 H) 5.39 (d, *J*=5.47 Hz, 1 H) 7.08 (ddd, *J*=13.92, 5.62, 5.28 Hz, 3 H) 7.14 - 7.34 (m, 4 H) | 384 |
| 21 | 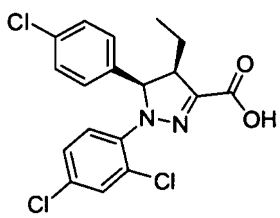 | *cis*-5-(4-Chloro-phenyl)-1-(2,4-dichloro-phenyl)-4-ethyl- 4,5-dihydro-1H-pyrazole-3-carboxylic acid | | 398 |
| 22 | 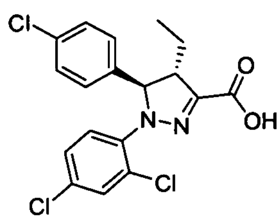 | *trans*-5-(4-Chloro-phenyl)-1-(2,4-dichloro-phenyl)-4-ethyl-4,5-dihydro-1H-pyrazole-3-carboxylic acid piperidin-1-ylamide | | 398 |
| 23 | 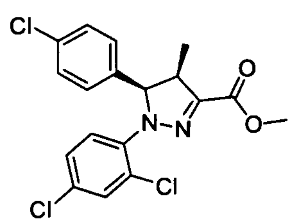 | *cis*-5-(4-Chloro-phenyl)-1- (2,4-dichloro-phenyl)-4-methyl-4.5-dihydro-1H-pyrazole-3-carboxylic acid methyl ester | 1H NMR(400 MHz, CHLOROFORM-*d*) δ ppm 0.94 (d, *J*=7.32 Hz,3 H) 3.80 (td, *J*=11.48, 7.32 Hz, 1 H) 3.88(s,3H) 5.82 (d, *J*=11.48 Hz, 1 H) 7.04 (m, 2 H) 7.10 (dd, *J*=8.67, 2.32 Hz, 1 H) | 398 |
| 24 | 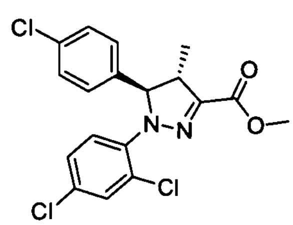 | *trans*-5-(4-Chloro-phenyl)-1- (2,4-dichloro-phenyl)-4-methyl-4,5-dihydro-1H-pyrazole-3-carboxylic acid methyl ester | 1H NMR (400 MHz, CHLOROFORM-*d*) δ ppm 1.49 (d, *J*=7.32 Hz, 3 H) 3.50 (td, *J*=7.32 Hz, 5.60 1 H) 3.90 (s, 3 H) 5.35 (d, *J*=5.60 Hz, 1 H) 7.04 (m, 2 H) 7.10 (m, 1 H) 7.20 - 7.27 (m, 4 H) | 398 |

(continued)

| N° | STRUCTURE | Name | $^1$H-NMR | MS (M+H)$^+$ |
|---|---|---|---|---|
| 25 | 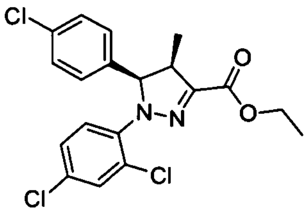 | *cis*-5-(4-Chloro-phenyl)-1-(2,4-dichloro-phenyl)-4-methyl-4,5-dihydro-1H-pyrazole-3-carboxylic acid ethyl ester | | 411 |
| 26 | 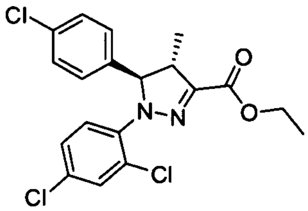 | trans-5-(4-Chloro-phenyl)-1-(2,4-dichloro-phenyl)-4-methyl-4,5-dihydro-1H-pyrazole-3-carboxylic acid ethyl ester | | 411 |
| 27 | 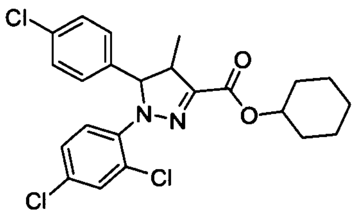 | 5-(4-Chloro-phenyl)-1-(2,4-dichloro-phenyl)-4-methyl-4,5-dihydro-1H-pyrazole-3-carboxylic acid cyclohe-xyl ester | | 465 |
| 28 | 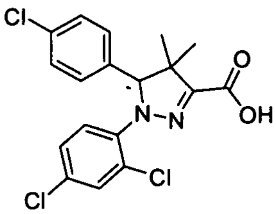 | 5-(4-Chloro-phenyl)-1-(2,4-dichloro-phenyl)-4,4-dimethyl-4,5-dihydro-1-H-pyrazole-3-carboxylic acid | | 397 |
| 29 | 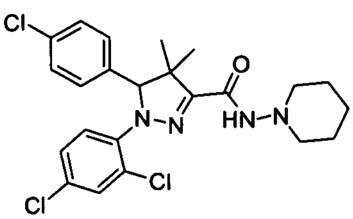 | 5-(4-Chloro-phenyl)-1-(2,4-dichloro-phenyl)-4,4-dimethyl-4,5-dihydro-1H-pyrazole-3-carboxylic acid piperi- | | 479 |
| 30 | 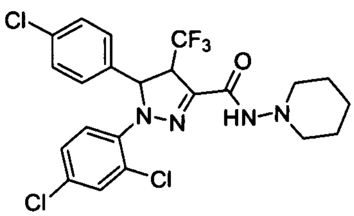 | 5-(4-Chloro-phenyl)-1-(2,4-dichloro-phenyl)-4-trifluoromethyl-4,5-dihydro-1H-pyrazole-3-carboxylic acid piperidin-1-ylamide | | 519 |

(continued)

| N° | STRUCTURE | Name | 1H-NMR | MS (M+H)+ |
|---|---|---|---|---|
| 31 | 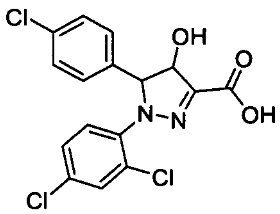 | 5-(4-Chloro-phenyl)-1-(2,4-dichloro-phenyl)-4-hydroxy-4,5- dihydro-1H-pyrazole-3-carboxylic acid | | 385 |
| 32 | 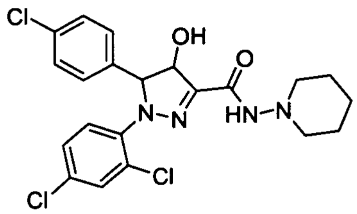 | 5-(4-Chloro-phenyl)-1-(2,4-dichloro-phenyl)-4-hydroxy-4,5- dihydro-1H-pyrazole-3-carboxylic acid piperidin-1-ylamide | | 467 |
| 33 | 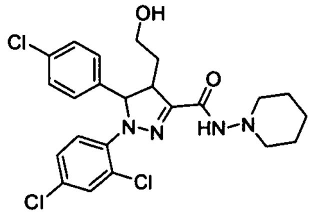 | 5-(4-Chloro-phenyl)-1-(2,4-dichloro-phenyl)-4-(2-hydroxy-ethyl)- 4,5-dihydro-1H-pyrazole-3-carboxylic acid piperidin-1- | | 495 |
| 34 | 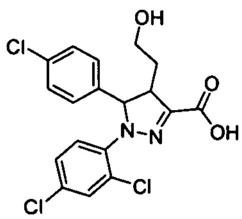 | 5-(4-Chloro-phenyl)-1-(2,4-dichloro-phenyl)-4-(2-hydroxy-ethyl)- 4,5-di-hydro-1H-pyrazole-3-carboxylic acid | | 413 |
| 35 | 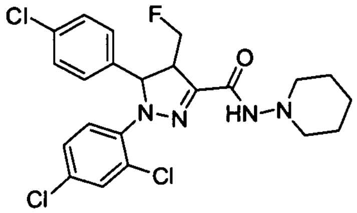 | 5-(4-Chloro-phenyl)-1-(2,4-dichloro-phenyl)-4-fluoromethyl-4,5-dihydro-1H-pyrazole-3-carboxylic acid piperidin-1-ylamide | | 483 |
| 36 | 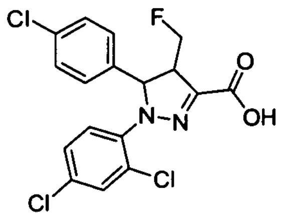 | 5-(4-Chloro-phenyl)-1-(2,4-dichloro-phenyl)-4-fluoromethyl-4,5-dihydro-1H-pyrazole-3-carboxylic acid | | 401 |

(continued)

| N° | STRUCTURE | Name | $^1$H-NMR | MS (M+H)$^+$ |
|---|---|---|---|---|
| 37 | 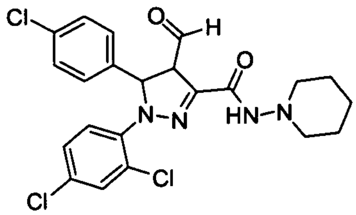 | 5-(4-Chloro-phenyl)-1-(2,4-dichloro-phenyl)-4-formyl-4,5-dihydro-1H-pyrazole-3-carboxylic acid piperidin-1-ylamide | | 479 |
| 38 | 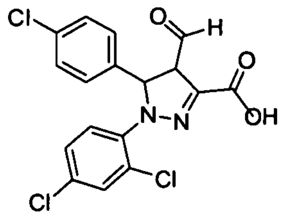 | 5-(4-Chloro-phenyl)-1-(2,4-dichloro-phenyl)-4-formyl-4,5-dihydro-1H-pyrazole-3-carboxylic acid | | 397 |
| 39 | 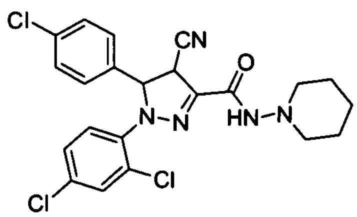 | 5-(4-chlorophenyl)-4-cyano-1-(2,4-dichlorophenyl)-N-(piperidin-1-yl)-4,5-dihydro-1H-pyrazole-3-carboxamide | | 476 |
| 40 | 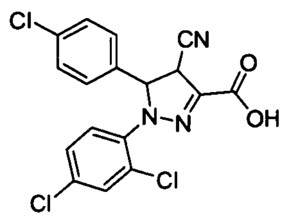 | 5-(4-Chloro-phenyl)-4-cyano-1-(2,4-dichloro-phenyl)-4,5-dihydro- 1H-pyrazole-3-carboxylic acid | | 394 |
| 41 | 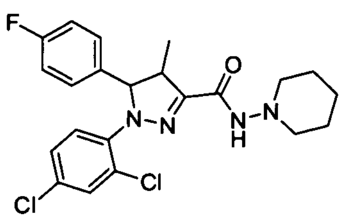 | 1-(2,4-Dichloro-phenyl)-5-(4-fluoro-phenyl)-4-methyl-4,5-dihydro-1H-pyrazole-3-carboxylic acid piperidin-1-ylamide | | 449 |
| 42 | 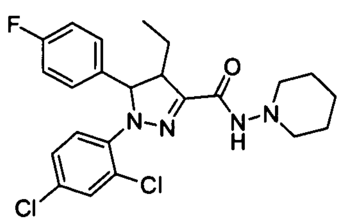 | 1-(2,4-Dichloro-phenyl)-4-ethyl-5-(4-fluoro-phenyl)-4,5-dihydro-1H-pyrazole-3-carboxylic acid piperidin-1-ylamide | | 463 |

(continued)

| N° | STRUCTURE | Name | $^1$H-NMR | MS $(M+H)^+$ |
|---|---|---|---|---|
| 43 | 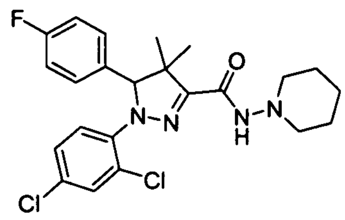 | 1-(2,4-Dichloro-phenyl)-5-(4-fluoro-phenyl)-4,4-dimethyl-4,5-dihydro-1H-pyrazole-3-carboxylic acid piperidin-1-ylamide | | 463 |
| 44 | 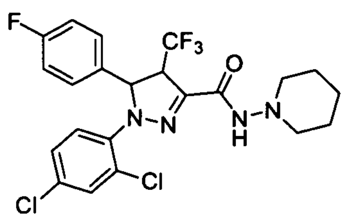 | 1-(2,4-Dichloro-phenyl)-5-(4-fluoro-phenyl)-4-trifluoromethyl-4,5-dihydro-1H-pyrazole-3-carboxylic acid piperidin-1-ylamide | | 503 |
| 45 | 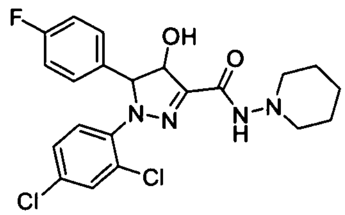 | 1-(2,4-Dichloro-phenyl)-5-(4-fluoro-phenyl)-4-hydroxy-4,5- dihydro-1H-pyrazole-3-carboxylic acid piperidin-1-ylamide | | 451 |
| 46 | 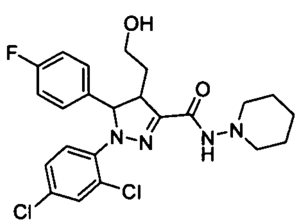 | 1-(2,4-Dichloro-phenyl)-5-(4-fluoro-phenyl)-4-(2-hydroxy-ethyl)- 4,5-di-hydro-1H-pyrazole-3-carboxylic acid | | 479 |
| 47 | 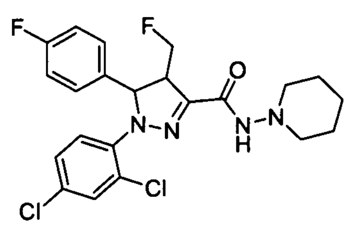 | 1-(2,4-Dichloro-phenyl)-4-fluoromethyl-5-(4-fluoro-phenyl)-4,5-dihydro-1H-pyrazole-3-carboxylic acid piperidin-1-ylamide | | 467 |
| 48 | 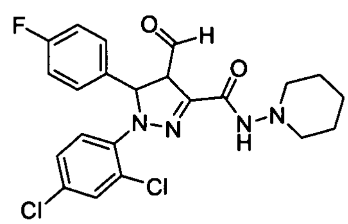 | 1-(2,4-Dichloro-phenyl)-5-(4-fluoro-phenyl)-4-formyl-4,5-dihydro-1 H-pyrazole-3-carboxylic acid piperidin-1-ylamide | | 463 |

(continued)

| N° | STRUCTURE | Name | $^1$H-NMR | MS (M+H)$^+$ |
|---|---|---|---|---|
| 49 | 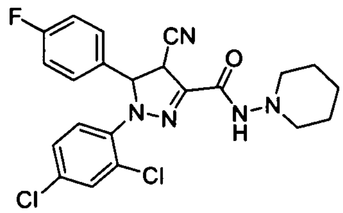 | 4-Cyano-1-(2,4-dichloro-phenyl)-5-(4-fluoro-phenyl)-4,5-dihydro-1H-pyrazole-3-carboxylic acid piperidin-1-ylamide | | 460 |
| 50 | 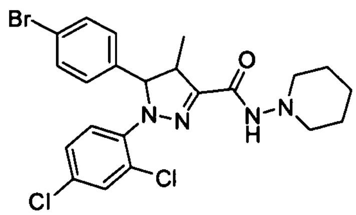 | 5-(4-Bromo-phenyl)-1-(2,4-dichloro-phenyl)-4-methyl-4,5-dihydro-1H-pyrazole-3-carboxylic acid piperidin-1-ylamide | | 509 |
| 51 | 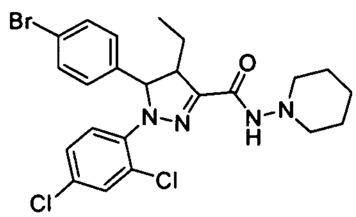 | 5-(4-Bromo-phenyl)-1-(2,4-dichloro-phenyl)-4-ethyl-4,5-dihydro-1H-pyrazole-3-carboxylic acid piperidin-1-ylamide | | 523 |
| 52 | 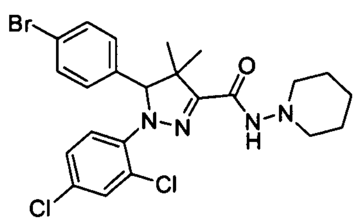 | 5-(4-Bromo-phenyl)-1-(2,4-dichloro-phenyl)-4,4-dimethyl-4,5-dihydro-1H-pyrazole-3-carboxylic acid piperidin-1-ylamide | | 523 |
| 53 | 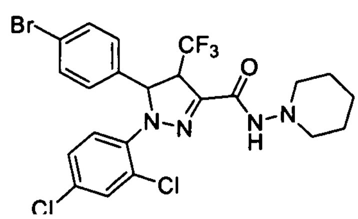 | 5-(4-Bromo-phenyl)-1-(2,4-dichloro-phenyl)-4-trifluoromethyl-4,5-dihydro-1H-pyrazole-3-carboxylic acid piperidin-1-ylamide | | 563 |
| 54 | 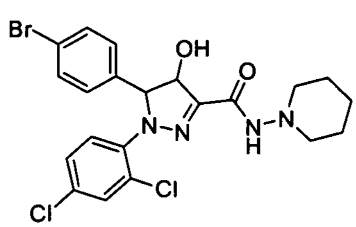 | 5-(4-Bromo-phenyl)-1-(2,4-dichloro-phenyl)-4-hydroxy-4,5- dihydro-1H-pyrazole-3-carboxylic acid piperidin-1-ylamide | | 511 |

(continued)

| N° | STRUCTURE | Name | $^1$H-NMR | MS (M+H)$^+$ |
|---|---|---|---|---|
| 55 | 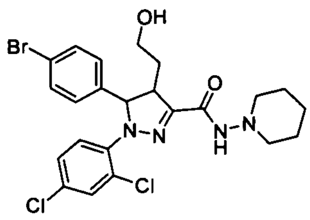 | 5-(4-Bromo-phenyl)-1-(2,4-dichloro-phenyl)-4-(2-hydroxy-ethyl)- 4,5-dihydro-1H-pyrazole-3-carboxylic acid piperidin-1-ylamide | | 539 |
| 56 | 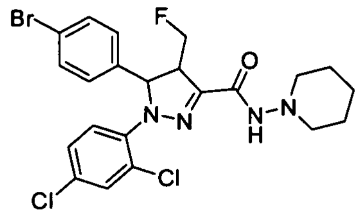 | 5-(4-Bromo-phenyl)-1-(2,4-dichloro-phenyl)-4-fluoromethyl-4,5-dihydro-1H-pyrazole-3-carboxylic acid piperidin-1-ylamide | | 527 |
| 57 | 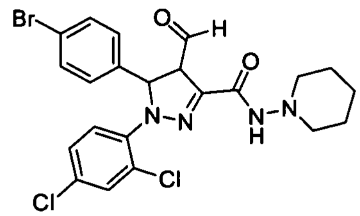 | 5-(4-Bromo-phenyl)-1-(2,4-dichloro-phenyl)-4-formyl-4,5-dihydro-1H-pyrazole-3-carboxylic acid piperidin-1-ylamide | | 523 |
| 58 | 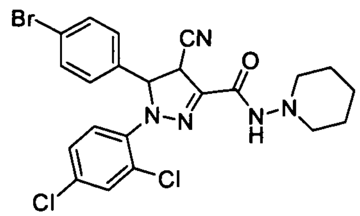 | 5-(4-Bromo-phenyl)-4-cyano-1-(2,4-dichloro-phenyl)-4,5-dihydro-1 H-pyrazole-3-carboxylic acid piperidin-1-ylamide | | 520 |
| 59 | 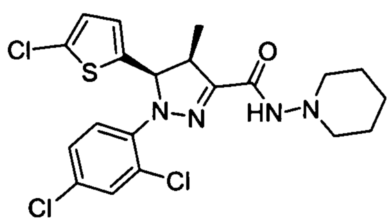 | *cis*-5-(5-Chloro-thiophen-2-yl)-1-(2,4-dichloro-phenyl)-4-methyl-4,5-dihydro-1H-pyrazole-3-carboxylic acid piperidin-1-ylamide | | 471 |
| 60 | 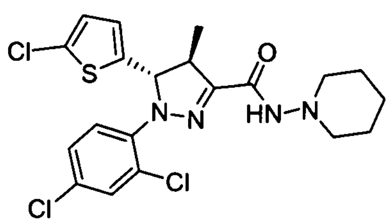 | *trans*-5-(5-Chloro-thiophen-2-yl)-1-(2,4-dichloro-phenyl)-4-methyl-4,5-dihydro-1H-pyrazole-3-carboxylic acid piperidin-1-ylamide | | 471 |

(continued)

| N° | STRUCTURE | Name | $^1$H-NMR | MS (M+H)$^+$ |
|---|---|---|---|---|
| 61 | 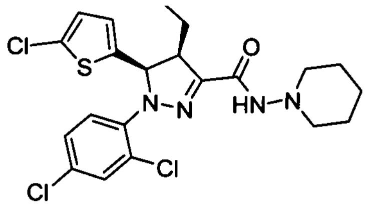 | *cis*-5-(5-Chloro-thiophen-2-yl)-1-(2,4-dichloro-phenyl)-4-ethyl-4,5-dihydro-1H-pyrazole-3-carboxylic acid piperidin-1-ylamide | | 485 |
| 62 | 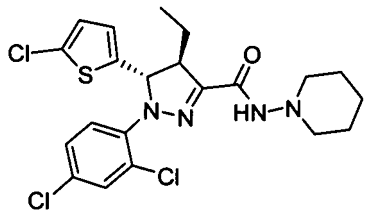 | trans-5-(5-Chloro-thiophen-2-yl)-1-(2,4-dichloro-phenyl)-4-ethyl-4,5-dihydro-1H-pyrazole-3-carboxylic acid piperidin-1-ylamide | | 485 |
| 63 | 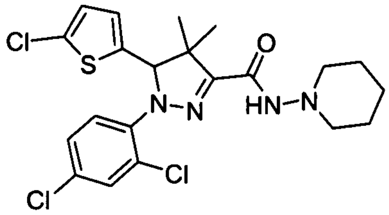 | 5-(5-Chloro-thiophen-2-yl)-1-(2,4-d ichloro-phenyl)-4,4-dimethyl- 4,5-di hydro-1H-pyrazole-3-carboxylic acid | | 485 |
| 64 | 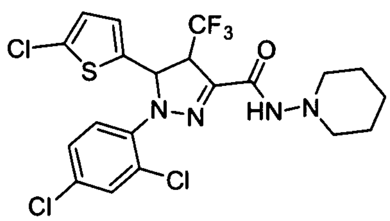 | 5-(5-Chloro-thiophen-2-yl)-1-(2,4-dichloro-phenyl)-4-trifluoromethyl- 4,5-dihydro-1H-pyrazole-3-carboxylic | | 525 |
| 65 | 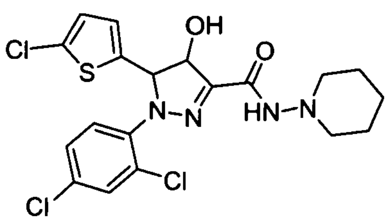 | 5-(5-Chloro-thiophen-2-yl)-1-(2,4-dichloro-phenyl)-4-hydroxy-4,5-dihydro-1H-pyrazole-3-carboxylic acid piperidin-1- | | 473 |
| 66 | 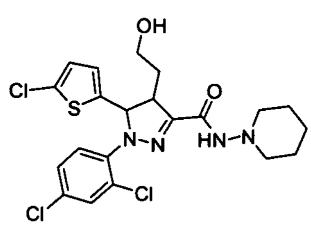 | 5-(5-Chloro-thiophen-2-yl)-1-(2,4-dichloro-phenyl)-4-(2-hydroxy-ethyl)-4,5-dihydro-1H-pyrazole-3-carboxylic acid piperidin-1- | | 501 |

(continued)

| N° | STRUCTURE | Name | 1H-NMR | MS (M+H)+ |
|---|---|---|---|---|
| 67 | 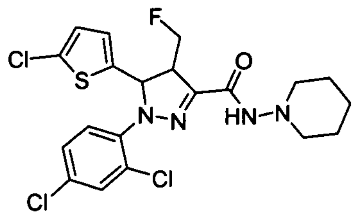 | 5-(5-Chloro-thiophen-2-yl)-1-(2,4-dichloro-phenyl)-4-fluoromethyl-4,5-dihydro-1H-pyrazole-3-carboxylic | | 489 |
| 68 | 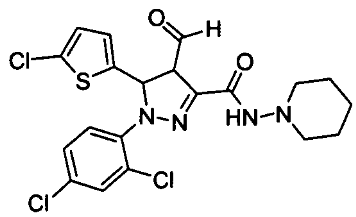 | 5-(5-Chloro-thiophen-2-yl)-1-(2,4-dichloro-phenyl)-4-formyl-4,5-dihydro-1H-pyrazole-3-carboxylic acid piperidin-1-ylamide | | 485 |
| 69 | 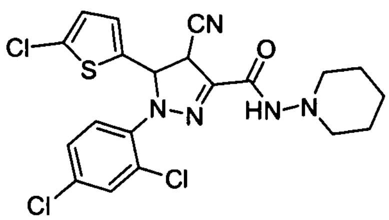 | 5-(5-Chloro-thiophen-2-yl)-4-cyano-1-(2,4-dichloro-phenyl)-4,5-dihydro-1H-pyrazole-3-carboxylic acid piperidin-1-ylamide | | 482 |
| 70 | 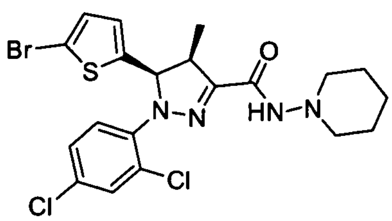 | *cis*-5-(5-Bromo-thiophen-2-yl)-1-(2,4-dichloro-phenyl)-4-methyl-4,5-dihydro-1H-pyrazole-3-carboxylic acid piperidin-1-ylamide | | 515 |
| 71 | 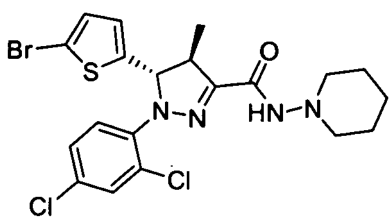 | *trans*-5-(5-Bromo-thiophen-2-yl)-1-(2,4-dichloro-phenyl)-4-methyl-4,5-dihydro-1H-pyrazole-3-carboxylic acid piperidin-1-ylamide | | 515 |
| 72 | 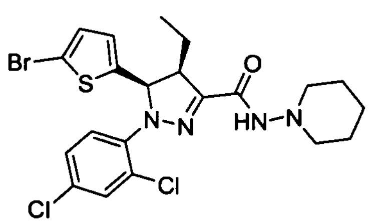 | *cis*-5-(5-Bromo-thiophen-2-yl)-1-(2,4-dichloro-phenyl)-4-ethyl-4,5-dihydro-1H-pyrazole-3-carboxylic acid piperidin-1-ylamide | | 529 |

(continued)

| N° | STRUCTURE | Name | $^1$H-NMR | MS (M+H)$^+$ |
|---|---|---|---|---|
| 73 | 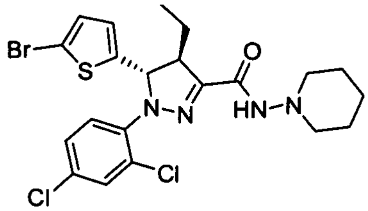 | *trans*-5-(5-Bromo-thiophen-2-yl)-1-(2,4-dichloro-phenyl)-4-ethyl-4,5-dihydro-1H-pyrazole-3-carboxylic acid piperidin-1-ylamide | | 529 |
| 74 | 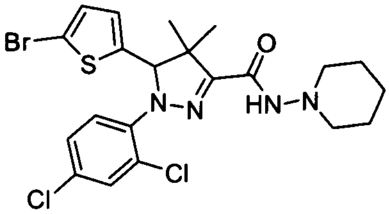 | 5-(5-Bromo-thiophen-2-yl)-1-(2,4-di-chloro-phenyl)-4,4-dimethyl- 4,5-di-hydro-1H-pyrazole-3-carboxylic acid | | 529 |
| 75 | 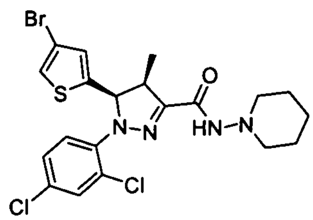 | *cis*-5-(4-Bromo-thiophen-2-yl)-1-(2,4-dichloro-phenyl)-4-methyl-4,5-dihydro-1H-pyrazole-3-carboxylic acid piperidin-1-ylamide | | 515 |
| 76 | 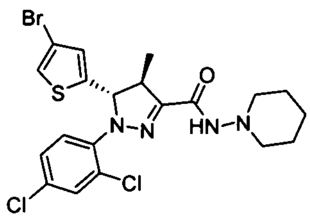 | *trans*-5-(4-Bromo-thiophen-2-yl)-1-(2,4-dichloro-phenyl)-4-methyl-4,5-dihydro-1H-pyrazole-3-carboxylic acid | | 515 |
| 77 | 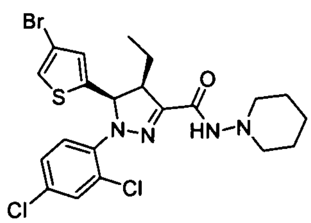 | *cis*-5-(4-Bromo-thiophen-2-yl)-1-(2,4-dichloro-phenyl)-4-ethyl-4,5-dihydro-1H-pyrazole-3-carboxylic acid piperidin-1-ylamide | | 529 |
| 78 | 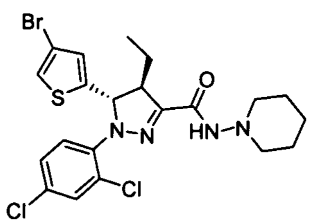 | *trans*-5-(4-Bromo-thiophen-2-yl)-1-(2,4-dichloro-phenyl)-4-ethyl-4,5-dihydro-1H-pyrazole-3-carboxylic acid piperidin-1-ylamide | | 529 |

(continued)

| N° | STRUCTURE | Name | $^1$H-NMR | MS (M+H)$^+$ |
|---|---|---|---|---|
| 79 | 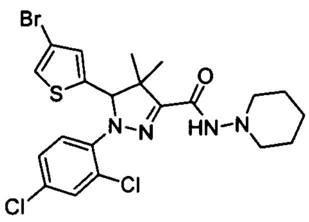 | 5-(4-Bromo-thiophen-2-yl)-1-(2,4-di chloro-phenyl)-4,4-dimethyl- 4,5-dihydro-1H-pyrazole-3-carboxylic acid | | 529 |
| 80 | 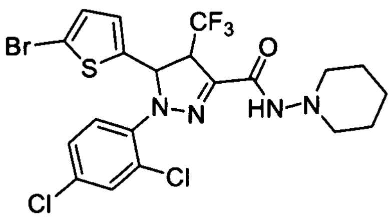 | 5-(5-Bromo-thiophen-2-yl)-1-(2,4-dichloro-phenyl)-4-trifluoromethyl- 4,5-dihydro-1H-pyrazole-3-carboxylic acid piperidin-1-ylamide | | 569 |
| 81 | 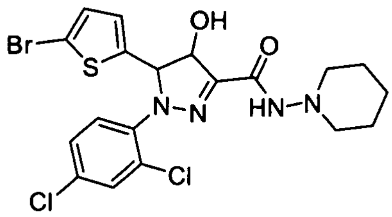 | 5-(5-Bromo-thiophen-2-yl)-1-(2,4-di-chloro-phenyl)-4-hydroxy-4,5- dihydro-1H-pyrazole-3-carboxylic acid piperidin-1-ylamide | | 517 |
| 82 | 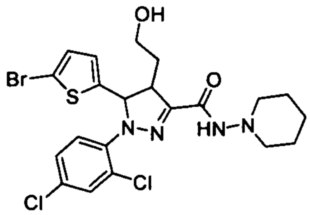 | 5-(5-Bromo-thiophen-2-yl)-1-(2,4-di-chloro-phenyl)-4-(2-hydroxy- ethyl)-4,5-dihydro-1H-pyrazole-3-carboxylic acid piperidin-1- | | 545 |
| 83 | 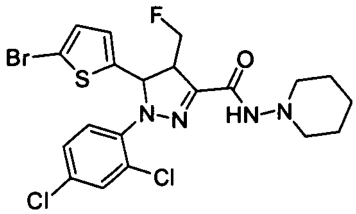 | 5-(5-Bromo-thiophen-2-yl)-1-(2,4-di-chloro-phenyl)-4-fluoromethyl-4,5-dihydro-1H-pyrazole-3-carboxylic acid piperidin-1- | | 533 |
| 84 | 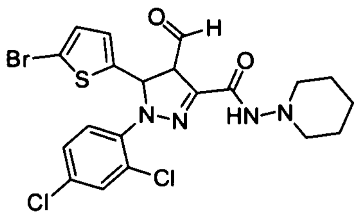 | 5-(5-Bromo-thiophen-2-yl)-1-(2,4-di-chloro-phenyl)-4-formyl-4,5-dihydro-1H-pyrazole-3-carboxylic acid piperidin-1-ylamide | | 529 |

(continued)

| N° | STRUCTURE | Name | 1H-NMR | MS (M+H)+ |
|---|---|---|---|---|
| 85 | 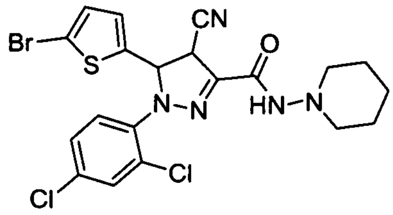 | 5-(5-Bromo-thiophen-2-yl)-4-cyano-1-(2,4-dichloro-phenyl)-4,5-dihydro- 1H-pyrazole-3-carboxylic acid piperidin-1-ylamide | | 526 |
| 86 | 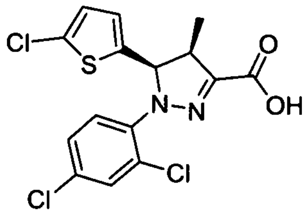 | cis-5-(5-Chloro-thiophen-2-yl)-1-(2,4-dichloro-phenyl)-4- methyl-4,5-dihydro-1H-pyrazole-3-carboxylic acid | | 389 |
| 87 | 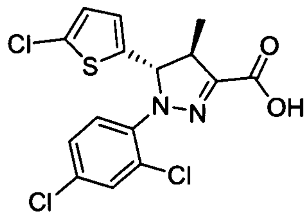 | *trans*-[5-(5-Chloro-thiophen-2-yl)-1-(2,4-dichloro-phenyl)-4-methyl-4,5-dihydro-1H-pyrazol-3-yl]-piperidin-1-yl-methanone | | 389 |
| 88 | 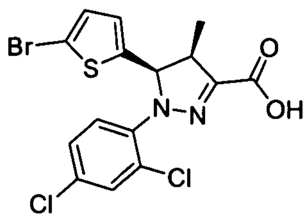 | cis-5-(5-Bromo-thiophen-2-yl)-1-(2,4-dichloro-phenyl)-4-methyl-4,5-dihydro-1H-pyrazole-3-carboxylic acid | | 433 |
| 89 | 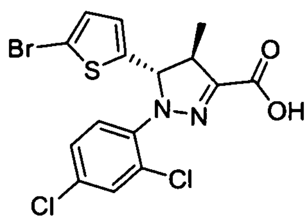 | *trans*-5-(5-Bromo-thiophen-2-yl)-1-(2,4-dichloro-phenyl)-4- methyl-4,5-dihydro-1 H-pyrazole-3-carboxylic acid | | 433 |
| 90 | 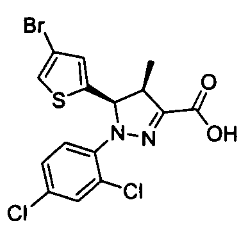 | *cis*-5-(4-Bromo-thiophen-2-yl)-1-(2,4-dichloro-phenyl)-4- methyl-4,5-dihydro-1 H-pyrazole-3-carboxylic acid | | 433 |

(continued)

| N° | STRUCTURE | Name | 1H-NMR | MS (M+H)+ |
|---|---|---|---|---|
| 91 | 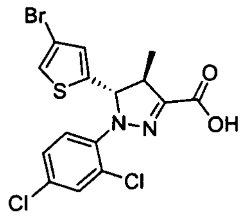 | *trans*-5-(4-Bromo-thiophen-2-yl)-1-(2,4-dichloro-phenyl)-4- methyl-4,5-dihydro-1 H-pyrazole-3-carboxylic acid | | 433 |
| 446 | 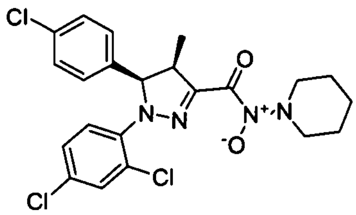 | *cis*-5-(4-chlorophenyl)-1-(2,4-dichlorophenyl)-4-methyl-N-(piperidin-1-yl)-4,5-dihydro-1H-pyrazole-3-carboxamide N-oxide | | 480 |
| 447 | 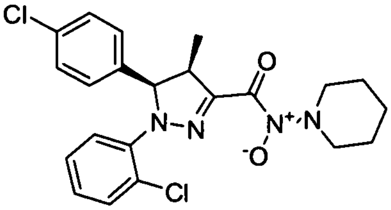 | *cis*-5-(4-chlorophenyl)-1-(2-chlorophenyl)-4-methyl-N-(pipeddin-1-yl)-4,5-dihydro-1H-pyrazole-3-carboxamide N-oxide | | 446 |
| 448 | 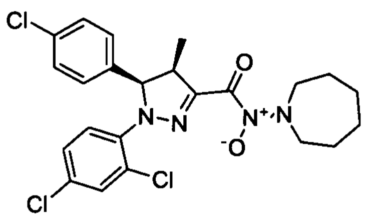 | *cis*-N-(azepan-1-yl)-5-(4-chlorophenyl)-1-(2,4-dichlorophenyl)-4-methyl-4,5-dihydro-1H-pyrazole-3- carboxamide N-oxide | | 494 |
| 449 | 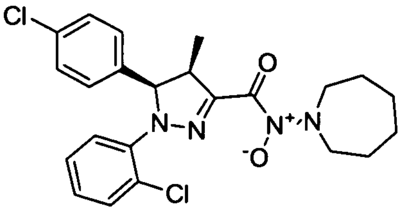 | *cis*-N-(azepan-1-yl)-5-(4-chlorophenyl)-1-(2-chlorophenyl)-4-methyl-4,5-dihydro-1H-pyrazole-3-carboxamide N-oxide | | 460 |
| 450 | 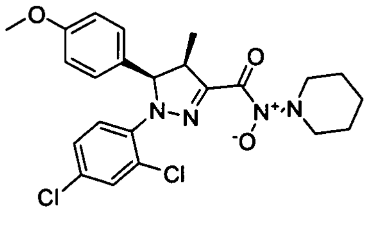 | *cis*-1-(2,4-dichlorophenyl)-5-(4-methoxyphenyl)-4-methyl-N-(piperidin-1-yl)-4,5-dihydro-1H-pyrazole-3-carboxamide N-oxide | | 476 |

(continued)

| N° | STRUCTURE | Name | $^1$H-NMR | MS (M+H)$^+$ |
|---|---|---|---|---|
| 451 | 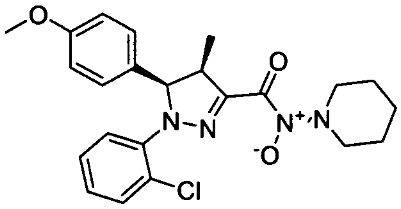 | *cis*-1-(2-chlorophenyl)-5-(4-methoxyphenyl)-4-methyl-N-(piperidin-1-yl)-4,5-dihydro-1 H-pyrazole-3-carboxamide N-oxide | | 442 |

| | |
|---|---|
| 92 | (+)-cis-5-(4-chlorophenyl)-1-(2,4-dichlorophenyl)-4-methyl-N-(piperidin-1-yl)-4,5-dihydro-1H-pyrazole-3-carboxamide hydrochloride |
| 93 | (-)-cis-5-(4-chlorophenyl)-1-(2,4-dichlorophenyl)-4-methyl-N-(piperidin-1 -yl)-4,5-dihydro-1H-pyrazole-3-carboxamide hydrochloride |
| 94 | *cis*-1-(2-chlorophenyl)-5-(4-chlorophenyl)-4-methyl-N-(piperidin-1-yl)-4,5-dihydro-1H-pyrazole-3-carboxamide |
| 95 | *trans*-1-(2-chlorophenyl)-5-(4-chlorophenyl)-4-methyl-N-(piperidin-1-yl)-4,5-dihydro-1H-pyrazole-3-carboxamide |
| 96 | *cis*-5-(4-Chlorophenyl)-1-(2,4-dichlorophenyl)-4-methyl-4,5-dihydro-1H-pyrazole-3-carboxylic acid azepan-1-ylamide hydrochloride |
| 97 | *trans*-5-(4-Chlorophenyl)-1-(2,4-dichloro-phenyl)-4-methyl-4,5-dihydro-1H-pyrazole-3-carboxylic acid azepan-1-ylamide hydrochloride |
| 98 | *cis*-N-(azepan-1-yl)-1-(2-chlorophenyl)-5-(4-chlorophenyl)-4-methyl-4,5-dihydro-1H-pyrazole-3-carboxamide |
| 99 | *trans*-N-(azepan-1-yl)-1-(2-chlorophenyl)-5-(4-chlorophenyl)-4-methyl-4,5-dihydro-1H-pyrazole-3-carboxamide |
| 100 | *cis*-5-(4-chlorophenyl)-N-(4-cyclopentylpiperazin-1-yl)-1-(2,4-dichlorophenyl)-4-methyl-4,5-dihydro-1H-pyrazole-3-carboxamide hydrochloride |
| 101 | *cis*-5-(4-chlorophenyl)-1-(2,4-dichlorophenyl)-4-methyl-N-morpholino-4,5-dihydro-1H-pyrazole-3-carboxamide |
| 102 | *cis*-1-(2-chlorophenyl)-5-(4-chlorophenyl)-4-methyl-N-(4-methylpiperidin-1-yl)-4,5-dihydro-1H-pyrazole-3-carboxamide |
| 103 | *cis*-5-(4-chlorophenyl)-1-(2,4-dichlorophenyl)-N-(2,6-dimethylpiperidin-1-yl)-4-methyl-4,5-dihydro-1H-pyrazole-3-carboxamide |
| 104 | *cis*-5-(4-Chlorophenyl)-1-(2,4-dichlorophenyl)-4-methyl-4,5-dihydro-1H-pyrazole-3-carboxylic acid (1,3-dioxo-1 H,3H-benzo[de]isoquinolin-2-yl)-amide |
| 105 | *cis*-5-(4-Chlorophenyl)-1-(2,4-dichlorophenyl)-4-methyl-4,5-dihydro-1H-pyrazole-3-carboxylic acid (1H,3H-benzo[de]isoquinolin-2-yl)-amide |
| 106 | *cis*-5-(4-chlorophenyl)-1 -(2,4-dichlorophenyl)-N-(®-2-(methoxymethyl)pyrrolidin-1-yl)-4-methyl-4.5-dihydro-1H-pyrazole-3-carboxamide hydrochloride |
| 107 | *cis*-5-(4-chlorophenyl)-1-(2,4-dichlorophenyl)-N-((S)-2-(methoxymethyl)pyrrolidin-1-yl)-4-methyl-4,5-dihydro-1H-pyrazole-3-carboxamide hydrochloride |
| 108 | *cis*-5-(4-Chlorophenyl)-1-(2,4-dichlorophenyl)-4-methyl-4,5-dihydro-1H-pyrazole-3-carboxylic acid (hexahydro-cyclopenta[c]pyrrol-2-yl)-amide hydrochloride |
| 109 | *cis*-1-(2-chlorophenyl)-5-(4-chlorophenyl)-N-(hexahydrocyclopenta[c]pyrrol-2(1H)-yl)-4-methyl-4,5-dihydro-1H-pyrazole-3-carboxamide |

(continued)

| | |
|---|---|
| 110 | *trans*-1-(2-chlorophenyl)-5-(4-chlorophenyl)-N-(hexahydrocyclopenta[c]pyrrol-2(1H)-yl)-4-methyl-4,5-dihydro-1H-pyrazole-3-carboxamide |
| 111 | *cis*-1-(2-chlorophenyl)-5-(4-chlorophenyl)-N-(indolin-1-yl)-4-methyl-4,5-dihydro-1H-pyrazole-3-carboxamide |
| 112 | *trans*-1-(2-chlorophenyl)-5-(4-chlorophenyl)-N-(indolin-1-yl)-4-methyl-4,5-dihydro-1H-pyrazole-3-carboxamide |
| 113 | *cis*-5-(4-chlorophenyl)-1-(2,4-dichlorophenyl)-4-methyl-N-(2-methylindolin-1-yl)-4,5-dihydro-1H-pyrazole-3-carboxamide |
| 114 | *cis*-5-(4-chlorophenyl)-N-(cyclohexylmethyl)-1-(2,4-dichlorophenyl)-4-methyl-4,5-dihydro-1H-pyrazole-3-carboxamide |
| 115 | *cis*-5-(4-chlorophenyl)-N-(cycloheptylmethyl)-1-(2,4-dichlorophenyl)-4-methyl-4,5-dihydro-1H-pyrazole-3-carboxamide |
| 116 | *cis*-5-(4-chlorophenyl)-N-cyclododecyl-1-(2,4-dichlorophenyl)-4-methyl-4,5-dihydro-1 H-pyrazole-3-carboxamide |
| 117 | *cis*-5-(4-chlorophenyl)-1-(2,4-dichlorophenyl)-4-methyl-N-(1,2,3,4-tetrahydronaphthalen-1-yl)-4,5-dihydro-1 H-pyrazole-3-carboxamide |
| 118 | *cis*-N-(4-tert-butylcyclohexyl)-5-(4-chlorophenyl)-1-(2,4-dichlorophenyl)-4-methyl-4,5-dihydro-1 H-pyrazole-3-carboxamide |
| 119 | cis-5-(4-chlorophenyl)-N-cyclooctyl-1-(2,4-dichlorophenyl)-4-methyl-4,5-dihydro-1 H-pyrazole-3-carboxamide |
| 120 | cis-N-(azocan-1-yl)-5-(4-chlorophenyl)-1-(2,4-dichlorophenyl)-4-methyl-4,5-dihydro-1 H-pyrazole-3-carboxamide |
| 121 | *cis*-N-(azocan-1-yl)-1-(2-chlorophenyl)-5-(4-chlorophenyl)-4-methyl-4,5-dihydro-1H-pyrazole-3-carboxamide |
| 122 | *cis*-azocan-1-yl(cis-5-(4-chlorophenyl)-1-(2,4-dichlorophenyl)-4-methyl-4,5-dihydro-1H-pyrazol-3-yl)metha none |
| 123 | *cis*-5-(4-chlorophenyl)-N-cycloheptyl-1-(2,4-dichlorophenyl)-4-methyl-4,5-dihydro-1 H-pyrazole-3-carboxamide |
| 124 | *cis*-1-(2-chlorophenyl)-5-(4-chlorophenyl)-N-cycloheptyl-4-methyl-4,5-dihydro-1 H-pyrazole-3-carboxamide |
| 125 | *trans*-5-(4-chlorophenyl)-N-cycloheptyl-1-(2,4-dichlorophenyl)-4-methyl-4,5-dihydro-1H-pyrazole-3-carboxamide |
| 126 | *trans*-1-(2-chlorophenyl)-5-(4-chlorophenyl)-N-cycloheptyl-4-methyl-4,5-dihydro-1H-pyrazole-3-carboxamide |
| 127 | *cis*-5-(4-chlorophenyl)-N-(cyclohexylmethyl)-1-(2,4-dichlorophenyl)-4-methyl-4,5-dihydro-1H-pyrazole-3-carboxamide |
| 128 | (+)-cis-5-(4-chlorophenyl)-1-(2,4-dichlorophenyl)-N-((1S,2S)-2-hydroxycyclohexyl)-4-methyl-4,5-dihydro-1H-pyrazole-3-carboxamide |
| 129 | (-)-cis-5-(4-chlorophenyl)-1-(2,4-dichlorophenyl)-N-((1S,2S)-2-hydroxycyclohexyl)-4-methyl-4,5-dihydro-1H-pyrazole-3-carboxamide |
| 130 | (-)-cis-5-(4-chlorophenyl)-1-(2,4-dichlorophenyl)-N-((1R,2R)-2-hydroxycyclohexyl)-4-methyl-4,5-dihydro-1H-pyrazole-3-carboxamide |
| 131 | (+)-cis-5-(4-chlorophenyl)-1-(2,4-dichlorophenyl)-N-((1R,2R)-2-hydroxycyclohexyl)-4-methyl-4,5-dihydro-1H-pyrazole-3-carboxamide |
| 132 | (+)-cis-1-(2-chlorophenyl)-5-(4-chlorophenyl)-N-((1S,2S)-2-hydroxycyclohexyl)-4-methyl-4,5-dihydro-1H-pyrazole-3-carboxamide |

(continued)

| | |
|---|---|
| 133 | (-)-cis-1-(2-chlorophenyl)-5-(4-chlorophenyl)-N-((1S,2S)-2-hydroxycyclohexyl)-4-methyl-4,5-dihydro-1H-pyrazole-3-carboxamide |
| 134 | *cis*-5-(4-chlorophenyl)-1-(2,4-dichlorophenyl)-N-(4-hydroxypiperidin-1 -yl)-4-methyl-4,5-dihydro-1H-pyrazole-3-carboxamide |
| 135 | *cis*-1-(2-chlorophenyl)-5-(4-chlorophenyl)-N-(4-hydroxypiperidin-1-yl)-4-methyl-4,5-dihydro-1H-pyrazole-3-carboxamide |
| 136 | *cis*-5-(4-chlorophenyl)-1-(2,4-dichlorophenyl)-N-(3-hydroxypiperidin-1-yl)-4-methyl-4,5-dihydro-1H-pyrazole-3-carboxamide |
| 137 | *cis*-1-(2-chlorophenyl)-5-(4-chlorophenyl)-N-(3-hydroxypiperidin-1-yl)-4-methyl-4,5-dihydro-1H-pyrazole-3-carboxamide |
| 138 | *cis*-5-(4-chlorophenyl)-1-(2,4-dichlorophenyl)-N-(2-hydroxypiperidin-1-yl)-4-methyl-4,5-dihydro-1H-pyrazole-3-carboxamide |
| 139 | *cis*-1-(2-chlorophenyl)-5-(4-chlorophenyl)-N-(2-hydroxypiperidin-1-yl)-4-methyl-4,5-dihydro-1H-pyrazole-3-carboxamide |
| 140 | *cis*-5-(4-chlorophenyl)-1-(2,4-dichlorophenyl)-4-methyl-N-(4-oxopiperidin-1-yl)-4,5-dihydro-1H-pyrazole-3-carboxamide |
| 141 | *cis*-1-(2-chlorophenyl)-5-(4-chlorophenyl)-4-methyl-N-(4-oxopiperidin-1-yl)-4,5-dihydro-1H-pyrazole-3-carboxamide |
| 142 | *cis*-5-(4-chlorophenyl)-1-(2,4-dichlorophenyl)-4-methyl-N-(3-oxopiperidin-1-yl)-4,5-dihydro-1H-pyrazole-3-carboxamide |
| 143 | *cis*-1-(2-chlorophenyl)-5-(4-chlorophenyl)-4-methyl-N-(3-oxopiperidin-1-yl)-4,5-dihydro-1H-pyrazole-3-carboxamide |
| 144 | *cis*-5-(4-chlorophenyl)-1-(2,4-dichlorophenyl)-N-(3,4-dihydropyridin-1(2H)-yl)-4-methyl-4,5-dihydro-1H-pyrazole-3-carboxamide |
| 145 | *cis*-1-(2-chlorophenyl)-5-(4-chlorophenyl)-N-(3,4-dihydropyridin-1(2H)-yl)-4-methyl-4,5-dihydro-1H-pyrazole-3-carboxamide |
| 146 | *cis*-5-(4-chlorophenyl)-1-(2,4-dichlorophenyl)-N-(5,6-dihydropyridin-1(2H)-yl)-4-methyl-4,5-dihydro-1H-pyrazole-3-carboxamide |
| 147 | *cis*-1-(2-chlorophenyl)-5-(4-chlorophenyl)-N-(5,6-dihydropyridin-1(2H)-yl)-4-methyl-4,5-dihydro-1H-pyrazole-3-carboxamide |
| 148 | *cis*-1-(2-chlorophenyl)-5-(4-chlorophenyl)-4-methyl-4,5-dihydro-1H-pyrazole-3-carboxylic acid |
| 149 | *trans*-1-(2-chlorophenyl)-5-(4-chlorophenyl)-4-methyl-4,5-dihydro-1H-pyrazole-3-carboxylic acid |
| 150 | *cis*-5-(4-Chlorophenyl)-1-(2,4-dichlorophenyl)-4-methyl-4,5-dihydro-1H-py-razole-3-carboxylic acid cyclohexyl ester |
| 151 | N-(azepan-1-yl)-5-(4-chlorophenyl)-1-(2,4-dichlorophenyl)-4,4-dimethyl-4,5-dihydro-1H-pyrazole-3-carboxamide |
| 152 | 1-(2-chlorophenyl)-5-(4-chlorophenyl)-4,4-dimethyl-N-(piperidin-1-yl)-4,5-dihydro-1H-pyrazole-3-carboxamide |
| 153 | N-(azepan-1-yl)-1-(2-chlorophenyl)-5-(4-chlorophenyl)-4,4-dimethyl-4,5-dihydro-1H-pyrazole-3-carboxamide |
| 154 | *cis*-5-(4-Chlorophenyl)-1-(2,4-dichlorophenyl)-4-trifluoromethyl-4,5-dihydro-1H-pyrazole-3-carboxylic acid piperidin-1-ylamide |
| 155 | *trans*-5-(4-Chloro-phenyl)-1-(2,4-dichlorophenyl)-4-trifluoromethyl-4,5-dihydro-1H-pyrazole-3-carboxylic acid piperidin-1-ylamide |

(continued)

| | |
|---|---|
| 156 | *cis*-N-(azepan-1-yl)-5-(4-chlorophenyl)-1-(2,4-dichlorophenyl)-4-(trifluoromethyl)-4,5-dihydro-1H-pyrazole-3-carboxamide |
| 157 | *trans*-N-(azepan-1-yl)-5-(4-chlorophenyl)-1-(2,4-dichlorophenyl)-4-(trifluoromethyl)-4,5-dihydro-1H-pyrazole-3-carboxamide |
| 158 | *cis*-1-(2-chlorophenyl)-5-(4-chlorophenyl)-N-(piperidin-1-yl)-4-(trifluoromethyl)-4,5-dihydro-1H-pyrazole-3-carboxamide |
| 159 | *trans* 1-(2-chloropheny)-5-(4-chloropheny)-N-(piperidin-1-y)-4-(trifluoromethy)-4.5-dihydro-1H-pyrazole-3-carboxamide |
| 160 | 5-(4-chlorophenyl)-1-(2,4-dichlorophenyl)-3-(piperidin-1-ylcarbamoyl)-4,5-dihydro-1H-pyrazole-4-carboxylic acid |
| 161 | 5-(4-chlorophenyl)-1-(2,4-dichlorophenyl)-4,4-difluoro-N-(piperidin-1-yl)-4,5 -dihydro-1 H-pyrazole-3-carboxamide |
| 162 | *cis*-5-(4-chlorophenyl)-4-cyano-1-(2,4-dichlorophenyl)-N-(piperidin-1-yl)-4,5-dihydro-1H-pyrazole-3-carboxamide |
| 163 | *cis*-N-(azepan-1-yl)-5-(4-chlorophenyl)-4-cyano-1-(2,4-dichlorophenyl)-4,5-dihydro-1H-pyrazole-3-carboxamide |
| 164 | *cis*-1-(2-chlorophenyl)-5-(4-chlorophenyl)-4-cyano-N-(piperidin-1-yl)-4,5-dihydro-1H-pyrazole-3-carboxamide |
| 165 | *cis*-N-(azepan-1-yl)-1-(2-chlorophenyl)-5-(4-chlorophenyl)-4-cyano-4,5-dihydro-1H-pyrazole-3-carboxamide |
| 166 | *trans*-5-(4-chlorophenyl)-4-cyano-1-(2,4-dichlorophenyl)-N-(piperidin-1-yl)-4,5-dihydro-1H-pyrazole-3-carboxamide |
| 167 | *trans*-N-(azepan-1-yl)-5-(4-chlorophenyl)-4-cyano-1-(2,4-dichlorophenyl)-4,5-dihydro-1H-pyrazole-3-carboxamide |
| 168 | *trans*-1-(2-chlorophenyl)-5-(4-chlorophenyl)-4-cyano-N-(piperidin-1-yl)-4,5-dihydro-1H-pyrazole-3-carboxamide |
| 169 | *cis*-1-(2-chlorophenyl)-5-(4-chlorophenyl)-4-ethyl-4,5-dihydro-1H-pyrazole-3-carboxylic acid |
| 170 | *trans*-1-(2-chlorophenyl)-5-(4-chlorophenyl)-4-ethyl-4,5-dihydro-1H-pyrazole-3-carboxylic acid |
| 171 | *cis*-5-(4-chlorophenyl)-1-(2,4-dichlorophenyl)-4-ethyl-N-(piperidin-1-yl)-4,5-dihydro-1H-pyrazole-3-carboxamide |
| 172 | *trans*-5-(4-chlorophenyl)-1-(2,4-dichlorophenyl)-4-ethyl-N-(piperidin-1-yl)-4,5-dihydro-1H-pyrazole-3-carboxamide |
| 173 | *cis*-1-(2-chlorophenyl)-5-(4-chlorophenyl)-4-ethyl-N-(piperidin-1-yl)-4,5-dihydro-1H-pyrazole-3-carboxamide |
| 174 | *trans*-1-(2-chlorophenyl)-5-(4-chlorophenyl)-4-ethyl-N-(piperidin-1-yl)-4,5-dihydro-1H-pyrazole-3-carboxamide |
| 175 | *cis* -N-(azepan-1-yl)-5-(4-chlorophenyl)-1-(2,4-dichlorophenyl)-4-ethyl-4,5-dihydro-1H-pyrazole-3-carboxamide |
| 176 | *trans*-N-(azepan-1-yl)-5-(4-chlorophenyl)-1-(2,4-dichlorophenyl)-4-ethyl-4,5-dihydro-1H-pyrazole-3-carboxamide |
| 177 | *cis*-N-(azepan-1-yl)-1-(2-chlorophenyl)-5-(4-chlorophenyl)-4-ethyl-4,5-dihydro-1H-pyrazole-3-carboxamide |
| 178 | *trans*-N-(azepan-1-yl)-1-(2-chlorophenyl)-5-(4-chlorophenyl)-4-ethyl-4,5-dihydro-1H-pyrazole-3-carboxamide |
| 179 | *cis*-5-(4-chlorophenyl)-N-cycloheptyl-1-(2,4-dichlorophenyl)-4-ethyl-4,5-dihydro-1H-pyrazole-3-carboxamide |

(continued)

| | |
|---|---|
| 180 | *cis*-1-(2-chlorophenyl)-5-(4-chlorophenyl)-N-cycloheptyl-4-ethyl-4,5-dihydro-1H-pyrazole-3-carboxamide |
| 181 | *cis*-5-(4-chlorophenyl)-1-(2,4-dichlorophenyl)-4-ethyl-N-(hexahydrocyclopenta[c]pyrrol-2(1H)-yl)-4,5-dihydro-1H-pyrazole-3-carboxamide |
| 182 | *cis*-1-(2-chlorophenyl)-5-(4-chlorophenyl)-4-ethyl-N-(hexahydrocyclopenta[c]pyrrol-2(1H)-yl)-4,5-dihydro-1H-pyrazole-3-carboxamide |
| 183 | *cis*-5-(4-chlorophenyl)-1-(2,4-dichlorophenyl)-4-ethyl-N-(indolin-1-yl)-4,5-dihydro-1 H-pyrazole-3-carboxamide |
| 184 | *cis*-1-(2-chlorophenyl)-5-(4-chlorophenyl)-4-ethyl-N-(indolin-1-yl)-4,5-dihydro-1H-pyrazole-3-carboxamide |
| 185 | *cis*-5-(4-bromophenyl)-1-(2,4-dichlorophenyl)-4-ethyl-4,5-dihydro-1H-pyrazole-3-carboxylic acid |
| 186 | *trans*-5-(4-bromophenyl)-1-(2,4-dichlorophenyl)-4-ethyl-4,5-dihydro-1H-pyrazole-3-carboxylic acid |
| 187 | *cis*-5-(4-bromophenyl)-1-(2,4-dichlorophenyl)-4-ethyl-N-(piperidin-1-yl)-4,5-dihydro-1 H-pyrazole-3-carboxamide |
| 188 | *trans*-5-(4-bromophenyl)-1-(2,4-dichlorophenyl)-4-ethyl-N-(piperidin-1-yl)-4,5-dihydro-1 H-pyrazole-3-carboxamide |
| 189 | *cis*-5-(4-bromophenyl)-1-(2-chlorophenyl)-4-ethyl-N-(piperidin-1-yl)-4,5-dihydro-1 H-pyrazole-3-carboxamide |
| 190 | *trans*-5-(4-bromophenyl)-1-(2-chlorophenyl)-4-ethyl-N-(pipeddin-1-yl)-4,5-dihydro-1 H-pyrazole-3-carboxamide |
| 191 | *cis*-N-(azepan-1-yl)-5-(4-bromophenyl)-1-(2,4-dich)orophenyl)-4-ethyl-4,5-dihydro-1 H-pyrazole-3-carboxamide |
| 192 | *cis*-N-(azepan-1-yl)-5-(4-bromophenyl)-1-(2-chlorophenyl)-4-ethyl-4,5-dihydro-1H-pyrazole-3-carboxamide |
| 193 | *cis*-5-(4-bromophenyl)-N-cycloheptyl-1-(2,4-dichlorophenyl)-4-ethyl-4,5-dihydro-1H-pyrazole-3-carboxamide |
| 194 | *cis*-5-(4-bromophenyl)-1-(2-chlorophenyl)-N-cycloheptyl-4-ethyl-4,5-dihydro-1H-pyrazole-3-carboxamide |
| 195 | *cis*-5-(4-bromophenyl)-1-(2,4-dichlorophenyl)-4-ethyl-N-(hexahydrocyclopenta[c]pyrrol-2(1H)-yl)-4,5-dihydro-1H-pyrazole-3-carboxamide |
| 196 | *cis*-5-(4-bromophenyl)-1-(2-chlorophenyl)-4-ethyl-N-(hexahydrocyclopenta[c]pyrrol-2(1H)-yl)-4,5-dihydro-1H-pyrazole-3-carboxamide |
| 197 | *cis*-5-(4-bromophenyl)-1-(2,4-dichlorophenyl)-4-ethyl-N-(indolin-1-yl)-4,5-dihydro-1H-pyrazole-3-carboxamide |
| 198 | *cis*-5-(4-bromophenyl)-1-(2-chlorophenyl)-4-ethyl-N-(indolin-1-yl)-4,5-dihydro-1H-pyrazole-3-carboxamide |
| 199 | *cis*-5-(4-bromophenyl)-1-(2,4-dichlorophenyl)-4-methyl-4,5-dihydro-1H-pyrazole-3-carboxylic acid |
| 200 | *trans*-5-(4-bromophenyl)-1-(2,4-dichlorophenyl)-4-methyl-4,5-dihydro-1H-pyrazole-3-carboxylic acid |
| 201 | *cis*-5-(4-bromophenyl)-1-(2-chlorophenyl)-4-methyl-4,5-dihydro-1H-pyrazole-3-carboxylic acid |
| 202 | *trans* -5-(4-bromophenyl)-1-(2-chlorophenyl)-4-methyl-4,5-dihydro-1H-pyrazole-3-carboxylic acid |
| 203 | *cis*-5-(4-bromophenyl)-1-(2,4-dichlorophenyl)-4-methyl-N-(piperidin-1-yl)-4,5-dihydro-1H-pyrazole-3-carboxamide |
| 204 | *trans*-5-(4-bromophenyl)-1-(2,4-dichlorophenyl)-4-methyl-N-(piperidin-1-yl)-4,5-dihydro-1H-pyrazole-3-carboxamide |
| 205 | *cis* 5-(4-bromophenyl)-1-(2-chlorophenyl)-4-methyl-N-(piperidin-1-yl)-4,5-dihydro-1H-pyrazole-3-carboxamide |
| 206 | *trans*-5-(4-bromophenyl)-1-(2-chlorophenyl)-4-methyl-N-(piperidin-1-yl)-4,5-dihydro-1H-pyrazole-3-carboxamide |

(continued)

| | |
|---|---|
| 207 | *cis*-N-(azepan-1-yl)-5-(4-bromophenyl)-1-(2,4-dichlorophenyl)-4-methyl-4,5-dihydro-1H-pyrazole-3-carboxamide hydrochloride |
| 208 | *trans*-N-(azepan-1 -yl)-5-(4-bromophenyl)-1-(2,4-dichlorophenyl)-4-methyl-4,5-dihydro-1H-pyrazole-3-carboxamide hydrochloride |
| 209 | *cis*-N-(azepan-1-yl)-5-(4-bromophenyl)-1-(2-chlorophenyl)-4-methyl-4,5-dihydro-H-pyrazole-3-carboxamide |
| 210 | *cis*-N-(azepan-1-yl)-5-(4-bromophenyl)-1-(2-chlorophenyl)-4-methyl-4,5-dihydro-1H-pyrazole-3-carboxamide |
| 211 | *cis*-5-(4-bromophenyl)-N-cycloheptyl-1-(2,4-dichlorophenyl)-4-methyl-4,5-dihydro-1H-pyrazole-3-carboxamide |
| 212 | *cis*-5-(4-bromophenyl)-1-(2-chlorophenyl)-N-cycloheptyl-4-methyl-4,5-dihydro-1H-pyrazole-3-carboxamide |
| 213 | *cis*-5-(4-bromophenyl)-1-(2,4-dichlorophenyl)-N-(hexahydrocyclopenta[c]pyrrol-2(1H)-yl)-4-methyl-4,5-dihydro-1H-pyrazole-3-carboxamide hydrochloride |
| 214 | *cis*-5-(4-bromophenyl)-1-(2-chlorophenyl)-N-(hexahydrocyclopenta[c]pyrrol-2(1H)-yl)-4-methyl-4,5-dihydro-1H-pyrazole-3-carboxamide |
| 215 | *cis*-5-(4-bromophenyl)-1-(2,4-dichlorophenyl)-N-(indolin-1-yl)-4-methyl-4,5-dihydro-1H-pyrazole-3-carboxamide |
| 216 | *cis*-5-(4-bromophenyl)-1-(2-chlorophenyl)-N-(indolin-1-yl)-4-methyl-4,5-dihydro-1H-pyrazole-3-carboxamide |
| 217 | (+)-cis-5-(4-bromophenyl)-1-(2,4-dichlorophenyl)-N-((1S,2S)-2-hydroxycyclohexyl)-4-methyl-4,5-dihydro-1H-pyrazole-3-carboxamide |
| 218 | (-)-cis-5-(4-bromophenyl)-1-(2,4-dichlorophenyl)-N-((1S,2S)-2-hydroxycyclohexyl)-4-methyl-4,5-dihydro-1 H-pyrazole-3-carboxamide |
| 219 | (+)-cis-5-(4-bromophenyl)-1-(2-chlorophenyl)-N-((1S,2S)-2-hydroxycyclohexyl)-4-methyl-4,5-dihydro-1H-pyrazole-3-carboxamide |
| 220 | (-)-cis-5-(4-bromopheny)-1-(2-chloropheny)-N-((1S,2S)-2-hydroxycyclohexyl)-4-methyl-4,5-dihydro-1H-pyrazole-3-carboxamide |
| 221 | *cis*-1-(2,4-dichlorophenyl)-5-(4-fluorophenyl)-4-methyl-4,5-dihydro-1H-pyrazole-3-carboxylic acid |
| 222 | *trans*-1-(2,4-dichlorophenyl)-5-(4-fluorophenyl)-4-methyl-4,5-dihydro-1H-pyrazole-3-carboxylic acid |
| 223 | *cis*-1-*(2,*4-dichlorophenyl)-5-(4-fluorophenyl)-4-methyl-N-(piperidin-1-yl)-4,5-dihydro-1H-pyrazole-3-carboxamide |
| 224 | *trans*-1-(2,4-dichlorophenyl)-5-(4-fluorophenyl)-4-methyl-N-(piperidin-1-yl)-4,5-dihydro-1H-pyrazole-3-carboxamide |
| 225 | *cis*-1-(2-chlorophenyl)-5-(4-fluorophenyl)-4-methyl-N-(piperidin-1-yl)-4,5-dihydro-1H-pyrazole-3-carboxamide |
| 226 | *trans*-1-(2-chlorophenyl)-5-(4-fluorophenyl)-4-methyl-N-(piperidin-1-yl)-4,5-dihydro-1H-pyrazole-3-carboxamide |
| 227 | *cis*-N-(azepan-1-yl)-1-(2,4-dichlorophenyl)-5-(4-fluorophenyl)-4-methyl-4,5-dihydro-1H-pyrazole-3-carboxamide hydrochloride |
| 228 | *trans*-N-(azepan-1-yl)-1-(2,4-dichlorophenyl)-5-(4-fluorophenyl)-4-methyl-4,5-dihydro-1H-pyrazole-3-carboxamide hydrochloride |
| 229 | *cis*-N-(azepan-1-yl)-1-(2-chlorophenyl)-5-(4-fluorophenyl)-4-methyl-4,5-dihydro-1H-pyrazole-3-carboxamide |
| 230 | *trans*-N-(azepan-1-yl)-1-(2-chlorophenyl)-5-(4-fluorophenyl)-4-methyl-4,5-dihydro-1H-pyrazole-3-carboxamide |

(continued)

| | |
|---|---|
| 231 | *cis*-N-cycloheptyl-1-(2,4-dichlorophenyl)-5-(4-fluorophenyl)-4-methyl-4,5-dihydro-1H-pyrazole-3-carboxamide |
| 232 | *cis*-1-(2-chlorophenyl)-N-cycloheptyl-5-(4-fluorophenyl)-4-methyl-4,5-dihydro-1H-pyrazole-3-carboxamide |
| 233 | *cis*-1-(2,4-dichlorophenyl)-5-(4-fluorophenyl)-N-(hexahydrocyclopenta[c]pyrrol-2(1H)-yl)-4-methyl-4,5-dihydro-1H-pyrazole-3-carboxamide hydrochloride |
| 234 | *cis*-1-(2-chlorophenyl)-5-(4-fluorophenyl)-N-(hexahydrocydopenta[c]pyrrol-2(1H)-yl)-4-methyl-4,5-dihydro-1H-pyrazole-3-carboxamide |
| 235 | *cis*-1-(2,4-dichlorophenyl)-5-(4-fluorophenyl)-N-(indolin-1-yl)-4-methyl-4,5-dihydro-1H-pyrazole-3-carboxamide |
| 236 | *cis*-1-(2-chlorophenyl)-5-(4-fluorophenyl)-N-(indolin-1-yl)-4-methyl-4,5-dihydro-1H-pyrazole-3-carboxamide |
| 237 | (+)-cis-1-(2,4-dichlorophenyl)-5-(4-fluorophenyl)-N-((1S,2S)-2-hydroxycyclohexyl)-4-methyl-4,5-dihydro-1H-pyrazole-3-carboxamide |
| 238 | (-)-cis-1-(2,4-dichlorophenyl)-5-(4-fluorophenyl)-N-((1S,2S)-2-hydroxycyclohexyl)-4-methyl-4,5-dihydro-1H-pyrazole-3-carboxamide |
| 239 | (+)-cis-1-(2-chlorophenyl)-5-(4-fluorophenyl)-N-((1S,2S)-2-hydroxycyclohexyl)-4-methyl-4,5-dihydro-1H-pyrazole-3-carboxamide |
| 240 | (-)-cis-1-(2-chlorophenyl)-5-(4-fluorophenyl)-N-((1S,2S)-2-hydroxycyclohexyl)-4-methyl-4,5-dihydro-1H-pyrazole-3-carboxamide |
| 241 | *cis*-1-(2,4-dichlorophenyl)-5-(4-iodophenyl)-4-methyl-4,5-dihydro-1H-pyrazole-3-carboxylic acid |
| 242 | *trans*-1-(2,4-dichlorophenyl)-5-(4-iodophenyl)-4-methyl-4,5-dihydro-1H-pyrazole-3-carboxylic acid |
| 243 | *cis*-1-(2,4-dichlorophenyl)-5-(4-iodophenyl)-4-methyl-N-(piperidin-1-yl)-4,5-dihydro-1H-pyrazole-3-carboxamide |
| 244 | *trans*-1-(2,4-dichlorophenyl)-5-(4-iodophenyl)-4-methyl-N-(piperidin-1-yl)-4,5-dihydro-1H-pyrazole-3-carboxamide |
| 245 | *cis*-1-(2-chlorophenyl)-5-(4-iodophenyl)-4-methyl-N-(piperidin-1-yl)-4,5-dihydro-1H-pyrazole-3-carboxamide |
| 246 | *trans*-1-(2-chlorophenyl)-5-(4-iodophenyl)-4-methyl-N-(piperidin-1-yl)-4,5-dihydro-1H-pyrazole-3-carboxamide |
| 247 | *cis*-N-(azepan-1-yl)-1-(2,4-dichlorophenyl)-5-(4-iodophenyl)-4-methyl-4,5-dihydro-1H-pyrazole-3-carboxamide |
| 248 | *trans*-N-(azepan-1-yl)-1-(2,4-dichlorophenyl)-5-(4-iodophenyl)-4-methyl-4,5-dihydro-1H-pyrazole-3-carboxamide |
| 249 | *cis*-N-(azepan-1-yl)-1-(2-chlorophenyl)-5-(4-iodophenyl)-4-methyl-4,5-dihydro-1H-pyrazole-3-carboxamide |
| 250 | *trans*-N-(azepan-1-yl)-1-(2-chlorophenyl)-5-(4-iodophenyl)-4-methyl-4,5-dihydro-1H-pyrazole-3-carboxamide |
| 251 | *cis*-N-cycloheptyl-1-(2,4-dichlorophenyl)-5-(4-iodophenyl)-4-methyl-4,5-dihydro-1H-pyrazole-3-carboxamide |
| 252 | *cis*-1-(2-chlorophenyl)-N-cycloheptyl-5-(4-iodophenyl)-4-methyl-4,5-dihydro-1H-pyrazole-3-carboxamide |
| 253 | *cis*-1-(2,4-dichlorophenyl)-N-(hexahydrocyclopenta[c]pyrrol-2(1H)-yl)-5-(4-iodophenyl)-4-methyl-4,5-dihydro-1H-pyrazole-3-carboxamide |
| 254 | *cis*-1-(2-chlorophenyl)-N-(hexahydrocyclopenta[c]pyrrol-2(1H)-yl)-5-(4-iodophenyl)-4-methyl-4,5-dihydro-1H-pyrazole-3-carboxamide |
| 255 | *cis*-1-(2,4-dichlorophenyl)-N-(indolin-1-yl)-5-(4-iodophenyl)-4-methyl-4,5-dihydro-1H-pyrazole-3-carboxamide |

(continued)

| | |
|---|---|
| 256 | *cis*-1-(2-chlorophenyl)-N-(indolin-1-yl)-5-(4-iodophenyl)-4-methyl-4,5-dihydro-1H-pyrazole-3-carboxamide |
| 257 | (+)-*cis*-1-(2,4-dichlorophenyl)-N-((1S,2S)-2-hydroxycyclohexyl)-5-(4-iodophenyl)-4-methyl-4,5-dihydro-1H-pyrazole-3-carboxamide |
| 258 | (-)-cis-1-(2,4-dichlorophenyl)-N-((1S,2S)-2-hydroxycyclohexyl)-5-(4-iodophenyl)-4-methyl-4,5-dihydro-1H-pyrazole-3-carboxamide |
| 259 | (+)-cis-1-(2-chlorophenyl)-N-((1S,2S)-2-hydroxycyclohexyl)-5-(4-iodophenyl)-4-methyl-4,5-dihydro-1H-pyrazole-3-carboxamide |
| 260 | (-)-cis-1-(2-chlorophenyl)-N-((1S,2S)-2-hydroxycyclohexyl)-5-(4-iodophenyl)-4-methyl-4,5-dihydro-1H-pyrazole-3-carboxamide |
| 261 | *cis*-1-(2,4-dichlorophenyl)-5-(4-methoxyphenyl)-4-methyl-4,5-dihydro-1H-pyrazole-3-carboxylic acid |
| 262 | *trans*-1-(2,4-dichlorophenyl)-5-(4-methoxyphenyl)-4-methyl-4,5-dihydro-1H-pyrazole-3-carboxylic acid |
| 263 | *cis-1-(2-chlorophenyl)-5-(4-methoxyphenyl)-4-methyl-4,5-dihydro-1H-pyrazole-3-carboxylic acid* |
| 264 | trans-1-(2-chlorophenyl)-5-(4-methoxyphenyl)-4-methyl-4,5-dihydro-1H-pyrazole-3-carboxylic acid |
| 265 | cis-1-(2,4-dichlorophenyl)-5-(4-methoxyphenyl)-4-methyl-N-(piperidin-1-yl)-4,5-dihydro-1H-pyrazole-3-carboxamide hydrochloride |
| 266 | (+)-cis-1-(2,4-dichlorophenyl)-5-(4-methoxyphenyl)-4-methyl-N-(piperidin-1-yl)-4,5-dihydro-1H-pyrazole-3-carboxamide hydrochloride |
| 267 | (-)-cis-1-(2,4-dichlorophenyl)-5-(4-methoxyphenyl)-4-methyl-N-(piperidin-1-yl)-4,5-dihydro-1H-pyrazole-3-carboxamide hydrochloride |
| 268 | *trans*-1-(2,4-dichlorophenyl)-5-(4-methoxyphenyl)-4-methyl-N-(piperidin-1-yl)-4,5-dihydro-1H-pyrazole-3-carboxamide |
| 269 | *cis*-1-(2-chlorophenyl)-5-(4-methoxyphenyl)-4-methyl-N-(piperidin-1-yl)-4,5-dihydro-1H-pyrazole-3-carboxamide |
| 270 | *trans*-1-(2-chlorophenyl)-5-(4-methoxyphenyl)-4-methyl-N-(piperidin-1-yl)-4,5-dihydro-1H-pyrazole-3-carboxamide |
| 271 | *cis*-N-(azepan-1-yl)-1-(2,4-dichlorophenyl)-5-(4-methoxyphenyl)-4-methyl-4,5-dihydro-1H-pyrazole-3-carboxamide hydrochloride |
| 272 | *trans*-N-(azepan-1-yl)-1-(2,4-dichlorophenyl)-5-(4-methoxyphenyl)-4-methyl-4,5-dihydro-1H-pyrazole-3-carboxamide hydrochloride |
| 273 | *cis*-N-(azepan-1-yl)-1-(2-chlorophenyl)-5-(4-methoxyphenyl)-4-methyl-4,5-dihydro-1H-pyrazole-3-carboxamide |
| 274 | *trans*-N-(azepan-1-yl)-1-(2-chlorophenyl)-5-(4-methoxyphenyl)-4-methyl-4,5-dihydro-1H-pyrazole-3-carboxamide |
| 275 | *cis*-N-cycloheptyl-1-(2,4-dichlorophenyl)-5-(4-methoxyphenyl)-4-methyl-4,5-dihydro-1H-pyrazole-3-carboxamide |
| 276 | *cis*-1-(2-chlorophenyl)-N-cycloheptyl-5-(4-methoxyphenyl)-4-methyl-4,5-dihydro-1H-pyrazole-3-carboxamide |
| 277 | *cis*-1-(2,4-dichlorophenyl)-N-(hexahydrocyclopenta[c]pyrrol-2(1H)-yl)-5-(4-methoxyphenyl)-4-methyl-4,5-dihydro-1H-pyrazole-3-carboxamide hydrochloride |
| 278 | *cis*-1-(2-chlorophenyl)-N-(hexahydrocyclopenta[c]pyrrol-2(1H)-yl)-5-(4-methoxyphenyl)-4-methyl-4,5-dihydro-1H-pyrazole-3-carboxamide |
| 279 | *cis*-1-(2,4-dichlorophenyl)-N-(indolin-1-yl)-5-(4-methoxyphenyl)-4-methyl-4,5-dihydro-1H-pyrazole-3-carboxamide |
| 280 | *cis*-1-(2-chlorophenyl)-N-(indolin-1-yl)-5-(4-methoxyphenyl)-4-methyl-4,5-dihydro-1H-pyrazole-3-carboxamide |

(continued)

| | |
|---|---|
| 281 | (+)-cis-1-(2,4-dichlorophenyl)-N-((1S,2S)-2-hydroxycyclohexyl)-5-(4-methoxyphenyl)-4-methyl-4,5-dihydro-1H-pyrazole-3-carboxamide |
| 282 | (-)-cis-1-(2,4-dichlorophenyl)-N-((1S,2S)-2-hydroxycyclohexyl)-5-(4-methoxyphenyl)-4-methyl-4,5-dihydro-1H-pyrazole-3-carboxamide |
| 283 | (+)-cis-1-(2-chlorophenyl)-N-((1S,2S)-2-hydroxycyclohexyl)-5-(4-methoxyphenyl)-4-methyl-4,5-dihydro-1H-pyrazole-3-carboxamide |
| 284 | (-)-cis-1-(2-ch)orophenyl)-N-((1S,2S)-2-hydroxycyclohexyl)-5-(4-methoxyphenyl)-4-methyl-4,5-dihydro-1H-pyrazole-3-carboxamide |
| 285 | *cis*-1-(2,4-dichlorophenyl)-N-(4-hydroxypiperidin-1-yl)-5-(4-methoxyphenyl)-4-methyl-4,5-dihydro-1H-pyrazole-3-carboxamide |
| 286 | *cis*-1-(2-chlorophenyl)-N-(4-hydroxypiperidin-1-yl)-5-(4-methoxyphenyl)-4-methyl-4,5-dihydro-1H-pyrazole-3-carboxamide |
| 287 | *cis*-1-(2,4-dichlorophenyl)-N-(3-hydroxypiperidin-1-yl)-5-(4-methoxyphenyl)-4-methyl-4,5-dihydro-1H-pyrazole-3-carboxamide |
| 288 | *cis*-1-(2-chlorophenyl)-N-(3-hydroxypiperidin-1-yl)-5-(4-methoxyphenyl)-4-methyl-4,5-dihydro-1H-pyrazole-3-carboxamide |
| 289 | *cis*-1-(2,4-dichlorophenyl)-N-(2-hydroxypiperidin-1-yl)-5-(4-methoxyphenyl)-4-methyl-4,5-dihydro-1H-pyrazole-3-carboxamide |
| 290 | *cis*-1-(2-chlorophenyl)-N-(2-hydroxypiperidin-1-yl)-5-(4-methoxyphenyl)-4-methyl-4,5-dihydro-1H-pyrazole-3-carboxamide |
| 291 | *cis*-1-(2,4-dichlorophenyl)-5-(4-methoxyphenyl)-4-methyl-N-(4-oxopiperidin-1-yl)-4,5-dihydro-1H-pyrazole-3-carboxamide |
| 292 | *cis*-1-(2-chlorophenyl)-5-(4-methoxyphenyl)-4-methyl-N-(4-oxopiperidin-1-yl)-4,5-dihydro-1H-pyrazole-3-carboxamide |
| 293 | *cis*-1-(2,4-dichlorophenyl)-5-(4-methoxyphenyl)-4-methyl-N-(3-oxopiperidin-1-yl)-4,5-dihydro-1H-pyrazole-3-carboxamide |
| 294 | *cis*-1-(2-chlorophenyl)-5-(4-methoxyphenyl)-4-methyl-N-(3-oxopiperidin-1-yl)-4,5-dihydro-1H-pyrazole-3-carboxamide |
| 295 | *cis*-1-(2,4-dichlorophenyl)-N-(3,4-dihydropyridin-1(2H)-yl)-5-(4-methoxyphenyl)-4-methyl-4,5-dihydro-1H-pyrazole-3-carboxamide |
| 296 | *cis*-1-(2-chlorophenyl)-N-(3,4-dihydropyridin-1(2H)-yl)-5-(4-methoxyphenyl)-4-methyl-4,5-dihydro-1H-pyrazole-3-carboxamide |
| 297 | *cis*-1-(2,4-dichlorophenyl)-N-(5,6-dihydropyridin-1(2H)-yl)-5-(4-methoxyphenyl)-4-methyl-4,5-dihydro-1H-pyrazole-3-carboxamide |
| 298 | *cis*-1-(2-chlorophenyl)-N-(5,6-dihydropyridin-1(2H)-yl)-5-(4-methoxyphenyl)-4-methyl-4,5-dihydro-1H-pyrazole-3-carboxamide |
| 299 | *cis*-1-(2,4-dichlorophenyl)-5-(4-methoxyphenyl)-N-(piperidin-1-yl)-4-(trifluoromethyl)-4,5-dihydro-1H-pyrazole-3-carboxamide |
| 300 | *trans*-1-(2,4-dichlorophenyl)-5-(4-methoxyphenyl)-N-(piperidin-1-yl)-4-(trifluoromethyl)-4,5-dihydro-1H-pyrazole-3-carboxamide |
| 301 | *cis*-N-(azepan-1-yl)-1-(2,4-dichlorophenyl)-5-(4-methoxyphenyl)-4-(trifluoromethyl)-4,5-dihydro-1H-pyrazole-3-carboxamide |
| 302 | *trans*-N-(azepan-1-yl)-1-(2,4-dichlorophenyl)-5-(4-methoxyphenyl)-4-(trifluoromethyl)-4,5-dihydro-1H-pyrazole-3-carboxamide |
| 303 | *cis*-1-(2-chlorophenyl)-5-(4-methoxyphenyl)-N-(piperidin-1-yl)-4-(trifluoromethyl)-4,5-dihydro-1H-pyrazole-3-carboxamide |

(continued)

| | |
|---|---|
| 304 | *trans*-1-(2-chlorophenyl)-5-(4-methoxyphenyl)-N-(piperidin-1-yl)-4-(trifluoromethyl)-4,5-dihydro-1H-pyrazole-3-carboxamide |
| 305 | 1-(2,4-dichlorophenyl)-5-(4-methoxyphenyl)-4,4-dimethyl-N-(piperidin-1-yl)-4,5-dihydro-1H-pyrazole-3-carboxamide |
| 306 | N-(azepan-1-yl)-1-(2,4-dichlorophenyl)-5-(4-methoxyphenyl)-4,4-dimethyl-4,5-dihydro-1H-pyrazole-3-carboxamide |
| 307 | 1-(2-chlorophenyl)-5-(4-methoxyphenyl)-4,4-dimethyl-N-(piperidin-1-yl)-4,5-dihydro-1H-pyrazole-3-carboxamide |
| 308 | 1-(2,4-dichlorophenyl)-4-formyl-5-(4-methoxyphenyl)-N-(piperidin-1-yl)-4,5-dihydro-1H-pyrazole-3-carboxamide |
| 309 | 1-(2,4-dichlorophenyl)-5-(4-methoxyphenyl)-3-(piperidin-1-ylcarbamoyl)-4,5-dihydro-1H-pyrazole-4-carboxylic acid |
| 310 | 1-(2,4-dichlorophenyl)-4-(2-hydroxyethyl)-5-(4-methoxyphenyl)-N-(piperidin-1-yl)-4,5-dihydro-1H-pyrazole-3-carboxamide |
| 311 | 1-(2,4-dichlorophenyl)-4-(fluoromethyl)-5-(4-methoxyphenyl)-N-(piperidin-1-yl)-4,5-dihydro-1H-pyrazole-3-carboxamide |
| 312 | *cis*-4-cyano-1-(2,4-dichlorophenyl)-5-(4-methoxyphenyl)-N-(piperidin-1-yl)-4,5-dihydro-1H-pyrazole-3-carboxamide |
| 313 | *trans*-4-cyano-1-(2,4-dichlorophenyl)-5-(4-methoxyphenyl)-N-(pipeddin-1-yl)-4,5-dihydro-1H-pyrazole-3-carboxamide |
| 314 | *cis*-N-(azepan-1-yl)-4-cyano-1-(2,4-dichlorophenyl)-5-(4-methoxyphenyl)-4,5-dihydro-1H-pyrazole-3-carboxamide |
| 315 | *trans*-N-(azepan-1-yl)-4-cyano-1-(2,4-dichlorophenyl)-5-(4-methoxyphenyl)-4,5-dihydro-1H-pyrazole-3-carboxamide |
| 316 | *cis* -1-(2-chlorophenyl)-4-cyano-5-(4-methoxyphenyl)-N-(piperidin-1-yl)-4,5-dihydro-1H-pyrazole-3-carboxamide |
| 317 | *trans* -1-(2-chlorophenyl)-4-cyano-5-(4-methoxyphenyl)-N-(piperidin-1-yl)-4,5-dihydro-1H-pyrazole-3-carboxamide |
| 318 | 1-(2,4-dichlorophenyl)-4-hydroxy-5-(4-methoxyphenyl)-N-(piperidin-1-yl)-4,5-dihydro-1H-pyrazole-3-carboxamide |
| 319 | 1-(2-chlorophenyl)-4,4-difluoro-5-(4-methoxyphenyl)-N-(piperidin-1-yl)-4,5-dihydro-1H-pyrazole-3-carboxamide |
| 320 | 1-(2-chlorophenyl)-4-formyl-5-(4-methoxyphenyl)-N-(piperidin-1-yl)-4,5-dihydro-1H-pyrazole-3-carboxamide |
| 321 | 1-(2-chlorophenyl)-5-(4-methoxyphenyl)-3-(piperidin-1-ylcarbamoyl)-4,5-dihydro-1H-pyrazole-4-carboxylic acid |
| 322 | 1-(2-chlorophenyl)-4-(2-hydroxyethyl)-5-(4-methoxyphenyl)-N-(piperidin-1-yl)-4,5-dihydro-1H-pyrazole-3-carboxamide |
| 323 | 1-(2-chlorophenyl)-4-(fluoromethyl)-5-(4-methoxyphenyl)-N-(piperidin-1-yl)-4,5-dihydro-1H-pyrazole-3-carboxamide |
| 324 | 1-(2-chlorophenyl)-4-hydroxy-5-(4-methoxyphenyl)-N-(piperidin-1-yl)-4,5-dihydro-1H-pyrazole-3-carboxamide |
| 325 | *cis*-1-(2,4-dichlorophenyl)-5-(4-ethoxyphenyl)-4-methyl-N-(piperidin-1-yl)-4,5-dihydro-1H-pyrazole-3-carboxamide |
| 326 | *trans*-1-(2,4-dichlorophenyl)-5-(4-ethoxyphenyl)-4-methyl-N-(piperidin-1-yl)-4,5-dihydro-1H-pyrazole-3-carboxamide |

(continued)

| | |
|---|---|
| 327 | *cis*-1-(2-chlorophenyl)-5-(4-ethoxyphenyl)-4-methyl-N-(piperidin-1-yl)-4,5-dihydro-1H-pyrazole-3-carboxamide |
| 328 | *trans*-1-(2-chlorophenyl)-5-(4-ethoxyphenyl)-4-methyl-N-(piperidin-1-yl)-4,5-dihydro-1H-pyrazole-3-carboxamide |
| 329 | *cis*-N-(azepan-1-yl)-1-(2,4-dichlorophenyl)-5-(4-ethoxyphenyl)-4-methyl-4,5-dihydro-1H-pyrazole-3-carboxamide |
| 330 | *trans*-N-(azepan-1-yl)-1-(2,4-dichlorophenyl)-5-(4-ethoxyphenyl)-4-methyl-4,5-dihydro-1H-pyrazole-3-carboxamide |
| 331 | *cis*-N-(azepan-1-yl)-1-(2-chlorophenyl)-5-(4-ethoxyphenyl)-4-methyl-4,5-dihydro-1H-pyrazole-3-carboxamide |
| 332 | *trans*-N-(azepan-1-yl)-1-(2-chlorophenyl)-5-(4-ethoxyphenyl)-4-methyl-4,5-dihydro-1H-pyrazole-3-carboxamide |
| 333 | *cis*-N-cycloheptyl-1-(2,4-dichlorophenyl)-5-(4-ethoxyphenyl)-4-methyl-4,5-dihydro-1H-pyrazole-3-carboxamide |
| 334 | *cis*-1-(2-chlorophenyl)-N-cycloheptyl-5-(4-ethoxyphenyl)-4-methyl-4,5-dihydro-1H-pyrazole-3-carboxamide |
| 335 | *cis*-1-(2,4-dichlorophenyl)-5-(4-ethoxyphenyl)-N-(hexahydrocyclopenta[c]pyrrol-2(1H)-yl)-4-methyl-4,5-dihydro-1H-pyrazole-3-carboxamide |
| 336 | *cis*-1-(2-chlorophenyl)-5-(4-ethoxyphenyl)-N-(hexahydrocyclopenta[c]pyrrol-2(1H)-yl)-4-methyl-4,5-dihydro-1H-pyrazole-3-carboxamide |
| 337 | *cis*-1-(2,4-dichlorophenyl)-5-(4-ethoxyphenyl)-N-(indolin-1-yl)-4-methyl-4,5-dihydro-1H-pyrazole-3-carboxamide |
| 338 | *cis*-1-(2-chlorophenyl)-5-(4-ethoxyphenyl)-N-(indolin-1-yl)-4-methyl-4,5-dihydro-1H-pyrazole-3-carboxamide |
| 339 | (+)-cis-1-(2,4-dichlorophenyl)-5-(4-ethoxyphenyl)-N-((1S,2S)-2-hydroxycyclohexyl)-4-methyl-4,5-dihydro-1H-pyrazole-3-carboxamide |
| 340 | (-)-cis-1-(2,4-dichlorophenyl)-5-(4-ethoxyphenyl)-N-((1S,2S)-2-hydroxycyclohexyl)-4-methyl-4,5-dihydro-1H-pyrazole-3-carboxamide |
| 341 | (+)-cis-1-(2-chlorophenyl)-5-(4-ethoxyphenyl)-N-((1S,2S)-2-hydroxycyclohexyl)-4-methyl-4,5-dihydro-1H-pyrazole-3-carboxamide |
| 342 | (-)-cis-1-(2-chlorophenyl)-5-(4-ethoxyphenyl)-N-((1S,2S)-2-hydroxycyclohexyl)-4-methyl-4,5-dihydro-1H-pyrazole-3-carboxamide |
| 343 | *cis*-1-(2,4-dichlorophenyl)-5-(4-hydroxyphenyl)-4-methyl-N-(piperidin-1-yl)-4,5-dihydro-1H-pyrazole-3-carboxamide |
| 344 | *trans*-1-(2,4-dichlorophenyl)-5-(4-hydroxyphenyl)-4-methyl-N-(piperidin-1 -yl)-4,5-dihydro-1H-pyrazole-3-carboxamide |
| 345 | *cis*-1-(2-chlorophenyl)-5-(4-hydroxyphenyl)-4-methyl-N-(piperidin-1-yl)-4,5-dihydro-1H-pyrazole-3-carboxamide |
| 346 | *trans*-1-(2-chlorophenyl)-5-(4-hydroxyphenyl)-4-methyl-N-(piperidin-1-yl)-4,5-dihydro-1H-pyrazole-3-carboxamide |
| 347 | *cis*-N-(azepan-1-yl)-1-(2,4-dichlorophenyl)-5-(4-hydroxyphenyl)-4-methyl-4,5-dihydro-1H-pyrazole-3-carboxamide |
| 348 | *trans*-N-(azepan-1-yl)-1-(2,4-dichlorophenyl)-5-(4-hydroxyphenyl)-4-methyl-4,5-dihydro-1H-pyrazole-3-carboxamide |
| 349 | *cis*-N-(azepan-1-yl)-1-(2-chlorophenyl)-5-(4-hydroxyphenyl)-4-methyl-4,5-dihydro-1H-pyrazole-3-carboxamide |

(continued)

| | |
|---|---|
| 350 | *trans*-N-(azepan-1-yl)-1-(2-chlorophenyl)-5-(4-hydroxyphenyl)-4-methyl-4,5-dihydro-1H-pyrazole-3-carboxamide |
| 351 | *cis*-N-cycloheptyl-1-(2,4-dichlorophenyl)-5-(4-hydroxyphenyl)-4-methyl-4,5-dihydro-1H-pyrazole-3-carboxamide |
| 352 | *cis*-1-(2-chlorophenyl)-N-cycloheptyl-5-(4-hydroxyphenyl)-4-methyl-4,5-dihydro-1H-pyrazole-3-carboxamide |
| 353 | *cis*-1-(2,4-dichlorophenyl)-N-(hexahydrocyclopenta[c]pyrrol-2(1H)-yl)-5-(4-hydroxyphenyl)-4-methyl-4,5-dihydro-1H-pyrazole-3-carboxamide hydrochloride |
| 354 | *cis*-1-(2-chlorophenyl)-N-(hexahydrocyclopenta[c]pyrrol-2(1H)-yl)-5-(4-hydroxyphenyl)-4-methyl-4,5-dihydro-1H-pyrazole-3-carboxamide |
| 355 | *cis*-1-(2,4-dichlorophenyl)-5-(4-hydroxyphenyl)-N-(indolin-1-yl)-4-methyl-4,5-dihydro-1H-pyrazole-3-carboxamide |
| 356 | *cis*-1-(2-chlorophenyl)-5-(4-hydroxyphenyl)-N-(indolin-1-yl)-4-methyl-4,5-dihydro-1H-pyrazole-3-carboxamide |
| 357 | (+)-cis-1-(2,4-dichlorophenyl)-N-((S,2S)-2-hydroxycyclohexyl)-5-(4-hydroxyphenyl)-4-methyl-4,5-dihydro-1H-pyrazole-3-carboxamide |
| 358 | (-)-cis-1-(2,4-dichlorophenyl)-N-((1S,2S)-2-hydroxycyclohexyl)-5-(4-hydroxyphenyl)-4-methyl-4,5-dihydro-1H-pyrazole-3-carboxamide |
| 359 | (+)-cis-1-(2-chlorophenyl)-N-((1S,2S)-2-hydroxycyclohexyl)-5-(4-hydroxyphenyl)-4-methyl-4,5-dihydro-1H-pyrazole-3-carboxamide |
| 360 | (-)-cis-1-(2-ch)orophenyl)-N-((1S,2S)-2-hydroxycyclohexyl)-5-(4-hydroxyphenyl)-4-methyl-4.5-dihydro-1 H-pyrazole-3-carboxamide |
| 361 | *cis*-5-(5-chlorothiophen-2-yl)-1-(2,4-dichlorophenyl)-4-methyl-4,5-dihydro-1H-pyrazole-3-carboxylic acid |
| 362 | *trans*-5-(5-chlorothiophen-2-yl)-1-(2,4-dichlorophenyl)-4-methyl-4,5-dihydro-1H-pyrazole-3-carboxylic acid |
| 363 | *cis*-1-(2-chlorophenyl)-5-(5-chlorothiophen-2-yl)-4-methyl-4,5-dihydro-1 H-pyrazole-3-carboxylic acid |
| 364 | *trans*-1-(2-chlorophenyl)-5-(5-chlorothiophen-2-yl)-4-methyl-4,5-dihydro-1H-pyrazole-3-carboxylic acid |
| 365 | *cis*-5-(5-bromothiophen-2-yl)-1-(2,4-dichlorophenyl)-4-ethyl-4,5-dihydro-1H-pyrazole-3-carboxylic acid |
| 366 | *trans*-5-(5-bromothiophen-2-yl)-1-(2,4-dichlorophenyl)-4-ethyl-4,5-dihydro-1H-pyrazole-3-carboxylic acid |
| 367 | *cis*-1-(2,4-dichlorophenyl)-4-methyl-5-(thiophen-2-yl)-4,5-dihydro-1H-pyrazole-3-carboxylic acid |
| 368 | *trans*-1-(2,4-dichlorophenyl)-4-methyl-5-(thiophen-2-yl)-4,5-dihydro-1H-pyrazole-3-carboxylic acid |
| 369 | *cis*-1-(2,4-dichlorophenyl)-4-methyl-N-(piperidin-1-yl)-5-(thiophen-2-yl)-4,5-dihydro-1H-pyrazole-3-carboxamide |
| 370 | *trans*-1-(2,4-dichlorophenyl)-4-methyl-N-(piperidin-1-yl)-5-(thiophen-2-yl)-4,5-dihydro-1H-pyrazole-3-carboxamide |
| 371 | *cis*-1-(2-chlorophenyl)-4-methyl-N-(piperidin-1-yl)-5-(thiophen-2-yl)-4,5-dihydro-1H-pyrazole-3-carboxamide |
| 372 | *trans*-1-(2-chlorophenyl)-4-methyl-N-(piperidin-1-yl)-5-(thiophen-2-yl)-4,5-dihydro-1H-pyrazole-3-carboxamide |
| 373 | *cis*-1-(2,4-dichlorophenyl)-4-methyl-5-phenyl-4,5-dihydro-1H-pyrazole-3-carboxylic acid |
| 374 | *trans*-1-(2,4-dichlorophenyl)-4-methyl-5-phenyl-4,5-dihydro-1H-pyrazole-3-carboxylic acid |
| 375 | *cis*-1-(2,4-dichlorophenyl)-4-methyl-5-phenyl-N-(piperidin-1-yl)-4,5-dihydro-1H-pyrazole-3-carboxamide |
| 376 | *trans*-1-(2,4-dichlorophenyl)-4-methyl-5-phenyl-N-(piperidin-1-yl)-4,5-dihydro-1H-pyrazole-3-carboxamide |
| 377 | *cis*-1-(2-chlorophenyl)-4-methyl-5-phenyl-N-(piperidin-1-yl)-4,5-dihydro-1H-pyrazole-3-carboxamide |

(continued)

| | |
|---|---|
| 378 | *trans*-1-(2-chlorophenyl)-4-methyl-5-phenyl-N-(piperidin-1-yl)-4,5-dihydro-1H-pyrazole-3-carboxamide |
| 379 | *cis*-5-(4-chlorophenyl)-1-(2,4-dichlorophenyl)-4-methyl-N-(piperidin-1-yl)-4,5-dihydro-1 H-pyrazole-3-carbothioamide hydrochloride |
| 380 | *trans*-5-(4-chlorophenyl)-1-(2,4-dichlorophenyl)-4-methyl-N-(piperidin-1-yl)-4,5-dihydro-1H-pyrazole-3-carbothioamide |
| 381 | *cis* -1-(2-chlorophenyl)-5-(4-chlorophenyl)-4-methyl-N-(piperidin-1-yl)-4,5-dihydro-1H-pyrazole-3-carbothioamide |
| 382 | *trans*-1-(2-chlorophenyl)-5-(4-chlorophenyl)-4-methyl-N-(piperidin-1-yl)-4,5-dihydro-1H-pyrazole-3-carbothioamide |
| 383 | *cis*-N-(azepan-1-yl)-5-(4-chlorophenyl)-1-(2,4-dichlorophenyl)-4-methyl-4,5-dihydro-1H-pyrazole-3-carbothioamide |
| 384 | *cis*-N-(azepan-1-yl)-1-(2-chlorophenyl)-5-(4-chlorophenyl)-4-methyl-4,5-dihydro-1H-pyrazole-3-carbothioamide |
| 385 | *cis*-5-(4-bromophenyl)-1-(2,4-dichlorophenyl)-4-methyl-N-(piperidin-1-yl)-4,5-dihydro-1H-pyrazole-3-carbothioamide |
| 386 | *trans*-5-(4-bromophenyl)-1 -(2,4-dichlorophenyl)-4-methyl-N-(piperidin-1-yl)-4,5-dihydro-1H-pyrazole-3-carbothioamide |
| 387 | *cis*-5-(4-bromophenyl)-1-(2-chlorophenyl)-4-methyl-N-(piperidin-1-yl)-4,5-dihydro-1H-pyrazole-3-carbothioamide |
| 388 | *trans*-5-(4-bromophenyl)-1-(2-chlorophenyl)-4-methyl-N-(piperidin-1-yl)-4,5-dihydro-1H-pyrazole-3-carbothioamide |
| 389 | *cis*-1-(2,4-dichlorophenyl)-5-(4-fluorophenyl)-4-methyl-N-(piperidin-1-yl)-4,5-dihydro-1H-pyrazole-3-carbothioamide |
| 390 | *trans*-1-(2,4-dichlorophenyl)-5-(4-fluorophenyl)-4-methyl-N-(piperidin-1-yl)-4,5-dihydro-1H-pyrazole-3-carbothioamide |
| 391 | *cis*-1-(2-chlorophenyl)-5-(4-fluorophenyl)-4-methyl-N-(piperidin-1-yl)-4,5-dihydro-1H-pyrazole-3-carbothioamide |
| 392 | *trans*-1-(2-chlorophenyl)-5-(4-fluorophenyl)-4-methyl-N-(piperidin-1-yl)-4,5-dihydro-1H-pyrazole-3-carbothioamide |
| 393 | *cis*-1-(2,4-dichlorophenyl)-5-(4-methoxyphenyl)-4-methyl-N-(piperidin-1-yl)-4,5-dihydro-1 H-pyrazole-3-carbothioamide |
| 394 | *cis*-N-(azepan-1-yl)-1-(2,4-dichlorophenyl)-5-(4-methoxyphenyl)-4-methyl-4,5-dihydro-1H-pyrazole-3-carbothioamide |
| 395 | *trans*-1-(2,4-dichlorophenyl)-5-(4-methoxyphenyl)-4-methyl-N-(piperidin-1-yl)-4,5-dihydro-1H-pyrazole-3-carbothioamide |
| 396 | *cis*-1-(2-chlorophenyl)-5-(4-methoxyphenyl)-4-methyl-N-(piperidin-1-yl)-4,5-dihydro-1H-pyrazole-3-carbothioamide |
| 397 | cis-N-(azepan-1-yl)-1-(2-chloropheny)-5-(4-methoxypheny)-4-methyl-4,5-dihydro-1H-pyrazole-3-carbothioamide |
| 398 | *trans*-1-(2-chlorophenyl)-5-(4-methoxyphenyl)-4-methyl-N-(piperidin-1-yl)-4,5-dihydro-1H-pyrazole-3-carbothioamide |
| 399 | *cis*-1-(2,4-dichlorophenyl)-5-(4-ethoxyphenyl)-4-methyl-N-(piperidin-1-yl)-4,5-dihydro-1H-pyrazole-3-carbothioamide |
| 400 | *trans*-1-(2,4-dichlorophenyl)-5-(4-ethoxyphenyl)-4-methyl-N-(piperidin-1-yl)-4,5-dihydro-1H-pyrazole-3-carbothioamide |

(continued)

| | |
|---|---|
| 401 | *cis*-1-(2-chlorophenyl)-5-(4-ethoxyphenyl)-4-methyl-N-(piperidin-1-yl)-4,5-dihydro-1H-pyrazole-3-carbothioamide |
| 402 | *trans*-1-(2-chlorophenyl)-5-(4-ethoxyphenyl)-4-methyl-N-(piperidin-1-yl)-4,5-dihydro-1H-pyrazole-3-carbothioamide |
| 403 | *cis*-1-(2,4-dichlorophenyl)-5-(4-hydroxyphenyl)-4-methyl-N-(piperidin-1-yl)-4,5-dihydro-1H-pyrazole-3-carbothioamide |
| 404 | *trans*-1-(2,4-dichlorophenyl)-5-(4-hydroxyphenyl)-4-methyl-N-(piperidin-1-yl)-4,5-dihydro-1H-pyrazole-3-carbothioamide |
| 405 | *cis*-1-(2-chlorophenyl)-5-(4-hydroxyphenyl)-4-methyl-N-(piperidin-1-yl)-4,5-dihydro-1H-pyrazole-3-carbothioamide |
| 406 | *trans*-1-(2-chlorophenyl)-5-(4-hydroxyphenyl)-4-methyl-N-(piperidin-1-yl)-4,5-dihydro-1H-pyrazole-3-carbothioamide |
| 407 | 7-(4-Chloro-phenyl)-6-(2,4-dichlorophenyl)-5,6-diaza-spiro[2.4]hept-4-ene-4-carboxylic acid piperidin-1-ylamide |
| 408 | 6-(2,4-Dichloro-phenyl)-7-(4-methoxy-phenyl)-5,6-diaza-spiro[2.4]hept-4-ene-4-carboxylic acid piperidin-1-ylamide ; |
| 409 | (+)-*cis*-N-((1S,2S)-2-(benzyloxy)cyclohexyl)-5-(4-chlorophenyl)-1-(2,4-dichlorophenyl)-4-methyl-4,5-dihydro-1H-pyrazole-3-carboxamide |
| [410] | *cis*-1-(2,4-dichlorophenyl)-5-(4-methoxyphenyl)-4-methyl-N-(piperidin-1-yl)-4,5-dihydro-1H-pyrazole-3-carboxamide |
| [411] | *cis*-1-(2,4-dichlorophenyl)-5-(4-methoxyphenyl)-4-methyl-N-(piperidin-1-yl)-4,5-dihydro-1H-pyrazole-3-carboxamide |
| [412] | (4R,5S)-1-(2,4-dichlorophenyl)-5-(4-methoxyphenyl)-4-methyl-4,5-dihydro-1H-pyrazole-3-carboxylic acid |
| [413] | cis-5-(4-bromophenyl)-1-(2,4-dichlorophenyl)-4-methyl-4,5-dihydro-1H-pyrazole-3-carboxylic acid |
| [414] | cis-N-(azepan-1-yl)-1-(2,4-dichlorophenyl)-5-(4-fluorophenyl)-4-methyl-4,5-dihydro-1H-pyrazole-3-carboxamide |
| 415 | *cis*-5-(4-bromophenyl)-1-(2,4-dichlorophenyl)-N-(indolin-1-yl)-4-methyl-4,5-dihydro-1H-pyrazole-3-carboxamide |
| 416 | *trans*-ethyl 5-(4-chlorophenyl)-1-(2,4-dichlorophenyl)-4-(trifluoromethyl)-4,5-dihydro-1H-pyrazole-3-carboxylate |
| 417 | *cis*-1-(2,4-dichlorophenyl)-5-(4-fluorophenyl)-4-methyl-N-(piperidin-1-yl)-4,5-dihydro-1H-pyrazole-3-carboxamide hydrochloride |
| 418 | *cis*-5-(4-bromophenyl)-1-(2,4-dichlorophenyl)-4-methyl-N-(piperidin-1-yl)-4,5-dihydro-1H-pyrazole-3-carboxamide hydrochloride |
| 419 | (-)-*cis*-5-(4-chlorophenyl)-1-(2,4-dichlorophenyl)-4-methyl-N-(piperidin-1-yl)-4,5-dihydro-1H-pyrazole-3-carboxamide |
| 420 | (+)-*cis*-5-(4-chlorophenyl)-1-(2,4-dichlorophenyl)-4-methyl-N-(piperidin-1-yl)-4,5-dihydro-1H-pyrazole-3-carboxamide |
| 421 | *trans*-ethyl 1-(2,4-dichlorophenyl)-4-methyl-5-phenyl-4,5-dihydro-1H-pyrazole-3-carboxylate |
| 422 | *cis*-5-(4-chlorophenyl)-1-(2,4-dichlorophenyl)-4-methyl-N-((1R,2S,4R)-1,7,7-trimethylbicyclo[2.2.1]heptan-2-yl)-4,5-dihydro-1H-pyrazole-3-carboxamide |
| 423 | *cis*-5-(4-chlorophenyl)-1-(2,4-dichlorophenyl)-4-methyl-N-(3-methylcyclohexyl)-4,5-dihydro-1H-pyrazole-3-carboxamide |
| 424 | *cis*-5-(4-chlorophenyl)-1-(2,4-dichlorophenyl)-4-methyl-N-(2-methylcyclohexyl)-4,5-dihydro-1H-pyrazole-3-carboxamide |

(continued)

| | |
|---|---|
| 426 | *trans*-5-(5-chlorothiophen-2-yl)-1-(2,4-dichlorophenyl)-4-methyl-N-(piperidin-1-yl)-4,5-dihydro-1H-pyrazole-3-carboxamide hydrochloride |
| 427 | *cis*-5-(5-chlorothiophen-2-yl)-1-(2,4-dichlorophenyl)-4-methyl-N-(piperidin-1-yl)-4,5-dihydro-1H-pyrazole-3-carboxamide hydrochloride |
| 428 | *cis*-5-(4-bromophenyl)-1-(2,4-dichlorophenyl)-4-ethyl-N-(piperidin-1-yl)-4,5-dihydro-1H-pyrazole-3-carboxamide hydrochloride |
| [429] | 1-(2,4-dichlorophenyl)-4-methyl-5-phenyl-4,5-dihydro-1H-pyrazole-3-carboxylic acid |
| [430] | cis-1-(2,4-dichlorophenyl)-4-methyl-N-(piperidin-1-yl)-5-(thiophen-2-yl)-4,5-dihydro-1H-pyrazole-3-carboxamide hydrochloride |
| [431] | trans-5-(4-chlorophenyl)-1-(2,4-dichlorophenyl)-4-methyl-N-(piperidin-1-yl)-4,5-dihydro-1H-pyrazole-3-carboxamide |
| 432 | *cis*-5-(4-chlorophenyl)-1-(2,4-dichlorophenyl)-4-ethyl-N-(piperidin-1-yl)-4,5-dihydro-1H-pyrazole-3-carboxamide |
| 434 | *cis*-5-(4-chlorophenyl)-1-(2,4-dichlorophenyl)-4-methyl-N-(piperidin-1-yl)-4,5-dihydro-1H-pyrazole-3-carboxamide |
| 436 | (+)-*cis*-N-((1S,2S)-2-(benzyloxy)cyclohexyl)-5-(4-chlorophenyl)-1-(2,4-dichlorophenyl)-4-methyl-4,5-dihydro-1H-pyrazole-3-carboxamide |
| 437 | (-)-*cis*-N-((1R,2R)-2-(benzyloxy)cyclohexyl)-5-(4-chlorophenyl)-1-(2,4-dichlorophenyl)-4-methyl-4,5-dihydro-1H-pyrazole-3-carboxamide |
| 438 | *cis*-N-(azocan-1-yl)-1-(2,4-dichlorophenyl)-5-(4-methoxyphenyl)-4-methyl-4,5-dihydro-1H-pyrazole-3-carboxamide |
| 439 | *cis*-N-(azocan-1-yl)-1-(2-chlorophenyl)-5-(4-methoxyphenyl)-4-methyl-4,5-dihydro-1H-pyrazole-3-carboxamide |
| 440 | *cis*-N-(azocan-1-yl)-5-(4-bromophenyl)-1-(2,4-dichlorophenyl)-4-methyl-4,5-dihydro-1H-pyrazole-3-carboxamide |
| 441 | *cis*-N-(azocan-1-yl)-5-(4-bromophenyl)-1-(2-chlorophenyl)-4-methyl-4,5-dihydro-1H-pyrazole-3-carboxamide |
| 442 | *cis*-N-(azocan-1-yl)-1-(2,4-dichlorophenyl)-5-(4-fluorophenyl)-4-methyl-4,5-dihydro-1H-pyrazole-3-carboxamide |
| 443 | *cis*-N-(azocan-1-yl)-1-(2-chlorophenyl)-5-(4-fluorophenyl)-4-methyl-4,5-dihydro-1H-pyrazole-3-carboxamide |
| 444 | (+)-*cis*-5-(4-Chlorophenyl)-1-(2,4-dichlorophenyl)-4-methyl-4,5-dihydro-1H-pyrazole-3-carboxylic acid azepan-1-ylamide hydrochloride |
| 445 | (-)-cis- (4-Chlorophenyl)-1-(2,4-dichlorophenyl)-4-methyl-4,5-dihydro-1H-pyrazole-3-carboxylic acid azepan-1-ylamide hydrochloride |

**PHARMACOLOGICAL PART:**

## EXAMPLES

(rac)-N-piperidinyl-5-(4-chlorophenyl)-1-(2,4-dichlorophenyl)-4,5-dihydro-1H-pyrazole-3-carboxamide (hereafter Compound C) is described in Subgroup A1, Chemical Example 1, (R)-N-piperidinyl-5-(4-chlorophenyl)-1-(2,4-dichlorophenyl)-4,5-dihydro-1H-pyrazole-3-carboxamide (hereafter Compound A) is described in Subgroup A2, Chemical Example 1, and (S)-N-piperidinyl-5-(4-chlorophenyl)-1-(2,4-dichlorophenyl)-4,5-dihydro-1H-pyrazole-3-carboxamide (hereafter Compound B) is described in Subgroup A2, Chemical Example 2.

**Pharmaceutical Example 1: Determination of the affinities of (rac)-N-piperidinyl-5-(4-chlorophenyl)-1-(2,4-dichlorophenyl)-4,5-dihydro-1H-pyrazole-3-carboxamide (hereafter Compound C), (R)-N-piperidinyl-5-(4-chlorophenyl)-1-(2,4-dichlorophenyl)-4,5-dihydro-1H-pyrazole-3-carboxamide (hereafter Compound A)**

**and (S)-N-piperidinyl-5-(4-chlorophenyl)-1-(2,4-dichlorophenyl)-4,5-dihydro-1H-pyrazole-3-carboxamide (hereafter Compound B) for rat CB1 receptors.**

**Methods**

Binding affinity to CB1 receptor was evaluated according to the method of Govaerts et al (2004) with the following modifications Briefly, cerebella from male Wistar rats (250-300g) were carefully dissected on ice and homogenates were prepared in cold 50 mM Tris-HCl, 5 mM $MgCl_2$, 1 mM EDTA, 0.25 M sucrose, pH 7.4 with a Potter-Heljheim homogenizer and the suspension was centrifuged at 1,000 x g for 5 minutes. The supernatants were collected and centrifuged 50,000 x g for 15 min. The resulting pellets were resuspended in Tris-HCl buffer without sucrose, homogenized and incubated for 15 min at 37˚C in an orbital shaker bath and centrifuged again at 50,000 x g for 15 min. Pellets were weighed, resuspended in Tris-HCl buffer without sucrose, homogenized with an Ultraturrax at 13,500 rpm for 3 x 5 seconds and aliquoted in 0.9 ml volumes in Eppendorf tubes. Aliquots were centrifuged at 20,800 x g for 5 minutes, supernatants discarded and pellets were frozen at -80˚C until use. Total protein concentration was determined using a Bio-Rad commercial assay base on the Lowry method. Competitive binding experiments were performed in presence of 1 nM [$^3$H]-CP 55,940 in siliconized glass tubes containing 100 μg protein/tube resuspended in 1ml final volume of 50 mM Tris-HCl, 5 mM $MgCl_2$ , 1 mM EDTA, 0.5% (w/v) bovine serum albumin, pH 7.4. Competitors were present at various concentrations and the non-specific binding was determined in the presence of 10 μM HU-210. After 1 hour incubation at 30˚C, the suspension was rapidly filtered through 0.5% PEI pre-treated GF/B fiber filters on a 96-well harvester and washed 3 times with 3ml ice-cold binding buffer without bovine serum albumin. Radioactivity on filters was measured with Wallac Winspectral 1414 counter by liquid scintillation in 6 ml Ecoscint H (National Diagnostics, U.K.). Assays were made in triplicate.

Binding data were analyzed by non-linear regression with the software GraphPad Prism Version 3.03.

**Results**

When the affinities of COMPOUND C, COMPOUND B and COMPOUND A for the rat CB1 receptor subtype were determined in vitro, COMPOUND C and COMPOUND A were found to have good affinity for these receptors with a $IC_{50}$ values of 26 nM and 22 nM, respectively (Table 1). On the other hand, with an $IC_{50}$ value of 813 nM, COMPOUND B has no pharmacologically relevant affinity for rat CB1 receptors (Table 1).

Table 1 The affinities of various compounds for rat CB1 receptors

| Example | $pIC_{50}$ | $IC_{50}$ |
|---|---|---|
| COMPOUND C | 7.65 ± 0.29 | 26 |
| COMPOUND B | 6.09 ± 0.09 | 813 |
| COMPOUND A | 7.66 ± 0.08 | 22 |

**Pharmaceutical Example 2: Effect of COMPOUND C ((rac)-N-piperidinyl-5-(4-chlorophenyl)-1-(2,4-dichlorophenyl)-4,5-dihydro-1H-pyrazole-3-carboxamide) on glycaemic control and on other metabolic disturbances associated with the aetiology of impaired glucose tolerance/insulin resistance and/or Type 2 diabetes.**

**METHODS**

**Experiment in male, dietary-induced (DIO) Sprague-Dawley rats**

The experiment was performed on selectively bred, male, Sprague-Dawley rats that were assessed at 3-5 weeks of age as having a predisposition to become obese. These animals were maintained on a normal light cycle (Lights on between 06.00h and 18.00h) under controlled temperature conditions. These rats were then given ad lib access to a commercial, energy-dense, high-fat diet. The animals for the study were 24 weeks old at the start of the experiment.

Groups of rats were injected intraperitoneally with COMPOUND C (10 mg/kg in 0.1% Tween in saline; n = 15) or vehicle (0.1% Tween in saline; n = 10). Animals were injected daily between 09.00h and 11.00h and food intake and body-weights were measured daily at the time of injection from Day-0.

**Oral glucose tolerance test**

On Day-21, groups of compound- and vehicle-treated mildly fasted rats were administered 2 g/kg glucose (as a 2g/ml solution) via a gastric tube placed into the stomach. Blood samples for plasma insulin determinations were taken at -30 min, 0, 15, 30, 60 and 120 min.

**Blood sampling**

Blood samples were taken from the tail veins of mildly fasted rats (restricted to 50% of their normal food intake in the 20h period before sampling) for the determination of plasma concentrations of triglycerides FFAs and insulin on Day-1 and Day-21.

**Determination of plasma lipid and insulin concentrations**

Plasma concentrations of triacylglycerol were determined using a standard automated enzymatic assay and FFAs were quantified using an acyl-CoA oxidase based colourimetric assay. Plasma insulin was determined by an ultra-sensitive ELISA method.

**Fat depots analysis**

On the day of termination of the experiment, inguinal, epidiymal, mesenterial and retroperitoneal fat depots were removed and weighed.

**Statistical analyses**

Calculations and statistical analyses were performed using GraphPad and Statview software packages. One-way ANOVA followed by Fischer's post hoc test was used to analyse the experimental data. $P < 0.05$ was set as the acceptance level for significant difference between groups.

**Experiment in female, dietary-induced (DIO), female Wistar rats**

**Animals**

The experiment was performed on female Wistar rats (originally in the weight range 250-300g). Animals were maintained on a reverse phase light-dark cycle (lights off for 8 h from 09.30-17.30 h) during which time the room was illuminated by red light. Animals had free access to powdered high fat diet (VRF1 plus 20% lard), ground chocolate, ground peanuts and tap water at all times. The three different diets were contained in separate glass feeding jars with aluminium lids with a 3-4 cm hole cut in it to allow access to the food. Animals were housed in pairs for twelve weeks for the induction of obesity. Animals were then housed singly in polypropylene cages with wire grid floors to enable the food intake of each rat to be recorded. Polypropylene trays with cage pads were placed beneath each cage to detect any food spillage. Animals were accustomed to these new conditions (individual housing and the wire-grid floor cages) for at least 14 days before the start of the baseline readings.

**Experimental procedures for the feeding study**

**Approximately a week before the start of the study, animals were weighed (to the nearest 0.1 g using an electronic top-pan balance) and allocated into 2 weight-matched treatment groups, each containing 10 animals. Following a 7 day baseline run-in period, during which the animals were dosed by the appropriate route (eg orally) once a day with vehicle (1% methylcellulose; 1 ml/kg) or the test drug, ie COMPOUND C (30 mg/kg po) once daily for 28 days.**

Body weight was measured once daily.

Statistical comparisons between the body weights of the different treatment groups have been made by analysis of covariance followed by multiple t tests (two-tailed) to compare each treatment group with the vehicle-treated controls. For the body weight data (body weights each day), the weights of the animals on Day 1 (ie immediately before the first drug treatment) were used as the covariate. Thus, means were adjusted for differences in body weight between the groups on Day 1 and standard errors of the mean (SEM) were calculated from the residuals of the statistical model. All

mean body weight values on Day 1 are identical (and SEM are therefore zero) because the analysis of covariance adjusts them all to equal the 'grand mean' of the values of this day.

**Plasma analyses**

Terminal blood samples were taken via cardiac puncture from the animals immediately after death into Sarstedt 4.5 ml lithium-heparin tubes (Cat No. 32.331). Tubes were then centrifuged at 1,500 g for 5 minutes at 4˚C and the plasma removed and immediately frozen on dry ice (as 3 aliquots per sample in sealed Eppendorf tubes). Plasma samples were stored at -75˚C until required for analysis. All determinations were performed on plasma samples that had been thawed only once.

**Insulin assay**

Plasma insulin concentration was determined using Mercodia ultrasensitive rat insulin ELISA kits according to the manufacturers 50 μl sample protocol. Unknown samples were diluted 20 times using the zero standard prior to assay in duplicate. Optical density at 450 nm was determined using a Molecular Devices VERSAmax tunable microplate reader and readings transferred to GraphPad Prism. A cubic spline curve was fitted to the calibration data and unknown values extrapolated from this curve fit.

**Glycerol and triacylglycerol assay**

Plasma triacylglycerol was determined by modification of a discrete assay kit (Sigma Triglyceride determination kit, Cat No. TR0100) to a 96-well format. The kit actually determines liberated glycerol following enzymic hydrolysis of triacylglycerol to free fatty acids and glycerol (1 mole glycerol per mole of triacylglycerol). For this reason, correction for the glycerol concentration originally present in the plasma is required. Plasma samples were, therefore, assayed in the absence (free plasma glycerol) or presence (total triacylglycerol) of lipoprotein lipase. Subtraction of free glycerol from total triacylglycerol concentration then derives the true triacylglycerol concentration.

A 2.5 mg/ml triolein equivalent glycerol standard was obtained from Sigma. The supplied Sigma mM conversion factor for this batch was 1.13 (ie 2.83 mM glycerol). A 10 mg/ml glycerol stock solution was prepared in saline and then diluted in saline to create a series of standard concentrations from 0.218 to 4.34 mM. Standards and plasma samples (undiluted) were pipetted into the 96-well plates as 10 μl additions in duplicate. Assay plates and reagents were preheated at 37˚C and the reaction started by adding 200 μl of either the glycerol or total triacylglycerol reagent (reconstituted as per manufacturer's protocol) followed by incubation at 37˚C for 5 minutes after which optical density at 540 nm was determined using a Molecular Devices VERSAmax tunable microplate reader. Optical density readings were then transferred to GraphPad Prism and unknown values extrapolated from a linear regression fit to the standard curve. The assay was linear up to the highest glycerol standard employed (4.34 mM). Subtraction of the glycerol concentration in the plasma samples from the total triacylglycerol concentration derived the true triacylglycerol concentration.

**Leptin assay**

Plasma leptin concentration was determined using Assay Designs rat leptin enzyme immunometric assay kits (Cat No. 900-015) according to the manufacturer's regular protocol. Unknown samples were diluted 100 times using leptin assay buffer prior to assay in duplicate. Optical density at 450 nm was determined using a Molecular Devices VERSAmax tunable microplate reader and readings transferred to GraphPad Prism. A second order polynomial curve was fitted to the calibration data and unknown values extrapolated from this curve fit.

**Determination of carcass fat content**

Total ether-extractable fat in the freeze-dried samples was estimated by the Soxhlet principle using a Foss Soxtec HT2 system. The protocol employed was based on the manufacturer's recommendations for meat samples, although freeze-dried samples obviated the necessity for either a fat hydrolysis step or mixing the sample with sand to ensure efficient extraction.

Samples of approximately 1 g were accurately weighed into 26 mm x 60 mm cellulose extraction thimbles which were then plugged with approximately 0.8 g cotton wool. Sample fat was extracted into pre-weighed (including a few boiling chips) standard metal extraction cups using 70 ml of petroleum ether at 40-60˚C, a circulator temperature of 115˚C and a cooling flow rate of 2 l/min. A boiling stage of 25 min was employed, followed by a 40 min wash stage. A 10 min drying stage sufficed to remove ether from the extraction cups.

Percentage fat in the freeze-dried sample was calculated as follows:

$$\% \text{ fat} = [100 \times (\text{final cup weight} - \text{initial cup weight})]/\text{sample weight}$$

Percentage fat in the original sample was calculated using the % water as follows:

$$\% \text{ fat in original sample} = \% \text{ fat} \times [(100 - \% \text{ water})/100]$$

**Determination of carcass protein content**

Total protein in the freeze-dried samples was estimated using the Kjeldahl principle. A micro-Kjeldahl procedure was performed using a Foss 2012 digestion block and a Foss 2200 distilling unit. The protocol used was based on the manufacturer's recommendations for meat samples.

Approximately 0.3 g of freeze-dried sample was accurately weighed into 100 ml digestion tubes (Foss UK Ltd) in racks of 12 tubes. Each rack included at least one blank tube to determine a reagent blank. To each tube was added two Kjeltabs CQ and one Antifoam S tablet followed by 10 ml of 98% sulphuric acid. Samples were digested at 420˚C for 1 h to convert protein into ammonium sulphate. Once tubes had cooled, free ammonia was liberated by the addition of an excess of 40% NaOH and steam distilled into 30 ml of 4% boric acid indicator solution. Ammonia was then determined by titration against volumetric grade 0.1 M HCl using a Brand 25 ml digital burette. Nitrogen and protein content of the samples were calculated as follows:

$$\% \text{ nitrogen} = [(\text{sample titration} - \text{blank titration}) \times 0.1401]/\text{sample weight (g)}$$

$$\% \text{ protein} = \% \text{ nitrogen} \times 6.25$$

**Determination of carcass ash content**

Total ash in the freeze-dried samples was estimated by determining the non-combustible ash remaining after firing samples at high temperatures using a Carbolite OAF 11/1 muffle furnace.

Squat form silica crucibles were prefired (600˚C) for at least 2 h to stabilise weights. Crucibles were allowed to cool (<300˚C) and transferred to silica gel drying cabinets. Crucibles were then weighed, approximately 1 g of sample added and the crucible reweighed. A number of empty crucibles were also included. Crucibles were fired at 600˚C for 4 h to convert samples to ash and then allowed to cool (<300˚C) prior to transfer to silica gel drying cabinets. Final weights were determined when crucibles had fully cooled.

Percentage ash was determined by expressing the residual sample weight after firing as a percentage of the original sample weight. The average variation in blank crucible weight was - 0.02 ± 0.04 mg (mean ± SEM, n=14) and individual weights varied by 0.5 mg or less.

**Experiment in growing, male Wistar rats**

Adult, male, Wistar rats aged between 11 and 12 weeks and weighing 250-280 g were used in the experiment. After 5 days of quarantine, they were housed initially in groups of 5, but were individually housed for use in the experiment. The animals were given free access to normal laboratory rat chow. The rats were maintained on a normal 12h -12h dark-light cycle and were kept in a vivarium at 18 - 24˚C and 37 - 70% humidity.

After 2 weeks of acclimatization when food and water intake and bodyweights were measured on a daily basis, groups of rats were administered COMPOUND C (10 mg/kg or 30 mg/kg as a suspension in 5% gum arabic in saline; n = 15) or vehicle (5% gum arabic in saline; n = 15) by intragastric gavage. Animals were administered drug or vehicle once-daily for 14 days and food intake and body weights were measured daily at the time of injection.

**Blood sampling**

On Day-15, terminal blood samples were taken from 5 rats from each group for the determination of plasma concentrations of total cholesterol and triglycerides.

**Determination of plasma lipid and concentrations**

Plasma concentrations of total cholesterol and triacylglycerol were determined using a standard analyses performed in a Cardio Check™ Cardio reflectance photometer

**Statistical analyses**

Calculations and statistical analyses were performed using GraphPad and Statview software packages. Two-way ANOVA was used to analyse the body weight data. The total cholesterol, triglycerides and glucose concentration results were analysed by means of ANOVA RM followed by Dunnett's test. $P < 0.05$ was set as the acceptance level for significant difference between groups.

**RESULTS**

**Effects of COMPOUND C on -glycaemic control**

Effects of COMPOUND C (10 mg/kg ip) on plasma insulin concentrations and responsiveness to an oral glucose challenge in male, DIO, Sprague-Dawley rats

Chronic administration of COMPOUND C improved the glycaemic profiles of these obese rats by statistically significantly ($p < 0.001$) reducing plasma insulin levels compared to the vehicle-treated control group (Figure 1).

When these rats were subjected to an oral glucose challenge test, the area under the curve (AUC) for the insulin excursion was significantly lower in the obese rats repeatedly given COMPOUND C than those given vehicle (Figure 2).

Effects of COMPOUND C (30 mg/kg po) on plasma insulin concentrations in female, DIO Wistar rats

Chronic administration of COMPOUND C improved the glycaemic profiles of these obese rats by reducing plasma insulin levels compared to the vehicle-treated control group (Figure 3).

**Effects of COMPOUND C on plasma lipid concentrations**

Effects of COMPOUND C (10 mg/kg ip) on plasma triglycerides and free fatty acid (FFA) concentrations in male, DIO, Sprague-Dawley rats

Chronic administration of COMPOUND C improved the lipid profiles of these obese rats statistically significantly ($p < 0.05$), reducing plasma trigyceride levels and FFA levels compared to the vehicle-treated control group (Figure 4).

Effects of COMPOUND C (30 mg/kg po) on plasma glycerol and triacylgycerol concentrations in female, DIO Wistar rats

Chronic administration of COMPOUND C significantly improved the lipid profiles of these obese rats by reducing plasma glycerol levels (by 23.4%) and triacylglycerol levels (by 37.2%) compared to the vehicle-treated control group (Figure 5).

Chronic effects of COMPOUND C (10 and 30 mg/kg po) on plasma triglyceride and cholesterol and concentrations in growing, male Wistar rats

Chronic administration of COMPOUND C (10 and 30 mg/kg po) dose-dependently improved the lipid profiles of growing, Sprague-Dawley rats by reducing plasma triglyceride levels compared to the vehicle-treated control group (Figure 6). The reduction was statistically significantly ($p < 0.05$) at the higher dose of COMPOUND C (Figure 6). COMPOUND C also dose-dependently reduced plasma cholesterol levels compared to the vehicle-treated control group (Figure 7). The reduction was statistically significantly ($p < 0.05$) at the higher dose of COMPOUND C (Figure 7).

**Effects of COMPOUND C on bodyweight**

Chronic effect of COMPOUND C (10 mg/kq ip) on the body weights of male, DIO, Sprague-Dawley rats

When COMPOUND C (10 mg/kg ip) was administered to the male, DIO, Sprague-Dawley rats once daily for

a period of 21 days, it evoked a marked and sustained decrease in the body weights of these obese rats (Figure 8). The reductions in body weights were highly statistically significant ($p < 0.0001$) after 7 and 21 days day of administration of COMPOUND C (10 mg/kg ip) (Figure 8).

Chronic effect of COMPOUND C (30 mg/kg po) on the body weights of female, DIO Wistar rats

When COMPOUND C (30 mg/kg po) was administered to the female, cafeteria-fed, DIO Wistar rats once daily for a period of 28 days, it evoked a marked and sustained decrease in the body weights of these obese rats (Figure 9). The reductions in body weights were statistically significant after 1 day of administration of COMPOUND C (30 mg/kg po) (Figure 9).

Chronic effects of COMPOUND C (10 and 30 mg/kg po) on the body weights of growing, male, Wistar rats

When COMPOUND C (10 and 30 mg/kg po) was administered to the male, Wistar rats once daily for a period of 14 days, it evoked a marked and sustained decrease in the body weights of these rats (Figure 10). At Day 14, the reductions in body weights were statistically significant $p < 0.002$ for COMPOUND C (30 mg/kg po) (Figure 10).

Effect of COMPOUND C (10 mg/kg ip) on the visceral fat-pad weights of male, DIO, Sprague-Dawley rats

Consistent with a marked reduction in overall adiposity as shown by the decrease of bodyweight (Figure 8), measurement of the weights of the major visceral fat-pads, ie inguinal, epididymal, retroperitoneal and mesenterial, similarly revealed large reductions (Figure 11). These decreases were statistically significant for all of the visceral fat depots, except the epididymal pads (Figure 11).

Effects of COMPOUND C (30 mg/kg po) on the body composition of female, DIO Wistar rats

The body composition of the vehicle-treated group of animals was 49.0 ± 1.0% water, 31.9 ± 1.3% fat, 15.5 ± 0.3% protein and 2.77 ± 0.09% ash (total of 99.2%). The final mean carcass weight of the vehicle-treated control group was 473.0 ± 8.1 g. COMPOUND C significantly reduced final carcass weight by 72.8 g.

Chronic administration of COMPOUND C significantly reduced total carcass water by 11.8 g when compared to the vehicle-treated control. As the total carcass water content reduced (in proportion) by much less than the reduction in carcass weight, the percentage water content of the carcasses was actually significantly increased in the COMPOUND C-treated animals (from 49.0% to 54.3% Figure 12). COMPOUND C significantly and substantially reduced total carcass fat and percentage fat. Mean body fat mass was significantly reduced from 156.3 g in the vehicle group to 98.6 g following COMPOUND C treatment. Expressed as a percentage of carcass weight, fat content was significantly reduced from 31.9% in the vehicle-control group to 24.2% in the COMPOUND C -treated group (Figure 12). Thus, COMPOUND C produced a decrease in absolute body fat of 57.7 g compared to the vehicle-treated control group, representing 79.3% of the total weight-loss observed. This was equivalent to a marked 36.9% decrease in fat mass compared to the vehicle-treated control group. Percentage protein content was significantly increased from 15.5% in the vehicle-treated group to 17.1% in the COMPOUND C-treated group (Figure 12). COMPOUND C did not significantly alter total ash content, but significantly increased percentage ash content from 2.77% to 3.26% (Figure 12).

Effects of COMPOUND C (30 mg/kg po) on plasma leptin concentrations in female, DIO Wistar rats

Consistent with a marked reduction in the white adipose tissue mass, plasma leptin concentrations were markedly and significantly reduced following COMPOUND C (-48.2%) treatment compared to the vehicle-treated control group (Figure 13).

**Pharmaceutical Example 3: Effect of COMPOUND B ((S)-N-piperidinyl-5-(4-chlorophenyl)-1-(2,4-dichlorophenyl)-4,5-dihydro-1H-pyrazole-3-carboxamide) and COMPOUND A (R)-N-piperidinyl-5-(4-chlorophenyl)-1-(2,4-dichlorophenyl)-4,5-dihydro-1H-pyrazole-3-carboxamide) on glycaemic control and on other metabolic disturbances associated with the aetiology of impaired glucose tolerance/insulin resistance and/or Type 2 diabetes.**

**Animals**

The experiment was performed on female Wistar rats (originally in the weight range 250-300 g). The rats were housed in pairs in polypropylene cages with solid floors and sawdust bedding at a temperature of 21±4°C and 55±20% humidity. Animals were maintained on a reverse phase light-dark cycle (lights off for 8 h from 09.30-17.30 h) during

which time the room was illuminated by red light. Animals had free access to powdered high fat diet (VRF1 plus 20% lard), ground chocolate, ground peanuts and tap water at all times. The three different diets were contained in separate glass feeding jars with aluminum lids with a 3-4 cm hole cut in it to allow access to the food. Animals were housed in pairs for twelve weeks for the induction of obesity. Animals were then housed singly in polypropylene cages with wire grid floors to enable the food intake of each rat to be recorded. Polypropylene trays with cage pads were placed beneath each cage to detect any food spillage. Animals were accustomed to these new conditions (individual housing and the wire-grid floor cages) for at least 14 days before the start of the baseline readings.

**Experimental procedures for the feeding study**

**Approximately a week before the start of the study, animals were weighed (to the nearest 0.1 g using an electronic top-pan balance) and allocated into 3 weight-matched treatment groups, each containing 9-10 animals. Following a 7 day baseline run-in period, during which the animals were dosed by the appropriate route (eg orally) once a day with vehicle (0.5% hydroxy-propyl-methylcellulose [HPMC]; 1 ml/kg) or the test drug, ie COMPOUND A (10mg and 30 mg/kg po) and COMPOUND B (10 mg and 30 mg/kg po) once daily for 28 days.**

Food intake and body weight were measured once daily. Variations in body weight and energy levels of the different types of food were accounted for by expressing the food intake results in terms of kJ/kg rat weight.

Statistical comparisons between the body weights and daily food intakes of the different treatment groups have been made by analysis of covariance followed by Dunnett's test to compare each treatment group with the vehicle-treated controls. For the body weight data (body weights each day), the weights of the animals on Day 1 (ie immediately before the first drug treatment) were used as the covariate. Thus, means were adjusted for differences in body weight between the groups on Day 1 and standard errors of the mean (SEM) were calculated from the residuals of the statistical model. All mean body weight values on Day 1 are identical (and SEM are therefore zero) because the analysis of covariance adjusts them all to equal the 'grand mean' of the values of this day.

**Plasma analyses**

Terminal blood samples via cardiac puncture were taken from the animals immediately after death into Sarstedt 4.5 ml lithium-heparin tubes (Cat No. 32.331). Tubes were then centrifuged at 1,500 g for 5 minutes at 4°C and the plasma removed and immediately frozen on dry ice (as 3 aliquots per sample in sealed Eppendorf tubes). Plasma samples were stored at -75°C until required for analysis. All determinations were performed on plasma samples that had been thawed only once.

**Insulin assay**

Plasma insulin concentration was determined using Mercodia ultrasensitive rat insulin ELISA kits according to the manufacturers 50 μl sample protocol. Unknown samples were diluted 20 times using the zero standard prior to assay in duplicate. Optical density at 450 nm was determined using a Molecular Devices VERSAmax tunable microplate reader and readings transferred to GraphPad Prism. A cubic spline curve was fitted to the calibration data and unknown values extrapolated from this curve fit.

**NEFA assay**

Non-esterified (or free) fatty acids (NEFA) were determined by modification of a commercial clinical analyser assay kit (Wako NEFA C ACS-ACOD method) to a 96-well format. The supplied oleic acid standard (1 mM) was diluted in deionised water to create a series of standard concentrations from 50 μM to 1000 μM. Plasma samples were assayed after a 2 times dilution in deionised water. Standards and diluted plasma samples were pipetted into the plates (Alpha Laboratories, flat bottomed FX9200) as 10 μl additions in duplicate. Assay plates and reagents A and B were preheated at 37°C and the reaction started by adding 75 μl of reagent A followed by incubation at 37°C. After exactly 10 minutes, 150 μl of reagent B was added and the plate incubated for a further 10 minutes after which optical density at 540 nm was determined using a Molecular Devices VERSAmax tunable microplate reader. Optical density readings were then transferred to GraphPad Prism and values for plasma samples extrapolated from a linear regression fit to the standard curve. The assay was linear up to the highest standard employed (1000 μM).

**Glycerol and triacylglycerol assay**

Plasma triacylglycerol was determined by modification of a discrete assay kit (Sigma Triglyceride determination

kit, Cat No. TR0100) to a 96-well format. The kit actually determines liberated glycerol following enzymic hydrolysis of triacylglycerol to free fatty acids and glycerol (1 mole glycerol per mole of triacylglycerol). For this reason, correction for the glycerol concentration originally present in the plasma is required. Plasma samples were, therefore, assayed in the absence (free plasma glycerol) or presence (total triacylglycerol) of lipoprotein lipase. Subtraction of free glycerol from total triacylglycerol concentration then derives the true triacylglycerol concentration.

A 2.5 mg/ml triolein equivalent glycerol standard was obtained from Sigma. The supplied Sigma mM conversion factor for this batch was 1.13 (ie 2.83 mM glycerol). A 10 mg/ml glycerol stock solution was prepared in saline and then diluted in saline to create a series of standard concentrations from 0.218 to 4.34 mM. Standards and plasma samples (undiluted) were pipetted into the 96-well plates as 10 μl additions in duplicate. Assay plates and reagents were preheated at 37°C and the reaction started by adding 200 μl of either the glycerol or total triacylglycerol reagent (reconstituted as per manufacturer's protocol) followed by incubation at 37°C for 5 minutes after which optical density at 540 nm was determined using a Molecular Devices VERSAmax tunable microplate reader. Optical density readings were then transferred to GraphPad Prism and unknown values extrapolated from a linear regression fit to the standard curve. The assay was linear up to the highest glycerol standard employed (4.34 mM). Subtraction of the glycerol concentration in the plasma samples from the total triacylglycerol concentration derived the true triacylglycerol concentration.

**Leptin assay**

Plasma leptin concentration was determined using Assay Designs rat leptin enzyme immunometric assay kits (Cat No. 900-015) according to the manufacturer's regular protocol. Unknown samples were diluted 100 times using leptin assay buffer prior to assay in duplicate.

Optical density at 450 nm was determined using a Molecular Devices VERSAmax tunable microplate reader and readings transferred to GraphPad Prism. A second order polynomial curve was fitted to the calibration data and unknown values extrapolated from this curve fit.

**Determination of carcass fat content**

Total ether-extractable fat in the freeze-dried samples was estimated by the Soxhlet principle using a Foss Soxtec HT2 system. The protocol employed was based on the manufacturer's recommendations for meat samples, although freeze-dried samples obviated the necessity for either a fat hydrolysis step or mixing the sample with sand to ensure efficient extraction.

Samples of approximately 1 g were accurately weighed into 26 mm x 60 mm cellulose extraction thimbles which were then plugged with approximately 0.8 g cotton wool. Sample fat was extracted into pre-weighed (including a few boiling chips) standard metal extraction cups using 70 ml of petroleum ether at 40-60°C, a circulator temperature of 115°C and a cooling flow rate of 2 l/min. A boiling stage of 25 min was employed, followed by a 40 min wash stage. A 10 min drying stage sufficed to remove ether from the extraction cups.

Percentage fat in the freeze-dried sample was calculated as follows:

$$\% \text{ fat} = [100 \times (\text{final cup weight} - \text{initial cup weight})]/\text{sample weight}$$

Percentage fat in the original sample was calculated using the % water as follows:

$$\% \text{ fat in original sample} = \% \text{ fat} \times [(100 - \% \text{ water})/100]$$

**Determination of carcass protein content**

Total protein in the freeze-dried samples was estimated using the Kjeldahl principle. A micro-Kjeldahl procedure was performed using a Foss 2012 digestion block and a Foss 2200 distilling unit. The protocol used was based on the manufacturer's recommendations for meat samples.

Approximately 0.3 g of freeze-dried sample was accurately weighed into 100 ml digestion tubes (Foss UK Ltd) in racks of 12 tubes. Each rack included at least one blank tube to determine a reagent blank. To each tube was added two Kjeltabs CQ and one Antifoam S tablet followed by 10 ml of 98% sulphuric acid. Samples were digested at 420°C for 1 h to convert protein into ammonium sulphate. Once tubes had cooled, free ammonia was liberated by the addition of an excess of 40% NaOH and steam distilled into 30 ml of 4% boric acid indicator solution. Ammonia was then

determined by titration against volumetric grade 0.1 M HCl using a Brand 25 ml digital burette. Nitrogen and protein content of the samples were calculated as follows:

$$\% \text{ nitrogen} = [(\text{sample titration} - \text{blank titration}) \times 0.1401]/\text{sample weight (g)}$$

$$\% \text{ protein} = \% \text{ nitrogen} \times 6.25$$

**Determination of carcass ash content**

Total ash in the freeze-dried samples was estimated by determining the non-combustible ash remaining after firing samples at high temperatures using a Carbolite OAF 11/1 muffle furnace.

Squat form silica crucibles were prefired (600˚C) for at least 2 h to stabilise weights. Crucibles were allowed to cool (<300˚C) and transferred to silica gel drying cabinets. Crucibles were then weighed, approximately 1 g of sample added and the crucible reweighed. A number of empty crucibles were also included. Crucibles were fired at 600˚C for 4 h to convert samples to ash and then allowed to cool (<300˚C) prior to transfer to silica gel drying cabinets. Final weights were determined when crucibles had fully cooled.

Percentage ash was determined by expressing the residual sample weight after firing as a percentage of the original sample weight. The average variation in blank crucible weight was - 0.02 ± 0.04 mg (mean ± SEM, n=14) and individual weights varied by 0.5 mg or less.

**Results**

**Effects of COMPOUND B and COMPOUND A on glycaemic control**

Chronic effects of COMPOUND B (10 and 30 mg/kg po) and COMPOUND A (10 and 30 mg/kg po) on plasma insulin concentrations in obese rats given access to highly palatable food sources

The effects of COMPOUND A (10 and 30 mg/kg po) and COMPOUND B (10 and 30 mg/kg po) on plasma insulin levels are shown in Figure 14.

COMPOUND B (10 and 30 mg/kg po) reduced plasma insulin levels by 15% to 28% compared to the control group (Figure 14).

COMPOUND A (10 and 30 mg/kg po) significantly decreased plasma insulin levels by 38.9% and 41.8%, respectively compared to the vehicle-treated control group (Figure 14).

**Effects of COMPOUND B and COMPOUND A on plasma lipid concentrations**

Chronic effects of COMPOUND B (10 and 30 mg/kg po) and COMPOUND A (10 and 30 mg/kg po) on plasma NEFA concentrations in obese rats given access to highly palatable, food sources

The effects of repeated administration of COMPOUND B (10 and 30 mg/kg po) and COMPOUND A (10 and 30 mg/kg po) on plasma NEFA concentrations are shown in Figure 15.

COMPOUND B (30 mg/kg po) significantly decreased plasma NEFA levels by 24.4% compared to the vehicle-treated control group (Figure 15). The lower dose of this compound was, however, without effect on this lipid parameter. In contrast, despite the increased weight-loss and decreased adiposity resulting from repeated administration of COMPOUND A (10 and 30 mg/kg po), neither dose of this compound had any effect to reduce plasma NEFA levels (Figure 15).

Chronic effects of COMPOUND B (10 and 30 mg/kg po) and COMPOUND A (10 and 30 mg/kg po) on plasma triacylglycerol and glycerol concentrations in obese rats given access to highly palatable, food sources

The effects of repeated administration of COMPOUND B (10 and 30 mg/kg po) and COMPOUND A (10 and 30 mg/kg po) on plasma triacylglycerol and glycerol concentrations are shown in Figures 16 and 17.

COMPOUND B (30 mg/kg po) evoked highly significant reductions in the plasma concentrations of triacylglycerol of 52.1 % ($p < 0.001$) and plasma glycerol levels of 38.9% ($p < 0.01$), although treatment with the lower dose of 10 mg/kg was without effect on these lipid parameters (Figures 16 and 17).

COMPOUND A (30 mg/kg po) also significantly reduced plasma triacylglycerol concentrations by 42.0% ($p < 0.05$) and plasma glycerol levels by 33.6% ($p < 0.05$) (Figures 16 and 17). In spite of the fact that COMPOUND A (10 mg/kg po) induced a greater weight-loss and reduction in adiposity than COMPOUND B (30 mg/kg po), it had no

significant effect on plasma triacylglycerol or glycerol levels (Figures 16 and 17).

## Effects of COMPOUND B and COMPOUND A on bodyweight

Chronic effects of COMPOUND A (10 and 30 mg/kg po) and COMPOUND B (10 and 30 mg/kg po) on the body weights of obese rats given access to highly palatable, food sources

The body weights of the vehicle-treated control group slowly increased throughout the study (Figures 18 and 19).

Consistent with the moderate reductions of food intake described above, chronic administration of COMPOUND B (10 and 30 mg/kg po), evoked a small dose-related decreases in the body weights of these obese rats that were sustained throughout the duration of drug treatment (Figure 18). The body weight of animals given COMPOUND B (30 mg/kg po) did not reach the level of statistical significance compared to the control group until Day 6 and with a significant maximum decrease on Day 29 of 7.1% (Figure 18). The body weights of animals administered COMPOUND B (10 mg/kg po) tended to be lower than the vehicle-treated control group throughout the study (Figure 18), but these reductions did not reach statistical significance compared to the vehicle group with the exception of Day 3. At the end of the 28 day drug treatment period, the body weights of dietary-induced obese, female Wistar rats COMPOUND B (10 mg/kg po) were 4.0% lower than those of the vehicle-treated controls (Figure 18).

Chronic administration of COMPOUND A (10 and 30 mg/kg po) produced much greater dose-related decreases in body weight than COMPOUND B (Figure 19). The body weights of animals given either dose of COMPOUND A were significantly lower than the control group on Day 2 (ie 24 h after the first dose of the drug) and on every other day of the drug treatment period (Figure 19). Thus, at the end of the 28 day drug treatment period, the body weights of dietary-induced obese, female Wistar rats given COMPOUND A (10 and 30 mg/kg po) were 12.0% and 19.0% lower, respectively, than those of the vehicle-treated controls (Figure 19).

## Effects of COMPOUND B and COMPOUND A on adiposity

Chronic effects of COMPOUND A (10 and 30 mg/kq po) and COMPOUND B (10 and 30 mg/kq po) on the adiposity of obese rats given access to highly Palatable, macronutrient food sources

The effects of chronic administration of COMPOUND B and COMPOUND A for 28 days on body composition are shown in Figures 20 - 23 (fat, water, protein and ash content is expressed as g per rat).

Repeated administration with COMPOUND B (10 and 30 mg/kg po) evoked dose-related reductions in carcass fat, but this effect was only statistically significant at the higher dose (Figure 20). COMPOUND B (10 and 30 mg/kg po) produced a decreases in absolute body fat of 26.2 g and 11.1 g, respectively, compared to the vehicle-treated control group (Figure 20).

By comparison, COMPOUND A (10 and 30 mg/kg po) produced much larger decreases in body fat of 42.8 g and 67.8 g, respectively, compared to the vehicle-treated control group (Figure 20)

COMPOUND B (10 and 30 mg/kg po) and COMPOUND A (10 and 30 mg/kg po) did not significantly alter total water, protein or ash content of the carcasses (expressed as weight per rat) with the exception of COMPOUND A (30 mg/kg po) which slightly, but significantly, decreased total water (Figures 21 - 23)

Consistent with the relative reductions in body fat shown in Figure 20, plasma leptin levels were decreased by 13.7% (non-significant) and 26.2% after chronic treatment with COMPOUND B (10 and 30 mg/kg po) and by 36.1%, 61.7% after COMPOUND A (10 and 30 mg/kg po) when compared to the vehicle-treated control group (Figure 24).

## Pharmaceutical Example 4: Effect of repeated administration of COMPOUND C ((rac)-N-piperidinyl-5-(4-chlorophenyl)-1-(2,4-dichlorophenyl)-4,5-dihydro-1H-pyrazole-3-carboxamide) on drug-induced weight-gain

### METHODS

### Experiment in female Sprague-Dawley rats

#### Introduction

It is well known by those skilled in art that insulin and many of the drugs used to treat Type 1 and Type 2 diabetes cause weight-gain in subjects taking them (eg see review by Pijl & Meinders, 1996) and that such weight-gain can be deleterious to health and a significant cause of non-compliance to drug therapy. In this experiment, we have used the atypical antipsychotic, olanzapine, as a prototypical drug to cause weight-gain and obesity. The rationale for this choice was because olanzapine treatment causes a mean increase in body weight of ~5 kg, but the effect can be

much greater in some individuals, especially children and adolescents (Haapasalo-Pesu & Saarijarvi, 2001; Ratzoni et al, 2002), and in addition, treatment of psychiatric disorders with this antipsychotic has also been reported to cause dyslipidaemia and Type 2 diabetes (see Newcomer, 2005; Melkersson & Dahl, 2004). For these reasons, olanzapine was chosen to exemplify the beneficial effects of pyrazoline compounds of the general formula 1 exemplified by COMPOUND C ((rac)-N-piperidinyl-5-(4-chlorophenyl)-1-(2,4-dichlorophenyl)-4,5-dihydro-1H-pyrazole-3-carboxamide) to prevent or reduce insulin- or antidiabetic drug-induced weight-gain or obesity.

**Animals**

The experiment was performed on 40 female Sprague-Dawley rats (originally in the weight range 250-300 g). Animals were individually housed in polypropylene cages with metal grid floors. The animal room was maintained at a temperature of 21±4°C and 55±20% humidity and on a reverse phase light-dark cycle (lights off for 8 h from 09.30-17.30 h) during which time the room was illuminated by red light. Animals had free access to powdered high fat diet (VRF1 plus 20% lard). The diet was contained in a glass feeding jar with an aluminium lid with a 3-4 cm hole cut in it to allow access to the food. Animals were housed singly in polypropylene cages with wire grid floors to enable the food intake of each rat to be recorded. Polypropylene trays with cage pads were placed beneath each cage to detect any food spillage. Animals were accustomed to these new conditions (individual housing and the wire-grid floor cages) for at least 14 days before the start of the baseline readings.

**Experimental procedures for the feeding study**

**Approximately a week before the start of the study, animals were weighed (to the nearest 0.1 g using an electronic top-pan balance) and allocated into 4 weight-matched treatment groups, each containing 10 animals, ie vehicle (0.5% HPMC (hydroxyl-propyl-methylcellulose) 3ml/kg po), olanzapine (3 mg/kg po in 0.5% HPMC), COMPOUND C (30 mg/kg po in 0.5% HPMC) and olanzapine (3 mg/kg po in 0.5% HPMC), + COMPOUND C (30 mg/kg po in 0.5% HPMC). Following a 7 day baseline run-in period, during which the animals were dosed with orally with vehicle to acclimatise them to dosing/handling, the treatments above were then given once daily for 14 days.**

Food intake and body weight were measured once daily. Variations in body weight were accounted for by expressing the food intake results in terms of g/kg rat weight.

Statistical comparisons between the food intakes of the different treatment groups were made by analysis of covariance followed by multiple t tests (two-tailed) to compare each treatment group with the vehicle-treated controls. Food intake of the animals at baseline (ie average of Day -6 to 0) was used as the covariate. Standard errors of the mean (SEM) were calculated from the residuals of the statistical model.

Statistical comparisons between the body weights of the different treatment groups were made by analysis of covariance followed by multiple t tests (two-tailed) to compare each treatment group with the vehicle-treated controls. For the body weight data (body weights each day), the weights of the animals on Day 1 (ie immediately before the first drug treatment) were used as the covariate. Thus, means were adjusted for differences in body weight between the groups on Day 1 and standard errors of the mean (SEM) were calculated from the residuals of the statistical model. All mean body weight values on Day 1 are identical (and SEM are therefore zero) because the analysis of covariance adjusts them all to equal the 'grand mean' of the values of this day.

**RESULTS**

**Effects of COMPOUND C on bodyweight and on olanzapine-induced weight-gain in female Sprague-Dawley rats**

As shown in Figure 25, when olanzapine (3 mg/kg po) was administered once daily for a period of 14 days to female, Sprague-Dawley rats that were given access to a high-fat diet, it evoked a marked and sustained increase in the body weights of these rats when compared to the vehicle treated control (Figure 25). On Day 15, the mean weight of the olanzapine (3 mg/kg po)-treated group of rats was statistically ($p < 0.01$) greater than that of the vehicle-treated controls (Figure 25). In contrast, the group of rats given COMPOUND C (30 mg/kg po) alone showed a gradual decrease in body weight compared to the vehicle-treated controls that was prolonged and sustained (Figure 25). On Day 15, the mean weight of the COMPOUND C (30 mg/kg po)-treated group of rats was statistically ($p < 0.02$) lower than that of the vehicle-treated controls (Figure 25). When rats were orally administered the combination medicament comprising a fixed dose 30 mg/kg of COMPOUND C + 3 mg/kg of olanzapine, COMPOUND C not only prevented the weight-gain evoked by the atypical antipsychotic, but it also surprisingly reduced the body weights of the rats to less than those treated with vehicle alone, and in fact, to the same degree as those treated with COMPOUND C alone (Figure 25). On Day 15, the mean decrease in the body weights of the rats treated with COMPOUND C + olanzapine was statistically

lower than those treated with olanzapine (3 mg/kg po; $p < 0.001$) or vehicle ($p < 0.02$) and were not statistically different from those of the group of rats administered COMPOUND C (30 mg/kg po) alone (Figure 25).

**Effects of COMPOUND C on food intake and on olanzapine-induced hyperphagia in female Sprague-Dawley rats**

As shown in Figure 26, when olanzapine (3 mg/kg po) was administered once daily for a period of 14 days to female, Sprague-Dawley rats that were given access to a high-fat diet, it evoked a an increase in the food intake when compared to the vehicle-treated controls. The increase in daily food intake, which was particularly pronounced from Day 2 to Day 7 of olanzapine administration (Figure 26). The cumulative intake of food by the olanzapine (3 mg/kg po)-treated group of rats was statistically greater ($p < 0.05$) than that of the vehicle controls over the first week of the experiment (Figure 27). In contrast the group of rats given COMPOUND C (30 mg/kg po) alone markedly reduced their food intakes compared to the vehicle-treated controls (Figure 26). The cumulative intake of food by the COMPOUND C (30 mg/kg po)-treated group of rats was statistically lower ($p < 0.01$) than that of the vehicle controls over the first week of the experiment (Figure 27). When rats were orally administered the combination medicament comprising a fixed dose 30 mg/kg of COMPOUND C +3 mg/kg of olanzapine, COMPOUND C not only prevented the hyperphagia evoked by the atypical antipsychotic, but it also surprisingly reduced the food intake of the rats to less than those treated with vehicle alone, and in fact, to the same degree as those treated with COMPOUND C alone (Figure 26). The decrease in cumulative food consumption over the first week of administration with COMPOUND C + olanzapine was statistically lower than that of the groups of rats treated with olanzapine (3 mg/kg po; $p < 0.001$) or vehicle ($p < 0.05$) and was not statistically different from that of the group of rats administered COMPOUND C (30 mg/kg po) alone (Figure 27).

**DISCUSSION**

In **Example 1,** it was shown that COMPOUND C and COMPOUND A are good to moderately potent ligands of the rat CB1 receptor, whereas COMPOUND B has no pharmacologically relevant affinity for this receptor.

In **Example 2** (consisting of 3 separate experimental studies) and **Example 3,** experiments in normal growing rats and in cafeteria-fed obese rats have demonstrated that pyrazoline compounds of the general formula 1 described herein exemplified by COMPOUND C, ((rac)-N-piperidinyl-5-(4-chlorophenyl)-1-(2,4-dichlorophenyl)-4,5-dihydro-1H-pyrazole-3-carboxamide), COMPOUND A ((R)-N-piperidinyl-5-(4-chlorophenyl)-1-(2,4-dichlorophenyl)-4,5-dihydro-1H-pyrazole-3-carboxamide), and COMPOUND B ((S)-N-piperidinyl-5-(4-chlorophenyl)-1-(2,4-dichlorophenyl)-4,5-dihydro-1H-pyrazole-3-carboxamide), are viable as medicaments for the prophylaxis and/or treatment of impaired glucose tolerance/insulin resistance and/or Type 2 diabetes. Thus, COMPOUND C, COMPOUND A and COMPOUND B improve glycaemic control as demonstrated by 2 outcome measures. First, administration of COMPOUND C, COMPOUND A or COMPOUND B reduces plasma insulin concentrations, and second, COMPOUND C causes a decreased excursion of plasma insulin in response to a glucose challenge. Both are key indicators of insulin resistance and/or impaired glucose tolerance. These actions are predicted to be at least additive when used in a combination medicament with at least 1 of the "compounds to treat diabetes" described in the non-limiting list of examples.

As described above, increased circulating free fatty acids are believed to be key mediators of insulin resistance and impaired glucose tolerance which ultimately leads to the development of Type 2 diabetes (see reviews by Boden & Laakso, 2004 and Bays et al, 2004). Conversely, Type 2 diabetes is strongly associated with dyslipidaemia, which is characterised by raised plasma levels of small, dense LDL-cholesterol particles, elevated triglycerides and low concentrations of HDL-cholesterol particles (Boden & Laakso, 2004). As shown, by the experiments performed, chronic treatment with COMPOUND C, COMPOUND A and COMPOUND B significantly and markedly decreased the circulating concentrations of plasma triglycerides and glycerol and triacylglycerol in obese female rats demonstrating that the compound also has beneficial effects on this key metabolic driver of insulin resistance/impaired glucose tolerance and/or Type 2 diabetes. Other improvements in plasma lipids include a reduction in plasma FFAs in obese DIO male rats (a key driver of insulin resistance and a predisposing factor in the development of Type 2 diabetes) evoked by COMPOUND C and a reduction in plasma NEFAs in female DIO rats evoked by COMPOUND B. Together, these findings show that these compounds heve beneficial effects on many aspects of lipid abnormalities associated with impaired glycaemic control and dyslipaemia associated with insulin resistance/impaired glucose tolerance or Type 2 diabetes that will be of synergistic benefit with 1 or more "compounds to treat diabetes" in the said combination medicament.

Insulin resistance/impaired glucose tolerance and/or Type 2 diabetes are known to be associated with obesity (see reviews by Aronne & Segal, 2002, Pi-Sunyer et al, 2002 and Reaven et al, 2004), and in particular with visceral adiposity (see reviews by Aronne & Segal, 2002, Pi-Sunyer et al, 2002 and Reaven et al, 2004). When COMPOUND C, COMPOUND A and COMPOUND B were administered to rats for prolonged periods, they reduced body weight and the levels of adiposity as demonstrated by the body composition analyses of the DIO female rats and the accompanying fall in their plasma leptin concentrations. In terms of central adiposity, the visceral fat depots in the study performed in DIO male rats, COMPOUND C treatment is beneficial in decreasing the levels of visceral adiposity when the compound

is given repeatedly. Together, these findings show that these compounds have beneficial effects on many aspects of obesity and adiposity related to impaired glycaemic control and dyslipaemia associated with insulin resistance/impaired glucose tolerance or Type 2 diabetes that will be of synergistic benefit with 1 or more "compounds to treat diabetes" in the said combination medicament. This is especially the case first because insulin used to treat Type 1 and Type 2 and many of the commonly prescribed drugs used to treat Type 2 diabetes cause significant weight-gain that counter-balances the benefits that they provide in the treatment of these metabolic disorders.

In the list of that pyrazoline compounds of the general formula 1 described herein that are claimed in this filing, some are CB1 receptor antagonists or inverse agonists, as exemplified by COMPOUND C and COMPOUND A. CB1 receptor antagonists or inverse agonists will be of benefit by a weight-loss independent mechanism because the endocannabinoid system in patients with hyperglycaemia has been shown to be overactive (Matias et al, 2001). Since it has been shown in animal studies that cannabinoid receptors are known to be present on pancreatic β-cells where they regulate insulin secretion (Juan-Pico et al, 2006; Bermudez-Siva et al, 2006) and activation of CB1 receptors induces glucose intolerance (Bermudez-Siva et al, 2006), treatment with a CB1 receptor antagonist or inverse agonist will improve insulin resistance/impaired glucose tolerance and/or Type 2 diabetes by attenuating this aberrant signalling. Peripheral CB1 receptors are also present on adipocytes, and on adipocytes in visceral fat pads (Cota et al, 2003) and in liver where their activation by the over-active endocannabinoid signalling in Type 2 diabetes and obesity (Matias et al, 2001, Osei-Hyiaman et al, 2005; Cota et al, 2003) leads to lipogenesis in both tissues which in turn is a driver of increased insulin resistance/impaired glucose tolerance thereby exacerbating impaired diabetic status. Once again, treatment of a patient with a CB1 receptor antagonist or inverse agonist will improve insulin resistance/impaired glucose tolerance and/or Type 2 diabetes by attenuating this aberrant signalling. Together, these findings demonstrate that pyrazoline compounds of the general formula 1 described herein which are CB1 receptor antagonists or inverse agonists, will be of synergistic benefit with 1 or more "compounds to treat diabetes" in the said combination medicament as a result of their weight-loss independent peripheral actions improve glycaemic control and prevent obesity and adiposity.

In the list of pyrazoline compounds of the general formula 1 described herein that are claimed in this filing, COMPOUND B is clearly not either a CB1 receptor antagonist or inverse agonist in rats because it lacks pharmacologically relevant affinity for the rat CB1 receptor, ie it has an $IC_{50}$ value of 813 nM. The beneficial effect of COMPOUND B to decrease plasma NEFAs, triacylglycerol, glycerol and insulin levels is greater than that which can be accounted for by weight-loss alone. Dyslipidaemia is a major driver of insulin resistance/impaired glucose tolerance and the progression to Type 2 diabetes and it is clearly evident that COMPOUND B has weight-loss-independent benefits on these cardio-metabolic risk factors. Together, these findings demonstrate that pyrazoline compounds of the general formula 1 described herein, which are not CB1 receptor antagonists or inverse agonists, will be of synergistic benefit with 1 or more "compounds to treat diabetes" in the said combination medicament as a result of their weight-loss independent peripheral actions to decrease plasma NEFAs, triacylglycerol, glycerol and insulin levels and thereby improve glycaemic control.

Many of the therapeutic classes of compounds used to treat Type 1 and Type 2 diabetes including insulin preparations, sulphonylureas, meglitinides, PPARγ agonists, and potentially also inhibitors of hepatic glucose production, cause significant weight-gain in patients. In this filing we have used olanzapine, as a model compound to cause drug-induced weight-gain because it is a drug that is known not only to be one of the worst offenders amongst all drugs that cause weight increase (Pijl; & Meinders, 1996). The findings reported here show that pyrazoline compounds of the general formula 1 described herein as exemplified by COMPOUND C not only prevents the weight-gain that was evoked by olanzapine, but it also evokes weight-loss in the group of rats treated with a combination of COMPOUND C + olanzapine. Furthermore, the degree of weight-loss observed in the group of rats administered the COMPOUND C + olanzapine combination medicament was not significantly different from that observed when COMPOUND C was given alone. In defining the pharmacological mechanisms responsible for these effects, it is evident that the weight-gain observed with olanzapine treatment was caused by an increase in food consumption and the action of COMPOUND C to prevent this weight-gain and to cause weight-loss was due to a reduction in food intake to a level significantly lower than that of the vehicle-treated control group. Together, these findings demonstrate that a pyrazoline compound of the general formula 1 described herein will be of synergistic benefit with 1 or more "compounds to treat diabetes" that cause weight-gain in the said combination medicament as a result of its ability to prevent this side-effect. Moreover, the weight-loss evoked by the pyrazoline compound of the general formula 1 described herein in the said active substance combination medicament will be of especial benefit to overweight or obese subjects with Type 1 diabetes or insulin resistance/impaired glucose tolerance and/or Type 2 diabetes.

Because of the extreme magnitude of its action to evoke weight-gain, olanzapine was selected as a model drug to mimic the "worst case" weight-gain caused by insulin and various anti-diabetic drugs. The finding that the weight-loss produced by the combination medicament comprising E6776 + olanzapine was not different from that evoked by COMPOUND C alone clearly demonstrates that the pharmacological actions of olanzapine (in this case, dopamine $D_2$ and 5-hydroxytryptamine 5-$HT_2$ receptor antagonism) do not reduce the cardio-metabolic benefits evoked by COMPOUND C treatment. Insulin and the anti-diabetic drugs that cause weight-gain produce their clinical benefits by 1 of the following mechanisms, ie sulphonylureas ($K^+_{ATP}$ channel blockers), the meglitinides ($K^+_{ATP}$ channel blockers),

thiazolidinediones (peroxisome proliferator activated receptors type gamma [PPARγ] agonists, PPARγ/α agonist, PPARγ/α/δ agonists, glucagon antagonists, glucose-6-phosphatase inhibitors, glycogen phosphorylase inhibitors, insulin (agonism at insulin receptors), dual-acting insulin secretagogues/insulin sensitisers (pituitary adenylate cyclase-activating peptide/ vasoactive intestinal peptide receptor-2 [VPAC-2] receptor agonists), modulators of glucose uptake (GLUT-4 translocators, retinoid X receptor agonists, |K β kinase [IKK] β inhibitors, p38 MAP kinase inhibitors, protein tyrosine phosphatase 1 B [PTP-1 B] inhibitors, insulin receptor tyrosine kinase activators, a-glucosidase inhibitors, sodium/glucose co-transport inhibitors, glycogen synthase kinase-3 [GSK-3] inhibitors) and combinations thereof. The implication is that because neither insulin nor the anti-diabetic drugs act through CB1 agonism, any drug to treat diabetes selected for use in the said combination medicament will not attenuate the cardio-metabolic benefits of a pyrazoline compound of the general formula 1 described herein. Conversely, as neither insulin nor the anti-diabetic drugs that are known to produce weight-gain produce their therapeutic actions via the stimulation of CB1 receptors that are located either in the brain or in peripheral tissues, it can be concluded that the selected that pyrazoline compounds of the general formula 1 described herein will not detract from the clinical benefits provided the drug(s) selected for use in the said combination medicament.

In conclusion, therefore, on the evidence presented an active substance combination according to the invention comprising at least one pyrazoline compound of general formula 1 as described herein, together with 1 or more "compounds to treat diabetes" will be of synergistic benefit to patients who require treatment for their Type 1 diabetes or insulin resistance/impaired glucose tolerance and/or Type 2 diabetes. Some of the beneficial actions of pyrazoline compounds of the general formula 1 described herein on cardio-metabolic risk factors are mediated through weight-loss, and as a consequence, the said combination medicament will be synergistically beneficial to patients, who are overweight or obese. However, because other beneficial actions of the pyrazoline compounds of the general formula 1 described herein on cardio-metabolic risk factors are weight-loss-independent, the said combination medicament will also be synergistically beneficial to patients, who are non-obese, even if the anti-diabetic in the said medicament does not produce weight-gain. Since pyrazoline compounds of the general formula 1 described herein prevent drug-induced weight-gain and cause weight-loss, the said combination medicament will be synergistically beneficial to patients when the anti-diabetic in the said combination medicament produces weight-gain, especially if they already overweight or obese.

## REFERENCES


American Heart Association. Third Report of the National Cholesterol Education Program (NCEP) Expert Panel on Detection Evaluation, and Treatment of High Blood Cholesterol in Adults (Adult Treatment Panel III) Final Report. Circulation 2002;106:3143-3420.

Aronne LJ, Segal KR. Adiposity and fat distribution outcome measures: assessment and clinical implications. Obes Res 2002;10:14S-21S.

American Diabetes Association (ADA), National Institute of Diabetes, Digestive and Kidney Diseases (NIDDKD). Prevention or delay of Type 2 diabetes. Diabetes Care 2004;27:S47-S54.

Bays H, Mandarino L, DeFronzo RA. Mechanisms of endocrine disease. Role of the adipocyte, free fatty acids, and ectopic fat in pathogenesis of Type 2 diabetes mellitus: peroxisomal proliferator-activated receptor agonists provide a rational therapeutic approach. J Clin Endocrine Metab 2004;89:463-478.

Bermudez-Siva FJ, Serrano A, Diaz-Molina FJ, Sanchez Vera I, Juan-Pico P, Nadal A, Fuentes E, Rodriguez de Fonseca F. Activation of cannabinoid CB1 receptors induces glucose ntolerance in rats. Eur J Pharmacol. 2006;531: 282-4.

Boden G, Laakso M. Lipids and glucose in Type 2 diabetes. What is the cause and effect? Diabetes Care 2004;27:2253-2259.

Cota D, Marsicano G, Tschop M, Grubler Y, Flachskamm C, Schubert M, Auer D, Yassouridis A, Thone-Reineke C, Ortmann S, Tomassoni F, Cervino C, Nisoli E, Linthorst AC, Pasquali R, Lutz B, Stalla GK, Pagotto U. The endogenous cannabinoid system affects energy balance via central orexigenic drive and peripheral lipogenesis. J Clin Invest. 2003; 112:423-31.

Govaerts SJ, Hermans E, Lambert DM. Comparison of cannabinoid ligand affinities and efficacies in murine tissues and in transfected cells expressing human recombinant cannabinoid receptors. Eur J Pharm Sci. 2004; 23: 233 - 43.

Haapasalo-Pesu KM, Saarijarvi S. Olanzapine induces remarkable weight gain in adolescent patients. Eur Child Adolesc Psychiatry. 2001;10:205-8.

IDF consensus worldwide definition of the Metabolic Syndrome. IDF 2005. www.idf.org. Juan-Pico P, Fuentes E, Bermudez-Silva FJ, Javier Diaz-Molina F, Ripoll C, Rodriguez de Fonseca F, Nadal A. Cannabinoid receptors regulate Ca(2+) signals and insulin secretion in pancreatic β-cell. Cell Calcium. 2006;39:155-62.

Matias I, Gonthier MP, Orlando P, Martiadis V, De Petrocellis L, Cervino C, Petrosino S, Hoareau L, Festy F,

Pasquali R, Roche R, Maj M, Pagotto U, Monteleone P, Di Marzo V. Regulation, function, and dysregulation of endocannabinoids in models of adipose and β-pancreatic cells and in obesity and hyperglycemia. J Clin Endocrinol Metab. 2006;91:3171-80.

Melkersson K, Dahl ML. Adverse metabolic effects associated with atypical antipsychotics: literature review and clinical implications. Drugs. 2004;64:701-23.

Newcomer JW. Second-generation (atypical) antipsychotics and metabolic effects: a comprehensive literature review. CNS Drugs. 2005;19 Suppl 1:1-93.

Osei-Hyiaman D, DePetrillo M, Pacher P, Liu J, Radaeva S, Batkai S, Harvey-White J, Mackie K, Offertaler L, Wang L, Kunos G. Endocannabinoid activation at hepatic CB1 receptors stimulates fatty acid synthesis and contributes to diet-induced obesity. J Clin Invest. 2005;115:1298-305.

Pijl H, Meinders AE. Bodyweight change as an adverse effect of drug treatment. Mechanisms and management. Drug Saf. 1996;14:329-42.

Pi-Sunyer FX. The obesity epidemic: pathophysiology and consequences of obesity. Obes Res 2002;10:97S-104S.

Ratzoni G, Gothelf D, Brand-Gothelf A, Reidman J, Kikinzon L, Gal G, Phillip M, Apter A, Weizman R. Weight gain associated with olanzapine and risperidone in adolescent patients: a comparative prospective study. J Am Acad Child Adolesc Psychiatry. 2002;41:337-43.

Reaven G, Abbassi F, McLaughlin T. Obesity, insulin resistance and cardiovascular disease. J Clin Endocrine Metab 2004;207-223. Sheehan MT. Polycystic ovarian syndrome: diagnosis and management. Clin Med Res 2004;2: 13-27.

WHO: World Health Organization. Definition, diagnosis and classification of diabetes mellitus and its complications. Report of a WHO consultation 1999.

## Claims

**1.** Active substance combination comprising

(A) at least one substituted pyrazoline compound of general formula I

I

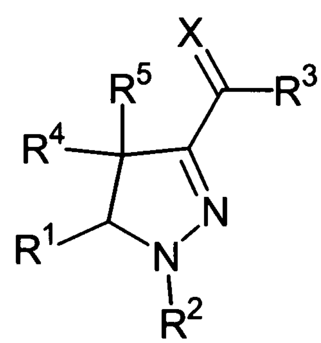

wherein

X is O or S;

$R^1$ and $R^2$, independently of one another, in each case represent an unsubstituted or at least mono-substituted aryl or heteroaryl radical, which may be condensed with an unsubstituted or at least mono-substituted mono- or polycyclic ring system;

or a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bridged by at least one unsubstituted or at least mono-substituted alkylene group;

$R^3$ an unsubstituted or at least mono-substituted aryl or heteroaryl radical, which may be condensed with an unsubstituted or at least mono-substituted mono- or polycyclic ring system;

a saturated or unsaturated, unsubstituted or at least mono-substituted, optionally at least one heteroatom

as a ring member containing cycloaliphatic radical, which may be condensed with an unsubstituted or at least mono-substituted mono- or polycyclic ring system and/or may be bridged by at least one unsubstituted or at least mono-substituted alkylene group;
a saturated or unsaturated, unsubstituted or at least mono-substituted, optionally at least one heteroatom as a ring member containing cycloaliphatic radical which together with a saturated or unsaturated, unsubstituted or at least mono-substituted, optionally at least one heteroatom as a ring member containing cycloaliphatic radical forms a saturated or unsaturated, unsubstituted or at least mono-substituted, optionally at least one heteroatom as a ring member containing spirocyclic residue via a common ring atom;
a -O-$R^6$ moiety; a -$NR^7R^8$ moiety or a -$NR^9$-O-$R^{10}$ moiety;
$R^4$ represents H; F; Cl; Br; I; -CN; -$NO_2$; -NC; -OH; -$NH_2$; -SH; -C(=O)-H; -C(=O)-OH; -C(=O)-$OR^{17}$;
a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
an unsubstituted or at least mono-substituted aryl or heteroaryl radical, which may be condensed with an unsubstituted or at least mono-substituted mono- or polycyclic ring system and/or may be bonded via an optionally at least mono-substituted alkylene group, alkenylene group or alkinylene group;
a -O-$R^{11}$ moiety; a -S-$R^{12}$ moiety; a -NH-$R^{13}$ moiety or a -$NR^{14}R^{15}$ moiety;
$R^5$ represents H; F; Cl; Br; I; -CN; -$NO_2$; -NC; -OH; -$NH_2$; -SH; -C(=O)-H; -C(=O)-OH; -C(=O)-$OR^{17}$;
a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;
a -O-$R^{11}$ moiety; a -S-$R^{12}$ moiety; a -NH-$R^{13}$ moiety or a -$NR^{14}R^{15}$ moiety;

or $R^4$ and $R^5$ together with the bridging carbon atom form a saturated or unsaturated, unsubstituted or at least mono-substituted, optionally at least one heteroatom as a ring member containing cycloaliphatic radical;

$R^6$ represents a hydrogen atom;
a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;
a -P(=O)($OR^{16}$)$_2$ moiety; a -C(=O)-$OR^{17}$ moiety; a -C(=O)-NH-$R^{18}$ moiety or a - C(=O)-$R^{19}$ moiety;
$R^7$ and $R^8$, independently of one another, in each case represent a hydrogen atom;
a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
a saturated or unsaturated, unsubstituted or at least mono-substituted, optionally at least one heteroatom as a ring member containing cycloaliphatic radical which together with a saturated or unsaturated, unsubstituted or at least mono-substituted, optionally at least one heteroatom as a ring member containing cycloaliphatic radical forms a saturated or unsaturated, unsubstituted or at least mono-substituted, optionally at least one heteroatom as a ring member containing spirocyclic residue via a common ring atom;

an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;

a $-P(=O)(OR^{16})_2$ moiety; a $-C(=O)-OR^{17}$ moiety; a $-C(=O)-NH-R^{18}$ moiety; a $-C(=O)-R^{19}$ moiety; a $-S(=O)_2-R^{20}$ moiety; or a $-NR^{21}R^{22}$ moiety;

$R^9$ represents hydrogen or a saturated or unsaturated, unsubstituted or at least mono-substituted aliphatic radical;

$R^{10}$, $R^{11}$, $R^{12}$, $R^{13}$, $R^{14}$, $R^{15}$ and $R^{20}$, independently of one another, in each case represent a saturated or unsaturated, unsubstituted or at least mono-substituted aliphatic radical;

a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;

or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;

$R^{16}$, $R^{17}$, $R^{18}$ and $R^{19}$, independently of one another, in each case represent a saturated or unsaturated, unsubstituted or at least mono-substituted aliphatic radical;

or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group; and

$R^{21}$ and $R^{22}$, independently of one another, in each case represent a hydrogen atom;

a saturated or unsaturated, unsubstituted or at least mono-substituted aliphatic radical;

a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group;

a saturated or unsaturated, unsubstituted or at least mono-substituted, optionally at least one heteroatom as a ring member containing cycloaliphatic radical which together with a saturated or unsaturated, unsubstituted or at least mono-substituted, optionally at least one heteroatom as a ring member containing cycloaliphatic radical forms a saturated or unsaturated, unsubstituted or at least mono-substituted, optionally at least one heteroatom as a ring member containing spirocyclic residue via a common ring atom;

or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;

optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

and

(B) at least one compound (B) used to treat diabetes.

2. Active substance combination according to claim 1, **characterized in that** for the compound (A) according to formula I

X is O or S;

$R^1$ and $R^2$, independently of one another, in each case represent an unsubstituted or at least mono-substituted 6- or 10-membered aryl radical, which may be condensed with an unsubstituted or at least mono-substituted mono- or polycyclic ring system;

an unsubstituted or at least mono-substituted 5- to 14-membered heteroaryl radical, which may be condensed with an unsubstituted or at least mono-substituted mono- or polycyclic ring system;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group;

or an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bridged by at least one unsubstituted or at least mono-substituted $C_{1-5}$

alkylene group;
$R^3$ represents an unsubstituted or at least mono-substituted 6- or 10-membered aryl radical, which may be condensed with an unsubstituted or at least mono-substituted mono- or polycyclic ring system;
an unsubstituted or at least mono-substituted 5- to 14-membered heteroaryl radical, which may be condensed with an unsubstituted or at least mono-substituted mono- or polycyclic ring system;
an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group;
an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group;
an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical which together with an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical forms an unsubstituted or at least mono-substituted spirocyclic residue via a common ring atom;
a -O-$R^6$ moiety; a -$NR^7R^8$ moiety or a -$NR^9$-O-$R^{10}$ moiety;
$R^4$ represents H; F; Cl; Br; I; -CN; -$NO_2$; -NC; -OH; -$NH_2$; -SH; -C(=O)-H; -C(=O)-OH; -C(=O)-$OR^{17}$;
an unsubstituted or at least mono-substituted $C_{1-16}$ alkyl radical, $C_{2-16}$ alkenyl radical or $C_{2-16}$ alkinyl radical;
an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$alkinylene group and/or may be bridged by at least one unsubstituted or at least mono-substituted $C_{1-5}$ alkylene group;
an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$alkenylene group or $C_{2-5}$alkinylene group and/or may be bridged by at least one unsubstituted or at least mono-substituted $C_{1-5}$ alkylene group;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$alkylene group, $C_{2-5}$alkenylene group or $C_{2-5}$ alkinylene group;
or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$alkylene group, $C_{2-5}$alkenylene group or $C_{2-5}$alkinylene group;
a -O-$R^{11}$ moiety; a -S-$R^{12}$ moiety; a -NH-$R^{13}$ moiety or a -$NR^{14}R^{15}$ moiety;
$R^5$ represents H; F; Cl; Br; I; -CN; -$NO_2$; -NC; -OH; -$NH_2$; -SH; -C(=O)-H; -C(=O)-OH; -C(=O)-$OR^{17}$;
an unsubstituted or at least mono-substituted $C_{1-16}$ alkyl radical, $C_{2-16}$ alkenyl radical or $C_{2-16}$ alkinyl radical;
an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group and/or may be bridged by at least one unsubstituted or at least mono-substituted $C_{1-5}$alkylene group;
an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bonded via an unsubstituted or at least mono-substituted $C_{1-5}$alkylene group, $C_{2-5}$ alkenylene group or $C_{2-5}$ alkinylene group and/or may be bridged by at least one unsubstituted or at least mono-substituted $C_{1-5}$ alkylene group;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;
or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$alkylene group, $C_{2-5}$ alkenylene group or $C_{2-5}$alkinylene group;
-O-$R^{11}$ moiety; a -S-$R^{12}$ moiety; a -NH-$R^{13}$ moiety or a -$NR^{14}R^{15}$ moiety;
or $R^4$ and $R^5$ together with the bridging carbon atom form an unsubstituted or at least mono-substituted $C_{3-16}$ cycloalkyl radical or $C_{4-16}$ cycloalkenyl radical;
$R^6$ represents a hydrogen atom;

an unsubstituted or at least mono-substituted $C_{1-16}$alkyl radical, $C_{2-16}$ alkenyl radical or $C_{2-16}$alkinyl radical;
an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group and/or may be bonded via an unsubstituted or at least mono-substituted $C_{1-5}$ alkylene group, $C_{2-5}$ alkenylene group or $C_{2-5}$ alkinylene group;
an unsubstituted or at least mono-substituted $C_{4-16}$ heterocycloalkyl radical or $C_{516}$ heterocycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group and/or may be bonded via an unsubstituted or at least mono-substituted $C_{1-5}$ alkylene group, $C_{2-5}$ alkenylene group or $C_{2-5}$ alkinylene group;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;
an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;
a $-P(=O)(OR^{16})_2$ moiety; a $-C(=O)-OR^{17}$ moiety; a $-C(=O)-NH-R^{18}$ moiety or a - $C(=O)-R^{19}$ moiety;
$R^7$ and $R^8$, independently of one another, in each case represent a hydrogen atom;
an unsubstituted or at least mono-substituted $C_{1-16}$ alkyl radical, $C_{2-16}$alkenyl radical or $C_{2-16}$alkinyl radical;
an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group and/or may be bonded via an unsubstituted or at least mono-substituted $C_{1-5}$alkylene group, $C_{2-5}$alkenylene group or $C_{2-5}$alkinylene group;
an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group and/or may be bonded via an unsubstituted or at least mono-substituted $C_{1-5}$ alkylene group, $C_{2-5}$alkenylene group or $C_{2-5}$alkinylene group;
an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical which together with an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical forms an unsubstituted or at least mono-substituted spirocyclic residue via a common ring atom;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$alkenylene group or $C_{2-5}$ alkinylene group;
an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$alkylene group, $C_{2-5}$alkenylene group or $C_{2-5}$alkinylene group;
a $-P(=O)(OR^{16})_2$ moiety; a $-C(=O)-OR^{17}$ moiety; a $-C(=O)-NH-R^{18}$ moiety; a - $C(=O)-R^{19}$ moiety; a $-S(=O)_2-R^{20}$ moiety; or a $-NR^{21}R^{22}$ moiety;
$R^9$ represents a hydrogen atom or an unsubstituted or at least mono-substituted $C_{116}$alkyl radical, $C_{2-16}$alkenyl radical or $C_{2-16}$alkinyl radical;
$R^{10}$, $R^{11}$, $R^{12}$, $R^{13}$, $R^{14}$, $R^{15}$ and $R^{20}$, independently of one another, in each case represent an unsubstituted or at least mono-substituted $C_{1-16}$alkyl radical, $C_{2-16}$alkenyl radical or $C_{2-16}$alkinyl radical;
an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group and/or may be bonded via an unsubstituted or at least mono-substituted $C_{1-5}$ alkylene group, $C_{2-5}$alkenylene group or $C_{2-5}$alkinylene group;
an unsubstituted or at least mono-substituted $C_{4-16}$ heterocycloalkyl radical or $C_{516}$ heterocycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group and/or may be bonded via an unsubstituted or at least mono-substituted $C_{1-5}$alkylene group, $C_{2-5}$alkenylene group or $C_{2-5}$ alkinylene group;

an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$alkylene group, $C_{2-5}$alkenylene group or $C_{2-5}$ alkinylene group; or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$alkylene group, $C_{2-5}$ alkenylene group or $C_{2-5}$ alkinylene group;

$R^{16}$, $R^{17}$, $R^{18}$ and $R^{19}$, independently of one another, in each case represent an unsubstituted or at least mono-substituted $C_{1-16}$alkyl radical, $C_{2-16}$alkenyl radical or $C_{2-16}$alkinyl radical;

or an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$alkylene group, $C_{2-5}$ alkenylene group or $C_{2-5}$ alkinylene group;

or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$alkinylene group; and

$R^{21}$ and $R^{22}$, independently of one another, in each case represent a hydrogen atom;

an unsubstituted or at least mono-substituted $C_{1-16}$alkyl radical, $C_{2-16}$ alkenyl radical or $C_{2-16}$ alkinyl radical;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_2$-$_5$alkinylene group;

an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$alkenylene group or $C_{2-5}$alkinylene group;

an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical which together with an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical forms an unsubstituted or at least mono-substituted spirocyclic residue via a common ring atom;

an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$alkylene group, $C_{2-5}$alkenylene group or $C_{2-5}$ alkinylene group; or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$alkylene group, $C_{2-5}$alkenylene group or $C_{2-5}$ alkinylene group;

whereby

the rings of the aforementioned ring system are in each case independently of one another 5- 6- or 7-membered and may in each case independently of one another optionally contain 1, 2 or 3 heteroatom(s) independently selected from the group consisting of nitrogen, oxygen and sulfur;

the aforementioned heteroaryl radicals in each case optionally contain 1, 2, 3 or 4 heteroatom(s) independently selected from the group consisting of nitrogen, oxygen and sulfur as ring member(s);

the aforementioned heterocycloalkyl radicals and heterocycloalkenyl radicals in each case optionally contain 1, 2, 3 or 4 heteroatom(s) independently selected from the group consisting of nitrogen, oxygen and sulfur as ring member (s);

optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

**3.** Active substance combination according to claim 1, **characterized in that** for the compound (A) according to formula I

X is O or S;

$R^1$ represents a radical selected from the group consisting of phenyl and thienyl (thiophenyl), which may optionally be substituted with 1, 2, 3, 4 or 5 substituent(s) independently selected from the group consisting of -OH, -O-$CH_3$, -O-$C_2H_5$, F, Cl, Br and I;

$R^2$ represents a phenyl radical, which may be substituted with 1, 2, 3, 4 or 5 substituent(s) independently selected from the group consisting of -OH, -O-$CH_3$, - O-$C_2H_5$, F, Cl, Br and I;

$R^3$ represents a radical selected from the group consisting of

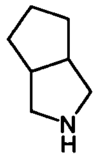 , 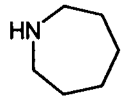 , 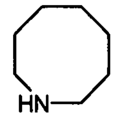 , 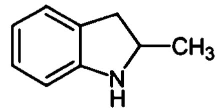 ,

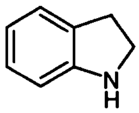 , 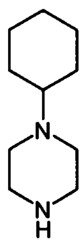 , 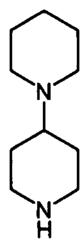 , 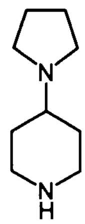 , 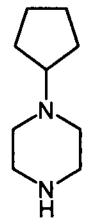 ,

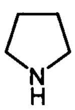 , 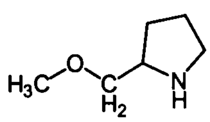 , 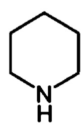 , 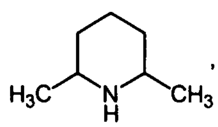 , 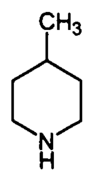 , 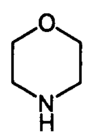 ,

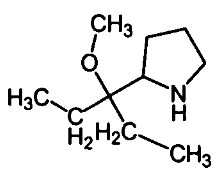 , 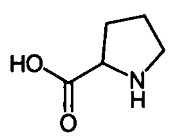 , 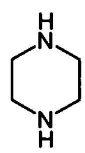 , 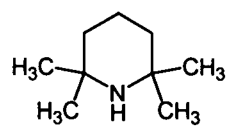 ,

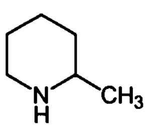 , 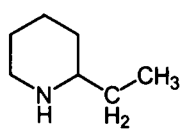 , 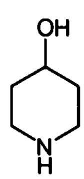 , 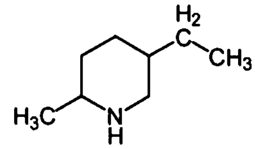 ,

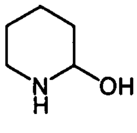 , 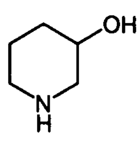 , 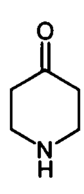 , 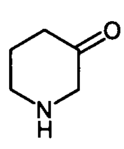 , 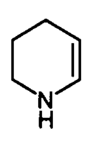 , 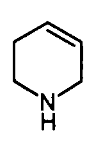 ,

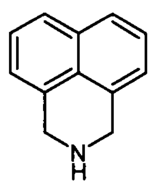 and 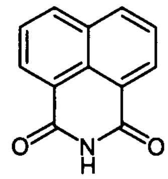 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;

a $-OR^6$-moiety or a $-NR^7R^8$ moiety;
$R^4$ represents H; F; Cl; Br; I; -OH, -CN; -C(=O)-H; -C(=O)-OH; $-C(=O)-OR^{17}$;
a radical selected from the group consisting of methyl, $-CF_3$, $-CH_2F$, $-CF_2H$, $-C_2F_5$, ethyl, $-CH_2-CN$, $-CH_2-OH$, n-propyl, isopropyl, $-CH_2-CH_2-CN$, $-CH_2-CH_2-OH$, n-butyl, $-CH_2-CH_2-CH_2-CN$, $-CH_2-CH_2-CH_2-OH$, isobutyl, sec-butyl, tert-butyl, n-pentyl, 2-pentyl, 3-pentyl, neo-pentyl, n-hexyl, 2-hexyl and 3-hexyl;
a benzyl radical or a $-O-R^{11}$ moiety;
$R^5$ represents H; F; Cl; Br;-C(=O)-OH; $-C(=O)-OR^{17}$; or a radical selected from the group consisting of methyl, ethyl, n-propyl, iso-propyl and n-butyl;
or $R^4$ and $R^5$ together with the bridging carbon atom form a radical selected from the group consisting of cyclopropyl, cyclobutyl, cyclopentyl, cyclohexyl, cycloheptyl, cyclooctyl, cyclopentenyl, cyclohexenyl, cycloheptenyl and cyclooctenyl;
$R^6$ represents a hydrogen atom; a radical selected from the group consisting of methyl, ethyl, n-propyl, isopropyl, n-butyl and tert-butyl or a radical selected from the group consisting of cyclopropyl, cyclobutyl, cyclopentyl, cyclohexyl and cycloheptyl;
$R^7$ represents a hydrogen atom or a radical selected from the group consisting of methyl, ethyl, n-propyl, isopropyl, n-butyl, sec-butyl and tert-butyl;
$R^8$ represents a radical selected from the group consisting of [1,2,3,4]-tetrahydronaphthyl, bicyclo[2.2.1]heptyl, cyclopropyl, cyclobutyl, cyclopentyl, cyclohexyl, cycloheptyl, cyclooctyl, cyclononyl, cyclodecyl, cycloundecyl and cyclododecyl, which may be bonded via a $-(CH_2)-$, $-(CH_2)-(CH_2)-$ or $-(CH_2)-(CH_2)-(CH_2)-$group and/or subsituted with 1, 2, 3 or 4 substituents selected from the group consisting of methyl, ethyl, n-propyl, isopropyl, n-butyl, sec-butyl, tert-butyl, -OH, $-O-CH_3$ and $-O-C_2H_5$;

or a radical selected from the group consisting of

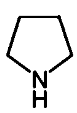 , 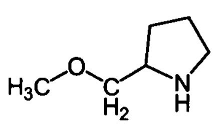 , 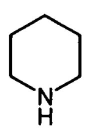 , 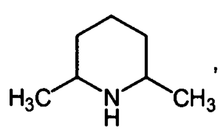 , 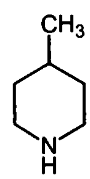 , 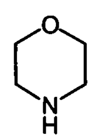 ,

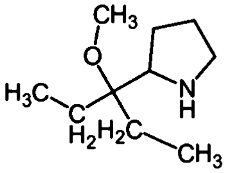 , 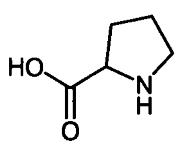 , 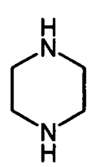 , 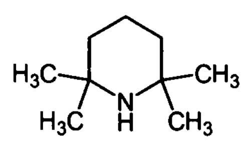 ,

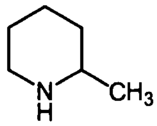, 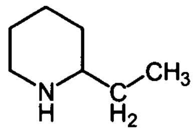, 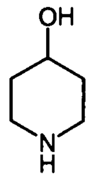, 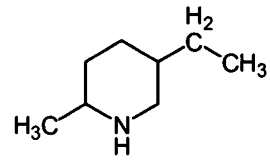,

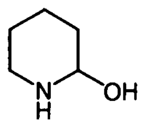, 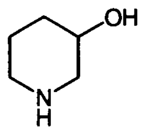, 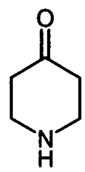, 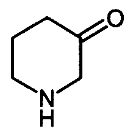, 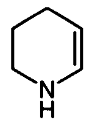, 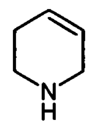,

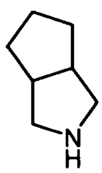, 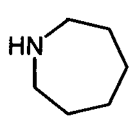, 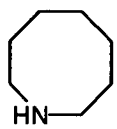, 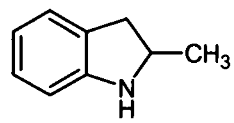,

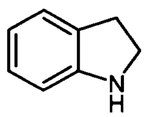, 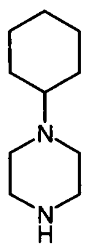, 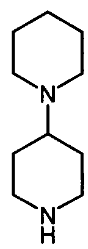, 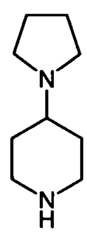, 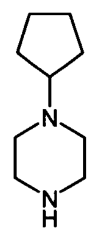,

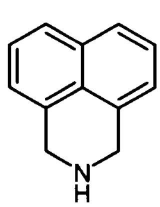 and 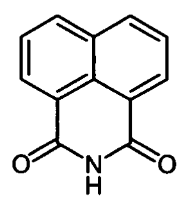 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
and
$R^{11}$ and $R^{17}$, independently of one another, each represent a radical selected from the group consisting of methyl, ethyl, n-propyl, isopropyl, n-butyl, isobutyl, sec-butyl, tert-butyl, n-pentyl, 2-pentyl, 3-pentyl and neo-pentyl;
optionally in form of one of its stereoisomers, preferably enantiomers or diastereomers, a racemate or in form of a mixture of at least two of its stereoisomers, preferably enantiomers and/or diastereomers, in any mixing ratio, or a

corresponding N-oxide thereof, or a physiologically acceptable salt thereof, or a corresponding solvate thereof.

**4.** Active substance combination according to claim 1, 2 or 3, **characterized in that** for the compound (A) of subgroup A1 according to formula I
X represents O
$R^1$ represents a phenyl ring, which is mono-substituted with a halogen atom, preferably a chlorine atom, in its 4-position,
$R^2$ represents a phenyl ring, which is di-substituted with two halogen atoms, preferably chlorine atoms, in its 2- and 4-position,
$R^3$ represents a pyrrolidinyl group; a piperidinyl group; a piperazinyl group; a homo-piperazinyl group; a morpholinyl group; or an -$NR^7R^8$-moiety,
$R^4$ represents a hydrogen atom,
$R^5$ represents a hydrogen atom
$R^7$ represents a hydrogen atom or a linear or branched $C_{1-6}$-alkyl group,
$R^8$ represents a linear or branched $C_{1-6}$ alkyl group; an $SO_2$-$R^{20}$-moiety; a pyrrolidinyl group; a piperidinyl group; a piperazinyl group; a homo-piperazinyl group; a morpholinyl group; or a triazolyl group, whereby each of the heterocyclic rings may be substituted with one or more, identical or different, $C_{1-6}$-alkyl groups, and
$R^{20}$ represents a phenyl group, which is optionally substituted with one or more $C_{1-6}$ alkyl groups, which may be identical or different,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

**5.** Active substance combination according to claim 4, **characterized in that** the compound (A) of subgroup A1 according to formula I is selected from the group consisting of:

N-piperidinyl-5-(4-chloro-phenyl)-1-(2,4-dichlorophenyl)-4,5-dihydro-1H-pyrazol-3-carboxamide,
5-(4-Chloro-phenyl)-1-(2,4-dichloro-phenyl)-4,5-dihydro-1H-pyrazole-3-carboxylic acid-[1,2,4]-triazole-4-yl-amide,
5-(4-Chloro-phenyl)-1-(2,4-dichloro-phenyl)-4,5-dihydro-1H-pyrazole-3-carboxylic acid-(4-methyl-piperazin-1-yl)-amide,
5-(4-Chloro-phenyl)-1-(2,4-dichloro-phenyl)-4,5-dihydro-1H-pyrazole-3-carboxylic acid diethylamide,
[5-(4-Chloro-phenyl)-1-(2,4-dichloro-phenyl)-4,5-dihydro-1H-pyrazole-3-yl]-piperidine-1-yl-methanone,
N-[5-(4-Chloro-phenyl)-1-(2,4-dichlorophenyl)-4,5-dihydro-1H-pyrazole-3-carbonyl]-4-methylphenylsulfonamide,
optionally in the form of a corresponding N-oxide, a corresponding salt or a corresponding solvate.

**6.** Active substance combination according to claim 1, 2 or 3, **characterized in that** for the compound (A) of subgroup A2 according to formula Ia or Ib

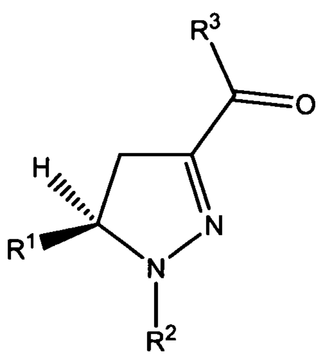

Ia

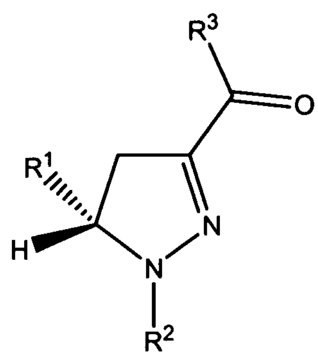

Ib

wherein
X represents O

$R^1$ represents a phenyl ring, which is mono-substituted with a halogen atom, preferably a chlorine atom, in its 4-position,
$R^2$ represents a phenyl ring, which is di-substituted with two halogen atoms, preferably chlorine atoms, in its 2- and 4-position,
$R^3$ represents a pyrrolidinyl group, a piperidinyl group, a piperazinyl group, a homo-piperazinyl group, a morpholinyl group, or an -$NR^7R^8$-moiety,
$R^4$ represents a hydrogen atom,
$R^5$ represents a hydrogen atom,
$R^7$ represents a hydrogen atom or a linear or branched $C_{1-6}$-alkyl group,
$R^8$ represents a linear or branched $C_{1-6}$ alkyl group; an -$SO_2$-$R^{20}$-moiety; a pyrrolidinyl group; a piperidinyl group; a piperazinyl group; a homo-piperazinyl group; a morpholinyl group; or a triazolyl group whereby each of the heterocyclic rings may be substituted with one or more, identical or different, $C_{1-6}$-alkyl groups, and
$R^{20}$ represents a phenyl group, which is optionally substituted with one or more $C_{1-6}$ alkyl groups, which may be identical or different,
optionally in form of a corresponding N-oxide thereof, or a corresponding salt thereof, or a corresponding solvate thereof.

**7.** Active substance combination according to claim 6, **characterized in that** the compound (A) of subgroup A2 is a compound selected from:

- (R)-N-piperidinyl-5-(4-chlorophenyl)-1-(2,4-dichlorophenyl)-4,5-dihydro-1H-pyrazole-3-carboxamide and
- (S)-N-piperidinyl-5-(4-chlorophenyl)-1-(2,4-dichlorophenyl)-4,5-dihydro-1H-pyrazole-3-carboxamide;

optionally in form of a corresponding N-oxide thereof, or a corresponding salt thereof, or a corresponding solvate thereof.

**8.** Active substance combination according to claim 1, 2 or 3, **characterized in that** for the compound (A) of subgroup A3 according to formula I

X is O;
$R^1$ represents a radical selected from the group consisting of

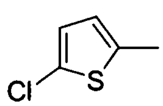 , 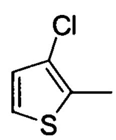 , 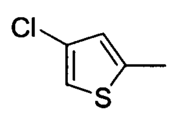 , 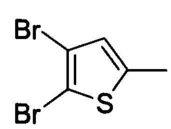 , 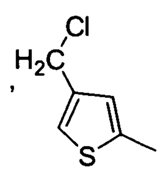 ,

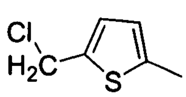 , 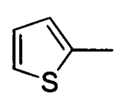 , 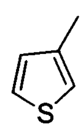 , 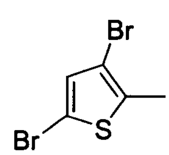 , 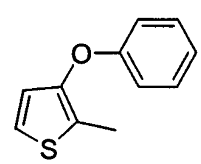 ,

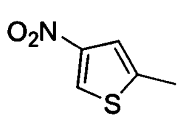 , 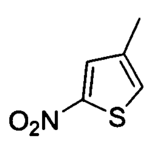 , 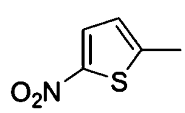 , 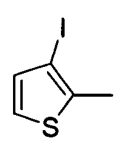 , 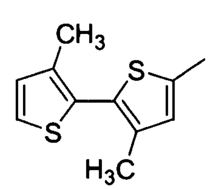 ,

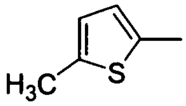 , 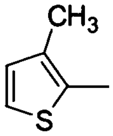 , 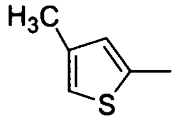 , 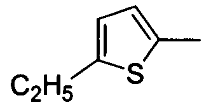 , 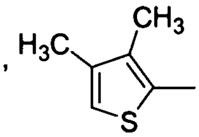 ,

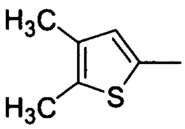 , 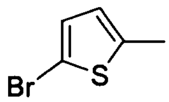 , 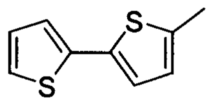 , 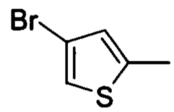 ,

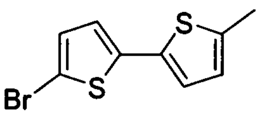 , 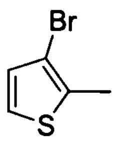 , 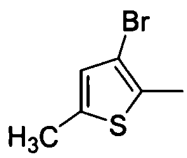 , 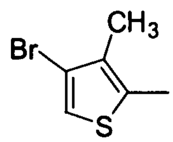 ,

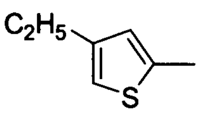 , 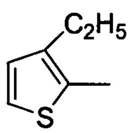 , 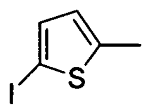 and 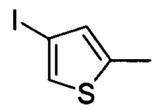 ;

wherein the single line in each case represents the bond to the pyrazoline compound of general formula I;
$R^2$ represents a phenyl radical, which may be substituted with 1, 2, 3, 4 or 5 substituent(s) independently selected from the group consisting of F, Cl, Br and I;
$R^3$ represents a radical selected from the group consisting

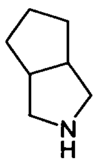 , 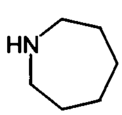 , 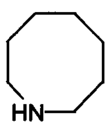 , 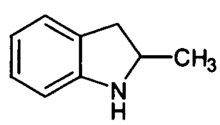 ,

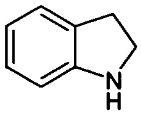 , 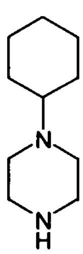 , 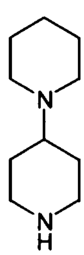 , 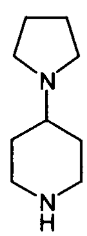 ,

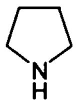 , 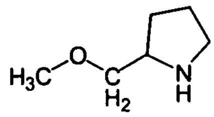 , 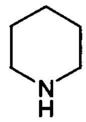 , 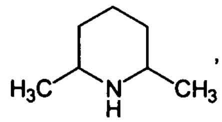 , 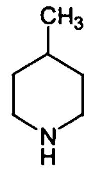 ,

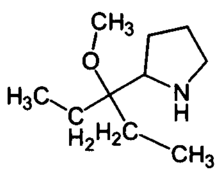 , 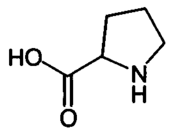 , 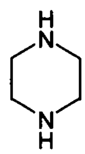 , 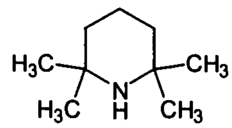 ,

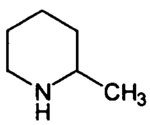 , 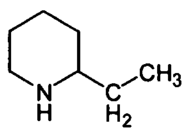 , 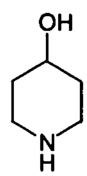 and 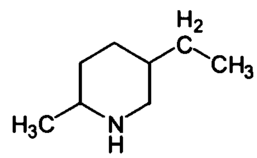 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
a -O-$R^6$ moiety or a -$NR^7R^8$-moiety;
$R^4$ represents a hydrogen atom;
$R^5$ represents a hydrogen atom;
$R^6$ represents a hydrogen atom, a radical selected from the group consisting of methyl, ethyl, n-propyl, isopropyl, n-butyl and tert-butyl or a radical selected from the group consisting of cyclopropyl, cyclobutyl, cyclopentyl, cyclohexyl and cycloheptyl;
$R^7$ represents a hydrogen atom;
$R^8$ a radical selected from the group consisting of cyclopropyl, cyclobutyl, cyclopentyl, cyclohexyl and cycloheptyl, which may be bonded via a $-(CH_2)-$, $-(CH_2)-(CH_2)-$ or $-(CH_2)-(CH_2)-(CH_2)-$group ;

or a radical selected from the group consisting of

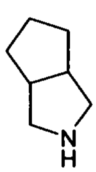 , 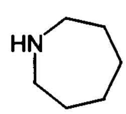 , 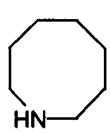 , 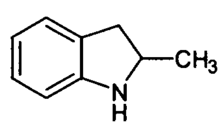 ,

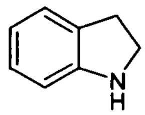, 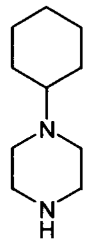, 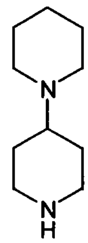, 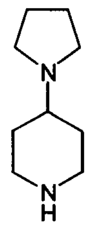,

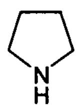, 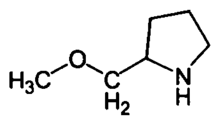, 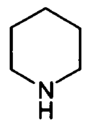, 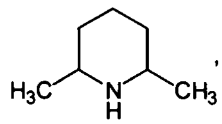, 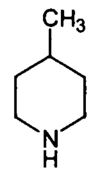,

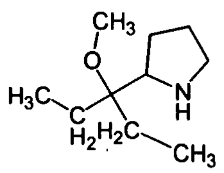, 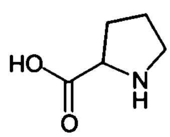, 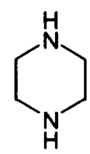, 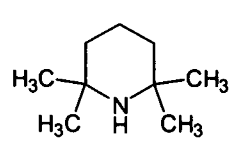,

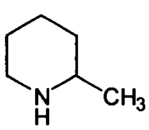, 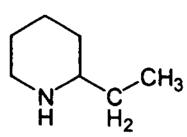, 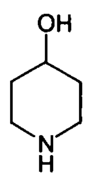 and 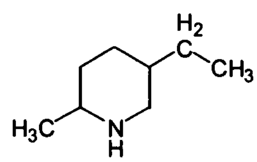;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

**9.** Active substance combination according to claim 8, **characterized in that** the compound (A) of subgroup A3 is a compound selected from:

[1] 5-(5-Chloro-thiophen-2-yl)-1-(2,4-dichloro-phenyl)-4,5-dihydro-1H-pyrazole-3-carboxylic acid piperidin-1-ylamide
[2] 5-(5-Chloro-thiophen-2-yl)-1-(2,4-dichloro-phenyl)-4,5-dihydro-1H-pyrazole-3-carboxylic acid azepan-1-ylamide
[3] 5-(5-Chloro-thiophen-2-yl)-1-(2,4-dichloro-phenyl)-4,5-dihydro-1H-pyrazole-3-carboxylic acid pyrrolidin-1-ylamide
[4] 5-(5-Chloro-thiophen-2-yl)-1-(2,4-dichloro-phenyl)-4,5-dihydro-1H-pyrazole-3-carboxylic acid cyclohexylamide
[5] [5-(5-Chloro-thiophen-2-yl)-1-(2,4-dichloro-phenyl)-4,5-dihydro-1H-pyrazol-3-yl]-pyrrolidin-1-yl-methanone
[6] [5-(5-Chloro-thiophen-2-yl)-1-(2,4-dichloro-phenyl)-4,5-dihydro-1H-pyrazol-3-yl]-(4-cyclohexyl-piperazin-1-yl)-methanone
[7] 5-(5-Chloro-thiophen-2-yl)-1-(2,4-dichloro-phenyl)-4,5-dihydro-1H-pyrazole-3-carboxylic acid cyclohexylamide
[8] 5-(5-Chloro-thiophen-2-yl)-1-(2,4-dichloro-phenyl)-4,5-dihydro-1H-pyra zole-3-carboxylic acid
[9] 5-(5-Chloro-thiophen-2-yl)-1-(2,4-dichloro-phenyl)-4,5-dihydro-1H-pyrazole-3-carboxylic acid methyl ester
[10] 5-(5-Chloro-thiophen-2-yl)-1-(2,4-dichloro-phenyl)-4,5-dihydro-1H-pyrazole-3-carboxylic acid ethyl ester
[11] 5-(5-Chloro-thiophen-2-yl)-1-(2,4-dichloro-phenyl)-4,5-dihydro-1H-pyrazole-3-carboxylic acid cyclohexyl ester
[12] 5-(3-Chloro-thiophen-2-yl)-1-(2,4-dichloro-phenyl)-4,5-dihydro-1H-pyrazole-3-carboxylic acid piperidin-1-ylamide
[13] 5-(5-Bromo-thiophen-2-yl)-1-(2,4-dichloro-phenyl)-4,5-dihydro-1H-pyrazole-3-carboxylic acid piperidin-1-ylamide
[14] 5-(5-Bromo-thiophen-2-yl)-1-(2,4-dichloro-phenyl)-4,5-dihydro-1H-pyrazole-3-carboxylic acid (4-methyl-piperidin-1-yl)-amide
[15] 5-(5-Bromo-thiophen-2-yl)-1-(2,4-dichloro-phenyl)-4,5-dihydro-1H-pyrazole-3-carboxylic acid (hexahydro-cyclopenta[c]pyrrol-2-yl)-amide
[16] 5-(5-Bromo-thiophen-2-yl)-1-(2,4-dichloro-phenyl)-4,5-dihydro-1H-pyrazole-3-carboxylic acid (2,3-dihydroindol-1-yl)-amide
[17] 5-(5-Bromo-thiophen-2-yl)-1-(2,4-dichloro-phenyl)-4,5-dihydro-1H-pyrazole-3-carboxylic acid cyclopropylamide
[18] 5-(5-Bromo-thiophen-2-yl)-1-(2,4-dichloro-phenyl)-4,5-dihydro-1H-pyrazole-3-carboxylic acid cyclohexyl-methyl-amide
[19] 5-(5-Bromo-thiophen-2-yl)-1-(2,4-dichloro-phenyl)-4,5-dihydro-1H-pyrazole-3-carboxylic acid
[20] 5-(5-Bromo-thiophen-2-yl)-1-(2,4-dichloro-phenyl)-4,5-dihydro-1H-pyrazole-3-carboxylic acid cyclohexyl ester
[21] 5-(5-Bromo-thiophen-2-yl)-1-(2,4-dichloro-phenyl)-4,5-dihydro-1H-pyrazole-3-carboxylic acid methyl ester
[22] 5-(5-Bromo-thiophen-2-yl)-1-(2,4-dichloro-phenyl)-4,5-dihydro-1H-pyrazole-3-carboxylic acid ethyl ester
[23] 5-(4-Bromo-thiophen-2-yl)-1-(2,4-dichloro-phenyl)-4,5-dihydro-1H-pyrazole-3-carboxylic acid piperidin-1-ylamide hydrochloride
[24] 5-(4-Bromo-thiophen-2-yl)-1-(2,4-dichloro-phenyl)-4,5-dihydro-1H-pyrazole-3-carboxylic acid azepan-1-ylamide
[25] 5-(4-Bromo-thiophen-2-yl)-1-(2,4-dichloro-phenyl)-4,5-dihydno-1H-pyrazole-3-carboxylic acid (4-methyl-piperidin-1-yl)-amide
[26] [5-(4-Bromo-thiophen-2-yl)-1-(2,4-dichloro-phenyl)-4,5-dihydro-1H-pyra zol-3-yl]-piperidin-1-yl-methanone
[27] 5-(4-Bromo-thiophen-2-yl)-1-(2,4-dichloro-phenyl)-4,5-dihydro-1H-pyrazole-3-carboxylic acid
[28] 5-(4-Bromo-thiophen-2-yl)-1-(2,4-dichloro-phenyl)-4,5-dihydro-1H-pyrazole-3-carboxylic acid methyl ester
[29] 5-(4-Bromo-thiophen-2-yl)-1-(2,4-dichloro-phenyl)-4,5-dihydro-1H-pyrazole-3-carboxylic acid ethyl ester
[30] 5-(3-Bromo-thiophen-2-yl)-1-(2.4-dichloro-phenyl)-4,5-dihydro-1H-pyrazole-3-carboxylic acid piperidin-1-ylamide
[31] 5-(3-Bromo-5-methyl-thiophen-2-yl)-1-(2.4-dichloro-phenyl)-4.5-dihydro-1H-pyrazole-3-carboxylic acid piperidin-1-ylamide
[32] 5-(4-Bromo-3-methyl-thiophen-2-yl)-1-(2,4-dichloro-phenyl)-4,5-dihydro-1H-pyrazole-3-carboxylic acid piperidin-1-ylamide
[33] 5-(4.5-Dibromo-thiophen-2-yl)-1-(2,4-dichloro-phenyl)-4,5-dihydlro-1H-pyrazote-3-carboxylic acid piperidin-1-ylamide
[34] 5-(3,5-Dibromo-thiophen-2-yl)-1-(2 ,4-dichloro-phenyl)-4,5-dihydro-1 H-pyrazole-3-carboxylic acid piperidin-1-ylamide
[35] 1-(2,4-Dichloro-phenyl)-5-(3-iodo-thiophen-2-yl)-4,5-dihydro-1H-pyrazole-3-carboxylic acid piperidin-1-ylamide
[36] 5-(4-Chloromethyl-thiophen-2-yl)-1-(2,4-dichloro-phenyl)-4,5-dihydro-1H-pyrazole-3-carboxylic acid piperidin-1-ylamide
[37] 1-(2,4-Dichloro-phenyl)-5-(3,4-dimethyl-thiophen-2-yl)-4,5-dihydro-1H-pyrazole-3-carboxylic acid piperidin-1-ylamide
[38] 1-(2,4-Dichloro-phenyl)-5-(4,5-dimethyl-thiophen-2-yl)-4,5-dihydro-1H-pyrazole-3-carboxylic acid piperidin-1-ylamide
[39] 5-[2,2']Bithiophenyl-5-yl-1-(2,4-dichloro-phenyl)-4,5-dihydro-1H-pyrazole-3-carboxylic acid piperidin-1-ylamide

[40] 1-(2,4-Dichloro-phenyl)-5-(5-methyl-thiophen-2-yl)-4,5-dihydro-1H-pyrazole-3-carboxylic acid piperidin-1-ylamide
[41] 1-(2,4-Dichloro-phenyl)-5-(3-methyl-thiophen-2-yl)-4,5-dihydro-1H-pyrazole-3-carboxylic acid piperidin-1-ylamide
[42] 1-(2,4-Dichloro-phenyl)-5-(5-ethyl-thiophen-2-yl)-4,5-dihydro-1H-pyrazole-3-carboxylic acid piperidin-1-ylamide
[43] 1-(2,4-Dichloro-phenyl)-5-(3,3'-dimethyl-[2,2']bithiophenyl-5-yl)-4,5-dihydro-1H-pyrazole-3-carboxylic acid piperidin-1-ylamide
[44] 1-(2,4-Dichloro-phenyl)-5-(5-nitro-thiophen-2-yl)-4,5-dihydro-1H-pyrazole-3-carboxylic acid piperidin-1-ylamide
[45] 1-(2,4-Dichloro-phenyl)-5-(4-nitro-thiophen-2-yl)-4,5-dihydro-1H-pyrazole-3-carboxylic acid piperidin-1-ylamide
[46] 1-(2,4-Dichloro-phenyl)-5-thiophen-2-yl-4,5-dihydro-1H-pyrazole-3-carboxylic acid piperidin-1-ylamide
[47] 1-(2,4-Dichloro-phenyl)-5-thiophen-3-yl-4,5-dihydro-1H-pyrazole-3-carboxylic acid piperidin-1-ylamide and
[48] 1-(2,4-Dichloro-phenyl)-5-(5-nitro-thiophen-3-yl)-4,5-dihydro-1H-pyrazole-3-carboxylic acid piperidin-1-ylamide;

optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

**10.** Active substance combination according to claim 1, 2 or 3, **characterized in that** for the compound (A) of subgroup A4 according to formula I

X represents S;
$R^1$ represents a phenyl radical which may be substituted with 1, 2, 3, 4 or 5 substituent(s) independently selected from the group consisting of F, Cl, Br, I, $-O-CH_3$ and $-O-C_2H_5$;
$R^2$ represents a phenyl radical which may be substituted with 1, 2, 3, 4 or 5 substituent(s) independently selected from the group consisting of F, Cl, Br and I;
$R^3$ represents a radical selected from the group consisting of

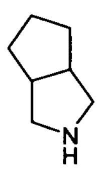 , 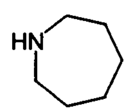 , 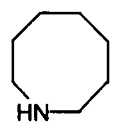 , 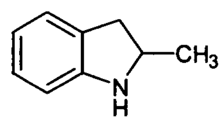 ,

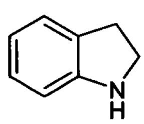 , 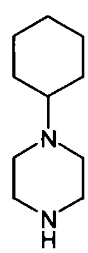 , 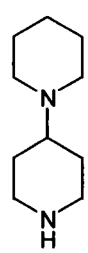 , 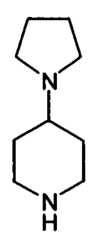 ,

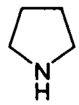 , 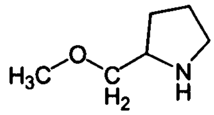 , 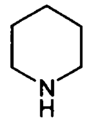 , 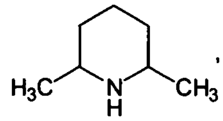 , 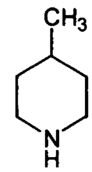 ,

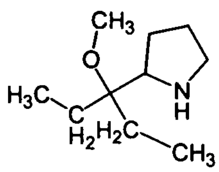 , 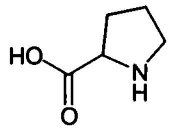 , 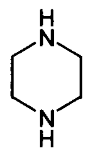 , 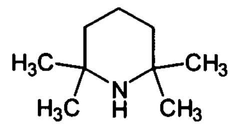 ,

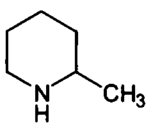 , 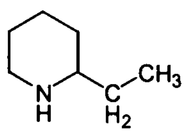 , 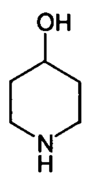 and 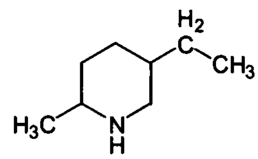 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
a -O-$R^6$ moiety or a -$NR^7R^8$-moiety;

$R^4$ represents a hydrogen atom;
$R^5$ represents a hydrogen atom;
$R^6$ represents a hydrogen atom or a radical selected from the group consisting of cyclopropyl, cyclobutyl, cyclopentyl, cyclohexyl and cycloheptyl;
$R^7$ represents a hydrogen atom;
$R^8$ a radical selected from the group consisting of cyclopropyl, cyclobutyl, cyclopentyl, cyclohexyl and cycloheptyl, which may be bonded via a - $(CH_2)$-, -$(CH_2)$-$(CH_2)$- or -$(CH_2)$-$(CH_2)$-$(CH_2)$-group;
or a radical selected from the group consisting of

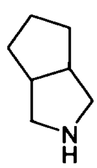 , 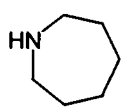 , 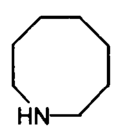 , 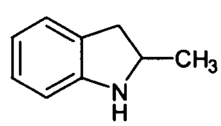 ,

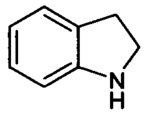, 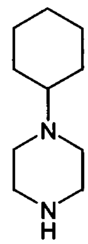, 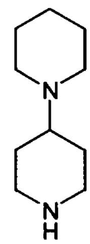, 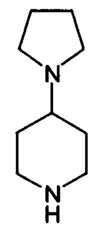,

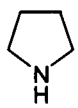, 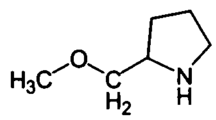, 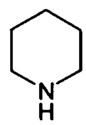, 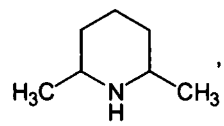, 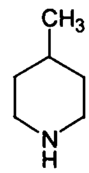,

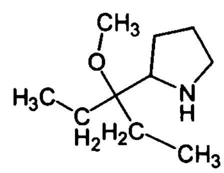, 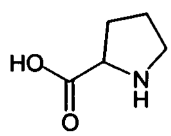, 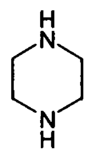, 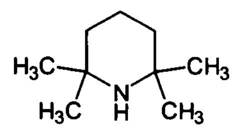,

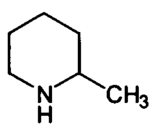, 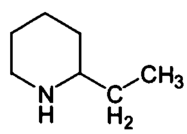, 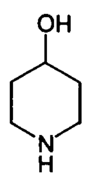 and 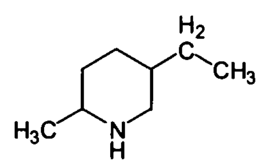;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;

optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

**11.** Active substance combination according to claim 10, **characterized in that** the compound (A) of subgroup A4 is a compound selected from:

[1] 5-(4-Chloro-phenyl)-1-(2,4-dichloro-phenyl)-4,5-dihydro-1H-pyrazole-3-carbothioic acid piperidin-1-ylamide
[2] 5-(4-Bromo-phenyl)-1-(2,4-dichloro-phenyl)-4,5-dihydro-1H-pyrazole-3-carbothioic acid piperidin-1-ylamide
[3] 1-(2,4-Dichloro-phenyl)-5-(4-iodo-phenyl)-4,5-dihydro-1H-pyrazole-3-carbothioic acid piperidin-1-ylamide
[4] 1-(2,4-Dichloro-phenyl)-5-(4-fluoro-phenyl)-4,5-dihydro-1H-pyrazole-3-carbothioic acid piperidin-1-ylamide
[5] 1-(2,4-Dichloro-phenyl)-5-(4-methoxy)-4.5-dihydro-1H-pyrazole-3-carbothioic acid piperidin-1-ylamide
[6] 5-(4-Chloro-phenyl)-1-(2,4-dichloro-phenyl)-4,5-dihydro-1H-pyrazole-3-carbothioic acid morpholin-4-ylamide
[7] 5-(4-Bromo-phenyl)-1-(2,4-dichloro-phenyl)-4,5-dihydro-1H-pyrazole-3-carbothioic acid morpholin-4-ylamide

[8] 1-(2,4-Dichloro-phenyl)-5-(4-iodo-phenyl)-4,5-dihydro-1H-pyrazole-3-carbothioic acid morpholin-4-ylamide
[9] 1-(2,4-Dichloro-phenyl)-5-(4-fluoro-phenyl)-4,5-dihydro-1H-pyrazole-3-carbothioic acid morpholin-4-ylamide
[10] 1-(2,4-Dichloro-phenyl)-5-(4-methoxy-phenyl)-4,5-dihydro-1H-pyrazole-3-carbothioic acid morpholin-4-ylamide
[11] 5-(4-Chloro-phenyl)-1-(2,4-dichbro-phenyl)-4,5-dihydro-1H-pyrazole-3-carbothioic acid (hexahydro-cyclopenta[c]pyrrol-2-yl)-amide
[12] 5-(4-Bromo-phenyl)-1-(2,4-dichloro-phenyl)-4,5-dihydro-1H-pyrazole-3-carbothioic acid (hexahydro-cyclopenta[c]pyrrol-2-yl)-amide
[13] 1-(2,4-Dichloro-phenyl)-5-(4-fluoro-phenyl)-4,5-dihydro-1H-pyrazole-3-carbothioic acid (hexahydro-cyclopenta[c]pyrrol-2-yl)-amide
[14] 1-(2,4-Dichloro-phenyl)-5-(4-iodo-phenyl)-4,5-dihydro-1H-pyrazole-3-carbothioic acid (hexahydro-cyclopenta[c]pyrrol-2-yl)amide
[15] 1-(2,4-Dichloro-phenyl)-5-(4-methoxy-phenyl)-4,5-dihydro-1H-pyrazole-3-carbothioic acid (hexahydro-cyclopenta[c]pyrrol-2-yl)-amide
[16] 1-(2,4-Dichloro-phenyl)-5-(4-iodo-phenyl)-4,5-dihydro-1H-pyrazole-3-carbothioic acid azepan-1-ylamide
[17] 5-(4-Chloro-phenyl)-1-(2,4-dichloro-phenyl)-4,5-dihydro-1H-pyrazole-3-carbothioic acid azepan-1-ylamide
[18] 5-(4-Bromo-phenyl)-1-(2,4-dichloro-phenyl)-4,5-dihydro-1H-pyrazole-3-carbothioic acid azepan-1-ylamide
[19] 1-(2,4-Dichloro-phenyl)-5-(4-fluoro-phenyl)-4,5-dihydro-1H-pyrazole-3-carbothioic acid azepan-1-ylamide
[20] 1-(2,4-Dichloro-phenyl)-5-(4-methoxy-phenyl)-4,5-dihydro-1H-pyrazole-3-carbothioic acid azepan-1-ylamide
[21] 1-(2,4-Dichloro-phenyl)-5-(4-methoxy-phenyl)-4,5-dihydro-1H-pyrazole-3-carbothioic acid pyrrolidin-1-ylamide
[22] 5-(4-Bromo-phenyl)-1-(2,4-dichloro-phenyl)-4,5-dihydro-1H-pyrazole-3-carbothioic acid pyrrolidin-1-ylamide
[23] 5-(4-Chloro-phenyl)-1-(2,4-dichloro-phenyl)-4,5-dihydro-1H-pyrazole-3-carbothioic acid pyrrolidin-1-ylamide
[24] 1-(2,4-Dichloro-phenyl)-5-(4-fluoro-phenyl)-4,5-dihydro-1H-pyrazole-3-carbothioic acid pyrrolidin-1-ylamide
[25] 1-(2,4-Dichloro-phenyl)-5-(4-iodo-phenyl)-4,5-dihydro-1H-pyrazole-3-carbothioic acid pyrrolidin-1-ylamide
[26] 1-(2,4-Dichloro-phenyl)-5-(4-methoxy-phenyl)-4,5-dihydro-1H-pyrazole-3-carbothioic acid (4-cyclopentyl-piperazin-1-yl)-amide
[27] 5-(4-Chloro-phenyl)-1-(2,4-dichloro-phenyl)-4,5-dihydro-1H-pyrazole-3-carbothioic acid (4-cyclopentyl-piperazin-1-yl)-amide
[28] 5-(4-Bromo-phenyl)-1-(2,4-dichloro-phenyl)-4,5-dihydro-1H-pyrazole-3-carbothioic acid (1H,3H-benzo[de]isoquinolin-2-yl)amide
[29] 5-(4-Chloro-phenyl)-1-(2,4-dichloro-phenyl)-4,5-dihydro-1 H-pyrazole-3-carbothioic acid (1 H,3H-benzo[de]isoquinolin-2-yl)-amide
[30] 5-(4-Chloro-phenyl)-1-(2,4-dichloro-phenyl)-4,5-dihydro-1 H-pyrazole-3-carbothioic acid cyclopentylamide
[31] Azocan-1-yl-[5-(4-chloro-phenyl)-1-(2,4-dichloro-phenyl)-4,5-dihydro-1 H-pyrazole-3-yl]-methanethione and
[32] [5-(4-Chloro-phenyl)-1-(2,4-dichloro-phenyl)-4,5-dihydro-1H-pyrazole-3-yl]-piperidin-1-yl-methanethione;

optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

**12.** Active substance combination according to claim 1, 2 or 3, **characterized in that** for the compound (A) of subgroup A5.1 according to formula I
X represents O
$R^1$ represents a phenyl radical that is substituted with a hydroxy, fluorine, chlorine, bromine or iodine atom or a -O-$CH_3$-group in the para-(4-)-position of the phenyl radical;
$R^2$ represents a (2,4)-dichloro-phenyl radical;
$R^3$ represents a radical selected from the group consisting of

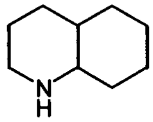 , 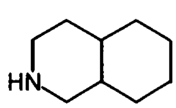 , 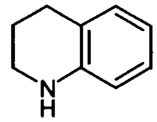 , 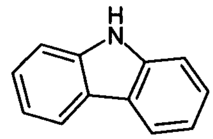 ,

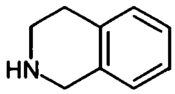 , 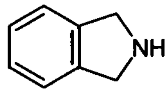 , 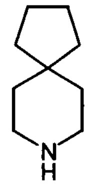 , 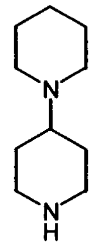 ,

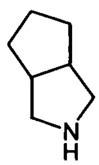 , 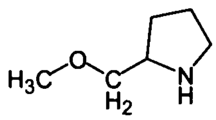 , 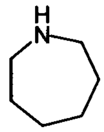 , 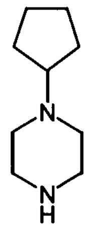 , 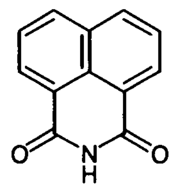 ,

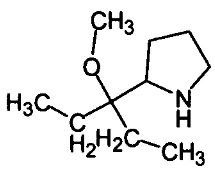 , 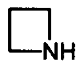 , 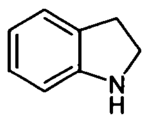 , 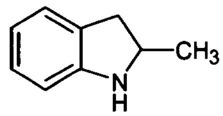 ,

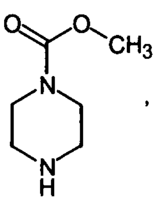 , 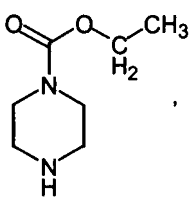 , 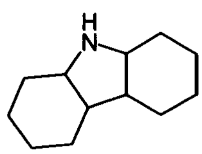 , 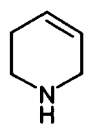 , 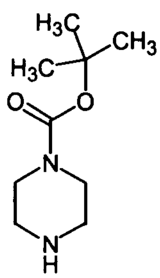 ,

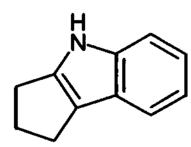 , 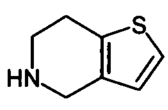 , 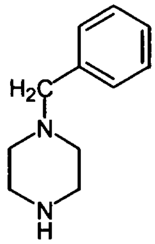 , 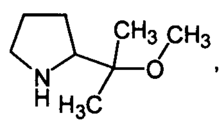 ,

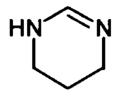 , 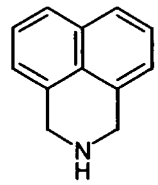 , 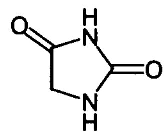 , 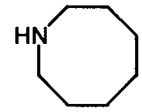 and 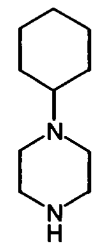 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
or a $-NR^7R^8$ moiety;
$R^4$ represents a hydrogen atom;
$R^5$ represents a hydrogen atom;
$R^7$ represents a hydrogen atom;
$R^8$ represents a radical selected from the group consisting of 2-heptyl, 3-heptyl, 4-heptyl, 2-octyl, 3-octyl, 4-octyl, $-CH_2-N(CH_3)_2$, $-CH_2-N(C_2H_5)_2$, $-CH_2-CH_2-N(CH_3)_2$, $-CH_2-CH_2-N(C_2H_5)_2$, $-CH_2-CH_2-CH_2-N(CH_3)_2$ and $-CH_2-CH_2-CH_2-N(C_2H_5)_2$;
a radical selected from the group consisting of adamantyl, bicyclo[2.2.1]heptyl, bicyclo[3.1.1]heptyl, (1,7,7)-trimethyl-bicyclo[2.2.1]heptyl, [1,2,3,4]-tetrahydronaphthyl, cyclononyl, cyclodecyl, cycloundecyl and cyclododecyl;
a radical selected from the group consisting of cyclopropyl, cyclobutyl, cyclopentyl, cyclohexyl, cycloheptyl, cyclooctyl, pyrrolidinyl, piperidinyl, piperazinyl, homopiperazinyl, morpholinyl, azepanyl, diazepanyl and azocanyl, which is bonded via a $-(CH_2)-$, $-(CH_2)-(CH_2)-$ or $-(CH_2)-(CH_2)-(CH_2)-$group;
a substituted radical selected from the group consisting of cyclopentyl, cyclohexyl and cyloheptyl which is substituted with a -O-Benzyl radical;
or a radical selected from the group consisting of

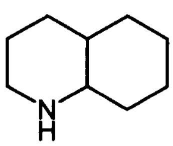 , 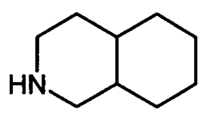 , 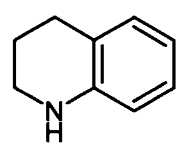 ,

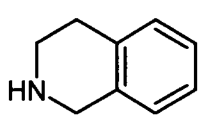 , 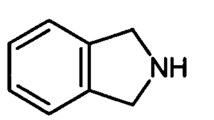 , 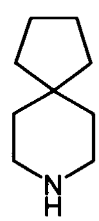 , 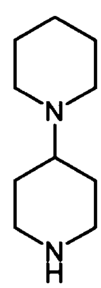 ,

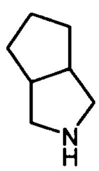 , 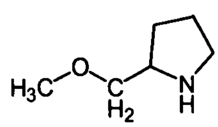 , 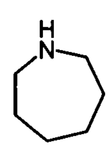 , 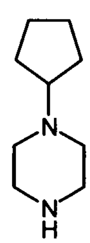 ,

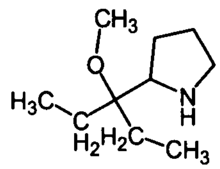 , 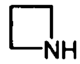 , 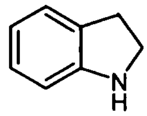 , 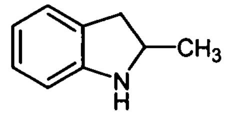 ,

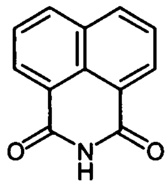 , 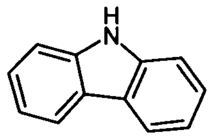 , 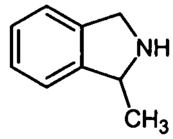 ,

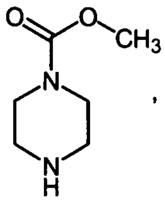 , 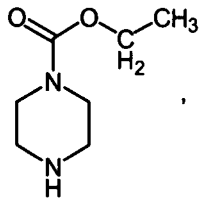 , 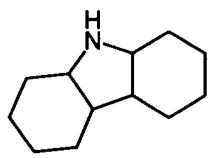 , 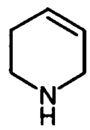 , 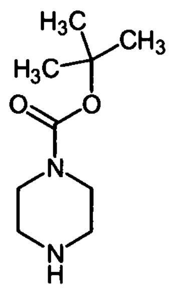 ,

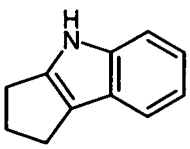 , 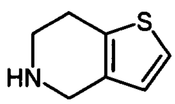 , 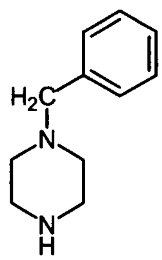 , 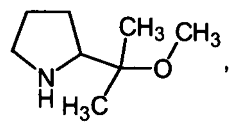 ,

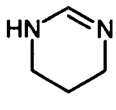 , 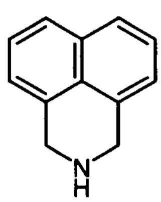 , 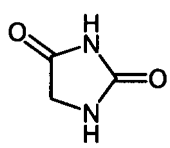 , 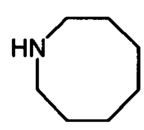 and 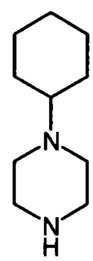 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

acceptable salt thereof, or a corresponding solvate thereof.

**13.** Active substance combination according to claim 12, **characterized in that** the compound (A) of subgroup A5.1 is a compound selected from:

[10] 5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide
[13] 5-(4-Chloro-phenyl)-1-(2,4-dichloro-phenyl)-4,5-dihydro-1H-pyrazole-3-carboxylic acid [2-(S)-(1-methoxy-1-methyl-ethyl)-pyrrolidin-1-yl]-amide
[14] 5-(4-Chloro-phenyl)-1-(2,4-dichloro-phenyl)-4,5-dihydro-1H-pyrazole-3-carboxylic acid [2-(R)-(1-methoxy-1-methyl-ethyl)-pyrrolidin-1-yl]-amide
[15] 5-(4-Chloro-phenyl)-1-(2,4-dichloro-phenyl)-4,5-dihydro-1H-pyrazole-3-carboxylic acid [2-(1-methoxy-1-methyl-ethyl)-pyrrolidin-1-yl]-amide
[16] 5-(4-Chloro-phenyl)-1-(2,4-dichloro-phenyl)-4,5-dichloro-1H-pyrazole-3-carboxylic acid azepan-1-ylamide
[21] [5-(4-Chloro-phenyl)-1-(2,4-dichloro-phenyl)-4,5-dihydro-1H-pyrazole-3-yl]-(4-cyclohexyl-piperazin-1-yl)-methanone
[22] [5-(4-Chloro-phenyl)-1-(2,4-dichloro-phenyl)-4,5-dihydro-1H-pyrazole-3-yl]-(octahydro-isoquinolin-2-yl)-methanone
[24] 5-(4-Chloro-phenyl)-1-(2,4-dichloro-phenyl)-4,5-dihydro-1H-pyrazole-3-carboxylic acid (1,3-dioxo-1H,3H-benzo[de]isoquinolin-2-yl)-amide
[25] 5-(4-Chloro-phenyl)-1-(2,4-dichloro-phenyl)-4,5-dihydro-1H-pyrazole-3-carboxylic acid (1H,3H-benzo[de] isoquinolin-2-yl)-amide
[26] 5-(4-Chloro-phenyl)-1-(2,4-dichloro-phenyl)-4,5-dihydro-1H-pyrazole-3-carboxylic acid (4-cyclopentyl-piperazin-1-yl)-amide
[27] 5-(4-Chloro-phenyl)-1-(2,4-dichloro-phenyl)-4,5-dihydro-1H-pyrazole-3-carboxylic acid (2-methyl-2,3-dihydro-indol-1-yl)-amide
[28] 5-(4-Chloro-phenyl)-1-(2,4-dichloro-phenyl)-4,5-dihydro-1H-pyrazole-3-carboxylic acid (2,3-dihydro-indol-1-yl)-amide
[32] 5-(4-Chloro-phenyl)-1-(2,4-dichloro-phenyl)-4,5-dihydro-1H-pyrazole-3-carboxylic acid cyclohexylmethyl-amide
[35] 5-(4-Chloro-phenyl)-1-(2,4-dichloro-phenyl)-4,5-dihydro-1H-pyrazole-3-carboxylic acid (1-methyl-hexyl)-amide
[38] Azocan-1-yl-[5-(4-chloro-phenyl)-1-(2,4-dichloro-phenyl)-4,5-dihydro-1H-pyrazol-3-yl]-methanone
[39] [5-(4-Chloro-phenyl)-1-(2,4-dichloro-phenyl)-4,5-dihydro-1H-pyrazole-3-yl]-(1,3-dihydro-isoindol-2-yl)-methanone
[40] Azetidin-1-yl-[5-(4-chloro-phenyl)-1-(2,4-dichloro-phenyl)-4,5-dihydro-1H-pyrazol-3-yl]-methanone
[42] [1,4']Bipiperidin-1'-yl-[5-(4-chloro-phenyl)-1-(2,4-dichloro-phenyl)-4,5-dihydro-1H-pyrazol-3-yl]-methanone
[44] 4-[5-(4-Chloro-phenyl)-1-(2,4-dichloro-phenyl)-4,5-dihydro-1H-pyrazole-3-carbonyl]-piperazine-1-carboxylic acid ethyl ester
[45] [5-(4-Chloro-phenyl)-1-(2,4-dichloro-phenyl)-4,5-dihydro-1H-pyrazole-3-yl]-(3,4-dihydro-1H-isoquinolin-2-yl)-methanone
[48] 5-(4-Chloro-phenyl)-1-(2,4-dichloro-phenyl)-4,5-dihydro-1H-pyrazole-3-carboxylic acid (2,4-dioxo-imidazolidin-1-yl)-amide
[49] 5-(4-Chloro-phenyl)-1-(2,4-dichloro-phenyl)-4,5-dihydro-1H-pyrazole-3-carboxylic acid cyclododecylamide
[52] (4-Benzyl-piperazin-1-yl)-[5-(4-chloro-phenyl)-1-(2,4-dichloro-phenyl)-4,5-dihydro-1 H-pyrazol-3-yl]-methanone
[56] [5-(4-Chloro-phenyl)-1-(2,4-dichloro-phenyl)-4,5-dihydro-1H-pyrazole-3-yl]-(6,7-dihydro-4H-thieno[3,2-c] pyridin-5-yl)-methanone
[58] 5-(4-Chloro-phenyl)-1-(2,4-dichloro-phenyl)-4,5-dihydro-1H-pyrazole-3-carboxylic acid (1,7,7-trimethyl-bicyclo[2.2.1]hept-2-yl)-amide
[63] 5-(4-Bromo-phenyl)-1-(2,4-dichloro-phenyl)-4,5-dihydro-1H-pyrazole-3-carboxylic acid (hexahydro-cyclopenta[c]pyrrol-2-yl)-amide
[64] 5-(4-Bromo-phenyl)-1-(2,4-dichloro-phenyl)-4,5-dihydro-1H-pyrazole-3-carboxylic acid (4-cyclopentyl-piperazin-1-yl)-amide
[65] 5-(4-Bromo-phenyl)-1-(2,4-dichloro-phenyl)-4,5-dihydro-1H-pyrazole-3-carboxylic acid (1 H,3H-benzo[de] isoquinolin-2-yl)-amide
[66] 5-(4-Bromo-phenyl)-1-(2,4-dichloro-phenyl)-4,5-dihydro-1H-pyrazole-3-carboxylic acid (2,3-dihydro-indol-

1-yl)-amide hydrochloride
[68] 5-(4-Bromo-phenyl)-1-(2,4-dichloro-phenyl)-4,5-dihydro-1H-pyrazole-3-carboxylic acid azepan-1-yl-amide
[71] 5-(4-Chloro-phenyl)-1-(2,4-dichloro-phenyl)-4,5-dihydro-1H-pyrazole-3-carboxylic acid (2-morpholin-4-yl-ethyl)-amide
[73] 5-(4-Chloro-phenyl)-1-(2,4-dichloro-phenyl)-4,5-dihydro-1H-pyrazole-3-carboxylic acid (3-dimethylamino-propyl)-amide
[74] [5-(4-Chloro-phenyl)-1-(2,4-dichloro-phenyl)-4,5-dihydro-1H-pyrazole-3-yl]-(3,6-dihydro-2H-pyridin-1-yl)-methanone
[75] [5-(4-Chloro-phenyl)-1-(2,4-dichloro-phenyl)-4,5-dihydro-1H-pyrazole-3-yl]-(5,6-dihydro-4H-pyrimidin-1-yl)-methanone
[76] 5-(4-Chloro-phenyl)-1-(2,4-dichloro-phenyl)-4,5-dihydro-1H-pyrazole-3-carboxylic acid (1,2,3,4-tetrahydro-naphthalen-1-yl)-amide
[78] [5-(4-Chloro-phenyl)-1-(2,4-dichloro-phenyl)-4,5-dihydro-1H-pyrazole-3-yl]-(2,3-dihydro-1H-cyclopenta[b]indol-4-yl)-methanone
[81] [5-(4-Chloro-phenyl)-1-(2,4-dichloro-phenyl)-4,5-dihydro-1H-pyrazole-3-yl]-(3,4-dihydro-2H-quinolin-1-yl)-methanone
[84] 5-(4-Chloro-phenyl)-1-(2,4-dichloro-phenyl)-4,5-dihydro-1H-pyrazole-3-carboxylic acid (2-cyclohexyl-ethyl)-amide
[85] [5-(4-Chloro-phenyl)-1-(2,4-dichloro-phenyl)-4,5-dihydro-1H-pyrazole-3-yl]-(dodecahydro-carbazol-9-yl)-methanone
[87] 5-(4-Chloro-phenyl)-1-(2,4-dichloro-phenyl)-4,5-dihydro-1H-pyrazole-3-carboxylic acid (2-cyclopentyl-ethyl)-amide
[88] 5-(4-Chloro-phenyl)-(2,4-dichloro-phenyl)-4,5-dihydro-1H-pyrazole-3-carboxylic acid bicyclo[2.2.1]hept-2-ylamide
[91] 1-(2,4-Dichloro-phenyl)-5-(4-fluoro-phenyl)-4,5-dihydro-1H-pyrazole-3-carboxylic acid (hexahydro-cyclopenta[c]pyrrol-2-yl)-amide hydrochloride
[92] 1-(2,4-Dichloro-phenyl)-5-(4-fluoro-phenyl)-4,5-dihydro-1H-pyrazole-3-carboxylic acid (4-cyclopentyl-piperazin-1-yl)-amide
[93] 1-(2,4-Dichloro-phenyl)-5-(4-fluoro-phenyl)-4,5-dihydro-1H-pyrazole-3-carboxylic acid (1 H,3H-benzo[de]isoquinolin-2-yl)-amide
[94] 1-(2,4-Dichloro-phenyl)-5-(4-fluoro-phenyl)-4,5-dihydro-1H-pyrazole-3-carboxylic acid (2,3-dihydro-indol-1-yl)-amide
[96] 1-(2,4-Dichloro-phenyl)-5-(4-fluoro-phenyl)-4,5-dihydro-1H-pyrazole-3-carboxylic acid azepan-1-yl-amide
[100] 1-(2,4-Dichloro-phenyl)-5-(4-iodo-phenyl)-4,5-dihydro-1H-pyrazole-3-carboxylic acid (hexahydro-cyclopenta[c]pyrrol-2-yl)-amide
[101] 1-(2,4-Dichloro-phenyl)-5-(4-iodo-phenyl)-4,5-dihydro-1H-pyrazole-3-carboxylic acid (4-cyclopentyl-piperazin-1-yl)-amide
[102] 1-(2,4-Dichloro-phenyl)-5-(4-iodo-phenyl)-4,5-dihydro-1H-pyrazole-3-carboxylic acid (1 H,3H-benzo[de]isoquinolin-2-yl)-amide
[103] 1-(2,4-Dichloro-phenyl)-5-(4-iodo-phenyl)-4,5-dihydro-1H-pyrazole-3-carboxylic acid (2,3-dihydro-indol-1-yl)-amide
[105] 1-(2,4-Dichloro-phenyl)-5-(4-iodo-phenyl)-4,5-dihydro-1H-pyrazole-3-carboxylic acid azepan-1-ylamide;
[109] 1-(2,4-Dichloro-phenyl)-5-(4-methoxy-phenyl)-4,5-dihydro-1H-pyrazole-3-carboxylic acid (hexahydro-cyclopenta[c]pyrrol-2-yl)-amide hydrochloride
[110] 1-(2,4-Dichloro-phenyl)-5-(4-methoxy-phenyl)-4,5-dihydro-1H-pyrazole-3-carboxylic acid (4-cyclopentyl-piperazin-1-yl)-amide
[111] 1-(2,4-Dichloro-phenyl)-5-(4-methoxy-phenyl)-4,5-dihydro-1H-pyrazole-3-carboxylic acid (1 H,3H-benzo[de]isoquinolin-2-yl)-amide
[112] 1-(2,4-Dichloro-phenyl)-5-(4-methoxy-phenyl)-4,5-dihydro-1H-pyrazole-3-carboxylic acid (2,3-dihydro-indol-1-yl)-amide hydrochloride
[114] 1-(2,4-Dichloro-phenyl)-5-(4-methoxy-phenyl)-4,5-dihydro-1H-pyrazole-3-carboxylic acid azepan-1-ylamide hydrochloride
[145] 5-(4-Chloro-phenyl)-1-(2,4-dichloro-phenyl)-4,5-dihydro-1H-pyrazole-3-carboxylic acid azocan-1-ylamide hydrochloride
[146] 5-(4-bromo-phenyl)-1-(2,4-dichloro-phenyl)-4,5-dihydro-1H-pyrazole-3-carboxylic acid azocan-1-ylamide
[147] 5-(4-fluoro-phenyl)-1-(2,4-dichloro-phenyl)-4,5-dihydro-1H-pyrazole-3-carboxylic acid azocan-1-ylamide
[148] 5-(4-methoxy-phenyl)-1-(2,4-dichloro-phenyl)-4,5-dihydro-1H-pyrazole-3-carboxylic acid azocan-1-ylamide

[149] 5-(4-hydroxy-phenyl)-1-(2,4-dichbro-phenyl)-4,5-dihydro-1H-pyrazole-3-carboxylic acid azocan-1-ylamide hydrochloride
[150] 5-(4-iodo-phenyl)-1-(2,4-dichloro-phenyl)-4,5-dihydro-1H-pyrazole-3-carboxylic acid azocan-1-ylamide
[151] N-((1S,2S)-2-(benzyloxy)cyclohexyl)-5-(4-chlorophenyl)-1-(2,4-dichlorophenyl)-4,5-dihydro-1H-pyrazole-3-carboxamide
[158] 5-(4-Chloro-phenyl)-1-(2,4-dichloro-phenyl)-4,5-dihydro-1H-pyrazole-3-carboxylic acid adamantan-1-ylamide
[159] (R)-5-(4-Chloro-phenyl)-1-(2,4-dichloro-phenyl)-4,5-dihydro-1H-pyrazole-3-carboxylic acid adamantan-1-ylamide
[160] (S)-5-(4-Chloro-phenyl)-1-(2,4-dichloro-phenyl)-4,5-dihydro-1H-pyrazole-3-carboxylic acid adamantan-1-ylamide
[161] 5-(4-bromo-phenyl)-1-(2,4-dichloro-phenyl)-4,5-dihydro-1H-pyrazole-3-carboxylic acid adamantan-1-ylamide
[162] 5-(4-fluoro-phenyl)-1-(2,4-dichloro-phenyl)-4,5-dihydro-1H-pyrazole-3-carboxylic acid adamantan-1-ylamide
[163] 5-(4-methoxy-phenyl)-1-(2,4-dichloro-phenyl)-4,5-dihydro-1H-pyrazole-3-carboxylic acid adamantan-1-ylamide
[164] 5-(4-hydroxy-phenyl)-1-(2,4-dichloro-phenyl)-4,5-dihydro-1H-pyrazole-3-carboxylic acid adamantan-1-ylamide
[165] 5-(4-iodo-phenyl)-1-(2,4-dichloro-phenyl)-4,5-dihydro-1H-pyrazole-3-carboxylic acid adamantan-1-ylamide
[166] 5-(4-Chloro-phenyl)-1-(2,4-dichloro-phenyl)-4,5-dihydro-1H-pyrazole-3-carboxylic acid adamantan-2-ylamide
[167] 5-(4-bromo-phenyl)-1-(2,4-dichloro-phenyl)-4,5-dihydro-1H-pyrazole-3-carboxylic acid adamantan-2-ylamide
[168] 5-(4-fluoro-phenyl)-1-(2,4-dichloro-phenyl)-4,5-dihydro-1H-pyrazole-3-carboxylic acid adamantan-2-ylamide
[169] 5-(4-methoxy-phenyl)-1-(2,4-dichloro-phenyl)-4,5-dihydro-1H-pyrazole-3-carboxylic acid adamantan-2-ylamide
[170] 5-(4-hydroxy-phenyl)-1-(2,4-dichloro-phenyl)-4,5-dihydro-1H-pyrazole-3-carboxylic acid adamantan-2-ylamide
[171] 5-(4-iodo-phenyl)-1-(2,4-dichloro-phenyl)-4,5-dihydro-1H-pyrazole-3-carboxylic acid adamantan-2-ylamide
[173] 1-(2,4-Dichloro-phenyl)-5-(4-hydroxy-phenyl)-4,5-dihydro-1H-pyrazole-3 -carboxylic acid azepan-1-ylamide hydrochloride
[174] 1-(2,4-Dichloro-phenyl)-5-(4-hydroxy-phenyl)-4,5-dihydro-1H-pyrazole-3 -carboxylic acid (hexahydro-cyclopenta[c]pyrrol-2-yl)-amide hydrochloride
[175] 1-(2,4-Dichloro-phenyl)-5-(4-hydroxy-phenyl)-4,5-dihydro-1H-pyrazole-3 -carboxylic acid (2,3-dihydro-indol-1-yl)-amide hydrochloride
[176] (R)-5-(4-Chloro-phenyl)-1-(2,4-dichloro-phenyl)-4,5-dihydro-1H-pyrazole-3-carboxylic acid azocan-1-ylamide hydrochloride
[177] (S)-5-(4-Chloro-phenyl)-1-(2,4-dichloro-phenyl)-4,5-dihydro-1H-pyrazole-3-carboxylic acid azocan-1-ylamide hydrochloride,
[178] N-((1 R,2R)-2-(benzyloxy)cyclohexyl)-5-(4-chlorophenyl)-1-(2,4-dichlorophenyl)-4,5-dihydro-1H-pyrazole-3-carboxamide
[179] 1-(2,4-Dichloro-phenyl)-5-(4-fluoro-phenyl)-4,5-dihydro-1H-pyrazole-3-carboxylic acid (2,3-dihydro-indol-1-yl)-amide hydrochloride
[181] (R)-5-(4-Chloro-phenyl)-1-(2,4-dichloro-phenyl)-4,5-dihydro-1H-pyrazole-3-carboxylic acid (2,3-dihydro-indol-1-yl)-amide hydrochloride
[182] (S)-5-(4-Chloro-phenyl)-1-(2.4-dichloro-phenyl)-4,5-dihydro-1H-pyrazole-3-carboxylic acid (2,3-dihydro-indol-1-yl)-amide hydrochloride
[183] (S)-5-(4-Chloro-phenyl)-1-(2,4-dichloro-phenyl)-4,5-dihydro-1H-pyrazole-3-carboxylic acid azepan-1-ylamide hydrochloride
[184] (S)-5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide hydrochloride
[185] (S)-5-(4-Chloro-phenyl)-1-(2,4-dichloro-phenyl)-4,5-dihydro-1H-pyrazole-3-carboxylic acid azepan-1-ylamide
[187] (R)-5-(4-Chloro-phenyl)-1-(2,4-dichloro-phenyl)-4,5-dihydro-1H-pyrazole-3-carboxylic acid azepan-1-ylamide hydrochloride

[188] (R)-5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide

[190] (R)- 5-(4-Chloro-phenyl)-1-(2,4-dichloro-phenyl)-4,5-dihydro-1H-pyrazole-3-carboxylic acid (2,3-dihydro-indol-1-yl)-amide

[191] 5-(4-Chloro-phenyl)-1-(2,4-dichloro-phenyl)-4,5-dihydro-1H-pyrazole-3-carboxylic acid cycloheptylmethyl-amide

[193] 5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide hydrochloride

optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

**14.** Active substance combination according to claim 1, 2 or 3, **characterized in that** for the compound (A) of subgroup A6.1 according to formula I

X is O or S;

$R^1$ represents a radical selected from the group consisting of phenyl and thienyl (thiophenyl), which may optionally be substituted with 1, 2, 3, 4 or 5 substituent(s) independently selected from the group consisting of -OH, $-O-CH_3$, $-O-C_2H_5$, F, Cl, Br and I;

$R^2$ represents a phenyl radical, which may be substituted with 1, 2, 3, 4 or 5 substituent(s) independently selected from the group consisting of -OH, $-O-CH_3$, - $O-C_2H_5$, F, Cl, Br and I ;

$R^3$ represents a radical selected from the group consisting of

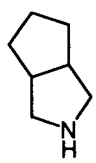 , 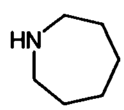 , 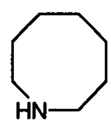 , 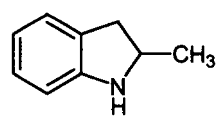 ,

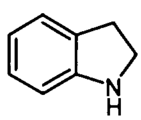 , 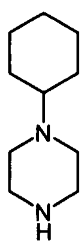 , 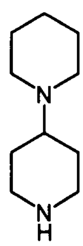 , 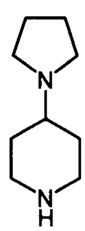 , 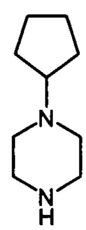 ,

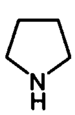 , 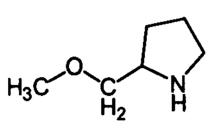 , 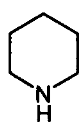 , 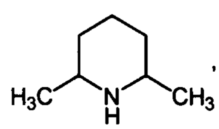 , 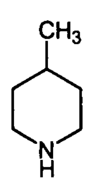 , 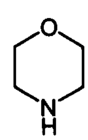 ,

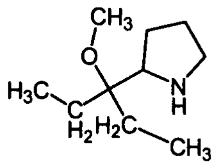 , 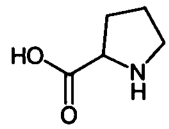 , 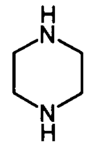 , 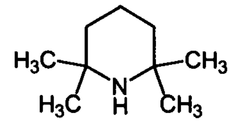 ,

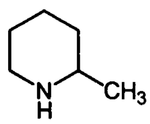 , 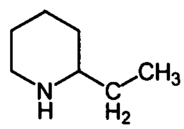 , 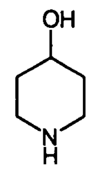 , 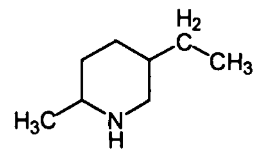 ,

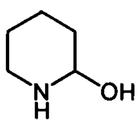 , 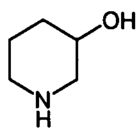 , 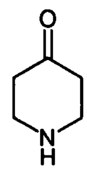 , 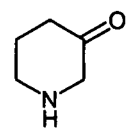 , 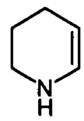 , 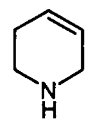 ,

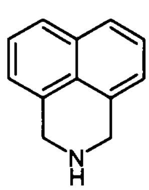 and 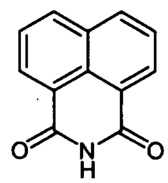 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;

a $-OR^6$-moiety or a $-NR^7R^8$ moiety;

$R^4$ represents F; Cl; Br; I; -OH, -CN; -C(=O)-H; -C(=O)-OH; $-C(=O)-OR^{17}$;
a radical selected from the group consisting of methyl, $-CF_3$, $-CH_2F$, $-CF_2H$, $-C_2F_5$, ethyl, $-CH_2-CN$, $-CH_2-OH$, n-propyl, isopropyl, $-CH_2-CH_2-CN$, $-CH_2-CH_2-OH$, n-butyl, $-CH_2-CH_2-CH_2-CN$, $-CH_2-CH_2-CH_2-OH$, isobutyl, sec-butyl, tert-butyl, n-pentyl, 2-pentyl, 3-pentyl, neo-pentyl, n-hexyl, 2-hexyl and 3-hexyl;
a benzyl radical or a $-O-R^{11}$ moiety;
$R^5$ represents H; F; Cl; Br;-C(=O)-OH; $-C(=O)-OR^{17}$; or a radical selected from the group consisting of methyl, ethyl, n-propyl, iso-propyl and n-butyl;
or $R^4$ and $R^5$ together with the bridging carbon atom form a radical selected from the group consisting of cyclopropyl, cyclobutyl, cyclopentyl, cyclohexyl, cycloheptyl, cyclooctyl, cyclopentenyl, cyclohexenyl, cycloheptenyl and cyclooctenyl;
$R^6$ represents a hydrogen atom; a radical selected from the group consisting of methyl, ethyl, n-propyl, isopropyl, n-butyl and tert-butyl or a radical selected from the group consisting of cyclopropyl, cyclobutyl, cyclopentyl, cyclohexyl and cycloheptyl;
$R^7$ represents a hydrogen atom or a radical selected from the group consisting of methyl, ethyl, n-propyl, isopropyl, n-butyl, sec-butyl and tert-butyl;
$R^8$ represents a radical selected from the group consisting of [1,2,3,4]-tetrahydronaphthyl, bicyclo[2.2.1]heptyl,

cyclopropyl, cyclobutyl, cyclopentyl, cyclohexyl, cycloheptyl, cyclooctyl, cyclononyl, cyclodecyl, cycloundecyl and cyclododecyl, which may be bonded via a $-(CH_2)-$, $-(CH_2)-(CH_2)-$ or $-(CH_2)-(CH_2)-(CH_2)-$group and/or subsituted with 1, 2, 3 or 4 substituents selected from the group consisting of methyl, ethyl, n-propyl, isopropyl, n-butyl, sec-butyl, tert-butyl, -OH, $-O-CH_3$ and $-O-C_2H_5$;
or a radical selected from the group consisting of

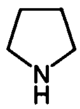 , 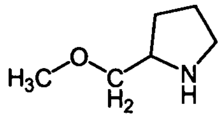 , 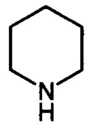 , 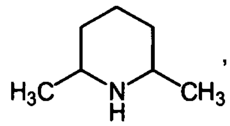 , 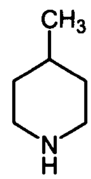 , 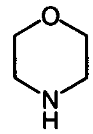 ,

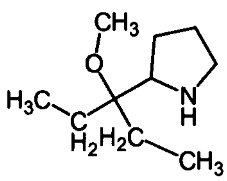 , 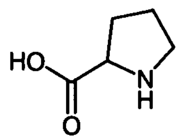 , 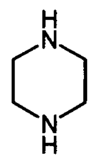 , 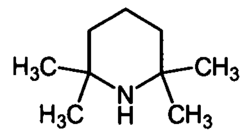 ,

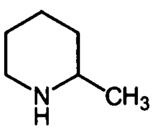 , 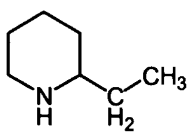 , 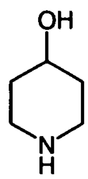 , 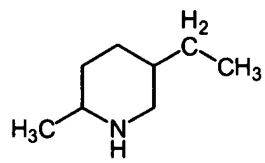 ,

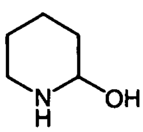 , 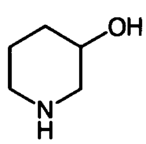 , 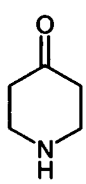 , 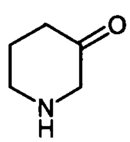 , 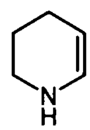 , 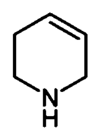 ,

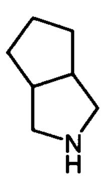 , 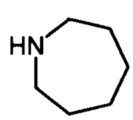 , 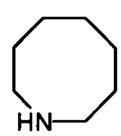 , 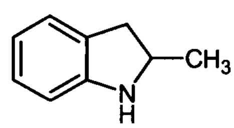 ,

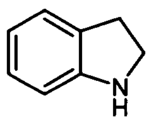 , 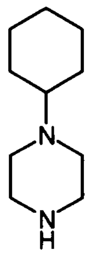 , 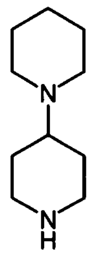 , 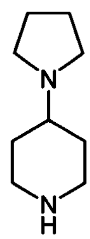 , 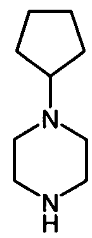 ,

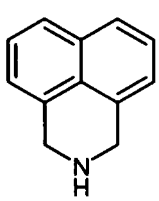 and 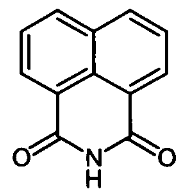 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals; and

$R^{11}$ and $R^{17}$, independently of one another, each represent a radical selected from the group consisting of methyl, ethyl, n-propyl, isopropyl, n-butyl, isobutyl, sec-butyl, tert-butyl, n-pentyl, 2-pentyl, 3-pentyl and neo-pentyl; optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

**15.** Active substance combination according to claim 14, **characterized in that** the compound (A) of subgroup A6.1 is a compound selected from:

[1] *cis*-5-(4-Chloro-phenyl)-1-(2,4-dichloro-phenyl)-4-methyl-4,5-dihydro-1H-pyrazole-3-carboxylic acid piperidin-1-ylamide hydro-chloride
[2] *trans*-5-(4-Chloro-phenyl)-1-(2,4-dichloro-phenyl)-4-methyl-4,5-dihydro-1H-pyrazole-3-carboxylic acid piperidin-1-ylamide hydro-chloride
[3] *cis*-5-(4-Chloro-phenyl)-1-(2,4-dichloro-phenyl)-4-methyl-4,5-dihydro-1H-pyrazole-3-carboxylic acid azepan-1-ylamide
[4] *trans*-5-(4-Chloro-phenyl)-1-(2,4-dichloro-phenyl)-4-methyl-4,5-dihydro-1H-pyrazole-3-carboxylic acid azepan-1-ylamide
[5] *cis*-[5-(4-Chloro-phenyl)-1-(2,4-dichloro-phenyl)-4-methyl-4,5-dihydro-1H-pyrazol-3-yl]-(4-cyclohexyl-piperazin-1-yl)-methanone
[6] *trans*-[5-(4-Chloro-phenyl)-1-(2,4-dichloro-phenyl)-4-methyl-4,5-dihydro-1H-pyrazol-3-yl]-(4-cyclohexyl-piperazin-1-yl)-methanone
[7] *cis*-5-(4-Chloro-phenylyl-(2,4-dichloro-phenyl)-4-methyl-4,5-dihydro-1H-pyrazole-3-carboxylic acid pyrrolidin-1-ylamide
[8] *trans*-5-(4-Chloro-phenyl)-1-(2,4-dichloro-phenyl)-4-methyl-4,5-dihydro-1H-pyrazole-3-carboxylic acid pyrrolidin-1-ylamide
[9] *cis*-5-(4-Chloro-phenyl)-1-(2,4-dichloro-phenyl)-4-methyl-4,5-dihydro-1H-pyrazole-3-carboxylic acid (4-methyl-piperidin-1-yl)-amide
[10] *trans*-5-(4-Chloro-phenyl)-1-(2,4-dichloro-phenyl)-4-methyl-4,5-dihydro-1H-pyrazole-3-carboxylic acid (4-methyl-piperidin-1-yl)-amide
[11] *cis*-[5-(4-Chloro-phenyl)-1-(2,4-dichloro-phenyl)-4-methyl-4,5-dihydro-1H-pyrazol-3-yl]-piperidin-1-yl-methanone

[12] *trans*-[5-(4-Chloro-phenyl)-1-(2,4-dichloro-phenyl)-4-methyl-4,5-dihydro-1H-pyrazol-3-yl]-piperidin-1-yl-methanone

[13] *cis*-5-(4-Chloro-phenyl)-1-(2,4-dichloro-phenyl)-4-methyl-4,5-dihydro-1H-pyrazole-3-carboxylic acid (hexahydro-cyclopenta[c]pyrrol-2-yl)-amide

[14] *trans*-5-(4-Chloro-phenyl)-1-(2,4-dichloro-phenyl)-4-methyl-4,5-dihydro-1H-pyrazole-3-carboxylic acid (hexahydro-cyclopenta-[c]py-rrol-2-yl)-amide

[15] *cis*-5-(4-Chloro-phenyl)-1-(2,4-dichloro-phenyl)-4-methyl-4,5-dihydro-1H-pyrazole-3-carboxylic acid (2,3-dihydro-indol-1-yl)-amide

[16] *trans*-5-(4-Chloro-phenyl)-1-(2,4-dichloro-phenyl)-4-methyl-4,5-dihydro-1H-pyrazole-3-carboxylic acid (2,3-dihydro-indol-1-yl)-amide

[17] *cis*-5-(4-Chloro-phenyl)-1-(2,4-dihloro-phenyl)-4-methyl-4,5-dihydro-1H-pyrazole-3-carboxylic acid cyclobutylamide

[18] 5-(4-Chloro-phenyl)-1-(2,4-dichloro-phenyl)-4-methyl-4,5-dihydro-1H-pyrazole-3-carboxylic acid cyclohexyl methyl-amide

[19] *cis*-5-(4-Chloro-phenyl)-1-(2,4-dichloro-phenyl)-4-methyl-4,5-dihydro-1H-pyrazole-3-carboxylic acid

[20] *trans*-5-(4-Chloro-phenyl)-1-(2,4-dichloro-phenyl)-4-methyl-4,5-dihydro-1H-pyrazole-3-carboxylic acid

[21] *cis*-5-(4-Chloro-phenyl)-1-(2,4-dichloro-phenyl)-4-ethyl-4,5-dihydro-1H-pyrazole-3-carboxylic acid

[22] *trans*-5-(4-Chloro-phenyl)-1-(2,4-dichloro-phenyl)-4-ethyl-4,5-dihydro-1H-pyrazole-3-carboxylic acid piperidin-1-ylamide

[23] *cis*-5-(4-Chloro-phenyl)-1-(2,4-dichloro-phenyl)-4-methyl-4,5-dihydro-1H-pyrazole-3-carboxylic acid methyl ester

[24] *trans*-5-(4-Chloro-phenyl)-1-(2,4-dichloro-phenyl)-4-methyl-4,5-dihydro-1H-pyrazole-3-carboxylic acid methyl ester

[25] *cis*-5-(4-Chloro-phenyl)-1-(2,4-dichloro-phenyl)-4-methyl-4,5-dihydro-1H-pyrazole-3-carboxylic acid ethyl ester

[26] *trans*-5-(4-Chloro-phenyl)-1-(2,4-dichloro-phenyl)-4-methyl-4,5-dihydro-1H-pyrazole-3-carboxylic acid ethyl ester

[27] 5-(4-Chloro-phenyl)-1-(2,4-dichloro-phenyl)-4-methyl-4,5-dihydro-1H-pyrazole-3-carboxylic acid cyclohexyl ester

[28] 5-(4-Chloro-phenyl)-1-(2,4-dichloro-phenyl)-4,4-dimethyl-4,5-dihydro-1-H-pyrazole-3-carboxylic acid

[29] 5-(4-Chloro-phenyl)-1-(2,4-dichloro-phenyl)-4,4-dimethyl-4,5-dihydro-1-H-pyrazole-3-carboxylic acid piperidin-1-ylamide

[30] 5-(4-Chloro-phenyl)-1-(2,4-dichloro-phenyl)-4-trifluoromethyl-4,5-dihydro-1H-pyrazole-3-carboxylic acid piperidin-1-ylamide

[31] 5-(4-Chloro-phenyl)-1-(2,4-dichloro-phenyl)-4-hydroxy-4,5-dihydro-1H-pyrazole-3-carboxylic acid

[32] 5-(4-Chloro-phenyl)-1-(2,4-dichloro-phenyl)-4-hydroxy-4,5-dihydro-1H-pyrazole-3-carboxylic acid piperidin-1-ylamide

[33] 5-(4-Chloro-phenyl)-1-(2,4-dichloro-phenyl)-4-(2-hydroxy-ethyl)-4,5-di-hydro-1H-pyrazole-3-carboxylic acid piperidin-1-ylamide

[34] 5-(4-Chloro-phenyl)-1-(2,4-dichloro-phenyl)-4-(2-hydroxy-ethyl)-4,5-di-hydro-1H-pyrazole-3-carboxylic acid

[35] 5-(4-Chloro-phenyl)-1-(2,4-dichloro-phenyl)-4-fluoromethyl-4,5-dihydro-1H-pyrazole-3-carboxylic acid piperidin-1-ylamide

[36] 5-(4-Chloro-phenyl)-1-(2,4-dichloro-phenyl)-4-fluoromethyl-4,5-dihydro-1H-pyrazole-3-carboxylic acid

[37] 5-(4-Chloro-phenyl)-1-(2,4-dichloro-phenyl)-4-formyl-4,5-dihydro-1H-pyrazole-3-carboxylic acid piperidin-1-ylamide

[38] 5-(4-Chloro-phenyl)-1-(2,4-dichloro-phenyl)-4-formyl-4,5-dihydro-1H-pyrazole-3-carboxylic acid

[39] 5-(4-chlorophenyl)-4-cyano-1-(2,4-dichlorophenyl)-N-(piperidin-1-yl)-4,5-dihydro-1 H-pyrazole-3-carboxamide

[40] 5-(4-Chloro-phenyl)-4-cyano-1-(2,4-dichloro-phenyl)-4,5-dihydro-1H-pyrazole-3-carboxylic acid

[41] 1-(2,4-Dichloro-phenyl)-5-(4-fluoro-phenyl)-4-methyl-4,5-dihydro-1H-pyrazole-3-carboxylic acid piperidin-1-ylamide

[42] 1-(2,4-Dichloro-phenyl)-4-ethyl-5-(4-fluoro-phenyl)-4,5-dihydro-1H-pyrazole-3-carboxylic acid piperidin-1-ylamide

[43] 1-(2,4-Dichloro-phenyl)-5-(4-fluoro-phenyl)-4,4-dimethyl-4,5-dihydro-1-H-pyrazole-3-carboxylic acid piperidin-1-ylamide

[44] 1-(2,4-Dichloro-phenyl)-5-(4-fluoro-phenyl)-4-trifluoromethyl-4,5-dihydro-1H-pyrazole-3-carboxylic acid piperidin-1-ylamide

[45] 1-(2,4-Dichloro-phenyl)-5-(4-fluoro-phenyl)-4-hydroxy-4,5-dihydro-1H-pyrazole-3-carboxylic acid piperidin-1-ylamide
[46] 1-(2,4-Dichloro-phenyl)-5-(4-fluoro-phenyl)-4-(2-hydroxy-ethyl)-4,5-dihydro-1H-pyrazole-3-carboxylic acid piperidin-1-ylamide
[47] 1-(2,4-Dichloro-phenyl)-4-fluoromethyl-5-(4-fluoro-phenyl)-4,5-dihydro-1H-pyrazole-3-carboxylic acid piperidin-1-ylamide
[48] 1-(2,4-Dichloro-phenyl)-5-(4-fluoro-phenyl)-4-formyl-4,5-dihydro-1H-pyrazole-3-carboxylic acid piperidin-1-ylamide
[49] 4-Cyano-1-(2,4-dichloro-phenyl)-5-(4-fluoro-phenyl)-4,5-dihydro-1H-pyrazole-3-carboxylic acid piperidin-1-ylamide
[50] 5-(4-Bromo-phenyl)-1-(2,4-dichloro-phenyl)-4-methyl-4,5-dihydro-1H-pyrazole-3-carboxylic acid piperidin-1-ylamide
[51] 5-(4-Bromo-phenyl)-1-(2,4-dichloro-phenyl)-4-ethyl-4,5-dihydro-1H-pyrazole-3-carboxylic acid piperidin-1-ylamide
[52] 5-(4-Bromo-phenyl)-1-(2,4-dichloro-phenyl)-4,4-dimethyl-4,5-dihydro-1H-pyrazole-3-carboxylic acid piperidin-1-ylamide
[53] 5-(4-Bromo-phenyl)-1-(2,4-dichloro-phenyl)-4-trifluoromethyl-4,5-dihydro-1H-pyrazole-3-carboxylic acid piperidin-1-ylamide
[54] 5-(4-Bromo-phenyl)-1-(2,4-dichloro-phenyl)-4-hydroxy-4,5-dihydro-1H-pyrazole-3-carboxylic acid piperidin-1-ylamide
[55] 5-(4-Bromo-phenyl)-1-(2,4-dichloro-phenyl)-4-(2-hydroxy-ethyl)-4,5-dihydro-1H-pyrazole-3-carboxylic acid piperidin-1-ylamide
[56] 5-(4-Bromo-phenyl)-1-(2,4-dichloro-phenyl)-4-fluoromethyl-4,5-dihydro-1H-pyrazole-3-carboxylic acid piperidin-1-ylamide
[57] 5-(4-Bromo-phenyl)-1-(2,4-dichloro-phenyl)-4-formyl-4,5-dihydro-1H-pyrazole-3-carboxylic acid piperidin-1-ylamide
[58] 5-(4-Bromo-phenyl)-4-cyano-1-(2,4-dichloro-phenyl)-4,5-dihydro-1H-pyrazole-3-carboxylic acid piperidin-1-ylamide
[59] *cis*-5-(5-Chloro-thiophen-2-yl)-1-(2,4-dichloro-phenyl)-4-methyl-4,5-dihydro-1H-pyrazole-3-carboxylic acid piperidin-1-ylamide
[60] *trans*-5-(5-Chloro-thiophen-2-yl)-(2,4-dichloro-phenyl)-4-methyl-4,5-dihydro-1H-pyrazole-3-carboxylic acid piperidin-1-ylamide
[61] *cis*-5-(5-Chloro-thiophen-2-yl)-1-(2,4-dichloro-phenyl)-4-ethyl-4,5-dihydro-1H-pyrazole-3-carboxylic acid piperidin-1-ylamide
[62] *trans*-5-(5-Chloro-thiophen-2-yl)-1-(2,4-dichloro-phenyl)-4-ethyl-4,5-dihydro-1H-pyrazole-3-carboxylic acid piperidin-1-ylamide
[63] 5-(5-Chloro-thiophen-2-yl)-1-(2,4-dichloro-phenyl)-4,4-dimethyl-4,5-dihydro-1H-pyrazole-3-carboxylic acid piperidin-1-ylamide
[64] 5-(5-Chloro-thiophen-2-yl)-1-(2,4-dichloro-phenyl)-4-trifluoromethyl-4,5-dihydro-1H-pyrazole-3-carboxylic acid piperidin-1-ylamide
[65] 5-(5-Chloro-thiophen-2-yl)-1-(2,4-dichloro-phenyl)-4-hydroxy-4,5-dihydro-1H-pyrazole-3-carboxylic acid piperidin-1-ylamide
[66] 5-(5-Chloro-thiophen-2-yl)-1-(2,4-dichloro-phenyl)-4-(2-hydroxy-ethyl)-4,5-dihydro-1H-pyrazole-3-carboxylic acid piperidin-1-ylamide
[67] 5-(5-Chloro-thiophen-2-yl)-1-(2,4-dichloro-phenyl)-4-fluoromethyl-4,5-dihydro-1H-pyrazole-3-carboxylic acid piperidin-1-ylamide
[68] 5-(5-Chloro-thiophen-2-yl)-1-(2,4-dichloro-phenyl)-4-formyl-4,5-dihydro-1H-pyrazole-3-carboxylic acid piperidin-1-ylamide
[69] 5-(5-Chloro-thiophen-2-yl)-4-cyano-1-(2,4-dichloro-phenyl)-4,5-dihydro-1H-pyrazole-3-carboxylic acid piperidin-1-ylamide
[70] *cis*-5-(5-Bromo-thiophen-2-yl)-1-(2,4-dichloro-phenyl)-4-methyl-4,5-dihydro-1H-pyrazole-3-carboxylic acid piperidin-1-ylamide
[71] *trans*-5-(5-Bromo-thiophen-2-yl)-1-(2,4-dichloro-phenyl)-4-methyl-4,5-dihydro-1H-pyrazole-3-carboxylic acid piperidin-1-ylamide
[72] *cis*-5-(5-Bromo-thiophen-2-yl)-1-(2,4-dichloro-phenyl)-4-ethyl-4,5-dihydro-1H-pyrazole-3-carboxylic acid piperidin-1-ylamide
[73] *trans*-5-(5-Bromo-thiophen-2-yl)-1-(2,4-dichloro-phenyl)-ethyl-4,5-dihydro-1H-pyrazole-3-carboxylic acid piperidin-1-ylamide

[74] 5-(5-Bromo-thiophen-2-yl)-1-(2,4-di-chloro-phenyl)-4,4-dimethyl-4,5-dihydro-1H-pyrazole-3-carboxylic acid piperidin-1-ylamide
[75] *cis*-5-(4-Bromo-thiophen-2-yl)-1-(2,4-dichloro-phenyl)-4-methyl-4,5-dihydro-1H-pyrazole-3-carboxylic acid piperidin-1-ylamide
[76] *trans*-5-(4-Bromo-thiophen-2-yl)-1-(2,4-dichloro-phenyl)-4-methyl-4,5-dihydro-1H-pyrazole-3-carboxylic acid piperidin-1-ylamide
[77] *cis*-5-(4-Bromo-thiophen-2-yl)-1-(2,4-dichloro-phenyl)-4-ethyl-4,5-dihydro-1H-pyrazole-3-carboxylic acid piperidin-1-ylamide
[78] *trans*-5-(4-Bromo-thiophen-2-yl)-1-(2,4-dichloro-phenyl)-4-ethyl-4,5-dihydro-1H-pyrazole-3-carboxylic acid piperidin-1-ylamide
[79] 5-(4-Bromo-thiophen-2-yl)-1-(2,4-dichloro-phenyl)-4,4-dimethyl-4,5-dihydro-1H-pyrazole-3-carboxylic acid piperidin-1-ylamide
[80] 5-(5-Bromo-thiophen-2-yl)-1-(2,4-dichloro-phenyl)-4-trifluoromethyl-4,5-dihydro-1H-pyrazole-3-carboxylic acid piperidin-1-ylamide
[81] 5-(5-Bromo-thiophen-2-yl)-1-(2,4-di-chloro-phenyl)-4-hydroxy-4,5-dihydro-1H-pyrazole-3-carboxylic acid piperidin-1-ylamide
[82] 5-(5-Bromo-thiophen-2-yl)-1-(2,4-di-chloro-phenyl)-4-(2-hydroxy-ethyl)-4,5-dihydro-1H-pyrazole-3-carboxylic acid piperidin-1-ylamide
[83] 5-(5-Bromo-thiophen-2-yl)-1-(2,4-di-chloro-phenyl)-4-fluoromethyl-4,5-dihydro-1H-pyrazole-3-carboxylic acid piperidin-1-ylamide
[84] 5-(5-Bromo-thiophen-2-yl)-1-(2,4-di-chloro-phenyl)-4-formyl-4,5-dihydro-1H-pyrazole-3-carboxylic acid piperidin-1-ylamide
[85] 5-(5-Bromo-thiophen-2-yl)-4-cyano-1-(2,4-dichloro-phenyl)-4,5-dihydro-1 H-pyrazole-3-carboxylic acid piperidin-1-ylamide
[86] *cis*-5-(5-Chloro-thiophen-2-yl)-1-(2,4-dichloro-phenyl)-4-methyl-4,5-dihydro-1H-pyrazole-3-carboxylic acid
[87] *trans*-[5-(5-Chloro-thiophen-2-yl)-1-(2,4-dichloro-phenyl)-4-methyl-4,5-dihydro-1 H-pyrazol-3-yl]-piperidin-1-yl-methanone
[88] *cis*-5-(5-Bromo-thiophen-2-yl)-1-(2,4-dichloro-phenyl)-4-methyl-4,5-dihydro-1H-pyrazole-3-carboxylic acid
[89] *trans*-5-(5-Bromo-thiophen-2-yl)-1-(2,4-dichloro-phenyl)-4-methyl-4,5-dihydro-1H-pyrazole-3-carboxylic acid
[90] *cis*-5-(4-Bromo-thiophen-2-yl)-(2,4-dichloro-phenyl)-4-methyl-4,5-dihydro-1H-pyrazole-3-carboxylic acid
[91] *trans*-5-(4-Bromo-thiophen-2-yl)-1-(2,4-dichloro-phenyl)-4-methyl-4,5-dihydro-1H-pyrazole-3-carboxylic acid,
92 (+)-cis-5-(4-chlorophenyl)-1-(2,4-dichlorophenyl)-4-methyl-N-(piperidin-1-yl)-4,5-dihydro-1H-pyrazole-3-carboxamide hydrochloride
93 (-)-cis-5-(4-chlorophenyl)-1-(2,4-dichlorophenyl)-4-methyl-N-(piperidin-1-yl)-4,5-dihydro-1H-pyrazole-3-carboxamide hydrochloride
94 *cis*-1-(2-chlorophenyl)-5-(4-chlorophenyl)-4-methyl-N-(piperidin-1-yl)-4,5-dihydro-1 H-pyrazole-3-carboxamide
95 *trans*-1-(2-chlorophenyl)-5-(4-chlorophenyl)-4-methyl-N-(piperidin-1-yl)-4,5-dihydro-1H-pyrazole-3-carboxamide
96 *cis*-5-(4-Chlorophenyl)-1-(2,4-dichlorophenyl)-4-methyl-4,5-dihydro-1H-pyrazole-3-carboxylic acid azepan-1-ylamide hydrochloride
97 *trans*-5-(4-Chlorophenyl)-1-(2,4-dichloro-phenyl)-4-methyl-4,5-dihydro-1H-pyrazole-3-carboxylic acid azepan-1-ylamide hydrochloride
98 *cis*-N-(azepan-1-yl)-1-(2-chlorophenyl)-5-(4-chlorophenyl)-4-methyl-4,5-dihydro-1 H-pyrazole-3-carboxamide
99 *tran*s-N-(azepan-1-yl)-1-(2-chlorophenyl)-5-(4-chlorophenyl)-4-methyl-4,5-dihydro-1H-pyrazole-3-carboxamide
100 *cis*-5-(4-chlorophenyl)-N-(4-cyclopentylpiperazin-1-yl)-1-(2,4-dichlorophenyl)-4-methyl-4,5-dihydro-1H-pyrazole-3-carboxamide hydrochloride
101 *cis*-5-(4-chlorophenyl)-(2,4-dichlorophenyl)-4-methyl-N-morpholino-4,5-dihydro-1H-pyrazole-3-carboxamide
102 *cis*-1-(2-chlorophenyl)-5-(4-chlorophenyl)-4-methyl-N-(4-methylpiperidin-1-yl)-4,5-dihydro-1H-pyrazole-3-carboxamide
103 *cis*-5-(4-chlorophenyl)-1-(2,4-dichlorophenyl)-N-(2,6-dimethylpiperidin-1-yl)-4-methyl-4,5-dihydro-1H-pyrazole-3-carboxamide
104 *cis*-5-(4-Chlorophenyl)-1-(2,4-dichlorophenyl)-4-methyl-4,5-dihydro-1H-pyrazole-3-carboxylic acid (1,3-dioxo-1 H,3H-benzo[de]isoquinolin-2-yl)-*amide*

105 *cis*-5-(4-Chlorophenyl)-1-(2,4-dichlorophenyl)-4-methyl-4,5-dihydro-1H-pyrazole-3-carboxylic acid (1 H, 3H-benzo[de]isoquinolin-2-yl)-amide

106 *cis*-5-(4-chlorophenyl)-1-(2,4-dichlorophenyl)-N-((R)-2-(methoxymethyl)pyrrolidin-1-yl)-4-methyl-4,5-dihydro-1H-pyrazole-3-carboxamide hydrochloride

107 *cis*-5-(4-chlorophenyl)-1-(2,4-dichlorophenyl)-N-((S)-2-(methoxymethyl)pyrrolidin-1-yl)-4-methyl-4,5-dihydro-1H-pyrazole-3-carboxamide hydrochloride

108 *cis*-5-(4-Chlorophenyl)-1-(2,4-dichlorophenyl)-4-methyl-4,5-dihydro-1H-pyrazole-3-carboxylic acid (hexahydro-cyclopenta[c]pyrrol-2-yl)-amide hydrochloride

109 *cis*-1-(2-chlorophenyl)-5-(4-chlorophenyl)-N-(hexahydrocyclopenta[c]pyrrol-2(1H)-yl)-4-methyl-4,5-dihydro-1H-pyrazole-3-carboxamide

110 *trans*-1-(2-chlorophenyl)-5-(4-chlorophenyl)-N-(hexahydrocyclopenta[c]pyrrol-2(1H)-yl)-4-methy)-4,5-dihydro-1H-pyrazole-3-carboxamide

111 *cis*-1-(2-chlorophenyl)-5-(4-chlorophenyl)-N-(indolin-1-yl)-4-methyl-4,5-dihydro-1H-pyrazole-3-carboxamide

112 *trans*-1-(2-chlorophenyl)-5-(4-chlorophenyl)-N-(indolin-1-yl)-4-methyl-4,5-dihydro-1H-pyrazole-3-carboxamide

113 *cis*-5-(4-chlorophenyl)-1-(2,4-dichlorophenyl)-4-methyl-N-(2-methylindolin-1-yl)-4,5-dihydro-1H-pyrazole-3-carboxamide

114 *cis*-5-(4-chlorophenyl)-N-(cyclohexylmethyl)-1-(2,4-dichlorophenyl)-4-methyl-4,5-dihydro-1H-pyrazole-3-carboxamide

115 *cis*-5-(4-chlorophenyl)-N-(cycloheptylmethyl)-1-(2,4-dichlorophenyl)-4-methyl-4,5-dihydro-1H-pyrazole-3-carboxamide

116 *cis*-5-(4-chlorophenyl)-N-cyclododecyl-1-(2,4-dichlorophenyl)-4-methyl-4,5-dihydro-1H-pyrazole-3-carboxamide

117 *cis*-5-(4-chlorophenyl)-1-(2,4-dichlorophenyl)-4-methyl-N-(1,2,3,4-tetrahydronaphthalen-1-yl)-4,5-dihydro-1H-pyrazole-3-carboxamide

118 *cis*-N-(4-tert-butylcyclohexyl)-5-(4-chlorophenyl)-1-(2,4-dichlorophenyl)-4-methyl-4,5-dihydro-1H-pyrazole-3-carboxamide

119 *cis*-5-(4-chlorophenyl)-N-cyclooctyl-1-(2,4-dichlorophenyl)-4-methyl-4,5-dihydro-1 H-pyrazole-3-carboxamide

120 cis-N-(azocan-1-yl)-5-(4-chlorophenyl)-1-(2,4-dichlorophenyl)-4-methyl-4,5-dihydro-1H-pyrazole-3-carboxamide

121 *cis*-N-(azocan-1-yl)-1-(2-chlorophenyl)-5-(4-chlorophenyl)-4-methyl-4,5-dihydro-1H-pyrazole-3-carboxamide

122 *cis*-azocan-1-yl(cis-5-(4-chlorophenyl)-1-(2,4-dichlorophenyl)-4-methyl-4,5-dihydro-1H-pyrazol-3-yl)methanone

123 *cis*-5-(4-chlorophenyl)-N-cycloheptyl-1-(2,4-dichlorophenyl)-4-methyl-4,5-dihydro-1H-pyrazole-3-carboxamide

124 *cis*-1-(2-chlorophenyl)-5-(4-chlorophenyl)-N-cycloheptyl-4-methyl-4,5-dihydro-1H-pyrazole-3-carboxamide

125 *trans*-5-(4-chlorophenyl)-N-cycloheptyl-1-(2,4-dichlorophenyl)-4-methyl-4,5-dihydro-1H-pyrazole-3-carboxamide

126 *trans*-1-(2-chlorophenyl)-5-(4-chlorophenyl)-N-cycloheptyl-4-methyl-4,5-dihydro-1 H-pyrazole-3-carboxamide

127 *cis*-5-(4-chlorophenyl)-N-(cyclohexylmethyl)-1-(2,4-dichlorophenyl)-4-methyl-4,5-dihydro-1H-pyrazole-3-carboxamide

128 (+)-cis-5-(4-chlorophenyl)-1-(2,4-dichlorophenyl)-N-((1S,2S)-2-hydroxycyclohexyl)-4-methyl-4,5-dihydro-1H-pyrazole-3-carboxamide

129 (-)-cis-5-(4-chlorophenyl)-1-(2,4-dichlorophenyl)-N-((1S,2S)-2-hydroxycyclohexyl)-4-methyl-4,5-dihydro-1H-pyrazole-3-carboxamide

130 (-)-cis-5-(4-chlorophenyl)-1-(2,4-dichlorophenyl)-N-((1R,2R)-2-hydroxycyclohexyl)-4-methyl-4,5-dihydro-1H-pyrazole-3-carboxamide

131 (+)-cis-5-(4-chlorophenyl)-1-(2,4-dichlorophenyl)-N-((1R,2R)-2-hydroxycyclohexyl)-4-methyl-4,5-dihydro-1H-pyrazole-3-carboxamide

132 (+)-cis-1-(2-chlorophenyl)-5-(4-chlorophenyl)-N-((1S,2S)-2-hydroxycyclohexyl)-4-methyl-4,5-dihydro-1H-pyrazole-3-carboxamide

133 (-)-cis-1-(2-chlorophenyl)-5-(4-chlorophenyl)-N-((1S,2S)-2-hydroxycyclohexyl)-4-methyl-4,5-dihydro-1H-pyrazole-3-carboxamide
134 *cis*-5-(4-chlorophenyl)-1-(2,4-dichlorophenyl)-N-(4-hydroxypiperidin-1-yl)-4-methyl-4,5-dihydro-1H-pyrazole-3-carboxamide
135 *cis*-1-(2-chlorophenyl)-5-(4-chlorophenyl)-N-(4-hydroxypiperidin-1-yl)-4-methyl-4,5-dihydro-1 H-pyrazole-3-carboxamide
136 *cis*-5-(4-chlorophenyl)-1-(2,4-dichlorophenyl)-N-(3-hydroxypiperidin-1-yl)-4-methyl-4,5-dihydro-1 H-pyrazole-3-carboxamide
137 *cis*-1-(2-chlorophenyl)-5-(4-chlorophenyl)-N-(3-hydroxypiperidin-1-yl)-4-methyl-4,5-dihydro-1H-pyrazole-3-carboxamide
138 *cis*-5-(4-chlorophenyl)-1-(2,4-dichlorophenyl)-N-(2-hydroxypiperidin-1-yl)-4-methyl-4,5-dihydro-1 H-pyrazole-3-carboxamide
139 *cis*-1-(2-chlorophenyl)-5-(4-chlorophenyl)-N-(2-hydroxypiperidin-1-yl)-4-methyl-4,5-dihydro-1 H-pyrazole-3-carboxamide
140 *cis*-5-(4-chlorophenyl)-1-(2,4-dichlorophenyl)-4-methyl-N-(4-oxopiperidin-1-yl)-4,5-dihydro-1H-pyrazole-3-carboxamide
141 *cis*-1-(2-chlorophenyl)-5-(4-chlorophenyl)-4-methyl-N-(4-oxopiperidin-1-yl)-4,5-dihydro-1H-pyrazole-3-carboxamide
142 *cis*-5-(4-chlorophenyl)-1-(2,4-dichlorophenyl)-4-methyl-N-(3-oxopiperidin-1-yl)-4,5-dihydro-1H-pyrazole-3-carboxamide
143 *cis*-1-(2-chlorophenyl)-5-(4-chlorophenyl)-4-methyl-N-(3-oxopiperidin-1-yl)-4,5-dihydro-1H-pyrazole-3-carboxamide
144 *cis*-5-(4-chlorophenyl)-1-(2,4-dichlorophenyl)-N-(3,4-dihydropyridin-1(2H)-yl)-4-methyl-4,5-dihydro-1H-pyrazole-3-carboxamide
145 *cis*-1-(2-chlorophenyl)-5-(4-chlorophenyl)-N-(3,4-dihydropyridin-1(2H)-yl)-4-methyl-4,5-dihydro-1H-pyrazole-3-carboxamide
146 *cis*-5-(4-chlorophenyl)-1-(2,4-dichlorophenyl)-N-(5,6-dihydropyridin-1(2H)-yl)-4-methyl-4,5-dihydro-1H-pyrazole-3-carboxamide
147 *cis*-1-(2-chlorophenyl)-5-(4-chlorophenyl)-N-(5,6-dihydropyridin-1(2H)-yl)-4-methyl-4,5-dihydro-1H-pyrazole-3-carboxamide
148 *cis*-1-(2-chlorophenyl)-5-(4-chlorophenyl)-4-methyl-4,5-dihydro-1H-pyrazole-3-carboxylic acid
149 *trans*-1-(2-chlorophenyl)-5-(4-chlorophenyl)-4-methyl-4,5-dihydro-1H-pyrazole-3-carboxylic acid
150 *cis*-5-(4-Chlorophenyl)-1-(2,4-dichlorophenyl)-4-methyl-4,5-dihydro-1H-py-razole-3-carboxylic acid cyclohexyl ester
151 N-(azepan-1-yl)-5-(4-chlorophenyl)-1-(2,4-dichlorophenyl)-4,4-dimethyl-4,5-dihydro-1H-pyrazole-3-carboxamide
152 1-(2-chlorophenyl)-5-(4-chlorophenyl)-4,4-dimethyl-N-(piperidin-1-yl)-4,5-dihydro-1 H-pyrazole-3-carboxamide
153 N-(azepan-1-yl)-1-(2-chlorophenyl)-5-(4-chlorophenyl)-4,4-dimethyl-4,5-dihydro-1 H-pyrazole-3-carboxamide
154 *cis*-5-(4-Chlorophenyl)-1-(2,4-dichlorophenyl)-4-trifluoromethyl-4,5-dihydro-1H-pyrazole-3-carboxylic acid piperidin-1-ylamide
155 *trans*-5-(4-Chloro-phenyl)-1-(2,4-dichlorophenyl)-4-trifluoromethyl-4,5-dihydro-1H-pyrazole-3-carboxylic acid piperidin-1-ylamide
156 *cis*-N-(azepan-1-yl)-5-(4-chlorophenyl)-1-(2,4-dichlorophenyl)-4-(trifluoromethyl)-4,5-dihydro-1H-pyrazole-3-carboxa mide
157 *trans*-N-(azepan-1-yl)-5-(4-chlorophenyl)-1-(2,4-dichlorophenyl)-4-(trifluoromethyl)-4,5-dihydro-1H-pyrazole-3-carboxamide
158 *cis*-1-(2-chlorophenyl)-5-(4-chlorophenyl)-N-(piperidin-1-yl)-4-(trifluoromethyl)-4,5-dihydro-1H-pyrazole-3-carboxamide
159 *trans* 1-(2-chlorophenyl)-5-(4-chlorophenyl)-N-(piperidin-1-yl)-4-(trifluoromethyl)-4,5-dihydro-1H-pyrazole-3-carboxamide
160 5-(4-chlorophenyl)-1-(2,4-dichlorophenyl)-3-(piperidin-1-ylcarbamoyl)-4,5-dihydro-1H-pyrazole-4-carboxylic acid
161 5-(4-chlorophenyl)-1-(2,4-dichlorophenyl)-4,4-difluoro-N-(piperidin-1-yl)-4,5-dihydro-1H-pyrazole-3-carboxamide
162 *cis*-5-(4-chlorophenyl)-4-cyano-1-(2,4-dichlorophenyl)-N-(piperidin-1-yl)-4,5-dihydro-1H-pyrazole-3-carboxamide
163 *cis*-N-(azepan-1-yl)-5-(4-chlorophenyl)-4-cyano-1-(2,4-dichlorophenyl)-4,5-dihydro-1H-pyrazole-3-carboxamide
164 *cis*-1-(2-chlorophenyl)-5-(4-chlorophenyl)-4-cyano-N-(piperidin-1-yl)-4,5-dihydro-1 H-pyrazole-3-carboxamide
165 *cis*-N-(azepan-1-yl)-1-(2-chlorophenyl)-5-(4-chlorophenyl)-4-cyano-4,5-dihydro-1 H-pyrazole-3-carboxamide
166 *trans*-5-(4-chlorophenyl)-4-cyano-1-(2,4-dichlorophenyl)-N-(piperidin-1-yl)-4,5-dihydro-1H-pyrazole-3-carboxamide
167 *trans*-N-(azepan-1-yl)-5-(4-chlorophenyl)-4-cyano-1-(2,4-dichlorophenyl)-4,5-dihydro-1H-pyrazole-3-carboxamide
168 *trans*-1-(2-chlorophenyl)-5-(4-chlorophenyl)-4-cyano-N-(piperidin-1-yl)-4,5-dihydro-1H-pyrazole-3-carboxamide
169 *cis*-1-(2-chlorophenyl)-5-(4-chlorophenyl)-4-ethyl-4,5-dihydro-1H-pyrazole-3-carboxylic acid
170 *trans*-1-(2-chlorophenyl)-5-(4-chlorophenyl)-4-ethyl-4,5-dihydro-1H-pyrazole-3-carboxylic acid
171 *cis*-5-(4-chlorophenyl)-1-(2,4-dichlorophenyl)-4-ethyl-N-(piperidin-1-yl)-4,5-dihydro-1H-pyrazole-3-carboxamide
172 *trans*-5-(4-chlorophenyl)-1-(2,4-dichlorophenyl)-4-ethyl-N-(piperidin-1-yl)-4,5-dihydro-1H-pyrazole-3-carboxa mide
173 *cis*-1-(2-chlorophenyl)-5-(4-chlorophenyl)-4-ethyl-N-(piperidin-1-yl)-4,5-dihydro-1 H-pyrazole-3-carboxamide
174 *trans*-1-(2-chlorophenyl)-5-(4-chlorophenyl)-4-ethyl-N-(piperidin-1-yl)-4,5-dihydro-1 H-pyrazole-3-carboxamide
175 *cis* -N-(azepan-1-yl)-5-(4-chlorophenyl)-1-(2,4-dichlorophenyl)-4-ethyl-4,5-dihydro-1H-pyrazole-3-carboxamide
176 *trans*-N-(azepan-1-yl)-5-(4-chlorophenyl)-1-(2,4-dichlorophenyl)-4-ethyl-4,5-dihydro-1H-pyrazole-3-carboxamide
177 *cis*-N-(azepan-1-yl)-(2-chlorophenyl)-5-(4-chlorophenyl)-4-ethyl-4,5-dihydro-1H-pyrazole-3-carboxamide
178 *trans*-N-(azepan-1-yl)-1-(2-chlorophenyl)-5-(4-chlorophenyl)-4-ethyl-4,5-dihydro-1 H-pyrazole-3-carboxamide
179 *cis*-5-(4-chlorophenyl)-N-cycloheptyl-1-(2,4-dichlorophenyl)-4-ethyl-4,5-dihydro-1 H-pyrazole-3-carboxamide
180 *cis*-1-(2-chlorophenyl)-5-(4-chlorophenyl)-N-cycloheptyl-4-ethyl-4,5-dihydro-1H-pyrazole-3-carboxamide
181 *cis*-5-(4-chlorophenyl)-1-(2,4-dichlorophenyl)-4-ethyl-N-(hexahydrocyclopenta[c]pyrrol-2(1H)-yl)-4,5-dihydro-1H-pyrazole-3-carboxamide
182 *cis*-1-(2-chlorophenyl)-5-(4-chlorophenyl)-4-ethyl-N-(hexahydrocyclopenta[c]pyrrol-2(1H)-yl)-4,5-dihydro-1H-pyrazole-3-carboxamide
183 *cis*-5-(4-chlorophenyl)-1-(2,4-dichlorophenyl)-4-ethyl-N-(indolin-1-yl)-4,5-dihydro-1H-pyrazole-3-carboxamide
184 *cis*-1-(2-chlorophenyl)-5-(4-chlorophenyl)-4-ethyl-N-(indolin-1-yl)-4,5-dihydro-1H-pyrazole-3-carboxamide
185 *cis*-5-(4-bromophenyl)-1-(2,4-dichlorophenyl)-4-ethyl-4,5-dihydro-1H-pyrazole-3-carboxylic acid
186 *trans*-5-(4-bromophenyl)-1-(2,4-dichlorophenyl)-4-ethyl-4,5-dihydro-1H-pyrazole-3-carboxylic acid
187 *cis*-5-(4-bromophenyl)-1-(2,4-dichlorophenyl)-4-ethyl-N-(piperidin-1-yl)-4,5-dihydro-1H-pyrazole-3-carboxamide
188 *trans*-5-(4-bromophenyl)1-(2,4-dichlorophenyl)-4-ethyl-N-(piperidin-1-yl)-4,5-dihydro-1H-pyrazole-3-carboxamide
189 *cis*-5-(4-bromophenyl)1-(2-chlorophenyl)4-ethyl-N-(piperidin-1-yl)-4,5-dihydro-1H-pyrazole-3-carboxamide
190 *trans*-5-(4-bromophenyl)-1-(2-chlorophenyl)-4-ethyl-N-(piperidin-1-yl)-4,5-dihydro-1 H-pyrazole-3-carboxamide
191 *cis*-N-(azepan-1-yl)-(4-bromophenyl)-(2,4-dichlorophenyl)-4-ethyl-4,5-dihydro-1H-pyrazole-3-carboxamide
192 *cis*-N-(azepan-1-yl)-5-(4-bromophenyl)-(2-chlorophenyl)-4-ethyl-4,5-dihydro-1H-pyrazole-3-carboxamide
193 *cis*-5-(4-bromophenyl)-N-cycloheptyl-1-(2,4-dichlorophenyl)-4-ethyl-4,5-dihydro-1 H-pyrazole-3-carboxamide
194 *cis*-5-(4-bromophenyl)-1-(2-chlorophenyl)-N-cycloheptyl-4-ethyl-4,5-dihydro-1H-pyrazole-3-carboxamide
195 *cis*-5-(4-bromophenyl)-1-(2,4-dichlorophenyl)-4-ethyl-N-(hexahydrocyclopenta[c]pyrrol-2(1H)-yl)-4,5-dihydro-1H-pyrazole-3-carboxamide
196 *cis*-5-(4-bromophenyl)-1-(2-chlorophenyl)-4-ethyl-N-(hexahydrocyclopenta[c]pyrrol-2(1H)-yl)-4,5-dihydro-1H-pyrazole-3-carboxamide
197 *cis*-5-(4-bromophenyl)-1-(2,4-dichlorophenyl)-4-ethyl-N-(indolin-1-yl)-4,5-dihydro-1H-pyrazole-3-carboxamide
198 *cis*-5-(4-bromophenyl)-1-(2-chlorophenyl)-4-ethyl-N-(indolin-1-yl)-4,5-dihydro-1H-pyrazole-3-carboxamide
199 *cis*-5-(4-bromophenyl)-1-(2,4-dichlorophenyl)-4-methyl-4,5-dihydro-1H-pyrazole-3-carboxylic acid
200 *trans*-5-(4-bromophenyl)-1-(2,4-dichlorophenyl)-4-methyl-4,5-dihydro-1H-pyrazole-3-carboxylic acid
201 *cis*-5-(4-bromophenyl)-1-(2-chlorophenyl)-4-methyl-4,5-dihydro-1H-pyrazole-3-carboxylic acid
202 *trans* -5-(4-bromophenyl)-1-(2-chlorophenyl)-4-methyl-4,5-dihydro-1H-pyrazole-3-carboxylic acid
203 *cis*-5-(4-bromophenyl)-1-(2,4-dichlorophenyl)-4-methyl-N-(piperidin-1-yl)-4,5-dihydro-1H-pyrazole-3-carboxamide
204 *trans*-5-(4-bromophenyl)-1-(2,4-dichlorophenyl)-4-methyl-N-(piperidin-1-yl)-4,5-dihydro-1H-pyrazole-3-carboxamide
205 *cis* 5-(4-bromophenyl)-1-(2-chlorophenyl)-4-methyl-N-(piperidin-1-yl)-4,5-dihydro-1 H-pyrazole-3-carboxamide
206 *trans*-5-(4-bromophenyl)-1-(2-chlorophenyl)-4-methyl-N-(piperidin-1-yl)-4,5-dihydro-1H-pyrazole-3-carboxamide
207 *cis*-N-(azepan-1-yl)-5-(4-bromophenyl)-1-(2,4-dichlorophenyl)-4-methyl-4,5-dihydro-1H-pyrazole-3-carboxamide hydrochloride
208 *trans*-N-(azepan-1-yl)-5-(4-bromophenyl)-1-(2,4-dichlorophenyl)-4-methyl-4,5-dihydro-1H-pyrazole-3-carboxamide hydrochloride
209 *cis*-N-(azepan-1-yl)-5-(4-bromophenyl)-1-(2-chlorophenyl)-4-methyl-4,5-dihydro-1 H-pyrazole-3-carboxamide
210 *cis*-N-(azepan-1-yl)-5-(4-bromophenyl)-1-(2-chlorophenyl)-4-methyl-4,5-dihydro-1H-pyrazole-3-carboxamide
211 *cis*-5-(4-bromophenyl)-N-cycloheptyl-1-(2,4-dichlorophenyl)-4-methyl-4,5-dihydro-1 H-pyrazole-3-carboxamide
212 *cis*-5-(4-bromophenyl)-1-(2-chlorophenyl)-N-cycloheptyl-4-methyl-4,5-dihydro-1H-pyrazole-3-carboxamide
213 *cis*-5-(4-bromophenyl)-1-(2,4-dichlorophenyl)-N-(hexahydrocyclopenta[c]pyrrol-2(1H)-yl)-4-methyl-4,5-dihydro-1H-pyrazole-3-carboxamide hydrochloride
214 *cis*-5-(4-bromophenyl)-1-(2-chlorophenyl)-N-(hexahydrocyclopenta[c]pyrrol-2(1H)-yl)-4-methyl-4,5-dihydro-1H-pyrazole-3-carboxamide
215 *cis*-5-(4-bromophenyl)-1-(2,4-dichlorophenyl)-N-(indolin-1-yl)-4-methyl-4,5-dihydro-1H-pyrazole-3-carboxamide
216 *cis*-5-(4-bromophenyl)-(2-chlorophenyl)-N-(indolin-1-yl)-4-methyl-4,5-dihydro-1H-pyrazole-3-carboxamide
217 (+)-cis-5-(4-bromophenyl)-1-(2,4-dichlorophenyl)-N-((1S,2S)-2-hydroxycyclohexyl)-4-methyl-4,5-dihydro-1H-pyrazole-3-carboxamide
218 (-)-cis-5-(4-bromophenyl)-1-(2,4-dichlorophenyl)-N-((1S,2S)-2-hydroxycyclohexyl)-4-methyl-4,5-dihydro-1H-pyrazole-3-carboxamide
219 (+)-cis-5-(4-bromophenyl)-1-(2-chlorophenyl)-N-((1S,2S)-2-hydroxycyclohexyl)-4-methyl-4,5-dihydro-1H-pyrazole-3-carboxamide
220 (-)-cis-5-(4-bromophenyl)-1-(2-chlorophenyl)-N-((1S,2S)-2-hydroxycyclohexyl)-4-methyl-4,5-dihydro-1H-pyrazole-3-carboxamide
221 *cis*-1-(2,4-dichlorophenyl)-5-(4-fluorophenyl)-4-methyl-4,5-dihydro-1H-pyrazole-3-carboxylic acid
222 *trans*-1-(2,4-dichlorophenyl)-5-(4-fluorophenyl)-4-methyl-4,5-dihydro-1H-pyrazole-3-carboxylic acid
223 *cis*-1-(2,4-dichlorophenyl)-5-(4-fluorophenyl)-4-methyl-N-(piperidin-1-yl)-4,5-dihydro-1H-pyrazole-3-carboxamide
224 *trans*-1-(2,4-dichlorophenyl)-5-(4-fluorophenyl)-4-methyl-N-(piperidin-1-yl)-4,5-dihydro-1H-pyrazole-3-carboxamide
225 *cis*-1-(2-chlorophenyl)-5-(4-fluorophenyl)-4-methyl-N-(piperidin-1-yl)-4,5-dihydro-1H-pyrazole-3-carboxamide
226 *trans*-1-(2-chlorophenyl)-5-(4-fluorophenyl)-4-methyl-N-(piperidin-1-yl)-4,5-dihydro-1H-pyrazole-3-carboxamide
227 *cis*-N-(azepan-1-yl)-1-(2,4-dichlorophenyl)-5-(4-fluorophenyl)-4-methyl-4,5-dihydro-1H-pyrazole-3-carboxamide hydrochloride
228 *trans*-N-(azepan-1-yl)-1-(2,4-dichlorophenyl)-5-(4-fluorophenyl)-4-methyl-4,5-dihydro-1H-pyrazole-3-carboxamide hydrochloride
229 *cis*-N-(azepan-1-yl)-1-(2-chlorophenyl)-5-(4-fluorophenyl)-4-methyl-4,5-dihydro-1 H-pyrazole-3-carboxamide
230 *trans*-N-(azepan-1-yl)-1-(2-chlorophenyl)-5-(4-fluorophenyl)-4-methyl-4,5-dihydro-1 H-pyrazole-3-carboxamide
231 *cis*-N-cycloheptyl-1-(2,4-dichlorophenyl)-5-(4-fluorophenyl)-4-methyl-4,5-dihydro-1 H-pyrazole-3-carboxamide
232 *cis*-1-(2-chlorophenyl)-N-cycloheptyl-5-(4-fluorophenyl)-4-methyl-4,5-dihydro-1H-pyrazole-3-carboxamide
233 *cis*-1-(2,4-dichlorophenyl)-5-(4-fluorophenyl)-N-(hexahydrocyclopenta[c]pyrrol-2(1H)-yl)-4-methyl-4,5-dihydro-1H-pyrazole-3-carboxamide hydrochloride
234 *cis*-1-(2-chlorophenyl)-5-(4-fluorophenyl)-N-(hexahydrocyclopenta[c]pyrrol-2(1H)-yl)-4-methyl-4,5-dihydro-1 H-pyrazole-3-carboxamide
235 *cis*-1-(2,4-dichlorophenyl)-5-(4-fluorophenyl)-N-(indolin-1-yl)-4-methyl-4,5-dihydro-1H-pyrazole-3-carboxamide
236 *cis*-1-(2-chlorophenyl)-5-(4-fluorophenyl)-N-(indolin-1-yl)-4-methyl-4,5-dihydro-1 H-pyrazole-3-carboxamide
237 (+)-cis-1-(2,4-dichlorophenyl)-5-(4-fluorophenyl)-N-((1S,2S)-2-hydroxycyclohexyl)-4-methyl-4,5-dihydro-1H-pyrazole-3-carboxamide
238 (-)-cis-1-(2,4-dichbrophenyl)-5-(4-fluorophenyl)-N-((1S,2S)-2-hydroxycyclohexyl)-4-methyl-4,5-dihydro-1H-pyrazole-3-carboxamide
239 (+)-cis-1-(2-chlorophenyl)-5-(4-fluorophenyl)-N-((1S,2S)-2-hydroxycyclohexyl)-4-methyl-4,5-dihydro-1H-pyrazole-3-carboxamide
240 (-)-cis-1-(2-chlorophenyl)-5-(4-fluorophenyl)-N-((1S.2S)-2-hydroxycyclohexyl)-4-methyl-4,5-dihydro-1H-pyrazole-3-carboxamide
241 *cis*-1-(2,4-dichlorophenyl)-5-(4-iodophenyl)-4-methyl-4,5-dihydro-1H-pyrazole-3-carboxylic acid
242 *trans*-1-(2,4-dichlorophenyl)-5-(4-iodophenyl)-4-methyl-4,5-dihydro-1H-pyrazole-3-carboxylic acid
243 *cis*-1-(2,4-dichlorophenyl)-5-(4-iodophenyl)-4-methyl-N-(piperidin-1-yl)-4,5-dihydro-1 H-pyrazole-3-carboxamide
244 *trans*-1-(2,4-dichlorophenyl)-5-(4-iodophenyl)-4-methyl-N-(piperidin-1-yl)-4,5-dihydro-1 H-pyrazole-3-carboxamide
245 *cis*-1-(2-chlorophenyl)-5-(4-iodophenyl)-4-methyl-N-(piperidin-1-yl)-4,5-dihydro-1 H-pyrazole-3-carboxamide
246 *trans*-1-(2-chlorophenyl)-5-(4-iodophenyl)-4-methyl-N-(piperidin-1-yl)-4,5-dihydro-1 H-pyrazole-3-carboxamide
247 *cis*-N-(azepan-1-yl)-1-(2,4-dichlorophenyl)-5-(4-iodophenyl)-4-methyl-4,5-dihydro-1 H-pyrazole-3-carboxamide
248 *trans*-N-(azepan-1-yl)-1-(2,4-dichlorophenyl)-5-(4-dichlorophenyl)-4-methyl-4,5-dihydro-1H-pyrazole-3-carboxamide
249 *cis*-N-(azepan-1-yl)-1-(2-chlorophenyl)-5-(4-iodophenyl)-4-methyl-4,5-dihydro-1H-pyrazole-3-carboxamide
250 *trans*-N-(azepan-1-yl)-1-(2-chlorophenyl)-5-(4-iodophenyl)-4-methyl-4,5-dihydro-1H-pyrazole-3-carboxamide
251 *cis*-N-cycloheptyl-1-(2,4-dichlorophenyl)-5-(4-iodophenyl)-methyl-4,5-dihydro-1 H-pyrazole-3-carboxamide
252 *cis*-1-(2-chlorophenyl)-N-cycloheptyl-5-(4-iodophenyl)-4-methyl-4,5-dihydro-1H-pyrazole-3-carboxamide
253 *cis*-1-(2,4-dichlorophenyl)-N-(hexahydrocyclopenta[c]pyrrol-2(1H)-yl)-5-(4-iodophenyl)-4-methyl-4,5-dihydro-1H-pyrazole-3-carboxamide
254 *cis*-1-(2-chlorophenyl)-N-(hexahydrocyclopenta[c]pyrrol-2(1H)-yl)-5-(4-iodophenyl)-4-methyl-4,5-dihydro-1H-pyrazole-3-carboxamide
255 *cis*-1-(2,4-dichlorophenyl)-N-(indolin-1-yl)-5-(4-iodophenyl)-4-methyl-4,5-dihydro-1 H-pyrazole-3-carboxamide
256 *cis*-1-(2-chlorophenyl)-N-(indolin-1-yl)-5-(4-iodophenyl)-4-methyl-4,5-dihydro-1H-pyrazole-3-carboxamide
257 (+)-cis-1-(2,4-dichlorophenyl)-N-((1S,2S)-2-hydroxycyclohexyl)-5-(4-iodophenyl)-4-methyl-4,5-dihydro-1H-pyrazole-3-carboxamide
258 (-)-cis-1-(2,4-dichlorophenyl)-N-((1S,2S)-2-hydroxycyclohexyl)-5-(4-iodophenyl)-4-methyl-4,5-dihydro-1H-pyrazole-3-carboxamide

259 (+)-cis-1-(2-chlorophenyl)-N-((1S,2S)-2-hydroxycyclohexyl)-5-(4-iodophenyl)-4-methyl-4,5-dihydro-1H-pyrazole-3-carboxamide

260 (-)-cis-1-(2-chlorophenyl)-N-((1S,2S)-2-hydroxycyclohexyl)-5-(4-iodophenyl)-4-methyl-4,5-dihydro-1H-pyrazole-3-carboxamide

261 *cis*-1-(2,4-dichlorophenyl)-5-(4-methoxyphenyl)-4-methyl-4,5-dihydro-1H-pyrazole-3-carboxylic acid

262 *trans*-1-(2,4-dichlorophenyl)-5-(4-methoxyphenyl)-4-methyl-4,5-dihydro-1H-pyrazole-3-carboxylic acid

263 *cis*-1-(2-chlorophenyl)-5-(4-methoxyphenyl)-4-methyl-4,5-dihydro-1H-pyrazole-3-carboxylic acid

264 *trans*-1-(2-chlorophenyl)-5-(4-methoxyphenyl)-4-methyl-4,5-dihydro-1H-pyrazole-3-carboxylic acid

265 *cis*-1-(2,4-dichlorophenyl)-5-(4-methoxyphenyl)-4-methyl-N-(piperidin-1-yl)-4,5-dihydro-1H-pyrazole-3-carboxamide hydrochloride

266 (+)-cis-1-(2,4-dichlorophenyl)-5-(4-methoxyphenyl)-4-methyl-N-(piperidin-1-yl)-4,5-dihydro-1H-pyrazole-3-carboxamide hydrochloride

267 (-)-cis-1-(2,4-dichlorophenyl)-5-(4-methoxyphenyl)-4-methyl-N-(piperidin-1-yl)-4,5-dihydro-1H-pyrazole-3-carboxamide hydrochloride

268 *trans*-1-(2,4-dichlorophenyl)-5-(4-methoxyphenyl)-4-methyl-N-(piperidin-1-yl)-4,5-dihydro-1 H-pyrazole-3-carboxamide

269 *cis*-1-(2-chlorophenyl)-5-(4-methoxyphenyl)-4-methyl-N-(piperidin-1-yl)-4,5-dihydro-1 H-pyrazole-3-carboxamide

270 *trans*-1-(2-chlorophenyl)-5-(4-methoxyphenyl)-4-methyl-N-(piperidin-1-yl)-4,5-dihydro-1H-pyrazole-3-carboxamide

271 *cis*-N-(azepan-1-yl)-1-(2,4-dichlorophenyl)-5-(4-methoxyphenyl)-4-methyl-4,5-dihydro-1H-pyrazole-3-carboxamide hydrochloride

272 *trans*-N-(azepan-1-yl)-1-(2,4-dichlorophenyl)-5-(4-methoxyphenyl)-4-methyl-4,5-dihydro-1H-pyrazole-3-carboxamide hydrochloride

273 *cis*-N-(azepan-1-yl)-1-(2-chlorophenyl)-5-(4-methoxyphenyl)-4-methyl-4,5-dihydro-1H-pyrazole-3-carboxamide

274 *trans*-N-(azepan-1-yl)-1-(2-chlorophenyl)-5-(4-methoxyphenyl)-4-methyl-4,5-dihydro-1H-pyrazole-3-carboxamide

275 *cis*-N-cycloheptyl-1-(2,4-dichlorophenyl)-5-(4-methoxyphenyl)-4-methyl-4,5-dihydro-1H-pyrazole-3-carboxamide

276 *cis*-1-(2-chlorophenyl)-N-cycloheptyl-5-(4-methoxyphenyl)-4-methyl-4,5-dihydro-1H-pyrazole-3-carboxamide

277 *cis*-1-(2,4-dichlorophenyl)-N-(hexahydrocyclopenta[c]pyrrol-2(1H)-yl)-5-(4-methoxyphenyl)-4-methyl-4,5-dihydro-1H-pyrazole-3-carboxamide hydrochloride

278 *cis*-1-(2-chlorophenyl)-N-(hexahydrocyclopenta[c]pyrrol-2(1H)-yl)-5-(4-methoxyphenyl)-4-methyl-4,5-dihydro-1H-pyrazole-3-carboxamide

279 *cis*-1-(2,4-dichlorophenyl)-N-(indolin-1-yl)-5-(4-methoxyphenyl)-4-methyl-4,5-dihydro-1H-pyrazole-3-carboxamide

280 *cis*-1-(2-chlorophenyl)-N-(indolin-1-yl)-5-(4-methoxyphenyl)-4-methyl-4,5-dihydro-1 H-pyrazole-3-carboxamide

281 (+)-cis-1-(2,4-dichlorophenyl)-N-((1S,2S)-2-hydroxycyclohexyl)-5-(4-methoxyphenyl)-4-methyl-4,5-dihydro-1H-pyrazole-3-carboxamide

282 (-)-cis-1-(2,4-dichlorophenyl)-N-((1S,2S)-2-hydroxycyclohexyl)-5-(4-methoxyphenyl)-4-methyl-4,5-dihydro-1H-pyrazole-3-carboxamide

283 (+)-cis-1-(2-chlorophenyl)-N-((1S,2S)-2-hydroxycyclohexyl)-5-(4-methoxyphenyl)-4-methyl-4,5-dihydro-1 H-pyrazole-3-carboxamide

284 (-)-cis-1-(2-chlorophenyl)-N-((1S,2S)-2-hydroxycyclohexyl)-5-(4-methoxyphenyl)-4-methyl-4,5-dihydro-1H-pyrazole-3-carboxamide

285 *cis*-1-(2,4-dichlorophenyl)-N-(4-hydroxypiperidin-1-yl)-5-(4-methoxyphenyl)-4-methyl-4,5-dihydro-1H-pyrazole-3-carboxamide

286 *cis*-1-(2-chlorophenyl)-N-(4-hydroxypiperidin-1-yl)-5-(4-methoxyphenyl)-4-methyl-4,5-dihydro-1H-pyrazole-3-carboxamide

287 *cis*-1-(2,4-dichlorophenyl)-N-(3-hydroxypiperidin-1-yl)-5-(4-methoxyphenyl)-4-methyl-4,5-dihydro-1H-pyrazole-3-carboxamide

288 *cis*-1-(2-chlorophenyl)-N-(3-hydroxypiperidin-1-yl)-5-(4-methoxyphenyl)-4-methyl-4,5-dihydro-1H-pyrazole-3-carboxamide

289 *cis*-1-(2,4-dichlorophenyl)-N-(2-hydroxypiperidin-1-yl)-5-(4-methoxyphenyl)-4-methyl-4,5-dihydro-1H-pyrazole-3-carboxamide

290 *cis*-1-(2-chlorophenyl)-N-(2-hydroxypiperidin-1-yl)-5-(4-methoxyphenyl)-4-methyl-4,5-dihydro-1H-pyrazole-3-carboxamide

291 *cis*-1-(2,4-dichlorophenyl)-5-(4-methoxyphenyl)-4-methyl-N-(4-oxopiperidin-1-yl)-4,5-dihydro-1H-pyrazole-3-carboxamide

292 *cis*-1-(2-chlorophenyl)-5-(4-methoxyphenyl)-4-methyl-N-(4-oxopiperidin-1-yl)-4,5-dihydro-1H-pyrazole-3-carboxamide

293 *cis*-1-(2,4-dichlorophenyl)-5-(4-methoxyphenyl)-4-methyl-N-(3-oxopiperidin-1-yl)-4,5-dihydro-1H-pyrazole-3-carboxamide

294 *cis*-1-(2-chlorophenyl)-5-(4-methoxyphenyl)-4-methyl-N-(3-oxopiperidin-1-yl)-4,5-dihydro-1H-pyrazole-3-carboxamide

295 *cis*-1-(2,4-dichlorophenyl)-N-(3,4-dihydropyridin-1(2H)-yl)-5-(4-methoxyphenyl)-4-methyl-4,5-dihydro-1H-pyrazole-3-carboxamide

296 *cis*-1-(2-chlorophenyl)-N-(3,4-dihydropyridin-1(2H)-yl)-5-(4-methoxyphenyl)-4-methyl-4,5-dihydro-1H-pyrazole-3-carboxamide

297 *cis*-1-(2,4-dichlorophenyl)-N-(5,6-dihydropyridin-1(2H)-yl)-5-(4-methoxyphenyl)-4-methyl-4,5-dihydro-1H-pyrazole-3-carboxamide

298 *cis*-1-(2-chlorophenyl)-N-(5,6-dihydropyridin-1(2H)-yl)-5-(4-methoxyphenyl)-4-methyl-4,5-dihydro-1H-pyrazole-3-carboxamide

299 *cis*-1-(2,4-dichlorophenyl)-5-(4-methoxyphenyl)-N-(piperidin-1-yl)-4-(trifluoromethyl)-4,5-dihydro-1H-pyrazole-3-carboxamide

300 *trans*-1-(2,4-dichlorophenyl)-5-(4-methoxyphenyl)-N-(piperidin-1-yl)-4-(trifluoromethyl)-4,5-dihydro-1H-pyrazole-3-carboxamide

301 *cis*-N-(azepan-1-yl)-1-(2,4-dichlorophenyl)-5-(4-methoxyphenyl)-4-(trifluoromethyl)-4,5-dihydro-1H-pyrazole-3-carboxamide

302 *trans*-N-(azepan-1-yl)-1-(2,4-dichlorophenyl)-5-(4-methoxyphenyl)-4-(trifluoromethyl)-4,5-dihydro-1H-pyrazole-3-carboxamide

303 *cis*-1-(2-chlorophenyl)-5-(4-methoxyphenyl)-N-(piperidin-1-yl)-4-(trifluoromethyl)-4,5-dihydro-1H-pyrazole-3-carboxamide

304 *trans*-1-(2-chlorophenyl)-5-(4-methoxyphenyl)-N-(piperidin-1-yl)-4-(trifluoromethyl)-4,5-dihydro-1H-pyrazole-3-carboxamide

305 1-(2,4-dichlorophenyl)-5-(4-methoxyphenyl)-4,4-dimethyl-N-(piperidin-1-yl)-4,5-dihydro-1H-pyrazole-3-carboxamide

306 N-(azepan-1-yl)-1-(2,4-dichlorophenyl)-5-(4-methoxyphenyl)-4,4-dimethyl-4,5-dihydro-1H-pyrazole-3-carboxamide

307 1-(2-chlorophenyl)-5-(4-methoxyphenyl)-4,4-dimethyl-N-(piperidin-1-yl)-4,5-dihydro-1H-pyrazole-3-carboxamide

308 1-(2,4-dichlorophenyl)-4-formyl-5-(4-methoxyphenyl)-N-(piperidin-1-yl)-4,5-dihydro-1H-pyrazole-3-carboxamide

309 1-(2,4-dichlorophenyl)-5-(4-methoxyphenyl)-3-(piperidin-1-ylcarbamoyl)-4,5-dihydro-1H-pyrazole-4-carboxylic acid

310 1-(2,4-dichlorophenyl)-4-(2-hydroxyethyl)-5-(4-methoxyphenyl)-N-(piperidin-1-yl)-4,5-dihydro-1H-pyrazole-3-carboxamide

311 1-(2,4-dichlorophenyl)-4-(fluoromethyl)-5-(4-methoxyphenyl)-N-(piperidin-1-yl)-4,5-dihydro-1H-pyrazole-3-carboxamide

312 *cis*-4-cyano-1-(2,4-dichlorophenyl)-5-(4-methoxyphenyl)-N-(piperidin-1-yl)-4,5-dihydro-1H-pyrazole-3-carboxamide

313 *trans*-4-cyano-1-(2,4-dichlorophenyl)-5-(4-methoxyphenyl)-N-(piperidin-1-yl)-4,5-dihydro-1H-pyrazole-3-carboxamide

314 *cis*-N-(azepan-1-yl)-4-cyano-1-(2,4-dichlorophenyl)-5-(4-methoxyphenyl)-4,5-dihydro-1H-pyrazole-3-carboxamide

315 *trans*-N-(azepan-1-yl)-4-cyano-1-(2,4-dichlorophenyl)-5-(4-methoxyphenyl)-4,5-dihydro-1H-pyrazole-3-carboxamide

316 cis -1-(2-chlorophenyl)-4-cyano-5-(4-methoxyphenyl)-N-(piperidin-1-yl)-4,5-dihydro-1H-pyrazole-3-carboxamide

317 *trans* -1-(2-chlorophenyl)-4-cyano-5-(4-methoxyphenyl)-N-(piperidin-1-yl)-4,5-dihydro-1H-pyrazole-3-carboxamide

318 1-(2,4-dichlorophenyl)-4-hydroxy-5-(4-methoxyphenyl)-N-(piperidin-1-yl)-4,5-dihydro-1H-pyrazole-3-carboxamide

319 1-(2-chlorophenyl)-4,4-difluoro-5-(4-methoxyphenyl)-N-(piperidin-1-yl)-4,5-dihydro-1H-pyrazole-3-carboxamide

320 1-(2-chlorophenyl)-4-formyl-5-(4-methoxyphenyl)-N-(piperidin-1-yl)-4,5-dihydro-1 H-pyrazole-3-carboxamide

321 1-(2-chlorophenyl)-5-(4-methoxyphenyl)-3-(piperidin-1-ylcarbamoyl)-4,5-dihydro-1 H-pyrazole-4-carboxylic acid

322 1-(2-chlorophenyl)-4-(2-hydroxyethyl)-5-(4-methoxyphenyl)-N-(piperidin-1-yl)-4,5-dihydro-1H-pyrazole-3-carboxamide

323 1-(2-chlorophenyl)-4-(fluoromethyl)-5-(4-methoxyphenyl)-N-(piperidin-1-yl)-4,5-dihydro-1H-pyrazole-3-carboxamide

324 1-(2-chlorophenyl)-4-hydroxy-5-(4-methoxyphenyl)-N-(piperidin-1-yl)-4,5-dihydro-1H-pyrazole-3-carboxamide

325 *cis*-1-(2,4-dichlorophenyl)-5-(4-ethoxyphenyl)-4-methyl-N-(piperidin-1-yl)-4,5-dihydro-1H-pyrazole-3-carboxamide

326 *trans*-1-(2,4-dichlorophenyl)-5-(4-ethoxyphenyl)-4-methyl-N-(pipendin-1-yl)-4,5-dihydro-1H-pyrazole-3-carboxamide

327 *cis*-1-(2-chlorophenyl)-5-(4-ethoxyphenyl)-4-methyl-N-(piperidin-1-yl)-4,5-dihydro-1 H-pyrazole-3-carboxamide

328 *trans*-1-(2-chlorophenyl)-5-(4-ethoxyphenyl)-4-methyl-N-(piperidin-1-yl)-4,5-dihydro-1H-pyrazole-3-carboxamide

329 *cis*-N-(azepan-1-yl)-1-(2,4-dichlorophenyl)-5-(4-ethoxyphenyl)-4-methyl-4,5-dihydro-1H-pyrazole-3-carboxamide

330 *trans*-N-(azepan-1-yl)-1-(2,4-dichlorophenyl)-5-(4-ethoxyphenyl)-4-methyl-4,5-dihydro-1H-pyrazole-3-carboxamide

331 *cis*-N-(azepan-1-yl)-1-(2-chlorophenyl)-5-(4-ethoxyphenyl)-4-methyl-4,5-dihydro-1 H-pyrazole-3-carboxamide

332 *trans*-N-(azepan-1-yl)-1-(2-chlorophenyl)-5-(4-ethoxyphenyl)-4-methyl-4,5-dihydro-1H-pyrazole-3-carboxamide

333 *cis*-N-cycloheptyl-1-(2,4-dichlorophenyl)-5-(4-ethoxyphenyl)-4-methyl-4,5-dihydro-1 H-pyrazole-3-carboxamide

334 *cis*-1-(2-chlorophenyl)-N-cycloheptyl-5-(4-ethoxyphenyl)-4-methyl-4,5-dihydro-1 H-pyrazole-3-carboxamide

335 *cis*-1-(2,4-dichlorophenyl)-5-(4-ethoxyphenyl)-N-(hexahydrocyclopenta[c]pyrrol-2(1 H)-yl)-4-methyl-4,5-dihydro-1H-pyrazole-3-carboxamide

336 *cis*-1-(2-chlorophenyl)-5-(4-ethoxyphenyl)-N-(hexahydrocyclopenta[c]pyrrol-2(1 H)-yl)-4-methyl-4,5-dihydro-1 H-pyrazole-3-carboxamide

337 *cis*-1-(2,4-dichlorophenyl)-5-(4-ethoxyphenyl)-N-(indolin-1-yl)-4-methyl-4,5-dihydro-1 H-pyrazole-3-carboxamide

338 *cis*-1-(2-chlorophenyl)-5-(4-ethoxyphenyl)-N-(indolin-1-yl)-4-methyl-4,5-dihydro-1 H-pyrazole-3-carboxamide

339 (+)-cis-1-(2,4-dichlorophenyl)-5-(4-ethoxyphenyl)-N-((1S,2S)-2-hydroxycyclohexyl)-4-methyl-4,5-dihydro-1H-pyrazole-3-carboxamide

340 (-)-cis-1-(2,4-dichlorophenyl)-5-(4-ethoxyphenyl)-N-((1S,2S)-2-hydroxycyclohexyl)-4-methyl-4,5-dihydro-1H-pyrazole-3-carboxamide

341 (+)-cis-1-(2-chlorophenyl)-5-(4-ethoxyphenyl)-N-((1S,2S)-2-hydroxycyclohexyl)-4-methyl-4,5-dihydro-1H-pyrazole-3-carboxamide

342 (-)-cis-1-(2-chlorophenyl)-5-(4-ethoxyphenyl)-N-((1S,2S)-2-hydroxycyclohexyl)-4-methyl-4,5-dihydro-1H-pyrazole-3-carboxamide

343 *cis*-1-(2,4-dichlorophenyl)-5-(4-hydroxyphenyl)-4-methyl-N-(piperidin-1-yl)-4,5-dihydro-1H-pyrazole-3-carboxamide

344 *trans*-1-(2,4-dichlorophenyl)-5-(4-hydroxyphenyl)-4-methyl-N-(piperidin-1-yl)-4,5-dihydro-1 H-pyrazole-3-carboxamide

345 *cis*-1-(2-chlorophenyl)-5-(4-hydroxyphenyl)-4-methyl-N-(piperidin-1-yl)-4,5-dihydro-1 H-pyrazole-3-carboxamide

346 *trans*-1-(2-chlorophenyl)-5-(4-hydroxyphenyl)-4-methyl-N-(piperidin-1-yl)-4,5-dihydro-1 H-pyrazole-3-carboxamide

347 *cis*-N-(azepan-1-yl)-1-(2,4-dichlorophenyl)-5-(4-hydroxyphenyl)-4-methyl-4,5-dihydro-1H-pyrazole-3-carboxamide

348 *trans*-N-(azepan-1-yl)-1-(2,4-dichlorophenyl)-5-(4-hydroxyphenyl)-4-methyl-4,5-dihydro-1H-pyrazole-3-carboxamide
349 *cis*-N-(azepan-1-yl)-1-(2-chlorophenyl)-5-(4-hydroxyphenyl)-4-methyl-4,5-dihydro-1H-pyrazole-3-carboxamide
350 *trans*-N-(azepan-1-yl)-1-(2-chlorophenyl)-5-(4-hydroxyphenyl)-4-methyl-4,5-dihydro-1H-pyrazole-3-carboxamide
351 *cis*-N-cclyoheptyl-(2,4-dichlorophenyl)-5-(4-hydroxyphenyl)-4-methyl-4,5-dihydro-1H-pyrazole-3-carboxamide
352 *cis*-1-(2-chlorophenyl)-N-cycloheptyl-5-(4-hydroxyphenyl)-4-methyl-4,5-dihydro-1 H-pyrazole-3-carboxamide
353 *cis*-1-(2,4-dichlorophenyl)-N-(hexahydrocyclopenta[c]pyrrol-2(1H)-yl)-5-(4-hydroxyphenyl)-4-methyl-4,5-dihydro-1H-pyrazole-3-carboxamide hydrochloride
354 *cis*-1-(2-chlorophenyl)-N-(hexahydrocyclopenta[c]pyrrol-2(1H)-yl)-5-(4-hydroxyphenyl)-4-methyl-4,5-dihydro-1H-pyrazole-3-carboxamide
355 *cis*-1-(2,4-dichlorophenyl)-5-(4-hydroxyphenyl)-N-(indolin-1-yl)-4-methyl-4,5-dihydro-1H-pyrazole-3-carboxamide
356 *cis*-1-(2-chlorophenyl)-5-(4-hydroxyphenyl)-N-(indolin-1-yl)-4-methyl-4,5-dihydro-1 H-pyrazole-3-carboxamide
357 (+)-cis-1-(2,4-dichlorophenyl)-N-((1S,2S)-2-hydroxycyclohexyl)-5-(4-hydroxyphenyl)-4-methyl-4,5-dihydro-1H-pyrazole-3-carboxamide
358 (-)-cis-1-(2,4-dichlorophenyl)-N-((1S,2S)-2-hydroxycyclohexyl)-5-(4-hydroxyphenyl)-4-methyl-4,5-dihydro-1H-pyrazole-3-carboxamide
359 (+)-cis-1-(2-chlorophenyl)-N-((1S,2S)-2-hydroxycyclohexyl)-5-(4-hydroxyphenyl)-4-methyl-4,5-dihydro-1H-pyrazole-3-carboxamide
360 (-)-cis-1-(2-chlorophenyl)-N-((1S,2S)-2-hydroxycyclohexyl)-5-(4-hydroxyphenyl)-4-methyl-4,5-dihydro-1H-pyrazole-3-carboxamide
361 *cis*-5-(5-chlorothiophen-2-yl)-1-(2,4-dichlorophenyl)-4-methyl-4,5-dihydro-1H-pyrazole-3-carboxylic acid
362 *trans*-5-(5-chlorothiophen-2-yl)-1-(2,4-dichlorophenyl)-4-methyl-4,5-dihydro-1H-pyrazole-3-carboxylic acid
363 *cis*-1-(2-chlorophenyl)-5-(5-chlorothiophen-2-yl)-4-methyl-4,5-dihydro-1H-pyrazole-3-carboxylic acid
364 *trans*-1-(2-chlorophenyl)-5-(5-chlorothiophen-2-yl)-4-methyl-4,5-dihydro-1H-pyrazole-3-carboxylic acid
365 *cis*-5-(5-bromothiophen-2-yl)-1-(2,4-dichlorophenyl)-4-ethyl-4,5-dihydro-1 H-pyrazole-3-carboxylic acid
366 *trans*-5-(5-bromothiophen-2-yl)-1-(2,4-dichlorophenyl)-4-ethyl-4,5-dihydro-1H-pyrazole-3-carboxylic acid
367 cis-1-(2,4-dichlorophenyl)-4-methyl-5-(thiophen-2-yl)-4,5-dihydro-1H-pyrazole-3-carboxylic acid
368 *trans*-1-(2,4-dichlorophenyl)-4-methyl-5-(thiophen-2-yl)-4,5-dihydro-1H-pyrazole-3-carboxylic acid
369 *cis*-1-(2,4-dichlorophenyl)-4-methyl-N-(piperidin-1-yl)-5-(thiophen-2-yl)-4,5-dihydro-1 H-pyrazole-3-carboxamide
370 *trans*-1-(2,4-dichlorophenyl)-4-methyl-N-(piperidin-1-yl)-5-(thiophen-2-yl)-4,5-dihydro-1H-pyrazole-3-carboxamide
371 *cis*-1-(2-chlorophenyl)-4-methyl-N-(piperidin-1-yl)-5-(thiophen-2-yl)-4,5-dihydro-1 H-pyrazole-3-carboxamide
372 *trans*-1-(2-chlorophenyl)-4-methyl-N-(piperidin-1-yl)-5-(thiophen-2-yl)-4,5-dihydro-1H-pyrazole-3-carboxamide
373 *cis*-1-(2,4-dichlorophenyl)-4-methyl-5-phenyl-4,5-dihydro-1H-pyrazole-3-carboxylic acid
374 *trans*-1-(2,4-dichlorophenyl)-4-methyl-5-phenyl-4,5-dihydro-1H-pyrazole-3-carboxylic acid
375 *cis*-1-(2,4-dichlorophenyl)-4-methyl-5-phenyl-N-(piperidin-1-yl)-4,5-dihydro-1H-pyrazole-3-carboxamide
376 *trans* -1-(2,4-dichlorophenyl)-4-methyl-5-phenyl-N-(piperidin-1-yl)-4,5-dihydro-1H-pyrazole-3-carboxamide
377 *cis*-1-(2-chlorophenyl)-4-methyl-5-phenyl-N-(piperidin-1-yl)-4,5-dihydro-1H-pyrazole-3-carboxamide
378 *trans*-1-(2-chlorophenyl)-4-methyl-5-phenyl-N-(piperidin-1-yl)-4,5-dihydro-1H-pyrazole-3-carboxamide
379 *cis*-5-(4-chlorophenyl)-1-(2,4-dichlorophenyl)-4-methyl-N-(piperidin-1-yl)-4,5-dihydro-1H-pyrazole-3-carbothioamide hydrochloride
380 *trans*-5-(4-chlorophenyl)-1-(2,4-dichlorophenyl)-4-methyl-N-(piperidin-1-yl)-4,5-dihydro-1H-pyrazole-3-carbothioamide
381 *cis* -1-(2-chlorophenyl)-5-(4-chlorophenyl)-4-methyl-N-(piperidin-1-yl)-4,5-dihydro-1H-pyrazole-3-carbothioamide
382 *trans*-1-(2-chlorophenyl)-5-(4-chlorophenyl)-4-methyl-N-(piperidin-1-yl)-4,5-dihydro-1H-pyrazole-3-carbothioamide

383 *cis*-N-(azepan-1-yl)-5-(4-chlorophenyl)-1-(2,4-dichlorophenyl)-4-methyl-4,5-dihydro-1H-pyrazole-3-carbothioamide

384 *cis*-N-(azepan-1-yl)-1-(2-chlorophenyl)-5-(4-chlorophenyl)-4-methyl-4,5-dihydro-1H-pyrazole-3-carbothioamide

385 *cis*-5-(4-bromophenyl)-1-(2,4-dichlorophenyl)-4-methyl-N-(piperidin-1-yl)-4,5-dihydro-1H-pyrazole-3-carbothioamide

386 *trans*-5-(4-bromophenyl)-1-(2,4-dichlorophenyl)-4-methyl-N-(piperidin-1-yl)-4,5-dihydro-1H-pyrazole-3-carbothioamide

387 *cis*-5-(4-bromophenyl)-1-(2-ch)orophenyl)-4-methyl-N-(piperidin-1-yl)-4,5-dihydro-1H-pyrazole-3-carbothioamide

388 *trans*-5-(4-bromophenyl)-1-(2-ch)orophenyl)-4-methyl-N-(piperidin-1-yl)-4,5-dihydro-1 H-pyrazole-3-carbothioamide

389 *cis*-1-(2,4-dichlorophenyl)-5-(4-fluorophenyl)-4-methyl-N-(piperidin-1-yl)-4,5-dihydro-1 H-pyrazole-3-carbothioamide

390 *trans*-1 -(2,4-dichlorophenyl)-5-(4-fluorophenyl)-4-methyl-N-(piperidin-1-yl)-4,5-dihydro-1H-pyrazole-3-carbothioamide

391 cis-1-(2-ch)orophenyl)-5-(4-fluorophenyl)-4-methyl-N-(piperidin-1-yl)-4,5-dihydro-1 H-pyrazole-3-carbothioamide

392 *trans*-1-(2-chlorophenyl)-5-(4-fluorophenyl)-4-methyl-N-(piperidin-1-yl)-4,5-dihydro-1H-pyrazole-3-carbothioamide

393 *cis*-1-(2,4-dichlorophenyl)-5-(4-methoxyphenyl)-4-methyl-N-(piperidin-1-yl)-4,5-dihydro-1H-pyrazole-3-carbothioamide

394 *cis*-N-(azepan-1-yl)-1-(2,4-dichlorophenyl)-5-(4-methoxyphenyl)-4-methyl-4,5-dihydro-1H-pyrazole-3-carbothioamide

395 *trans*-1-(2,4-dichlorophenyl)-5-(4-methoxyphenyl)-4-methyl-N-(pipeddin-1-yl)-4,5-dihydro-1H-pyrazole-3-carbothioamide

396 *cis*-1-(2-chlorophenyl)-5-(4-methoxyphenyl)-4-methyl-N-(piperidin-1-yl)-4,5-dihydro-1H-pyrazole-3-carbothioamide

397 cis-N-(azepan-1-yl)-1-(2-chlorophenyl)-5-(4-methoxyphenyl)-4-methyl-4,5-dihydro-1H-pyrazole-3-carbothioamide

398 *trans*-1-(2-chlorophenyl)-5-(4-methoxyphenyl)-4-methyl-N-(piperidin-1-yl)-4,5-dihydro-1H-pyrazole-3-carbothioamide

399 *cis*-1-(2,4-dichlorophenyl)-5-(4-ethoxyphenyl)-4-methyl-N-(piperidin-1-yl)-4,5-dihydro-1H-pyrazole-3-carbothioamide

400 *trans*-1-(2,4-dichlorophenyl)-5-(4-ethoxyphenyl)-4-methyl-N-(piperidin-1-yl)-4,5-dihydro-1H-pyrazole-3-carbothioamide

401 *cis*-1-(2-chlorophenyl)-5-(4-ethoxyphenyl)-4-methyl-N-(piperidin-1-yl)-4,5-dihydro-1H-pyrazole-3-carbothioamide

402 *trans*-1-(2-chlorophenyl)-5-(4-ethoxyphenyl)-4-methyl-N-(piperidin-1-yl)-4,5-dihydro-1 H-pyrazole-3-carbothioamide

403 *cis*-1-(2,4-dichlorophenyl)5-(4-hydroxyphenyl)-4-methyl-N-(piperidin-1-yl)-4,5-dihydro-1 H-pyrazole-3-carbothioamide

404 *trans*-1-(2,4-dichlorophenyl)-5-(4-hydroxyphenyl)-4-methyl-N-(piperidin-1-yl)-4,5-dihydro-1H-pyrazole-3-carbothioamide

405 *cis*-1-(2-chlorophenyl)-5-(4-hydroxyphenyl)-4-methyl-N-(piperidin-1-yl)-4,5-dihydro-1 H-pyrazole-3-carbothioamide

406 *trans*-1-(2-chlorophenyl)-5-(4-hydroxyphenyl)-4-methyl-N-(piperidin-1-yl)-4,5-dihydro-1H-pyrazole-3-carbothioamide

407 7-(4-Chloro-phenyl)-6-(2,4-dichlorophenyl)-5,6-diaza-spiro[2.4]hept-4-ene-4-carboxylic acid piperidin-1-ylamide

408 6-(2,4-Dichloro-phenyl)-7-(4-methoxy-phenyl)-5,6-diaza-spiro[2.4]hept-4-ene-4-carboxylic acid piperidin-1-ylamide

409 (+)-*cis*-N-((1S,2S)-2-(benzyloxy)cyclohexyl)-5-(4-chlorophenyl)-1-(2,4-dichlorophenyl)-4-methyl-4,5-dihydro-1H-pyrazole-3-carboxamide

[410] *cis*-1-(2,4-dichlorophenyl)-5-(4-methoxyphenyl)-4-methyl-N-(piperidin-1-yl)-4,5-dihydro-1 H-pyrazole-3-carboxamide

[411] *cis*-1-(2,4-dichlorophenyl)-5-(4-methoxyphenyl)-4-methyl-N-(piperidin-1-yl)-4,5-dihydro-1 H-pyrazole-3-carboxamide

[412] (4R,5S)-1-(2,4-dichlorophenyl)-5-(4-methoxyphenyl)-4-methyl-4,5-dihydro-1H-pyrazole-3-carboxylic acid

[413] cis-5-(4-bromophenyl)-1-(2,4-dichlorophenyl)-4-methyl-4,5-dihydro-1H-pyrazole-3-carboxylic acid

[414] cis-N-(azepan-1-yl)-1-(2,4-dichlorophenyl)-5-(4-fluorophenyl)-4-methyl-4,5-dihydro-1 H-pyrazole-3-carboxamide

415 *cis*-5-(4-bromophenyl)-1-(2,4-dichlorophenyl)-N-(indolin-1-yl)-4-methyl-4,5-dihydro-1H-pyrazole-3-carboxamide

416 *trans*-ethyl 5-(4-chlorophenyl)-1-(2,4-dichlorophenyl)-4-(trifluoromethyl)-4,5-dihydro-1H-pyrazole-3-carboxylate

417 *cis*-1-(2,4-dichlorophenyl)-5-(4-fluorophenyl)-4-methyl-N-(piperidin-1-yl)-4,5-dihydro-1H-pyrazole-3-carboxamide hydrochloride

418 *cis*-5-(4-bromophenyl)-1-(2,4-dichlorophenyl)-4-methyl-N-(piperidin-1-yl)-4,5-dihydro-1H-pyrazole-3-carboxamide hydrochloride

419 (-)-*cis*-5-(4-chlorophenyl)-1-(2,4-dichlorophenyl)-4-methyl-N-(piperidin-1-yl)-4,5-dihydro-1H-pyrazole-3-carboxamide

420 (+)-*cis*-5-(4-chlorophenyl)-1-(2,4-dichlorophenyl)-4-methyl-N-(piperidin-1-yl)-4,5-dihydro-1 H-pyrazole-3-carboxamide

421 *trans*-ethyl 1-(2,4-dichlorophenyl)-4-methyl-5-phenyl-4,5-dihydro-1H-pyrazole-3-ca rboxylate

422 *cis*-5-(4-chlorophenyl)-1-(2,4-dichlorophenyl)-4-methyl-N-((1R,2S,4R)-1,7,7-trimethylbicyclo[2.2.1]heptan-2-yl)-4,5-dihydro-1 H-pyrazole-3-carboxamide

423 *cis*-5-(4-chlorophenyl)-1-(2,4-dichlorophenyl)-4-methyl-N-(3-methylcyclohexyl)-4,5-dihydro-1H-pyrazole-3-carboxamide

424 *cis*-5-(4-chlorophenyl)-1-(2,4-dichlorophenyl)-4-methyl-N-(2-methylcyclohexyl)-4,5-dihydro-1H-pyrazole-3-carboxamide

426 *trans*-5-(5-chlorothiophen-2-yl)-1-(2,4-dichlorophenyl)-4-methyl-N-(piperidin-1-yl)-4,5-dihydro-1H-pyrazole-3-carboxamide hydrochloride

427 *cis*-5-(5-chlorothiophen-2-yl)-1-(2,4-dichlorophenyl)-4-methyl-N-(piperidin-1-yl)-4,5-dihydro-1H-pyrazole-3-carboxamide hydrochloride

428 *cis*-5-(4-bromophenyl)-1-(2,4-dichlorophenyl)-4-ethyl-N-(piperidin-1-yl)-4,5-dihydro-1H-pyrazole-3-carboxamide hydrochloride

[429] 1-(2,4-dichlorophenyl)-4-methyl-5-phenyl-4,5-dihydro-1H-pyrazole-3-carboxylic acid

[430] cis-1-(2,4-dichlorophenyl)-4-methyl-N-(piperidin-1-yl)-5-(thiophen-2-yl)-4,5-dihydro-1H-pyrazole-3-carboxamide hydrochloride

[431] *trans*-5-(4-chlorophenyl)-1-(2,4-dichlorophenyl)-4-methyl-N-(piperidin-1-yl)-4,5-dihydro-1H-pyrazole-3-carboxamide

432 *cis*-5-(4-chlorophenyl)-1-(2,4-dichlorophenyl)-4-ethyl-N-(piperidin-1-yl)-4,5-dihydro-1H-pyrazole-3-carboxamide

434 *cis*-5-(4-chlorophenyl)-1-(2,4-dichlorophenyl)-4-methyl-N-(piperidin-1-yl)-4,5-dihydro-1H-pyrazole-3-carboxamide

436 (+)-*cis*-N-((1S,2S)-2-(benzyloxy)cyclohexyl)-5-(4-chlorophenyl)-1-(2,4-dichlorophenyl)-4-methyl-4,5-dihydro-1H-pyrazole-3-carboxamide

437 (-)-*cis*-N-((1R,2R)-2-(benzyloxylcyclohexyl)-5-(4-chlorophenyl)-1-(2,4-dichlorophenyl)-4-methyl-4,5-dihydro-1H-pyrazole-3-carboxamide

438 *cis*-N-(azocan-1-yl)-1-(2,4-dichlorophenyl)-5-(4-methoxyphenyl)-4-methyl-4,5-dihydro-1H-pyrazole-3-carboxamide

439 *cis*-N-(azocan-1-yl)-1-(2-chlorophenyl)-5-(4-methoxyphenyl)-4-methyl-4,5-dihydro-1H-pyrazole-3-carboxamide

440 *cis*-N-(azocan-1-yl)-5-(4-bromophenyl)-1-(2,4-dichlorophenyl)-4-methyl-4,5-dihydro-1H-pyrazole-3-carboxamide

441 *cis*-N-(azocan-1-yl)-5-(4-bromophenyl)-1-(2-chlorophenyl)-4-methyl-4,5-dihydro-1H-pyrazole-3-carboxamide

442 *cis*-N-(azocan-1-yl)-1-(2,4-dichlorophenyl)-5-(4-fluorophenyl)-4-methyl-4,5-dihydro-1H-pyrazole-3-carboxamide

443 *cis*-N-(azocan-1-yl)-1-(2-chlorophenyl)-5-(4-fluorophenyl)-4-methyl-4,5-dihydro-1H-pyrazole-3-carboxamide

444 (+)-cis-5-(4-Chlorophenyl)-1 -(2,4-dichlorophenyl)-4-methyl-4,5-dihydro-1H-pyrazole-3-carboxylic acid azepan-1-ylamide hydrochloride

445 (-)-*cis*-(4-Chlorophenyl)-1-(2,4-dichlorophenyl)-4-methyl-4,5-dihydro-1H-pyrazole-3-carboxylic acid

azepan-1-ylamide hydrochloride
[446] *cis*-5-(4-chlorophenyl)-1-(2,4-dichlorophenyl)-4-methyl-N-(piperidin-1-yl)-4,5-dihydro-1H-pyrazole-3-carboxamide N-oxide
[447] *cis*-5-(4-chlorophenyl)-1-(2-chlorophenyl)-4-methyl-N-(piperidin-1-yl)-4,5-dihydro-1 H-pyrazole-3-carboxamide N-oxide
[448] *cis*-N-(azepan-1-yl)-5-(4-chlorophenyl)-1-(2,4-dichlorophenyl)-4-methyl-4,5-dihydro-1H-pyrazole-3-carboxamide N-oxide
[449] *cis*-N-(azepan-1-yl)-5-(4-chlorophenyl)-1-(2-chlorophenyl)-4-methyl-4,5-dihydro-1 H-pyrazole-3-carboxamide N-oxide
[450] *cis*-1-(2,4-dichlorophenyl)-5-(4-methoxyphenyl)-4-methyl-N-(piperidin-1-yl)-4,5-dihydro-1H-pyrazole-3-carboxamide N-oxide and
[451] *cis*-1-(2-chlorophenyl)-5-(4-methoxyphenyl)-4-methyl-N-(piperidin-1-yl)-4,5-dihydro-1H-pyrazole-3-carboxamide N-oxide;

optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide, thereof, or a physiologically acceptable salt thereof, or a corresponding solvate thereof.

**16.** Active substance combination according to one or more of claims 1-15, wherein the drug of component (B) is selected from
sulphonylureas, including chlorpropamide, tolbutamide, acetoheximide, tolazamide, glibenclamide, glipizide, gliclazide, glimepiride and gliquidone, short-acting post-prandial insulin secreters, including nateglinide, repaglinide and mitiglitinide, PPARγ agonists, including thiazolidinediones including troglitazone, rosiglitazone, pioglitazone, ciglitazone, darglitazone, PPARα/γ agonists, including sipoglitazar, tesaglitazar, muraglitazar, reglitazar, peliglitazar, cevoglitazar, ragaglitazar, naveglitazar, imiglitazar, aloglitazar, sodelglitazar, peliglitazar, pemoglitazar and netoglitazone, biguanides, including metformin, dipeptidyl peptidase-4 (DPP-IV) inhibitors, saxagliptin, sitagliptin, vildagliptin, denagliptin and alogliptin, incretins and incretin analogues, including exendin-4 and liraglutide, amylin analogues, including pramlintide, α-glucosidase inhibitors, including a carbose, voglibose and miglitol, sodium/glucose co-transport inhibitors, including sergliflozin, and insulin preparations, including insulin lispro (human), insulin aspart (human), neutral insulin injection, human neutral insulin (pyr), human neutral insulin (prb), bovine neutral insulin, porcine neutral insulin, human isophane insulin, isophase insulin (prb), human isophane insulin (pyr), porcine isophane insulin, bovine isophane insulin, human insulin zinc suspension (pyr), humulin (prb), porcine insulin zinc suspension, human insulin zinc suspension (prb) 30% amorphous 70% crystalline, insulin glargine (human), protamine zinc insulin injection (crystalline (prb), bovine insulin zinc suspension, bovine protamine zinc insulin, biphasic human insulin (pyr), biphasic human insulin (prb), biphasic neutral and isophane human, insulin (prb), human insulin (emp), biphasic porcine neutral and isophane insulin, biphasic insulin aspart and insulin determir.

**17.** Active substance combination according to one or more of claims 1-15 comprising

(A) one substituted pyrazoline compounds of general formula I selected from the group consisting of
(rac)-N-piperidinyl-5-(4-chloro-phenyl)-1-(2 ,4-dichlorophenyl)-4,5-dihydro-1H-pyrazol-3-carboxamide,
(R)-N-piperidinyl-5-(4-chloro-phenyl)-1-(2,4-dichlorophenyl)-4,5-dihydro-1H-pyrazol-3-carboxamide, or
(S)-N-piperidinyl-5-(4-chloro-phenyl)-1-(2,4-dichlorophenyl)-4,5-dihydro-1H-pyrazol-3-carboxamide,
in any mixing ratio, or a corresponding N-oxide thereof, or a corresponding salt thereof, or a corresponding solvate thereof,
and
(B) at least one drug of component (B) selected from sulphonylureas, including chlorpropamide, tolbutamide, acetoheximide, tolazamide, glibenclamide, glipizide, gliclazide, glimepiride and gliquidone, short-acting post-prandial insulin secreters, including nateglinide, repaglinide and mitiglitinide, PPARγ agonists, including thiazolidinediones including troglitazone, rosiglitazone, pioglitazone, ciglitazone, darglitazone, PPARα/γ agonists, including sipoglitazar, tesaglitazar, muraglitazar, reglitazar, peliglitazar, cevoglitazar, ragaglitazar, naveglitazar, imiglitazar, aloglitazar, sodelglitazar, peliglitazar, pemoglitazar and netoglitazone, biguanides, including metformin, dipeptidyl peptidase-4 (DPP-IV) inhibitors, saxagliptin, sitagliptin, vildagliptin, denagliptin and alogliptin, incretins and incretin analogues, including exendin-4 and liraglutide, amylin analogues, including pramlintide, α-glucosidase inhibitors, including acarbose, voglibose and miglitol, sodium/glucose co-transport inhibitors, including sergliflozin, and insulin preparations, including insulin lispro (human), insulin aspart (human), neutral insulin injection, human neutral insulin (pyr), human neutral insulin (prb), bovine neutral insulin, porcine neutral insulin, human isophane insulin, isophase insulin (prb), human isophane insulin (pyr), porcine isophane insulin,

bovine isophane insulin, human insulin zinc suspension (pyr), humulin (prb), porcine insulin zinc suspension, human insulin zinc suspension (prb) 30% amorphous 70% crystalline, insulin glargine (human), protamine zinc insulin injection (crystalline (prb), bovine insulin zinc suspension, bovine protamine zinc insulin, biphasic human insulin (pyr), biphasic human insulin (prb), biphasic neutral and isophane human, insulin (prb), human insulin (emp), biphasic porcine neutral and isophane insulin, biphasic insulin aspart and insulin determir.

**18.** Active substance combination according to one or more of claims 1-17, **characterized in that** the molar ratio of component (A) to component (B) is in the range of 1:10 to 10:1, preferably 1:5 to 5:1.

**19.** Medicament comprising an active substance combination according to one or more of claims 1 to 18 and optionally at least one further active substance and/or optionally at least one auxiliary substance.

**20.** Use of an active substance combination according to to one or more of claims 1 to 18 for the production of a medicament for the treatment of metabolic disorders including, but not limited to, pre-diabetes (insulin resistance, impaired glucose tolerance) or Type 2 diabetes and/or obesity and/or appetency disorders and/or dyslipidaemia and/or Metabolic Syndrome, including drug-induced weight-gain or drug-induced obesity.

Figure 1 Effect of chronic administration of COMPOUND C 10 mg/kg ip on plasma insulin in male, dietary-induced obese, Sprague-Dawley rats

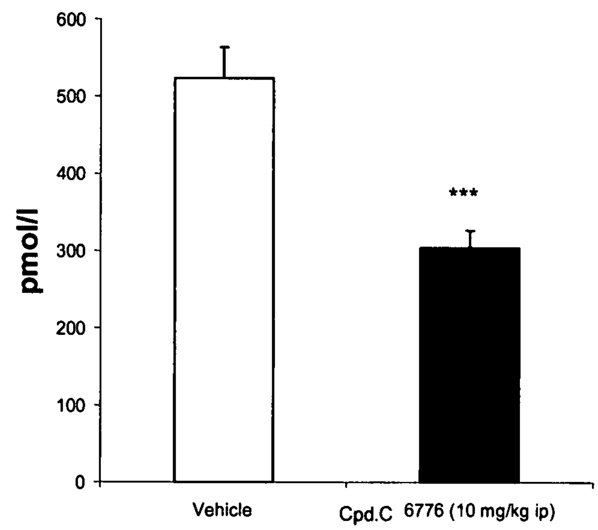


ANOVA factorial, Fisher's post-hoc test
***p<0,001 vs.

Figure 2 Effect of chronic administration of COMPOUND C 10 mg/kg ip in an oral glucose tolerance test in male, dietary-induced obese, Sprague-Dawley rats

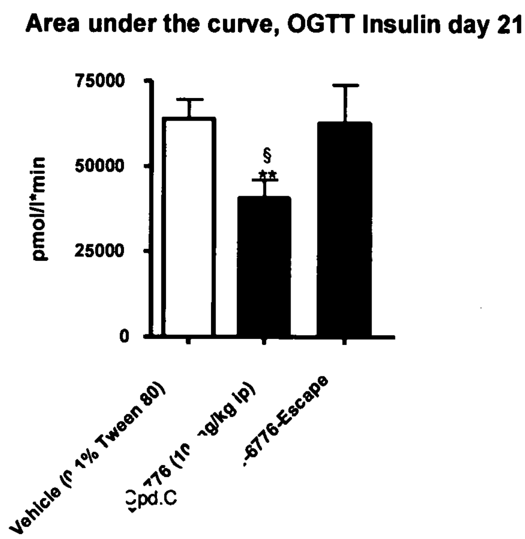

Figure 3 Effect of chronic administration of COMPOUND C 30 mg/kg po on plasma insulin levels in dietary-induced obese, female Wistar rats

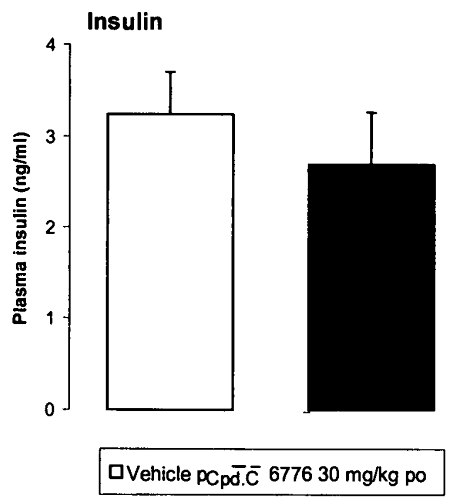


Results are expressed as treatment group means (adjusted for bleeding order and for differences between the groups in body weight at baseline (Day 1)) + SEM (calculated from the residuals of the statistical model); n = 10. Multiple comparisons against the vehicle-treated control group were determined by a multiple t test.

Figure 4 Effect of chronic administration of COMPOUND C 10 mg/kg ip on plasma triglycerides and free fatty acid levels in male, dietary-induced obese, Sprague-Dawley rats

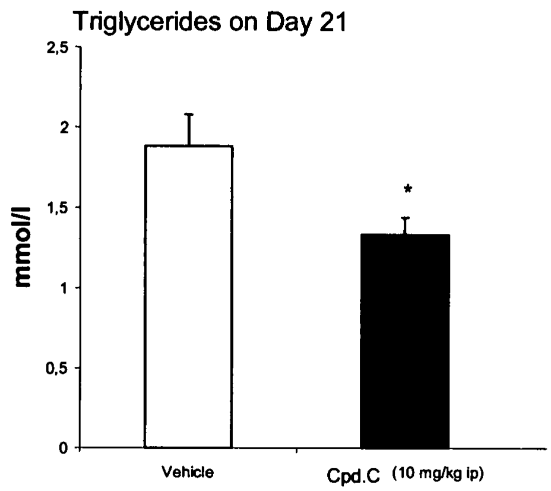


ANOVA factorial, Fisher's post-hoc test
* p<0,05 vs. vehicle

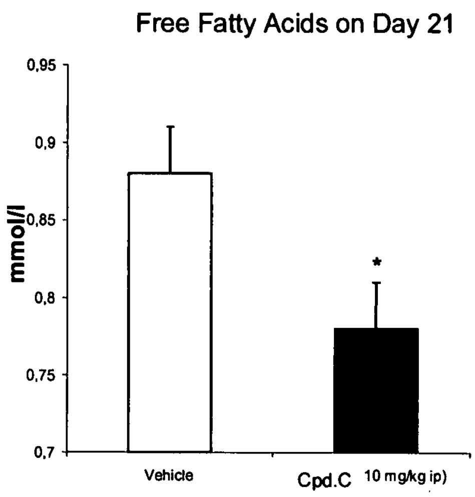


ANOVA factorial, Fisher's post-hoc test
* p<0,05 vs. vehicle

Figure 5 Effect of chronic administration of COMPOUND C on plasma glycerol and triacyglycerol levels in dietary-induced obese, female Wistar rats

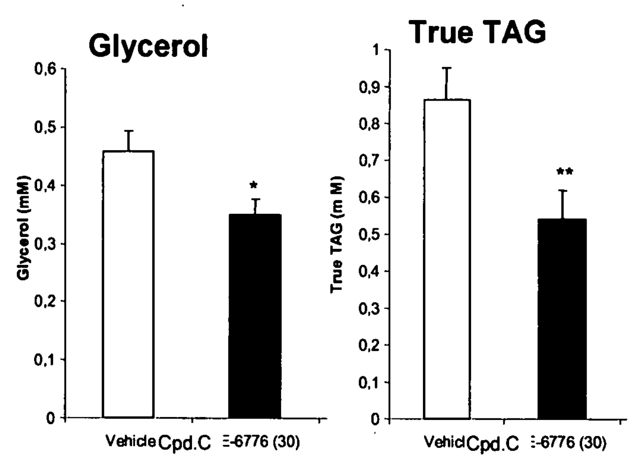


Results are expressed as treatment group means (adjusted for bleeding order and for differences between the groups in body weight at baseline (Day 1)) + SEM (calculated from the residuals of the statistical model); n = 10. Multiple comparisons against the vehicle-treated control group were determined by a multiple t test. Significant differences from the control group are denoted by *p<0.05 and **p<0.01.

Figure 6 Effect of COMPOUND C 10 and 30 mg/kg po on blood levels of triglycerides in growing male Wistar rats after 14 days of treatment

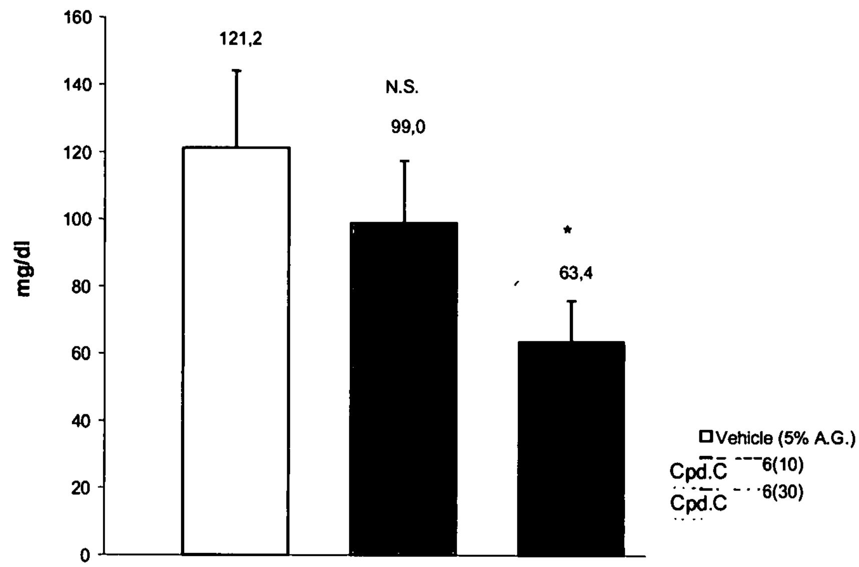

Figure 7 Effect of COMPOUND C 10 and 30 mg/kg po on blood levels of total cholesterol in male Wistar rats after 14 days of treatment

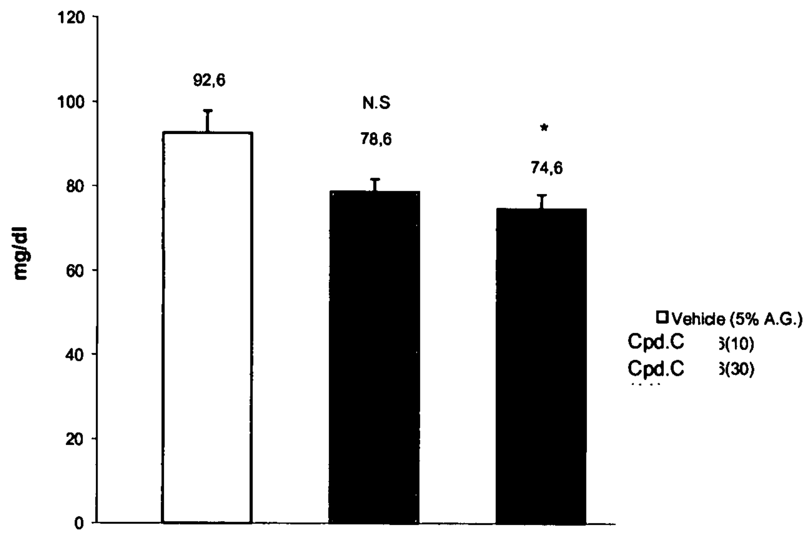

Figure 8 Effect of COMPOUND C 10 mg/kg ip on the temporal course of body weight in % compared with day 0 in male, dietary-induced obese, Sprague-Dawley rats

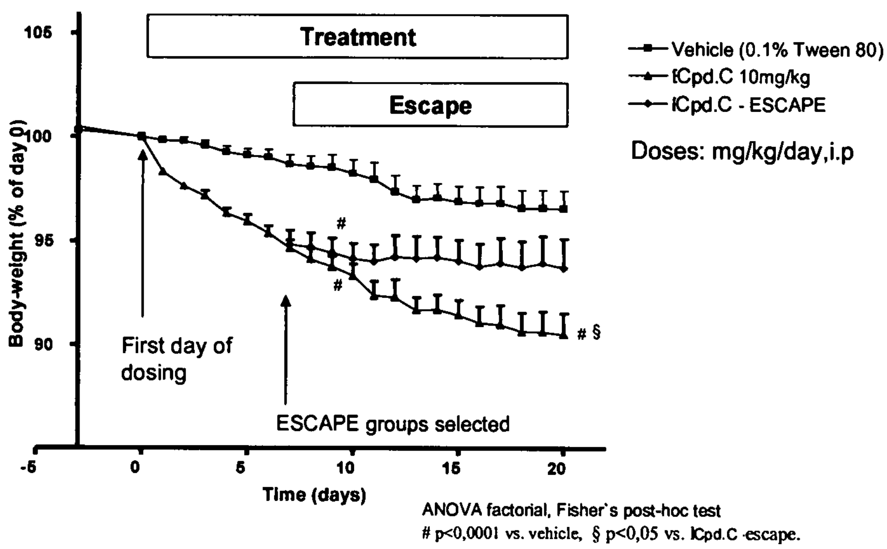

Figure 9 Effect of chronic administration of COMPOUND C 30 mg/kg po on body weight in dietary-induced obese, female Wistar rats

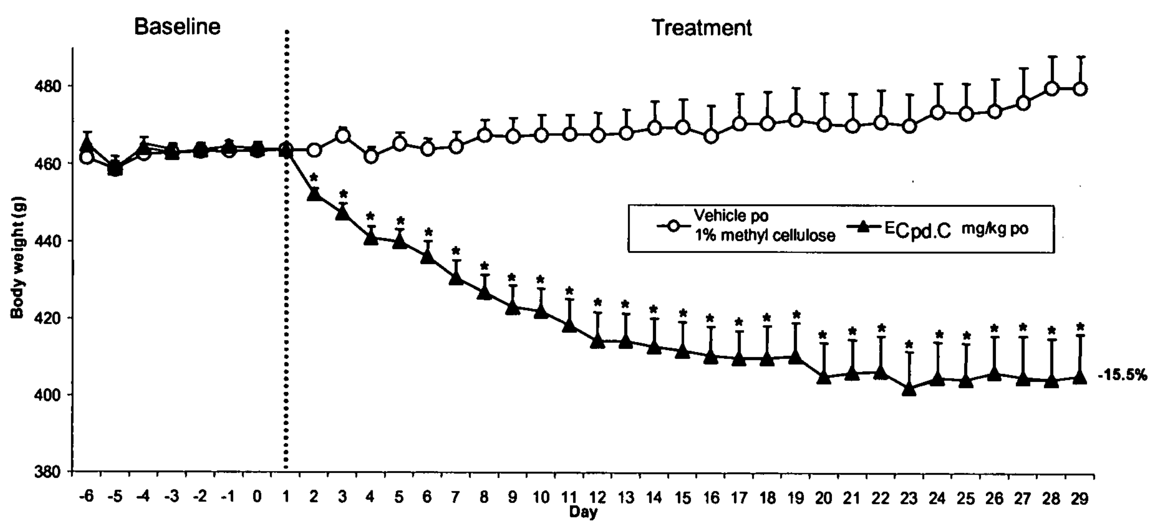


Results are means (adjusted for differences between the groups at baseline (Day 1)) ± SEM (calculated from the residuals of the statistical model); n = 9-10. Multiple comparisons against the vehicle-treated control group were by a multiple t test. Significant differences from the control group are denoted by *p<0.05 (p levels have only been given at one level for clarity).

Figure 10 Effects of COMPOUND C (10 and 30 mg/kg po) on body weight in male Wistar rats

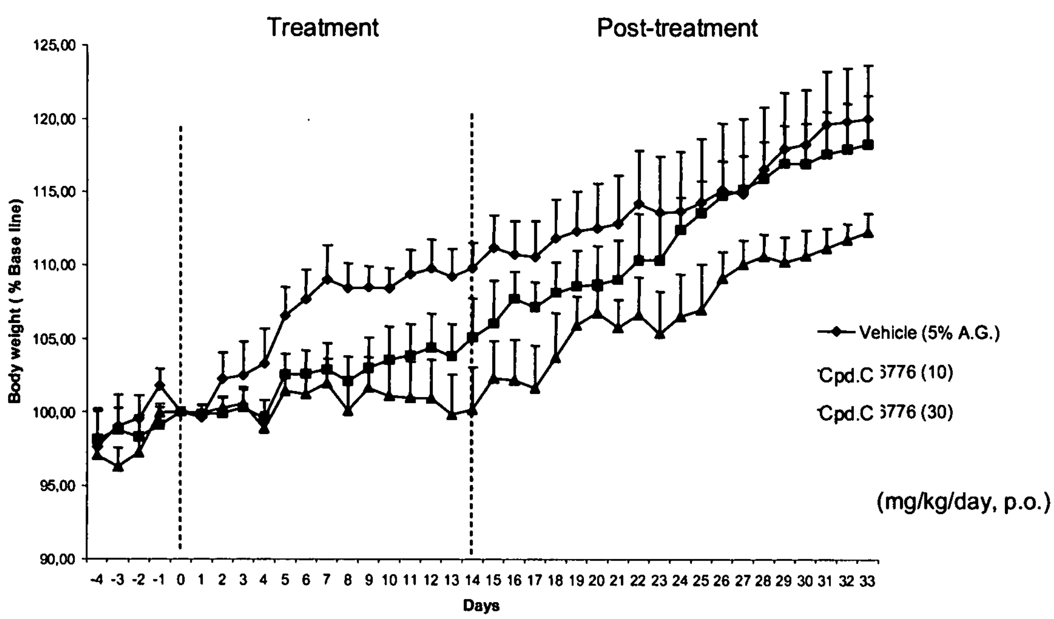

Figure 11 Effect of COMPOUND C 10 mg/kg ip on the fat pad depots in male, dietary-induced obese, Sprague-Dawley rats

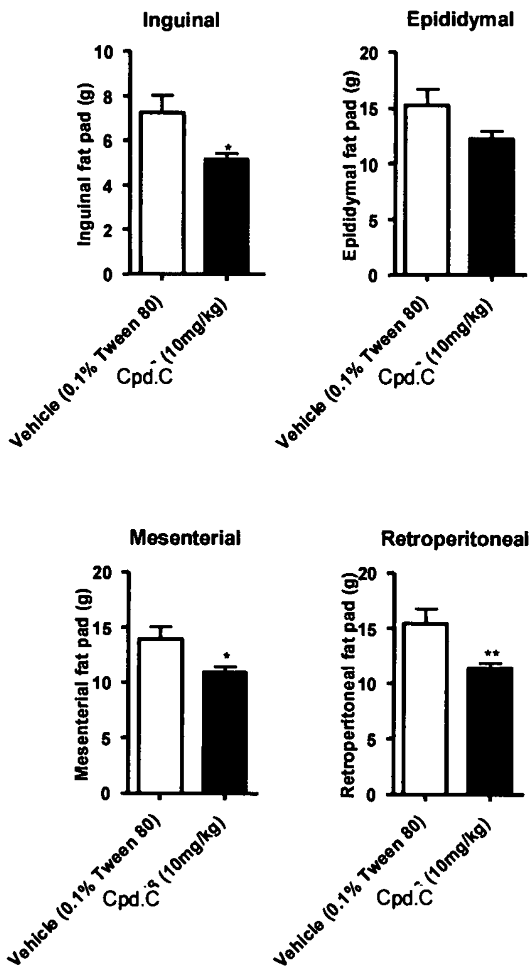

Figure 12 Effect of chronic administration of COMPOUND C 30 mg/kg po on carcass composition

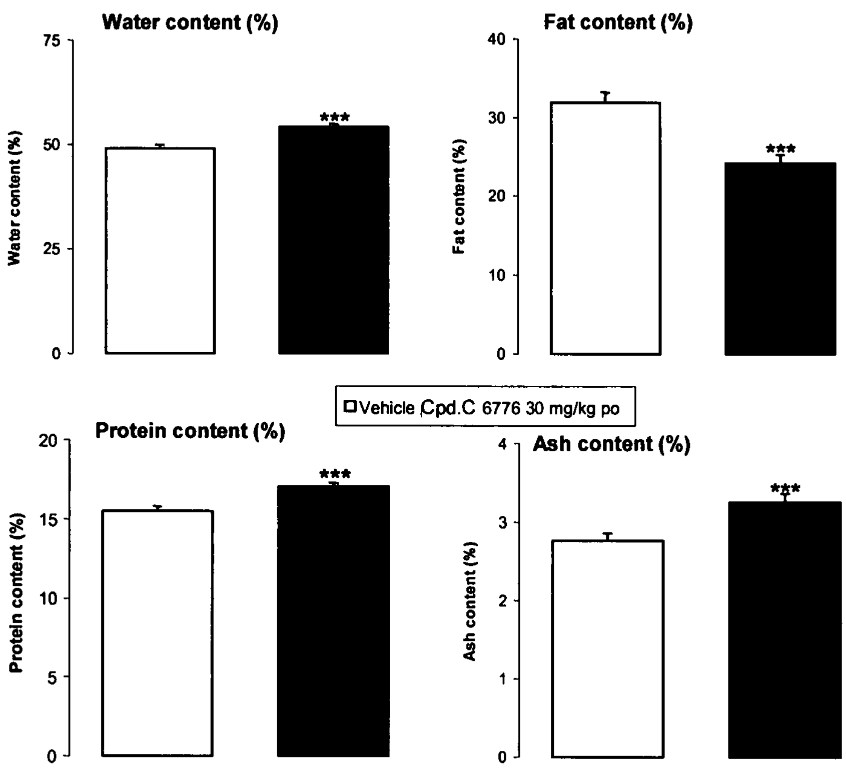


Data are expressed as percentage of final carcass weight.
Results are expressed as treatment group means (adjusted for differences between the groups in body weight at baseline (Day 1)) + SEM (calculated from the residuals of the statistical model); n = 10. Multiple comparisons against the vehicle-treated control group were determined by a multiple t test Significant differences from the control group are denoted by ***p<0.001.

Figure 13 Effect of chronic administration of COMPOUND C 30 mg/kg po plasma leptin levels in dietary-induced obese, female Wistar rats

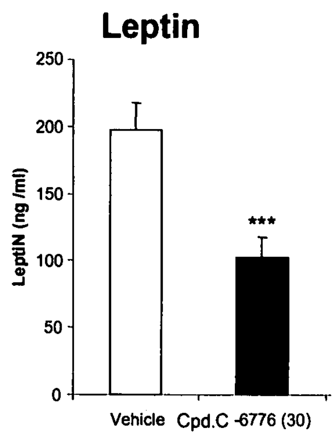


Results are expressed as treatment group means (adjusted for bleeding order and for differences between the groups in body weight at baseline (Day 1)) + SEM (calculated from the residuals of the statistical model); n = 10. Multiple comparisons against the vehicle-treated control group were determined by a multiple t test. Significant differences from the control group are denoted by ***p<0.001.

Figure 14:

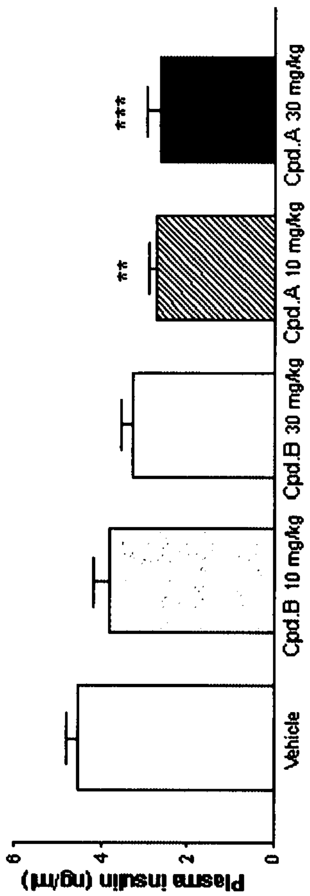
Plasma insulin (ng/ml)
0
2
4
6
Vehicle
Cpd.B 10 mg/kg
Cpd.B 30 mg/kg
Cpd.A 10 mg/kg
Cpd.A 30 mg/kg
**
***

Figure 15:

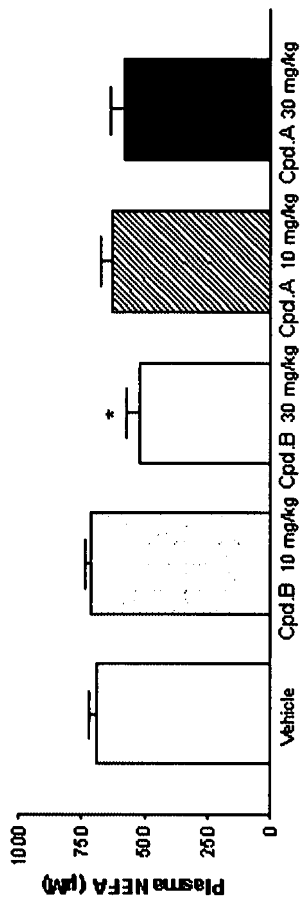
Plasma NEFA (μM)
1000
750
500
250
0
Vehicle
Cpd.B 10 mg/kg
*
Cpd.B 30 mg/kg
Cpd.A 10 mg/kg
Cpd.A 30 mg/kg

Figure 16:

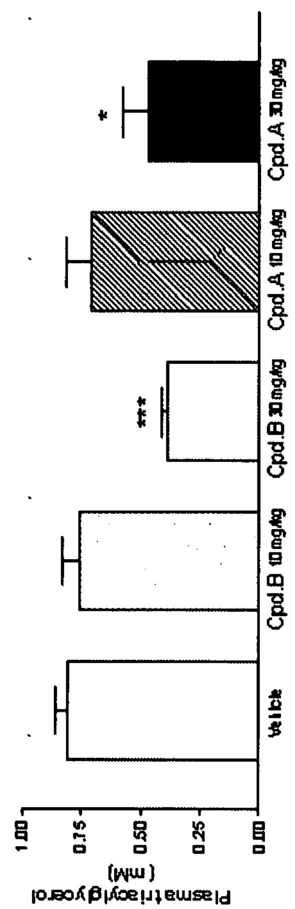
Plasma triacylglycerol (mM)
1.00
0.75
0.50
0.25
0.00
Vehicle
Cpd.B 10 mg/kg
Cpd.B 30 mg/kg
Cpd.A 10 mg/kg
Cpd.A 30 mg/kg
***
*

Figure 18:

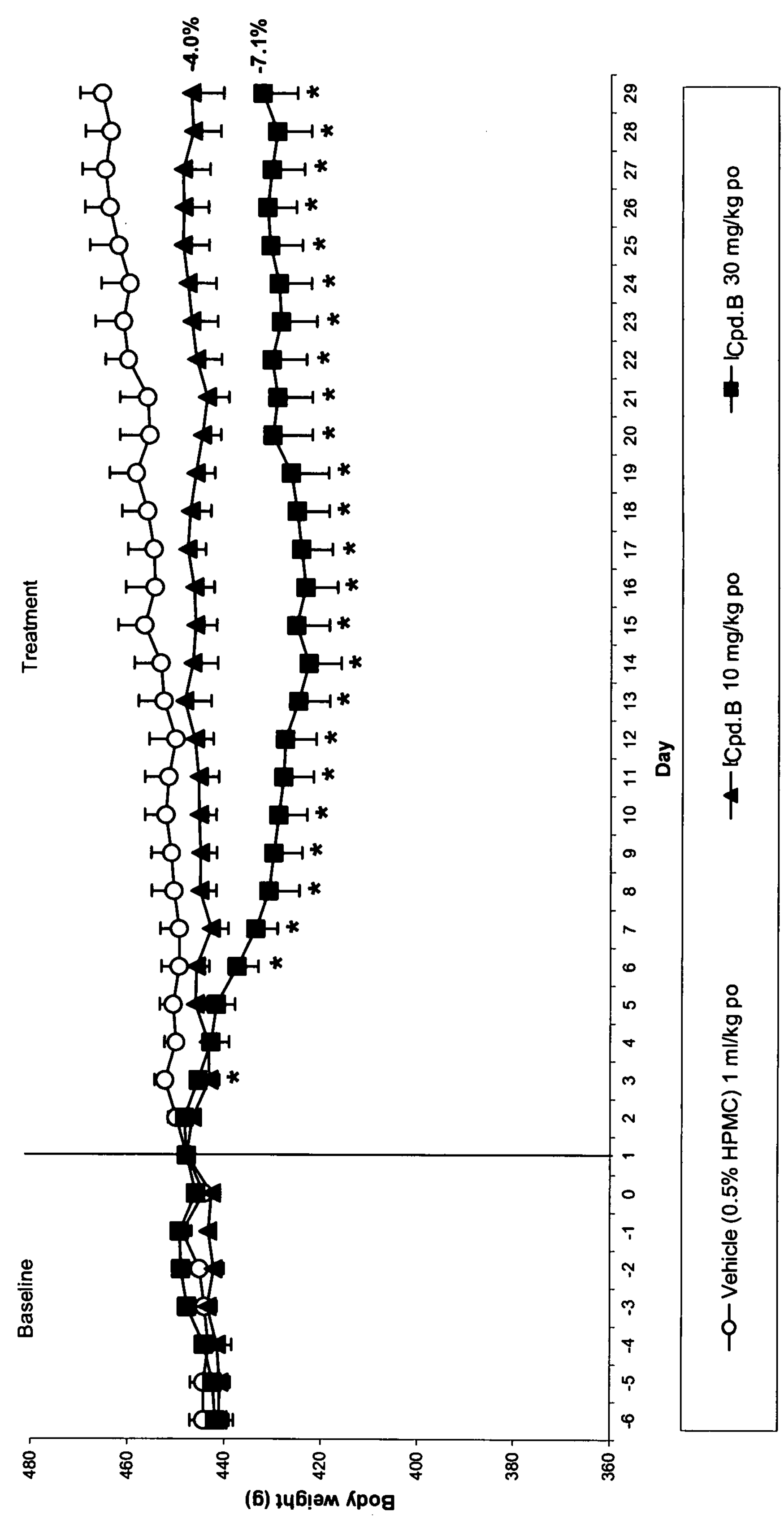
Baseline
Treatment
-4.0%
-7.1%
Body weight (g)
480
460
440
420
400
380
360
-6
-5
-4
-3
-2
-1
0
1
2
3
4
5
6
7
8
9
10
11
12
13
14
15
16
17
18
19
20
21
22
23
24
25
26
27
28
29
Day
Vehicle (0.5% HPMC) 1 ml/kg po
Cpd.B 10 mg/kg po
Cpd.B 30 mg/kg po

**Figure 19:**

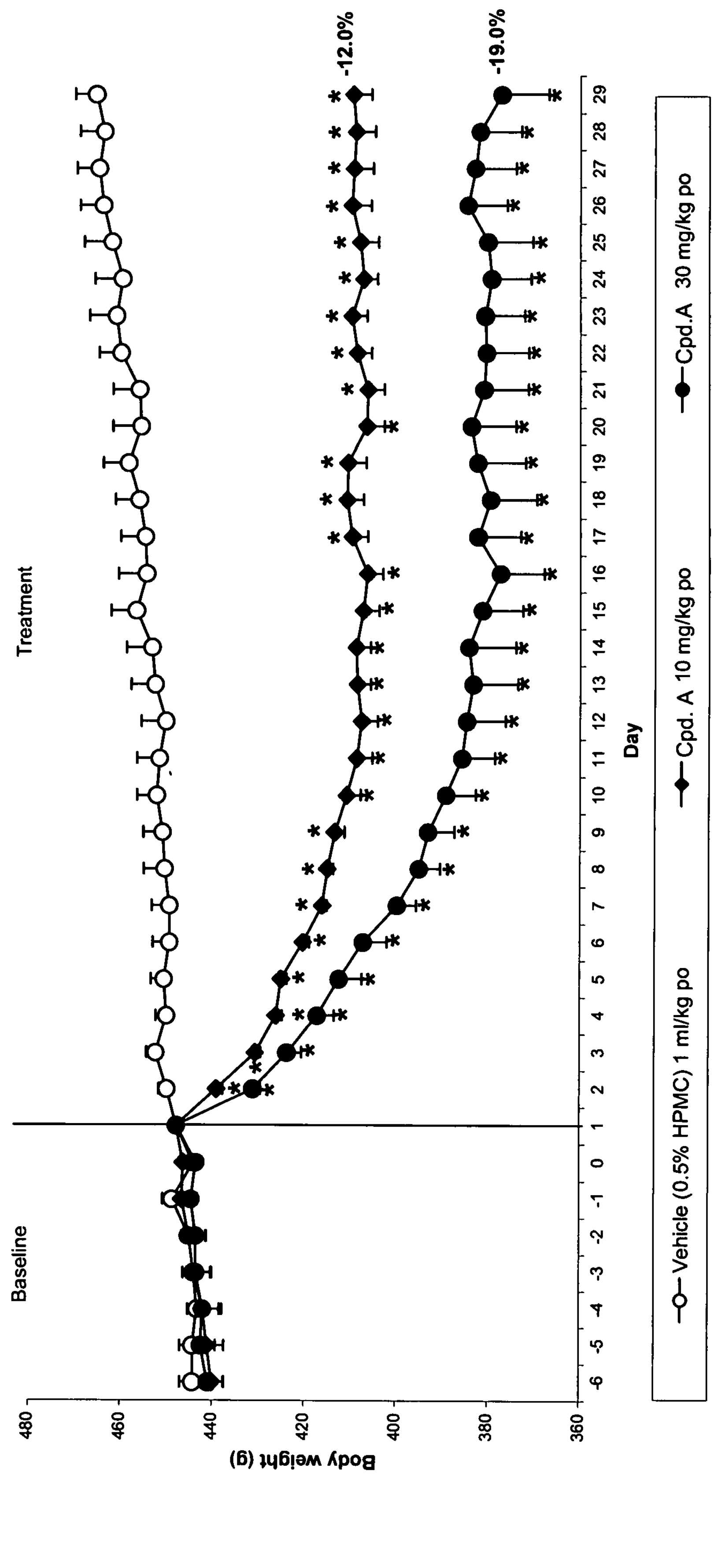
Baseline
Treatment
-12.0%
-19.0%
Body weight (g)
480
460
440
420
400
380
360
Day
-6 -5 -4 -3 -2 -1 0 1 2 3 4 5 6 7 8 9 10 11 12 13 14 15 16 17 18 19 20 21 22 23 24 25 26 27 28 29
Vehicle (0.5% HPMC) 1 ml/kg po
Cpd. A 10 mg/kg po
Cpd.A 30 mg/kg po

Figure 20:

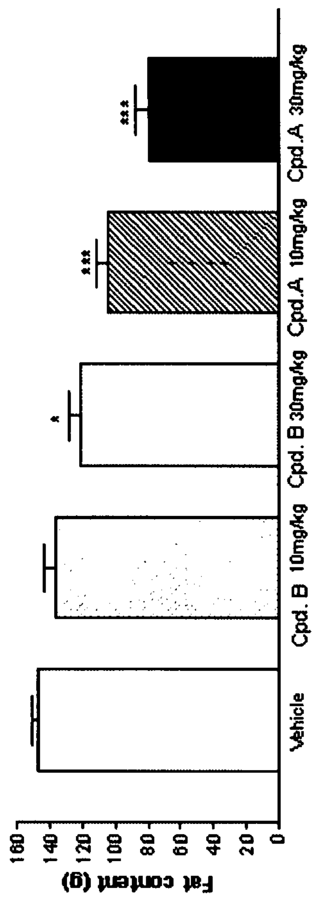
Fat content (g)
0
20
40
60
80
100
120
140
160
Vehicle
Cpd. B 10mg/kg
Cpd. B 30mg/kg
Cpd.A 10mg/kg
Cpd.A 30mg/kg
*
***
***

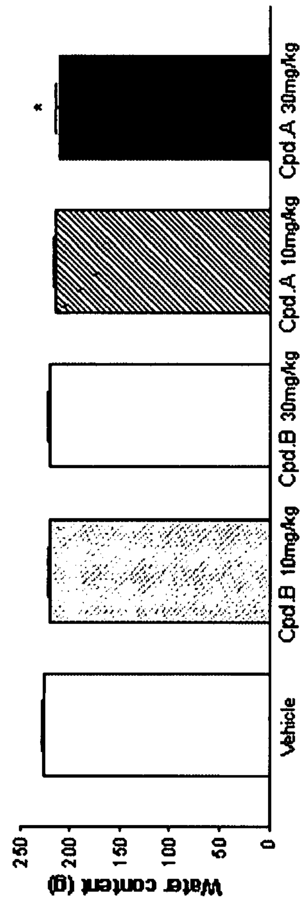
Water content (g)
250
200
150
100
50
0
Vehicle
Cpd.B 10mg/kg
Cpd.B 30mg/kg
Cpd.A 10mg/kg
Cpd.A 30mg/kg
*

Figure 21:

Figure 22:

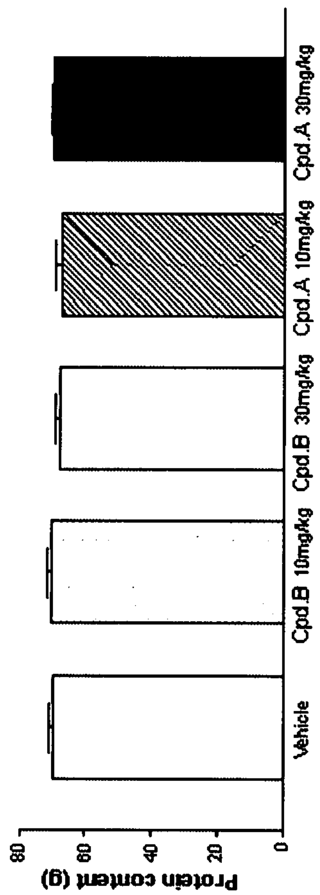
Protein content (g)
0
20
40
60
80
Vehicle
Cpd.B 10mg/kg
Cpd.B 30mg/kg
Cpd.A 10mg/kg
Cpd.A 30mg/kg

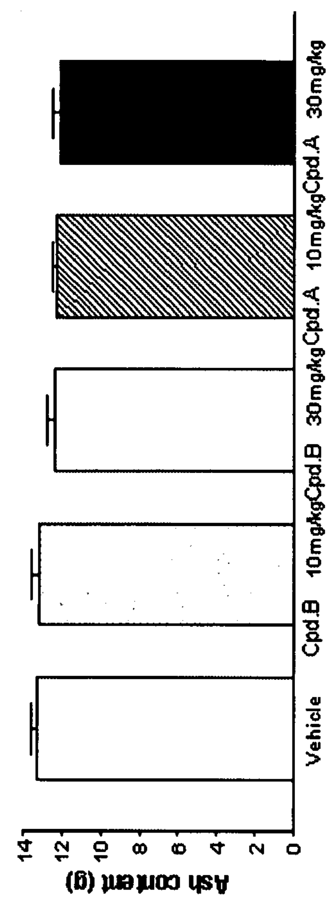
Ash content (g)
0
2
4
6
8
10
12
14
Vehicle
Cpd.B 10mg/kg
Cpd.B 30mg/kg
Cpd.A 10mg/kg
Cpd.A 30mg/kg

Figure 23:

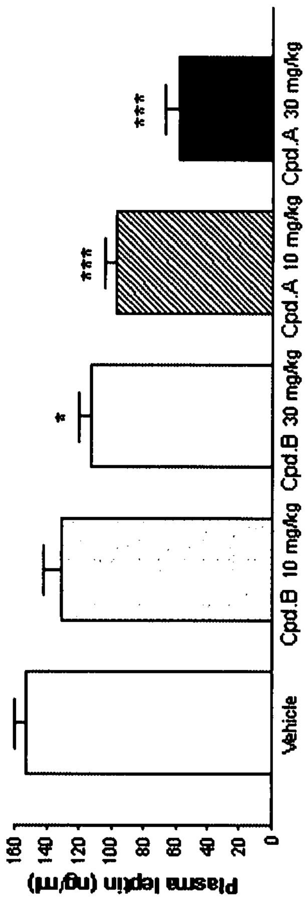
Plasma leptin (ng/ml)
160
140
120
100
80
60
40
20
0
Vehicle
Cpd.B 10 mg/kg
Cpd.B 30 mg/kg
Cpd.A 10 mg/kg
Cpd.A 30 mg/kg
*
***
***

Figure 24:

**Figure 17:**

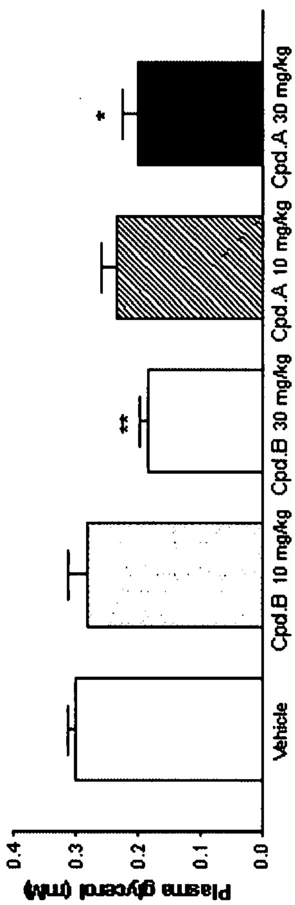
Plasma glycerol (mM)
0.0
0.1
0.2
0.3
0.4
Vehicle
Cpd.B 10 mg/kg
Cpd.B 30 mg/kg
Cpd.A 10 mg/kg
Cpd.A 30 mg/kg
**
*

Figure 25 Effect of chronic oral administration of olanzapine and Compound C dosed alone and in combination on the body weight of female Sprague-Dawley rats maintained on a high-fat diet

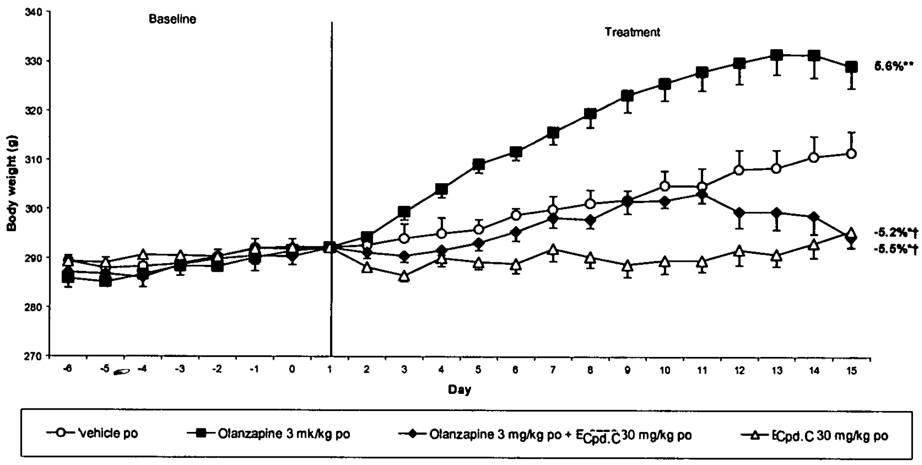


Data are means (adjusted for differences between the groups in body weight at Day 1). SEMs are calculated from residuals of the statistical model; n=10. Significant difference from vehicle assessed using Student's t-test: $^{*}p<0.02$, $^{**}p<0.01$ vs control, $^{\dagger}p<0.001$ vs olanzapine. Numbers represent % reduction compared to the vehicle-treated control group on Day 15 (ie after 14 days of treatment).

Figure 26 Effect of chronic oral administration of olanzapine and Compoud C dosed alone and in combination on the food intake of female Sprague-Dawley rats maintained on a high-fat diet

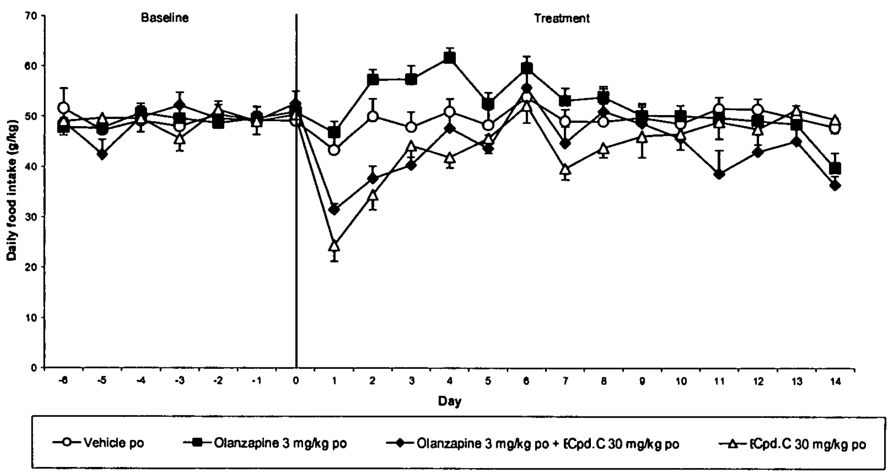


Data are means (adjusted for differences between treatment groups at baseline (average of Days -6 to 1)). SEMs are calculated from residuals of the statistical model; n=10.

Figure 27 Effect of chronic oral administration of olanzapine and Compound C dosed alone and in combination on the food intake of female Sprague-Dawley rats maintained on a high-fat diet expressed as average daily food intake per week

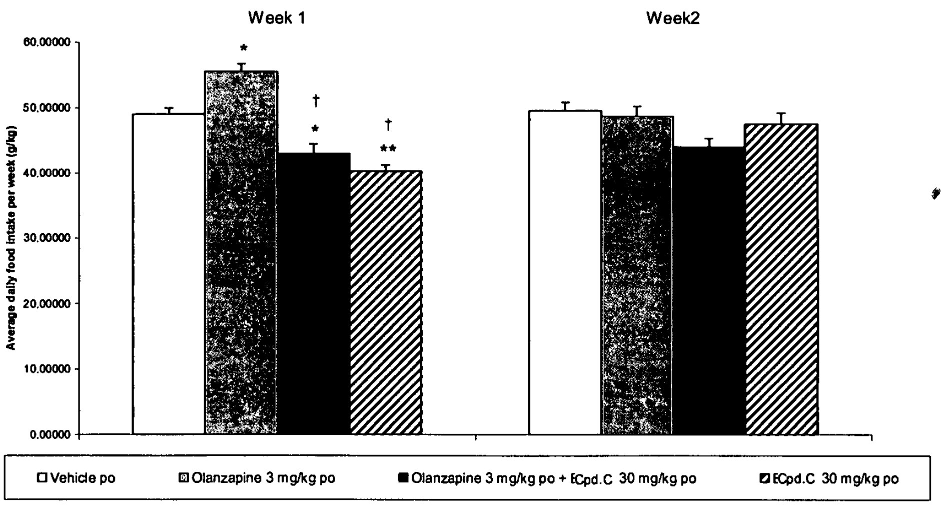


Data are means (adjusted for differences between treatment groups at baseline (average of Days -6 to 1)). SEMs are calculated from residuals of the statistical model; n=10. Data analysed by Student's t- test: *$p<0.05$, **$p<0.01$ vs vehicle, †$p<0.001$ vs olanzapine.

European Patent Office

# EUROPEAN SEARCH REPORT

Application Number
EP 07 38 4008

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | FOR THE RIO-EUROPE STUDY GROUP VAN GAAL L F ET AL: "Effects of the cannabinoid-1 receptor blocker rimonabant on weight reduction and cardiovascular risk factors in overweight patients: 1-year experience from the RIO-Europe study" LANCET THE, LANCET LIMITED. LONDON, GB, vol. 365, no. 9468, 16 April 2005 (2005-04-16), pages 1389-1397, XP004849986 ISSN: 0140-6736 * page 1395, left-hand column; table 2 * ----- | 1-20 | INV. A61K45/06 A61P3/04 |
| Y | LANGE J H M ET AL: "Keynote review: Medicinal chemistry strategies to CB1 cannabinoid receptor antagonists" DRUG DISCOVERY TODAY, ELSEVIER, RAHWAY, NJ, US, vol. 10, no. 10, 15 May 2005 (2005-05-15), pages 693-702, XP004894759 ISSN: 1359-6446 * page 696, left-hand column, paragraph 4 - page 697, right-hand column, paragraph 1 * ----- | 1-20 | TECHNICAL FIELDS SEARCHED (IPC) A61K A61P |
| Y | SCHEEN ANDRE J ET AL: "Efficacy and tolerability of rimonabant in overweight or obese patients with type 2 diabetes: a randomised controlled study" LANCET (NORTH AMERICAN EDITION), vol. 368, no. 9548, November 2006 (2006-11), pages 1660-1672, XP002436878 ISSN: 0140-6736 * abstract * * page 1665, left-hand column, paragraph 3 * ----- -/-- | 1-20 | |

The present search report has been drawn up for all claims

| Place of search | Date of completion of the search | Examiner |
|---|---|---|
| Munich | 8 June 2007 | ESCOLAR BLASCO, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

2

European Patent Office

# EUROPEAN SEARCH REPORT

Application Number
EP 07 38 4008

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | LANGE J H M ET AL: "BIOISOSTERIC REPLACEMENTS OF THE PYRAZOLE MOIETY OF RIMONABANT: SYNTHESIS, BIOLOGICAL PROPERTIES, AND MOLECULAR MODELING INVESTIGATIONS OF THIAZOLES, TRIAZOLES, AND IMIDAZOLES AS POTENT AND SELECTIVE CB1 CANNABINOID RECEPTOR ANTAGONISTS" JOURNAL OF MEDICINAL CHEMISTRY, AMERICAN CHEMICAL SOCIETY. WASHINGTON, US, vol. 48, 2005, pages 1823-1838, XP002339942 ISSN: 0022-2623 * page 1828, right-hand column; figure 1 * ----- | 1-20 | |
| X | US 2006/189658 A1 (CUBERES ALTISEN ROSA [ES] ET AL) 24 August 2006 (2006-08-24) * claims; examples * ----- | 1-20 | |
| A | WO 2005/074920 A (SOLVAY PHARM BV [NL]; LANGE JOSEPHUS H M [NL]; KRUSE CORNELIS G [NL];) 18 August 2005 (2005-08-18) * page 11, lines 15-38; claims; compounds 2-4 * ----- | 1-20 | TECHNICAL FIELDS SEARCHED (IPC) |
| A | GOBSHTIS NIKOLAI ET AL: "Antidepressant-induced undesirable weight gain: Prevention with rimonabant without interference with behavioral effectiveness" EUROPEAN JOURNAL OF PHARMACOLOGY, vol. 554, no. 2-3, 12 January 2007 (2007-01-12), pages 155-163, XP002436841 ISSN: 0014-2999 * abstract * ----- | 1-20 | |

The present search report has been drawn up for all claims

| Place of search | Date of completion of the search | Examiner |
|---|---|---|
| Munich | 8 June 2007 | ESCOLAR BLASCO, P |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
......................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 07 38 4008

This annex lists the patent family members relating to the patent documents cited in the above-mentioned European search report.
The members are as contained in the European Patent Office EDP file on
The European Patent Office is in no way liable for these particulars which are merely given for the purpose of information.

08-06-2007

| Patent document cited in search report | Publication date | Patent family member(s) | Publication date |
|---|---|---|---|
| US 2006189658 A1 | 24-08-2006 | NONE | |
| WO 2005074920 A | 18-08-2005 | AU 2005210163 A1 | 18-08-2005 |
| | | CA 2552940 A1 | 18-08-2005 |
| | | KR 20060131861 A | 20-12-2006 |
| | | MX PA06008593 A | 28-08-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 88005046 A


### Non-patent literature cited in the description


- *Synthetic Communications,* 1996, vol. 26 (11), 2229-33
- **PASCUAL, A.** *J. Prakt Chem.,* 1999, vol. 341 (7), 695-700
- **LIN, S. et al.** *Heterocycles,* 2001, vol. 55 (2), 265-277
- **RAO, P. et al.** *J. Org. Chem.,* 2000, vol. 65 (22), 7323-7344
- **PEARSON D.E ; BUEHLER, C.A.** *Synthesis,* 1972, 533-542
- *Tetrahedron Lett.,* 2004, vol. 45, 4977
- *Tetrahedron Lett.,* 1998, vol. 39, 1487
- *Tetrahedron Lett.,* 2002, vol. 43, 7685
- *Tetrahedron,* 2005, vol. 81, 5235-5240
- *Tetrahedron Asymmetry,* 2001, vol. 12, 1923-1928
- *Tetrahedron Lett.,* 1998, vol. 39 (44), 8163-8166
- *J. Chem. Soc. Perkin Trans 1,* 1999, vol. 21, 3069-3070
- *Tetrahedron,* 1999, vol. 55 (36), 11127-11142
- *J. Heterocyclic Chem.,* 1986, vol. 23, 1199
- *Chemistry of Heterocyclic Compounds,* 1997, vol. 33 (6
- *Indian J. Chem.,* 1981, vol. 20B, 1090
- *Indian J. Chem.,* 1990, vol. 29B, 887
- *J. Indian Chem. Soc.,* 1997, vol. 74 (3), 202-205
- *Tetrahedron,* 1994, vol. 50 (44), 12727-12742
- *Zhumal Obshchei Khimii,* 1986, vol. 56 (2), 347-353
- *J. Chem. Soc. Perkin Trans 1,* 1995, 741-742
- *J. Chem. Engineering Data,* 1984, vol. 29 (2), 225-229
- *Indian J. Chem.,* 1988, vol. 27B (3), 245-249
- *Bull. Chem. Soc. Japan,* 1984, vol. 57 (3), 787-790
- *Chem. Lett.,* 1982, 543-546
- *Synth. Commun.,* 2001, vol. 31 (1), 111-115
- *Tetrahedron,* 1994, vol. 50 (25), 7543-7556
- *Synthesis,* 1975, 333
- *J. Chem. Soc. Perkin Trans. 1,* 1977, 2092
- *Heterocycles,* 1991, vol. 32 (6), 1101-1107
- *J. Chem. Soc. Perkin Transaction 1,* 1998, vol. 24, 4103-4106
- *Tetrahedron Asymmetry,* 2000, vol. 11 (9), 1975-1983
- *Tetrahedron,* 1998, vol. 54 (49), 14859-14868
- *Synthesis,* 1996, vol. 9, 1076-1078
- *Synthesis,* 1995, vol. 12, 1483-1484
- *Chem. Lett.,* 1983, 507-510
- *Bull. Chem. Soc. Japan,* 1984, vol. 57 (9), 2689-2690
- *Synlett,* 1990, vol. 11, 705-706
- *J. Org. Chem.,* 2002, vol. 67, 6237-6239
- *J. Org. Chem.,* 1990, vol. 55, 3205-3209
- Protective groups in Organic Chemistry. Plenum Press, 1973
- **T.W. GREENE ; P.G.M. WUTS.** Protective Groups in Organic Chemistry. John Wiley & sons, 1991
- **R. B. SILVERMAN.** The Organic Chemistry of Drug Design and Drug Action. Academic Press, 1992
- Pharmaceutics: the Science of Dosage Forms. Churchill Livingstone, 2002
- Encyclopedia of Pharmaceutical Technology. Marcel Dekker, Inc, 2002
- Modem Pharmaceutics. Marcel Dekker, Inc, 2002
- The Theory and Practice of Industrial Pharmacy. Lea & Febiger, 1986
- Modified-Release Drug Delivery Technology. Marcel Dekker, Inc, 2002
- Handbook of Pharmaceutical Controlled Release Technology. Marcel Dekker, Inc, 2000
- Controlled Drug Delivery. Basic Concepts. CRC Press Inc, 1983, vol. I
- Oral Drug delivery. **TAKADA, K. ; YOSHIKAWA, H.** Encyclopedia of Controlled Drug Delivery. John Wiley & Sons, Inc, 1999, vol. 2, 728-742
- Oral drug delivery, small intestine and colon. **FIX, J.** Encylopedia of Controlled Drug Delivery. John Wiley & Sons, Inc, 1999, vol. 2, 698-728
- Pharmaceutical tablet coating. **JOHNSON, J.L.** Coatings Technology Handbook. Marcel Dekker, Inc, 2001, 863-866

- **CARSTENSEN, T.** Coating Tablets in Advanced Pharmaceutical Solids. Marcel Dekker, Inc, 2001, 455-468
- Coated dosage forms for colon-specific drug delivery. **LEOPOLD, C.S.** Pharmaceutical Science & Technology Today. 1999, vol. 2, 197-204
- **RHODES, C.T. ; PORTER, S.C.** Coatings, in Encyclopedia of Controlled Drug Delivery. John Wiley & Sons, Inc, 1999, vol. 1, 299-311
- Third Report of the National Cholesterol Education Program (NCEP) Expert Panel on Detection Evaluation, and Treatment of High Blood Cholesterol in Adults (Adult Treatment Panel III) Final Report. *Circulation,* 2002, vol. 106, 3143-3420
- **ARONNE LJ ; SEGAL KR.** Adiposity and fat distribution outcome measures: assessment and clinical implications. *Obes Res,* 2002, vol. 10, 14S-21S
- National Institute of Diabetes, Digestive and Kidney Diseases (NIDDKD). Prevention or delay of Type 2 diabetes. Diabetes Care. American Diabetes Association, 2004, vol. 27, S47-S54
- **BAYS H ; MANDARINO L ; DEFRONZO RA.** Mechanisms of endocrine disease. Role of the adipocyte, free fatty acids, and ectopic fat in pathogenesis of Type 2 diabetes mellitus: peroxisomal proliferator-activated receptor agonists provide a rational therapeutic approach. *J Clin Endocrine Metab,* 2004, vol. 89, 463-478
- **BERMUDEZ-SIVA FJ ; SERRANO A ; DIAZ-MOLINA FJ ; SANCHEZ VERA I ; JUAN-PICO P ; NADAL A ; FUENTES E ; RODRIGUEZ DE FONSECA F.** Activation of cannabinoid CB1 receptors induces glucose ntolerance in rats. *Eur J Pharmacol.,* 2006, vol. 531, 282-4
- **BODEN G ; LAAKSO M.** Lipids and glucose in Type 2 diabetes. What is the cause and effect. *Diabetes Care,* 2004, vol. 27, 2253-2259
- **COTA D ; MARSICANO G ; TSCHOP M ; GRUBLER Y ; FLACHSKAMM C ; SCHUBERT M ; AUER D ; YASSOURIDIS A ; THONE-REINEKE C ; ORTMANN S.** The endogenous cannabinoid system affects energy balance via central orexigenic drive and peripheral lipogenesis. *J Clin Invest.,* 2003, vol. 112, 423-31
- **GOVAERTS SJ ; HERMANS E ; LAMBERT DM.** Comparison of cannabinoid ligand affinities and efficacies in murine tissues and in transfected cells expressing human recombinant cannabinoid receptors. *Eur J Pharm Sci.,* 2004, vol. 23, 233-43
- **HAAPASALO-PESU KM ; SAARIJARVI S.** Olanzapine induces remarkable weight gain in adolescent patients. *Eur Child Adolesc Psychiatry,* 2001, vol. 10, 205-8
- IDF consensus worldwide definition of the Metabolic Syndrome. *IDF,* 2005, www.idf.org.
- **JUAN-PICO P ; FUENTES E ; BERMUDEZ-SILVA FJ ; JAVIER DIAZ-MOLINA F ; RIPOLL C ; RODRIGUEZ DE FONSECA F ; NADAL A.** Cannabinoid receptors regulate Ca(2+) signals and insulin secretion in pancreatic β-cell. *Cell Calcium.,* 2006, vol. 39, 155-62
- **MATIAS I ; GONTHIER MP ; ORLANDO P ; MARTIADIS V ; DE PETROCELLIS L ; CERVINO C ; PETROSINO S ; HOAREAU L ; FESTY F ; PASQUALI R.** Regulation, function, and dysregulation of endocannabinoids in models of adipose and β-pancreatic cells and in obesity and hyperglycemia. *J Clin Endocrinol Metab.,* 2006, vol. 91, 3171-80
- **MELKERSSON K ; DAHL ML.** Adverse metabolic effects associated with atypical antipsychotics: literature review and clinical implications. *Drugs,* 2004, vol. 64, 701-23
- **NEWCOMER JW.** Second-generation (atypical) antipsychotics and metabolic effects: a comprehensive literature review. *CNS Drugs,* 2005, vol. 19 (1), 1-93
- **OSEI-HYIAMAN D ; DEPETRILLO M ; PACHER P ; LIU J ; RADAEVA S ; BATKAI S ; HARVEY-WHITE J ; MACKIE K ; OFFERTALER L ; WANG L.** Endocannabinoid activation at hepatic CB1 receptors stimulates fatty acid synthesis and contributes to diet-induced obesity. *J Clin Invest.,* 2005, vol. 115, 1298-305
- **PIJL H ; MEINDERS AE.** Bodyweight change as an adverse effect of drug treatment. Mechanisms and management. *Drug Saf.,* 1996, vol. 14, 329-42
- **PI-SUNYER FX.** The obesity epidemic: pathophysiology and consequences of obesity. *Obes Res,* 2002, vol. 10, 97S-104S
- **RATZONI G ; GOTHELF D ; BRAND-GOTHELF A ; REIDMAN J ; KIKINZON L ; GAL G ; PHILLIP M ; APTER A ; WEIZMAN R.** Weight gain associated with olanzapine and risperidone in adolescent patients: a comparative prospective study. *J Am Acad Child Adolesc Psychiatry,* 2002, vol. 41, 337-43
- **REAVEN G ; ABBASSI F ; MCLAUGHLIN T.** Obesity, insulin resistance and cardiovascular disease. *J Clin Endocrine Metab,* 2004, 207-223
- **SHEEHAN MT.** Polycystic ovarian syndrome: diagnosis and management. *Clin Med Res,* 2004, vol. 2, 13-27
- WHO: World Health Organization. Definition, diagnosis and classification of diabetes mellitus and its complications. *Report of a WHO consultation,* 1999